# EUROPEAN PATENT APPLICATION

(11) **EP 2 821 493 A1**
(43) Date of publication of application: **07.01.2015**
(21) Application number: 14181169.5
(22) Date of filing: 19.05.2008
(51) Int. Cl.: C12N 15/82, C07K 14/415

(54) **Plants with increased tolerance and/or resistance to environmental stress and increased biomass production**

(30) Priority: 22.05.2007 EP 07108578; 22.05.2007 EP 07108579
(62) Divisional of application: 08759715.9
(71) Applicant: BASF Plant Science GmbH, 67056 Ludwigshafen (DE)
(72) Inventor: Bläsing, Oliver, 14482 Potsdam (DE); Puzio, Piotr, 9030 Mariakerke (BE); Thimm, Oliver, 67434 Neustadt (DE)
(74) Representative: Fitzner, Uwe

(57) **Abstract**

This invention relates generally to a plant cell with increased tolerance and/or resistance to environmental stress and increased biomass production as compared to a corresponding non-transformed wild type plant cell by increasing or generating one or more activities of polypeptides associated with abiotic stress responses and abiotic stress tolerance in plants.

## Description

This invention relates generally to a plant cell with increased tolerance and/or resistance to environmental stress and increased biomass production as compared to a corresponding non-transformed wild type plant cell by increasing or generating one or more activities of polypeptides associated with abiotic stress responses and abiotic stress tolerance in plants.

In particular, this invention relates to plants tailored to grow under conditions of water deficiency.

The invention also deals with methods of producing and screening for and breeding such plant cells or plants.

Under field conditions, plant performance in terms of growth, development, biomass accumulation and yield depends on acclimation ability to the environmental changes and stresses. Abiotic environmental stresses such as drought stress, salinity stress, heat stress and cold stress, are major limiting factors of plant growth and productivity (Boyer. 1982. Science 218, 443-448). Plants exposed to heat and/or low water or drought conditions typically have low yields of plant material, seeds, fruit and other edible products. Crop losses and crop yield losses of major crops such as rice, maize (corn) and wheat caused by these stresses represent a significant economic and political factor and contribute to food shortages in many underdeveloped countries.

Drought, heat, cold and salt stress have a common theme important for plant growth and that is water availability. Plants are typically exposed during their life cycle to conditions of reduced environmental water content. Most plants have evolved strategies to protect themselves against these conditions of low water or desiccation. However, if the severity and duration of the drought conditions are too great, the effects on plant development, growth and yield of most crop plants are profound. Continuous exposure to drought causes major alterations in the plant metabolism. These great changes in metabolism ultimately lead to cell death and consequently yield losses.

Developing stress-tolerant and/or resistant plants is a strategy that has the potential to solve or mediate at least some of these problems (McKersie and Leshem, 1994. Stress and Stress Coping in Cultivated Plants, Kluwer Academic Publishers). However, traditional plant breeding strategies to develop new lines of plants that exhibit resistance (tolerance) to these types of stress are relatively slow and require specific resistant lines for crossing with the desired line. Limited germplasm resources for stress tolerance and incompatibility in crosses between distantly related plant species represent significant problems encountered in conventional breeding. Additionally, the cellular processes leading to drought, cold and salt tolerance and/or resistance are complex in nature and involve multiple mechanisms of cellular adaptation and numerous metabolic pathways (McKersie and Leshem, 1994. Stress and Stress Coping in Cultivated Plants, Kluwer Academic Publishers). This multicomponent nature of stress tolerance and/or resistance has not only made breeding for tolerance and/or resistance largely unsuccessful, but has also limited the ability to genetically engineer stress tolerance plants using biotechnological methods.

Plants are exposed during their life cycle also to heat, cold and salt stress. The protection strategies are similar to those of drought resistance. Since high salt content in some soils results in less available water for cell intake, its effect is similar to those observed under drought conditions. Likewise, under freezing temperatures, plant cells loose water as a result of ice formation that starts in the apoplast and withdraws water from the symplast (McKersie and Leshem, 1994. Stress and Stress Coping in Cultivated Plants, Kluwer Academic Publishers). Physiologically these stresses are also interconnected and may induce similar cellular damage. For example drought and salt stress are manifested primarily as osmotic stress, leading to the disruption of homeostasis and ion distribution in the cell (Serrano et al., 1999; Zhu, 2001 a; Wang et al., 2003). Oxidative stress, which frequently accompanies high temperature, salinity or drought stress, may cause denaturation of functional or structural proteins (Smirnoff, 1998). As a consequence these abiotic stresses often activate similar signaling pathways (Shinozaki and Ymaguchi-Shinozaki, 2000; Knight and Knight, 2001; Zhu 2001 b, 2002) and cellular responses, e.g. the production of certain stress proteins, antioxidants and compatible solutes (Vierling and Kimpel, 1992; Zhu et al., 1997; .Cushman and Bohnert, 2000).

The results of current research indicate that drought tolerance and/or resistance is a complex quantitative trait and that no real diagnostic marker is available yet. This lack of a mechanistic understanding makes it difficult to design a transgenic approach to improve water stress tolerance and/or resistance.

At the moment many genetical and biotechnological approaches are known in order to obtain plants growing under conditions of low water availability.

These approaches are generally based on the introduction and expression of genes in plant cell coding for different enzymes as disclosed for example in WO2004011888, WO2006032708, US20050097640, US 20060037108, US20050108791, Serrano et al. (1999; Scientia Horticulturae 78: 261-269) and many others.

For example the overexpression of antioxidant enzymes or ROS-scavenging enzymes is one possibility to engineer tolerance, e.g.transgenic alfalfa plants expressing Mn-superoxide dismutase tend to have reduced injury after water-deficit stress (McKersie et al., 1996. Plant Physiol. 111, 1177-1181). These same transgenic plants have increased biomass production in field trials (McKersie et al., 1999. Plant Physiology, 119: 839-847; McKersie et al., 1996. Plant Physiol. 111, 1177-1181). Transgenic plants that overproduce osmolytes such as mannitol, fructans, proline or glycine-betaine also show increased resistance to some forms of abiotic stress and it is proposed that the synthesized osmolytes act as ROS scavengers (Tarczynski. et al. 1993 Science 259, 508-510; Sheveleva,. et al. 1997. Plant Physiol.115, 1211-1219).

The expression of genes from the family of glutaredoxin and thioredoxin confers increase tolerance to environmental stress, specially to salinity or cold (EP1 529 112 A). These plants had higher seed yields, photosynthesis and dry matter production than susceptible plants. Nothing is known about the development of these plants under condition of sparsly nutrient disposability.

Generally the transformed and stress resistant plants cited exhibit slower growth and reduced biomass, due to an imbalance in development and physiology of the plant, thus having significant fitness cost (Kasuga et al., 1999, Danby and Gehring et al., 2005). Despite maintaining basic metabolic function this leads to severe biomass and yield loss. Sometimes the root/shoot dry weight ratio increase as plant water stress develops. The increase is mostly due to a relative reduction in shoot dry weight. The ratio of seed yield to above-ground dry weight is relatively stable under many environmental conditions and so a robust correlation between plant size and grain yield can often be obtained. These processes are intrinsically linked because the majority of grain biomass is dependent on current stored photosynthetic productivity by the leaves and stem of the plant. Therefore selecting for plant size, even at early stages of development, has been used as an indicator for future potential.

In some cases (US20060037108) an increased biomass, mainly a greater shoot biomass was observed after a drought treatment by withholding water for 6 to 8 days.

There is still a need to identify genes expressed in stress tolerant plants that have the capacity to confer stress resistance to its host plant and to other plant species, specially to confer increased tolerance and/or resistance to environmental stress, preferably under conditions of water deficiency and confers increased biomass production. It is a object of this invention to identify new methods to confer stress tolerance and/or resistance in plants or plant cells. Complex traits of abiotic stress phenomena make genetic optimisation difficult. However, the modification of a single gene e.g. transcription factors or antiporters resulted in several cases in a significant increase in stress tolerance (Wang etal., 2003

It is further an object of this invention to put plants at disposal, which are water stress resistant for a period of at least 1.0, preferably 1.5 days of water deficiency as compared to a corresponding non-transformed wild type plant, and exhibit additionally under conditions of low water or desiccation an equal, preferably an increased biomass production.

There is further a need to identify genes expressed in stress tolerant plants that have the capacity to confer stress resistance and increased biomass production, specially under any sub-optimal growing condition.

Accordingly, in a first embodiment, the present invention provides a method for producing a transgenic plant cell with increased tolerance and/or resistance to environmental stress and increased biomass production as compared to a corresponding non-transformed wild type plant cell by increasing or generating one or more activities selected from the group consisting of: 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, YaI049c-protein, YCR059C-protein, YEL005C-protein, YER156C-protein, Yfr042w-protein, YGL045W-protein, and YOR024w-protein.

In one embodiment of the invention the proteins having a activity selected from the group consisting of: 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, YaI049c-protein, YCR059C-protein, YEL005C-protein, YER156C-protein, Yfr042w-protein, YGL045W-protein, and YOR024w-proteinand the polypeptides as depicted in table II, column 5 and 7 are named as "stress related protein" SRP.

As used herein, the term "environmental stress" refers to any sub-optimal growing condition and includes, but is not limited to, sub-optimal conditions associated with drought, cold or salinity or combinations thereof. In preferred embodiments, environmental stress is drought and low water content. Wherein drought stress means any environmental stress which leads to a lack of water in plants or reduction of water supply to plants.

In one embodiment of the invention the term "increased tolerance and/or resistance to environmental stress" relates to an increased resistance to water stress, which is produced as a secondary stress by cold, and salt, and, of course, as a primary stress during drought.

As used herein, the term "sub-optimal growing condition" refers also to limited nutrient availability and sub-optimal disposability.

In one embodiment, limited nutrient availability is drought and low water content.

In one embodiment, limited nutrient availability is a sub-optimal disposability in nutrients selected from the group consisting of phosphorus, potassium and nitrogen.

In one embodiment, limited nutrient availability is a sub-optimal disposability of nitrogen.

In one embodiment, the biomass of the transgenic plants of the invention is increased by an enhanced nutrient use efficiency (NUE). An improvement or increase in nutrient use efficiency of a plant may be manifested by improving a plant's general efficiency of nutrient assimilation (e.g. in terms of improvement of general nutrient uptake and/or transport, improving a plant's general transport mechanisms, assimilation pathway improvements, and the like), and/or by improving speciffic nutrient use efficiency of nutrients including, but not limited to, phosphorus, potassium, and nitrogen.
Plant nutrition is essential to the growth and development of plants and therefore also for quantity and quality of plant products. Because of the strong influence of the efficiency of nutrition uptake as well as nutrition utilization on plant yield and product quality, a huge amount of fertilizer is poured onto soils to optimize plant growth and quality.

In the present invention, the enhanced tolerance to limited nutrient availability may, for example and preferably, be determined according to the following method:

For high-throughput purposes plants are screened for biomass production on agar plates with limited supply of nitrogen (adapted from Estelle and Somerville, 1987). This screening pipeline consists of two level. Transgenic lines are subjected to subsequent level if biomass production is significantly improved in comparison to wild type plants. With each level number of replicates and statistical stringency is increased.
For the sowing, the seeds, which are stored in the refrigerator (at -20°C), are removed from the Eppendorf tubes with the aid of a toothpick and transferred onto the above-mentioned agar plates, with limited supply of nitrogen (0.05 mM KNO₃).
After the seeds have been sown, plates are subjected to stratification for 2-4 days in the dark at 4°C. After the stratification, the test plants are grown for 22 to 25 days at a 16-h-light, 8-h-dark rhythm at 20°C, an atmospheric humidity of 60% and a CO₂ concentration of approximately 400 ppm. The light sources used generates a light resembling the solar color spectrum with a light intensity of approximately 100 µE/m²s. After 10 to 11 days the plants are individualized. Improved growth under nitrogen limited conditions is assessed by biomass production of shoots and roots of transgenic plants in comparison to wild type control plants after 20-25 days growth.
Transgenic lines showing a significant improved biomass production in comparison to wild type plants are subjected to following experiment of the subsequent level:
In case of Arabidopsis thaliana, the seeds are sown in pots containing a 1:1 (v:v) mixture of nutrient depleted soil ("Einheitserde Typ 0", 30% clay, Tantau, Wansdorf Germany) and sand. Germination is induced by a four day period at 4°C, in the dark. Subsequently the plants are grown under standard growth conditions (photoperiod of 16 h light and 8 h dark, 20 °C, 60% relative humidity, and a photon flux density of 200 µE).
The plants are grown and cultured, inter alia they are watered every second day with a N-depleted nutrient solution. The N-depleted nutrient solution e.g. contains beneath water

| mineral nutrient | final concentration |
|---|---|
| KCl | 3.00 mM |
| MgSO₄ x 7 H₂O | 0.5 mM |
| CaCl₂ x 6 H₂O | 1.5 mM |
| K₂SO₄ | 1.5 mM |
| NaH₂PO₄ | 1.5 mM |
| Fe-EDTA | 40 µM |
| H₃BO₃ | 25 µM |
| MnSO₄ x H₂O | 1 µM |
| ZnSO₄ x 7H₂O | 0.5 µM |
| Cu₂SO₄ x 5 H₂O | 0.3 µM |
| Na₂MoO₄ x 2 H₂O | 0.05 µM |

After 9 to 10 days the plants are individualized. After a total time of 29 to 31 days the plants are harvested and rated by the fresh weight of the arial parts of the plants. The biomass increase ismeasured as ratio of the fresh weight of the aerial parts of the respective transgene plant and the non-transgenic wild type plant.

Accordingly, in one embodiment of the invention, the transgenic plant of the invention manifests a biomass increase compared to a wilod type control under the stress condition of limited nutrient, preferably nitrogen availability.

In one embodiment of the invention, the term "environmental stress" encompass even the absence of substantial abiotic stress.

In the present invention, the biomass increase may, for example and preferably, be determined according to the following method:

Transformed plants are grown in pots in a growth chamber (e.g. York, Mannheim, Germany). In case the plants are Arabidopsis thaliana seeds thereof are sown in pots containing a 3.5:1 (v:v) mixture of nutrient rich soil (GS90, Tantau, Wansdorf, Germany). Plants are grown under standard growth conditions. In case the plants are Arabidopsis thaliana, the standard growth conditions are: photoperiod of 16 h light and 8 h dark, 20 °C, 60% relative humidity, and a photon flux density of 220 µmol/m²s. Plants are grown and cultured. In case the plants are Arabidopsis thaliana they are watered every second day. After 13 to 14 days the plants are individualized. Transgenic events and wildtype control plants are evenly distributed over the chamber. Watering is carried out every two days after removing the covers in a standard experiment or, alternatively, every day. For measuring biomass performance, plant fresh weight is determined at harvest time (26-27 days after sowing) by cutting shoots and weighing them. Alternatively, the harvest time is 24-25 days after sowing. Besides weighing, phenotypic information is added in case of plants that differ from the wild type control. Plants are in the stage prior to flowering and prior to growth of inflorescence when harvested.

Accordingly, in one embodiment of the invention, the transgenic plant of the invention manifests a biomass increase compared to a wilod type control under the stress condition of low temperature.

In one embodiment of the invention the term " increased tolerance and/or resistance to environmental stress" relates to an increased cold resistance.

In one embodiment of the invention the term "increased cold resistance" relates to low temperature tolerance, comprising freezing tolerance and/or chilling tolerance.

Further, improved or enhanced "chilling tolerance" or variations thereof refers to improved adaptation to low but non-freezing temperatures around 10 °C, preferably temperatures between 1 to 18 °C, more preferably 4-14 °C, and most preferred 8 to 12 °C, 11 to 12 °C; hereinafter called "chilling temperature".

Improved or enhanced "freezing tolerance" or variations thereof refers to improved adaptation to temperatures near or below zero, namely preferably temperatures below 4 °C, more preferably below 3 or 2 °C, and particularly preferred at or below 0 (zero) °C or below -4 °C, or even extremely low temperatures down to -10 °C or lower; hereinafter called "freezing temperature.

More generally, "improved adaptation" to environmental stress like low temperatures e.g. freezing and/or chilling temperatures refers to increased biomass production as compared to a corresponding non-transformed wild type plant.

Accordingly, for the purposes of the description of the present invention, the term "low temperature" with respect to low temperature stress on a plant, and preferably a crop plant, refers to any of the low temperature conditions as described herein, preferably chilling and/or freezing temperatures as defined above, as the context requires. It is understood that a skilled artisan will be able to recognize from the particular context in the present description which temperature or temperature range is meant by "low temperature".

In the present invention, enhanced tolerance to low temperature may, for example and preferably, be determined according to the following method:

Transformed plants are grown in pots in a growth chamber (e.g. York, Mannheim, Germany). In case the plants are Arabidopsis thaliana seeds thereof are sown in pots containing a 3.5:1 (v:v) mixture of nutrient rich soil (GS90, Tantau, Wansdorf, Germany). Plants are grown under standard growth conditions. In case the plants are Arabidopsis thaliana, the standard growth conditions are: photoperiod of 16 h light and 8 h dark, 20 °C, 60% relative humidity, and a photon flux density of 200 µmol/m²s. Plants are grown and cultured. In case the plants are Arabidopsis thaliana they are watered every second day. After 12 to 13 days the plants are individualized. Cold (e.g. chilling at 11 - 12 °C) is applied 14 days after sowing until the end of the experiment. For measuring biomass performance, plant fresh weight was determined at harvest time (29-30 days after sowing) by cutting shoots and weighing them. Beside weighing, phenotypic information was added in case of plants that differ from the wild type control.

Accordingly, in one embodiment of the invention, the increased cold resistance manifests in an biomass increase of the transgenic plant of the invention compared to a wilod type control under the stress condition of low temperature.

In one embodiment of the invention the term "increased tolerance and/or resistance to environmental stress" relates to an increased cold resistance, meaning to low temperature tolerance, comprising freezing tolerance and/or chilling tolerance.

In one embodiment of the invention the term "increased tolerance and/or resistance to environmental stress" relates to an increased salt resistance.

In a preferred embodiment of the invention the term "increased tolerance and/or resistance to environmental stress" relates to an increased drought resistance.

In one embodiment increased drought resistance refers to resistance to drought cycles, meaning alternating periods of drought and re-watering.

In the present invention, enhanced tolerance to low temperature may, for example and preferably, be determined according to the following method:

Transformed plants are grown in pots in a growth chamber (e.g. York, Mannheim, Germany). In case the plants are Arabidopsis thaliana seeds thereof are sown in pots containing a 1:1 (v:v) mixture of nutrient rich soil (GS90, Tantau, Wansdorf, Germany). Plants are grown under standard growth conditions. In case the plants are Arabidopsis thaliana, the standard growth conditions are: photoperiod of 16 h light and 8 h dark, 20 °C, 60% relative humidity, and a photon flux density of 220 µmol/m²s. Plants are grown and cultured. After 13 to 14 days the plants are individualized. The water supply throughout the experiment was limited and plants were subjected to cycles of drought and re-watering. In case the plants are Arabidopsis thaliana watering is carried out at day 1 (before sowing), day 14 or day 15, day 21 or day 22, and, finally, day 27 or day 28. For measuring biomass production, plant fresh weight is determined one day after the final watering (day 28 or day 29) by cutting shoots and weighing them. Plants are in the stage prior to flowering and prior to growth of inflorescence when harvested. Significance values for the statistical significance of the biomass changes are calculated by applying the 'student's' t test (parameters: two-sided, unequal variance). Beside weighing, phenotypic information was added in case of plants that differ from the wild type control.

Accordingly, in one embodiment of the invention, the increased cold resistance manifests in an biomass increase of the transgenic plant of the invention compared to a wilod type control under the stress condition of cycling drought.

In an other preferred embodiment of the invention the term " increased tolerance and/or resistance to environmental stress" relates to an increased resistance to water stress, e.g. drought, cold and salt resistance. Water stress relates to conditions of low water or desiccation.

In one embodiment of the invention the term "increased tolerance and/or resistance to environmental stress" is defined as survival of plants under drought conditions longer than non-transformed wild type plant.
Drought conditions means under conditions of water deficiency, in other words the plants survives and growth under conditions of water deficiency in Arabidopsis for a period of at least 10, preferably 11, 12, more preferably 13 day or more without showing any symptoms of injury, such as wilting and leaf browning and/or rolling, on the other hand the plants being visually turgid and healthy green in color.

In one embodiment of the invention the term "increased biomass production" means that the plants exhibit an increased growth rate from the starting of withholding water as compared to a corresponding non-transformed wild type plant. An increased growth rate comprises an increased in biomass production of the whole plant, an increase in biomass of the visible part of the plant, e.g. of stem and leaves and florescence, visible higher and larger stem.

In one embodiment increased biomass production includes higher seed yield, higher photosynthesis and/or higher dry matter production.

In one embodiment of the invention the term "increased biomass production" means that the plants exhibit an prolonged growth from the starting of withholding water as compared to a corresponding non-transformed wild type plant. An prolonged growth comprises survival and/or continued growth of the whole plant at the moment when the non-transformed wild type plants show visual symptoms of injury.

In one embodiment of the invention the term "increased biomass production" means that the plants exhibit an increased growth rate and prolonged growth from the starting of withholding water as compared to a corresponding non-transformed wild type plant.

In one embodiment this invention fulfills in part the need to identify new, unique genes capable of conferring stress tolerance in combination with an increase in biomass production to plants upon expression or over-expression of endogenous and/or exogenous genes.

Accordingly, the present invention relates to a method for producing a transgenic plant cell, a plant or a part thereof with increased tolerance and/or resistance to environmental stress and increased biomass production as compared to a corresponding non-transformed wild type plant cell, a plant or a part thereof, which comprises
(a) increasing or generating one or more activities selected from the group consisting of: 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, YaI049c-protein, YCR059C-protein, YEL005C-protein, YER156C-protein, Yfr042w-protein, YGL045W-protein, and YOR024w-protein. in plant cell, a plant or a part thereof,
   and
(b) growing the plant cell, a plant or a part thereof under conditions which permit the development of a plant with increased tolerance and/or resistance to environmental stress and increased biomass production as compared to a corresponding non-transformed wild type plant.

In a preferred embodiment the present invention relates to a method for producing a transgenic plant cell, a plant or a part thereof with increased tolerance and/or resistance to environmental stress and increased biomass production as compared to a corresponding non-transformed wild type plant cell, a plant or a part thereof, which comprises
(a) increasing or generating one or more activities selected from the group consisting of: 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, YaI049c-protein, YCR059C-protein, YEL005C-protein, YER156C-protein, Yfr042w-protein, YGL045W-protein, and YOR024w-protein. in plant cell, a plant or a part thereof,
   and
(b) growing the plant cell, a plant or a part thereof together with non-transformed wildtype plants
(c) imposing at water stress by withholding water,
(d) after the non-transformed wild type plants show visual symptoms of injury selecting the plant with increased tolerance and/or resistance to environmental stress and increased biomass production as compared to a corresponding non-transformed wild type plant.

In one embodiment of the invention the increased water stress resistance is determinated and quantified according to the following method:

Transformed plants are grown individually in pots in a growth chamber (York Industriekälte GmbH, Mannheim, Germany).
Germination is induced. In case the plants are Arabidopsis thaliana sown seeds are kept at 4°C, in the dark, for 3 days in order to induce germination. Subsequently conditions are changed for 3 days to 20°C/6°C day/night temperature with a 16/8h day-night cycle at 150 µE/m²s.
Subsequently the plants are grown under standard growth conditions. In case the plants are Arabidopsis thaliana, the standard growth conditions are: photoperiod of 16 h light and 8 h dark, 20 °C, 60% relative humidity, and a photon flux density of 200 µE. Plants are grown and cultured until they develop leaves. In case the plants are Arabidopsis thaliana they are watered daily until they were approximately 3 weeks old. Starting at that time drought was imposed by withholding water.

After the non-transformed wild type plants show visual symptoms of injury, the evaluation starts and plants are scored for symptoms of drought symptoms and biomass production comparison to wild type and neighboring plants for 5 - 6 days in succession.

Visual symptoms of injury stating for one or any combination of two, three or more of the following features:
a) wilting
b) leaf browning
c) loss of turgor, which results in drooping of leaves or needles stems, and flowers,
d) drooping and/or shedding of leaves or needles,
e) the leaves are green but leaf angled slightly toward the ground compared with controls,
f) leaf blades begun to fold (curl) inward,
g) premature senescence of leaves or needles,
h) loss of chlorophyll in leaves or needles and/or yellowing.

In one embodiment the present invention relates to a method for producing a transgenic plant cell, a plant or a part thereof with increased tolerance and/or resistance to environmental stress and increased biomass production as compared to a corresponding non-transformed wild type plant cell, a plant or a part thereof, which comprises
(a) increasing or generating the activity of a protein as shown in table II, column 3 encoded by the nucleic acid sequences as shown in table I, column 5, in plant cell, a plant or a part thereof,
   and
(b) growing the plant cell, a plant or a part thereof under conditions which permit the development of a plant with increased tolerance and/or resistance to environmental stress and increased biomass production as compared to a corresponding non-transformed wild type plant.

Accordingly, the present invention relates to a method for producing a transgenic plant cell, a plant or a part thereof with increased tolerance and/or resistance to environmental stress and increased biomass production as compared to a corresponding non-transformed wild type plant cell, a plant or a part thereof, which comprises
(a) increasing or generating one or more activities selected from the group consisting of: 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, YaI049c-protein, YCR059C-protein, YEL005C-protein, YER156C-protein, Yfr042w-protein, YGL045W-protein, and YOR024w-protein. in the plastid of a plant cell,
   and
(b) growing the plant cell under conditions which permit the development of a plant with increased tolerance and/or resistance to environmental stress and increased biomass production as compared to a corresponding non-transformed wild type plant.

In one embodiment the present invention relates to a method for producing a transgenic plant cell, a plant or a part thereof with increased tolerance and/or resistance to environmental stress and increased biomass production as compared to a corresponding non-transformed wild type plant cell, a plant or a part thereof, which comprises
(a) increasing or generating the activity of a protein as shown in table II, column 3 encoded by the nucleic acid sequences as shown in table I, column 5 or 7, in the plastid of a plant cell,
   and
(b) growing the plant cell under conditions which permit the development of a plant with increased tolerance and/or resistance to environmental stress and increased biomass production as compared to a corresponding non-transformed wild type plant.

In another embodiment the present invention is related to a method for producing a transgenic plant cell, a plant or a part thereof with increased tolerance and/or resistance to environmental stress and increased biomass production as compared to a corresponding non-transformed wild type plant cell, a plant or a part thereof, which comprises
(a) increasing or generating one or more activities selected from the group consisting of: 2,3-dihydroxy-2,3-dihydrophenylpropionatedehydrogenase, 3-deoxy-D-arabinoheptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, YaI049c-protein, YCR059C-protein, YEL005C-protein, YER156C-protein, Yfr042w-protein, YGL045W-protein, and YOR024w-protein. in an organelle of a plant cell or
(b) increasing or generating the activity of a protein as shown in table II, column 3 encoded by the nucleic acid sequences as shown in table I, column 5 or 7, which are joined to a nucleic acid sequence encoding a transit peptide in a plant cell; or
(c) increasing or generating the activity of a protein as shown in table II, column 3 encoded by the nucleic acid sequences as shown in table I, column 5 or 7, which are joined to a nucleic acid sequence encoding chloroplast localization sequence, in a plant cell, and
(d) growing the plant cell under conditions which permit the development of a plant with increased tolerance and/or resistance to environmental stress and increased biomass production as compared to a corresponding non-transformed wild type plant.

In another embodiment, the present invention relates to a method for producing a transgenic plant cell, a plant or a part thereof with increased tolerance and/or resistance to environmental stress and increased biomass production as compared to a corresponding non-transformed wild type plant cell, a plant or a part thereof, which comprises
(a) increasing or generating the activity of a protein as shown in table II, column 3 encoded by the nucleic acid sequences as shown in table I, column 5 or 7, in an organelle of a plant through the transformation of the organelle, or
(b) increasing or generating the activity of a protein as shown in table II, column 3 encoded by the nucleic acid sequences as shown in table I, column 5 or 7 in the plastid of a plant, or in one or more parts thereof through the transformation of the plastids; and
(c) growing the plant cell under conditions which permit the development of a plant with increased tolerance and/or resistance to environmental stress and increased biomass production as compared to a corresponding non-transformed wild type plant.

In principle the nucleic acid sequence encoding a transit peptide can be isolated from every organism such as microorganisms such as algae or plants containing plastids preferably chloroplasts. A "transit peptide" is an amino acid sequence, whose encoding nucleic acid sequence is translated together with the corresponding structural gene. That means the transit peptide is an integral part of the translated protein and forms an amino terminal extension of the protein. Both are translated as so called "preprotein". In general the transit peptide is cleaved off from the preprotein during or just after import of the protein into the correct cell organelle such as a plastid to yield the mature protein. The transit peptide ensures correct localization of the mature protein by facilitating the transport of proteins through intracellular membranes. Preferred nucleic acid sequences encoding a transit peptide are derived from a nucleic acid sequence encoding a protein finally resided in the plastid and stemming from an organism selected from the group consisting of the genera

Acetabularia, Arabidopsis, Brassica, Capsicum, Chlamydomonas, Cururbita, Dunaliella, Euglena, Flaveria, Glycine, Helianthus, Hordeum, Lemna, Lolium, Lycopersion, Malus, Medicago, Mesembryanthemum, Nicotiana, Oenotherea, Oryza, Petunia, Phaseolus, Physcomitrella, Pinus, Pisum, Raphanus, Silene, Sinapis, Solanum, Spinacea, Stevia, Synechococcus, Triticum and Zea.

Advantageously such transit peptides, which are beneficially used in the inventive process, are derived from the nucleic acid sequence encoding a protein selected from the group consisting of

ribulose bisphosphate carboxylase/oxygenase, 5-enolpyruvylshikimate-3-phosphate synthase, acetolactate synthase, chloroplast ribosomal protein CS17, Cs protein, ferredoxin, plastocyanin, ribulose bisphosphate carboxylase activase, tryptophan synthase, acyl carrier protein, plastid chaperonin-60, cytochrome c₅₅₂, 22-kDA heat shock protein, 33-kDa Oxygen-evolving enhancer protein 1, ATP synthase γ subunit, ATP synthase δ subunit, chlorophyll-a/b-binding proteinII-1, Oxygenevolving enhancer protein 2, Oxygen-evolving enhancer protein 3, photosystem I: P21, photosystem I: P28, photosystem I: P30, photosystem I: P35, photosystem I: P37, glycerol-3-phosphate acyltransferases, chlorophyll a/b binding protein, CAB2 protein, hydroxymethyl-bilane synthase, pyruvate-orthophosphate dikinase, CAB3 protein, plastid ferritin, ferritin, early light-inducible protein, glutamate-1-semialdehyde aminotransferase, protochlorophyllide reductase, starch-granule-bound amylase synthase, light-harvesting chlorophyll a/b-binding protein of photosystem II, major pollen allergen Lol p 5a, plastid ClₚB ATP-dependent protease, superoxide dismutase, ferredoxin NADP oxidoreductase, 28-kDa ribonucleoprotein, 31-kDa ribonucleoprotein, 33-kDa ribonucleoprotein, acetolactate synthase, ATP synthase CF₀ subunit 1, ATP synthase CF₀ subunit 2, ATP synthase CFo subunit 3, ATP synthase CFo subunit 4, cytochrome f, ADP-glucose pyrophosphorylase, glutamine synthase, glutamine synthase 2, carbonic anhydrase, GapA protein, heat-shock-protein hsp21, phosphate translocator, plastid CIₚA ATP-dependent protease, plastid ribosomal protein CL24, plastid ribosomal protein CL9, plastid ribosomal protein PsCL18, plastid ribosomal protein PsCL25, DAHP synthase, starch phosphorylase, root acyl carrier protein II, betaine-aldehyde dehydrogenase, GapB protein, glutamine synthetase 2, phosphoribulokinase, nitrite reductase, ribosomal protein L12, ribosomal protein L13, ribosomal protein L21, ribosomal protein L35, ribosomal protein L40, triose phosphate-3-phosphoglyerate-phosphate translocator, ferredoxin-dependent glutamate synthase, glyceraldehyde-3-phosphate dehydrogenase, NADP-dependent malic enzyme and NADP-malate dehydrogenase.

More preferred the nucleic acid sequence encoding a transit peptide is derived from a nucleic acid sequence encoding a protein finally resided in the plastid and stemming from an organism selected from the group consisting of the species:

Acetabularia mediterranea, Arabidopsis thaliana, Brassica campestris, Brassica napus, Capsicum annuum, Chlamydomonas reinhardtii, Cururbita moschata, Dunaliella salina, Dunaliella tertiolecta, Euglena gracilis, Flaveria trinervia, Glycine max, Helianthus annuus, Hordeum vulgare, Lemna gibba, Lolium perenne, Lycopersion esculentum, Malus domestica, Medicago falcata, Medicago sativa, Mesembryanthemum crystallinum, Nicotiana plumbaginifolia, Nicotiana sylvestris, Nicotiana tabacum, Oenotherea hookeri, Oryza sativa, Petunia hybrida, Phaseolus vulgaris, Physcomitrella patens, Pinus tunbergii, Pisum sativum, Raphanus sativus, Silene pratensis, Sinapis alba, Solanum tuberosum, Spinacea oleracea, Stevia rebaudiana, Synechococcus, Synechocystis, Triticum aestivum and Zea mays.

Even more preferred nucleic acid sequences are encoding transit peptides as disclosed by von Heijne et al. [Plant Molecular Biology Reporter, Vol. 9 (2), 1991: 104 - 126], which are hereby incorparated by reference. Table V shows some examples of the transit peptide sequences disclosed by von Heijne et al. According to the disclosure of the invention especially in the examples the skilled worker is able to link other nucleic acid sequences disclosed by von Heijne et al. to the nucleic acid sequences shown in table I, columns 5 and 7. Most preferred nucleic acid sequences encoding transit peptides are derived from the genus Spinacia such as chlorplast 30S ribosomal protein PSrp-1, root acyl carrier protein II, acyl carrier protein, ATP synthase: γ subunit, ATP synthase: δ subunit, cytochrom f, ferredoxin I, ferredoxin NADP oxidoreductase (= FNR), nitrite reductase, phosphoribulokinase, plastocyanin or carbonic anhydrase. The skilled worker will recognize that various other nucleic acid sequences encoding transit peptides can easely isolated from plastid-localized proteins, which are expressed from nuclear genes as precursors and are then targeted to plastids. Such transit peptides encoding sequences can be used for the construction of other expression constructs. The transit peptides advantageously used in the inventive process and which are part of the inventive nucleic acid sequences and proteins are typically 20 to 120 amino acids, preferably 25 to 110, 30 to 100 or 35 to 90 amino acids, more preferably 40 to 85 amino acids and most preferably 45 to 80 amino acids in length and functions post-translationally to direct the protein to the plastid preferably to the chloroplast. The nucleic acid sequences encoding such transit peptides are localized upstream of nucleic acid sequence encoding the mature protein. For the correct molecular joining of the transit peptide encoding nucleic acid and the nucleic acid encoding the protein to be targeted it is sometimes necessary to introduce additional base pairs at the joining position, which forms restriction enzyme recognition sequences useful for the molecular joining of the different nucleic acid molecules. This procedure might lead to very few additional amino acids at the N-terminal of the mature imported protein, which usually and preferably do not interfer with the protein function. In any case, the additional base pairs at the joining position which forms restriction enzyme recognition sequences have to be choosen with care, in order to avoid the formation of stop codons or codons which encode amino acids with a strong influence on protein folding, like e.g. proline. It is preferred that such additional codons encode small structural flexible amino acids such as glycine or alanine.

As mentioned above the nucleic acid sequences coding for the proteins as shown in table II, column 3 and its homologs as disclosed in table I, columns 5 and 7 can be joined to a nucleic acid sequence encoding a transit peptide. This nucleic acid sequence encoding a transit peptide ensures transport of the protein to the plastid. The nucleic acid sequence of the gene to be expressed and the nucleic acid sequence encoding the transit peptide are operably linked. Therefore the transit peptide is fused in frame to the nucleic acid sequence coding for proteins as shown in table II, column 3 and its homologs as disclosed in table I, columns 5 and 7.

The term "organelle" according to the invention shall mean for example "mitochondria" or preferably "plastid" (throughout the specification the "plural" shall comprise the "singular" and vice versa). The term "plastid" according to the invention are intended to include various forms of plastids including proplastids, chloroplasts, chromoplasts, gerontoplasts, leucoplasts, amyloplasts, elaioplasts and etioplasts preferably chloroplasts. They all have as a common ancestor the aforementioned proplasts.

Other transit peptides are disclosed by Schmidt et al. [J. Biol. Chem., Vol. 268, No. 36, 1993: 27447 - 27457], Della-Cioppa et al. [Plant. Physiol. 84, 1987: 965 - 968], de Castro Silva Filho et al. [Plant Mol. Biol., 30, 1996: 769 - 780], Zhao et al. [J. Biol. Chem. Vol. 270, No. 11, 1995: 6081 - 6087], Römer et al. [Biochem. Biophys. Res. Commun., Vol. 196, No. 3, 1993 : 1414 - 1421], Keegstra et al. [Annu. Rev. Plant Physiol. Plant Mol. Biol., 40, 1989: 471 - 501], Lubben et al. [Photosynthesis Res., 17, 1988: 173 - 194] and Lawrence et al. [J. Biol. Chem., Vol. 272, No. 33, 1997: 20357 - 20363]. A general review about targeting is disclosed by Kermode Allison R. in Critical Reviews in Plant Science 15 (4): 285 - 423 (1996) under the title "Mechanisms of Intracellular Protein Transport and Targeting in Plant Cells."

Favored transit peptide sequences, which are used in the inventive process and which forms part of the inventive nucleic acid sequences are generally enriched in hydroxylated amino acid residues (serine and threonine), with these two residues generally constituting 20 - 35 % of the total. They often have an amino-terminal region empty of Gly, Pro, and charged residues. Furthermore they have a number of small hydrophobic amino acids such as valine and alanine and generally acidic amino acids are lacking. In addition they generally have a middle region rich in Ser, Thr, Lys and Arg. Overall they have very often a net positive charge.

Alternatively, nucleic acid sequences coding for the transit peptides may be chemically synthesized either in part or wholly according to structure of transit peptide sequences disclosed in the prior art. Said natural or chemically synthesized sequences can be directly linked to the sequences encoding the mature protein or via a linker nucleic acid sequence, which may be typically less than 500 base pairs, preferably less than 450, 400, 350, 300, 250 or 200 base pairs, more preferably less than 150, 100, 90, 80, 70, 60, 50, 40 or 30 base pairs and most preferably less than 25, 20, 15, 12, 9, 6 or 3 base pairs in length and are in frame to the coding sequence. Furthermore favorable nucleic acid sequences encoding transit peptides may comprise sequences derived from more than one biological and/or chemical source and may include a nucleic acid sequence derived from the amino-terminal region of the mature protein, which in its native state is linked to the transit peptide. In a preferred empodiment of the invention said amino-terminal region of the mature protein is typically less than 150 amino acids, preferably less than 140, 130, 120, 110, 100 or 90 amino acids, more preferably less than 80, 70, 60, 50, 40, 35, 30, 25 or 20 amino acids and most preferably less than 19, 18, 17, 16, 15, 14, 13, 12, 11 or 10 amino acids in length. But even shorter or longer stretches are also possible. In addition target sequences, which facilitate the transport of proteins to other cell compartments such as the vacuole, endo-plasmic reticulum, golgi complex, glyoxysomes, peroxisomes or mitochondria may be also part of the inventive nucleic acid sequence. The proteins translated from said inventive nucleic acid sequences are a kind of fusion proteins that means the nucleic acid sequences encoding the transit peptide for example the ones shown in table V , preferably the last one of the table are joint to the nucleic acid sequences shown in table I, columns 5 and 7. The person skilled in the art is able to join said sequences in a functional manner. Advantageously the transit peptide part is cleaved off from the protein part shown in table II, columns 5 and 7 during the transport preferably into the plastids. All products of the cleavage of the preferred transit peptide shown in the last line of table V have preferably the N-terminal amino acid sequences QIA CSS or QIA EFQLTT in front of the start methionine of the protein metioned in table II, columns 5 and 7. Other short amino acid sequences of an range of 1 to 20 amino acids preferable 2 to 15 amino acids, more preferable 3 to 10 amino acids most preferably 4 to 8 amino acids are also possible in front of the start methionine of the protein metioned in table II, columns 5 and 7. In case of the amino acid sequence QIA CSS the three amino acids in front of the start methionine are stemming from the LIC (= ligatation independent cloning) cassette. Said short amino acid sequence is preferred in the case of the expression of E. coli genes. In case of the amino acid sequence QIA EFQLTT the six amino acids in front of the start methionine are stemming from the LIC cassette. Said short amino acid sequence is preferred in the case of the expression of S. cerevisiae genes. The skilled worker knows that other short sequences are also useful in the expression of the genes metioned in table I, columns 5 and 7. Furthermore the skilled worker is aware of the fact that there is not a need for such short sequences in the expression of the genes.

**Table V: Examples of transit peptides disclosed by von Heijne et al.**

| Trans Pep | Organism | Transit Peptide | SEQ ID NO: | Reference |
|---|---|---|---|---|
| 1 | Acetabularia mediterranea | MASIMMNKSVVLSKECAKPLATPK VTLNKRGFATTIATKNREMMVWQP FNNKMFETFSFLPP | **17** | Mol. Gen. Genet. 218:445-452(1989) |
| 2 | Arabidopsis thaliana | MAASLQSTATFLQSAKIATAPSRG SSHLRSTQAVGKSFGLETSSARLT CSFQSDFKDFTGKCSDAVKIAGFA LATSALVVSGASAEGAPK | **18** | EMBO J. 8:3187-3194(1989 ) |
| 3 | Arabidopsis thaliana | MAQVSRICNGVQNPSLICNLSKSS QRKSPLSVSLKTQQHPRAYPISSS WGLKKSGMTLIGSELRPLKVMSSV STAEKASEIVLQPIREISGLIKLP | **19** | Mol. Gen. Genet. 210: 437 - 442 (1987) |
| 4 | Arabidopsis thaliana | MAAATTTTTTTSSSISFSTKPSPSS SKSPLPISRFSLPFSLNPNKSSSS SRRRGIKSSSP SS ISAVLNTTTNV TTTPSPTKPTKPETF ISRFAPDQP RKGA | **20** | Plant Physiol. 85:1110-1117 (1987) |
| 5 | Arabidopsis thaliana | MITSSLTCSLQALKLSSPFAHGST PLSSLSKPNSFPNHRMPALVPV | **21** | J. Biol. Chem. 2652763-2767 (1990) |
| 6 | Arabidopsis thaliana | MASLLGTSSSAIWASPSLSSPSSK PSSSPICFRPGKLFGSKLNAGIQI | **22** | EMBO J. 9:1337-1346 (1990) |
| | | RPKKNRSRYHVSVMNVATEINSTE QVVGKFDSKKSARPVYPFAAI | | |
| 7 | Arabidopsis thaliana | MASTALSSAIVGTSFIRRSPAPISL RSLPSANTQSLFGLKSGTARGG RVVAM | **23** | Plant Physiol. 93: 572-577 (1990) |
| 8 | Arabidopsis thaliana | MAASTMALSSPAFAGKAVNLSPAA SEVLGSGRVTNRKTV | **24** | Nucl. Acids Res. 14: 4051 - 4064 (1986) |
| 9 | Arabidopsis thaliana | MAAITSATVTIPSFTGLKLAVSSK PKTLSTISRSSSATRAPPKLALKS SLKDFGVIAVATAASIVLAGNAMA MEVLLGSDDGSLAFVPSEFT | **25** | Gene 65: 59-69 (1988) |
| 10 | Arabidopsis thaliana | MAAAVSTVGAINRAPLSLNGSGSG AVSAPASTFLGKKVVTVSRFAQSN KKSNGSFKVLAVKEDKQTDGDRWR GLAYDTSDDQIDI | **26** | Nucl. Acids Res. 17:2871 (1989) |
| 11 | Arabidopsis thaliana | MkSSMLSSTAWTSPAQATMVAPF TGLKSSASFPVTRKANNDITSITS NGGRVSC | **27** | Plant Mol. Biol. 11: 745 - 759 (1988) |
| 12 | Arabidopsis thaliana | MAASGTSATFRASVSSAPSSSSQL THLKSPFKAVKY TPLPS SRSKSSS FSVSCTIAKDPPVLMAAGSDPALW QRPDSFGRFGKFGGKYVPE | **28** | Proc. Natl. Acad. Sci. USA, 86: 4604-4608 (1989) |
| 13 | Brassica campestris | MSTTFCSSVCMQATSLAATTRISF QKPALVSTTNLSFNLRRSIPTRFS ISCAAKPETVEKVSKIVKKQLSLK DDQKVVAE | **29** | Nucl. Acids Res. 15:7197 (1987) |
| 14 | Brassica napus | MATTFSASVSMQATSLATTTRISF QKPVLVSNHGRTNLSFNLSRTRL- SISC | **30** | Eur. J. Biochem. 174: 287 - 295 (1988) |
| 15 | Chlamydomo-nas reinhardtii | MQALSSRVNIAAKPQRAQRLVVRA | **31** | Plant Mol. Biol. 12: 463 - 474 (1989) |
| | | EEVKAAPKKEVGPKRGSLVK | | |
| 16 | Cucurbita moschata | MAELIQDKESAQSAATAAAASSGY ERRNEPAHSRK- FLEVRSEEELLSCIKK | **32** | FEBS Lett. 238: 424 - 430 (1988) |
| 17 | Spinacea oleracea | MSTINGCLTSISPSRTQLKNTSTL RPTFIANSRVNPSSSVPPSLIRNQ PVFAAPAPIITPTL | **33** | J. Biol. Chem. 265: 105414-5417 (1990) |
| 18 | Spinacea oleracea | MTTAVTAAVSFPSTKTTSLSARCS SVISPDKISYKKVPLYYRNVSATG KMGPIRAQIASDVEAPPPAPAK- VEKMS | **34** | Curr. Genet. 13: 517-522 (1988) |
| 19 | Spinacea oleracea | MTTAVTAAVSFPSTKTTSLSARSS SVISPDKISYKKVPLYYRNVSATG KMGPIRA | **35** | |

Alternatively to the targeting of the sequences shown in table II, columns 5 and 7 preferably of sequences in general encoded in the nucleus with the aid of the targeting sequences mentioned for example in table V alone or in combination with other targeting sequences preferably into the plastids, the nucleic acids of the invention can directly be introduced into the plastidal genome. Therefore in a preferred embodiment the nucleic acid sequences shown in table I, columns 5 and 7 are directly introduced and expressed in plastids.

The term "introduced" in the context of this specification shall mean the insertion of a nucleic acid sequence into the organism by means of a "transfection", "transduction" or preferably by "transformation".
A plastid, such as a chloroplast, has been "transformed" by an exogenous (preferably foreign) nucleic acid sequence if nucleic acid sequence has been introduced into the plastid that means that this sequence has crossed the membrane or the membranes of the plastid. The foreign DNA may be integrated (covalently linked) into plastid DNA making up the genome of the plastid, or it may remain unintegrated (e.g., by including a chloroplast origin of replication). "Stably" integrated DNA sequences are those, which are inherited through plastid replication, thereby transferring new plastids, with the features of the integrated DNA sequence to the progeny.

For expression a person skilled in the art is familiar with different methods to introduce the nucleic acid sequences into different organelles such as the preferred plastids. Such methods are for example disclosed by Pal Maiga (Annu. Rev. Plant Biol., 2004, 55: 289 - 313), Thomas Evans (WO 2004/040973), Kevin E. McBride et al. (US 5,455,818), Henry Daniell et al. (US 5,932,479 and US 5,693,507) and Jeffrey M. Straub et al. (US 6,781,033). A preferred method is the transformation of microspore-derived hypocotyl or cotyledonary tissue (which are green and thus contain numerous plastids) leaf tissue and afterwards the regeneration of shoots from said transformed plant material on selective medium. As methods for the transformation bombarding of the plant material or the use of independently replicating shuttle vectors are well known by the skilled worker. But also a PEG-mediated transformation of the plastids or Agrobacterium transformation with binary vectors is possible. Useful markers for the transformation of plastids are positive selection markers for example the chloramphenicol-, streptomycin-, kanamycin-, neomycin-, amikamycin-, spectinomycin-, triazine- and/or lincomycin-resistance genes. As additional markers named in the literature often as secondary markers, genes coding for the resistance against herbicides such as phosphinothricin (= glufosinate, BASTA^{™}, Liberty^{™}, encoded by the bar gene), glyphosate (= N-(phosphonomethyl)glycine, Roundup Ready^{™}, encoded by the 5-enolpyruvylshikimate-3-phosphate synthase gene = epsps), sulfonylurea (= Staple^{™}, encoded by the acetolactate synthase gene), imidazolinone [= IMI, imazethapyr, imazamox, Clearfield^{™}, encoded by the acetohydroxyacid synthase (AHAS) gene, also known as acetolactate synthase (ALS) gene] or bromoxynil (= Buctril^{™}, encoded by the oxy gene) or genes coding for antibiotics such as hygromycin or G418 are useful for further selection. Such secondary markers are useful in the case when most genome copies are transformed. In addition negative selection markers such as the bacterial cytosine deaminase (encoded by the codA gene) are also useful for the transformation of plastids.

To increase the possibility of identification of transformants it is also diserable to use reporter genes other then the aforementioned resistance genes or in addition to said genes. Reporter genes are for example β-galactosidase-, β-glucuronidase- (GUS), alkaline phosphatase- and/or green-fluorescent protein-genes (GFP).

For the inventive process it is of great advantage that by transforming the plastids the intraspecies specific transgene flow is blocked, because a lot of species such as corn, cotton and rice have a strict maternal inheritance of plastids. By placing the genes specified in table I, columns 5 and 7 or active fragments thereof in the plastids of plants, these genes will not be present in the pollen of said plants.
A further preferred embodiment of the invention relates to the use of so called "chloroplast localization sequences", in which a first RNA sequence or molecule is capable of transporting or "chaperoning" a second RNA sequence, such as a RNA sequence transcribed from the sequences depicted in table I, columns 5 and 7 or a sequence encoding a protein, as depicted in table II, columns 5 and 7, from an external environment inside a cell or outside a plastid into a chloroplast. In one embodiment the chloroplast localization signal is substantially similar or complementary to a complete or intact viroid sequence. The chloroplast localization signal may be encoded by a DNA sequence, which is transcribed into the chloroplast localization RNA. The term "viroid" refers to a naturally occurring single stranded RNA molecule (Flores, C R Acad Sci III. 2001 Oct; 324(10):943-52). Viroids usually contain about 200-500 nucleotides and generally exist as circular molecules. Examples of viroids that contain chloroplast localization signals include but are not limeted to ASBVd, PLMVd, CChMVd and ELVd. The viroid sequence or a functional part of it can be fused to the sequences depicted in table I, columns 5 and 7 or a sequence encoding a protein, as depicted in table II, columns 5 and 7 in such a manner that the viroid sequence transports a sequence transcribed from a sequence as depicted in table I, columns 5 and 7 or a sequence encoding a protein as depicted in table II, columns 5 and 7 into the chloroplasts. A preferred embodiment uses a modified ASBVd (Navarro et al., Virology. 2000 Mar 1;268(1):218-25).

In a further specific embodiment the protein to be expressed in the plastids such as the proteins depicted in table II, columns 5 and 7 are encoded by different nucleic acids. Such a method is disclosed in WO 2004/040973, which shall be incorporated by reference. WO 2004/040973 teaches a method, which relates to the translocation of an RNA corresponding to a gene or gene fragment into the chloroplast by means of a chloroplast localization sequence. The genes, which should be expressed in the plant or plants cells, are split into nucleic acid fragments, which are introduced into different compartments in the plant e.g. the nucleus, the plastids and/or mitochondria. Additionally plant cells are described in which the chloroplast contains a ribozyme fused at one end to an RNA encoding a fragment of a protein used in the inventive process such that the ribozyme can trans-splice the translocated fusion RNA to the RNA encoding the gene fragment to form and as the case may be reunite the nucleic acid fragments to an intact mRNA encoding a functional protein for example as disclosed in table II, columns 5 and 7.

In a preferred embodiment of the invention the nucleic acid sequences as shown in table I, columns 5 and 7 used in the inventive process are transformed into plastids, which are metabolical active. Those plastids should preferably maintain at a high copy number in the plant or plant tissue of interest, most preferably the chloroplasts found in green plant tissues, such as leaves or cotyledons or in seeds.

For a good expression in the plastids the nucleic acid sequences as shown in table I, columns 5 and 7 are introduced into an expression cassette using a preferably a promoter and terminator, which are active in plastids preferably a chloroplast promoter. Examples of such promoters include the psbA promoter from the gene from spinach or pea, the rbcL promoter, and the atpB promoter from corn.

For the purposes of the description of the present invention, the terms "cytoplasmic" and "non-targeted" are exchangable and shall indicate, that the nucleic acid of the invention is expressed without the addition of an non-natural transit peptide encoding sequence. A non-natural transit peptide encoding sequence is a sequence which is not a natural part of a nucleic acid of the invention, e.g. of the nucleic acids depicted in table I column 5 or 7, but is rather added by molecular manipulation steps as for example described in the example under "plastid targeted expression". Therfore the terms "cytoplasmic" and "non-targeted" shall not exclude a targeted localisation to any cell compartment for the products of the inventive nucleic acid sequences by their naturally occuring sequence properties within the background of the transgenic organism. The subcellular location of the mature polypetide derived from the enclosed sequences can be predicted by a skilled person for the organism (plant) by using software tools like TargetP (Emanuelsson et al., (2000), Predicting subcellular localization of proteins based on their N-terminal amino acid sequence., J.Mol. Biol. 300, 1005-1016.), ChloroP (Emanuelsson et al. (1999), ChloroP, a neural network-based method for predicting chloroplast transit peptides and their cleavage sites., Protein Science, 8: 978-984.) or other predictive software tools (Emanuelsson et al. (2007), Locating proteins in the cell using TargetP, SignalP, and related tools., Nature Protocols 2, 953-971).

Comprises/comprising and grammatical variations thereof when used in this specification are to be taken to specify the presence of stated features, integers, steps or components or groups thereof, but not to preclude the presence or addition of one or more other features, integers, steps, components or groups thereof.

In accordance with the invention, the term "plant cell" or the term "organism" as understood herein relates always to a plant cell or a organelle thereof, preferably a plastid, more preferably chloroplast.
As used herein, "plant" is meant to include not only a whole plant but also a part thereof i.e., one or more cells, and tissues, including for example, leaves, stems, shoots, roots, flowers, fruits and seeds.

Surprisingly it was found, that the transgenic expression of the Saccaromyces cerevisiae protein as shown in table II, column 3 and/or the transgenic expression of the *E. coli* protein as shown in table II, column 3 in a plant and/or the transgenic expression of the *Synechocystis sp.* protein as shown in table II, column 3 in a plant such as Arabidopsis thaliana for example, conferred transgenic a plant cell, a plant or a part thereof with increased tolerance and/or resistance to environmental stress and increased biomass production as compared to a corresponding non-transformed wild type plant cell, a plant or a part thereof.

Accordingly, in one embodiment, in case the activity of the Escherichia coli K12 nucleic acid molecule or a polypeptide comprising the nucleic acid SEQ ID NO.: 38 or polypeptide SEQ ID NO.: 39, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 38 or polypeptide SEQ ID NO.: 39, respectively is increased or generated or if the activity "b0081-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 54 or polypeptide SEQ ID NO.: 55, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 54 or polypeptide SEQ ID NO.: 55, respectively is increased or generated or if the activity "transporter subunit / periplasmic-binding component of ABC superfamily" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 70 or polypeptide SEQ ID NO.: 71, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 70 or polypeptide SEQ ID NO.: 71, respectively is increased or generated or if the activity "b0482-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 89 or polypeptide SEQ ID NO.: 90, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 89 or polypeptide SEQ ID NO.: 90, respectively is increased or generated or if the activity "universal stress protein UP12" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 143 or polypeptide SEQ ID NO.: 144, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 143 or polypeptide SEQ ID NO.: 144, respectively is increased or generated or if the activity "transcriptional regulator protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 162 or polypeptide SEQ ID NO.: 163, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 162 or polypeptide SEQ ID NO.: 163, respectively is increased or generated or if the activity "b0631-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 213 or polypeptide SEQ ID NO.: 214, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 213 or polypeptide SEQ ID NO.: 214, respectively is increased or generated or if the activity "potassium-transporting ATPase (subunit B)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 358 or polypeptide SEQ ID NO.: 359, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 358 or polypeptide SEQ ID NO.: 359, respectively is increased or generated or if the activity "b0753-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 367 or polypeptide SEQ ID NO.: 368, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 367 or polypeptide SEQ ID NO.: 368, respectively is increased or generated or if the activity "threonine and homoserine efflux system" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 420 or polypeptide SEQ ID NO.: 421, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 420 or polypeptide SEQ ID NO.: 421, respectively is increased or generated or if the activity "predicted transporter protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 455 or polypeptide SEQ ID NO.: 456, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 455 or polypeptide SEQ ID NO.: 456, respectively is increased or generated or if the activity "b0866-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 535 or polypeptide SEQ ID NO.: 536, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 535 or polypeptide SEQ ID NO.: 536, respectively is increased or generated or if the activity "methylglyoxal synthase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 618 or polypeptide SEQ ID NO.: 619, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 618 or polypeptide SEQ ID NO.: 619, respectively is increased or generated or if the activity "HyaA/HyaB-processing protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 671 or polypeptide SEQ ID NO.: 672, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 671 or polypeptide SEQ ID NO.: 672, respectively is increased or generated or if the activity "predicted oxidoreductase (flavin:NADH component)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 764 or polypeptide SEQ ID NO.: 765, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 764 or polypeptide SEQ ID NO.: 765, respectively is increased or generated or if the activity "b1052-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 768 or polypeptide SEQ ID NO.: 769, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 768 or polypeptide SEQ ID NO.: 769, respectively is increased or generated or if the activity "3-oxoacyl-(acyl carrier protein) synthase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 907 or polypeptide SEQ ID NO.: 908, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 907 or polypeptide SEQ ID NO.: 908, respectively is increased or generated or if the activity "b1161-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 927 or polypeptide SEQ ID NO.: 928, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 927 or polypeptide SEQ ID NO.: 928, respectively is increased or generated or if the activity "sodium/proton antiporter" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 1009 or polypeptide SEQ ID NO.: 1010, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1009 or polypeptide SEQ ID NO.: 1010, respectively is increased or generated or if the activity "predicted antimicrobial peptide transporter subunit" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 1154 or polypeptide SEQ ID NO.: 1155, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1154 or polypeptide SEQ ID NO.: 1155, respectively is increased or generated or if the activity "predicted antimicrobial peptide transporter subunit" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 1308 or polypeptide SEQ ID NO.: 1309, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1308 or polypeptide SEQ ID NO.: 1309, respectively is increased or generated or if the activity "b1423-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 1368 or polypeptide SEQ ID NO.: 1369, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1368 or polypeptide SEQ ID NO.: 1369, respectively is increased or generated or if the activity "acid shock protein precursor" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 1374 or polypeptide SEQ ID NO.: 1375, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1374 or polypeptide SEQ ID NO.: 1375, respectively is increased or generated or if the activity "predicted arginine/ornithine transporter" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 1507 or polypeptide SEQ ID NO.: 1508, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1507 or polypeptide SEQ ID NO.: 1508, respectively is increased or generated or if the activity "3-deoxy-D-arabino-heptulosonate-7-phosphatesynthase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 1953 or polypeptide SEQ ID NO.: 1954, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1953 or polypeptide SEQ ID NO.: 1954, respectively is increased or generated or if the activity "N,N'-diacetylchitobiose-specific enzyme IIA component of PTS" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 2156 or polypeptide SEQ ID NO.: 2157, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2156 or polypeptide SEQ ID NO.: 2157, respectively is increased or generated or if the activity "neutral amino-acid efflux system" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 2195 or polypeptide SEQ ID NO.: 2196, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2195 or polypeptide SEQ ID NO.: 2196, respectively is increased or generated or if the activity "b1878-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 2219 or polypeptide SEQ ID NO.: 2220, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2219 or polypeptide SEQ ID NO.: 2220, respectively is increased or generated or if the activity "L-arabinose transporter subunit" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 2277 or polypeptide SEQ ID NO.: 2278, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2277 or polypeptide SEQ ID NO.: 2278, respectively is increased or generated or if the activity "phosphatidylglycerophosphate synthetase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 2470 or polypeptide SEQ ID NO.: 2471, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2470 or polypeptide SEQ ID NO.: 2471, respectively is increased or generated or if the activity "regulator of length of O-antigen component of lipopolysaccharide chains" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 2493 or polypeptide SEQ ID NO.: 2494, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2493 or polypeptide SEQ ID NO.: 2494, respectively is increased or generated or if the activity "glucose-1-phosphate thymidylyltransferase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 2627 or polypeptide SEQ ID NO.: 2628, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2627 or polypeptide SEQ ID NO.: 2628, respectively is increased or generated or if the activity "multidrug efflux system (subunit B)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 2858 or polypeptide SEQ ID NO.: 2859, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2858 or polypeptide SEQ ID NO.: 2859, respectively is increased or generated or if the activity "GTP cyclohydrolase I" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 2942 or polypeptide SEQ ID NO.: 2943, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2942 or polypeptide SEQ ID NO.: 2943, respectively is increased or generated or if the activity "heme lyase (CcmH subunit)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 2965 or polypeptide SEQ ID NO.: 2966, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2965 or polypeptide SEQ ID NO.: 2966, respectively is increased or generated or if the activity "b2226-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 2981 or polypeptide SEQ ID NO.: 2982, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2981 or polypeptide SEQ ID NO.: 2982, respectively is increased or generated or if the activity "histidine/lysine/arginine/ornithine transporter subunit protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 3130 or polypeptide SEQ ID NO.: 3131, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3130 or polypeptide SEQ ID NO.: 3131, respectively is increased or generated or if the activity "sensory histidine kinase in two-component regulatory system with NarP (NarL)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 3216 or polypeptide SEQ ID NO.: 3217, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3216 or polypeptide SEQ ID NO.: 3217, respectively is increased or generated or if the activity "b2475-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 3335 or polypeptide SEQ ID NO.: 3336, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3335 or polypeptide SEQ ID NO.: 3336, respectively is increased or generated or if the activity "NADH dehydrogenase (subunit N)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 3401 or polypeptide SEQ ID NO.: 3402, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3401 or polypeptide SEQ ID NO.: 3402, respectively is increased or generated or if the activity "2,3-dihydroxy-2,3-dihydrophenylpropionatedehydrogenase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 3590 or polypeptide SEQ ID NO.: 3591, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3590 or polypeptide SEQ ID NO.: 3591, respectively is increased or generated or if the activity "tRNA-specific adenosine deaminase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 3831 or polypeptide SEQ ID NO.: 3832, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3831 or polypeptide SEQ ID NO.: 3832, respectively is increased or generated or if the activity "predicted outer membrane lipoprotein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 3857 or polypeptide SEQ ID NO.: 3858, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3857 or polypeptide SEQ ID NO.: 3858, respectively is increased or generated or if the activity "CP4-57 prophage/ RNase LS" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 3861 or polypeptide SEQ ID NO.: 3862, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3861 or polypeptide SEQ ID NO.: 3862, respectively is increased or generated or if the activity "glycine betaine transporter subunit protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 4022 or polypeptide SEQ ID NO.: 4023, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4022 or polypeptide SEQ ID NO.: 4023, respectively is increased or generated or if the activity "cellobiose/arbutin/salicin-specific PTS enzyme (IIB component/IC component)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 4059 or polypeptide SEQ ID NO.: 4060, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4059 or polypeptide SEQ ID NO.: 4060, respectively is increased or generated or if the activity "predicted kinase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 4076 or polypeptide SEQ ID NO.: 4077, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4076 or polypeptide SEQ ID NO.: 4077, respectively is increased or generated or if the activity "tRNA pseudouridine synthase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 4157 or polypeptide SEQ ID NO.: 4158, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4157 or polypeptide SEQ ID NO.: 4158, respectively is increased or generated or if the activity "predicted ligase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 4260 or polypeptide SEQ ID NO.: 4261, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4260 or polypeptide SEQ ID NO.: 4261, respectively is increased or generated or if the activity "ornithine decarboxylase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 4350 or polypeptide SEQ ID NO.: 4351, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4350 or polypeptide SEQ ID NO.: 4351, respectively is increased or generated or if the activity "phosphate transporter" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 4350 or polypeptide SEQ ID NO.: 4351, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4350 or polypeptide SEQ ID NO.: 4351, respectively is increased or generated or if the activity "phosphate transporter" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 4459 or polypeptide SEQ ID NO.: 4460, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4459 or polypeptide SEQ ID NO.: 4460, respectively is increased or generated or if the activity "hexuronate transporter" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 4505 or polypeptide SEQ ID NO.: 4506, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4505 or polypeptide SEQ ID NO.: 4506, respectively is increased or generated or if the activity "peptidyl-prolyl cis-trans isomerase A (rotamase A)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 4640 or polypeptide SEQ ID NO.: 4641, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4640 or polypeptide SEQ ID NO.: 4641, respectively is increased or generated or if the activity "glycogen synthase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 4806 or polypeptide SEQ ID NO.: 4807, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4806 or polypeptide SEQ ID NO.: 4807, respectively is increased or generated or if the activity "D-xylose transporter subunit" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 5124 or polypeptide SEQ ID NO.: 5125, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5124 or polypeptide SEQ ID NO.: 5125, respectively is increased or generated or if the activity "L-threonine 3-dehydrogenase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 5124 or polypeptide SEQ ID NO.: 5125, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5124 or polypeptide SEQ ID NO.: 5125, respectively is increased or generated or if the activity "L-threonine 3-dehydrogenase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 5417 or polypeptide SEQ ID NO.: 5418, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5417 or polypeptide SEQ ID NO.: 5418, respectively is increased or generated or if the activity "predicted hydrolase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 5495 or polypeptide SEQ ID NO.: 5496, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5495 or polypeptide SEQ ID NO.: 5496, respectively is increased or generated or if the activity "predicted PTS enzymes (IIB component/IIC component)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 5585 or polypeptide SEQ ID NO.: 5586, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5585 or polypeptide SEQ ID NO.: 5586, respectively is increased or generated or if the activity "ribonuclease activity regulator protein RraA" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 5800 or polypeptide SEQ ID NO.: 5801, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5800 or polypeptide SEQ ID NO.: 5801, respectively is increased or generated or if the activity "transcriptional repressor protein MetJ" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 5850 or polypeptide SEQ ID NO.: 5851, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5850 or polypeptide SEQ ID NO.: 5851, respectively is increased or generated or if the activity "pantothenate kinase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 5992 or polypeptide SEQ ID NO.: 5993, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5992 or polypeptide SEQ ID NO.: 5993, respectively is increased or generated or if the activity "heat shock protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 5999 or polypeptide SEQ ID NO.: 6000, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5999 or polypeptide SEQ ID NO.: 6000, respectively is increased or generated or if the activity "predicted porin" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 6056 or polypeptide SEQ ID NO.: 6057, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 6056 or polypeptide SEQ ID NO.: 6057, respectively is increased or generated or if the activity "aspartate ammonia-lyase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 6500 or polypeptide SEQ ID NO.: 6501, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 6500 or polypeptide SEQ ID NO.: 6501, respectively is increased or generated or if the activity "nicotinamide-nucleotide adenylyltransferase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Synechocystis sp. PCC 6803 nucleic acid molecule or a polypeptide comprising the nucleic acid SEQ ID NO.: 6542 or polypeptide SEQ ID NO.: 6543, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 6542 or polypeptide SEQ ID NO.: 6543, respectively is increased or generated or if the activity "polyphosphate kinase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 6823 or polypeptide SEQ ID NO.: 6824, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 6823 or polypeptide SEQ ID NO.: 6824, respectively is increased or generated or if the activity "Ya1049c-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 6870 or polypeptide SEQ ID NO.: 6871, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 6870 or polypeptide SEQ ID NO.: 6871, respectively is increased or generated or if the activity "YCR059C-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 6910 or polypeptide SEQ ID NO.: 6911, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 6910 or polypeptide SEQ ID NO.: 6911, respectively is increased or generated or if the activity "3-deoxy-D-arabino-heptulosonate-7-phosphate (DAHP) synthase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 7261 or polypeptide SEQ ID NO.: 7262, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7261 or polypeptide SEQ ID NO.: 7262, respectively is increased or generated or if the activity "YEL005C-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 7265 or polypeptide SEQ ID NO.: 7266, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7265 or polypeptide SEQ ID NO.: 7266, respectively is increased or generated or if the activity "Lsm (Like Sm) protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 7301 or polypeptide SEQ ID NO.: 7302, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7301 or polypeptide SEQ ID NO.: 7302, respectively is increased or generated or if the activity "YER156C-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 7384 or polypeptide SEQ ID NO.: 7385, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7384 or polypeptide SEQ ID NO.: 7385, respectively is increased or generated or if the activity "Checkpoint protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 7407 or polypeptide SEQ ID NO.: 7408, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7407 or polypeptide SEQ ID NO.: 7408, respectively is increased or generated or if the activity "YGL045W-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 7429 or polypeptide SEQ ID NO.: 7430, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7429 or polypeptide SEQ ID NO.: 7430, respectively is increased or generated or if the activity "Protein component of the small (40S) ribosomal subunit" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 7558 or polypeptide SEQ ID NO.: 7559, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7558 or polypeptide SEQ ID NO.: 7559, respectively is increased or generated or if the activity "Dihydrouridine synthase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 7606 or polypeptide SEQ ID NO.: 7607, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7606 or polypeptide SEQ ID NO.: 7607, respectively is increased or generated or if the activity "YOR024w-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 7610 or polypeptide SEQ ID NO.: 7611, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7610 or polypeptide SEQ ID NO.: 7611, respectively is increased or generated or if the activity "Glutamine tRNA synthetase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 7685 or polypeptide SEQ ID NO.: 7686, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7685 or polypeptide SEQ ID NO.: 7686, respectively is increased or generated or if the activity "Splicing factor" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 1201 or polypeptide SEQ ID NO.: 1202, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1201 or polypeptide SEQ ID NO.: 1202, respectively is increased or generated or if the activity "gamma-Glu-putrescine synthase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 7741 or polypeptide SEQ ID NO.: 7742, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7741 or polypeptide SEQ ID NO.: 7742, respectively is increased or generated or if the activity "inner membrane protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 7850 or polypeptide SEQ ID NO.: 7851, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7850 or polypeptide SEQ ID NO.: 7851, respectively is increased or generated or if the activity "heat shock protein HtpX" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 7971 or polypeptide SEQ ID NO.: 7972, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7971 or polypeptide SEQ ID NO.: 7972, respectively is increased or generated or if the activity "DNA-binding transcriptional dual regulator protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 8021 or polypeptide SEQ ID NO.: 8022, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8021 or polypeptide SEQ ID NO.: 8022, respectively is increased or generated or if the activity "predicted serine transporter protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 8177 or polypeptide SEQ ID NO.: 8178, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8177 or polypeptide SEQ ID NO.: 8178, respectively is increased or generated or if the activity "glutathione-dependent oxidoreductase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 8272 or polypeptide SEQ ID NO.: 8273, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8272 or polypeptide SEQ ID NO.: 8273, respectively is increased or generated or if the activity "Yfr042w-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 8288 or polypeptide SEQ ID NO.: 8289, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8288 or polypeptide SEQ ID NO.: 8289, respectively is increased or generated or if the activity "Protein component of the small (40S) ribosomal subunit" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 8438 or polypeptide SEQ ID NO.: 8439, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8438 or polypeptide SEQ ID NO.: 8439, respectively is increased or generated or if the activity "transcriptional regulator protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 8630 or polypeptide SEQ ID NO.: 8631, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8630 or polypeptide SEQ ID NO.: 8631, respectively is increased or generated or if the activity "predicted oxidoreductase (flavin:NADH component)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 9268 or polypeptide SEQ ID NO.: 9269, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9268 or polypeptide SEQ ID NO.: 9269, respectively is increased or generated or if the activity "cellobiose/arbutin/salicin-specific PTS enzyme (IIB component/IC component)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 9444 or polypeptide SEQ ID NO.: 9445, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9444 or polypeptide SEQ ID NO.: 9445, respectively is increased or generated or if the activity "predicted PTS enzymes (IIB component/IIC component)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 9824 or polypeptide SEQ ID NO.: 9825, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9824 or polypeptide SEQ ID NO.: 9825, respectively is increased or generated or if the activity "nicotinamide-nucleotide adenylyltransferase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 9905 or polypeptide SEQ ID NO.: 9906, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9905 or polypeptide SEQ ID NO.: 9906, respectively is increased or generated or if the activity "YGL045W-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 9193 or polypeptide SEQ ID NO.: 9194, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9193 or polypeptide SEQ ID NO.: 9194, respectively is increased or generated or if the activity "DNA-binding transcriptional dual regulator protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 8497 or polypeptide SEQ ID NO.: 8498, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8497 or polypeptide SEQ ID NO.: 8498, respectively is increased or generated or if the activity "methylglyoxal synthase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 8742 or polypeptide SEQ ID NO.: 8743, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8742 or polypeptide SEQ ID NO.: 8743, respectively is increased or generated or if the activity "gamma-Glu-putrescine synthase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 8891 or polypeptide SEQ ID NO.: 8892, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8891 or polypeptide SEQ ID NO.: 8892, respectively is increased or generated or if the activity "acid shock protein precursor" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli k12 nucleic acid molecule or a polypeptide comprising the nucleic acid SEQ ID NO.: 9031 or polypeptide SEQ ID NO.: 9032, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9031 or polypeptide SEQ ID NO.: 9032, respectively is increased or generated or if the activity "regulator of length of O-antigen component of lipopolysaccharide chains " is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 9315 or polypeptide SEQ ID NO.: 9316, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9315 or polypeptide SEQ ID NO.: 9316, respectively is increased or generated or if the activity "ornithine decarboxylase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 9529 or polypeptide SEQ ID NO.: 9530, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9529 or polypeptide SEQ ID NO.: 9530, respectively is increased or generated or if the activity "aspartate ammonia-lyase " is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 8462 or polypeptide SEQ ID NO.: 8463, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8462 or polypeptide SEQ ID NO.: 8463, respectively is increased or generated or if the activity "predicted transporter protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 8973 or polypeptide SEQ ID NO.: 8974, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8973 or polypeptide SEQ ID NO.: 8974, respectively is increased or generated or if the activity "L-arabinose transporter subunit" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 9883 or polypeptide SEQ ID NO.: 9884, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9883 or polypeptide SEQ ID NO.: 9884, respectively is increased or generated or if the activity "Lsm (Like Sm) protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 8934 or polypeptide SEQ ID NO.: 8935, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8934 or polypeptide SEQ ID NO.: 8935, respectively is increased or generated or if the activity "neutral amino-acid efflux system" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 9093 or polypeptide SEQ ID NO.: 9094, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9093 or polypeptide SEQ ID NO.: 9094, respectively is increased or generated or if the activity "b2226-protein" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli K12 nucleic acid molecule or a polypeptide comprising the nucleic acid SEQ ID NO.: 9109 or polypeptide SEQ ID NO.: 9110, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9109 or polypeptide SEQ ID NO.: 9110, respectively is increased or generated or if the activity "sensory histidine kinase in two-component regulatory system with NarP (NarL)" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Saccharomyces cerevisiae nucleic acid molecule or a polypeptide comprising the nucleic acid SEQ ID NO.: 9931 or polypeptide SEQ ID NO.: 9932, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9931 or polypeptide SEQ ID NO.: 9932, respectively is increased or generated or if the activity "Glutamine tRNA synthetase" is increased or generated in an plant cell, plant or part thereof an increase in tolerance and/or resistance to environmental stress and an increase biomass production as compared to a corresponding non-transformed wild type plant cell, a plant or a part thereof is conferred. Accordingly, in one embodiment, in case the activity of the Escherichia coli nucleic acid molecule or a polypeptide comprising the nucleic acid SEQ ID NO.: 10096 or polypeptide SEQ ID NO.: 10097, respectively is increased or generated, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 10096 or polypeptide SEQ ID NO.: 10097, respectively is increased or generated or if the activity "gluconate transporter" is increased or generated in an plant cell, plant or part thereof an increase in tolerance and/or resistance to environmental stress and an increase biomass production as compared to a corresponding non-transformed wild type plant cell, a plant or a part thereof is conferred..

For the purposes of the invention, as a rule the plural is intended to encompass the singular and vice versa.

Unless otherwise specified, the terms "polynucleotides", "nucleic acid" and "nucleic acid molecule" are interchangeably in the present context. Unless otherwise specified, the terms "peptide", "polypeptide" and "protein" are interchangeably in the present context. The term "sequence" may relate to polynucleotides, nucleic acids, nucleic acid molecules, peptides, polypeptides and proteins, depending on the context in which the term "sequence" is used. The terms "gene(s)", "polynucleotide", "nucleic acid sequence", "nucleotide sequence", or "nucleic acid molecule(s)" as used herein refers to a polymeric form of nucleotides of any length, either ribonucleotides or deoxyribonucleotides. The terms refer only to the primary structure of the molecule.

Thus, the terms "gene(s)", "polynucleotide", "nucleic acid sequence", "nucleotide sequence", or "nucleic acid molecule(s)" as used herein include double- and single-stranded DNA and/or RNA. They also include known types of modifications, for example, methylation, "caps", substitutions of one or more of the naturally occurring nucleotides with an analog. Preferably, the DNA or RNA sequence comprises a coding sequence encoding the herein defined polypeptide.

A "coding sequence" is a nucleotide sequence, which is transcribed into an RNA, e.g. a regulatory RNA, such as a miRNA, a ta-siRNA, cosuppression molecule, an RNAi, a ribozyme, etc. or into a mRNA which is translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleotide sequences or genomic DNA, while introns may be present as well under certain circumstances.

As used in the present context a nucleic acid molecule may also encompass the untranslated sequence located at the 3' and at the 5' end of the coding gene region, for example at least 500, preferably 200, especially preferably 100, nucleotides of the sequence upstream of the 5' end of the coding region and at least 100, preferably 50, especially preferably 20, nucleotides of the sequence downstream of the 3' end of the coding gene region. In the event for example the antisense, RNAi, snRNA, dsRNA, siRNA, miRNA, ta-siRNA, cosuppression molecule, ribozyme etc. technology is used coding regions as well as the 5'- and/or 3'-regions can advantageously be used.

However, it is often advantageous only to choose the coding region for cloning and expression purposes.

"Polypeptide" refers to a polymer of amino acid (amino acid sequence) and does not refer to a specific length of the molecule. Thus, peptides and oligopeptides are included within the definition of polypeptide. This term does also refer to or include post-translational modifications of the polypeptide, for example, glycosylations, acetylations, phosphorylations and the like. Included within the definition are, for example, polypeptides containing one or more analogs of an amino acid (including, for example, unnatural amino acids, etc.), polypeptides with substituted linkages, as well as other modifications known in the art, both naturally occurring and non-naturally occurring.

The term "Table I" used in this specification is to be taken to specify the content of Table I A and Table I B. The term "Table II" used in this specification is to be taken to specify the content of Table II A and Table II B. The term "Table I A" used in this specification is to be taken to specify the content of Table I A. The term "Table I B" used in this specification is to be taken to specify the content of Table I B. The term "Table II A" used in this specification is to be taken to specify the content of Table II A. The term "Table II B" used in this specification is to be taken to specify the content of Table II B. In one preferred embodiment, the term "Table I" means Table I B. In one preferred embodiment, the term "Table II" means Table II B.

The terms "comprise" or "comprising" and grammatical variations thereof when used in this specification are to be taken to specify the presence of stated features, integers, steps or components or groups thereof, but not to preclude the presence or addition of one or more other features, integers, steps, components or groups thereof.

In accordance with the invention, a protein or polypeptide has the "activity of an protein as shown in table II, column 3" if its de *novo* activity, or its increased expression directly or indirectly leads to and confers an increased tolerance and/or resistance to environmental stress and increased biomass production as compared to a corresponding non-transformed wild type plant cell, plant or part thereof and the protein has the above mentioned activities of a protein as shown in table II, column 3. Throughout the specification the activity or preferably the biological activity of such a protein or polypeptide or an nucleic acid molecule or sequence encoding such protein or polypeptide is identical or similar if it still has the biological or enzymatic activity of a protein as shown in table II, column 3, or which has at least 10% of the original enzymatic activity, preferably 20%, particularly preferably 30%, most particularly preferably 40% in comparison to a protein as shown in table II, column 3 of E. coli, Saccharomyces cerevisiae or *Synechocystis sp.*

The terms "increased", "rised", "extended", "enhanced", "improved" or "amplified" relate to a corresponding change of a property in a plant, an organism, a part of an organism such as a tissue, seed, root, leave, flower etc. or in a cell and are interchangeable. Preferably, the overall activity in the volume is increased or enhanced in cases if the increase or enhancement is related to the increase or enhancement of an activity of a gene product, independent whether the amount of gene product or the specific activity of the gene product or both is increased or enhanced or whether the amount, stability or translation efficacy of the nucleic acid sequence or gene encoding for the gene product is increased or enhanced.

The terms "increase" relate to a corresponding change of a property an organism or in a part of a plant, an organism, such as a tissue, seed, root, leave, flower etc. or in a cell. Preferably, the overall activity in the volume is increased in cases the increase relates to the increase of an activity of a gene product, independent whether the amount of gene product or the specific activity of the gene product or both is increased or generated or whether the amount, stability or translation efficacy of the nucleic acid sequence or gene encoding for the gene product is increased.

Under "change of a property" it is understood that the activity, expression level or amount of a gene product or the metabolite content is changed in a specific volume relative to a corresponding volume of a control, reference or wild type, including the de novo creation of the activity or expression.

The terms "increase" include the change of said property in only parts of the subject of the present invention, for example, the modification can be found in compartment of a cell, like a organelle, or in a part of a plant, like tissue, seed, root, leave, flower etc. but is not detectable if the overall subject, i.e. complete cell or plant, is tested.

Accordingly, the term "increase" means that the specific activity of an enzyme as well as the amount of a compound or metabolite, e.g. of a polypeptide, a nucleic acid molecule of the invention or an encoding mRNA or DNA, can be increased in a volume.

The terms "wild type", "control" or "reference" are exchangeable and can be a cell or a part of organisms such as an organelle like a chloroplast or a tissue, or an organism, in particular a plant, which was not modified or treated according to the herein described process according to the invention. Accordingly, the cell or a part of organisms such as an organelle like a chloroplast or a tissue, or an organism, in particular a plant used as wild typ, control or reference corresponds to the cell, organism, plant or part thereof as much as possible and is in any other property but in the result of the process of the invention as identical to the subject matter of the invention as possible. Thus, the wild type, control or reference is treated identically or as identical as possible, saying that only conditions or properties might be different which do not influence the quality of the tested property.

Preferably, any comparison is carried out under analogous conditions. The term "analogous conditions" means that all conditions such as, for example, culture or growing conditions, water content of the soil, temperature, humidity or surrounding air or soil, assay conditions (such as buffer composition, temperature, substrates, pathogen strain, concentrations and the like) are kept identical between the experiments to be compared.

The "reference", "control", or "wild type" is preferably a subject, e.g. an organelle, a cell, a tissue, an organism, in particular a plant, which was not modified or treated according to the herein described process of the invention and is in any other property as similar to the subject matter of the invention as possible. The reference, control or wild type is in its genome, transcriptome, proteome or metabolome as similar as possible to the subject of the present invention. Preferably, the term "reference-" "control-" or "wild type-"-organelle, -cell, -tissue or-organism, in particular plant, relates to an organelle, cell, tissue or organism, in particular plant, which is nearly genetically identical to the organelle, cell, tissue or organism, in particular plant, of the present invention or a part thereof preferably 95%, more preferred are 98%, even more preferred are 99,00%, in particular 99,10%, 99,30%, 99,50%, 99,70%, 99,90%, 99,99%, 99,999% or more. Most preferable the "reference", "control", or "wild type" is a subject, e.g. an organelle, a cell, a tissue, an organism, which is genetically identical to the organism, cell or organelle used according to the process of the invention except that the responsible or activity conferring nucleic acid molecules or the gene product encoded by them are amended, manipulated, exchanged or introduced according to the inventive process.

In case, a control, reference or wild type differing from the subject of the present invention only by not being subject of the process of the invention can not be provided, a control, reference or wild type can be an organism in which the cause for the modulation of an activity conferring the increased tolerance and/or resistance to environmental stress and increased biomass production as compared to a corresponding non-transformed wild type plant cell, plant or part thereof or expression of the nucleic acid molecule of the invention as described herein has been switched back or off, e.g. by knocking out the expression of responsible gene product, e.g. by antisense inhibition, by inactivation of an activator or agonist, by activation of an inhibitor or antagonist, by inhibition through adding inhibitory antibodies, by adding active compounds as e.g. hormones, by introducing negative dominant mutants, etc. A gene production can for example be knocked out by introducing inactivating point mutations, which lead to an enzymatic activity inhibition or a destabilization or an inhibition of the ability to bind to cofactors etc.

Accordingly, preferred reference subject is the starting subject of the present process of the invention. Preferably, the reference and the subject matter of the invention are compared after standardization and normalization, e.g. to the amount of total RNA, DNA, or Protein or activity or expression of reference genes, like housekeeping genes, such as ubiquitin, actin or ribosomal proteins.

The increase or modulation according to this invention can be constitutive, e.g. due to a stable permanent transgenic expression or to a stable mutation in the corresponding endogenous gene encoding the nucleic acid molecule of the invention or to a modulation of the expression or of the behavior of a gene conferring the expression of the polypeptide of the invention, or transient, e.g. due to an transient transformation or temporary addition of a modulator such as a agonist or antagonist or inducible, e.g. after transformation with a inducible construct carrying the nucleic acid molecule of the invention under control of a inducible promoter and adding the inducer, e.g. tetracycline or as described herein below.

The increase in activity of the polypeptide amounts in a cell, a tissue, a organelle, an organ or an organism or a part thereof preferably to at least 5%, preferably to at least 20% or at to least 50%, especially preferably to at least 70%, 80%, 90% or more, very especially preferably are to at least 200%, 300% or 400%, most preferably are to at least 500% or more in comparison to the control, reference or wild type.
In one embodiment the term increase means the increase in amount in relation to the weight of the organism or part thereof (w/w).

In one embodiments the increase in activity of the polypeptide amounts in an organelle such as a plastid.

The specific activity of a polypeptide encoded by a nucleic acid molecule of the present invention or of the polypeptide of the present invention can be tested as described in the examples. In particular, the expression of a protein in question in a cell, e.g. a plant cell in comparison to a control is an easy test and can be performed as described in the state of the art.

The term "increase" includes, that a compound or an activity is introduced into a cell or a subcellular compartment or organelle *de novo* or that the compound or the activity has not been detectable before, in other words it is "generated".

Accordingly, in the following, the term "increasing" also comprises the term "generating" or "stimulating". The increased activity manifests itself in an increase of the increased tolerance and/or resistance to environmental stress and increased biomass production as compared to a corresponding non-transformed wild type plant cell, plant or part thereof.

The sequence of B0081 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as b0081-protein.

Accordingly, in one embodiment, the process of the present invention comprises increasing or generating the activity of a gene product with the activity of a "b0081-protein" from Escherichia coli K12 or its functional equivalent or its homolog, e.g. the increase of
(a) a gene product of a gene comprising the nucleic acid molecule as shown in column 5 of Table I and being depicted in the same respective line as said B0081 or a functional equivalent or a homologue thereof as shown depicted in column 7 of Table I, preferably a homologue or functional equivalent as shown depicted in column 7 of Table I B, and being depicted in the same respective line as said B0081; or
(b) a polypeptide comprising a polypeptide, a consensus sequence or a polypeptide motif as shown depicted in column 5 of Table II, and being depicted in the same respective line as said B0081 or a functional equivalent or a homologue thereof as depicted in column 7 of Table II or IV, preferably a homologue or functional equivalent as depicted in column 7 of Table II B, and being depicted in the same respective line as said B0081,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "b0081-protein", preferably it is the molecule of section (a) or (b) of this paragraph.

In one embodiment, said molecule, which activity is to be increased in the process of the invention and which is the gene product with an activity of described as a "b0081-protein", is increased non-targeted.

The sequence of B0445 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as transporter subunit / periplasmic-binding component of ABC superfamily.

Accordingly, in one embodiment, the process of the present invention comprises increasing or generating the activity of a gene product with the activity of a "transporter subunit / periplasmic-binding component of ABC superfamily" from Escherichia coli K12 or its functional equivalent or its homolog, e.g. the increase of
(a) a gene product of a gene comprising the nucleic acid molecule as shown in column 5 of Table I and being depicted in the same respective line as said B0445 or a functional equivalent or a homologue thereof as shown depicted in column 7 of Table I, preferably a homologue or functional equivalent as shown depicted in column 7 of Table I B, and being depicted in the same respective line as said B0445; or
(b) a polypeptide comprising a polypeptide, a consensus sequence or a polypeptide motif as shown depicted in column 5 of Table II, and being depicted in the same respective line as said B0445 or a functional equivalent or a homologue thereof as depicted in column 7 of Table II or IV, preferably a homologue or functional equivalent as depicted in column 7 of Table II B, and being depicted in the same respective line as said B0445,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "transporter subunit / periplasmic-binding component of ABC superfamily", preferably it is the molecule of section (a) or (b) of this paragraph.

In one embodiment, said molecule, which activity is to be increased in the process of the invention and which is the gene product with an activity of described as a "transporter subunit / periplasmic-binding component of ABC superfamily", is increased non-targeted.

The sequence of B0482 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as b0482-protein.

Accordingly, in one embodiment, the process of the present invention comprises increasing or generating the activity of a gene product with the activity of a "b0482-protein" from Escherichia coli K12 or its functional equivalent or its homolog, e.g. the increase of
(a) a gene product of a gene comprising the nucleic acid molecule as shown in column 5 of Table I and being depicted in the same respective line as said B0482 or a functional equivalent or a homologue thereof as shown depicted in column 7 of Table I, preferably a homologue or functional equivalent as shown depicted in column 7 of Table I B, and being depicted in the same respective line as said B0482; or
(b) a polypeptide comprising a polypeptide, a consensus sequence or a polypeptide motif as shown depicted in column 5 of Table II, and being depicted in the same respective line as said B0482 or a functional equivalent or a homologue thereof as depicted in column 7 of Table II or IV, preferably a homologue or functional equivalent as depicted in column 7 of Table II B, and being depicted in the same respective line as said B0482,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "b0482-protein", preferably it is the molecule of section (a) or (b) of this paragraph.

In one embodiment, said molecule, which activity is to be increased in the process of the invention and which is the gene product with an activity of described as a "b0482-protein", is increased non-targeted.

The sequence of B0607 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as universal stress protein UP12.

Accordingly, in one embodiment, the process of the present invention comprises increasing or generating the activity of a gene product with the activity of a "universal stress protein UP12" from Escherichia coli K12 or its functional equivalent or its homolog, e.g. the increase of
(a) a gene product of a gene comprising the nucleic acid molecule as shown in column 5 of Table I and being depicted in the same respective line as said B0607 or a functional equivalent or a homologue thereof as shown depicted in column 7 of Table I, preferably a homologue or functional equivalent as shown depicted in column 7 of Table I B, and being depicted in the same respective line as said B0607; or
(b) a polypeptide comprising a polypeptide, a consensus sequence or a polypeptide motif as shown depicted in column 5 of Table II, and being depicted in the same respective line as said B0607 or a functional equivalent or a homologue thereof as depicted in column 7 of Table II or IV, preferably a homologue or functional equivalent as depicted in column 7 of Table II B, and being depicted in the same respective line as said B0607,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "universal stress protein UP12", preferably it is the molecule of section (a) or (b) of this paragraph.

In one embodiment, said molecule, which activity is to be increased in the process of the invention and which is the gene product with an activity of described as a "universal stress protein UP12", is increased non-targeted.

The sequence of B0629 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as transcriptional regulator protein.

Accordingly, in one embodiment, the process of the present invention comprises increasing or generating the activity of a gene product with the activity of a "transcriptional regulator protein" from Escherichia coli K12 or its functional equivalent or its homolog, e.g. the increase of
(a) a gene product of a gene comprising the nucleic acid molecule as shown in column 5 of Table I and being depicted in the same respective line as said B0629 or a functional equivalent or a homologue thereof as shown depicted in column 7 of Table I, preferably a homologue or functional equivalent as shown depicted in column 7 of Table I B, and being depicted in the same respective line as said B0629; or
(b) a polypeptide comprising a polypeptide, a consensus sequence or a polypeptide motif as shown depicted in column 5 of Table II, and being depicted in the same respective line as said B0629 or a functional equivalent or a homologue thereof as depicted in column 7 of Table II or IV, preferably a homologue or functional equivalent as depicted in column 7 of Table II B, and being depicted in the same respective line as said B0629,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "transcriptional regulator protein", preferably it is the molecule of section (a) or (b) of this paragraph.

In one embodiment, said molecule, which activity is to be increased in the process of the invention and which is the gene product with an activity of described as a "transcriptional regulator protein", is increased non-targeted.

The sequence of B0631 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as b0631-protein.

Accordingly, in one embodiment, the process of the present invention comprises increasing or generating the activity of a gene product with the activity of a "b0631-protein" from Escherichia coli K12 or its functional equivalent or its homolog, e.g. the increase of
(a) a gene product of a gene comprising the nucleic acid molecule as shown in column 5 of Table I and being depicted in the same respective line as said B0631 or a functional equivalent or a homologue thereof as shown depicted in column 7 of Table I, preferably a homologue or functional equivalent as shown depicted in column 7 of Table I B, and being depicted in the same respective line as said B0631; or
(b) a polypeptide comprising a polypeptide, a consensus sequence or a polypeptide motif as shown depicted in column 5 of Table II, and being depicted in the same respective line as said B0631 or a functional equivalent or a homologue thereof as depicted in column 7 of Table II or IV, preferably a homologue or functional equivalent as depicted in column 7 of Table II B, and being depicted in the same respective line as said B0631,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "b0631-protein", preferably it is the molecule of section (a) or (b) of this paragraph.

In one embodiment, said molecule, which activity is to be increased in the process of the invention and which is the gene product with an activity of described as a "b0631-protein", is increased non-targeted.

The sequence of B0697 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as potassium-transporting ATPase (subunit B).

Accordingly, in one embodiment, the process of the present invention comprises increasing or generating the activity of a gene product with the activity of a "potassium-transporting ATPase (subunit B)" from Escherichia coli K12 or its functional equivalent or its homolog, e.g. the increase of
(a) a gene product of a gene comprising the nucleic acid molecule as shown in column 5 of Table I and being depicted in the same respective line as said B0697 or a functional equivalent or a homologue thereof as shown depicted in column 7 of Table I, preferably a homologue or functional equivalent as shown depicted in column 7 of Table I B, and being depicted in the same respective line as said B0697; or
(b) a polypeptide comprising a polypeptide, a consensus sequence or a polypeptide motif as shown depicted in column 5 of Table II, and being depicted in the same respective line as said B0697 or a functional equivalent or a homologue thereof as depicted in column 7 of Table II or IV, preferably a homologue or functional equivalent as depicted in column 7 of Table II B, and being depicted in the same respective line as said B0697,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "potassium-transporting ATPase (subunit B)", preferably it is the molecule of section (a) or (b) of this paragraph.

In one embodiment, said molecule, which activity is to be increased in the process of the invention and which is the gene product with an activity of described as a "potassium-transporting ATPase (subunit B)", is increased non-targeted.

The sequence of B0753 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as b0753-protein.

Accordingly, in one embodiment, the process of the present invention comprises increasing or generating the activity of a gene product with the activity of a "b0753-protein" from Escherichia coli K12 or its functional equivalent or its homolog, e.g. the increase of
(a) a gene product of a gene comprising the nucleic acid molecule as shown in column 5 of Table I and being depicted in the same respective line as said B0753 or a functional equivalent or a homologue thereof as shown depicted in column 7 of Table I, preferably a homologue or functional equivalent as shown depicted in column 7 of Table I B, and being depicted in the same respective line as said B0753; or
(b) a polypeptide comprising a polypeptide, a consensus sequence or a polypeptide motif as shown depicted in column 5 of Table II, and being depicted in the same respective line as said B0753 or a functional equivalent or a homologue thereof as depicted in column 7 of Table II or IV, preferably a homologue or functional equivalent as depicted in column 7 of Table II B, and being depicted in the same respective line as said B0753,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "b0753-protein", preferably it is the molecule of section (a) or (b) of this paragraph.

In one embodiment, said molecule, which activity is to be increased in the process of the invention and which is the gene product with an activity of described as a "b0753-protein", is increased non-targeted.

The sequence of B0813 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as threonine and homoserine efflux system.

Accordingly, in one embodiment, the process of the present invention comprises increasing or generating the activity of a gene product with the activity of a "threonine and homoserine efflux system" from Escherichia coli K12 or its functional equivalent or its homolog, e.g. the increase of
(a) a gene product of a gene comprising the nucleic acid molecule as shown in column 5 of Table I and being depicted in the same respective line as said B0813 or a functional equivalent or a homologue thereof as shown depicted in column 7 of Table I, preferably a homologue or functional equivalent as shown depicted in column 7 of Table I B, and being depicted in the same respective line as said B0813; or
(b) a polypeptide comprising a polypeptide, a consensus sequence or a polypeptide motif as shown depicted in column 5 of Table II, and being depicted in the same respective line as said B0813 or a functional equivalent or a homologue thereof as depicted in column 7 of Table II or IV, preferably a homologue or functional equivalent as depicted in column 7 of Table II B, and being depicted in the same respective line as said B0813,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "threonine and homoserine efflux system", preferably it is the molecule of section (a) or (b) of this paragraph.

In one embodiment, said molecule, which activity is to be increased in the process of the invention and which is the gene product with an activity of described as a "threonine and homoserine efflux system", is increased non-targeted.

The sequence of B0845 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted transporter protein.

Accordingly, in one embodiment, the process of the present invention comprises increasing or generating the activity of a gene product with the activity of a "predicted transporter protein" from Escherichia coli K12 or its functional equivalent or its homolog, e.g. the increase of
(a) a gene product of a gene comprising the nucleic acid molecule as shown in column 5 of Table I and being depicted in the same respective line as said B0845 or a functional equivalent or a homologue thereof as shown depicted in column 7 of Table I, preferably a homologue or functional equivalent as shown depicted in column 7 of Table I B, and being depicted in the same respective line as said B0845; or
(b) a polypeptide comprising a polypeptide, a consensus sequence or a polypeptide motif as shown depicted in column 5 of Table II, and being depicted in the same respective line as said B0845 or a functional equivalent or a homologue thereof as depicted in column 7 of Table II or IV, preferably a homologue or functional equivalent as depicted in column 7 of Table II B, and being depicted in the same respective line as said B0845,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted transporter protein", preferably it is the molecule of section (a) or (b) of this paragraph.

In one embodiment, said molecule, which activity is to be increased in the process of the invention and which is the gene product with an activity of described as a "predicted transporter protein", is increased non-targeted.

The sequence of B0866 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as b0866-protein.

Accordingly, in one embodiment, the process of the present invention comprises increasing or generating the activity of a gene product with the activity of a "b0866-protein" from Escherichia coli K12 or its functional equivalent or its homolog, e.g. the increase of
(a) a gene product of a gene comprising the nucleic acid molecule as shown in column 5 of Table I and being depicted in the same respective line as said B0866 or a functional equivalent or a homologue thereof as shown depicted in column 7 of Table I, preferably a homologue or functional equivalent as shown depicted in column 7 of Table I B, and being depicted in the same respective line as said B0866; or
(b) a polypeptide comprising a polypeptide, a consensus sequence or a polypeptide motif as shown depicted in column 5 of Table II, and being depicted in the same respective line as said B0866 or a functional equivalent or a homologue thereof as depicted in column 7 of Table II or IV, preferably a homologue or functional equivalent as depicted in column 7 of Table II B, and being depicted in the same respective line as said B0866,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "b0866-protein", preferably it is the molecule of section (a) or (b) of this paragraph.

In one embodiment, said molecule, which activity is to be increased in the process of the invention and which is the gene product with an activity of described as a "b0866-protein", is increased non-targeted.

The sequence of B0963 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as methylglyoxal synthase.

Accordingly, in one embodiment, the process of the present invention comprises increasing or generating the activity of a gene product with the activity of a "methylglyoxal synthase" from Escherichia coli K12 or its functional equivalent or its homolog, e.g. the increase of
(a) a gene product of a gene comprising the nucleic acid molecule as shown in column 5 of Table I and being depicted in the same respective line as said B0963 or a functional equivalent or a homologue thereof as shown depicted in column 7 of Table I, preferably a homologue or functional equivalent as shown depicted in column 7 of Table I B, and being depicted in the same respective line as said B0963; or
(b) a polypeptide comprising a polypeptide, a consensus sequence or a polypeptide motif as shown depicted in column 5 of Table II, and being depicted in the same respective line as said B0963 or a functional equivalent or a homologue thereof as depicted in column 7 of Table II or IV, preferably a homologue or functional equivalent as depicted in column 7 of Table II B, and being depicted in the same respective line as said B0963,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "methylglyoxal synthase", preferably it is the molecule of section (a) or (b) of this paragraph.

In one embodiment, said molecule, which activity is to be increased in the process of the invention and which is the gene product with an activity of described as a "methylglyoxal synthase", is increased non-targeted.

The sequence of B0975 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as HyaA/HyaB-processing protein.

Accordingly, in one embodiment, the process of the present invention comprises increasing or generating the activity of a gene product with the activity of a "HyaA/HyaB-processing protein" from Escherichia coli K12 or its functional equivalent or its homolog, e.g. the increase of
(a) a gene product of a gene comprising the nucleic acid molecule as shown in column 5 of Table I and being depicted in the same respective line as said B0975 or a functional equivalent or a homologue thereof as shown depicted in column 7 of Table I, preferably a homologue or functional equivalent as shown depicted in column 7 of Table I B, and being depicted in the same respective line as said B0975; or
(b) a polypeptide comprising a polypeptide, a consensus sequence or a polypeptide motif as shown depicted in column 5 of Table II, and being depicted in the same respective line as said B0975 or a functional equivalent or a homologue thereof as depicted in column 7 of Table II or IV, preferably a homologue or functional equivalent as depicted in column 7 of Table II B, and being depicted in the same respective line as said B0975,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "HyaA/HyaB-processing protein", preferably it is the molecule of section (a) or (b) of this paragraph.

In one embodiment, said molecule, which activity is to be increased in the process of the invention and which is the gene product with an activity of described as a "HyaA/HyaB-processing protein", is increased non-targeted.

The sequence of B1007 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted oxidoreductase (flavin:NADH component).

Accordingly, in one embodiment, the process of the present invention comprises increasing or generating the activity of a gene product with the activity of a "predicted oxidoreductase (flavin:NADH component)" from Escherichia coli K12 or its functional equivalent or its homolog, e.g. the increase of
(a) a gene product of a gene comprising the nucleic acid molecule as shown in column 5 of Table I and being depicted in the same respective line as said B1007 or a functional equivalent or a homologue thereof as shown depicted in column 7 of Table I, preferably a homologue or functional equivalent as shown depicted in column 7 of Table I B, and being depicted in the same respective line as said B1007; or
(b) a polypeptide comprising a polypeptide, a consensus sequence or a polypeptide motif as shown depicted in column 5 of Table II, and being depicted in the same respective line as said B1007 or a functional equivalent or a homologue thereof as depicted in column 7 of Table II or IV, preferably a homologue or functional equivalent as depicted in column 7 of Table II B, and being depicted in the same respective line as said B1007,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted oxidoreductase (flavin:NADH component)", preferably it is the molecule of section (a) or (b) of this paragraph.

In one embodiment, said molecule, which activity is to be increased in the process of the invention and which is the gene product with an activity of described as a "predicted oxidoreductase (flavin:NADH component)", is increased non-targeted.

The sequence of B1052 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as b1052-protein.

Accordingly, in one embodiment, the process of the present invention comprises increasing or generating the activity of a gene product with the activity of a "b1052-protein" from Escherichia coli K12 or its functional equivalent or its homolog, e.g. the increase of
(a) a gene product of a gene comprising the nucleic acid molecule as shown in column 5 of Table I and being depicted in the same respective line as said B1052 or a functional equivalent or a homologue thereof as shown depicted in column 7 of Table I, preferably a homologue or functional equivalent as shown depicted in column 7 of Table I B, and being depicted in the same respective line as said B1052; or
(b) a polypeptide comprising a polypeptide, a consensus sequence or a polypeptide motif as shown depicted in column 5 of Table II, and being depicted in the same respective line as said B1052 or a functional equivalent or a homologue thereof as depicted in column 7 of Table II or IV, preferably a homologue or functional equivalent as depicted in column 7 of Table II B, and being depicted in the same respective line as said B1052,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "b1052-protein", preferably it is the molecule of section (a) or (b) of this paragraph.

In one embodiment, said molecule, which activity is to be increased in the process of the invention and which is the gene product with an activity of described as a "b1052-protein", is increased non-targeted.

The sequence of B1091 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as 3-oxoacyl-(acyl carrier protein) synthase.

Accordingly, in one embodiment, the process of the present invention comprises increasing or generating the activity of a gene product with the activity of a "3-oxoacyl-(acyl carrier protein) synthase" from Escherichia coli K12 or its functional equivalent or its homolog, e.g. the increase of
(a) a gene product of a gene comprising the nucleic acid molecule as shown in column 5 of Table I and being depicted in the same respective line as said B1091 or a functional equivalent or a homologue thereof as shown depicted in column 7 of Table I, preferably a homologue or functional equivalent as shown depicted in column 7 of Table I B, and being depicted in the same respective line as said B1091; or
(b) a polypeptide comprising a polypeptide, a consensus sequence or a polypeptide motif as shown depicted in column 5 of Table II, and being depicted in the same respective line as said B1091 or a functional equivalent or a homologue thereof as depicted in column 7 of Table II or IV, preferably a homologue or functional equivalent as depicted in column 7 of Table II B, and being depicted in the same respective line as said B1091,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "3-oxoacyl-(acyl carrier protein) synthase", preferably it is the molecule of section (a) or (b) of this paragraph.

In one embodiment, said molecule, which activity is to be increased in the process of the invention and which is the gene product with an activity of described as a "3-oxoacyl-(acyl carrier protein) synthase", is increased plastidic.

The sequence of B1161 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as b1161-protein.

Accordingly, in one embodiment, the process of the present invention comprises increasing or generating the activity of a gene product with the activity of a "b1161-protein" from Escherichia coli K12 or its functional equivalent or its homolog, e.g. the increase of
(a) a gene product of a gene comprising the nucleic acid molecule as shown in column 5 of Table I and being depicted in the same respective line as said B1161 or a functional equivalent or a homologue thereof as shown depicted in column 7 of Table I, preferably a homologue or functional equivalent as shown depicted in column 7 of Table I B, and being depicted in the same respective line as said B1161; or
(b) a polypeptide comprising a polypeptide, a consensus sequence or a polypeptide motif as shown depicted in column 5 of Table II, and being depicted in the same respective line as said B1161 or a functional equivalent or a homologue thereof as depicted in column 7 of Table II or IV, preferably a homologue or functional equivalent as depicted in column 7 of Table II B, and being depicted in the same respective line as said B1161,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "b1161-protein", preferably it is the molecule of section (a) or (b) of this paragraph.

In one embodiment, said molecule, which activity is to be increased in the process of the invention and which is the gene product with an activity of described as a "b1161-protein", is increased non-targeted.

The sequence of B1186 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as sodium/proton antiporter.

Accordingly, in one embodiment, the process of the present invention comprises increasing or generating the activity of a gene product with the activity of a "sodium/proton antiporter" from Escherichia coli K12 or its functional equivalent or its homolog, e.g. the increase of
(a) a gene product of a gene comprising the nucleic acid molecule as shown in column 5 of Table I and being depicted in the same respective line as said B1186 or a functional equivalent or a homologue thereof as shown depicted in column 7 of Table I, preferably a homologue or functional equivalent as shown depicted in column 7 of Table I B, and being depicted in the same respective line as said B1186; or
(b) a polypeptide comprising a polypeptide, a consensus sequence or a polypeptide motif as shown depicted in column 5 of Table II, and being depicted in the same respective line as said B1186 or a functional equivalent or a homologue thereof as depicted in column 7 of Table II or IV, preferably a homologue or functional equivalent as depicted in column 7 of Table II B, and being depicted in the same respective line as said B1186,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "sodium/proton antiporter", preferably it is the molecule of section (a) or (b) of this paragraph.

In one embodiment, said molecule, which activity is to be increased in the process of the invention and which is the gene product with an activity of described as a "sodium/proton antiporter", is increased non-targeted.

The sequence of B1291 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted antimicrobial peptide transporter subunit.

Accordingly, in one embodiment, the process of the present invention comprises increasing or generating the activity of a gene product with the activity of a "predicted antimicrobial peptide transporter subunit" from Escherichia coli K12 or its functional equivalent or its homolog, e.g. the increase of
(a) a gene product of a gene comprising the nucleic acid molecule as shown in column 5 of Table I and being depicted in the same respective line as said B1291 or a functional equivalent or a homologue thereof as shown depicted in column 7 of Table I, preferably a homologue or functional equivalent as shown depicted in column 7 of Table I B, and being depicted in the same respective line as said B1291; or
(b) a polypeptide comprising a polypeptide, a consensus sequence or a polypeptide motif as shown depicted in column 5 of Table II, and being depicted in the same respective line as said B1291 or a functional equivalent or a homologue thereof as depicted in column 7 of Table II or IV, preferably a homologue or functional equivalent as depicted in column 7 of Table II B, and being depicted in the same respective line as said B1291,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted antimicrobial peptide transporter subunit", preferably it is the molecule of section (a) or (b) of this paragraph.

In one embodiment, said molecule, which activity is to be increased in the process of the invention and which is the gene product with an activity of described as a "predicted antimicrobial peptide transporter subunit", is increased plastidic.

The sequence of B1294 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted antimicrobial peptide transporter subunit.

Accordingly, in one embodiment, the process of the present invention comprises increasing or generating the activity of a gene product with the activity of a "predicted antimicrobial peptide transporter subunit" from Escherichia coli K12 or its functional equivalent or its homolog, e.g. the increase of
(a) a gene product of a gene comprising the nucleic acid molecule as shown in column 5 of Table I and being depicted in the same respective line as said B1294 or a functional equivalent or a homologue thereof as shown depicted in column 7 of Table I, preferably a homologue or functional equivalent as shown depicted in column 7 of Table I B, and being depicted in the same respective line as said B1294; or
(b) a polypeptide comprising a polypeptide, a consensus sequence or a polypeptide motif as shown depicted in column 5 of Table II, and being depicted in the same respective line as said B1294 or a functional equivalent or a homologue thereof as depicted in column 7 of Table II or IV, preferably a homologue or functional equivalent as depicted in column 7 of Table II B, and being depicted in the same respective line as said B1294,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted antimicrobial peptide transporter subunit", preferably it is the molecule of section (a) or (b) of this paragraph.

In one embodiment, said molecule, which activity is to be increased in the process of the invention and which is the gene product with an activity of described as a "predicted antimicrobial peptide transporter subunit", is increased plastidic.

The sequence of B1423 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as b1423-protein.

Accordingly, in one embodiment, the process of the present invention comprises increasing or generating the activity of a gene product with the activity of a "b1423-protein" from Escherichia coli K12 or its functional equivalent or its homolog, e.g. the increase of
(a) a gene product of a gene comprising the nucleic acid molecule as shown in column 5 of Table I and being depicted in the same respective line as said B1423 or a functional equivalent or a homologue thereof as shown depicted in column 7 of Table I, preferably a homologue or functional equivalent as shown depicted in column 7 of Table I B, and being depicted in the same respective line as said B1423; or
(b) a polypeptide comprising a polypeptide, a consensus sequence or a polypeptide motif as shown depicted in column 5 of Table II, and being depicted in the same respective line as said B1423 or a functional equivalent or a homologue thereof as depicted in column 7 of Table II or IV, preferably a homologue or functional equivalent as depicted in column 7 of Table II B, and being depicted in the same respective line as said B1423,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "b1423-protein", preferably it is the molecule of section (a) or (b) of this paragraph.

In one embodiment, said molecule, which activity is to be increased in the process of the invention and which is the gene product with an activity of described as a "b1423-protein", is increased non-targeted.

The sequence of B1597 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as acid shock protein precursor.

Accordingly, in one embodiment, the process of the present invention comprises increasing or generating the activity of a gene product with the activity of a "acid shock protein precursor" from Escherichia coli K12 or its functional equivalent or its homolog, e.g. the increase of
(a) a gene product of a gene comprising the nucleic acid molecule as shown in column 5 of Table I and being depicted in the same respective line as said B1597 or a functional equivalent or a homologue thereof as shown depicted in column 7 of Table I, preferably a homologue or functional equivalent as shown depicted in column 7 of Table I B, and being depicted in the same respective line as said B1597; or
(b) a polypeptide comprising a polypeptide, a consensus sequence or a polypeptide motif as shown depicted in column 5 of Table II, and being depicted in the same respective line as said B1597 or a functional equivalent or a homologue thereof as depicted in column 7 of Table II or IV, preferably a homologue or functional equivalent as depicted in column 7 of Table II B, and being depicted in the same respective line as said B1597,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "acid shock protein precursor", preferably it is the molecule of section (a) or (b) of this paragraph.

In one embodiment, said molecule, which activity is to be increased in the process of the invention and which is the gene product with an activity of described as a "acid shock protein precursor", is increased non-targeted.

The sequence of B1605 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted arginine/ornithine transporter.

Accordingly, in one embodiment, the process of the present invention comprises increasing or generating the activity of a gene product with the activity of a "predicted arginine/ornithine transporter" from Escherichia coli K12 or its functional equivalent or its homolog, e.g. the increase of
(a) a gene product of a gene comprising the nucleic acid molecule as shown in column 5 of Table I and being depicted in the same respective line as said B1605 or a functional equivalent or a homologue thereof as shown depicted in column 7 of Table I, preferably a homologue or functional equivalent as shown depicted in column 7 of Table I B, and being depicted in the same respective line as said B1605; or
(b) a polypeptide comprising a polypeptide, a consensus sequence or a polypeptide motif as shown depicted in column 5 of Table II, and being depicted in the same respective line as said B1605 or a functional equivalent or a homologue thereof as depicted in column 7 of Table II or IV, preferably a homologue or functional equivalent as depicted in column 7 of Table II B, and being depicted in the same respective line as said B1605,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted arginine/ornithine transporter", preferably it is the molecule of section (a) or (b) of this paragraph.

In one embodiment, said molecule, which activity is to be increased in the process of the invention and which is the gene product with an activity of described as a "predicted arginine/ornithine transporter", is increased non-targeted.

The sequence of B1704 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as 3-deoxy-D-arabino-heptulosonate-7-phosphatesynthase.

Accordingly, in one embodiment, the process of the present invention comprises increasing or generating the activity of a gene product with the activity of a "3-deoxy-D-arabino-heptulosonate-7-phosphatesynthase" from Escherichia coli K12 or its functional equivalent or its homolog, e.g. the increase of
(a) a gene product of a gene comprising the nucleic acid molecule as shown in column 5 of Table I and being depicted in the same respective line as said B1704 or a functional equivalent or a homologue thereof as shown depicted in column 7 of Table I, preferably a homologue or functional equivalent as shown depicted in column 7 of Table I B, and being depicted in the same respective line as said B1704; or
(b) a polypeptide comprising a polypeptide, a consensus sequence or a polypeptide motif as shown depicted in column 5 of Table II, and being depicted in the same respective line as said B1704 or a functional equivalent or a homologue thereof as depicted in column 7 of Table II or IV, preferably a homologue or functional equivalent as depicted in column 7 of Table II B, and being depicted in the same respective line as said B1704,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "3-deoxy-D-arabino-heptulosonate-7-phosphatesynthase", preferably it is the molecule of section (a) or (b) of this paragraph.

In one embodiment, said molecule, which activity is to be increased in the process of the invention and which is the gene product with an activity of described as a "3-deoxy-D-arabino-heptulosonate-7-phosphatesynthase", is increased non-targeted.

The sequence of B1736 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as N,N'-diacetylchitobiose-specific enzyme IIA component of PTS.

Accordingly, in one embodiment, the process of the present invention comprises increasing or generating the activity of a gene product with the activity of a "N,N'-diacetylchitobiose-specific enzyme IIA component of PTS" from Escherichia coli K12 or its functional equivalent or its homolog, e.g. the increase of
(a) a gene product of a gene comprising the nucleic acid molecule as shown in column 5 of Table I and being depicted in the same respective line as said B1736 or a functional equivalent or a homologue thereof as shown depicted in column 7 of Table I, preferably a homologue or functional equivalent as shown depicted in column 7 of Table I B, and being depicted in the same respective line as said B1736; or
(b) a polypeptide comprising a polypeptide, a consensus sequence or a polypeptide motif as shown depicted in column 5 of Table II, and being depicted in the same respective line as said B1736 or a functional equivalent or a homologue thereof as depicted in column 7 of Table II or IV, preferably a homologue or functional equivalent as depicted in column 7 of Table II B, and being depicted in the same respective line as said B1736,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "N,N'-diacetylchitobiose-specific enzyme IIA component of PTS", preferably it is the molecule of section (a) or (b) of this paragraph.

In one embodiment, said molecule, which activity is to be increased in the process of the invention and which is the gene product with an activity of described as a "N,N'-diacetylchitobiose-specific enzyme IIA component of PTS", is increased plastidic.

The sequence of B1798 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as neutral amino-acid efflux system.

Accordingly, in one embodiment, the process of the present invention comprises increasing or generating the activity of a gene product with the activity of a "neutral amino-acid efflux system" from Escherichia coli K12 or its functional equivalent or its homolog, e.g. the increase of
(a) a gene product of a gene comprising the nucleic acid molecule as shown in column 5 of Table I and being depicted in the same respective line as said B1798 or a functional equivalent or a homologue thereof as shown depicted in column 7 of Table I, preferably a homologue or functional equivalent as shown depicted in column 7 of Table I B, and being depicted in the same respective line as said B1798; or
(b) a polypeptide comprising a polypeptide, a consensus sequence or a polypeptide motif as shown depicted in column 5 of Table II, and being depicted in the same respective line as said B1798 or a functional equivalent or a homologue thereof as depicted in column 7 of Table II or IV, preferably a homologue or functional equivalent as depicted in column 7 of Table II B, and being depicted in the same respective line as said B1798,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "neutral amino-acid efflux system", preferably it is the molecule of section (a) or (b) of this paragraph.

In one embodiment, said molecule, which activity is to be increased in the process of the invention and which is the gene product with an activity of described as a "neutral amino-acid efflux system", is increased non-targeted.

The sequence of B1878 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as b1878-protein.

Accordingly, in one embodiment, the process of the present invention comprises increasing or generating the activity of a gene product with the activity of a "b1878-protein" from Escherichia coli K12 or its functional equivalent or its homolog, e.g. the increase of
(a) a gene product of a gene comprising the nucleic acid molecule as shown in column 5 of Table I and being depicted in the same respective line as said B1878 or a functional equivalent or a homologue thereof as shown depicted in column 7 of Table I, preferably a homologue or functional equivalent as shown depicted in column 7 of Table I B, and being depicted in the same respective line as said B1878; or
(b) a polypeptide comprising a polypeptide, a consensus sequence or a polypeptide motif as shown depicted in column 5 of Table II, and being depicted in the same respective line as said B1878 or a functional equivalent or a homologue thereof as depicted in column 7 of Table II or IV, preferably a homologue or functional equivalent as depicted in column 7 of Table II B, and being depicted in the same respective line as said B1878,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "b1878-protein", preferably it is the molecule of section (a) or (b) of this paragraph.

In one embodiment, said molecule, which activity is to be increased in the process of the invention and which is the gene product with an activity of described as a "b1878-protein", is increased non-targeted.

The sequence of B1901 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as L-arabinose transporter subunit.

Accordingly, in one embodiment, the process of the present invention comprises increasing or generating the activity of a gene product with the activity of a "L-arabinose transporter subunit" from Escherichia coli K12 or its functional equivalent or its homolog, e.g. the increase of
(a) a gene product of a gene comprising the nucleic acid molecule as shown in column 5 of Table I and being depicted in the same respective line as said B1901 or a functional equivalent or a homologue thereof as shown depicted in column 7 of Table I, preferably a homologue or functional equivalent as shown depicted in column 7 of Table I B, and being depicted in the same respective line as said B1901; or
(b) a polypeptide comprising a polypeptide, a consensus sequence or a polypeptide motif as shown depicted in column 5 of Table II, and being depicted in the same respective line as said B1901 or a functional equivalent or a homologue thereof as depicted in column 7 of Table II or IV, preferably a homologue or functional equivalent as depicted in column 7 of Table II B, and being depicted in the same respective line as said B1901,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "L-arabinose transporter subunit", preferably it is the molecule of section (a) or (b) of this paragraph.

In one embodiment, said molecule, which activity is to be increased in the process of the invention and which is the gene product with an activity of described as a "L-arabinose transporter subunit", is increased plastidic.

The sequence of B1912 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as phosphatidylglycerophosphate synthetase.

Accordingly, in one embodiment, the process of the present invention comprises increasing or generating the activity of a gene product with the activity of a "phosphati-dylglycerophosphate synthetase" from Escherichia coli K12 or its functional equivalent or its homolog, e.g. the increase of
(a) a gene product of a gene comprising the nucleic acid molecule as shown in column 5 of Table I and being depicted in the same respective line as said B1912 or a functional equivalent or a homologue thereof as shown depicted in column 7 of Table I, preferably a homologue or functional equivalent as shown depicted in column 7 of Table I B, and being depicted in the same respective line as said B1912; or
(b) a polypeptide comprising a polypeptide, a consensus sequence or a polypeptide motif as shown depicted in column 5 of Table II, and being depicted in the same respective line as said B1912 or a functional equivalent or a homologue thereof as depicted in column 7 of Table II or IV, preferably a homologue or functional equivalent as depicted in column 7 of Table II B, and being depicted in the same respective line as said B1912,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "phosphatidylglycerophosphate synthetase", preferably it is the molecule of section (a) or (b) of this paragraph.

In one embodiment, said molecule, which activity is to be increased in the process of the invention and which is the gene product with an activity of described as a "phosphatidylglycerophosphate synthetase", is increased plastidic.

The sequence of B2027 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as regulator of length of O-antigen component of lipopolysaccharide chains.

Accordingly, in one embodiment, the process of the present invention comprises increasing or generating the activity of a gene product with the activity of a "regulator of length of O-antigen component of lipopolysaccharide chains" from Escherichia coli K12 or its functional equivalent or its homolog, e.g. the increase of
(a) a gene product of a gene comprising the nucleic acid molecule as shown in column 5 of Table I and being depicted in the same respective line as said B2027 or a functional equivalent or a homologue thereof as shown depicted in column 7 of Table I, preferably a homologue or functional equivalent as shown depicted in column 7 of Table I B, and being depicted in the same respective line as said B2027; or
(b) a polypeptide comprising a polypeptide, a consensus sequence or a polypeptide motif as shown depicted in column 5 of Table II, and being depicted in the same respective line as said B2027 or a functional equivalent or a homologue thereof as depicted in column 7 of Table II or IV, preferably a homologue or functional equivalent as depicted in column 7 of Table II B, and being depicted in the same respective line as said B2027,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "regulator of length of O-antigen component of lipopolysaccharide chains", preferably it is the molecule of section (a) or (b) of this paragraph.

In one embodiment, said molecule, which activity is to be increased in the process of the invention and which is the gene product with an activity of described as a "regulator of length of O-antigen component of lipopolysaccharide chains", is increased non-targeted.

The sequence of B2039 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as glucose-1-phosphate thymidylyltransferase.

Accordingly, in one embodiment, the process of the present invention comprises increasing or generating the activity of a gene product with the activity of a "glucose-1-phosphate thymidylyltransferase" from Escherichia coli K12 or its functional equivalent or its homolog, e.g. the increase of
(a) a gene product of a gene comprising the nucleic acid molecule as shown in column 5 of Table I and being depicted in the same respective line as said B2039 or a functional equivalent or a homologue thereof as shown depicted in column 7 of Table I, preferably a homologue or functional equivalent as shown depicted in column 7 of Table I B, and being depicted in the same respective line as said B2039; or
(b) a polypeptide comprising a polypeptide, a consensus sequence or a polypeptide motif as shown depicted in column 5 of Table II, and being depicted in the same respective line as said B2039 or a functional equivalent or a homologue thereof as depicted in column 7 of Table II or IV, preferably a homologue or functional equivalent as depicted in column 7 of Table II B, and being depicted in the same respective line as said B2039,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "glucose-1-phosphate thymidylyltransferase", preferably it is the molecule of section (a) or (b) of this paragraph.

In one embodiment, said molecule, which activity is to be increased in the process of the invention and which is the gene product with an activity of described as a "glucose-1-phosphate thymidylyltransferase", is increased non-targeted.

The sequence of B2075 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as multidrug efflux system (subunit B).

Accordingly, in one embodiment, the process of the present invention comprises increasing or generating the activity of a gene product with the activity of a "multidrug efflux system (subunit B)" from Escherichia coli K12 or its functional equivalent or its homolog, e.g. the increase of
(a) a gene product of a gene comprising the nucleic acid molecule as shown in column 5 of Table I and being depicted in the same respective line as said B2075 or a functional equivalent or a homologue thereof as shown depicted in column 7 of Table I, preferably a homologue or functional equivalent as shown depicted in column 7 of Table I B, and being depicted in the same respective line as said B2075; or
(b) a polypeptide comprising a polypeptide, a consensus sequence or a polypeptide motif as shown depicted in column 5 of Table II, and being depicted in the same respective line as said B2075 or a functional equivalent or a homologue thereof as depicted in column 7 of Table II or IV, preferably a homologue or functional equivalent as depicted in column 7 of Table II B, and being depicted in the same respective line as said B2075,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "multidrug efflux system (subunit B)", preferably it is the molecule of section (a) or (b) of this paragraph.

In one embodiment, said molecule, which activity is to be increased in the process of the invention and which is the gene product with an activity of described as a "multidrug efflux system (subunit B)", is increased non-targeted.

The sequence of B2153 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as GTP cyclohydrolase I.

Accordingly, in one embodiment, the process of the present invention comprises increasing or generating the activity of a gene product with the activity of a "GTP cyclohydrolase I" from Escherichia coli K12 or its functional equivalent or its homolog, e.g. the increase of
(a) a gene product of a gene comprising the nucleic acid molecule as shown in column 5 of Table I and being depicted in the same respective line as said B2153 or a functional equivalent or a homologue thereof as shown depicted in column 7 of Table I, preferably a homologue or functional equivalent as shown depicted in column 7 of Table I B, and being depicted in the same respective line as said B2153; or
(b) a polypeptide comprising a polypeptide, a consensus sequence or a polypeptide motif as shown depicted in column 5 of Table II, and being depicted in the same respective line as said B2153 or a functional equivalent or a homologue thereof as depicted in column 7 of Table II or IV, preferably a homologue or functional equivalent as depicted in column 7 of Table II B, and being depicted in the same respective line as said B2153,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "GTP cyclohydrolase I", preferably it is the molecule of section (a) or (b) of this paragraph.

In one embodiment, said molecule, which activity is to be increased in the process of the invention and which is the gene product with an activity of described as a "GTP cyclohydrolase I", is increased plastidic.

The sequence of B2194 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as heme lyase (CcmH subunit).

Accordingly, in one embodiment, the process of the present invention comprises increasing or generating the activity of a gene product with the activity of a "heme lyase (CcmH subunit)" from Escherichia coli K12 or its functional equivalent or its homolog, e.g. the increase of
(a) a gene product of a gene comprising the nucleic acid molecule as shown in column 5 of Table I and being depicted in the same respective line as said B2194 or a functional equivalent or a homologue thereof as shown depicted in column 7 of Table I, preferably a homologue or functional equivalent as shown depicted in column 7 of Table I B, and being depicted in the same respective line as said B2194; or
(b) a polypeptide comprising a polypeptide, a consensus sequence or a polypeptide motif as shown depicted in column 5 of Table II, and being depicted in the same respective line as said B2194 or a functional equivalent or a homologue thereof as depicted in column 7 of Table II or IV, preferably a homologue or functional equivalent as depicted in column 7 of Table II B, and being depicted in the same respective line as said B2194,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "heme lyase (CcmH subunit)", preferably it is the molecule of section (a) or (b) of this paragraph.

In one embodiment, said molecule, which activity is to be increased in the process of the invention and which is the gene product with an activity of described as a "heme lyase (CcmH subunit)", is increased non-targeted.

The sequence of B2226 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as b2226-protein.

Accordingly, in one embodiment, the process of the present invention comprises increasing or generating the activity of a gene product with the activity of a "b2226-protein" from Escherichia coli K12 or its functional equivalent or its homolog, e.g. the increase of
(a) a gene product of a gene comprising the nucleic acid molecule as shown in column 5 of Table I and being depicted in the same respective line as said B2226 or a functional equivalent or a homologue thereof as shown depicted in column 7 of Table I, preferably a homologue or functional equivalent as shown depicted in column 7 of Table I B, and being depicted in the same respective line as said B2226; or
(b) a polypeptide comprising a polypeptide, a consensus sequence or a polypeptide motif as shown depicted in column 5 of Table II, and being depicted in the same respective line as said B2226 or a functional equivalent or a homologue thereof as depicted in column 7 of Table II or IV, preferably a homologue or functional equivalent as depicted in column 7 of Table II B, and being depicted in the same respective line as said B2226,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "b2226-protein", preferably it is the molecule of section (a) or (b) of this paragraph.

In one embodiment, said molecule, which activity is to be increased in the process of the invention and which is the gene product with an activity of described as a "b2226-protein", is increased non-targeted.

The sequence of B2309 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as histidine/lysine/arginine/ornithine transporter subunit protein.

Accordingly, in one embodiment, the process of the present invention comprises increasing or generating the activity of a gene product with the activity of a "histidine/lysine/arginine/ornithine transporter subunit protein" from Escherichia coli K12 or its functional equivalent or its homolog, e.g. the increase of
(a) a gene product of a gene comprising the nucleic acid molecule as shown in column 5 of Table I and being depicted in the same respective line as said B2309 or a functional equivalent or a homologue thereof as shown depicted in column 7 of Table I, preferably a homologue or functional equivalent as shown depicted in column 7 of Table I B, and being depicted in the same respective line as said B2309; or
(b) a polypeptide comprising a polypeptide, a consensus sequence or a polypeptide motif as shown depicted in column 5 of Table II, and being depicted in the same respective line as said B2309 or a functional equivalent or a homologue thereof as depicted in column 7 of Table II or IV, preferably a homologue or functional equivalent as depicted in column 7 of Table II B, and being depicted in the same respective line as said B2309,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "histidine/lysine/arginine/ornithine transporter subunit protein", preferably it is the molecule of section (a) or (b) of this paragraph.

In one embodiment, said molecule, which activity is to be increased in the process of the invention and which is the gene product with an activity of described as a "histidine/lysine/arginine/ornithine transporter subunit protein", is increased plastidic.

The sequence of B2469 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as sensory histidine kinase in two-component regulatory system with NarP (NarL).

Accordingly, in one embodiment, the process of the present invention comprises increasing or generating the activity of a gene product with the activity of a "sensory histidine kinase in two-component regulatory system with NarP (NarL)" from Escherichia coli K12 or its functional equivalent or its homolog, e.g. the increase of
(a) a gene product of a gene comprising the nucleic acid molecule as shown in column 5 of Table I and being depicted in the same respective line as said B2469 or a functional equivalent or a homologue thereof as shown depicted in column 7 of Table I, preferably a homologue or functional equivalent as shown depicted in column 7 of Table I B, and being depicted in the same respective line as said B2469; or
(b) a polypeptide comprising a polypeptide, a consensus sequence or a polypeptide motif as shown depicted in column 5 of Table II, and being depicted in the same respective line as said B2469 or a functional equivalent or a homologue thereof as depicted in column 7 of Table II or IV, preferably a homologue or functional equivalent as depicted in column 7 of Table II B, and being depicted in the same respective line as said B2469,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "sensory histidine kinase in two-component regulatory system with NarP (NarL)", preferably it is the molecule of section (a) or (b) of this paragraph.

In one embodiment, said molecule, which activity is to be increased in the process of the invention and which is the gene product with an activity of described as a "sensory histidine kinase in two-component regulatory system with NarP (NarL)", is increased non-targeted.

The sequence of B2475 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as b2475-protein.

Accordingly, in one embodiment, the process of the present invention comprises increasing or generating the activity of a gene product with the activity of a "b2475-protein" from Escherichia coli K12 or its functional equivalent or its homolog, e.g. the increase of
(a) a gene product of a gene comprising the nucleic acid molecule as shown in column 5 of Table I and being depicted in the same respective line as said B2475 or a functional equivalent or a homologue thereof as shown depicted in column 7 of Table I, preferably a homologue or functional equivalent as shown depicted in column 7 of Table I B, and being depicted in the same respective line as said B2475; or
(b) a polypeptide comprising a polypeptide, a consensus sequence or a polypeptide motif as shown depicted in column 5 of Table II, and being depicted in the same respective line as said B2475 or a functional equivalent or a homologue thereof as depicted in column 7 of Table II or IV, preferably a homologue or functional equivalent as depicted in column 7 of Table II B, and being depicted in the same respective line as said B2475,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "b2475-protein", preferably it is the molecule of section (a) or (b) of this paragraph.

In one embodiment, said molecule, which activity is to be increased in the process of the invention and which is the gene product with an activity of described as a "b2475-protein", is increased non-targeted.

The sequence of B2482 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as NADH dehydrogenase (subunit N).

Accordingly, in one embodiment, the process of the present invention comprises increasing or generating the activity of a gene product with the activity of a "NADH dehydrogenase (subunit N)" from Escherichia coli K12 or its functional equivalent or its homolog, e.g. the increase of
(a) a gene product of a gene comprising the nucleic acid molecule as shown in column 5 of Table I and being depicted in the same respective line as said B2482 or a functional equivalent or a homologue thereof as shown depicted in column 7 of Table I, preferably a homologue or functional equivalent as shown depicted in column 7 of Table I B, and being depicted in the same respective line as said B2482; or
(b) a polypeptide comprising a polypeptide, a consensus sequence or a polypeptide motif as shown depicted in column 5 of Table II, and being depicted in the same respective line as said B2482 or a functional equivalent or a homologue thereof as depicted in column 7 of Table II or IV, preferably a homologue or functional equivalent as depicted in column 7 of Table II B, and being depicted in the same respective line as said B2482,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "NADH dehydrogenase (subunit N)", preferably it is the molecule of section (a) or (b) of this paragraph.

In one embodiment, said molecule, which activity is to be increased in the process of the invention and which is the gene product with an activity of described as a "NADH dehydrogenase (subunit N)", is increased non-targeted.

The sequence of B2541 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as 2,3-dihydroxy-2,3-dihydrophenylpropionatedehydrogenase.

Accordingly, in one embodiment, the process of the present invention comprises increasing or generating the activity of a gene product with the activity of a "2,3-dihydroxy-2,3-dihydrophenylpropionatedehydrogenase" from Escherichia coli K12 or its functional equivalent or its homolog, e.g. the increase of
(a) a gene product of a gene comprising the nucleic acid molecule as shown in column 5 of Table I and being depicted in the same respective line as said B2541 or a functional equivalent or a homologue thereof as shown depicted in column 7 of Table I, preferably a homologue or functional equivalent as shown depicted in column 7 of Table I B, and being depicted in the same respective line as said B2541; or
(b) a polypeptide comprising a polypeptide, a consensus sequence or a polypeptide motif as shown depicted in column 5 of Table II, and being depicted in the same respective line as said B2541 or a functional equivalent or a homologue thereof as depicted in column 7 of Table II or IV, preferably a homologue or functional equivalent as depicted in column 7 of Table II B, and being depicted in the same respective line as said B2541,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "2,3-dihydroxy-2,3-dihydrophenylpropionatedehydrogenase", preferably it is the molecule of section (a) or (b) of this paragraph.

In one embodiment, said molecule, which activity is to be increased in the process of the invention and which is the gene product with an activity of described as a "2,3-dihydroxy-2,3-dihydrophenylpropionatedehydrogenase", is increased non-targeted.

The sequence of B2559 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as tRNA-specific adenosine deaminase.

Accordingly, in one embodiment, the process of the present invention comprises increasing or generating the activity of a gene product with the activity of a "tRNA-specific adenosine deaminase" from Escherichia coli K12 or its functional equivalent or its homolog, e.g. the increase of
(a) a gene product of a gene comprising the nucleic acid molecule as shown in column 5 of Table I and being depicted in the same respective line as said B2559 or a functional equivalent or a homologue thereof as shown depicted in column 7 of Table I, preferably a homologue or functional equivalent as shown depicted in column 7 of Table I B, and being depicted in the same respective line as said B2559; or
(b) a polypeptide comprising a polypeptide, a consensus sequence or a polypeptide motif as shown depicted in column 5 of Table II, and being depicted in the same respective line as said B2559 or a functional equivalent or a homologue thereof as depicted in column 7 of Table II or IV, preferably a homologue or functional equivalent as depicted in column 7 of Table II B, and being depicted in the same respective line as said B2559,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "tRNA-specific adenosine deaminase", preferably it is the molecule of section (a) or (b) of this paragraph.

In one embodiment, said molecule, which activity is to be increased in the process of the invention and which is the gene product with an activity of described as a "tRNA-specific adenosine deaminase", is increased plastidic.

The sequence of B2605 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted outer membrane lipoprotein.

Accordingly, in one embodiment, the process of the present invention comprises increasing or generating the activity of a gene product with the activity of a "predicted outer membrane lipoprotein" from Escherichia coli K12 or its functional equivalent or its homolog, e.g. the increase of
(a) a gene product of a gene comprising the nucleic acid molecule as shown in column 5 of Table I and being depicted in the same respective line as said B2605 or a functional equivalent or a homologue thereof as shown depicted in column 7 of Table I, preferably a homologue or functional equivalent as shown depicted in column 7 of Table I B, and being depicted in the same respective line as said B2605; or
(b) a polypeptide comprising a polypeptide, a consensus sequence or a polypeptide motif as shown depicted in column 5 of Table II, and being depicted in the same respective line as said B2605 or a functional equivalent or a homologue thereof as depicted in column 7 of Table II or IV, preferably a homologue or functional equivalent as depicted in column 7 of Table II B, and being depicted in the same respective line as said B2605,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted outer membrane lipoprotein", preferably it is the molecule of section (a) or (b) of this paragraph.

In one embodiment, said molecule, which activity is to be increased in the process of the invention and which is the gene product with an activity of described as a "predicted outer membrane lipoprotein", is increased non-targeted.

The sequence of B2630 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as CP4-57 prophage/ RNase LS.

Accordingly, in one embodiment, the process of the present invention comprises increasing or generating the activity of a gene product with the activity of a "CP4-57 prophage/ RNase LS" from Escherichia coli K12 or its functional equivalent or its homolog, e.g. the increase of
(a) a gene product of a gene comprising the nucleic acid molecule as shown in column 5 of Table I and being depicted in the same respective line as said B2630 or a functional equivalent or a homologue thereof as shown depicted in column 7 of Table I, preferably a homologue or functional equivalent as shown depicted in column 7 of Table I B, and being depicted in the same respective line as said B2630; or
(b) a polypeptide comprising a polypeptide, a consensus sequence or a polypeptide motif as shown depicted in column 5 of Table II, and being depicted in the same respective line as said B2630 or a functional equivalent or a homologue thereof as depicted in column 7 of Table II or IV, preferably a homologue or functional equivalent as depicted in column 7 of Table II B, and being depicted in the same respective line as said B2630,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "CP4-57 prophage/ RNase LS", preferably it is the molecule of section (a) or (b) of this paragraph.

In one embodiment, said molecule, which activity is to be increased in the process of the invention and which is the gene product with an activity of described as a "CP4-57 prophage/ RNase LS", is increased non-targeted.

The sequence of B2678 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as glycine betaine transporter subunit protein.

Accordingly, in one embodiment, the process of the present invention comprises increasing or generating the activity of a gene product with the activity of a "glycine betaine transporter subunit protein" from Escherichia coli K12 or its functional equivalent or its homolog, e.g. the increase of
(a) a gene product of a gene comprising the nucleic acid molecule as shown in column 5 of Table I and being depicted in the same respective line as said B2678 or a functional equivalent or a homologue thereof as shown depicted in column 7 of Table I, preferably a homologue or functional equivalent as shown depicted in column 7 of Table I B, and being depicted in the same respective line as said B2678; or
(b) a polypeptide comprising a polypeptide, a consensus sequence or a polypeptide motif as shown depicted in column 5 of Table II, and being depicted in the same respective line as said B2678 or a functional equivalent or a homologue thereof as depicted in column 7 of Table II or IV, preferably a homologue or functional equivalent as depicted in column 7 of Table II B, and being depicted in the same respective line as said B2678,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "glycine betaine transporter subunit protein", preferably it is the molecule of section (a) or (b) of this paragraph.

In one embodiment, said molecule, which activity is to be increased in the process of the invention and which is the gene product with an activity of described as a "glycine betaine transporter subunit protein", is increased plastidic.

The sequence of B2715 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as cellobiose/arbutin/salicin-specific PTS enzyme (IIB component/IC component).

Accordingly, in one embodiment, the process of the present invention comprises increasing or generating the activity of a gene product with the activity of a "cellobiose/arbutin/salicin-specific PTS enzyme (IIB component/IC component)" from Escherichia coli K12 or its functional equivalent or its homolog, e.g. the increase of
(a) a gene product of a gene comprising the nucleic acid molecule as shown in column 5 of Table I and being depicted in the same respective line as said B2715 or a functional equivalent or a homologue thereof as shown depicted in column 7 of Table I, preferably a homologue or functional equivalent as shown depicted in column 7 of Table I B, and being depicted in the same respective line as said B2715; or
(b) a polypeptide comprising a polypeptide, a consensus sequence or a polypeptide motif as shown depicted in column 5 of Table II, and being depicted in the same respective line as said B2715 or a functional equivalent or a homologue thereof as depicted in column 7 of Table II or IV, preferably a homologue or functional equivalent as depicted in column 7 of Table II B, and being depicted in the same respective line as said B2715,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "cellobiose/arbutin/salicin-specific PTS enzyme (IIB component/IC component)", preferably it is the molecule of section (a) or (b) of this paragraph.

In one embodiment, said molecule, which activity is to be increased in the process of the invention and which is the gene product with an activity of described as a "cellobiose/arbutin/salicin-specific PTS enzyme (IIB component/IC component)", is increased plastidic.

The sequence of B2776 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted kinase.

Accordingly, in one embodiment, the process of the present invention comprises increasing or generating the activity of a gene product with the activity of a "predicted kinase" from Escherichia coli K12 or its functional equivalent or its homolog, e.g. the increase of
(a) a gene product of a gene comprising the nucleic acid molecule as shown in column 5 of Table I and being depicted in the same respective line as said B2776 or a functional equivalent or a homologue thereof as shown depicted in column 7 of Table I, preferably a homologue or functional equivalent as shown depicted in column 7 of Table I B, and being depicted in the same respective line as said B2776; or
(b) a polypeptide comprising a polypeptide, a consensus sequence or a polypeptide motif as shown depicted in column 5 of Table II, and being depicted in the same respective line as said B2776 or a functional equivalent or a homologue thereof as depicted in column 7 of Table II or IV, preferably a homologue or functional equivalent as depicted in column 7 of Table II B, and being depicted in the same respective line as said B2776,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted kinase", preferably it is the molecule of section (a) or (b) of this paragraph.

In one embodiment, said molecule, which activity is to be increased in the process of the invention and which is the gene product with an activity of described as a "predicted kinase", is increased non-targeted.

The sequence of B2791 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as tRNA pseudouridine synthase.

Accordingly, in one embodiment, the process of the present invention comprises increasing or generating the activity of a gene product with the activity of a "tRNA pseudouridine synthase" from Escherichia coli K12 or its functional equivalent or its homolog, e.g. the increase of
(a) a gene product of a gene comprising the nucleic acid molecule as shown in column 5 of Table I and being depicted in the same respective line as said B2791 or a functional equivalent or a homologue thereof as shown depicted in column 7 of Table I, preferably a homologue or functional equivalent as shown depicted in column 7 of Table I B, and being depicted in the same respective line as said B2791; or
(b) a polypeptide comprising a polypeptide, a consensus sequence or a polypeptide motif as shown depicted in column 5 of Table II, and being depicted in the same respective line as said B2791 or a functional equivalent or a homologue thereof as depicted in column 7 of Table II or IV, preferably a homologue or functional equivalent as depicted in column 7 of Table II B, and being depicted in the same respective line as said B2791,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "tRNA pseudouridine synthase", preferably it is the molecule of section (a) or (b) of this paragraph.

In one embodiment, said molecule, which activity is to be increased in the process of the invention and which is the gene product with an activity of described as a "tRNA pseudouridine synthase", is increased non-targeted.

The sequence of B2912 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted ligase.

Accordingly, in one embodiment, the process of the present invention comprises increasing or generating the activity of a gene product with the activity of a "predicted ligase" from Escherichia coli K12 or its functional equivalent or its homolog, e.g. the increase of
(a) a gene product of a gene comprising the nucleic acid molecule as shown in column 5 of Table I and being depicted in the same respective line as said B2912 or a functional equivalent or a homologue thereof as shown depicted in column 7 of Table I, preferably a homologue or functional equivalent as shown depicted in column 7 of Table I B, and being depicted in the same respective line as said B2912; or
(b) a polypeptide comprising a polypeptide, a consensus sequence or a polypeptide motif as shown depicted in column 5 of Table II, and being depicted in the same respective line as said B2912 or a functional equivalent or a homologue thereof as depicted in column 7 of Table II or IV, preferably a homologue or functional equivalent as depicted in column 7 of Table II B, and being depicted in the same respective line as said B2912,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted ligase", preferably it is the molecule of section (a) or (b) of this paragraph.

In one embodiment, said molecule, which activity is to be increased in the process of the invention and which is the gene product with an activity of described as a "predicted ligase", is increased non-targeted.

The sequence of B2965 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as ornithine decarboxylase.

Accordingly, in one embodiment, the process of the present invention comprises increasing or generating the activity of a gene product with the activity of a "ornithine decarboxylase" from Escherichia coli K12 or its functional equivalent or its homolog, e.g. the increase of
(a) a gene product of a gene comprising the nucleic acid molecule as shown in column 5 of Table I and being depicted in the same respective line as said B2965 or a functional equivalent or a homologue thereof as shown depicted in column 7 of Table I, preferably a homologue or functional equivalent as shown depicted in column 7 of Table I B, and being depicted in the same respective line as said B2965; or
(b) a polypeptide comprising a polypeptide, a consensus sequence or a polypeptide motif as shown depicted in column 5 of Table II, and being depicted in the same respective line as said B2965 or a functional equivalent or a homologue thereof as depicted in column 7 of Table II or IV, preferably a homologue or functional equivalent as depicted in column 7 of Table II B, and being depicted in the same respective line as said B2965,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "ornithine decarboxylase", preferably it is the molecule of section (a) or (b) of this paragraph.

In one embodiment, said molecule, which activity is to be increased in the process of the invention and which is the gene product with an activity of described as a "ornithine decarboxylase", is increased plastidic.

The sequence of B2987 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as phosphate transporter.

Accordingly, in one embodiment, the process of the present invention comprises increasing or generating the activity of a gene product with the activity of a "phosphate transporter" from Escherichia coli K12 or its functional equivalent or its homolog, e.g. the increase of
(a) a gene product of a gene comprising the nucleic acid molecule as shown in column 5 of Table I and being depicted in the same respective line as said B2987 or a functional equivalent or a homologue thereof as shown depicted in column 7 of Table I, preferably a homologue or functional equivalent as shown depicted in column 7 of Table I B, and being depicted in the same respective line as said B2987; or
(b) a polypeptide comprising a polypeptide, a consensus sequence or a polypeptide motif as shown depicted in column 5 of Table II, and being depicted in the same respective line as said B2987 or a functional equivalent or a homologue thereof as depicted in column 7 of Table II or IV, preferably a homologue or functional equivalent as depicted in column 7 of Table II B, and being depicted in the same respective line as said B2987,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "phosphate transporter", preferably it is the molecule of section (a) or (b) of this paragraph.

In one embodiment, said molecule, which activity is to be increased in the process of the invention and which is the gene product with an activity of described as a "phosphate transporter", is increased plastidic.

The sequence of B2987 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as phosphate transporter.

Accordingly, in one embodiment, the process of the present invention comprises increasing or generating the activity of a gene product with the activity of a "phosphate transporter" from Escherichia coli K12 or its functional equivalent or its homolog, e.g. the increase of
(a) a gene product of a gene comprising the nucleic acid molecule as shown in column 5 of Table I and being depicted in the same respective line as said B2987 or a functional equivalent or a homologue thereof as shown depicted in column 7 of Table I, preferably a homologue or functional equivalent as shown depicted in column 7 of Table I B, and being depicted in the same respective line as said B2987; or
(b) a polypeptide comprising a polypeptide, a consensus sequence or a polypeptide motif as shown depicted in column 5 of Table II, and being depicted in the same respective line as said B2987 or a functional equivalent or a homologue thereof as depicted in column 7 of Table II or IV, preferably a homologue or functional equivalent as depicted in column 7 of Table II B, and being depicted in the same respective line as said B2987,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "phosphate transporter", preferably it is the molecule of section (a) or (b) of this paragraph.

In one embodiment, said molecule, which activity is to be increased in the process of the invention and which is the gene product with an activity of described as a "phosphate transporter", is increased non-targeted.

The sequence of B3093 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as hexuronate transporter.

Accordingly, in one embodiment, the process of the present invention comprises increasing or generating the activity of a gene product with the activity of a "hexuronate transporter" from Escherichia coli K12 or its functional equivalent or its homolog, e.g. the increase of
(a) a gene product of a gene comprising the nucleic acid molecule as shown in column 5 of Table I and being depicted in the same respective line as said B3093 or a functional equivalent or a homologue thereof as shown depicted in column 7 of Table I, preferably a homologue or functional equivalent as shown depicted in column 7 of Table I B, and being depicted in the same respective line as said B3093; or
(b) a polypeptide comprising a polypeptide, a consensus sequence or a polypeptide motif as shown depicted in column 5 of Table II, and being depicted in the same respective line as said B3093 or a functional equivalent or a homologue thereof as depicted in column 7 of Table II or IV, preferably a homologue or functional equivalent as depicted in column 7 of Table II B, and being depicted in the same respective line as said B3093,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "hexuronate transporter", preferably it is the molecule of section (a) or (b) of this paragraph.

In one embodiment, said molecule, which activity is to be increased in the process of the invention and which is the gene product with an activity of described as a "hexuronate transporter", is increased plastidic.

The sequence of B3363 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as peptidyl-prolyl cis-trans isomerase A (rotamase A).

Accordingly, in one embodiment, the process of the present invention comprises increasing or generating the activity of a gene product with the activity of a "peptidyl-prolyl cis-trans isomerase A (rotamase A)" from Escherichia coli K12 or its functional equivalent or its homolog, e.g. the increase of
(a) a gene product of a gene comprising the nucleic acid molecule as shown in column 5 of Table I and being depicted in the same respective line as said B3363 or a functional equivalent or a homologue thereof as shown depicted in column 7 of Table I, preferably a homologue or functional equivalent as shown depicted in column 7 of Table I B, and being depicted in the same respective line as said B3363; or
(b) a polypeptide comprising a polypeptide, a consensus sequence or a polypeptide motif as shown depicted in column 5 of Table II, and being depicted in the same respective line as said B3363 or a functional equivalent or a homologue thereof as depicted in column 7 of Table II or IV, preferably a homologue or functional equivalent as depicted in column 7 of Table II B, and being depicted in the same respective line as said B3363,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "peptidyl-prolyl cis-trans isomerase A (rotamase A)", preferably it is the molecule of section (a) or (b) of this paragraph.

In one embodiment, said molecule, which activity is to be increased in the process of the invention and which is the gene product with an activity of described as a "peptidyl-prolyl cis-trans isomerase A (rotamase A)", is increased plastidic.

The sequence of B3429 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as glycogen synthase.

Accordingly, in one embodiment, the process of the present invention comprises increasing or generating the activity of a gene product with the activity of a "glycogen synthase" from Escherichia coli K12 or its functional equivalent or its homolog, e.g. the increase of
(a) a gene product of a gene comprising the nucleic acid molecule as shown in column 5 of Table I and being depicted in the same respective line as said B3429 or a functional equivalent or a homologue thereof as shown depicted in column 7 of Table I, preferably a homologue or functional equivalent as shown depicted in column 7 of Table I B, and being depicted in the same respective line as said B3429; or
(b) a polypeptide comprising a polypeptide, a consensus sequence or a polypeptide motif as shown depicted in column 5 of Table II, and being depicted in the same respective line as said B3429 or a functional equivalent or a homologue thereof as depicted in column 7 of Table II or IV, preferably a homologue or functional equivalent as depicted in column 7 of Table II B, and being depicted in the same respective line as said B3429,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "glycogen synthase", preferably it is the molecule of section (a) or (b) of this paragraph.

In one embodiment, said molecule, which activity is to be increased in the process of the invention and which is the gene product with an activity of described as a "glycogen synthase", is increased plastidic.

The sequence of B3568 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as D-xylose transporter subunit.

Accordingly, in one embodiment, the process of the present invention comprises increasing or generating the activity of a gene product with the activity of a "D-xylose transporter subunit" from Escherichia coli K12 or its functional equivalent or its homolog, e.g. the increase of
(a) a gene product of a gene comprising the nucleic acid molecule as shown in column 5 of Table I and being depicted in the same respective line as said B3568 or a functional equivalent or a homologue thereof as shown depicted in column 7 of Table I, preferably a homologue or functional equivalent as shown depicted in column 7 of Table I B, and being depicted in the same respective line as said B3568; or
(b) a polypeptide comprising a polypeptide, a consensus sequence or a polypeptide motif as shown depicted in column 5 of Table II, and being depicted in the same respective line as said B3568 or a functional equivalent or a homologue thereof as depicted in column 7 of Table II or IV, preferably a homologue or functional equivalent as depicted in column 7 of Table II B, and being depicted in the same respective line as said B3568,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "D-xylose transporter subunit", preferably it is the molecule of section (a) or (b) of this paragraph. In one embodiment, said molecule, which activity is to be increased in the process of the invention and which is the gene product with an activity of described as a "D-xylose transporter subunit", is increased plastidic.

The sequence of B3616 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as L-threonine 3-dehydrogenase.

Accordingly, in one embodiment, the process of the present invention comprises increasing or generating the activity of a gene product with the activity of a "L-threonine 3-dehydrogenase" from Escherichia coli K12 or its functional equivalent or its homolog, e.g. the increase of
(a) a gene product of a gene comprising the nucleic acid molecule as shown in column 5 of Table I and being depicted in the same respective line as said B3616 or a functional equivalent or a homologue thereof as shown depicted in column 7 of Table I, preferably a homologue or functional equivalent as shown depicted in column 7 of Table I B, and being depicted in the same respective line as said B3616; or
(b) a polypeptide comprising a polypeptide, a consensus sequence or a polypeptide motif as shown depicted in column 5 of Table II, and being depicted in the same respective line as said B3616 or a functional equivalent or a homologue thereof as depicted in column 7 of Table II or IV, preferably a homologue or functional equivalent as depicted in column 7 of Table II B, and being depicted in the same respective line as said B3616,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "L-threonine 3-dehydrogenase", preferably it is the molecule of section (a) or (b) of this paragraph.

In one embodiment, said molecule, which activity is to be increased in the process of the invention and which is the gene product with an activity of described as a "L-threonine 3-dehydrogenase", is increased plastidic.

The sequence of B3616 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as L-threonine 3-dehydrogenase.

Accordingly, in one embodiment, the process of the present invention comprises increasing or generating the activity of a gene product with the activity of a "L-threonine 3-dehydrogenase" from Escherichia coli K12 or its functional equivalent or its homolog, e.g. the increase of
(a) a gene product of a gene comprising the nucleic acid molecule as shown in column 5 of Table I and being depicted in the same respective line as said B3616 or a functional equivalent or a homologue thereof as shown depicted in column 7 of Table I, preferably a homologue or functional equivalent as shown depicted in column 7 of Table I B, and being depicted in the same respective line as said B3616; or
(b) a polypeptide comprising a polypeptide, a consensus sequence or a polypeptide motif as shown depicted in column 5 of Table II, and being depicted in the same respective line as said B3616 or a functional equivalent or a homologue thereof as depicted in column 7 of Table II or IV, preferably a homologue or functional equivalent as depicted in column 7 of Table II B, and being depicted in the same respective line as said B3616,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "L-threonine 3-dehydrogenase", preferably it is the molecule of section (a) or (b) of this paragraph.

In one embodiment, said molecule, which activity is to be increased in the process of the invention and which is the gene product with an activity of described as a "L-threonine 3-dehydrogenase", is increased non-targeted.

The sequence of B3812 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted hydrolase.

Accordingly, in one embodiment, the process of the present invention comprises increasing or generating the activity of a gene product with the activity of a "predicted hydrolase" from Escherichia coli K12 or its functional equivalent or its homolog, e.g. the increase of
(a) a gene product of a gene comprising the nucleic acid molecule as shown in column 5 of Table I and being depicted in the same respective line as said B3812 or a functional equivalent or a homologue thereof as shown depicted in column 7 of Table I, preferably a homologue or functional equivalent as shown depicted in column 7 of Table I B, and being depicted in the same respective line as said B3812; or
(b) a polypeptide comprising a polypeptide, a consensus sequence or a polypeptide motif as shown depicted in column 5 of Table II, and being depicted in the same respective line as said B3812 or a functional equivalent or a homologue thereof as depicted in column 7 of Table II or IV, preferably a homologue or functional equivalent as depicted in column 7 of Table II B, and being depicted in the same respective line as said B3812,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted hydrolase", preferably it is the molecule of section (a) or (b) of this paragraph.

In one embodiment, said molecule, which activity is to be increased in the process of the invention and which is the gene product with an activity of described as a "predicted hydrolase", is increased non-targeted.

The sequence of B3899 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted PTS enzymes (IIB component/IIC component).

Accordingly, in one embodiment, the process of the present invention comprises increasing or generating the activity of a gene product with the activity of a "predicted PTS enzymes (IIB component/IIC component)" from Escherichia coli K12 or its functional equivalent or its homolog, e.g. the increase of
(a) a gene product of a gene comprising the nucleic acid molecule as shown in column 5 of Table I and being depicted in the same respective line as said B3899 or a functional equivalent or a homologue thereof as shown depicted in column 7 of Table I, preferably a homologue or functional equivalent as shown depicted in column 7 of Table I B, and being depicted in the same respective line as said B3899; or
(b) a polypeptide comprising a polypeptide, a consensus sequence or a polypeptide motif as shown depicted in column 5 of Table II, and being depicted in the same respective line as said B3899 or a functional equivalent or a homologue thereof as depicted in column 7 of Table II or IV, preferably a homologue or functional equivalent as depicted in column 7 of Table II B, and being depicted in the same respective line as said B3899,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted PTS enzymes (IIB component/IIC component)", preferably it is the molecule of section (a) or (b) of this paragraph.

In one embodiment, said molecule, which activity is to be increased in the process of the invention and which is the gene product with an activity of described as a "predicted PTS enzymes (IIB component/IIC component)", is increased non-targeted.

The sequence of B3929 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as ribonuclease activity regulator protein RraA.

Accordingly, in one embodiment, the process of the present invention comprises increasing or generating the activity of a gene product with the activity of a "ribonuclease activity regulator protein RraA" from Escherichia coli K12 or its functional equivalent or its homolog, e.g. the increase of
(a) a gene product of a gene comprising the nucleic acid molecule as shown in column 5 of Table I and being depicted in the same respective line as said B3929 or a functional equivalent or a homologue thereof as shown depicted in column 7 of Table I, preferably a homologue or functional equivalent as shown depicted in column 7 of Table I B, and being depicted in the same respective line as said B3929; or
(b) a polypeptide comprising a polypeptide, a consensus sequence or a polypeptide motif as shown depicted in column 5 of Table II, and being depicted in the same respective line as said B3929 or a functional equivalent or a homologue thereof as depicted in column 7 of Table II or IV, preferably a homologue or functional equivalent as depicted in column 7 of Table II B, and being depicted in the same respective line as said B3929,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "ribonuclease activity regulator protein RraA", preferably it is the molecule of section (a) or (b) of this paragraph.

In one embodiment, said molecule, which activity is to be increased in the process of the invention and which is the gene product with an activity of described as a "ribonuclease activity regulator protein RraA", is increased plastidic.

The sequence of B3938 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as transcriptional repressor protein MetJ.

Accordingly, in one embodiment, the process of the present invention comprises increasing or generating the activity of a gene product with the activity of a "transcriptional repressor protein MetJ" from Escherichia coli K12 or its functional equivalent or its homolog, e.g. the increase of
(a) a gene product of a gene comprising the nucleic acid molecule as shown in column 5 of Table I and being depicted in the same respective line as said B3938 or a functional equivalent or a homologue thereof as shown depicted in column 7 of Table I, preferably a homologue or functional equivalent as shown depicted in column 7 of Table I B, and being depicted in the same respective line as said B3938; or
(b) a polypeptide comprising a polypeptide, a consensus sequence or a polypeptide motif as shown depicted in column 5 of Table II, and being depicted in the same respective line as said B3938 or a functional equivalent or a homologue thereof as depicted in column 7 of Table II or IV, preferably a homologue or functional equivalent as depicted in column 7 of Table II B, and being depicted in the same respective line as said B3938,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "transcriptional repressor protein MetJ", preferably it is the molecule of section (a) or (b) of this paragraph.

In one embodiment, said molecule, which activity is to be increased in the process of the invention and which is the gene product with an activity of described as a "transcriptional repressor protein MetJ", is increased non-targeted.

The sequence of B3974 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as pantothenate kinase.

Accordingly, in one embodiment, the process of the present invention comprises increasing or generating the activity of a gene product with the activity of a "pantothenate kinase" from Escherichia coli K12 or its functional equivalent or its homolog, e.g. the increase of
(a) a gene product of a gene comprising the nucleic acid molecule as shown in column 5 of Table I and being depicted in the same respective line as said B3974 or a functional equivalent or a homologue thereof as shown depicted in column 7 of Table I, preferably a homologue or functional equivalent as shown depicted in column 7 of Table I B, and being depicted in the same respective line as said B3974; or
(b) a polypeptide comprising a polypeptide, a consensus sequence or a polypeptide motif as shown depicted in column 5 of Table II, and being depicted in the same respective line as said B3974 or a functional equivalent or a homologue thereof as depicted in column 7 of Table II or IV, preferably a homologue or functional equivalent as depicted in column 7 of Table II B, and being depicted in the same respective line as said B3974,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "pantothenate kinase", preferably it is the molecule of section (a) or (b) of this paragraph.

In one embodiment, said molecule, which activity is to be increased in the process of the invention and which is the gene product with an activity of described as a "pantothenate kinase", is increased non-targeted.

The sequence of B3989 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as heat shock protein.

Accordingly, in one embodiment, the process of the present invention comprises increasing or generating the activity of a gene product with the activity of a "heat shock protein" from Escherichia coli K12 or its functional equivalent or its homolog, e.g. the increase of
(a) a gene product of a gene comprising the nucleic acid molecule as shown in column 5 of Table I and being depicted in the same respective line as said B3989 or a functional equivalent or a homologue thereof as shown depicted in column 7 of Table I, preferably a homologue or functional equivalent as shown depicted in column 7 of Table I B, and being depicted in the same respective line as said B3989; or
(b) a polypeptide comprising a polypeptide, a consensus sequence or a polypeptide motif as shown depicted in column 5 of Table II, and being depicted in the same respective line as said B3989 or a functional equivalent or a homologue thereof as depicted in column 7 of Table II or IV, preferably a homologue or functional equivalent as depicted in column 7 of Table II B, and being depicted in the same respective line as said B3989,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "heat shock protein", preferably it is the molecule of section (a) or (b) of this paragraph.

In one embodiment, said molecule, which activity is to be increased in the process of the invention and which is the gene product with an activity of described as a "heat shock protein", is increased non-targeted.

The sequence of B4029 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted porin.

Accordingly, in one embodiment, the process of the present invention comprises increasing or generating the activity of a gene product with the activity of a "predicted porin" from Escherichia coli K12 or its functional equivalent or its homolog, e.g. the increase of
(a) a gene product of a gene comprising the nucleic acid molecule as shown in column 5 of Table I and being depicted in the same respective line as said B4029 or a functional equivalent or a homologue thereof as shown depicted in column 7 of Table I, preferably a homologue or functional equivalent as shown depicted in column 7 of Table I B, and being depicted in the same respective line as said B4029; or
(b) a polypeptide comprising a polypeptide, a consensus sequence or a polypeptide motif as shown depicted in column 5 of Table II, and being depicted in the same respective line as said B4029 or a functional equivalent or a homologue thereof as depicted in column 7 of Table II or IV, preferably a homologue or functional equivalent as depicted in column 7 of Table II B, and being depicted in the same respective line as said B4029,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted porin", preferably it is the molecule of section (a) or (b) of this paragraph.

In one embodiment, said molecule, which activity is to be increased in the process of the invention and which is the gene product with an activity of described as a "predicted porin", is increased non-targeted.

The sequence of B4139 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as aspartate ammonia-lyase.

Accordingly, in one embodiment, the process of the present invention comprises increasing or generating the activity of a gene product with the activity of a "aspartate ammonia-lyase" from Escherichia coli K12 or its functional equivalent or its homolog, e.g. the increase of
(a) a gene product of a gene comprising the nucleic acid molecule as shown in column 5 of Table I and being depicted in the same respective line as said B4139 or a functional equivalent or a homologue thereof as shown depicted in column 7 of Table I, preferably a homologue or functional equivalent as shown depicted in column 7 of Table I B, and being depicted in the same respective line as said B4139; or
(b) a polypeptide comprising a polypeptide, a consensus sequence or a polypeptide motif as shown depicted in column 5 of Table II, and being depicted in the same respective line as said B4139 or a functional equivalent or a homologue thereof as depicted in column 7 of Table II or IV, preferably a homologue or functional equivalent as depicted in column 7 of Table II B, and being depicted in the same respective line as said B4139,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "aspartate ammonia-lyase", preferably it is the molecule of section (a) or (b) of this paragraph.

In one embodiment, said molecule, which activity is to be increased in the process of the invention and which is the gene product with an activity of described as a "aspartate ammonia-lyase", is increased plastidic.

The sequence of B4390 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as nicotinamide-nucleotide adenylyltransferase.

Accordingly, in one embodiment, the process of the present invention comprises increasing or generating the activity of a gene product with the activity of a "nicotinamide-nucleotide adenylyltransferase" from Escherichia coli K12 or its functional equivalent or its homolog, e.g. the increase of
(a) a gene product of a gene comprising the nucleic acid molecule as shown in column 5 of Table I and being depicted in the same respective line as said B4390 or a functional equivalent or a homologue thereof as shown depicted in column 7 of Table I, preferably a homologue or functional equivalent as shown depicted in column 7 of Table I B, and being depicted in the same respective line as said B4390; or
(b) a polypeptide comprising a polypeptide, a consensus sequence or a polypeptide motif as shown depicted in column 5 of Table II, and being depicted in the same respective line as said B4390 or a functional equivalent or a homologue thereof as depicted in column 7 of Table II or IV, preferably a homologue or functional equivalent as depicted in column 7 of Table II B, and being depicted in the same respective line as said B4390,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "nicotinamide-nucleotide adenylyltransferase", preferably it is the molecule of section (a) or (b) of this paragraph.

In one embodiment, said molecule, which activity is to be increased in the process of the invention and which is the gene product with an activity of described as a "nicotinamide-nucleotide adenylyltransferase", is increased non-targeted.

The sequence of SII0290 from Synechocystis sp. PCC 6803, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as polyphosphate kinase.

Accordingly, in one embodiment, the process of the present invention comprises increasing or generating the activity of a gene product with the activity of a "polyphosphate kinase" from Synechocystis sp. PCC 6803 or its functional equivalent or its homolog, e.g. the increase of
(a) a gene product of a gene comprising the nucleic acid molecule as shown in column 5 of Table I and being depicted in the same respective line as said SII0290 or a functional equivalent or a homologue thereof as shown depicted in column 7 of Table I, preferably a homologue or functional equivalent as shown depicted in column 7 of Table I B, and being depicted in the same respective line as said SII0290; or
(b) a polypeptide comprising a polypeptide, a consensus sequence or a polypeptide motif as shown depicted in column 5 of Table II, and being depicted in the same respective line as said SII0290 or a functional equivalent or a homologue thereof as depicted in column 7 of Table II or IV, preferably a homologue or functional equivalent as depicted in column 7 of Table II B, and being depicted in the same respective line as said SII0290,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "polyphosphate kinase", preferably it is the molecule of section (a) or (b) of this paragraph. In one embodiment, said molecule, which activity is to be increased in the process of the invention and which is the gene product with an activity of described as a "polyphosphate kinase", is increased non-targeted.

The sequence of YAL049C from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as Yal049c-protein.

Accordingly, in one embodiment, the process of the present invention comprises increasing or generating the activity of a gene product with the activity of a "Yal049c-protein" from Saccharomyces cerevisiae or its functional equivalent or its homolog, e.g. the increase of
(a) a gene product of a gene comprising the nucleic acid molecule as shown in column 5 of Table I and being depicted in the same respective line as said YAL049C or a functional equivalent or a homologue thereof as shown depicted in column 7 of Table I, preferably a homologue or functional equivalent as shown depicted in column 7 of Table I B, and being depicted in the same respective line as said YAL049C; or
(b) a polypeptide comprising a polypeptide, a consensus sequence or a polypeptide motif as shown depicted in column 5 of Table II, and being depicted in the same respective line as said YAL049C or a functional equivalent or a homologue thereof as depicted in column 7 of Table II or IV, preferably a homologue or functional equivalent as depicted in column 7 of Table II B, and being depicted in the same respective line as said YAL049C,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "Yal049c-protein", preferably it is the molecule of section (a) or (b) of this paragraph.

In one embodiment, said molecule, which activity is to be increased in the process of the invention and which is the gene product with an activity of described as a "Yal049c-protein", is increased non-targeted.

The sequence of YCR059C from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as YCR059C-protein.

Accordingly, in one embodiment, the process of the present invention comprises increasing or generating the activity of a gene product with the activity of a "YCR059C-protein" from Saccharomyces cerevisiae or its functional equivalent or its homolog, e.g. the increase of
(a) a gene product of a gene comprising the nucleic acid molecule as shown in column 5 of Table I and being depicted in the same respective line as said YCR059C or a functional equivalent or a homologue thereof as shown depicted in column 7 of Table I, preferably a homologue or functional equivalent as shown depicted in column 7 of Table I B, and being depicted in the same respective line as said YCR059C; or
(b) a polypeptide comprising a polypeptide, a consensus sequence or a polypeptide motif as shown depicted in column 5 of Table II, and being depicted in the same respective line as said YCR059C or a functional equivalent or a homologue thereof as depicted in column 7 of Table II or IV, preferably a homologue or functional equivalent as depicted in column 7 of Table II B, and being depicted in the same respective line as said YCR059C,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "YCR059C-protein", preferably it is the molecule of section (a) or (b) of this paragraph.

In one embodiment, said molecule, which activity is to be increased in the process of the invention and which is the gene product with an activity of described as a "YCR059C-protein", is increased non-targeted.

The sequence of YDR035W from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as 3-deoxy-D-arabinoheptulosonate-7-phosphate (DAHP) synthase.

Accordingly, in one embodiment, the process of the present invention comprises increasing or generating the activity of a gene product with the activity of a "3-deoxy-Darabino-heptulosonate-7-phosphate (DAHP) synthase" from Saccharomyces cerevisiae or its functional equivalent or its homolog, e.g. the increase of
(a) a gene product of a gene comprising the nucleic acid molecule as shown in column 5 of Table I and being depicted in the same respective line as said YDR035W or a functional equivalent or a homologue thereof as shown depicted in column 7 of Table I, preferably a homologue or functional equivalent as shown depicted in column 7 of Table I B, and being depicted in the same respective line as said YDR035W; or
(b) a polypeptide comprising a polypeptide, a consensus sequence or a polypeptide motif as shown depicted in column 5 of Table II, and being depicted in the same respective line as said YDR035W or a functional equivalent or a homologue thereof as depicted in column 7 of Table II or IV, preferably a homologue or functional equivalent as depicted in column 7 of Table II B, and being depicted in the same respective line as said YDR035W,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "3-deoxyD-arabino-heptulosonate-7-phosphate (DAHP) synthase", preferably it is the molecule of section (a) or (b) of this paragraph.

In one embodiment, said molecule, which activity is to be increased in the process of the invention and which is the gene product with an activity of described as a "3-deoxyD-arabino-heptulosonate-7-phosphate (DAHP) synthase", is increased plastidic.

The sequence of YEL005C from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as YEL005C-protein.

Accordingly, in one embodiment, the process of the present invention comprises increasing or generating the activity of a gene product with the activity of a "YEL005C-protein" from Saccharomyces cerevisiae or its functional equivalent or its homolog, e.g. the increase of
(a) a gene product of a gene comprising the nucleic acid molecule as shown in column 5 of Table I and being depicted in the same respective line as said YEL005C or a functional equivalent or a homologue thereof as shown depicted in column 7 of Table I, preferably a homologue or functional equivalent as shown depicted in column 7 of Table I B, and being depicted in the same respective line as said YEL005C; or
(b) a polypeptide comprising a polypeptide, a consensus sequence or a polypeptide motif as shown depicted in column 5 of Table II, and being depicted in the same respective line as said YEL005C or a functional equivalent or a homologue thereof as depicted in column 7 of Table II or IV, preferably a homologue or functional equivalent as depicted in column 7 of Table II B, and being depicted in the same respective line as said YEL005C,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "YEL005C-protein", preferably it is the molecule of section (a) or (b) of this paragraph.

In one embodiment, said molecule, which activity is to be increased in the process of the invention and which is the gene product with an activity of described as a "YEL005C-protein", is increased non-targeted.

The sequence of YER112W from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as Lsm (Like Sm) protein.

Accordingly, in one embodiment, the process of the present invention comprises increasing or generating the activity of a gene product with the activity of a "Lsm (Like Sm) protein" from Saccharomyces cerevisiae or its functional equivalent or its homolog, e.g. the increase of
(a) a gene product of a gene comprising the nucleic acid molecule as shown in column 5 of Table I and being depicted in the same respective line as said YER112W or a functional equivalent or a homologue thereof as shown depicted in column 7 of Table I, preferably a homologue or functional equivalent as shown depicted in column 7 of Table I B, and being depicted in the same respective line as said YER112W; or
(b) a polypeptide comprising a polypeptide, a consensus sequence or a polypeptide motif as shown depicted in column 5 of Table II, and being depicted in the same respective line as said YER112W or a functional equivalent or a homologue thereof as depicted in column 7 of Table II or IV, preferably a homologue or functional equivalent as depicted in column 7 of Table II B, and being depicted in the same respective line as said YER112W,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "Lsm (Like Sm) protein", preferably it is the molecule of section (a) or (b) of this paragraph.

In one embodiment, said molecule, which activity is to be increased in the process of the invention and which is the gene product with an activity of described as a "Lsm (Like Sm) protein", is increased non-targeted.

The sequence of YER156C from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as YER156C-protein.

Accordingly, in one embodiment, the process of the present invention comprises increasing or generating the activity of a gene product with the activity of a "YER156C-protein" from Saccharomyces cerevisiae or its functional equivalent or its homolog, e.g. the increase of
(a) a gene product of a gene comprising the nucleic acid molecule as shown in column 5 of Table I and being depicted in the same respective line as said YER156C or a functional equivalent or a homologue thereof as shown depicted in column 7 of Table I, preferably a homologue or functional equivalent as shown depicted in column 7 of Table I B, and being depicted in the same respective line as said YER156C; or
(b) a polypeptide comprising a polypeptide, a consensus sequence or a polypeptide motif as shown depicted in column 5 of Table II, and being depicted in the same respective line as said YER156C or a functional equivalent or a homologue thereof as depicted in column 7 of Table II or IV, preferably a homologue or functional equivalent as depicted in column 7 of Table II B, and being depicted in the same respective line as said YER156C,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "YER156C-protein", preferably it is the molecule of section (a) or (b) of this paragraph. In one embodiment, said molecule, which activity is to be increased in the process of the invention and which is the gene product with an activity of described as a "YER156C-protein", is increased non-targeted.

The sequence of YER173W from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as Checkpoint protein.

Accordingly, in one embodiment, the process of the present invention comprises increasing or generating the activity of a gene product with the activity of a "Checkpoint protein" from Saccharomyces cerevisiae or its functional equivalent or its homolog, e.g. the increase of
(a) a gene product of a gene comprising the nucleic acid molecule as shown in column 5 of Table I and being depicted in the same respective line as said YER173W or a functional equivalent or a homologue thereof as shown depicted in column 7 of Table I, preferably a homologue or functional equivalent as shown depicted in column 7 of Table I B, and being depicted in the same respective line as said YER173W; or
(b) a polypeptide comprising a polypeptide, a consensus sequence or a polypeptide motif as shown depicted in column 5 of Table II, and being depicted in the same respective line as said YER173W or a functional equivalent or a homologue thereof as depicted in column 7 of Table II or IV, preferably a homologue or functional equivalent as depicted in column 7 of Table II B, and being depicted in the same respective line as said YER173W,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "Checkpoint protein", preferably it is the molecule of section (a) or (b) of this paragraph.

In one embodiment, said molecule, which activity is to be increased in the process of the invention and which is the gene product with an activity of described as a "Checkpoint protein", is increased non-targeted.

The sequence of YGL045W from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as YGL045W-protein.

Accordingly, in one embodiment, the process of the present invention comprises increasing or generating the activity of a gene product with the activity of a "YGL045W-protein" from Saccharomyces cerevisiae or its functional equivalent or its homolog, e.g. the increase of
(a) a gene product of a gene comprising the nucleic acid molecule as shown in column 5 of Table I and being depicted in the same respective line as said YGL045W or a functional equivalent or a homologue thereof as shown depicted in column 7 of Table I, preferably a homologue or functional equivalent as shown depicted in column 7 of Table I B, and being depicted in the same respective line as said YGL045W; or
(b) a polypeptide comprising a polypeptide, a consensus sequence or a polypeptide motif as shown depicted in column 5 of Table II, and being depicted in the same respective line as said YGL045W or a functional equivalent or a homologue thereof as depicted in column 7 of Table I or IV, preferably a homologue or functional equivalent as depicted in column 7 of Table II B, and being depicted in the same respective line as said YGL045W,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "YGL045W-protein", preferably it is the molecule of section (a) or (b) of this paragraph.

In one embodiment, said molecule, which activity is to be increased in the process of the invention and which is the gene product with an activity of described as a "YGL045W-protein", is increased non-targeted.

The sequence of YGL189C from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as Protein component of the small (40S) ribosomal subunit.

Accordingly, in one embodiment, the process of the present invention comprises increasing or generating the activity of a gene product with the activity of a "Protein component of the small (40S) ribosomal subunit" from Saccharomyces cerevisiae or its functional equivalent or its homolog, e.g. the increase of
(a) a gene product of a gene comprising the nucleic acid molecule as shown in column 5 of Table I and being depicted in the same respective line as said YGL189C or a functional equivalent or a homologue thereof as shown depicted in column 7 of Table I, preferably a homologue or functional equivalent as shown depicted in column 7 of Table I B, and being depicted in the same respective line as said YGL189C; or
(b) a polypeptide comprising a polypeptide, a consensus sequence or a polypeptide motif as shown depicted in column 5 of Table II, and being depicted in the same respective line as said YGL189C or a functional equivalent or a homologue thereof as depicted in column 7 of Table II or IV, preferably a homologue or functional equivalent as depicted in column 7 of Table II B, and being depicted in the same respective line as said YGL189C,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "Protein component of the small (40S) ribosomal subunit", preferably it is the molecule of section (a) or (b) of this paragraph.

In one embodiment, said molecule, which activity is to be increased in the process of the invention and which is the gene product with an activity of described as a "Protein component of the small (40S) ribosomal subunit", is increased non-targeted.

The sequence of YNR015W from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as Dihydrouridine synthase.

Accordingly, in one embodiment, the process of the present invention comprises increasing or generating the activity of a gene product with the activity of a "Dihydrouridine synthase" from Saccharomyces cerevisiae or its functional equivalent or its homolog, e.g. the increase of
(a) a gene product of a gene comprising the nucleic acid molecule as shown in column 5 of Table I and being depicted in the same respective line as said YNR015W or a functional equivalent or a homologue thereof as shown depicted in column 7 of Table I, preferably a homologue or functional equivalent as shown depicted in column 7 of Table I B, and being depicted in the same respective line as said YNR015W; or
(b) a polypeptide comprising a polypeptide, a consensus sequence or a polypeptide motif as shown depicted in column 5 of Table II, and being depicted in the same respective line as said YNR015W or a functional equivalent or a homologue thereof as depicted in column 7 of Table II or IV, preferably a homologue or functional equivalent as depicted in column 7 of Table II B, and being depicted in the same respective line as said YNR015W,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "Dihydrouridine synthase", preferably it is the molecule of section (a) or (b) of this paragraph.

In one embodiment, said molecule, which activity is to be increased in the process of the invention and which is the gene product with an activity of described as a "Dihydrouridine synthase", is increased non-targeted.

The sequence of YOR024W from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as YOR024w-protein.

Accordingly, in one embodiment, the process of the present invention comprises increasing or generating the activity of a gene product with the activity of a "YOR024w-protein" from Saccharomyces cerevisiae or its functional equivalent or its homolog, e.g. the increase of
(a) a gene product of a gene comprising the nucleic acid molecule as shown in column 5 of Table I and being depicted in the same respective line as said YOR024W or a functional equivalent or a homologue thereof as shown depicted in column 7 of Table I, preferably a homologue or functional equivalent as shown depicted in column 7 of Table I B, and being depicted in the same respective line as said YOR024W; or
(b) a polypeptide comprising a polypeptide, a consensus sequence or a polypeptide motif as shown depicted in column 5 of Table II, and being depicted in the same respective line as said YOR024W or a functional equivalent or a homologue thereof as depicted in column 7 of Table II or IV, preferably a homologue or functional equivalent as depicted in column 7 of Table II B, and being depicted in the same respective line as said YOR024W,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "YOR024w-protein", preferably it is the molecule of section (a) or (b) of this paragraph.

In one embodiment, said molecule, which activity is to be increased in the process of the invention and which is the gene product with an activity of described as a "YOR024w-protein", is increased non-targeted.

The sequence of YOR168W from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as Glutamine tRNA synthetase.

Accordingly, in one embodiment, the process of the present invention comprises increasing or generating the activity of a gene product with the activity of a "Glutamine tRNA synthetase" from Saccharomyces cerevisiae or its functional equivalent or its homolog, e.g. the increase of
(a) a gene product of a gene comprising the nucleic acid molecule as shown in column 5 of Table I and being depicted in the same respective line as said YOR168W or a functional equivalent or a homologue thereof as shown depicted in column 7 of Table I, preferably a homologue or functional equivalent as shown depicted in column 7 of Table I B, and being depicted in the same respective line as said YOR168W; or
(b) a polypeptide comprising a polypeptide, a consensus sequence or a polypeptide motif as shown depicted in column 5 of Table II, and being depicted in the same respective line as said YOR168W or a functional equivalent or a homologue thereof as depicted in column 7 of Table II or IV, preferably a homologue or functional equivalent as depicted in column 7 of Table II B, and being depicted in the same respective line as said YOR168W,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "Glutamine tRNA synthetase", preferably it is the molecule of section (a) or (b) of this paragraph.

In one embodiment, said molecule, which activity is to be increased in the process of the invention and which is the gene product with an activity of described as a "Glutamine tRNA synthetase", is increased non-targeted.

The sequence of YPL151C from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as Splicing factor.

Accordingly, in one embodiment, the process of the present invention comprises increasing or generating the activity of a gene product with the activity of a "Splicing factor" from Saccharomyces cerevisiae or its functional equivalent or its homolog, e.g. the increase of
(a) a gene product of a gene comprising the nucleic acid molecule as shown in column 5 of Table I and being depicted in the same respective line as said YPL151C or a functional equivalent or a homologue thereof as shown depicted in column 7 of Table I, preferably a homologue or functional equivalent as shown depicted in column 7 of Table I B, and being depicted in the same respective line as said YPL151C; or
(b) a polypeptide comprising a polypeptide, a consensus sequence or a polypeptide motif as shown depicted in column 5 of Table II, and being depicted in the same respective line as said YPL151C or a functional equivalent or a homologue thereof as depicted in column 7 of Table II or IV, preferably a homologue or functional equivalent as depicted in column 7 of Table II B, and being depicted in the same respective line as said YPL151C,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "Splicing factor", preferably it is the molecule of section (a) or (b) of this paragraph.

In one embodiment, said molecule, which activity is to be increased in the process of the invention and which is the gene product with an activity of described as a "Splicing factor", is increased non-targeted.

The sequence of B1297 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as gamma-Glu-putrescine synthase.

Accordingly, in one embodiment, the process of the present invention comprises increasing or generating the activity of a gene product with the activity of a "gamma-Glu-putrescine synthase" from Escherichia coli K12 or its functional equivalent or its homolog, e.g. the increase of
(a) a gene product of a gene comprising the nucleic acid molecule as shown in column 5 of Table I and being depicted in the same respective line as said B1297 or a functional equivalent or a homologue thereof as shown depicted in column 7 of Table I, preferably a homologue or functional equivalent as shown depicted in column 7 of Table I B, and being depicted in the same respective line as said B1297; or
(b) a polypeptide comprising a polypeptide, a consensus sequence or a polypeptide motif as shown depicted in column 5 of Table II, and being depicted in the same respective line as said B1297 or a functional equivalent or a homologue thereof as depicted in column 7 of Table II or IV, preferably a homologue or functional equivalent as depicted in column 7 of Table II B, and being depicted in the same respective line as said B1297,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "gamma-Glu-putrescine synthase", preferably it is the molecule of section (a) or (b) of this paragraph.

In one embodiment, said molecule, which activity is to be increased in the process of the invention and which is the gene product with an activity of described as a "gamma-Glu-putrescine synthase", is increased plastidic.

The sequence of B0970 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as inner membrane protein.

Accordingly, in one embodiment, the process of the present invention comprises increasing or generating the activity of a gene product with the activity of a "inner membrane protein" from Escherichia coli K12 or its functional equivalent or its homolog, e.g. the increase of
(a) a gene product of a gene comprising the nucleic acid molecule as shown in column 5 of Table I and being depicted in the same respective line as said B0970 or a functional equivalent or a homologue thereof as shown depicted in column 7 of Table I, preferably a homologue or functional equivalent as shown depicted in column 7 of Table I B, and being depicted in the same respective line as said B0970; or
(b) a polypeptide comprising a polypeptide, a consensus sequence or a polypeptide motif as shown depicted in column 5 of Table II, and being depicted in the same respective line as said B0970 or a functional equivalent or a homologue thereof as depicted in column 7 of Table II or IV, preferably a homologue or functional equivalent as depicted in column 7 of Table II B, and being depicted in the same respective line as said B0970,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "inner membrane protein", preferably it is the molecule of section (a) or (b) of this paragraph.

In one embodiment, said molecule, which activity is to be increased in the process of the invention and which is the gene product with an activity of described as a "inner membrane protein", is increased non-targeted.

The sequence of B1829 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as heat shock protein HtpX.

Accordingly, in one embodiment, the process of the present invention comprises increasing or generating the activity of a gene product with the activity of a "heat shock protein HtpX" from Escherichia coli K12 or its functional equivalent or its homolog, e.g. the increase of
(a) a gene product of a gene comprising the nucleic acid molecule as shown in column 5 of Table I and being depicted in the same respective line as said B1829 or a functional equivalent or a homologue thereof as shown depicted in column 7 of Table I, preferably a homologue or functional equivalent as shown depicted in column 7 of Table I B, and being depicted in the same respective line as said B1829; or
(b) a polypeptide comprising a polypeptide, a consensus sequence or a polypeptide motif as shown depicted in column 5 of Table II, and being depicted in the same respective line as said B1829 or a functional equivalent or a homologue thereof as depicted in column 7 of Table II or IV, preferably a homologue or functional equivalent as depicted in column 7 of Table II B, and being depicted in the same respective line as said B1829,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "heat shock protein HtpX", preferably it is the molecule of section (a) or (b) of this paragraph.

In one embodiment, said molecule, which activity is to be increased in the process of the invention and which is the gene product with an activity of described as a "heat shock protein HtpX", is increased non-targeted.

The sequence of B2664 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as DNA-binding transcriptional dual regulator protein.

Accordingly, in one embodiment, the process of the present invention comprises increasing or generating the activity of a gene product with the activity of a "DNA-binding transcriptional dual regulator protein" from Escherichia coli K12 or its functional equivalent or its homolog, e.g. the increase of
(a) a gene product of a gene comprising the nucleic acid molecule as shown in column 5 of Table I and being depicted in the same respective line as said B2664 or a functional equivalent or a homologue thereof as shown depicted in column 7 of Table I, preferably a homologue or functional equivalent as shown depicted in column 7 of Table I B, and being depicted in the same respective line as said B2664; or
(b) a polypeptide comprising a polypeptide, a consensus sequence or a polypeptide motif as shown depicted in column 5 of Table II, and being depicted in the same respective line as said B2664 or a functional equivalent or a homologue thereof as depicted in column 7 of Table II or IV, preferably a homologue or functional equivalent as depicted in column 7 of Table II B, and being depicted in the same respective line as said B2664,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "DNA-binding transcriptional dual regulator protein", preferably it is the molecule of section (a) or (b) of this paragraph.

In one embodiment, said molecule, which activity is to be increased in the process of the invention and which is the gene product with an activity of described as a "DNA-binding transcriptional dual regulator protein", is increased non-targeted.

The sequence of B2796 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted serine transporter protein.

Accordingly, in one embodiment, the process of the present invention comprises increasing or generating the activity of a gene product with the activity of a "predicted serine transporter protein" from Escherichia coli K12 or its functional equivalent or its homolog, e.g. the increase of
(a) a gene product of a gene comprising the nucleic acid molecule as shown in column 5 of Table I and being depicted in the same respective line as said B2796 or a functional equivalent or a homologue thereof as shown depicted in column 7 of Table I, preferably a homologue or functional equivalent as shown depicted in column 7 of Table I B, and being depicted in the same respective line as said B2796; or
(b) a polypeptide comprising a polypeptide, a consensus sequence or a polypeptide motif as shown depicted in column 5 of Table II, and being depicted in the same respective line as said B2796 or a functional equivalent or a homologue thereof as depicted in column 7 of Table II or IV, preferably a homologue or functional equivalent as depicted in column 7 of Table II B, and being depicted in the same respective line as said B2796,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted serine transporter protein", preferably it is the molecule of section (a) or (b) of this paragraph.

In one embodiment, said molecule, which activity is to be increased in the process of the invention and which is the gene product with an activity of described as a "predicted serine transporter protein", is increased non-targeted.

The sequence of YER174C from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as glutathione-dependent oxidoreductase.

Accordingly, in one embodiment, the process of the present invention comprises increasing or generating the activity of a gene product with the activity of a "glutathione-dependent oxidoreductase" from Saccharomyces cerevisiae or its functional equivalent or its homolog, e.g. the increase of
(a) a gene product of a gene comprising the nucleic acid molecule as shown in column 5 of Table I and being depicted in the same respective line as said YER174C or a functional equivalent or a homologue thereof as shown depicted in column 7 of Table I, preferably a homologue or functional equivalent as shown depicted in column 7 of Table I B, and being depicted in the same respective line as said YER174C; or
(b) a polypeptide comprising a polypeptide, a consensus sequence or a polypeptide motif as shown depicted in column 5 of Table II, and being depicted in the same respective line as said YER174C or a functional equivalent or a homologue thereof as depicted in column 7 of Table II or IV, preferably a homologue or functional equivalent as depicted in column 7 of Table II B, and being depicted in the same respective line as said YER174C,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "glutathione-dependent oxidoreductase", preferably it is the molecule of section (a) or (b) of this paragraph.

In one embodiment, said molecule, which activity is to be increased in the process of the invention and which is the gene product with an activity of described as a "glutathione-dependent oxidoreductase", is increased non-targeted.

The sequence of YFR042W from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as Yfr042w-protein.

Accordingly, in one embodiment, the process of the present invention comprises increasing or generating the activity of a gene product with the activity of a "Yfr042w-protein" from Saccharomyces cerevisiae or its functional equivalent or its homolog, e.g. the increase of
(a) a gene product of a gene comprising the nucleic acid molecule as shown in column 5 of Table I and being depicted in the same respective line as said YFR042W or a functional equivalent or a homologue thereof as shown depicted in column 7 of Table I, preferably a homologue or functional equivalent as shown depicted in column 7 of Table I B, and being depicted in the same respective line as said YFR042W; or
(b) a polypeptide comprising a polypeptide, a consensus sequence or a polypeptide motif as shown depicted in column 5 of Table II, and being depicted in the same respective line as said YFR042W or a functional equivalent or a homologue thereof as depicted in column 7 of Table II or IV, preferably a homologue or functional equivalent as depicted in column 7 of Table II B, and being depicted in the same respective line as said YFR042W,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "Yfr042w-protein", preferably it is the molecule of section (a) or (b) of this paragraph.

In one embodiment, said molecule, which activity is to be increased in the process of the invention and which is the gene product with an activity of described as a "Yfr042w-protein", is increased non-targeted.

The sequence of YKR057W from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as Protein component of the small (40S) ribosomal subunit.

Accordingly, in one embodiment, the process of the present invention comprises increasing or generating the activity of a gene product with the activity of a "Protein component of the small (40S) ribosomal subunit" from Saccharomyces cerevisiae or its functional equivalent or its homolog, e.g. the increase of
(a) a gene product of a gene comprising the nucleic acid molecule as shown in column 5 of Table I and being depicted in the same respective line as said YKR057W or a functional equivalent or a homologue thereof as shown depicted in column 7 of Table I, preferably a homologue or functional equivalent as shown depicted in column 7 of Table I B, and being depicted in the same respective line as said YKR057W; or
(b) a polypeptide comprising a polypeptide, a consensus sequence or a polypeptide motif as shown depicted in column 5 of Table II, and being depicted in the same respective line as said YKR057W or a functional equivalent or a homologue thereof as depicted in column 7 of Table II or IV, preferably a homologue or functional equivalent as depicted in column 7 of Table II B, and being depicted in the same respective line as said YKR057W,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "Protein component of the small (40S) ribosomal subunit", preferably it is the molecule of section (a) or (b) of this paragraph.

In one embodiment, said molecule, which activity is to be increased in the process of the invention and which is the gene product with an activity of described as a "Protein component of the small (40S) ribosomal subunit", is increased non-targeted.

The sequence of B0629_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as transcriptional regulator protein.

Accordingly, in one embodiment, the process of the present invention comprises increasing or generating the activity of a gene product with the activity of a "transcriptional regulator protein" from Escherichia coli K12 or its functional equivalent or its homolog, e.g. the increase of
(a) a gene product of a gene comprising the nucleic acid molecule as shown in column 5 of Table I and being depicted in the same respective line as said B0629_2 or a functional equivalent or a homologue thereof as shown depicted in column 7 of Table I, preferably a homologue or functional equivalent as shown depicted in column 7 of Table I B, and being depicted in the same respective line as said B0629_2; or
(b) a polypeptide comprising a polypeptide, a consensus sequence or a polypeptide motif as shown depicted in column 5 of Table II, and being depicted in the same respective line as said B0629_2 or a functional equivalent or a homologue thereof as depicted in column 7 of Table II or IV, preferably a homologue or functional equivalent as depicted in column 7 of Table II B, and being depicted in the same respective line as said B0629_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "transcriptional regulator protein", preferably it is the molecule of section (a) or (b) of this paragraph.

In one embodiment, said molecule, which activity is to be increased in the process of the invention and which is the gene product with an activity of described as a "transcriptional regulator protein", is increased non-targeted.

The sequence of B1007_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted oxidoreductase (flavin:NADH component).

Accordingly, in one embodiment, the process of the present invention comprises increasing or generating the activity of a gene product with the activity of a "predicted oxidoreductase (flavin:NADH component)" from Escherichia coli K12 or its functional equivalent or its homolog, e.g. the increase of
(a) a gene product of a gene comprising the nucleic acid molecule as shown in column 5 of Table I and being depicted in the same respective line as said B1007_2 or a functional equivalent or a homologue thereof as shown depicted in column 7 of Table I, preferably a homologue or functional equivalent as shown depicted in column 7 of Table I B, and being depicted in the same respective line as said B1007_2; or
(b) a polypeptide comprising a polypeptide, a consensus sequence or a polypeptide motif as shown depicted in column 5 of Table II, and being depicted in the same respective line as said B1007_2 or a functional equivalent or a homologue thereof as depicted in column 7 of Table II or IV, preferably a homologue or functional equivalent as depicted in column 7 of Table II B, and being depicted in the same respective line as said B1007_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted oxidoreductase (flavin:NADH component)", preferably it is the molecule of section (a) or (b) of this paragraph.

In one embodiment, said molecule, which activity is to be increased in the process of the invention and which is the gene product with an activity of described as a "predicted oxidoreductase (flavin:NADH component)", is increased non-targeted.

The sequence of B2715_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as cellobiose/arbutin/salicin-specific PTS enzyme (IIB component/IC component).

Accordingly, in one embodiment, the process of the present invention comprises increasing or generating the activity of a gene product with the activity of a "cellobiose/arbutin/salicin-specific PTS enzyme (IIB component/IC component)" from Escherichia coli K12 or its functional equivalent or its homolog, e.g. the increase of
(a) a gene product of a gene comprising the nucleic acid molecule as shown in column 5 of Table I and being depicted in the same respective line as said B2715_2 or a functional equivalent or a homologue thereof as shown depicted in column 7 of Table I, preferably a homologue or functional equivalent as shown depicted in column 7 of Table I B, and being depicted in the same respective line as said B2715_2; or
(b) a polypeptide comprising a polypeptide, a consensus sequence or a polypeptide motif as shown depicted in column 5 of Table II, and being depicted in the same respective line as said B2715_2 or a functional equivalent or a homologue thereof as depicted in column 7 of Table II or IV, preferably a homologue or functional equivalent as depicted in column 7 of Table II B, and being depicted in the same respective line as said B2715_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "cellobiose/arbutin/salicin-specific PTS enzyme (IIB component/IC component)", preferably it is the molecule of section (a) or (b) of this paragraph.

In one embodiment, said molecule, which activity is to be increased in the process of the invention and which is the gene product with an activity of described as a "cellobiose/arbutin/salicin-specific PTS enzyme (IIB component/IC component)", is increased plastidic.

The sequence of B3899_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted PTS enzymes (IIB component/IIC component).

Accordingly, in one embodiment, the process of the present invention comprises increasing or generating the activity of a gene product with the activity of a "predicted PTS enzymes (IIB component/IIC component)" from Escherichia coli K12 or its functional equivalent or its homolog, e.g. the increase of
(a) a gene product of a gene comprising the nucleic acid molecule as shown in column 5 of Table I and being depicted in the same respective line as said B3899_2 or a functional equivalent or a homologue thereof as shown depicted in column 7 of Table I, preferably a homologue or functional equivalent as shown depicted in column 7 of Table I B, and being depicted in the same respective line as said B3899_2; or
(b) a polypeptide comprising a polypeptide, a consensus sequence or a polypeptide motif as shown depicted in column 5 of Table II, and being depicted in the same respective line as said B3899_2 or a functional equivalent or a homologue thereof as depicted in column 7 of Table II or IV, preferably a homologue or functional equivalent as depicted in column 7 of Table II B, and being depicted in the same respective line as said B3899_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted PTS enzymes (IIB component/IIC component)", preferably it is the molecule of section (a) or (b) of this paragraph.

In one embodiment, said molecule, which activity is to be increased in the process of the invention and which is the gene product with an activity of described as a "predicted PTS enzymes (IIB component/IIC component)", is increased non-targeted.

The sequence of B4390_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as nicotinamide-nucleotide adenylyltransferase.

Accordingly, in one embodiment, the process of the present invention comprises increasing or generating the activity of a gene product with the activity of a "nicotinamide-nucleotide adenylyltransferase" from Escherichia coli K12 or its functional equivalent or its homolog, e.g. the increase of
(a) a gene product of a gene comprising the nucleic acid molecule as shown in column 5 of Table I and being depicted in the same respective line as said B4390_2 or a functional equivalent or a homologue thereof as shown depicted in column 7 of Table I, preferably a homologue or functional equivalent as shown depicted in column 7 of Table I B, and being depicted in the same respective line as said B4390_2; or
(b) a polypeptide comprising a polypeptide, a consensus sequence or a polypeptide motif as shown depicted in column 5 of Table II, and being depicted in the same respective line as said B4390_2 or a functional equivalent or a homologue thereof as depicted in column 7 of Table II or IV, preferably a homologue or functional equivalent as depicted in column 7 of Table II B, and being depicted in the same respective line as said B4390_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "nicotinamide-nucleotide adenylyltransferase", preferably it is the molecule of section (a) or (b) of this paragraph.

In one embodiment, said molecule, which activity is to be increased in the process of the invention and which is the gene product with an activity of described as a "nicotinamide-nucleotide adenylyltransferase", is increased non-targeted.

The sequence of YGL045W_2 from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as YGL045W-protein. Accordingly, in one embodiment, the process of the present invention comprises increasing or generating the activity of a gene product with the activity of a "YGL045W-protein" from Saccharomyces cerevisiae or its functional equivalent or its homolog, e.g. the increase of
(a) a gene product of a gene comprising the nucleic acid molecule as shown in column 5 of Table I and being depicted in the same respective line as said YGL045W_2 or a functional equivalent or a homologue thereof as shown depicted in column 7 of Table I, preferably a homologue or functional equivalent as shown depicted in column 7 of Table I B, and being depicted in the same respective line as said YGL045W_2; or
(b) a polypeptide comprising a polypeptide, a consensus sequence or a polypeptide motif as shown depicted in column 5 of Table II, and being depicted in the same respective line as said YGL045W_2 or a functional equivalent or a homologue thereof as depicted in column 7 of Table II or IV, preferably a homologue or functional equivalent as depicted in column 7 of Table II B, and being depicted in the same respective line as said YGL045W_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "YGL045W-protein", preferably it is the molecule of section (a) or (b) of this paragraph.

In one embodiment, said molecule, which activity is to be increased in the process of the invention and which is the gene product with an activity of described as a "YGL045W-protein", is increased non-targeted.

The sequence of B2664_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as DNA-binding transcriptional dual regulator protein.

Accordingly, in one embodiment, the process of the present invention comprises increasing or generating the activity of a gene product with the activity of a "DNA-binding transcriptional dual regulator protein" from Escherichia coli K12 or its functional equivalent or its homolog, e.g. the increase of
(a) a gene product of a gene comprising the nucleic acid molecule as shown in column 5 of Table I and being depicted in the same respective line as said B2664_2 or a functional equivalent or a homologue thereof as shown depicted in column 7 of Table I, preferably a homologue or functional equivalent as shown depicted in column 7 of Table I B, and being depicted in the same respective line as said B2664_2; or
(b) a polypeptide comprising a polypeptide, a consensus sequence or a polypeptide motif as shown depicted in column 5 of Table II, and being depicted in the same respective line as said B2664_2 or a functional equivalent or a homologue thereof as depicted in column 7 of Table II or IV, preferably a homologue or functional equivalent as depicted in column 7 of Table II B, and being depicted in the same respective line as said B2664_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "DNA-binding transcriptional dual regulator protein", preferably it is the molecule of section (a) or (b) of this paragraph.

In one embodiment, said molecule, which activity is to be increased in the process of the invention and which is the gene product with an activity of described as a "DNA-binding transcriptional dual regulator protein", is increased non-targeted.

The sequence of B0963_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as methylglyoxal synthase.

Accordingly, in one embodiment, the process of the present invention comprises increasing or generating the activity of a gene product with the activity of a "methylglyoxal synthase" from Escherichia coli K12 or its functional equivalent or its homolog, e.g. the increase of
(a) a gene product of a gene comprising the nucleic acid molecule as shown in column 5 of Table I and being depicted in the same respective line as said B0963_2 or a functional equivalent or a homologue thereof as shown depicted in column 7 of Table I, preferably a homologue or functional equivalent as shown depicted in column 7 of Table I B, and being depicted in the same respective line as said B0963_2; or
(b) a polypeptide comprising a polypeptide, a consensus sequence or a polypeptide motif as shown depicted in column 5 of Table II, and being depicted in the same respective line as said B0963_2 or a functional equivalent or a homologue thereof as depicted in column 7 of Table II or IV, preferably a homologue or functional equivalent as depicted in column 7 of Table II B, and being depicted in the same respective line as said B0963_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "methylglyoxal synthase", preferably it is the molecule of section (a) or (b) of this paragraph.

In one embodiment, said molecule, which activity is to be increased in the process of the invention and which is the gene product with an activity of described as a "methylglyoxal synthase", is increased non-targeted.

The sequence of B1297_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as gamma-Glu-putrescine synthase.

Accordingly, in one embodiment, the process of the present invention comprises increasing or generating the activity of a gene product with the activity of a "gamma-Glu-putrescine synthase" from Escherichia coli K12 or its functional equivalent or its homolog, e.g. the increase of
(a) a gene product of a gene comprising the nucleic acid molecule as shown in column 5 of Table I and being depicted in the same respective line as said B1297_2 or a functional equivalent or a homologue thereof as shown depicted in column 7 of Table I, preferably a homologue or functional equivalent as shown depicted in column 7 of Table I B, and being depicted in the same respective line as said B1297_2; or
(b) a polypeptide comprising a polypeptide, a consensus sequence or a polypeptide motif as shown depicted in column 5 of Table II, and being depicted in the same respective line as said B1297_2 or a functional equivalent or a homologue thereof as depicted in column 7 of Table II or IV, preferably a homologue or functional equivalent as depicted in column 7 of Table II B, and being depicted in the same respective line as said B1297_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "gamma-Glu-putrescine synthase", preferably it is the molecule of section (a) or (b) of this paragraph.

In one embodiment, said molecule, which activity is to be increased in the process of the invention and which is the gene product with an activity of described as a "gamma-Glu-putrescine synthase", is increased plastidic.

The sequence of B1597_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as acid shock protein precursor.

Accordingly, in one embodiment, the process of the present invention comprises increasing or generating the activity of a gene product with the activity of a "acid shock protein precursor" from Escherichia coli K12 or its functional equivalent or its homolog, e.g. the increase of
(a) a gene product of a gene comprising the nucleic acid molecule as shown in column 5 of Table I and being depicted in the same respective line as said B1597_2 or a functional equivalent or a homologue thereof as shown depicted in column 7 of Table I, preferably a homologue or functional equivalent as shown depicted in column 7 of Table I B, and being depicted in the same respective line as said B1597_2; or
(b) a polypeptide comprising a polypeptide, a consensus sequence or a polypeptide motif as shown depicted in column 5 of Table II, and being depicted in the same respective line as said B1597_2 or a functional equivalent or a homologue thereof as depicted in column 7 of Table II or IV, preferably a homologue or functional equivalent as depicted in column 7 of Table II B, and being depicted in the same respective line as said B1597_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "acid shock protein precursor", preferably it is the molecule of section (a) or (b) of this paragraph.

In one embodiment, said molecule, which activity is to be increased in the process of the invention and which is the gene product with an activity of described as a "acid shock protein precursor", is increased non-targeted.

The sequence of B2027_2 from Escherichia coli k12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as regulator of length of O-antigen component of lipopolysaccharide chains.

Accordingly, in one embodiment, the process of the present invention comprises increasing or generating the activity of a gene product with the activity of a "regulator of length of O-antigen component of lipopolysaccharide chains " from Escherichia coli k12 or its functional equivalent or its homolog, e.g. the increase of
(a) a gene product of a gene comprising the nucleic acid molecule as shown in column 5 of Table I and being depicted in the same respective line as said B2027_2 or a functional equivalent or a homologue thereof as shown depicted in column 7 of Table I, preferably a homologue or functional equivalent as shown depicted in column 7 of Table I B, and being depicted in the same respective line as said B2027_2; or
(b) a polypeptide comprising a polypeptide, a consensus sequence or a polypeptide motif as shown depicted in column 5 of Table II, and being depicted in the same respective line as said B2027_2 or a functional equivalent or a homologue thereof as depicted in column 7 of Table II or IV, preferably a homologue or functional equivalent as depicted in column 7 of Table II B, and being depicted in the same respective line as said B2027_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "regulator of length of O-antigen component of lipopolysaccharide chains ", preferably it is the molecule of section (a) or (b) of this paragraph.

In one embodiment, said molecule, which activity is to be increased in the process of the invention and which is the gene product with an activity of described as a "regulator of length of O-antigen component of lipopolysaccharide chains ", is increased non-targeted.

The sequence of B2965_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as ornithine decarboxylase.

Accordingly, in one embodiment, the process of the present invention comprises increasing or generating the activity of a gene product with the activity of a "ornithine decarboxylase" from Escherichia coli K12 or its functional equivalent or its homolog, e.g. the increase of
(a) a gene product of a gene comprising the nucleic acid molecule as shown in column 5 of Table I and being depicted in the same respective line as said B2965_2 or a functional equivalent or a homologue thereof as shown depicted in column 7 of Table I, preferably a homologue or functional equivalent as shown depicted in column 7 of Table I B, and being depicted in the same respective line as said B2965_2; or
(b) a polypeptide comprising a polypeptide, a consensus sequence or a polypeptide motif as shown depicted in column 5 of Table II, and being depicted in the same respective line as said B2965_2 or a functional equivalent or a homologue thereof as depicted in column 7 of Table II or IV, preferably a homologue or functional equivalent as depicted in column 7 of Table II B, and being depicted in the same respective line as said B2965_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "ornithine decarboxylase", preferably it is the molecule of section (a) or (b) of this paragraph.

In one embodiment, said molecule, which activity is to be increased in the process of the invention and which is the gene product with an activity of described as a "ornithine decarboxylase", is increased plastidic.

The sequence of B4139_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as aspartate ammonia-lyase.

Accordingly, in one embodiment, the process of the present invention comprises increasing or generating the activity of a gene product with the activity of a "aspartate ammonia-lyase " from Escherichia coli K12 or its functional equivalent or its homolog, e.g. the increase of
(a) a gene product of a gene comprising the nucleic acid molecule as shown in column 5 of Table I and being depicted in the same respective line as said B4139_2 or a functional equivalent or a homologue thereof as shown depicted in column 7 of Table I, preferably a homologue or functional equivalent as shown depicted in column 7 of Table I B, and being depicted in the same respective line as said B4139_2; or
(b) a polypeptide comprising a polypeptide, a consensus sequence or a polypeptide motif as shown depicted in column 5 of Table II, and being depicted in the same respective line as said B4139_2 or a functional equivalent or a homologue thereof as depicted in column 7 of Table II or IV, preferably a homologue or functional equivalent as depicted in column 7 of Table II B, and being depicted in the same respective line as said B4139_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "aspartate ammonia-lyase ", preferably it is the molecule of section (a) or (b) of this paragraph.

In one embodiment, said molecule, which activity is to be increased in the process of the invention and which is the gene product with an activity of described as a "aspartate ammonia-lyase ", is increased plastidic.

The sequence of B0845_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as predicted transporter protein.

Accordingly, in one embodiment, the process of the present invention comprises increasing or generating the activity of a gene product with the activity of a "predicted transporter protein" from Escherichia coli K12 or its functional equivalent or its homolog, e.g. the increase of
(a) a gene product of a gene comprising the nucleic acid molecule as shown in column 5 of Table I and being depicted in the same respective line as said B0845_2 or a functional equivalent or a homologue thereof as shown depicted in column 7 of Table I, preferably a homologue or functional equivalent as shown depicted in column 7 of Table I B, and being depicted in the same respective line as said B0845_2; or
(b) a polypeptide comprising a polypeptide, a consensus sequence or a polypeptide motif as shown depicted in column 5 of Table II, and being depicted in the same respective line as said B0845_2 or a functional equivalent or a homologue thereof as depicted in column 7 of Table II or IV, preferably a homologue or functional equivalent as depicted in column 7 of Table II B, and being depicted in the same respective line as said B0845_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "predicted transporter protein", preferably it is the molecule of section (a) or (b) of this paragraph.

In one embodiment, said molecule, which activity is to be increased in the process of the invention and which is the gene product with an activity of described as a "predicted transporter protein", is increased non-targeted.

The sequence of B1901_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as L-arabinose transporter subunit.

Accordingly, in one embodiment, the process of the present invention comprises increasing or generating the activity of a gene product with the activity of a "L-arabinose transporter subunit" from Escherichia coli K12 or its functional equivalent or its homolog, e.g. the increase of
(a) a gene product of a gene comprising the nucleic acid molecule as shown in column 5 of Table I and being depicted in the same respective line as said B1901_2 or a functional equivalent or a homologue thereof as shown depicted in column 7 of Table I, preferably a homologue or functional equivalent as shown depicted in column 7 of Table I B, and being depicted in the same respective line as said B1901_2; or
(b) a polypeptide comprising a polypeptide, a consensus sequence or a polypeptide motif as shown depicted in column 5 of Table II, and being depicted in the same respective line as said B1901_2 or a functional equivalent or a homologue thereof as depicted in column 7 of Table II or IV, preferably a homologue or functional equivalent as depicted in column 7 of Table II B, and being depicted in the same respective line as said B1901_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "L-arabinose transporter subunit", preferably it is the molecule of section (a) or (b) of this paragraph.

In one embodiment, said molecule, which activity is to be increased in the process of the invention and which is the gene product with an activity of described as a "L-arabinose transporter subunit", is increased plastidic.

The sequence of YER112W_2 from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as Lsm (Like Sm) protein.

Accordingly, in one embodiment, the process of the present invention comprises increasing or generating the activity of a gene product with the activity of a "Lsm (Like Sm) protein" from Saccharomyces cerevisiae or its functional equivalent or its homolog, e.g. the increase of
(a) a gene product of a gene comprising the nucleic acid molecule as shown in column 5 of Table I and being depicted in the same respective line as said YER112W_2 or a functional equivalent or a homologue thereof as shown depicted in column 7 of Table I, preferably a homologue or functional equivalent as shown depicted in column 7 of Table I B, and being depicted in the same respective line as said YER112W_2; or
(b) a polypeptide comprising a polypeptide, a consensus sequence or a polypeptide motif as shown depicted in column 5 of Table II, and being depicted in the same respective line as said YER112W_2 or a functional equivalent or a homologue thereof as depicted in column 7 of Table II or IV, preferably a homologue or functional equivalent as depicted in column 7 of Table II B, and being depicted in the same respective line as said YER112W_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "Lsm (Like Sm) protein", preferably it is the molecule of section (a) or (b) of this paragraph. In one embodiment, said molecule, which activity is to be increased in the process of the invention and which is the gene product with an activity of described as a "Lsm (Like Sm) protein", is increased non-targeted.

The sequence of B1798_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as neutral amino-acid efflux system.

Accordingly, in one embodiment, the process of the present invention comprises increasing or generating the activity of a gene product with the activity of a "neutral amino-acid efflux system" from Escherichia coli K12 or its functional equivalent or its homolog, e.g. the increase of
(a) a gene product of a gene comprising the nucleic acid molecule as shown in column 5 of Table I and being depicted in the same respective line as said B1798_2 or a functional equivalent or a homologue thereof as shown depicted in column 7 of Table I, preferably a homologue or functional equivalent as shown depicted in column 7 of Table I B, and being depicted in the same respective line as said B1798_2; or
(b) a polypeptide comprising a polypeptide, a consensus sequence or a polypeptide motif as shown depicted in column 5 of Table II, and being depicted in the same respective line as said B1798_2 or a functional equivalent or a homologue thereof as depicted in column 7 of Table II or IV, preferably a homologue or functional equivalent as depicted in column 7 of Table II B, and being depicted in the same respective line as said B1798_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "neutral amino-acid efflux system", preferably it is the molecule of section (a) or (b) of this paragraph.

In one embodiment, said molecule, which activity is to be increased in the process of the invention and which is the gene product with an activity of described as a "neutral amino-acid efflux system", is increased non-targeted.

The sequence of B2226_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as b2226-protein.

Accordingly, in one embodiment, the process of the present invention comprises increasing or generating the activity of a gene product with the activity of a "b2226-protein" from Escherichia coli K12 or its functional equivalent or its homolog, e.g. the increase of
(a) a gene product of a gene comprising the nucleic acid molecule as shown in column 5 of Table I and being depicted in the same respective line as said B2226_2 or a functional equivalent or a homologue thereof as shown depicted in column 7 of Table I, preferably a homologue or functional equivalent as shown depicted in column 7 of Table I B, and being depicted in the same respective line as said B2226_2; or
(b) a polypeptide comprising a polypeptide, a consensus sequence or a polypeptide motif as shown depicted in column 5 of Table II, and being depicted in the same respective line as said B2226_2 or a functional equivalent or a homologue thereof as depicted in column 7 of Table II or IV, preferably a homologue or functional equivalent as depicted in column 7 of Table II B, and being depicted in the same respective line as said B2226_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "b2226-protein", preferably it is the molecule of section (a) or (b) of this paragraph.

In one embodiment, said molecule, which activity is to be increased in the process of the invention and which is the gene product with an activity of described as a "b2226-protein", is increased non-targeted.

The sequence of B2469_2 from Escherichia coli K12, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as sensory histidine kinase in two-component regulatory system with NarP (NarL).

Accordingly, in one embodiment, the process of the present invention comprises increasing or generating the activity of a gene product with the activity of a "sensory histidine kinase in two-component regulatory system with NarP (NarL)" from Escherichia coli K12 or its functional equivalent or its homolog, e.g. the increase of
(a) a gene product of a gene comprising the nucleic acid molecule as shown in column 5 of Table I and being depicted in the same respective line as said B2469_2 or a functional equivalent or a homologue thereof as shown depicted in column 7 of Table I, preferably a homologue or functional equivalent as shown depicted in column 7 of Table I B, and being depicted in the same respective line as said B2469_2; or
(b) a polypeptide comprising a polypeptide, a consensus sequence or a polypeptide motif as shown depicted in column 5 of Table II, and being depicted in the same respective line as said B2469_2 or a functional equivalent or a homologue thereof as depicted in column 7 of Table II or IV, preferably a homologue or functional equivalent as depicted in column 7 of Table II B, and being depicted in the same respective line as said B2469_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "sensory histidine kinase in two-component regulatory system with NarP (NarL)", preferably it is the molecule of section (a) or (b) of this paragraph.

In one embodiment, said molecule, which activity is to be increased in the process of the invention and which is the gene product with an activity of described as a "sensory histidine kinase in two-component regulatory system with NarP (NarL)", is increased non-targeted.

The sequence of YOR168W_2 from Saccharomyces cerevisiae, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocystis sp.* has been published in Kaneko and TAbata, Plant Cell Physiology 38 (11), 1997 and its activity is published described as Glutamine tRNA synthetase.

Accordingly, in one embodiment, the process of the present invention comprises increasing or generating the activity of a gene product with the activity of a "Glutamine tRNA synthetase" from Saccharomyces cerevisiae or its functional equivalent or its homolog, e.g. the increase of
(a) a gene product of a gene comprising the nucleic acid molecule as shown in column 5 of Table I and being depicted in the same respective line as said YOR168W_2 or a functional equivalent or a homologue thereof as shown depicted in column 7 of Table I, preferably a homologue or functional equivalent as shown depicted in column 7 of Table I B, and being depicted in the same respective line as said YOR168W_2; or
(b) a polypeptide comprising a polypeptide, a consensus sequence or a polypeptide motif as shown depicted in column 5 of Table II, and being depicted in the same respective line as said YOR168W_2 or a functional equivalent or a homologue thereof as depicted in column 7 of Table II or IV, preferably a homologue or functional equivalent as depicted in column 7 of Table II B, and being depicted in the same respective line as said YOR168W_2,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "Glutamine tRNA synthetase", preferably it is the molecule of section (a) or (b) of this paragraph.

In one embodiment, said molecule, which activity is to be increased in the process of the invention and which is the gene product with an activity of described as a "Glutamine tRNA synthetase", is increased non-targeted.

The sequence of B4321 from Escherichia coli, e.g. as shown in column 5 of Table I, [sequences from Saccharomyces cerevisiae has been published in Goffeau et al., Science 274 (5287), 546-547, 1996, sequences from Escherichia coli has been published in Blattner et al., Science 277 (5331), 1453-1474 (1997), sequences from *Synechocyst-is sp.* has been published in Kaneko and TAbata, Plant Cell Physiology 38 (11), 1997 and its activity is published described as gluconate transporter.

Accordingly, in one embodiment, the process of the present invention comprises increasing or generating the activity of a gene product with the activity of a "gluconate transporter" from Escherichia coli or its functional equivalent or its homolog, e.g. the increase of
(a) a gene product of a gene comprising the nucleic acid molecule as shown in column 5 of Table I and being depicted in the same respective line as said B4321 or a functional equivalent or a homologue thereof as shown depicted in column 7 of Table I, preferably a homologue or functional equivalent as shown depicted in column 7 of Table I B, and being depicted in the same respective line as said B4321; or
(b) a polypeptide comprising a polypeptide, a consensus sequence or a polypeptide motif as shown depicted in column 5 of Table II, and being depicted in the same respective line as said B4321 or a functional equivalent or a homologue thereof as depicted in column 7 of Table II or IV, preferably a homologue or functional equivalent as depicted in column 7 of Table II B, and being depicted in the same respective line as said B4321,
as mentioned herein, for the an increased tolerance and/or resistance to environmental stress and increased biomass production as compared to a corresponding non-transformed wild type plant cell, plant or part thereof in plant cell, plant or part thereof, as mentioned.

Accordingly, in one embodiment, the molecule which activity is to be increased in the process of the invention is the gene product with an activity of described as a "gluconate transporter", preferably it is the molecule of section (a) or (b) of this paragraph.

In one embodiment, said molecule, which activity is to be increased in the process of the invention and which is the gene product with an activity of described as a "gluconate transporter", is increased non-targeted.

Surprisingly, it was observed that a increasing or generating of at least one gene conferring an activity selected from the group consisting of: 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Ya1049c-protein, YCR059C-protein, YEL005C-protein, YER156C-protein, Yfr042w-protein, YGL045W-protein, and YOR024w-protein or of a gene comprising a nucleic acid sequence described in column 5 of Table I in Arabidopsis thaliana conferred an increased tolerance and/or resistance to environmental stress and increased biomass production in the transformed plants as compared to a corresponding non-transformed wild type plant.

Surprisingly, it was observed that a increasing or generating of at least one gene conferring an activity selected from the group consisting of: 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Ya1049c-protein, YCR059C-protein, YEL005C-protein, YER156C-protein, Yfr042w-protein, YGL045W-protein, and YOR024w-protein or of a gene comprising a nucleic acid sequence described in column 5 of Table I in Arabidopsis thaliana conferred an increased tolerance and/or resistance to environmental stress and increased biomass production in the transformed plants as compared to a corresponding non-transformed wild type plant.

In particular, it was observed that increasing or generating the activity of a gene product with the activity of a "b0081-protein" encoded by a gene comprising the nucleic acid sequence SEQ ID NO.: 38 in Arabidopsis thaliana conferred an increased drought resistance by surviving longer than the wild type control without showing any symptoms of injury for a period between 3.4 and 6 days as shown in the Examples.

It was further observed that increasing or generating the activity of a gene product with the activity of a "b0081-protein" encoded by a gene comprising the nucleic acid sequence SEQ ID NO.: 38 in Arabidopsis thaliana conferred an increased biomass production compared with the wild type control without showing any symptoms of injury for a period between 1.1 and 3 days as shown in the Examples.

In particular, it was observed that increasing or generating the activity of a gene product with the activity of a "transporter subunit / periplasmic-binding component of ABC superfamily" encoded by a gene comprising the nucleic acid sequence SEQ ID NO.: 54 in Arabidopsis thaliana conferred an increased drought resistance by surviving longer than the wild type control without showing any symptoms of injury for a period between 3 and 5 days as shown in the Examples.

It was further observed that increasing or generating the activity of a gene product with the activity of a "transporter subunit / periplasmic-binding component of ABC superfamily" encoded by a gene comprising the nucleic acid sequence SEQ ID NO.: 54 in Arabidopsis thaliana conferred an increased biomass production compared with the wild type control without showing any symptoms of injury for a period between 0.9 and 4 days as shown in the Examples.

In particular, it was observed that increasing or generating the activity of a gene product with the activity of a "b0482-protein" encoded by a gene comprising the nucleic acid sequence SEQ ID NO.: 70 in Arabidopsis thaliana conferred an increased drought resistance by surviving longer than the wild type control without showing any symptoms of injury for a period between 2.8 and 5 days as shown in the Examples.

It was further observed that increasing or generating the activity of a gene product with the activity of a "b0482-protein" encoded by a gene comprising the nucleic acid sequence SEQ ID NO.: 70 in Arabidopsis thaliana conferred an increased biomass production compared with the wild type control without showing any symptoms of injury for a period between 0.6 and 3 days as shown in the Examples.

In particular, it was observed that increasing or generating the activity of a gene product with the activity of a "universal stress protein UP12" encoded by a gene comprising the nucleic acid sequence SEQ ID NO.: 89 in Arabidopsis thaliana conferred an increased drought resistance by surviving longer than the wild type control without showing any symptoms of injury for a period between 2.9 and 5 days as shown in the Examples.

It was further observed that increasing or generating the activity of a gene product with the activity of a "universal stress protein UP12" encoded by a gene comprising the nucleic acid sequence SEQ ID NO.: 89 in Arabidopsis thaliana conferred an increased biomass production compared with the wild type control without showing any symptoms of injury for a period between 0.9 and 4 days as shown in the Examples.

In particular, it was observed that increasing or generating the activity of a gene product with the activity of a "transcriptional regulator protein" encoded by a gene comprising the nucleic acid sequence SEQ ID NO.: 143 in Arabidopsis thaliana conferred an increased drought resistance by surviving longer than the wild type control without showing any symptoms of injury for a period between 2.8 and 5 days as shown in the Examples.

It was further observed that increasing or generating the activity of a gene product with the activity of a "transcriptional regulator protein" encoded by a gene comprising the nucleic acid sequence SEQ ID NO.: 143 in Arabidopsis thaliana conferred an increased biomass production compared with the wild type control without showing any symptoms of injury for a period between 0.8 and 4 days as shown in the Examples.

In particular, it was observed that increasing or generating the activity of a gene product with the activity of a "b0631-protein" encoded by a gene comprising the nucleic acid sequence SEQ ID NO.: 162 in Arabidopsis thaliana conferred an increased drought resistance by surviving longer than the wild type control without showing any symptoms of injury for a period between 3.4 and 6 days as shown in the Examples.

It was further observed that increasing or generating the activity of a gene product with the activity of a "b0631-protein" encoded by a gene comprising the nucleic acid sequence SEQ ID NO.: 162 in Arabidopsis thaliana conferred an increased biomass production compared with the wild type control without showing any symptoms of injury for a period between 1.2 and 5 days as shown in the Examples.

In particular, it was observed that increasing or generating the activity of a gene product with the activity of a "potassium-transporting ATPase (subunit B)" encoded by a gene comprising the nucleic acid sequence SEQ ID NO.: 213 in Arabidopsis thaliana conferred an increased drought resistance by surviving longer than the wild type control without showing any symptoms of injury for a period between 2.1 and 3 days as shown in the Examples.

It was further observed that increasing or generating the activity of a gene product with the activity of a "potassium-transporting ATPase (subunit B)" encoded by a gene comprising the nucleic acid sequence SEQ ID NO.: 213 in Arabidopsis thaliana conferred an increased biomass production compared with the wild type control without showing any symptoms of injury for a period between 1.8 and 3 days as shown in the Examples.

In particular, it was observed that increasing or generating the activity of a gene product with the activity of a "b0753-protein" encoded by a gene comprising the nucleic acid sequence SEQ ID NO.: 358 in Arabidopsis thaliana conferred an increased drought resistance by surviving longer than the wild type control without showing any symptoms of injury for a period between 3.3 and 5 days as shown in the Examples.

It was further observed that increasing or generating the activity of a gene product with the activity of a "b0753-protein" encoded by a gene comprising the nucleic acid sequence SEQ ID NO.: 358 in Arabidopsis thaliana conferred an increased biomass production compared with the wild type control without showing any symptoms of injury for a period between 1.6 and 4 days as shown in the Examples.

In particular, it was observed that increasing or generating the activity of a gene product with the activity of a "threonine and homoserine efflux system" encoded by a gene comprising the nucleic acid sequence SEQ ID NO.: 367 in Arabidopsis thaliana conferred an increased drought resistance by surviving longer than the wild type control without showing any symptoms of injury for a period between 3 and 5 days as shown in the Examples.

It was further observed that increasing or generating the activity of a gene product with the activity of a "threonine and homoserine efflux system" encoded by a gene comprising the nucleic acid sequence SEQ ID NO.: 367 in Arabidopsis thaliana conferred an increased biomass production compared with the wild type control without showing any symptoms of injury for a period between 1.5 and 3 days as shown in the Examples.

In particular, it was observed that increasing or generating the activity of a gene product with the activity of a "predicted transporter protein" encoded by a gene comprising the nucleic acid sequence SEQ ID NO.: 420 in Arabidopsis thaliana conferred an increased drought resistance by surviving longer than the wild type control without showing any symptoms of injury for a period between 2.2 and 5 days as shown in the Examples.

It was further observed that increasing or generating the activity of a gene product with the activity of a "predicted transporter protein" encoded by a gene comprising the nucleic acid sequence SEQ ID NO.: 420 in Arabidopsis thaliana conferred an increased biomass production compared with the wild type control without showing any symptoms of injury for a period between 1 and 4 days as shown in the Examples.

In particular, it was observed that increasing or generating the activity of a gene product with the activity of a "b0866-protein" encoded by a gene comprising the nucleic acid sequence SEQ ID NO.: 455 in Arabidopsis thaliana conferred an increased drought resistance by surviving longer than the wild type control without showing any symptoms of injury for a period between 3 and 5 days as shown in the Examples.

It was further observed that increasing or generating the activity of a gene product with the activity of a "b0866-protein" encoded by a gene comprising the nucleic acid sequence SEQ ID NO.: 455 in Arabidopsis thaliana conferred an increased biomass production compared with the wild type control without showing any symptoms of injury for a period between 0.8 and 5 days as shown in the Examples.

In particular, it was observed that increasing or generating the activity of a gene product with the activity of a "methylglyoxal synthase" encoded by a gene comprising the nucleic acid sequence SEQ ID NO.: 535 in Arabidopsis thaliana conferred an increased drought resistance by surviving longer than the wild type control without showing any symptoms of injury for a period between 4.4 and 5 days as shown in the Examples.

It was further observed that increasing or generating the activity of a gene product with the activity of a "methylglyoxal synthase" encoded by a gene comprising the nucleic acid sequence SEQ ID NO.: 535 in Arabidopsis thaliana conferred an increased biomass production compared with the wild type control without showing any symptoms of injury for a period between 0.5 and 2 days as shown in the Examples.

In particular, it was observed that increasing or generating the activity of a gene product with the activity of a "HyaA/HyaB-processing protein" encoded by a gene comprising the nucleic acid sequence SEQ ID NO.: 618 in Arabidopsis thaliana conferred an increased drought resistance by surviving longer than the wild type control without showing any symptoms of injury for a period between 2.6 and 4 days as shown in the Examples.

It was further observed that increasing or generating the activity of a gene product with the activity of a "HyaA/HyaB-processing protein" encoded by a gene comprising the nucleic acid sequence SEQ ID NO.: 618 in Arabidopsis thaliana conferred an increased biomass production compared with the wild type control without showing any symptoms of injury for a period between 1.2 and 4 days as shown in the Examples.

In particular, it was observed that increasing or generating the activity of a gene product with the activity of a "predicted oxidoreductase (flavin:NADH component)" encoded by a gene comprising the nucleic acid sequence SEQ ID NO.: 671 in Arabidopsis thaliana conferred an increased drought resistance by surviving longer than the wild type control without showing any symptoms of injury for a period between 3.3 and 6 days as shown in the Examples.

It was further observed that increasing or generating the activity of a gene product with the activity of a "predicted oxidoreductase (flavin:NADH component)" encoded by a gene comprising the nucleic acid sequence SEQ ID NO.: 671 in Arabidopsis thaliana conferred an increased biomass production compared with the wild type control without showing any symptoms of injury for a period between 0.6 and 2 days as shown in the Examples.

In particular, it was observed that increasing or generating the activity of a gene product with the activity of a "b1052-protein" encoded by a gene comprising the nucleic acid sequence SEQ ID NO.: 764 in Arabidopsis thaliana conferred an increased drought resistance by surviving longer than the wild type control without showing any symptoms of injury for a period between 3 and 5 days as shown in the Examples.

It was further observed that increasing or generating the activity of a gene product with the activity of a "b1052-protein" encoded by a gene comprising the nucleic acid sequence SEQ ID NO.: 764 in Arabidopsis thaliana conferred an increased biomass production compared with the wild type control without showing any symptoms of injury for a period between 0.4 and 2 days as shown in the Examples.

In particular, it was observed that increasing or generating the activity of a gene product with the activity of a "3-oxoacyl-(acyl carrier protein) synthase" encoded by a gene comprising the nucleic acid sequence SEQ ID NO.: 768 in Arabidopsis thaliana conferred an increased drought resistance by surviving longer than the wild type control without showing any symptoms of injury for a period between 3.4 and 5 days as shown in the Examples.

It was further observed that increasing or generating the activity of a gene product with the activity of a "3-oxoacyl-(acyl carrier protein) synthase" encoded by a gene comprising the nucleic acid sequence SEQ ID NO.: 768 in Arabidopsis thaliana conferred an increased biomass production compared with the wild type control without showing any symptoms of injury for a period between 0.3 and 3 days as shown in the Examples.

In particular, it was observed that increasing or generating the activity of a gene product with the activity of a "b1161-protein" encoded by a gene comprising the nucleic acid sequence SEQ ID NO.: 907 in Arabidopsis thaliana conferred an increased drought resistance by surviving longer than the wild type control without showing any symptoms of injury for a period between 2.9 and 4 days as shown in the Examples.

It was further observed that increasing or generating the activity of a gene product with the activity of a "b1161-protein" encoded by a gene comprising the nucleic acid sequence SEQ ID NO.: 907 in Arabidopsis thaliana conferred an increased biomass production compared with the wild type control without showing any symptoms of injury for a period between 2 and 4 days as shown in the Examples.

In particular, it was observed that increasing or generating the activity of a gene product with the activity of a "sodium/proton antiporter" encoded by a gene comprising the nucleic acid sequence SEQ ID NO.: 927 in Arabidopsis thaliana conferred an increased drought resistance by surviving longer than the wild type control without showing any symptoms of injury for a period between 4 and 6 days as shown in the Examples.

It was further observed that increasing or generating the activity of a gene product with the activity of a "sodium/proton antiporter" encoded by a gene comprising the nucleic acid sequence SEQ ID NO.: 927 in Arabidopsis thaliana conferred an increased biomass production compared with the wild type control without showing any symptoms of injury for a period between 0.8 and 4 days as shown in the Examples.

In particular, it was observed that increasing or generating the activity of a gene product with the activity of a "predicted antimicrobial peptide transporter subunit" encoded by a gene comprising the nucleic acid sequence SEQ ID NO.: 1009 in Arabidopsis thaliana conferred an increased drought resistance by surviving longer than the wild type control without showing any symptoms of injury for a period between 3.4 and 5 days as shown in the Examples.

It was further observed that increasing or generating the activity of a gene product with the activity of a "predicted antimicrobial peptide transporter subunit" encoded by a gene comprising the nucleic acid sequence SEQ ID NO.: 1009 in Arabidopsis thaliana conferred an increased biomass production compared with the wild type control without showing any symptoms of injury for a period between 1.2 and 3 days as shown in the Examples.

In particular, it was observed that increasing or generating the activity of a gene product with the activity of a "predicted antimicrobial peptide transporter subunit" encoded by a gene comprising the nucleic acid sequence SEQ ID NO.: 1154 in Arabidopsis thaliana conferred an increased drought resistance by surviving longer than the wild type control without showing any symptoms of injury for a period between 2.3 and 5 days as shown in the Examples.

It was further observed that increasing or generating the activity of a gene product with the activity of a "predicted antimicrobial peptide transporter subunit" encoded by a gene comprising the nucleic acid sequence SEQ ID NO.: 1154 in Arabidopsis thaliana conferred an increased biomass production compared with the wild type control without showing any symptoms of injury for a period between 1 and 4 days as shown in the Examples.

In particular, it was observed that increasing or generating the activity of a gene product with the activity of a "b1423-protein" encoded by a gene comprising the nucleic acid sequence SEQ ID NO.: 1308 in Arabidopsis thaliana conferred an increased drought resistance by surviving longer than the wild type control without showing any symptoms of injury for a period between 3.1 and 6 days as shown in the Examples.

It was further observed that increasing or generating the activity of a gene product with the activity of a "b1423-protein" encoded by a gene comprising the nucleic acid sequence SEQ ID NO.: 1308 in Arabidopsis thaliana conferred an increased biomass production compared with the wild type control without showing any symptoms of injury for a period between 0.1 and 1 days as shown in the Examples.

In particular, it was observed that increasing or generating the activity of a gene product with the activity of a "acid shock protein precursor" encoded by a gene comprising the nucleic acid sequence SEQ ID NO.: 1368 in Arabidopsis thaliana conferred an increased drought resistance by surviving longer than the wild type control without showing any symptoms of injury for a period between 3.7 and 5 days as shown in the Examples.

It was further observed that increasing or generating the activity of a gene product with the activity of a "acid shock protein precursor" encoded by a gene comprising the nucleic acid sequence SEQ ID NO.: 1368 in Arabidopsis thaliana conferred an increased biomass production compared with the wild type control without showing any symptoms of injury for a period between 0.1 and 1 days as shown in the Examples.

In particular, it was observed that increasing or generating the activity of a gene product with the activity of a "predicted arginine/ornithine transporter" encoded by a gene comprising the nucleic acid sequence SEQ ID NO.: 1374 in Arabidopsis thaliana conferred an increased drought resistance by surviving longer than the wild type control without showing any symptoms of injury for a period between 2.8 and 5 days as shown in the Examples.

It was further observed that increasing or generating the activity of a gene product with the activity of a "predicted arginine/ornithine transporter" encoded by a gene comprising the nucleic acid sequence SEQ ID NO.: 1374 in Arabidopsis thaliana conferred an increased biomass production compared with the wild type control without showing any symptoms of injury for a period between 0.9 and 3 days as shown in the Examples.

In particular, it was observed that increasing or generating the activity of a gene product with the activity of a "3-deoxy-D-arabino-heptulosonate-7-phosphatesynthase" encoded by a gene comprising the nucleic acid sequence SEQ ID NO.: 1507 in Arabidopsis thaliana conferred an increased drought resistance by surviving longer than the wild type control without showing any symptoms of injury for a period between 4.2 and 5 days as shown in the Examples.

It was further observed that increasing or generating the activity of a gene product with the activity of a "3-deoxy-D-arabino-heptulosonate-7-phosphatesynthase" encoded by a gene comprising the nucleic acid sequence SEQ ID NO.: 1507 in Arabidopsis thaliana conferred an increased biomass production compared with the wild type control without showing any symptoms of injury for a period between 0.6 and 2 days as shown in the Examples.

In particular, it was observed that increasing or generating the activity of a gene product with the activity of a "N,N'-diacetylchitobiose-specific enzyme IIA component of PTS" encoded by a gene comprising the nucleic acid sequence SEQ ID NO.: 1953 in Arabidopsis thaliana conferred an increased drought resistance by surviving longer than the wild type control without showing any symptoms of injury for a period between 4.1 and 5 days as shown in the Examples.

It was further observed that increasing or generating the activity of a gene product with the activity of a "N,N'-diacetylchitobiose-specific enzyme IIA component of PTS" encoded by a gene comprising the nucleic acid sequence SEQ ID NO.: 1953 in Arabidopsis thaliana conferred an increased biomass production compared with the wild type control without showing any symptoms of injury for a period between 0.8 and 2 days as shown in the Examples.

In particular, it was observed that increasing or generating the activity of a gene product with the activity of a "neutral amino-acid efflux system" encoded by a gene comprising the nucleic acid sequence SEQ ID NO.: 2156 in Arabidopsis thaliana conferred an increased drought resistance by surviving longer than the wild type control without showing any symptoms of injury for a period between 3.4 and 5 days as shown in the Examples.

It was further observed that increasing or generating the activity of a gene product with the activity of a "neutral amino-acid efflux system" encoded by a gene comprising the nucleic acid sequence SEQ ID NO.: 2156 in Arabidopsis thaliana conferred an increased biomass production compared with the wild type control without showing any symptoms of injury for a period between 1 and 4 days as shown in the Examples.

In particular, it was observed that increasing or generating the activity of a gene product with the activity of a "b1878-protein" encoded by a gene comprising the nucleic acid sequence SEQ ID NO.: 2195 in Arabidopsis thaliana conferred an increased drought resistance by surviving longer than the wild type control without showing any symptoms of injury for a period between 3.4 and 5 days as shown in the Examples.

It was further observed that increasing or generating the activity of a gene product with the activity of a "b1878-protein" encoded by a gene comprising the nucleic acid sequence SEQ ID NO.: 2195 in Arabidopsis thaliana conferred an increased biomass production compared with the wild type control without showing any symptoms of injury for a period between 1.3 and 3 days as shown in the Examples.

In particular, it was observed that increasing or generating the activity of a gene product with the activity of a "L-arabinose transporter subunit" encoded by a gene comprising the nucleic acid sequence SEQ ID NO.: 2219 in Arabidopsis thaliana conferred an increased drought resistance by surviving longer than the wild type control without showing any symptoms of injury for a period between 2.8 and 4 days as shown in the Examples.

It was further observed that increasing or generating the activity of a gene product with the activity of a "L-arabinose transporter subunit" encoded by a gene comprising the nucleic acid sequence SEQ ID NO.: 2219 in Arabidopsis thaliana conferred an increased biomass production compared with the wild type control without showing any symptoms of injury for a period between 1.8 and 3 days as shown in the Examples.

In particular, it was observed that increasing or generating the activity of a gene product with the activity of a "phosphatidylglycerophosphate synthetase" encoded by a gene comprising the nucleic acid sequence SEQ ID NO.: 2277 in Arabidopsis thaliana conferred an increased drought resistance by surviving longer than the wild type control without showing any symptoms of injury for a period between 2.5 and 4 days as shown in the Examples.

It was further observed that increasing or generating the activity of a gene product with the activity of a "phosphatidylglycerophosphate synthetase" encoded by a gene comprising the nucleic acid sequence SEQ ID NO.: 2277 in Arabidopsis thaliana conferred an increased biomass production compared with the wild type control without showing any symptoms of injury for a period between 1.6 and 3 days as shown in the Examples.

In particular, it was observed that increasing or generating the activity of a gene product with the activity of a "regulator of length of O-antigen component of lipopolysaccharide chains" encoded by a gene comprising the nucleic acid sequence SEQ ID NO.: 2470 in Arabidopsis thaliana conferred an increased drought resistance by surviving longer than the wild type control without showing any symptoms of injury for a period between 2.6 and 4 days as shown in the Examples.

It was further observed that increasing or generating the activity of a gene product with the activity of a "regulator of length of O-antigen component of lipopolysaccharide chains" encoded by a gene comprising the nucleic acid sequence SEQ ID NO.: 2470 in Arabidopsis thaliana conferred an increased biomass production compared with the wild type control without showing any symptoms of injury for a period between 0.3 and 2 days as shown in the Examples.

In particular, it was observed that increasing or generating the activity of a gene product with the activity of a "glucose-1-phosphate thymidylyltransferase" encoded by a gene comprising the nucleic acid sequence SEQ ID NO.: 2493 in Arabidopsis thaliana conferred an increased drought resistance by surviving longer than the wild type control without showing any symptoms of injury for a period between 2.1 and 4 days as shown in the Examples.

It was further observed that increasing or generating the activity of a gene product with the activity of a "glucose-1-phosphate thymidylyltransferase" encoded by a gene comprising the nucleic acid sequence SEQ ID NO.: 2493 in Arabidopsis thaliana conferred an increased biomass production compared with the wild type control without showing any symptoms of injury for a period between 0.1 and 1 days as shown in the Examples.

In particular, it was observed that increasing or generating the activity of a gene product with the activity of a "multidrug efflux system (subunit B)" encoded by a gene comprising the nucleic acid sequence SEQ ID NO.: 2627 in Arabidopsis thaliana conferred an increased drought resistance by surviving longer than the wild type control without showing any symptoms of injury for a period between 2.5 and 4 days as shown in the Examples.

It was further observed that increasing or generating the activity of a gene product with the activity of a "multidrug efflux system (subunit B)" encoded by a gene comprising the nucleic acid sequence SEQ ID NO.: 2627 in Arabidopsis thaliana conferred an increased biomass production compared with the wild type control without showing any symptoms of injury for a period between 0.5 and 3 days as shown in the Examples.

In particular, it was observed that increasing or generating the activity of a gene product with the activity of a "GTP cyclohydrolase I" encoded by a gene comprising the nucleic acid sequence SEQ ID NO.: 2858 in Arabidopsis thaliana conferred an increased drought resistance by surviving longer than the wild type control without showing any symptoms of injury for a period between 2.5 and 4 days as shown in the Examples.

It was further observed that increasing or generating the activity of a gene product with the activity of a "GTP cyclohydrolase I" encoded by a gene comprising the nucleic acid sequence SEQ ID NO.: 2858 in Arabidopsis thaliana conferred an increased biomass production compared with the wild type control without showing any symptoms of injury for a period between 1.5 and 4 days as shown in the Examples.

In particular, it was observed that increasing or generating the activity of a gene product with the activity of a "heme lyase (CcmH subunit)" encoded by a gene comprising the nucleic acid sequence SEQ ID NO.: 2942 in Arabidopsis thaliana conferred an increased drought resistance by surviving longer than the wild type control without showing any symptoms of injury for a period between 3.4 and 6 days as shown in the Examples.

It was further observed that increasing or generating the activity of a gene product with the activity of a "heme lyase (CcmH subunit)" encoded by a gene comprising the nucleic acid sequence SEQ ID NO.: 2942 in Arabidopsis thaliana conferred an increased biomass production compared with the wild type control without showing any symptoms of injury for a period between 0.7 and 3 days as shown in the Examples.

In particular, it was observed that increasing or generating the activity of a gene product with the activity of a "b2226-protein" encoded by a gene comprising the nucleic acid sequence SEQ ID NO.: 2965 in Arabidopsis thaliana conferred an increased drought resistance by surviving longer than the wild type control without showing any symptoms of injury for a period between 2.9 and 4 days as shown in the Examples.

It was further observed that increasing or generating the activity of a gene product with the activity of a "b2226-protein" encoded by a gene comprising the nucleic acid sequence SEQ ID NO.: 2965 in Arabidopsis thaliana conferred an increased biomass production compared with the wild type control without showing any symptoms of injury for a period between 1.6 and 4 days as shown in the Examples.

In particular, it was observed that increasing or generating the activity of a gene product with the activity of a "histidine/lysine/arginine/ornithine transporter subunit protein" encoded by a gene comprising the nucleic acid sequence SEQ ID NO.: 2981 in Arabidopsis thaliana conferred an increased drought resistance by surviving longer than the wild type control without showing any symptoms of injury for a period between 1.9 and 3 days as shown in the Examples.

It was further observed that increasing or generating the activity of a gene product with the activity of a "histidine/lysine/arginine/ornithine transporter subunit protein" encoded by a gene comprising the nucleic acid sequence SEQ ID NO.: 2981 in Arabidopsis thaliana conferred an increased biomass production compared with the wild type control without showing any symptoms of injury for a period between 0.9 and 3 days as shown in the Examples.

In particular, it was observed that increasing or generating the activity of a gene product with the activity of a "sensory histidine kinase in two-component regulatory system with NarP (NarL)" encoded by a gene comprising the nucleic acid sequence SEQ ID NO.: 3130 in Arabidopsis thaliana conferred an increased drought resistance by surviving longer than the wild type control without showing any symptoms of injury for a period between 2.2 and 5 days as shown in the Examples.

It was further observed that increasing or generating the activity of a gene product with the activity of a "sensory histidine kinase in two-component regulatory system with NarP (NarL)" encoded by a gene comprising the nucleic acid sequence SEQ ID NO.: 3130 in Arabidopsis thaliana conferred an increased biomass production compared with the wild type control without showing any symptoms of injury for a period between 0.9 and 3 days as shown in the Examples.

In particular, it was observed that increasing or generating the activity of a gene product with the activity of a "b2475-protein" encoded by a gene comprising the nucleic acid sequence SEQ ID NO.: 3216 in Arabidopsis thaliana conferred an increased drought resistance by surviving longer than the wild type control without showing any symptoms of injury for a period between 2.9 and 5 days as shown in the Examples.

It was further observed that increasing or generating the activity of a gene product with the activity of a "b2475-protein" encoded by a gene comprising the nucleic acid sequence SEQ ID NO.: 3216 in Arabidopsis thaliana conferred an increased biomass production compared with the wild type control without showing any symptoms of injury for a period between 0.3 and 2 days as shown in the Examples.

In particular, it was observed that increasing or generating the activity of a gene product with the activity of a "NADH dehydrogenase (subunit N)" encoded by a gene comprising the nucleic acid sequence SEQ ID NO.: 3335 in Arabidopsis thaliana conferred an increased drought resistance by surviving longer than the wild type control without showing any symptoms of injury for a period between 1.9 and 3 days as shown in the Examples.

It was further observed that increasing or generating the activity of a gene product with the activity of a "NADH dehydrogenase (subunit N)" encoded by a gene comprising the nucleic acid sequence SEQ ID NO.: 3335 in Arabidopsis thaliana conferred an increased biomass production compared with the wild type control without showing any symptoms of injury for a period between 0.6 and 2 days as shown in the Examples.

In particular, it was observed that increasing or generating the activity of a gene product with the activity of a "2,3-dihydroxy-2,3-dihydrophenylpropionatedehydrogenase" encoded by a gene comprising the nucleic acid sequence SEQ ID NO.: 3401 in Arabidopsis thaliana conferred an increased drought resistance by surviving longer than the wild type control without showing any symptoms of injury for a period between 2.9 and 5 days as shown in the Examples.

It was further observed that increasing or generating the activity of a gene product with the activity of a "2,3-dihydroxy-2,3-dihydrophenylpropionatedehydrogenase" encoded by a gene comprising the nucleic acid sequence SEQ ID NO.: 3401 in Arabidopsis thaliana conferred an increased biomass production compared with the wild type control without showing any symptoms of injury for a period between 0.6 and 2 days as shown in the Examples.

In particular, it was observed that increasing or generating the activity of a gene product with the activity of a "tRNA-specific adenosine deaminase" encoded by a gene comprising the nucleic acid sequence SEQ ID NO.: 3590 in Arabidopsis thaliana conferred an increased drought resistance by surviving longer than the wild type control without showing any symptoms of injury for a period between 1.6 and 3 days as shown in the Examples.

It was further observed that increasing or generating the activity of a gene product with the activity of a "tRNA-specific adenosine deaminase" encoded by a gene comprising the nucleic acid sequence SEQ ID NO.: 3590 in Arabidopsis thaliana conferred an increased biomass production compared with the wild type control without showing any symptoms of injury for a period between 1.4 and 3 days as shown in the Examples.

In particular, it was observed that increasing or generating the activity of a gene product with the activity of a "predicted outer membrane lipoprotein" encoded by a gene comprising the nucleic acid sequence SEQ ID NO.: 3831 in Arabidopsis thaliana conferred an increased drought resistance by surviving longer than the wild type control without showing any symptoms of injury for a period between 1.6 and 4 days as shown in the Examples.

It was further observed that increasing or generating the activity of a gene product with the activity of a "predicted outer membrane lipoprotein" encoded by a gene comprising the nucleic acid sequence SEQ ID NO.: 3831 in Arabidopsis thaliana conferred an increased biomass production compared with the wild type control without showing any symptoms of injury for a period between 0.7 and 2 days as shown in the Examples.

In particular, it was observed that increasing or generating the activity of a gene product with the activity of a "CP4-57 prophage/ RNase LS" encoded by a gene comprising the nucleic acid sequence SEQ ID NO.: 3857 in Arabidopsis thaliana conferred an increased drought resistance by surviving longer than the wild type control without showing any symptoms of injury for a period between 1.8 and 3 days as shown in the Examples.

It was further observed that increasing or generating the activity of a gene product with the activity of a "CP4-57 prophage/ RNase LS" encoded by a gene comprising the nucleic acid sequence SEQ ID NO.: 3857 in Arabidopsis thaliana conferred an increased biomass production compared with the wild type control without showing any symptoms of injury for a period between 0.6 and 2 days as shown in the Examples.

In particular, it was observed that increasing or generating the activity of a gene product with the activity of a "glycine betaine transporter subunit protein" encoded by a gene comprising the nucleic acid sequence SEQ ID NO.: 3861 in Arabidopsis thaliana conferred an increased drought resistance by surviving longer than the wild type control without showing any symptoms of injury for a period between 4.3 and 5 days as shown in the Examples.

It was further observed that increasing or generating the activity of a gene product with the activity of a "glycine betaine transporter subunit protein" encoded by a gene comprising the nucleic acid sequence SEQ ID NO.: 3861 in Arabidopsis thaliana conferred an increased biomass production compared with the wild type control without showing any symptoms of injury for a period between 0.1 and 1 days as shown in the Examples. In particular, it was observed that increasing or generating the activity of a gene product with the activity of a "cellobiose/arbutin/salicin-specific PTS enzyme (IIB component/IC component)" encoded by a gene comprising the nucleic acid sequence SEQ ID NO.: 4022 in Arabidopsis thaliana conferred an increased drought resistance by surviving longer than the wild type control without showing any symptoms of injury for a period between 4 and 5 days as shown in the Examples.

It was further observed that increasing or generating the activity of a gene product with the activity of a "cellobiose/arbutin/salicin-specific PTS enzyme (IIB component/IC component)" encoded by a gene comprising the nucleic acid sequence SEQ ID NO.: 4022 in Arabidopsis thaliana conferred an increased biomass production compared with the wild type control without showing any symptoms of injury for a period between 0.4 and 2 days as shown in the Examples.

In particular, it was observed that increasing or generating the activity of a gene product with the activity of a "predicted kinase" encoded by a gene comprising the nucleic acid sequence SEQ ID NO.: 4059 in Arabidopsis thaliana conferred an increased drought resistance by surviving longer than the wild type control without showing any symptoms of injury for a period between 2.9 and 5 days as shown in the Examples.

It was further observed that increasing or generating the activity of a gene product with the activity of a "predicted kinase" encoded by a gene comprising the nucleic acid sequence SEQ ID NO.: 4059 in Arabidopsis thaliana conferred an increased biomass production compared with the wild type control without showing any symptoms of injury for a period between 0.4 and 2 days as shown in the Examples.

In particular, it was observed that increasing or generating the activity of a gene product with the activity of a "tRNA pseudouridine synthase" encoded by a gene comprising the nucleic acid sequence SEQ ID NO.: 4076 in Arabidopsis thaliana conferred an increased drought resistance by surviving longer than the wild type control without showing any symptoms of injury for a period between 3.2 and 6 days as shown in the Examples.

It was further observed that increasing or generating the activity of a gene product with the activity of a "tRNA pseudouridine synthase" encoded by a gene comprising the nucleic acid sequence SEQ ID NO.: 4076 in Arabidopsis thaliana conferred an increased biomass production compared with the wild type control without showing any symptoms of injury for a period between 0.7 and 3 days as shown in the Examples.

In particular, it was observed that increasing or generating the activity of a gene product with the activity of a "predicted ligase" encoded by a gene comprising the nucleic acid sequence SEQ ID NO.: 4157 in Arabidopsis thaliana conferred an increased drought resistance by surviving longer than the wild type control without showing any symptoms of injury for a period between 3 and 5 days as shown in the Examples.

It was further observed that increasing or generating the activity of a gene product with the activity of a "predicted ligase" encoded by a gene comprising the nucleic acid sequence SEQ ID NO.: 4157 in Arabidopsis thaliana conferred an increased biomass production compared with the wild type control without showing any symptoms of injury for a period between 1.7 and 3 days as shown in the Examples.

In particular, it was observed that increasing or generating the activity of a gene product with the activity of a "ornithine decarboxylase" encoded by a gene comprising the nucleic acid sequence SEQ ID NO.: 4260 in Arabidopsis thaliana conferred an increased drought resistance by surviving longer than the wild type control without showing any symptoms of injury for a period between 3.8 and 5 days as shown in the Examples.

It was further observed that increasing or generating the activity of a gene product with the activity of a "ornithine decarboxylase" encoded by a gene comprising the nucleic acid sequence SEQ ID NO.: 4260 in Arabidopsis thaliana conferred an increased biomass production compared with the wild type control without showing any symptoms of injury for a period between 0.7 and 3 days as shown in the Examples.

In particular, it was observed that increasing or generating the activity of a gene product with the activity of a "phosphate transporter" encoded by a gene comprising the nucleic acid sequence SEQ ID NO.: 4350 in Arabidopsis thaliana conferred an increased drought resistance by surviving longer than the wild type control without showing any symptoms of injury for a period between 2.9 and 4 days as shown in the Examples.

It was further observed that increasing or generating the activity of a gene product with the activity of a "phosphate transporter" encoded by a gene comprising the nucleic acid sequence SEQ ID NO.: 4350 in Arabidopsis thaliana conferred an increased biomass production compared with the wild type control without showing any symptoms of injury for a period between 1.3 and 4 days as shown in the Examples.

In particular, it was observed that increasing or generating the activity of a gene product with the activity of a "phosphate transporter" encoded by a gene comprising the nucleic acid sequence SEQ ID NO.: 4350 in Arabidopsis thaliana conferred an increased drought resistance by surviving longer than the wild type control without showing any symptoms of injury for a period between 2.7 and 4 days as shown in the Examples.

It was further observed that increasing or generating the activity of a gene product with the activity of a "phosphate transporter" encoded by a gene comprising the nucleic acid sequence SEQ ID NO.: 4350 in Arabidopsis thaliana conferred an increased biomass production compared with the wild type control without showing any symptoms of injury for a period between 0.1 and 0.1 days as shown in the Examples.

In particular, it was observed that increasing or generating the activity of a gene product with the activity of a "hexuronate transporter" encoded by a gene comprising the nucleic acid sequence SEQ ID NO.: 4459 in Arabidopsis thaliana conferred an increased drought resistance by surviving longer than the wild type control without showing any symptoms of injury for a period between 2.8 and 5 days as shown in the Examples.

It was further observed that increasing or generating the activity of a gene product with the activity of a "hexuronate transporter" encoded by a gene comprising the nucleic acid sequence SEQ ID NO.: 4459 in Arabidopsis thaliana conferred an increased biomass production compared with the wild type control without showing any symptoms of injury for a period between 1 and 3 days as shown in the Examples.

In particular, it was observed that increasing or generating the activity of a gene product with the activity of a "peptidyl-prolyl cis-trans isomerase A (rotamase A)" encoded by a gene comprising the nucleic acid sequence SEQ ID NO.: 4505 in Arabidopsis thaliana conferred an increased drought resistance by surviving longer than the wild type control without showing any symptoms of injury for a period between 3.7 and 4 days as shown in the Examples.

It was further observed that increasing or generating the activity of a gene product with the activity of a "peptidyl-prolyl cis-trans isomerase A (rotamase A)" encoded by a gene comprising the nucleic acid sequence SEQ ID NO.: 4505 in Arabidopsis thaliana conferred an increased biomass production compared with the wild type control without showing any symptoms of injury for a period between 0.1 and 0.1 days as shown in the Examples.

In particular, it was observed that increasing or generating the activity of a gene product with the activity of a "glycogen synthase" encoded by a gene comprising the nucleic acid sequence SEQ ID NO.: 4640 in Arabidopsis thaliana conferred an increased drought resistance by surviving longer than the wild type control without showing any symptoms of injury for a period between 2.8 and 4 days as shown in the Examples.

It was further observed that increasing or generating the activity of a gene product with the activity of a "glycogen synthase" encoded by a gene comprising the nucleic acid sequence SEQ ID NO.: 4640 in Arabidopsis thaliana conferred an increased biomass production compared with the wild type control without showing any symptoms of injury for a period between 0.6 and 2 days as shown in the Examples.

In particular, it was observed that increasing or generating the activity of a gene product with the activity of a "D-xylose transporter subunit" encoded by a gene comprising the nucleic acid sequence SEQ ID NO.: 4806 in Arabidopsis thaliana conferred an increased drought resistance by surviving longer than the wild type control without showing any symptoms of injury for a period between 2.7 and 4 days as shown in the Examples.

It was further observed that increasing or generating the activity of a gene product with the activity of a "D-xylose transporter subunit" encoded by a gene comprising the nucleic acid sequence SEQ ID NO.: 4806 in Arabidopsis thaliana conferred an increased biomass production compared with the wild type control without showing any symptoms of injury for a period between 0.8 and 3 days as shown in the Examples.

In particular, it was observed that increasing or generating the activity of a gene product with the activity of a "L-threonine 3-dehydrogenase" encoded by a gene comprising the nucleic acid sequence SEQ ID NO.: 5124 in Arabidopsis thaliana conferred an increased drought resistance by surviving longer than the wild type control without showing any symptoms of injury for a period between 2.5 and 5 days as shown in the Examples.

It was further observed that increasing or generating the activity of a gene product with the activity of a "L-threonine 3-dehydrogenase" encoded by a gene comprising the nucleic acid sequence SEQ ID NO.: 5124 in Arabidopsis thaliana conferred an increased biomass production compared with the wild type control without showing any symptoms of injury for a period between 1.1 and 5 days as shown in the Examples.

In particular, it was observed that increasing or generating the activity of a gene product with the activity of a "L-threonine 3-dehydrogenase" encoded by a gene comprising the nucleic acid sequence SEQ ID NO.: 5124 in Arabidopsis thaliana conferred an increased drought resistance by surviving longer than the wild type control without showing any symptoms of injury for a period between 2.8 and 5 days as shown in the Examples.

It was further observed that increasing or generating the activity of a gene product with the activity of a "L-threonine 3-dehydrogenase" encoded by a gene comprising the nucleic acid sequence SEQ ID NO.: 5124 in Arabidopsis thaliana conferred an increased biomass production compared with the wild type control without showing any symptoms of injury for a period between 1.3 and 3 days as shown in the Examples.

In particular, it was observed that increasing or generating the activity of a gene product with the activity of a "predicted hydrolase" encoded by a gene comprising the nucleic acid sequence SEQ ID NO.: 5417 in Arabidopsis thaliana conferred an increased drought resistance by surviving longer than the wild type control without showing any symptoms of injury for a period between 3 and 5 days as shown in the Examples.

It was further observed that increasing or generating the activity of a gene product with the activity of a "predicted hydrolase" encoded by a gene comprising the nucleic acid sequence SEQ ID NO.: 5417 in Arabidopsis thaliana conferred an increased biomass production compared with the wild type control without showing any symptoms of injury for a period between 0.9 and 3 days as shown in the Examples.

In particular, it was observed that increasing or generating the activity of a gene product with the activity of a "predicted PTS enzymes (IIB component/IIC component)" encoded by a gene comprising the nucleic acid sequence SEQ ID NO.: 5495 in Arabidopsis thaliana conferred an increased drought resistance by surviving longer than the wild type control without showing any symptoms of injury for a period between 2.5 and 4 days as shown in the Examples.

It was further observed that increasing or generating the activity of a gene product with the activity of a "predicted PTS enzymes (IIB component/IIC component)" encoded by a gene comprising the nucleic acid sequence SEQ ID NO.: 5495 in Arabidopsis thaliana conferred an increased biomass production compared with the wild type control without showing any symptoms of injury for a period between 0.8 and 3 days as shown in the Examples.

In particular, it was observed that increasing or generating the activity of a gene product with the activity of a "ribonuclease activity regulator protein RraA" encoded by a gene comprising the nucleic acid sequence SEQ ID NO.: 5585 in Arabidopsis thaliana conferred an increased drought resistance by surviving longer than the wild type control without showing any symptoms of injury for a period between 2.3 and 4 days as shown in the Examples.

It was further observed that increasing or generating the activity of a gene product with the activity of a "ribonuclease activity regulator protein RraA" encoded by a gene comprising the nucleic acid sequence SEQ ID NO.: 5585 in Arabidopsis thaliana conferred an increased biomass production compared with the wild type control without showing any symptoms of injury for a period between 0.7 and 3 days as shown in the Examples.

In particular, it was observed that increasing or generating the activity of a gene product with the activity of a "transcriptional repressor protein MetJ" encoded by a gene comprising the nucleic acid sequence SEQ ID NO.: 5800 in Arabidopsis thaliana conferred an increased drought resistance by surviving longer than the wild type control without showing any symptoms of injury for a period between 2.3 and 3 days as shown in the Examples.

It was further observed that increasing or generating the activity of a gene product with the activity of a "transcriptional repressor protein MetJ" encoded by a gene comprising the nucleic acid sequence SEQ ID NO.: 5800 in Arabidopsis thaliana conferred an increased biomass production compared with the wild type control without showing any symptoms of injury for a period between 1.1 and 3 days as shown in the Examples.

In particular, it was observed that increasing or generating the activity of a gene product with the activity of a "pantothenate kinase" encoded by a gene comprising the nucleic acid sequence SEQ ID NO.: 5850 in Arabidopsis thaliana conferred an increased drought resistance by surviving longer than the wild type control without showing any symptoms of injury for a period between 2.9 and 5 days as shown in the Examples.

It was further observed that increasing or generating the activity of a gene product with the activity of a "pantothenate kinase" encoded by a gene comprising the nucleic acid sequence SEQ ID NO.: 5850 in Arabidopsis thaliana conferred an increased biomass production compared with the wild type control without showing any symptoms of injury for a period between 1.4 and 2 days as shown in the Examples.

In particular, it was observed that increasing or generating the activity of a gene product with the activity of a "heat shock protein" encoded by a gene comprising the nucleic acid sequence SEQ ID NO.: 5992 in Arabidopsis thaliana conferred an increased drought resistance by surviving longer than the wild type control without showing any symptoms of injury for a period between 2.8 and 5 days as shown in the Examples.

It was further observed that increasing or generating the activity of a gene product with the activity of a "heat shock protein" encoded by a gene comprising the nucleic acid sequence SEQ ID NO.: 5992 in Arabidopsis thaliana conferred an increased biomass production compared with the wild type control without showing any symptoms of injury for a period between 0.7 and 2 days as shown in the Examples.

In particular, it was observed that increasing or generating the activity of a gene product with the activity of a "predicted porin" encoded by a gene comprising the nucleic acid sequence SEQ ID NO.: 5999 in Arabidopsis thaliana conferred an increased drought resistance by surviving longer than the wild type control without showing any symptoms of injury for a period between 3.2 and 5 days as shown in the Examples.

It was further observed that increasing or generating the activity of a gene product with the activity of a "predicted porin" encoded by a gene comprising the nucleic acid sequence SEQ ID NO.: 5999 in Arabidopsis thaliana conferred an increased biomass production compared with the wild type control without showing any symptoms of injury for a period between 0.5 and 3 days as shown in the Examples.

In particular, it was observed that increasing or generating the activity of a gene product with the activity of a "aspartate ammonia-lyase" encoded by a gene comprising the nucleic acid sequence SEQ ID NO.: 6056 in Arabidopsis thaliana conferred an increased drought resistance by surviving longer than the wild type control without showing any symptoms of injury for a period between 3.1 and 5 days as shown in the Examples.

It was further observed that increasing or generating the activity of a gene product with the activity of a "aspartate ammonia-lyase" encoded by a gene comprising the nucleic acid sequence SEQ ID NO.: 6056 in Arabidopsis thaliana conferred an increased biomass production compared with the wild type control without showing any symptoms of injury for a period between 1.6 and 4 days as shown in the Examples.

In particular, it was observed that increasing or generating the activity of a gene product with the activity of a "nicotinamide-nucleotide adenylyltransferase" encoded by a gene comprising the nucleic acid sequence SEQ ID NO.: 6500 in Arabidopsis thaliana conferred an increased drought resistance by surviving longer than the wild type control without showing any symptoms of injury for a period between 3.5 and 6 days as shown in the Examples.

It was further observed that increasing or generating the activity of a gene product with the activity of a "nicotinamide-nucleotide adenylyltransferase" encoded by a gene comprising the nucleic acid sequence SEQ ID NO.: 6500 in Arabidopsis thaliana conferred an increased biomass production compared with the wild type control without showing any symptoms of injury for a period between 0.8 and 3 days as shown in the Examples.

In particular, it was observed that increasing or generating the activity of a gene product with the activity of a "polyphosphate kinase" encoded by a gene comprising the nucleic acid sequence SEQ ID NO.: 6542 in Arabidopsis thaliana conferred an increased drought resistance by surviving longer than the wild type control without showing any symptoms of injury for a period between 2.5 and 4 days as shown in the Examples.

It was further observed that increasing or generating the activity of a gene product with the activity of a "polyphosphate kinase" encoded by a gene comprising the nucleic acid sequence SEQ ID NO.: 6542 in Arabidopsis thaliana conferred an increased biomass production compared with the wild type control without showing any symptoms of injury for a period between 0.9 and 3 days as shown in the Examples.

In particular, it was observed that increasing or generating the activity of a gene product with the activity of a "Ya1049c-protein" encoded by a gene comprising the nucleic acid sequence SEQ ID NO.: 6823 in Arabidopsis thaliana conferred an increased drought resistance by surviving longer than the wild type control without showing any symptoms of injury for a period between 3.5 and 5 days as shown in the Examples.

It was further observed that increasing or generating the activity of a gene product with the activity of a "Ya1049c-protein" encoded by a gene comprising the nucleic acid sequence SEQ ID NO.: 6823 in Arabidopsis thaliana conferred an increased biomass production compared with the wild type control without showing any symptoms of injury for a period between 0.7 and 2 days as shown in the Examples.

In particular, it was observed that increasing or generating the activity of a gene product with the activity of a "YCR059C-protein" encoded by a gene comprising the nucleic acid sequence SEQ ID NO.: 6870 in Arabidopsis thaliana conferred an increased drought resistance by surviving longer than the wild type control without showing any symptoms of injury for a period between 3.5 and 5 days as shown in the Examples.

It was further observed that increasing or generating the activity of a gene product with the activity of a "YCR059C-protein" encoded by a gene comprising the nucleic acid sequence SEQ ID NO.: 6870 in Arabidopsis thaliana conferred an increased biomass production compared with the wild type control without showing any symptoms of injury for a period between 1 and 3 days as shown in the Examples.

In particular, it was observed that increasing or generating the activity of a gene product with the activity of a "3-deoxy-D-arabino-heptulosonate-7-phosphate (DAHP) synthase" encoded by a gene comprising the nucleic acid sequence SEQ ID NO.: 6910 in Arabidopsis thaliana conferred an increased drought resistance by surviving longer than the wild type control without showing any symptoms of injury for a period between 2.7 and 4 days as shown in the Examples.

It was further observed that increasing or generating the activity of a gene product with the activity of a "3-deoxy-D-arabino-heptulosonate-7-phosphate (DAHP) synthase" encoded by a gene comprising the nucleic acid sequence SEQ ID NO.: 6910 in Arabidopsis thaliana conferred an increased biomass production compared with the wild type control without showing any symptoms of injury for a period between 1.3 and 3 days as shown in the Examples.

In particular, it was observed that increasing or generating the activity of a gene product with the activity of a "YEL005C-protein" encoded by a gene comprising the nucleic acid sequence SEQ ID NO.: 7261 in Arabidopsis thaliana conferred an increased drought resistance by surviving longer than the wild type control without showing any symptoms of injury for a period between 3.6 and 6 days as shown in the Examples.

It was further observed that increasing or generating the activity of a gene product with the activity of a "YEL005C-protein" encoded by a gene comprising the nucleic acid sequence SEQ ID NO.: 7261 in Arabidopsis thaliana conferred an increased biomass production compared with the wild type control without showing any symptoms of injury for a period between 1.1 and 4 days as shown in the Examples.

In particular, it was observed that increasing or generating the activity of a gene product with the activity of a "Lsm (Like Sm) protein" encoded by a gene comprising the nucleic acid sequence SEQ ID NO.: 7265 in Arabidopsis thaliana conferred an increased drought resistance by surviving longer than the wild type control without showing any symptoms of injury for a period between 3.1 and 4 days as shown in the Examples.

It was further observed that increasing or generating the activity of a gene product with the activity of a "Lsm (Like Sm) protein" encoded by a gene comprising the nucleic acid sequence SEQ ID NO.: 7265 in Arabidopsis thaliana conferred an increased biomass production compared with the wild type control without showing any symptoms of injury for a period between 0.8 and 2 days as shown in the Examples.

In particular, it was observed that increasing or generating the activity of a gene product with the activity of a "YER156C-protein" encoded by a gene comprising the nucleic acid sequence SEQ ID NO.: 7301 in Arabidopsis thaliana conferred an increased drought resistance by surviving longer than the wild type control without showing any symptoms of injury for a period between 2.2 and 4 days as shown in the Examples.

It was further observed that increasing or generating the activity of a gene product with the activity of a "YER156C-protein" encoded by a gene comprising the nucleic acid sequence SEQ ID NO.: 7301 in Arabidopsis thaliana conferred an increased biomass production compared with the wild type control without showing any symptoms of injury for a period between 0.1 and 0.1 days as shown in the Examples.

In particular, it was observed that increasing or generating the activity of a gene product with the activity of a "Checkpoint protein" encoded by a gene comprising the nucleic acid sequence SEQ ID NO.: 7384 in Arabidopsis thaliana conferred an increased drought resistance by surviving longer than the wild type control without showing any symptoms of injury for a period between 4.3 and 5 days as shown in the Examples.

It was further observed that increasing or generating the activity of a gene product with the activity of a "Checkpoint protein" encoded by a gene comprising the nucleic acid sequence SEQ ID NO.: 7384 in Arabidopsis thaliana conferred an increased biomass production compared with the wild type control without showing any symptoms of injury for a period between 0.2 and 1 days as shown in the Examples.

In particular, it was observed that increasing or generating the activity of a gene product with the activity of a "YGL045W-protein" encoded by a gene comprising the nucleic acid sequence SEQ ID NO.: 7407 in Arabidopsis thaliana conferred an increased drought resistance by surviving longer than the wild type control without showing any symptoms of injury for a period between 3.3 and 4 days as shown in the Examples.

It was further observed that increasing or generating the activity of a gene product with the activity of a "YGL045W-protein" encoded by a gene comprising the nucleic acid sequence SEQ ID NO.: 7407 in Arabidopsis thaliana conferred an increased biomass production compared with the wild type control without showing any symptoms of injury for a period between 1.2 and 4 days as shown in the Examples.

In particular, it was observed that increasing or generating the activity of a gene product with the activity of a "Protein component of the small (40S) ribosomal subunit" encoded by a gene comprising the nucleic acid sequence SEQ ID NO.: 7429 in Arabidopsis thaliana conferred an increased drought resistance by surviving longer than the wild type control without showing any symptoms of injury for a period between 2.3 and 4 days as shown in the Examples.

It was further observed that increasing or generating the activity of a gene product with the activity of a "Protein component of the small (40S) ribosomal subunit" encoded by a gene comprising the nucleic acid sequence SEQ ID NO.: 7429 in Arabidopsis thaliana conferred an increased biomass production compared with the wild type control without showing any symptoms of injury for a period between 0.1 and 0.1 days as shown in the Examples.

In particular, it was observed that increasing or generating the activity of a gene product with the activity of a "Dihydrouridine synthase" encoded by a gene comprising the nucleic acid sequence SEQ ID NO.: 7558 in Arabidopsis thaliana conferred an increased drought resistance by surviving longer than the wild type control without showing any symptoms of injury for a period between 4.5 and 7 days as shown in the Examples.

It was further observed that increasing or generating the activity of a gene product with the activity of a "Dihydrouridine synthase" encoded by a gene comprising the nucleic acid sequence SEQ ID NO.: 7558 in Arabidopsis thaliana conferred an increased biomass production compared with the wild type control without showing any symptoms of injury for a period between 0.1 and 0.1 days as shown in the Examples.

In particular, it was observed that increasing or generating the activity of a gene product with the activity of a "YOR024w-protein" encoded by a gene comprising the nucleic acid sequence SEQ ID NO.: 7606 in Arabidopsis thaliana conferred an increased drought resistance by surviving longer than the wild type control without showing any symptoms of injury for a period between 4.8 and 5 days as shown in the Examples.

It was further observed that increasing or generating the activity of a gene product with the activity of a "YOR024w-protein" encoded by a gene comprising the nucleic acid sequence SEQ ID NO.: 7606 in Arabidopsis thaliana conferred an increased biomass production compared with the wild type control without showing any symptoms of injury for a period between 0.6 and 3 days as shown in the Examples.

In particular, it was observed that increasing or generating the activity of a gene product with the activity of a "Glutamine tRNA synthetase" encoded by a gene comprising the nucleic acid sequence SEQ ID NO.: 7610 in Arabidopsis thaliana conferred an increased drought resistance by surviving longer than the wild type control without showing any symptoms of injury for a period between 3.1 and 5 days as shown in the Examples.

It was further observed that increasing or generating the activity of a gene product with the activity of a "Glutamine tRNA synthetase" encoded by a gene comprising the nucleic acid sequence SEQ ID NO.: 7610 in Arabidopsis thaliana conferred an increased biomass production compared with the wild type control without showing any symptoms of injury for a period between 0.6 and 2 days as shown in the Examples.

In particular, it was observed that increasing or generating the activity of a gene product with the activity of a "Splicing factor" encoded by a gene comprising the nucleic acid sequence SEQ ID NO.: 7685 in Arabidopsis thaliana conferred an increased drought resistance by surviving longer than the wild type control without showing any symptoms of injury for a period between 3.9 and 5 days as shown in the Examples.

It was further observed that increasing or generating the activity of a gene product with the activity of a "Splicing factor" encoded by a gene comprising the nucleic acid sequence SEQ ID NO.: 7685 in Arabidopsis thaliana conferred an increased biomass production compared with the wild type control without showing any symptoms of injury for a period between 0.1 and 0.1 days as shown in the Examples.

In particular, it was observed that increasing or generating the activity of a gene product with the activity of a "gamma-Glu-putrescine synthase" encoded by a gene comprising the nucleic acid sequence SEQ ID NO.: 1201 in Arabidopsis thaliana conferred an increased drought resistance by surviving longer than the wild type control without showing any symptoms of injury for a period between 2.1 and 3 days as shown in the Examples.

It was further observed that increasing or generating the activity of a gene product with the activity of a "gamma-Glu-putrescine synthase" encoded by a gene comprising the nucleic acid sequence SEQ ID NO.: 1201 in Arabidopsis thaliana conferred an increased biomass production compared with the wild type control without showing any symptoms of injury for a period between 1.1 and 2 days as shown in the Examples.

In particular, it was observed that increasing or generating the activity of a gene product with the activity of a "inner membrane protein" encoded by a gene comprising the nucleic acid sequence SEQ ID NO.: 7741 in Arabidopsis thaliana conferred an increased drought resistance by surviving longer than the wild type control without showing any symptoms of injury for a period between 3.2 and 5 days as shown in the Examples.

It was further observed that increasing or generating the activity of a gene product with the activity of a "inner membrane protein" encoded by a gene comprising the nucleic acid sequence SEQ ID NO.: 7741 in Arabidopsis thaliana conferred an increased biomass production compared with the wild type control without showing any symptoms of injury for a period between 0.2 and 2 days as shown in the Examples.

In particular, it was observed that increasing or generating the activity of a gene product with the activity of a "heat shock protein HtpX" encoded by a gene comprising the nucleic acid sequence SEQ ID NO.: 7850 in Arabidopsis thaliana conferred an increased drought resistance by surviving longer than the wild type control without showing any symptoms of injury for a period between 2.4 and 6 days as shown in the Examples.

It was further observed that increasing or generating the activity of a gene product with the activity of a "heat shock protein HtpX" encoded by a gene comprising the nucleic acid sequence SEQ ID NO.: 7850 in Arabidopsis thaliana conferred an increased biomass production compared with the wild type control without showing any symptoms of injury for a period between 1 and 3 days as shown in the Examples.

In particular, it was observed that increasing or generating the activity of a gene product with the activity of a "DNA-binding transcriptional dual regulator protein" encoded by a gene comprising the nucleic acid sequence SEQ ID NO.: 7971 in Arabidopsis thaliana conferred an increased drought resistance by surviving longer than the wild type control without showing any symptoms of injury for a period between 3.9 and 5 days as shown in the Examples.

It was further observed that increasing or generating the activity of a gene product with the activity of a "DNA-binding transcriptional dual regulator protein" encoded by a gene comprising the nucleic acid sequence SEQ ID NO.: 7971 in Arabidopsis thaliana conferred an increased biomass production compared with the wild type control without showing any symptoms of injury for a period between 0.4 and 2 days as shown in the Examples.

In particular, it was observed that increasing or generating the activity of a gene product with the activity of a "predicted serine transporter protein" encoded by a gene comprising the nucleic acid sequence SEQ ID NO.: 8021 in Arabidopsis thaliana conferred an increased drought resistance by surviving longer than the wild type control without showing any symptoms of injury for a period between 2.9 and 5 days as shown in the Examples.

It was further observed that increasing or generating the activity of a gene product with the activity of a "predicted serine transporter protein" encoded by a gene comprising the nucleic acid sequence SEQ ID NO.: 8021 in Arabidopsis thaliana conferred an increased biomass production compared with the wild type control without showing any symptoms of injury for a period between 0.7 and 4 days as shown in the Examples.

In particular, it was observed that increasing or generating the activity of a gene product with the activity of a "glutathione-dependent oxidoreductase" encoded by a gene comprising the nucleic acid sequence SEQ ID NO.: 8177 in Arabidopsis thaliana conferred an increased drought resistance by surviving longer than the wild type control without showing any symptoms of injury for a period between 4.5 and 5 days as shown in the Examples.

It was further observed that increasing or generating the activity of a gene product with the activity of a "glutathione-dependent oxidoreductase" encoded by a gene comprising the nucleic acid sequence SEQ ID NO.: 8177 in Arabidopsis thaliana conferred an increased biomass production compared with the wild type control without showing any symptoms of injury for a period between 0.1 and 0.1 days as shown in the Examples.

In particular, it was observed that increasing or generating the activity of a gene product with the activity of a "Yfr042w-protein" encoded by a gene comprising the nucleic acid sequence SEQ ID NO.: 8272 in Arabidopsis thaliana conferred an increased drought resistance by surviving longer than the wild type control without showing any symptoms of injury for a period between 3.7 and 5 days as shown in the Examples.

It was further observed that increasing or generating the activity of a gene product with the activity of a "Yfr042w-protein" encoded by a gene comprising the nucleic acid sequence SEQ ID NO.: 8272 in Arabidopsis thaliana conferred an increased biomass production compared with the wild type control without showing any symptoms of injury for a period between 1 and 4 days as shown in the Examples.

In particular, it was observed that increasing or generating the activity of a gene product with the activity of a "Protein component of the small (40S) ribosomal subunit" encoded by a gene comprising the nucleic acid sequence SEQ ID NO.: 8288 in Arabidopsis thaliana conferred an increased drought resistance by surviving longer than the wild type control without showing any symptoms of injury for a period between 3.3 and 5 days as shown in the Examples.

It was further observed that increasing or generating the activity of a gene product with the activity of a "Protein component of the small (40S) ribosomal subunit" encoded by a gene comprising the nucleic acid sequence SEQ ID NO.: 8288 in Arabidopsis thaliana conferred an increased biomass production compared with the wild type control without showing any symptoms of injury for a period between 1.9 and 4 days as shown in the Examples.

In particular, it was observed that increasing or generating the activity of a gene product with the activity of a "transcriptional regulator protein" encoded by a gene comprising the nucleic acid sequence SEQ ID NO.: 8438 in Arabidopsis thaliana conferred an increased drought resistance by surviving longer than the wild type control without showing any symptoms of injury for a period between 2.8 and 5 days as shown in the Examples.

It was further observed that increasing or generating the activity of a gene product with the activity of a "transcriptional regulator protein" encoded by a gene comprising the nucleic acid sequence SEQ ID NO.: 8438 in Arabidopsis thaliana conferred an increased biomass production compared with the wild type control without showing any symptoms of injury for a period between 0.8 and 4 days as shown in the Examples.

In particular, it was observed that increasing or generating the activity of a gene product with the activity of a "predicted oxidoreductase (flavin:NADH component)" encoded by a gene comprising the nucleic acid sequence SEQ ID NO.: 8630 in Arabidopsis thaliana conferred an increased drought resistance by surviving longer than the wild type control without showing any symptoms of injury for a period between 3.3 and 6 days as shown in the Examples.

It was further observed that increasing or generating the activity of a gene product with the activity of a "predicted oxidoreductase (flavin:NADH component)" encoded by a gene comprising the nucleic acid sequence SEQ ID NO.: 8630 in Arabidopsis thaliana conferred an increased biomass production compared with the wild type control without showing any symptoms of injury for a period between 0.6 and 2 days as shown in the Examples.

In particular, it was observed that increasing or generating the activity of a gene product with the activity of a "cellobiose/arbutin/salicin-specific PTS enzyme (IIB component/IC component)" encoded by a gene comprising the nucleic acid sequence SEQ ID NO.: 9268 in Arabidopsis thaliana conferred an increased drought resistance by surviving longer than the wild type control without showing any symptoms of injury for a period between 4 and 5 days as shown in the Examples.

It was further observed that increasing or generating the activity of a gene product with the activity of a "cellobiose/arbutin/salicin-specific PTS enzyme (IIB component/IC component)" encoded by a gene comprising the nucleic acid sequence SEQ ID NO.: 9268 in Arabidopsis thaliana conferred an increased biomass production compared with the wild type control without showing any symptoms of injury for a period between 0.4 and 2 days as shown in the Examples.

In particular, it was observed that increasing or generating the activity of a gene product with the activity of a "predicted PTS enzymes (IIB component/IIC component)" encoded by a gene comprising the nucleic acid sequence SEQ ID NO.: 9444 in Arabidopsis thaliana conferred an increased drought resistance by surviving longer than the wild type control without showing any symptoms of injury for a period between 2.5 and 4 days as shown in the Examples.

It was further observed that increasing or generating the activity of a gene product with the activity of a "predicted PTS enzymes (IIB component/IIC component)" encoded by a gene comprising the nucleic acid sequence SEQ ID NO.: 9444 in Arabidopsis thaliana conferred an increased biomass production compared with the wild type control without showing any symptoms of injury for a period between 0.8 and 3 days as shown in the Examples.

In particular, it was observed that increasing or generating the activity of a gene product with the activity of a "nicotinamide-nucleotide adenylyltransferase" encoded by a gene comprising the nucleic acid sequence SEQ ID NO.: 9824 in Arabidopsis thaliana conferred an increased drought resistance by surviving longer than the wild type control without showing any symptoms of injury for a period between 3.5 and 6 days as shown in the Examples.

It was further observed that increasing or generating the activity of a gene product with the activity of a "nicotinamide-nucleotide adenylyltransferase" encoded by a gene comprising the nucleic acid sequence SEQ ID NO.: 9824 in Arabidopsis thaliana conferred an increased biomass production compared with the wild type control without showing any symptoms of injury for a period between 0.8 and 3 days as shown in the Examples.

In particular, it was observed that increasing or generating the activity of a gene product with the activity of a "YGL045W-protein" encoded by a gene comprising the nucleic acid sequence SEQ ID NO.: 9905 in Arabidopsis thaliana conferred an increased drought resistance by surviving longer than the wild type control without showing any symptoms of injury for a period between 3.3 and 4 days as shown in the Examples.

It was further observed that increasing or generating the activity of a gene product with the activity of a "YGL045W-protein" encoded by a gene comprising the nucleic acid sequence SEQ ID NO.: 9905 in Arabidopsis thaliana conferred an increased biomass production compared with the wild type control without showing any symptoms of injury for a period between 1.2 and 4 days as shown in the Examples.

In particular, it was observed that increasing or generating the activity of a gene product with the activity of a "DNA-binding transcriptional dual regulator protein" encoded by a gene comprising the nucleic acid sequence SEQ ID NO.: 9193 in Arabidopsis thaliana conferred an increased drought resistance by surviving longer than the wild type control without showing any symptoms of injury for a period between 3.9 and 5 days as shown in the Examples.

It was further observed that increasing or generating the activity of a gene product with the activity of a "DNA-binding transcriptional dual regulator protein" encoded by a gene comprising the nucleic acid sequence SEQ ID NO.: 9193 in Arabidopsis thaliana conferred an increased biomass production compared with the wild type control without showing any symptoms of injury for a period between 0.4 and 2 days as shown in the Examples.

In particular, it was observed that increasing or generating the activity of a gene product with the activity of a "methylglyoxal synthase" encoded by a gene comprising the nucleic acid sequence SEQ ID NO.: 8497 in Arabidopsis thaliana conferred an increased drought resistance by surviving longer than the wild type control without showing any symptoms of injury for a period between 4.4 and 5 days as shown in the Examples.

It was further observed that increasing or generating the activity of a gene product with the activity of a "methylglyoxal synthase" encoded by a gene comprising the nucleic acid sequence SEQ ID NO.: 8497 in Arabidopsis thaliana conferred an increased biomass production compared with the wild type control without showing any symptoms of injury for a period between 0.5 and 2 days as shown in the Examples.

In particular, it was observed that increasing or generating the activity of a gene product with the activity of a "gamma-Glu-putrescine synthase" encoded by a gene comprising the nucleic acid sequence SEQ ID NO.: 8742 in Arabidopsis thaliana conferred an increased drought resistance by surviving longer than the wild type control without showing any symptoms of injury for a period between 2.1 and 3 days as shown in the Examples.

It was further observed that increasing or generating the activity of a gene product with the activity of a "gamma-Glu-putrescine synthase" encoded by a gene comprising the nucleic acid sequence SEQ ID NO.: 8742 in Arabidopsis thaliana conferred an increased biomass production compared with the wild type control without showing any symptoms of injury for a period between 1.1 and 2 days as shown in the Examples.

In particular, it was observed that increasing or generating the activity of a gene product with the activity of a "acid shock protein precursor" encoded by a gene comprising the nucleic acid sequence SEQ ID NO.: 8891 in Arabidopsis thaliana conferred an increased drought resistance by surviving longer than the wild type control without showing any symptoms of injury for a period between 3.7 and 5 days as shown in the Examples.

It was further observed that increasing or generating the activity of a gene product with the activity of a "acid shock protein precursor" encoded by a gene comprising the nucleic acid sequence SEQ ID NO.: 8891 in Arabidopsis thaliana conferred an increased biomass production compared with the wild type control without showing any symptoms of injury for a period between 0.1 and 1 days as shown in the Examples.

In particular, it was observed that increasing or generating the activity of a gene product with the activity of a "regulator of length of O-antigen component of lipopolysaccharide chains " encoded by a gene comprising the nucleic acid sequence SEQ ID NO.: 9031 in Arabidopsis thaliana conferred an increased drought resistance by surviving longer than the wild type control without showing any symptoms of injury for a period between 2.6 and 4 days as shown in the Examples.

It was further observed that increasing or generating the activity of a gene product with the activity of a "regulator of length of O-antigen component of lipopolysaccharide chains " encoded by a gene comprising the nucleic acid sequence SEQ ID NO.: 9031 in Arabidopsis thaliana conferred an increased biomass production compared with the wild type control without showing any symptoms of injury for a period between 0.3 and 2 days as shown in the Examples.

In particular, it was observed that increasing or generating the activity of a gene product with the activity of a "ornithine decarboxylase" encoded by a gene comprising the nucleic acid sequence SEQ ID NO.: 9315 in Arabidopsis thaliana conferred an increased drought resistance by surviving longer than the wild type control without showing any symptoms of injury for a period between 3.8 and 5 days as shown in the Examples.

It was further observed that increasing or generating the activity of a gene product with the activity of a "ornithine decarboxylase" encoded by a gene comprising the nucleic acid sequence SEQ ID NO.: 9315 in Arabidopsis thaliana conferred an increased biomass production compared with the wild type control without showing any symptoms of injury for a period between 0.7 and 3 days as shown in the Examples.

In particular, it was observed that increasing or generating the activity of a gene product with the activity of a "aspartate ammonia-lyase " encoded by a gene comprising the nucleic acid sequence SEQ ID NO.: 9529 in Arabidopsis thaliana conferred an increased drought resistance by surviving longer than the wild type control without showing any symptoms of injury for a period between 3.1 and 5 days as shown in the Examples.

It was further observed that increasing or generating the activity of a gene product with the activity of a "aspartate ammonia-lyase " encoded by a gene comprising the nucleic acid sequence SEQ ID NO.: 9529 in Arabidopsis thaliana conferred an increased biomass production compared with the wild type control without showing any symptoms of injury for a period between 1.6 and 4 days as shown in the Examples.

In particular, it was observed that increasing or generating the activity of a gene product with the activity of a "predicted transporter protein" encoded by a gene comprising the nucleic acid sequence SEQ ID NO.: 8462 in Arabidopsis thaliana conferred an increased drought resistance by surviving longer than the wild type control without showing any symptoms of injury for a period between 2.2 and 5 days as shown in the Examples.

It was further observed that increasing or generating the activity of a gene product with the activity of a "predicted transporter protein" encoded by a gene comprising the nucleic acid sequence SEQ ID NO.: 8462 in Arabidopsis thaliana conferred an increased biomass production compared with the wild type control without showing any symptoms of injury for a period between 1 and 4 days as shown in the Examples.

In particular, it was observed that increasing or generating the activity of a gene product with the activity of a "L-arabinose transporter subunit" encoded by a gene comprising the nucleic acid sequence SEQ ID NO.: 8973 in Arabidopsis thaliana conferred an increased drought resistance by surviving longer than the wild type control without showing any symptoms of injury for a period between 2.8 and 4 days as shown in the Examples.

It was further observed that increasing or generating the activity of a gene product with the activity of a "L-arabinose transporter subunit" encoded by a gene comprising the nucleic acid sequence SEQ ID NO.: 8973 in Arabidopsis thaliana conferred an increased biomass production compared with the wild type control without showing any symptoms of injury for a period between 1.8 and 3 days as shown in the Examples.

In particular, it was observed that increasing or generating the activity of a gene product with the activity of a "Lsm (Like Sm) protein" encoded by a gene comprising the nucleic acid sequence SEQ ID NO.: 9883 in Arabidopsis thaliana conferred an increased drought resistance by surviving longer than the wild type control without showing any symptoms of injury for a period between 3.1 and 4 days as shown in the Examples.

It was further observed that increasing or generating the activity of a gene product with the activity of a "Lsm (Like Sm) protein" encoded by a gene comprising the nucleic acid sequence SEQ ID NO.: 9883 in Arabidopsis thaliana conferred an increased biomass production compared with the wild type control without showing any symptoms of injury for a period between 0.8 and 2 days as shown in the Examples.

In particular, it was observed that increasing or generating the activity of a gene product with the activity of a "neutral amino-acid efflux system" encoded by a gene comprising the nucleic acid sequence SEQ ID NO.: 8934 in Arabidopsis thaliana conferred an increased drought resistance by surviving longer than the wild type control without showing any symptoms of injury for a period between 3.4 and 5 days as shown in the Examples.

It was further observed that increasing or generating the activity of a gene product with the activity of a "neutral amino-acid efflux system" encoded by a gene comprising the nucleic acid sequence SEQ ID NO.: 8934 in Arabidopsis thaliana conferred an increased biomass production compared with the wild type control without showing any symptoms of injury for a period between 1 and 4 days as shown in the Examples.

In particular, it was observed that increasing or generating the activity of a gene product with the activity of a "b2226-protein" encoded by a gene comprising the nucleic acid sequence SEQ ID NO.: 9093 in Arabidopsis thaliana conferred an increased drought resistance by surviving longer than the wild type control without showing any symptoms of injury for a period between 2.9 and 4 days as shown in the Examples.

It was further observed that increasing or generating the activity of a gene product with the activity of a "b2226-protein" encoded by a gene comprising the nucleic acid sequence SEQ ID NO.: 9093 in Arabidopsis thaliana conferred an increased biomass production compared with the wild type control without showing any symptoms of injury for a period between 1.6 and 4 days as shown in the Examples.

In particular, it was observed that increasing or generating the activity of a gene product with the activity of a "sensory histidine kinase in two-component regulatory system with NarP (NarL)" encoded by a gene comprising the nucleic acid sequence SEQ ID NO.: 9109 in Arabidopsis thaliana conferred an increased drought resistance by surviving longer than the wild type control without showing any symptoms of injury for a period between 2.2 and 5 days as shown in the Examples.

It was further observed that increasing or generating the activity of a gene product with the activity of a "sensory histidine kinase in two-component regulatory system with NarP (NarL)" encoded by a gene comprising the nucleic acid sequence SEQ ID NO.: 9109 in Arabidopsis thaliana conferred an increased biomass production compared with the wild type control without showing any symptoms of injury for a period between 0.9 and 3 days as shown in the Examples.

In particular, it was observed that increasing or generating the activity of a gene product with the activity of a "Glutamine tRNA synthetase" encoded by a gene comprising the nucleic acid sequence SEQ ID NO.: 9931 in Arabidopsis thaliana conferred an increased drought resistance by surviving longer than the wild type control without showing any symptoms of injury for a period between 3.1 and 5 days as shown in the Examples.

It was further observed that increasing or generating the activity of a gene product with the activity of a "Glutamine tRNA synthetase" encoded by a gene comprising the nucleic acid sequence SEQ ID NO.: 9931 in Arabidopsis thaliana conferred an increased biomass production compared with the wild type control without showing any symptoms of injury for a period between 0.6 and 2 days as shown in the Examples.

In particular, it was observed that increasing or generating the activity of a gene product with the activity of a "gluconate transporter" encoded by a gene comprising the nucleic acid sequence SEQ ID NO.: 10096 in Arabidopsis thaliana conferred an increased drought resistance by surviving longer than the wild type control without showing any symptoms of injury for a period between 2.5 and 4 days as shown in the Examples.

It was further observed that increasing or generating the activity of a gene product with the activity of a "gluconate transporter" encoded by a gene comprising the nucleic acid sequence SEQ ID NO.: 10096 in Arabidopsis thaliana conferred an increased biomass production compared with the wild type control without showing any symptoms of injury for a period between 0.8 and 3 days as shown in the Examples.

In particular, it was observed that increasing or generating the activity of a gene product with the activity of a "glutathione-dependent oxidoreductase" encoded by a gene comprising the nucleic acid sequence SEQ ID NO.: 8177 in Arabidopsis thaliana conferred an increased cold resistance by an increased biomass production compared with the wild type control of 38% to 45% as shown in the Examples.

In particular, it was observed that increasing or generating the activity of a gene product with the activity of a "glutathione-dependent oxidoreductase" encoded by a gene comprising the nucleic acid sequence SEQ ID NO.: 8177 in Arabidopsis thaliana conferred an increased resistance to cycling drought by an increased biomass production compared with the wild type control of 21% to 27% as shown in the Examples.

In particular, it was observed that increasing or generating the activity of a gene product with the activity of a "glutathione-dependent oxidoreductase" encoded by a gene comprising the nucleic acid sequence SEQ ID NO.: 8177 in Arabidopsis thaliana conferred an increased biomass production compared with the wild type control of 41% to 45% as shown in the Examples.

In particular, it was observed that increasing or generating the activity of a gene product with the activity of a "glutathione-dependent oxidoreductase" encoded by a gene comprising the nucleic acid sequence SEQ ID NO.: 8177 in Arabidopsis thaliana conferred an increased resistance to limited nitrogen availability by enhanced NUE by an increased biomass production compared with the wild type control of 30% to 60% as shown in the Examples.

Thus, according to the method of the invention for an increased tolerance and/or resistance to environmental stress and increased biomass production in a plant cell, plant or a part thereof compared to a control or wild type can be achieved.

Accordingly, in one embodiment, in case the activity of a polypeptide according to the polypeptide SEQ ID NO.: 39,or encoded by a nucleic acid molecule comprising the nucleic acid SEQ ID NO.: 38 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 38 or polypeptide SEQ ID NO.: 39, respectively is increased or generated or if the activity "b0081-protein" is increased or generated in an organism, preferably an increased drought resistance by surviving longer than the wild type control without showing any symptoms of injury for a period between 3.4 and 6 days or more and
an increased biomass production compared with the wild type control without showing any symptoms of injury for a period between 1.1 and 3 days
is conferred in said organism.

Accordingly, in one embodiment, in case the activity of a polypeptide according to the polypeptide SEQ ID NO.: 55,or encoded by a nucleic acid molecule comprising the nucleic acid SEQ ID NO.: 54 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 54 or polypeptide SEQ ID NO.: 55, respectively is increased or generated or if the activity "transporter subunit / periplasmic-binding component of ABC superfamily" is increased or generated in an organism, preferably
an increased drought resistance by surviving longer than the wild type control without showing any symptoms of injury for a period between 3 and 5 days or more and
an increased biomass production compared with the wild type control without showing any symptoms of injury for a period between 0.9 and 4 days
is conferred in said organism.

Accordingly, in one embodiment, in case the activity of a polypeptide according to the polypeptide SEQ ID NO.: 71 ,or encoded by a nucleic acid molecule comprising the nucleic acid SEQ ID NO.: 70 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 70 or polypeptide SEQ ID NO.: 71, respectively is increased or generated or if the activity "b0482-protein" is increased or generated in an organism, preferably an increased drought resistance by surviving longer than the wild type control without showing any symptoms of injury for a period between 2.8 and 5 days or more and
an increased biomass production compared with the wild type control without showing any symptoms of injury for a period between 0.6 and 3 days
is conferred in said organism.

Accordingly, in one embodiment, in case the activity of a polypeptide according to the polypeptide SEQ ID NO.: 90,or encoded by a nucleic acid molecule comprising the nucleic acid SEQ ID NO.: 89 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 89 or polypeptide SEQ ID NO.: 90, respectively is increased or generated or if the activity "universal stress protein UP12" is increased or generated in an organism, preferably
an increased drought resistance by surviving longer than the wild type control without showing any symptoms of injury for a period between 2.9 and 5 days or more and
an increased biomass production compared with the wild type control without showing any symptoms of injury for a period between 0.9 and 4 days
is conferred in said organism.

Accordingly, in one embodiment, in case the activity of a polypeptide according to the polypeptide SEQ ID NO.: 144,or encoded by a nucleic acid molecule comprising the nucleic acid SEQ ID NO.: 143 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 143 or polypeptide SEQ ID NO.: 144, respectively is increased or generated or if the activity "transcriptional regulator protein" is increased or generated in an organism, preferably
an increased drought resistance by surviving longer than the wild type control without showing any symptoms of injury for a period between 2.8 and 5 days or more and
an increased biomass production compared with the wild type control without showing any symptoms of injury for a period between 0.8 and 4 days
is conferred in said organism.

Accordingly, in one embodiment, in case the activity of a polypeptide according to the polypeptide SEQ ID NO.: 163,or encoded by a nucleic acid molecule comprising the nucleic acid SEQ ID NO.: 162 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 162 or polypeptide SEQ ID NO.: 163, respectively is increased or generated or if the activity "b0631-protein" is increased or generated in an organism, preferably
an increased drought resistance by surviving longer than the wild type control without showing any symptoms of injury for a period between 3.4 and 6 days or more and
an increased biomass production compared with the wild type control without showing any symptoms of injury for a period between 1.2 and 5 days
is conferred in said organism.

Accordingly, in one embodiment, in case the activity of a polypeptide according to the polypeptide SEQ ID NO.: 214,or encoded by a nucleic acid molecule comprising the nucleic acid SEQ ID NO.: 213 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 213 or polypeptide SEQ ID NO.: 214, respectively is increased or generated or if the activity "potassium-transporting ATPase (subunit B)" is increased or generated in an organism, preferably
an increased drought resistance by surviving longer than the wild type control without showing any symptoms of injury for a period between 2.1 and 3 days or more and
an increased biomass production compared with the wild type control without showing any symptoms of injury for a period between 1.8 and 3 days
is conferred in said organism.

Accordingly, in one embodiment, in case the activity of a polypeptide according to the polypeptide SEQ ID NO.: 359,or encoded by a nucleic acid molecule comprising the nucleic acid SEQ ID NO.: 358 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 358 or polypeptide SEQ ID NO.: 359, respectively is increased or generated or if the activity "b0753-protein" is increased or generated in an organism, preferably
an increased drought resistance by surviving longer than the wild type control without showing any symptoms of injury for a period between 3.3 and 5 days or more and
an increased biomass production compared with the wild type control without showing any symptoms of injury for a period between 1.6 and 4 days
is conferred in said organism.

Accordingly, in one embodiment, in case the activity of a polypeptide according to the polypeptide SEQ ID NO.: 368,or encoded by a nucleic acid molecule comprising the nucleic acid SEQ ID NO.: 367 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 367 or polypeptide SEQ ID NO.: 368, respectively is increased or generated or if the activity "threonine and homoserine efflux system" is increased or generated in an organism, preferably
an increased drought resistance by surviving longer than the wild type control without showing any symptoms of injury for a period between 3 and 5 days or more and
an increased biomass production compared with the wild type control without showing any symptoms of injury for a period between 1.5 and 3 days
is conferred in said organism.

Accordingly, in one embodiment, in case the activity of a polypeptide according to the polypeptide SEQ ID NO.: 421 ,or encoded by a nucleic acid molecule comprising the nucleic acid SEQ ID NO.: 420 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 420 or polypeptide SEQ ID NO.: 421, respectively is increased or generated or if the activity "predicted transporter protein" is increased or generated in an organism, preferably
an increased drought resistance by surviving longer than the wild type control without showing any symptoms of injury for a period between 2.2 and 5 days or more and
an increased biomass production compared with the wild type control without showing any symptoms of injury for a period between 1 and 4 days
is conferred in said organism.

Accordingly, in one embodiment, in case the activity of a polypeptide according to the polypeptide SEQ ID NO.: 456,or encoded by a nucleic acid molecule comprising the nucleic acid SEQ ID NO.: 455 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 455 or polypeptide SEQ ID NO.: 456, respectively is increased or generated or if the activity "b0866-protein" is increased or generated in an organism, preferably
an increased drought resistance by surviving longer than the wild type control without showing any symptoms of injury for a period between 3 and 5 days or more and
an increased biomass production compared with the wild type control without showing
any symptoms of injury for a period between 0.8 and 5 days
is conferred in said organism.

Accordingly, in one embodiment, in case the activity of a polypeptide according to the polypeptide SEQ ID NO.: 536,or encoded by a nucleic acid molecule comprising the nucleic acid SEQ ID NO.: 535 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 535 or polypeptide SEQ ID NO.: 536, respectively is increased or generated or if the activity "methylglyoxal synthase" is increased or generated in an organism, preferably
an increased drought resistance by surviving longer than the wild type control without showing any symptoms of injury for a period between 4.4 and 5 days or more and
an increased biomass production compared with the wild type control without showing any symptoms of injury for a period between 0.5 and 2 days
is conferred in said organism.

Accordingly, in one embodiment, in case the activity of a polypeptide according to the polypeptide SEQ ID NO.: 619,or encoded by a nucleic acid molecule comprising the nucleic acid SEQ ID NO.: 618 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 618 or polypeptide SEQ ID NO.: 619, respectively is increased or generated or if the activity "HyaA/HyaB-processing protein" is increased or generated in an organism, preferably
an increased drought resistance by surviving longer than the wild type control without showing any symptoms of injury for a period between 2.6 and 4 days or more and
an increased biomass production compared with the wild type control without showing any symptoms of injury for a period between 1.2 and 4 days
is conferred in said organism.

Accordingly, in one embodiment, in case the activity of a polypeptide according to the polypeptide SEQ ID NO.: 672,or encoded by a nucleic acid molecule comprising the nucleic acid SEQ ID NO.: 671 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 671 or polypeptide SEQ ID NO.: 672, respectively is increased or generated or if the activity "predicted oxidoreductase (flavin:NADH component)" is increased or generated in an organism, preferably
an increased drought resistance by surviving longer than the wild type control without showing any symptoms of injury for a period between 3.3 and 6 days or more and
an increased biomass production compared with the wild type control without showing any symptoms of injury for a period between 0.6 and 2 days
is conferred in said organism.

Accordingly, in one embodiment, in case the activity of a polypeptide according to the polypeptide SEQ ID NO.: 765,or encoded by a nucleic acid molecule comprising the nucleic acid SEQ ID NO.: 764 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 764 or polypeptide SEQ ID NO.: 765, respectively is increased or generated or if the activity "b1052-protein" is increased or generated in an organism, preferably
an increased drought resistance by surviving longer than the wild type control without showing any symptoms of injury for a period between 3 and 5 days or more and
an increased biomass production compared with the wild type control without showing any symptoms of injury for a period between 0.4 and 2 days
is conferred in said organism.

Accordingly, in one embodiment, in case the activity of a polypeptide according to the polypeptide SEQ ID NO.: 769,or encoded by a nucleic acid molecule comprising the nucleic acid SEQ ID NO.: 768 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 768 or polypeptide SEQ ID NO.: 769, respectively is increased or generated or if the activity "3-oxoacyl-(acyl carrier protein) synthase" is increased or generated in an organism, preferably
an increased drought resistance by surviving longer than the wild type control without showing any symptoms of injury for a period between 3.4 and 5 days or more and
an increased biomass production compared with the wild type control without showing any symptoms of injury for a period between 0.3 and 3 days
is conferred in said organism.

Accordingly, in one embodiment, in case the activity of a polypeptide according to the polypeptide SEQ ID NO.: 908,or encoded by a nucleic acid molecule comprising the nucleic acid SEQ ID NO.: 907 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 907 or polypeptide SEQ ID NO.: 908, respectively is increased or generated or if the activity "b1161-protein" is increased or generated in an organism, preferably
an increased drought resistance by surviving longer than the wild type control without showing any symptoms of injury for a period between 2.9 and 4 days or more and
an increased biomass production compared with the wild type control without showing any symptoms of injury for a period between 2 and 4 days
is conferred in said organism.

Accordingly, in one embodiment, in case the activity of a polypeptide according to the polypeptide SEQ ID NO.: 928,or encoded by a nucleic acid molecule comprising the nucleic acid SEQ ID NO.: 927 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 927 or polypeptide SEQ ID NO.: 928, respectively is increased or generated or if the activity "sodium/proton antiporter" is increased or generated in an organism, preferably
an increased drought resistance by surviving longer than the wild type control without showing any symptoms of injury for a period between 4 and 6 days or more and
an increased biomass production compared with the wild type control without showing any symptoms of injury for a period between 0.8 and 4 days
is conferred in said organism.

Accordingly, in one embodiment, in case the activity of a polypeptide according to the polypeptide SEQ ID NO.: 1010,or encoded by a nucleic acid molecule comprising the nucleic acid SEQ ID NO.: 1009 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1009 or polypeptide SEQ ID NO.: 1010, respectively is increased or generated or if the activity "predicted antimicrobial peptide transporter subunit" is increased or generated in an organism, preferably
an increased drought resistance by surviving longer than the wild type control without showing any symptoms of injury for a period between 3.4 and 5 days or more and
an increased biomass production compared with the wild type control without showing any symptoms of injury for a period between 1.2 and 3 days
is conferred in said organism.

Accordingly, in one embodiment, in case the activity of a polypeptide according to the polypeptide SEQ ID NO.: 1155,or encoded by a nucleic acid molecule comprising the nucleic acid SEQ ID NO.: 1154 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1154 or polypeptide SEQ ID NO.: 1155, respectively is increased or generated or if the activity "predicted antimicrobial peptide transporter subunit" is increased or generated in an organism, preferably
an increased drought resistance by surviving longer than the wild type control without showing any symptoms of injury for a period between 2.3 and 5 days or more and
an increased biomass production compared with the wild type control without showing any symptoms of injury for a period between 1 and 4 days
is conferred in said organism.

Accordingly, in one embodiment, in case the activity of a polypeptide according to the polypeptide SEQ ID NO.: 1309,or encoded by a nucleic acid molecule comprising the nucleic acid SEQ ID NO.: 1308 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1308 or polypeptide SEQ ID NO.: 1309, respectively is increased or generated or if the activity "b1423-protein" is increased or generated in an organism, preferably
an increased drought resistance by surviving longer than the wild type control without showing any symptoms of injury for a period between 3.1 and 6 days or more and
an increased biomass production compared with the wild type control without showing any symptoms of injury for a period between 0.1 and 1 days
is conferred in said organism.

Accordingly, in one embodiment, in case the activity of a polypeptide according to the polypeptide SEQ ID NO.: 1369,or encoded by a nucleic acid molecule comprising the nucleic acid SEQ ID NO.: 1368 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1368 or polypeptide SEQ ID NO.: 1369, respectively is increased or generated or if the activity "acid shock protein precursor" is increased or generated in an organism, preferably
an increased drought resistance by surviving longer than the wild type control without showing any symptoms of injury for a period between 3.7 and 5 days or more and
an increased biomass production compared with the wild type control without showing any symptoms of injury for a period between 0.1 and 1 days
is conferred in said organism.

Accordingly, in one embodiment, in case the activity of a polypeptide according to the polypeptide SEQ ID NO.: 1375,or encoded by a nucleic acid molecule comprising the nucleic acid SEQ ID NO.: 1374 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1374 or polypeptide SEQ ID NO.: 1375, respectively is increased or generated or if the activity "predicted arginine/ornithine transporter" is increased or generated in an organism, preferably
an increased drought resistance by surviving longer than the wild type control without showing any symptoms of injury for a period between 2.8 and 5 days or more and
an increased biomass production compared with the wild type control without showing any symptoms of injury for a period between 0.9 and 3 days
is conferred in said organism.

Accordingly, in one embodiment, in case the activity of a polypeptide according to the polypeptide SEQ ID NO.: 1508,or encoded by a nucleic acid molecule comprising the nucleic acid SEQ ID NO.: 1507 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1507 or polypeptide SEQ ID NO.: 1508, respectively is increased or generated or if the activity "3-deoxy-D-arabino-heptulosonate-7-phosphatesynthase" is increased or generated in an organism, preferably
an increased drought resistance by surviving longer than the wild type control without showing any symptoms of injury for a period between 4.2 and 5 days or more and
an increased biomass production compared with the wild type control without showing any symptoms of injury for a period between 0.6 and 2 days
is conferred in said organism.

Accordingly, in one embodiment, in case the activity of a polypeptide according to the polypeptide SEQ ID NO.: 1954,or encoded by a nucleic acid molecule comprising the nucleic acid SEQ ID NO.: 1953 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1953 or polypeptide SEQ ID NO.: 1954, respectively is increased or generated or if the activity "N,N'-diacetylchitobiose-specific enzyme IIA component of PTS" is increased or generated in an organism, preferably
an increased drought resistance by surviving longer than the wild type control without showing any symptoms of injury for a period between 4.1 and 5 days or more and
an increased biomass production compared with the wild type control without showing any symptoms of injury for a period between 0.8 and 2 days
is conferred in said organism.

Accordingly, in one embodiment, in case the activity of a polypeptide according to the polypeptide SEQ ID NO.: 2157,or encoded by a nucleic acid molecule comprising the nucleic acid SEQ ID NO.: 2156 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2156 or polypeptide SEQ ID NO.: 2157, respectively is increased or generated or if the activity "neutral amino-acid efflux system" is increased or generated in an organism, preferably
an increased drought resistance by surviving longer than the wild type control without showing any symptoms of injury for a period between 3.4 and 5 days or more and
an increased biomass production compared with the wild type control without showing any symptoms of injury for a period between 1 and 4 days
is conferred in said organism.

Accordingly, in one embodiment, in case the activity of a polypeptide according to the polypeptide SEQ ID NO.: 2196,or encoded by a nucleic acid molecule comprising the nucleic acid SEQ ID NO.: 2195 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2195 or polypeptide SEQ ID NO.: 2196, respectively is increased or generated or if the activity "b1878-protein" is increased or generated in an organism, preferably
an increased drought resistance by surviving longer than the wild type control without showing any symptoms of injury for a period between 3.4 and 5 days or more and
an increased biomass production compared with the wild type control without showing any symptoms of injury for a period between 1.3 and 3 days
is conferred in said organism.

Accordingly, in one embodiment, in case the activity of a polypeptide according to the polypeptide SEQ ID NO.: 2220,or encoded by a nucleic acid molecule comprising the nucleic acid SEQ ID NO.: 2219 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2219 or polypeptide SEQ ID NO.: 2220, respectively is increased or generated or if the activity "L-arabinose transporter subunit" is increased or generated in an organism, preferably
an increased drought resistance by surviving longer than the wild type control without showing any symptoms of injury for a period between 2.8 and 4 days or more and
an increased biomass production compared with the wild type control without showing any symptoms of injury for a period between 1.8 and 3 days
is conferred in said organism.

Accordingly, in one embodiment, in case the activity of a polypeptide according to the polypeptide SEQ ID NO.: 2278,or encoded by a nucleic acid molecule comprising the nucleic acid SEQ ID NO.: 2277 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2277 or polypeptide SEQ ID NO.: 2278, respectively is increased or generated or if the activity "phosphatidylglycerophosphate synthetase" is increased or generated in an organism, preferably
an increased drought resistance by surviving longer than the wild type control without showing any symptoms of injury for a period between 2.5 and 4 days or more and
an increased biomass production compared with the wild type control without showing any symptoms of injury for a period between 1.6 and 3 days
is conferred in said organism.

Accordingly, in one embodiment, in case the activity of a polypeptide according to the polypeptide SEQ ID NO.: 2471,or encoded by a nucleic acid molecule comprising the nucleic acid SEQ ID NO.: 2470 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2470 or polypeptide SEQ ID NO.: 2471, respectively is increased or generated or if the activity "regulator of length of O-antigen component of lipopolysaccharide chains" is increased or generated in an organism, preferably
an increased drought resistance by surviving longer than the wild type control without showing any symptoms of injury for a period between 2.6 and 4 days or more and
an increased biomass production compared with the wild type control without showing any symptoms of injury for a period between 0.3 and 2 days
is conferred in said organism.

Accordingly, in one embodiment, in case the activity of a polypeptide according to the polypeptide SEQ ID NO.: 2494,or encoded by a nucleic acid molecule comprising the nucleic acid SEQ ID NO.: 2493 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2493 or polypeptide SEQ ID NO.: 2494, respectively is increased or generated or if the activity "glucose-1-phosphate thymidylyltransferase" is increased or generated in an organism, preferably
an increased drought resistance by surviving longer than the wild type control without showing any symptoms of injury for a period between 2.1 and 4 days or more and
an increased biomass production compared with the wild type control without showing any symptoms of injury for a period between 0.1 and 1 days
is conferred in said organism.

Accordingly, in one embodiment, in case the activity of a polypeptide according to the polypeptide SEQ ID NO.: 2628,or encoded by a nucleic acid molecule comprising the nucleic acid SEQ ID NO.: 2627 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2627 or polypeptide SEQ ID NO.: 2628, respectively is increased or generated or if the activity "multidrug efflux system (subunit B)" is increased or generated in an organism, preferably
an increased drought resistance by surviving longer than the wild type control without showing any symptoms of injury for a period between 2.5 and 4 days or more and
an increased biomass production compared with the wild type control without showing any symptoms of injury for a period between 0.5 and 3 days
is conferred in said organism.

Accordingly, in one embodiment, in case the activity of a polypeptide according to the polypeptide SEQ ID NO.: 2859,or encoded by a nucleic acid molecule comprising the nucleic acid SEQ ID NO.: 2858 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2858 or polypeptide SEQ ID NO.: 2859, respectively is increased or generated or if the activity "GTP cyclohydrolase I" is increased or generated in an organism, preferably
an increased drought resistance by surviving longer than the wild type control without showing any symptoms of injury for a period between 2.5 and 4 days or more and
an increased biomass production compared with the wild type control without showing any symptoms of injury for a period between 1.5 and 4 days
is conferred in said organism.

Accordingly, in one embodiment, in case the activity of a polypeptide according to the polypeptide SEQ ID NO.: 2943,or encoded by a nucleic acid molecule comprising the nucleic acid SEQ ID NO.: 2942 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2942 or polypeptide SEQ ID NO.: 2943, respectively is increased or generated or if the activity "heme lyase (CcmH subunit)" is increased or generated in an organism, preferably
an increased drought resistance by surviving longer than the wild type control without showing any symptoms of injury for a period between 3.4 and 6 days or more and
an increased biomass production compared with the wild type control without showing any symptoms of injury for a period between 0.7 and 3 days
is conferred in said organism.

Accordingly, in one embodiment, in case the activity of a polypeptide according to the polypeptide SEQ ID NO.: 2966,or encoded by a nucleic acid molecule comprising the nucleic acid SEQ ID NO.: 2965 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2965 or polypeptide SEQ ID NO.: 2966, respectively is increased or generated or if the activity "b2226-protein" is increased or generated in an organism, preferably
an increased drought resistance by surviving longer than the wild type control without showing any symptoms of injury for a period between 2.9 and 4 days or more and
an increased biomass production compared with the wild type control without showing any symptoms of injury for a period between 1.6 and 4 days
is conferred in said organism.

Accordingly, in one embodiment, in case the activity of a polypeptide according to the polypeptide SEQ ID NO.: 2982,or encoded by a nucleic acid molecule comprising the nucleic acid SEQ ID NO.: 2981 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 2981 or polypeptide SEQ ID NO.: 2982, respectively is increased or generated or if the activity "histidine/lysine/arginine/ornithine transporter subunit protein" is increased or generated in an organism, preferably
an increased drought resistance by surviving longer than the wild type control without showing any symptoms of injury for a period between 1.9 and 3 days or more and
an increased biomass production compared with the wild type control without showing any symptoms of injury for a period between 0.9 and 3 days
is conferred in said organism.

Accordingly, in one embodiment, in case the activity of a polypeptide according to the polypeptide SEQ ID NO.: 3131 ,or encoded by a nucleic acid molecule comprising the nucleic acid SEQ ID NO.: 3130 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3130 or polypeptide SEQ ID NO.: 3131, respectively is increased or generated or if the activity "sensory histidine kinase in two-component regulatory system with NarP (NarL)" is increased or generated in an organism, preferably
an increased drought resistance by surviving longer than the wild type control without showing any symptoms of injury for a period between 2.2 and 5 days or more and
an increased biomass production compared with the wild type control without showing any symptoms of injury for a period between 0.9 and 3 days
is conferred in said organism.

Accordingly, in one embodiment, in case the activity of a polypeptide according to the polypeptide SEQ ID NO.: 3217,or encoded by a nucleic acid molecule comprising the nucleic acid SEQ ID NO.: 3216 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3216 or polypeptide SEQ ID NO.: 3217, respectively is increased or generated or if the activity "b2475-protein" is increased or generated in an organism, preferably
an increased drought resistance by surviving longer than the wild type control without showing any symptoms of injury for a period between 2.9 and 5 days or more and
an increased biomass production compared with the wild type control without showing any symptoms of injury for a period between 0.3 and 2 days
is conferred in said organism.

Accordingly, in one embodiment, in case the activity of a polypeptide according to the polypeptide SEQ ID NO.: 3336,or encoded by a nucleic acid molecule comprising the nucleic acid SEQ ID NO.: 3335 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3335 or polypeptide SEQ ID NO.: 3336, respectively is increased or generated or if the activity "NADH dehydrogenase (subunit N)" is increased or generated in an organism, preferably
an increased drought resistance by surviving longer than the wild type control without showing any symptoms of injury for a period between 1.9 and 3 days or more and
an increased biomass production compared with the wild type control without showing any symptoms of injury for a period between 0.6 and 2 days
is conferred in said organism.

Accordingly, in one embodiment, in case the activity of a polypeptide according to the polypeptide SEQ ID NO.: 3402,or encoded by a nucleic acid molecule comprising the nucleic acid SEQ ID NO.: 3401 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3401 or polypeptide SEQ ID NO.: 3402, respectively is increased or generated or if the activity "2,3-dihydroxy-2,3-dihydrophenylpropionatedehydrogenase" is increased or generated in an organism, preferably
an increased drought resistance by surviving longer than the wild type control without showing any symptoms of injury for a period between 2.9 and 5 days or more and
an increased biomass production compared with the wild type control without showing any symptoms of injury for a period between 0.6 and 2 days
is conferred in said organism.

Accordingly, in one embodiment, in case the activity of a polypeptide according to the polypeptide SEQ ID NO.: 3591 ,or encoded by a nucleic acid molecule comprising the nucleic acid SEQ ID NO.: 3590 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3590 or polypeptide SEQ ID NO.: 3591, respectively is increased or generated or if the activity "tRNA-specific adenosine deaminase" is increased or generated in an organism, preferably
an increased drought resistance by surviving longer than the wild type control without showing any symptoms of injury for a period between 1.6 and 3 days or more and
an increased biomass production compared with the wild type control without showing any symptoms of injury for a period between 1.4 and 3 days
is conferred in said organism.

Accordingly, in one embodiment, in case the activity of a polypeptide according to the polypeptide SEQ ID NO.: 3832,or encoded by a nucleic acid molecule comprising the nucleic acid SEQ ID NO.: 3831 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3831 or polypeptide SEQ ID NO.: 3832, respectively is increased or generated or if the activity "predicted outer membrane lipoprotein" is increased or generated in an organism, preferably
an increased drought resistance by surviving longer than the wild type control without showing any symptoms of injury for a period between 1.6 and 4 days or more and
an increased biomass production compared with the wild type control without showing any symptoms of injury for a period between 0.7 and 2 days
is conferred in said organism.

Accordingly, in one embodiment, in case the activity of a polypeptide according to the polypeptide SEQ ID NO.: 3858,or encoded by a nucleic acid molecule comprising the nucleic acid SEQ ID NO.: 3857 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3857 or polypeptide SEQ ID NO.: 3858, respectively is increased or generated or if the activity "CP4-57 prophage/ RNase LS" is increased or generated in an organism, preferably
an increased drought resistance by surviving longer than the wild type control without showing any symptoms of injury for a period between 1.8 and 3 days or more and
an increased biomass production compared with the wild type control without showing any symptoms of injury for a period between 0.6 and 2 days
is conferred in said organism.

Accordingly, in one embodiment, in case the activity of a polypeptide according to the polypeptide SEQ ID NO.: 3862,or encoded by a nucleic acid molecule comprising the nucleic acid SEQ ID NO.: 3861 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 3861 or polypeptide SEQ ID NO.: 3862, respectively is increased or generated or if the activity "glycine betaine transporter subunit protein" is increased or generated in an organism, preferably
an increased drought resistance by surviving longer than the wild type control without showing any symptoms of injury for a period between 4.3 and 5 days or more and
an increased biomass production compared with the wild type control without showing any symptoms of injury for a period between 0.1 and 1 days
is conferred in said organism.

Accordingly, in one embodiment, in case the activity of a polypeptide according to the polypeptide SEQ ID NO.: 4023,or encoded by a nucleic acid molecule comprising the nucleic acid SEQ ID NO.: 4022 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4022 or polypeptide SEQ ID NO.: 4023, respectively is increased or generated or if the activity "cellobiose/arbutin/salicin-specific PTS enzyme (IIB component/IC component)" is increased or generated in an organism, preferably
an increased drought resistance by surviving longer than the wild type control without showing any symptoms of injury for a period between 4 and 5 days or more and
an increased biomass production compared with the wild type control without showing any symptoms of injury for a period between 0.4 and 2 days
is conferred in said organism.

Accordingly, in one embodiment, in case the activity of a polypeptide according to the polypeptide SEQ ID NO.: 4060,or encoded by a nucleic acid molecule comprising the nucleic acid SEQ ID NO.: 4059 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4059 or polypeptide SEQ ID NO.: 4060, respectively is increased or generated or if the activity "predicted kinase" is increased or generated in an organism, preferably
an increased drought resistance by surviving longer than the wild type control without showing any symptoms of injury for a period between 2.9 and 5 days or more and
an increased biomass production compared with the wild type control without showing any symptoms of injury for a period between 0.4 and 2 days
is conferred in said organism.

Accordingly, in one embodiment, in case the activity of a polypeptide according to the polypeptide SEQ ID NO.: 4077,or encoded by a nucleic acid molecule comprising the nucleic acid SEQ ID NO.: 4076 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4076 or polypeptide SEQ ID NO.: 4077, respectively is increased or generated or if the activity "tRNA pseudouridine synthase" is increased or generated in an
organism, preferably
an increased drought resistance by surviving longer than the wild type control without showing any symptoms of injury for a period between 3.2 and 6 days or more and
an increased biomass production compared with the wild type control without showing any symptoms of injury for a period between 0.7 and 3 days
is conferred in said organism.

Accordingly, in one embodiment, in case the activity of a polypeptide according to the polypeptide SEQ ID NO.: 4158,or encoded by a nucleic acid molecule comprising the nucleic acid SEQ ID NO.: 4157 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4157 or polypeptide SEQ ID NO.: 4158, respectively is increased or generated or if the activity "predicted ligase" is increased or generated in an organism, preferably
an increased drought resistance by surviving longer than the wild type control without showing any symptoms of injury for a period between 3 and 5 days or more and
an increased biomass production compared with the wild type control without showing any symptoms of injury for a period between 1.7 and 3 days
is conferred in said organism.

Accordingly, in one embodiment, in case the activity of a polypeptide according to the polypeptide SEQ ID NO.: 4261 ,or encoded by a nucleic acid molecule comprising the nucleic acid SEQ ID NO.: 4260 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4260 or polypeptide SEQ ID NO.: 4261, respectively is increased or generated or if the activity "ornithine decarboxylase" is increased or generated in an organism, preferably
an increased drought resistance by surviving longer than the wild type control without showing any symptoms of injury for a period between 3.8 and 5 days or more and
an increased biomass production compared with the wild type control without showing any symptoms of injury for a period between 0.7 and 3 days
is conferred in said organism.

Accordingly, in one embodiment, in case the activity of a polypeptide according to the polypeptide SEQ ID NO.: 4351 ,or encoded by a nucleic acid molecule comprising the nucleic acid SEQ ID NO.: 4350 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4350 or polypeptide SEQ ID NO.: 4351, respectively is increased or generated or if the activity "phosphate transporter" is increased or generated in an organism, preferably
an increased drought resistance by surviving longer than the wild type control without showing any symptoms of injury for a period between 2.9 and 4 days or more and
an increased biomass production compared with the wild type control without showing any symptoms of injury for a period between 1.3 and 4 days
is conferred in said organism.

Accordingly, in one embodiment, in case the activity of a polypeptide according to the polypeptide SEQ ID NO.: 4351 ,or encoded by a nucleic acid molecule comprising the nucleic acid SEQ ID NO.: 4350 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4350 or polypeptide SEQ ID NO.: 4351, respectively is increased or generated or if the activity "phosphate transporter" is increased or generated in an organism, preferably
an increased drought resistance by surviving longer than the wild type control without showing any symptoms of injury for a period between 2.7 and 4 days or more and
an increased biomass production compared with the wild type control without showing any symptoms of injury for a period between 0.1 and 0.1 days
is conferred in said organism.

Accordingly, in one embodiment, in case the activity of a polypeptide according to the polypeptide SEQ ID NO.: 4460,or encoded by a nucleic acid molecule comprising the nucleic acid SEQ ID NO.: 4459 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4459 or polypeptide SEQ ID NO.: 4460, respectively is increased or generated or if the activity "hexuronate transporter" is increased or generated in an organism, preferably
an increased drought resistance by surviving longer than the wild type control without showing any symptoms of injury for a period between 2.8 and 5 days or more and
an increased biomass production compared with the wild type control without showing any symptoms of injury for a period between 1 and 3 days
is conferred in said organism.

Accordingly, in one embodiment, in case the activity of a polypeptide according to the polypeptide SEQ ID NO.: 4506,or encoded by a nucleic acid molecule comprising the nucleic acid SEQ ID NO.: 4505 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4505 or polypeptide SEQ ID NO.: 4506, respectively is increased or generated or if the activity "peptidyl-prolyl cis-trans isomerase A (rotamase A)" is increased or generated in an organism, preferably
an increased drought resistance by surviving longer than the wild type control without showing any symptoms of injury for a period between 3.7 and 4 days or more and
an increased biomass production compared with the wild type control without showing any symptoms of injury for a period between 0.1 and 0.1 days
is conferred in said organism.

Accordingly, in one embodiment, in case the activity of a polypeptide according to the polypeptide SEQ ID NO.: 4641 ,or encoded by a nucleic acid molecule comprising the nucleic acid SEQ ID NO.: 4640 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4640 or polypeptide SEQ ID NO.: 4641, respectively is increased or generated or if the activity "glycogen synthase" is increased or generated in an organism, preferably
an increased drought resistance by surviving longer than the wild type control without showing any symptoms of injury for a period between 2.8 and 4 days or more and
an increased biomass production compared with the wild type control without showing any symptoms of injury for a period between 0.6 and 2 days
is conferred in said organism.

Accordingly, in one embodiment, in case the activity of a polypeptide according to the polypeptide SEQ ID NO.: 4807,or encoded by a nucleic acid molecule comprising the nucleic acid SEQ ID NO.: 4806 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 4806 or polypeptide SEQ ID NO.: 4807, respectively is increased or generated or if the activity "D-xylose transporter subunit" is increased or generated in an organism, preferably
an increased drought resistance by surviving longer than the wild type control without showing any symptoms of injury for a period between 2.7 and 4 days or more and
an increased biomass production compared with the wild type control without showing any symptoms of injury for a period between 0.8 and 3 days
is conferred in said organism.

Accordingly, in one embodiment, in case the activity of a polypeptide according to the polypeptide SEQ ID NO.: 5125,or encoded by a nucleic acid molecule comprising the nucleic acid SEQ ID NO.: 5124 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5124 or polypeptide SEQ ID NO.: 5125, respectively is increased or generated or if the activity "L-threonine 3-dehydrogenase" is increased or generated in an organism, preferably
an increased drought resistance by surviving longer than the wild type control without showing any symptoms of injury for a period between 2.5 and 5 days or more and
an increased biomass production compared with the wild type control without showing any symptoms of injury for a period between 1.1 and 5 days
is conferred in said organism.

Accordingly, in one embodiment, in case the activity of a polypeptide according to the polypeptide SEQ ID NO.: 5125,or encoded by a nucleic acid molecule comprising the nucleic acid SEQ ID NO.: 5124 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5124 or polypeptide SEQ ID NO.: 5125, respectively is increased or generated or if the activity "L-threonine 3-dehydrogenase" is increased or generated in an organism, preferably
an increased drought resistance by surviving longer than the wild type control without showing any symptoms of injury for a period between 2.8 and 5 days or more and
an increased biomass production compared with the wild type control without showing any symptoms of injury for a period between 1.3 and 3 days
is conferred in said organism.

Accordingly, in one embodiment, in case the activity of a polypeptide according to the polypeptide SEQ ID NO.: 5418,or encoded by a nucleic acid molecule comprising the nucleic acid SEQ ID NO.: 5417 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5417 or polypeptide SEQ ID NO.: 5418, respectively is increased or generated or if the activity "predicted hydrolase" is increased or generated in an organism, preferably
an increased drought resistance by surviving longer than the wild type control without showing any symptoms of injury for a period between 3 and 5 days or more and
an increased biomass production compared with the wild type control without showing any symptoms of injury for a period between 0.9 and 3 days
is conferred in said organism.

Accordingly, in one embodiment, in case the activity of a polypeptide according to the polypeptide SEQ ID NO.: 5496,or encoded by a nucleic acid molecule comprising the nucleic acid SEQ ID NO.: 5495 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5495 or polypeptide SEQ ID NO.: 5496, respectively is increased or generated or if the activity "predicted PTS enzymes (IIB component/IIC component)" is increased or generated in an organism, preferably
an increased drought resistance by surviving longer than the wild type control without showing any symptoms of injury for a period between 2.5 and 4 days or more and
an increased biomass production compared with the wild type control without showing any symptoms of injury for a period between 0.8 and 3 days
is conferred in said organism.

Accordingly, in one embodiment, in case the activity of a polypeptide according to the polypeptide SEQ ID NO.: 5586,or encoded by a nucleic acid molecule comprising the nucleic acid SEQ ID NO.: 5585 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5585 or polypeptide SEQ ID NO.: 5586, respectively is increased or generated or if the activity "ribonuclease activity regulator protein RraA" is increased or generated in an organism, preferably
an increased drought resistance by surviving longer than the wild type control without showing any symptoms of injury for a period between 2.3 and 4 days or more and
an increased biomass production compared with the wild type control without showing any symptoms of injury for a period between 0.7 and 3 days
is conferred in said organism.

Accordingly, in one embodiment, in case the activity of a polypeptide according to the polypeptide SEQ ID NO.: 5801 ,or encoded by a nucleic acid molecule comprising the nucleic acid SEQ ID NO.: 5800 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5800 or polypeptide SEQ ID NO.: 5801, respectively is increased or generated or if the activity "transcriptional repressor protein MetJ" is increased or generated in an organism, preferably
an increased drought resistance by surviving longer than the wild type control without showing any symptoms of injury for a period between 2.3 and 3 days or more and
an increased biomass production compared with the wild type control without showing any symptoms of injury for a period between 1.1 and 3 days
is conferred in said organism.

Accordingly, in one embodiment, in case the activity of a polypeptide according to the polypeptide SEQ ID NO.: 5851 ,or encoded by a nucleic acid molecule comprising the nucleic acid SEQ ID NO.: 5850 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5850 or polypeptide SEQ ID NO.: 5851, respectively is increased or generated or if the activity "pantothenate kinase" is increased or generated in an organism, preferably
an increased drought resistance by surviving longer than the wild type control without showing any symptoms of injury for a period between 2.9 and 5 days or more and
an increased biomass production compared with the wild type control without showing any symptoms of injury for a period between 1.4 and 2 days
is conferred in said organism.

Accordingly, in one embodiment, in case the activity of a polypeptide according to the polypeptide SEQ ID NO.: 5993,or encoded by a nucleic acid molecule comprising the nucleic acid SEQ ID NO.: 5992 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5992 or polypeptide SEQ ID NO.: 5993, respectively is increased or generated or if the activity "heat shock protein" is increased or generated in an organism, preferably
an increased drought resistance by surviving longer than the wild type control without showing any symptoms of injury for a period between 2.8 and 5 days or more and
an increased biomass production compared with the wild type control without showing any symptoms of injury for a period between 0.7 and 2 days
is conferred in said organism.

Accordingly, in one embodiment, in case the activity of a polypeptide according to the polypeptide SEQ ID NO.: 6000,or encoded by a nucleic acid molecule comprising the nucleic acid SEQ ID NO.: 5999 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 5999 or polypeptide SEQ ID NO.: 6000, respectively is increased or generated or if the activity "predicted porin" is increased or generated in an organism, preferably
an increased drought resistance by surviving longer than the wild type control without showing any symptoms of injury for a period between 3.2 and 5 days or more and
an increased biomass production compared with the wild type control without showing any symptoms of injury for a period between 0.5 and 3 days
is conferred in said organism.

Accordingly, in one embodiment, in case the activity of a polypeptide according to the polypeptide SEQ ID NO.: 6057,or encoded by a nucleic acid molecule comprising the nucleic acid SEQ ID NO.: 6056 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 6056 or polypeptide SEQ ID NO.: 6057, respectively is increased or generated or if the activity "aspartate ammonia-lyase" is increased or generated in an organism, preferably
an increased drought resistance by surviving longer than the wild type control without showing any symptoms of injury for a period between 3.1 and 5 days or more and
an increased biomass production compared with the wild type control without showing any symptoms of injury for a period between 1.6 and 4 days
is conferred in said organism.

Accordingly, in one embodiment, in case the activity of a polypeptide according to the polypeptide SEQ ID NO.: 6501,or encoded by a nucleic acid molecule comprising the nucleic acid SEQ ID NO.: 6500 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 6500 or polypeptide SEQ ID NO.: 6501, respectively is increased or generated or if the activity "nicotinamide-nucleotide adenylyltransferase" is increased or generated in an organism, preferably
an increased drought resistance by surviving longer than the wild type control without showing any symptoms of injury for a period between 3.5 and 6 days or more and
an increased biomass production compared with the wild type control without showing any symptoms of injury for a period between 0.8 and 3 days
is conferred in said organism.

Accordingly, in one embodiment, in case the activity of a polypeptide according to the polypeptide SEQ ID NO.: 6543,or encoded by a nucleic acid molecule comprising the nucleic acid SEQ ID NO.: 6542 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 6542 or polypeptide SEQ ID NO.: 6543, respectively is increased or generated or if the activity "polyphosphate kinase" is increased or generated in an organism, preferably
an increased drought resistance by surviving longer than the wild type control without showing any symptoms of injury for a period between 2.5 and 4 days or more and
an increased biomass production compared with the wild type control without showing any symptoms of injury for a period between 0.9 and 3 days
is conferred in said organism.

Accordingly, in one embodiment, in case the activity of a polypeptide according to the polypeptide SEQ ID NO.: 6824,or encoded by a nucleic acid molecule comprising the nucleic acid SEQ ID NO.: 6823 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 6823 or polypeptide SEQ ID NO.: 6824, respectively is increased or generated or if the activity "Ya1049c-protein" is increased or generated in an organism, preferably
an increased drought resistance by surviving longer than the wild type control without showing any symptoms of injury for a period between 3.5 and 5 days or more and
an increased biomass production compared with the wild type control without showing any symptoms of injury for a period between 0.7 and 2 days
is conferred in said organism.

Accordingly, in one embodiment, in case the activity of a polypeptide according to the polypeptide SEQ ID NO.: 6871,or encoded by a nucleic acid molecule comprising the nucleic acid SEQ ID NO.: 6870 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 6870 or polypeptide SEQ ID NO.: 6871, respectively is increased or generated or if the activity "YCR059C-protein" is increased or generated in an organism, preferably
an increased drought resistance by surviving longer than the wild type control without showing any symptoms of injury for a period between 3.5 and 5 days or more and
an increased biomass production compared with the wild type control without showing any symptoms of injury for a period between 1 and 3 days
is conferred in said organism.

Accordingly, in one embodiment, in case the activity of a polypeptide according to the polypeptide SEQ ID NO.: 6911 ,or encoded by a nucleic acid molecule comprising the nucleic acid SEQ ID NO.: 6910 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 6910 or polypeptide SEQ ID NO.: 6911, respectively is increased or generated or if the activity "3-deoxy-D-arabino-heptulosonate-7-phosphate (DAHP) synthase" is increased or generated in an organism, preferably
an increased drought resistance by surviving longer than the wild type control without showing any symptoms of injury for a period between 2.7 and 4 days or more and
an increased biomass production compared with the wild type control without showing any symptoms of injury for a period between 1.3 and 3 days
is conferred in said organism.

Accordingly, in one embodiment, in case the activity of a polypeptide according to the polypeptide SEQ ID NO.: 7262,or encoded by a nucleic acid molecule comprising the nucleic acid SEQ ID NO.: 7261 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7261 or polypeptide SEQ ID NO.: 7262, respectively is increased or generated or if the activity "YEL005C-protein" is increased or generated in an organism, preferably
an increased drought resistance by surviving longer than the wild type control without showing any symptoms of injury for a period between 3.6 and 6 days or more and
an increased biomass production compared with the wild type control without showing any symptoms of injury for a period between 1.1 and 4 days
is conferred in said organism.

Accordingly, in one embodiment, in case the activity of a polypeptide according to the polypeptide SEQ ID NO.: 7266,or encoded by a nucleic acid molecule comprising the nucleic acid SEQ ID NO.: 7265 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7265 or polypeptide SEQ ID NO.: 7266, respectively is increased or generated or if the activity "Lsm (Like Sm) protein" is increased or generated in an organism, preferably
an increased drought resistance by surviving longer than the wild type control without showing any symptoms of injury for a period between 3.1 and 4 days or more and
an increased biomass production compared with the wild type control without showing any symptoms of injury for a period between 0.8 and 2 days
is conferred in said organism.

Accordingly, in one embodiment, in case the activity of a polypeptide according to the polypeptide SEQ ID NO.: 7302,or encoded by a nucleic acid molecule comprising the nucleic acid SEQ ID NO.: 7301 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7301 or polypeptide SEQ ID NO.: 7302, respectively is increased or generated or if the activity "YER156C-protein" is increased or generated in an organism, preferably
an increased drought resistance by surviving longer than the wild type control without showing any symptoms of injury for a period between 2.2 and 4 days or more and
an increased biomass production compared with the wild type control without showing any symptoms of injury for a period between 0.1 and 0.1 days
is conferred in said organism.

Accordingly, in one embodiment, in case the activity of a polypeptide according to the polypeptide SEQ ID NO.: 7385,or encoded by a nucleic acid molecule comprising the nucleic acid SEQ ID NO.: 7384 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7384 or polypeptide SEQ ID NO.: 7385, respectively is increased or generated or if the activity "Checkpoint protein" is increased or generated in an organism, preferably
an increased drought resistance by surviving longer than the wild type control without showing any symptoms of injury for a period between 4.3 and 5 days or more and
an increased biomass production compared with the wild type control without showing any symptoms of injury for a period between 0.2 and 1 days
is conferred in said organism.

Accordingly, in one embodiment, in case the activity of a polypeptide according to the polypeptide SEQ ID NO.: 7408,or encoded by a nucleic acid molecule comprising the nucleic acid SEQ ID NO.: 7407 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7407 or polypeptide SEQ ID NO.: 7408, respectively is increased or generated or if the activity "YGL045W-protein" is increased or generated in an organism, preferably
an increased drought resistance by surviving longer than the wild type control without showing any symptoms of injury for a period between 3.3 and 4 days or more and
an increased biomass production compared with the wild type control without showing any symptoms of injury for a period between 1.2 and 4 days
is conferred in said organism.

Accordingly, in one embodiment, in case the activity of a polypeptide according to the polypeptide SEQ ID NO.: 7430,or encoded by a nucleic acid molecule comprising the nucleic acid SEQ ID NO.: 7429 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7429 or polypeptide SEQ ID NO.: 7430, respectively is increased or generated or if the activity "Protein component of the small (40S) ribosomal subunit" is increased or generated in an organism, preferably
an increased drought resistance by surviving longer than the wild type control without showing any symptoms of injury for a period between 2.3 and 4 days or more and
an increased biomass production compared with the wild type control without showing any symptoms of injury for a period between 0.1 and 0.1 days
is conferred in said organism.

Accordingly, in one embodiment, in case the activity of a polypeptide according to the polypeptide SEQ ID NO.: 7559,or encoded by a nucleic acid molecule comprising the nucleic acid SEQ ID NO.: 7558 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7558 or polypeptide SEQ ID NO.: 7559, respectively is increased or generated or if the activity "Dihydrouridine synthase" is increased or generated in an organism, preferably
an increased drought resistance by surviving longer than the wild type control without showing any symptoms of injury for a period between 4.5 and 7 days or more and
an increased biomass production compared with the wild type control without showing any symptoms of injury for a period between 0.1 and 0.1 days
is conferred in said organism.

Accordingly, in one embodiment, in case the activity of a polypeptide according to the polypeptide SEQ ID NO.: 7607,or encoded by a nucleic acid molecule comprising the nucleic acid SEQ ID NO.: 7606 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7606 or polypeptide SEQ ID NO.: 7607, respectively is increased or generated or if the activity "YOR024w-protein" is increased or generated in an organism, preferably
an increased drought resistance by surviving longer than the wild type control without showing any symptoms of injury for a period between 4.8 and 5 days or more and
an increased biomass production compared with the wild type control without showing any symptoms of injury for a period between 0.6 and 3 days
is conferred in said organism.

Accordingly, in one embodiment, in case the activity of a polypeptide according to the polypeptide SEQ ID NO.: 7611 ,or encoded by a nucleic acid molecule comprising the nucleic acid SEQ ID NO.: 7610 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7610 or polypeptide SEQ ID NO.: 7611, respectively is increased or generated or if the activity "Glutamine tRNA synthetase" is increased or generated in an organism, preferably
an increased drought resistance by surviving longer than the wild type control without showing any symptoms of injury for a period between 3.1 and 5 days or more and
an increased biomass production compared with the wild type control without showing any symptoms of injury for a period between 0.6 and 2 days
is conferred in said organism.

Accordingly, in one embodiment, in case the activity of a polypeptide according to the polypeptide SEQ ID NO.: 7686,or encoded by a nucleic acid molecule comprising the nucleic acid SEQ ID NO.: 7685 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7685 or polypeptide SEQ ID NO.: 7686, respectively is increased or generated or if the activity "Splicing factor" is increased or generated in an organism, preferably
an increased drought resistance by surviving longer than the wild type control without showing any symptoms of injury for a period between 3.9 and 5 days or more and
an increased biomass production compared with the wild type control without showing any symptoms of injury for a period between 0.1 and 0.1 days
is conferred in said organism.

Accordingly, in one embodiment, in case the activity of a polypeptide according to the polypeptide SEQ ID NO.: 1202,or encoded by a nucleic acid molecule comprising the nucleic acid SEQ ID NO.: 1201 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 1201 or polypeptide SEQ ID NO.: 1202, respectively is increased or generated or if the activity "gamma-Glu-putrescine synthase" is increased or generated in an organism, preferably
an increased drought resistance by surviving longer than the wild type control without showing any symptoms of injury for a period between 2.1 and 3 days or more and
an increased biomass production compared with the wild type control without showing any symptoms of injury for a period between 1.1 and 2 days
is conferred in said organism.

Accordingly, in one embodiment, in case the activity of a polypeptide according to the polypeptide SEQ ID NO.: 7742,or encoded by a nucleic acid molecule comprising the nucleic acid SEQ ID NO.: 7741 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7741 or polypeptide SEQ ID NO.: 7742, respectively is increased or generated or if the activity "inner membrane protein" is increased or generated in an organism, preferably
an increased drought resistance by surviving longer than the wild type control without showing any symptoms of injury for a period between 3.2 and 5 days or more and
an increased biomass production compared with the wild type control without showing any symptoms of injury for a period between 0.2 and 2 days
is conferred in said organism.

Accordingly, in one embodiment, in case the activity of a polypeptide according to the polypeptide SEQ ID NO.: 7851 ,or encoded by a nucleic acid molecule comprising the nucleic acid SEQ ID NO.: 7850 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7850 or polypeptide SEQ ID NO.: 7851, respectively is increased or generated or if the activity "heat shock protein HtpX" is increased or generated in an organism, preferably
an increased drought resistance by surviving longer than the wild type control without showing any symptoms of injury for a period between 2.4 and 6 days or more and
an increased biomass production compared with the wild type control without showing any symptoms of injury for a period between 1 and 3 days
is conferred in said organism.

Accordingly, in one embodiment, in case the activity of a polypeptide according to the polypeptide SEQ ID NO.: 7972,or encoded by a nucleic acid molecule comprising the nucleic acid SEQ ID NO.: 7971 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 7971 or polypeptide SEQ ID NO.: 7972, respectively is increased or generated or if the activity "DNA-binding transcriptional dual regulator protein" is increased or generated in an organism, preferably
an increased drought resistance by surviving longer than the wild type control without showing any symptoms of injury for a period between 3.9 and 5 days or more and
an increased biomass production compared with the wild type control without showing any symptoms of injury for a period between 0.4 and 2 days
is conferred in said organism.

Accordingly, in one embodiment, in case the activity of a polypeptide according to the polypeptide SEQ ID NO.: 8022,or encoded by a nucleic acid molecule comprising the nucleic acid SEQ ID NO.: 8021 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8021 or polypeptide SEQ ID NO.: 8022, respectively is increased or generated or if the activity "predicted serine transporter protein" is increased or generated in an organism, preferably
an increased drought resistance by surviving longer than the wild type control without showing any symptoms of injury for a period between 2.9 and 5 days or more and
an increased biomass production compared with the wild type control without showing any symptoms of injury for a period between 0.7 and 4 days
is conferred in said organism.

Accordingly, in one embodiment, in case the activity of a polypeptide according to the polypeptide SEQ ID NO.: 8178,or encoded by a nucleic acid molecule comprising the nucleic acid SEQ ID NO.: 8177 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8177 or polypeptide SEQ ID NO.: 8178, respectively is increased or generated or if the activity "glutathione-dependent oxidoreductase" is increased or generated in an organism, preferably
an increased drought resistance by surviving longer than the wild type control without showing any symptoms of injury for a period between 4.5 and 5 days or more and
an increased biomass production compared with the wild type control without showing any symptoms of injury for a period between 0.1 and 0.1 days
is conferred in said organism.

Accordingly, in one embodiment, in case the activity of a polypeptide according to the polypeptide SEQ ID NO.: 8273,or encoded by a nucleic acid molecule comprising the nucleic acid SEQ ID NO.: 8272 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8272 or polypeptide SEQ ID NO.: 8273, respectively is increased or generated or if the activity "Yfr042w-protein" is increased or generated in an organism, preferably
an increased drought resistance by surviving longer than the wild type control without showing any symptoms of injury for a period between 3.7 and 5 days or more and
an increased biomass production compared with the wild type control without showing any symptoms of injury for a period between 1 and 4 days
is conferred in said organism.

Accordingly, in one embodiment, in case the activity of a polypeptide according to the polypeptide SEQ ID NO.: 8289,or encoded by a nucleic acid molecule comprising the nucleic acid SEQ ID NO.: 8288 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8288 or polypeptide SEQ ID NO.: 8289, respectively is increased or generated or if the activity "Protein component of the small (40S) ribosomal subunit" is increased or generated in an organism, preferably
an increased drought resistance by surviving longer than the wild type control without showing any symptoms of injury for a period between 3.3 and 5 days or more and
an increased biomass production compared with the wild type control without showing any symptoms of injury for a period between 1.9 and 4 days
is conferred in said organism.

Accordingly, in one embodiment, in case the activity of a polypeptide according to the polypeptide SEQ ID NO.: 8439,or encoded by a nucleic acid molecule comprising the nucleic acid SEQ ID NO.: 8438 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8438 or polypeptide SEQ ID NO.: 8439, respectively is increased or generated or if the activity "transcriptional regulator protein" is increased or generated in an organism, preferably
an increased drought resistance by surviving longer than the wild type control without showing any symptoms of injury for a period between 2.8 and 5 days or more and
an increased biomass production compared with the wild type control without showing any symptoms of injury for a period between 0.8 and 4 days
is conferred in said organism.

Accordingly, in one embodiment, in case the activity of a polypeptide according to the polypeptide SEQ ID NO.: 8631 ,or encoded by a nucleic acid molecule comprising the nucleic acid SEQ ID NO.: 8630 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8630 or polypeptide SEQ ID NO.: 8631, respectively is increased or generated or if the activity "predicted oxidoreductase (flavin:NADH component)" is increased or generated in an organism, preferably
an increased drought resistance by surviving longer than the wild type control without showing any symptoms of injury for a period between 3.3 and 6 days or more and
an increased biomass production compared with the wild type control without showing any symptoms of injury for a period between 0.6 and 2 days
is conferred in said organism.

Accordingly, in one embodiment, in case the activity of a polypeptide according to the polypeptide SEQ ID NO.: 9269,or encoded by a nucleic acid molecule comprising the nucleic acid SEQ ID NO.: 9268 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9268 or polypeptide SEQ ID NO.: 9269, respectively is increased or generated or if the activity "cellobiose/arbutin/salicin-specific PTS enzyme (IIB component/IC component)" is increased or generated in an organism, preferably an increased drought resistance by surviving longer than the wild type control without showing any symptoms of injury for a period between 4 and 5 days or more and
an increased biomass production compared with the wild type control without showing any symptoms of injury for a period between 0.4 and 2 days
is conferred in said organism.

Accordingly, in one embodiment, in case the activity of a polypeptide according to the polypeptide SEQ ID NO.: 9445,or encoded by a nucleic acid molecule comprising the nucleic acid SEQ ID NO.: 9444 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9444 or polypeptide SEQ ID NO.: 9445, respectively is increased or generated or if the activity "predicted PTS enzymes (IIB component/IIC component)" is increased or generated in an organism, preferably
an increased drought resistance by surviving longer than the wild type control without showing any symptoms of injury for a period between 2.5 and 4 days or more and
an increased biomass production compared with the wild type control without showing any symptoms of injury for a period between 0.8 and 3 days
is conferred in said organism.

Accordingly, in one embodiment, in case the activity of a polypeptide according to the polypeptide SEQ ID NO.: 9825,or encoded by a nucleic acid molecule comprising the nucleic acid SEQ ID NO.: 9824 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9824 or polypeptide SEQ ID NO.: 9825, respectively is increased or generated or if the activity "nicotinamide-nucleotide adenylyltransferase" is increased or generated in an organism, preferably
an increased drought resistance by surviving longer than the wild type control without showing any symptoms of injury for a period between 3.5 and 6 days or more and
an increased biomass production compared with the wild type control without showing any symptoms of injury for a period between 0.8 and 3 days
is conferred in said organism.

Accordingly, in one embodiment, in case the activity of a polypeptide according to the polypeptide SEQ ID NO.: 9906,or encoded by a nucleic acid molecule comprising the nucleic acid SEQ ID NO.: 9905 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9905 or polypeptide SEQ ID NO.: 9906, respectively is increased or generated or if the activity "YGL045W-protein" is increased or generated in an organism, preferably
an increased drought resistance by surviving longer than the wild type control without showing any symptoms of injury for a period between 3.3 and 4 days or more and
an increased biomass production compared with the wild type control without showing any symptoms of injury for a period between 1.2 and 4 days
is conferred in said organism.

Accordingly, in one embodiment, in case the activity of a polypeptide according to the polypeptide SEQ ID NO.: 9194,or encoded by a nucleic acid molecule comprising the nucleic acid SEQ ID NO.: 9193 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9193 or polypeptide SEQ ID NO.: 9194, respectively is increased or generated or if the activity "DNA-binding transcriptional dual regulator protein" is increased or generated in an organism, preferably
an increased drought resistance by surviving longer than the wild type control without showing any symptoms of injury for a period between 3.9 and 5 days or more and
an increased biomass production compared with the wild type control without showing any symptoms of injury for a period between 0.4 and 2 days
is conferred in said organism.

Accordingly, in one embodiment, in case the activity of a polypeptide according to the polypeptide SEQ ID NO.: 8498,or encoded by a nucleic acid molecule comprising the nucleic acid SEQ ID NO.: 8497 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8497 or polypeptide SEQ ID NO.: 8498, respectively is increased or generated or if the activity "methylglyoxal synthase" is increased or generated in an organism, preferably
an increased drought resistance by surviving longer than the wild type control without showing any symptoms of injury for a period between 4.4 and 5 days or more and
an increased biomass production compared with the wild type control without showing any symptoms of injury for a period between 0.5 and 2 days
is conferred in said organism.

Accordingly, in one embodiment, in case the activity of a polypeptide according to the polypeptide SEQ ID NO.: 8743,or encoded by a nucleic acid molecule comprising the nucleic acid SEQ ID NO.: 8742 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8742 or polypeptide SEQ ID NO.: 8743, respectively is increased or generated or if the activity "gamma-Glu-putrescine synthase" is increased or generated in an organism, preferably
an increased drought resistance by surviving longer than the wild type control without showing any symptoms of injury for a period between 2.1 and 3 days or more and
an increased biomass production compared with the wild type control without showing any symptoms of injury for a period between 1.1 and 2 days
is conferred in said organism.

Accordingly, in one embodiment, in case the activity of a polypeptide according to the polypeptide SEQ ID NO.: 8892,or encoded by a nucleic acid molecule comprising the nucleic acid SEQ ID NO.: 8891 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8891 or polypeptide SEQ ID NO.: 8892, respectively is increased or generated or if the activity "acid shock protein precursor" is increased or generated in an organism, preferably
an increased drought resistance by surviving longer than the wild type control without showing any symptoms of injury for a period between 3.7 and 5 days or more and
an increased biomass production compared with the wild type control without showing any symptoms of injury for a period between 0.1 and 1 days
is conferred in said organism.

Accordingly, in one embodiment, in case the activity of a polypeptide according to the polypeptide SEQ ID NO.: 9032,or encoded by a nucleic acid molecule comprising the nucleic acid SEQ ID NO.: 9031 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9031 or polypeptide SEQ ID NO.: 9032, respectively is increased or generated or if the activity "regulator of length of O-antigen component of lipopolysaccharide chains " is increased or generated in an organism, preferably an increased drought resistance by surviving longer than the wild type control without showing any symptoms of injury for a period between 2.6 and 4 days or more and
an increased biomass production compared with the wild type control without showing any symptoms of injury for a period between 0.3 and 2 days
is conferred in said organism.

Accordingly, in one embodiment, in case the activity of a polypeptide according to the polypeptide SEQ ID NO.: 9316,or encoded by a nucleic acid molecule comprising the nucleic acid SEQ ID NO.: 9315 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9315 or polypeptide SEQ ID NO.: 9316, respectively is increased or generated or if the activity "ornithine decarboxylase" is increased or generated in an organism, preferably
an increased drought resistance by surviving longer than the wild type control without showing any symptoms of injury for a period between 3.8 and 5 days or more and
an increased biomass production compared with the wild type control without showing any symptoms of injury for a period between 0.7 and 3 days
is conferred in said organism.

Accordingly, in one embodiment, in case the activity of a polypeptide according to the polypeptide SEQ ID NO.: 9530,or encoded by a nucleic acid molecule comprising the nucleic acid SEQ ID NO.: 9529 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9529 or polypeptide SEQ ID NO.: 9530, respectively is increased or generated or if the activity "aspartate ammonia-lyase " is increased or generated in an organism, preferably
an increased drought resistance by surviving longer than the wild type control without showing any symptoms of injury for a period between 3.1 and 5 days or more and
an increased biomass production compared with the wild type control without showing any symptoms of injury for a period between 1.6 and 4 days
is conferred in said organism.

Accordingly, in one embodiment, in case the activity of a polypeptide according to the polypeptide SEQ ID NO.: 8463,or encoded by a nucleic acid molecule comprising the nucleic acid SEQ ID NO.: 8462 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8462 or polypeptide SEQ ID NO.: 8463, respectively is increased or generated or if the activity "predicted transporter protein" is increased or generated in an
organism, preferably
an increased drought resistance by surviving longer than the wild type control without showing any symptoms of injury for a period between 2.2 and 5 days or more and
an increased biomass production compared with the wild type control without showing any symptoms of injury for a period between 1 and 4 days
is conferred in said organism.

Accordingly, in one embodiment, in case the activity of a polypeptide according to the polypeptide SEQ ID NO.: 8974,or encoded by a nucleic acid molecule comprising the nucleic acid SEQ ID NO.: 8973 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8973 or polypeptide SEQ ID NO.: 8974, respectively is increased or generated or if the activity "L-arabinose transporter subunit" is increased or generated in an organism, preferably
an increased drought resistance by surviving longer than the wild type control without showing any symptoms of injury for a period between 2.8 and 4 days or more and
an increased biomass production compared with the wild type control without showing any symptoms of injury for a period between 1.8 and 3 days
is conferred in said organism.

Accordingly, in one embodiment, in case the activity of a polypeptide according to the polypeptide SEQ ID NO.: 9884,or encoded by a nucleic acid molecule comprising the nucleic acid SEQ ID NO.: 9883 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9883 or polypeptide SEQ ID NO.: 9884, respectively is increased or generated or if the activity "Lsm (Like Sm) protein" is increased or generated in an organism, preferably
an increased drought resistance by surviving longer than the wild type control without showing any symptoms of injury for a period between 3.1 and 4 days or more and
an increased biomass production compared with the wild type control without showing any symptoms of injury for a period between 0.8 and 2 days
is conferred in said organism.

Accordingly, in one embodiment, in case the activity of a polypeptide according to the polypeptide SEQ ID NO.: 8935,or encoded by a nucleic acid molecule comprising the nucleic acid SEQ ID NO.: 8934 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8934 or polypeptide SEQ ID NO.: 8935, respectively is increased or generated or if the activity "neutral amino-acid efflux system" is increased or generated in an organism, preferably
an increased drought resistance by surviving longer than the wild type control without showing any symptoms of injury for a period between 3.4 and 5 days or more and
an increased biomass production compared with the wild type control without showing any symptoms of injury for a period between 1 and 4 days
is conferred in said organism.

Accordingly, in one embodiment, in case the activity of a polypeptide according to the polypeptide SEQ ID NO.: 9094,or encoded by a nucleic acid molecule comprising the nucleic acid SEQ ID NO.: 9093 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9093 or polypeptide SEQ ID NO.: 9094, respectively is increased or generated or if the activity "b2226-protein" is increased or generated in an organism, preferably
an increased drought resistance by surviving longer than the wild type control without showing any symptoms of injury for a period between 2.9 and 4 days or more and
an increased biomass production compared with the wild type control without showing any symptoms of injury for a period between 1.6 and 4 days
is conferred in said organism.

Accordingly, in one embodiment, in case the activity of a polypeptide according to the polypeptide SEQ ID NO.: 9110,or encoded by a nucleic acid molecule comprising the nucleic acid SEQ ID NO.: 9109 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9109 or polypeptide SEQ ID NO.: 9110, respectively is increased or generated or if the activity "sensory histidine kinase in two-component regulatory system with NarP (NarL)" is increased or generated in an organism, preferably
an increased drought resistance by surviving longer than the wild type control without showing any symptoms of injury for a period between 2.2 and 5 days or more and
an increased biomass production compared with the wild type control without showing any symptoms of injury for a period between 0.9 and 3 days
is conferred in said organism.

Accordingly, in one embodiment, in case the activity of a polypeptide according to the polypeptide SEQ ID NO.: 9932,or encoded by a nucleic acid molecule comprising the nucleic acid SEQ ID NO.: 9931 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 9931 or polypeptide SEQ ID NO.: 9932, respectively is increased or generated or if the activity "Glutamine tRNA synthetase" is increased or generated in an organism, preferably
an increased drought resistance by surviving longer than the wild type control without showing any symptoms of injury for a period between 3.1 and 5 days or more and
an increased biomass production compared with the wild type control without showing any symptoms of injury for a period between 0.6 and 2 days
is conferred in said organism.

Accordingly, in one embodiment, in case the activity of a polypeptide according to the polypeptide SEQ ID NO.: 10097,or encoded by a nucleic acid molecule comprising the nucleic acid SEQ ID NO.: 10096 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 10096 or polypeptide SEQ ID NO.: 10097, respectively is increased or generated or if the activity "gluconate transporter" is increased or generated in an organism, preferably
an increased drought resistance by surviving longer than the wild type control without showing any symptoms of injury for a period between 2.5 and 4 days or more and
an increased biomass production compared with the wild type control without showing any symptoms of injury for a period between 0.8 and 3 days
is conferred in said organism.

In this embodiment, said molecule, which activity is to be increased in the process of the invention and which is the gene product with an activity of described as a "glutathione-dependent oxidoreductase", is expressed non-targeted by using the constituteive promoter.

In one embodiment, according to the method of the invention for an increased tolerance and/or resistance to low temperatur stress and increased biomass production in a plant cell, plant or a part thereof compared to a control or wild type can be achieved.

Accordingly, in one embodiment, in case the activity of a polypeptide according to the polypeptide SEQ ID NO.: 8178,or encoded by a nucleic acid molecule comprising the nucleic acid SEQ ID NO.: 8177 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8177 or polypeptide SEQ ID NO.: 8178, respectively is increased or generated or if the activity "glutathione-dependent oxidoreductase" is increased or generated in an organism, preferably
an increased low temperature resistance, preferably chilling resistance, by an increased biomass production compared with the wild type control of 5% to 100% or even more, preferably 10% to 90%, 20% to 80%, more preferably 25% to 60%, 35% to 50%, 38% to 45% is conferred in said organism.

In this embodiment, said molecule, which activity is to be increased in the process of the invention and which is the gene product with an activity of described as a "glutathione-dependent oxidoreductase", is expressed non-targeted by using the promoter USP (Bäumlein et al., Mol Gen Genet. 225(3):459-67 (1991)).

In one embodiment, according to the method of the invention for an increased tolerance and/or resistance to cycling drought stress and increased biomass production in a plant cell, plant or a part thereof compared to a control or wild type can be achieved.

Accordingly, in one embodiment, in case the activity of a polypeptide according to the polypeptide SEQ ID NO.: 8178,or encoded by a nucleic acid molecule comprising the nucleic acid SEQ ID NO.: 8177 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8177 or polypeptide SEQ ID NO.: 8178, respectively is increased or generated or if the activity "glutathione-dependent oxidoreductase" is increased or generated in an organism, preferably
an increased resistance to cycling drought, by an increased biomass production compared with the wild type control of 5% to 100% or even more, preferably 10% to 50%, 15% to 40%, more preferably 20% to 30%, 21% to 28%, 21% to 27% is conferred in said organism.

In this embodiment, said molecule, which activity is to be increased in the process of the invention and which is the gene product with an activity of described as a "glutathione-dependent oxidoreductase", is expressed plastidic by using the constitutive promoter.

In one embodiment, according to the method of the invention an increased biomass production in a plant cell, plant or a part thereof compared to a control or wild type can be achieved.

Accordingly, in one embodiment, in case the activity of a polypeptide according to the polypeptide SEQ ID NO.: 8178,or encoded by a nucleic acid molecule comprising the nucleic acid SEQ ID NO.: 8177 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8177 or polypeptide SEQ ID NO.: 8178, respectively is increased or generated or if the activity "glutathione-dependent oxidoreductase" is increased or generated in an organism, preferably
an increased biomass production compared with the wild type control of 5% to 100% or even more, preferably 10% to 90%, 20% to 80%, more preferably 30% to 70%, 35% to 60%, 40% to 50%, 41% to 45% is conferred in said organism.

In this embodiment, said molecule, which activity is to be increased in the process of the invention and which is the gene product with an activity of described as a "glutathione-dependent oxidoreductase", is expressed non-targeted by using the promoter USP.

In one embodiment, according to the method of the invention an increased resistance to limited nutrient, preferably nitrogen availability by enhanced NUE and an increased biomass production in a plant cell, plant or a part thereof compared to a control or wild type can be achieved.

Accordingly, in one embodiment, in case the activity of a polypeptide according to the polypeptide SEQ ID NO.: 8178,or encoded by a nucleic acid molecule comprising the nucleic acid SEQ ID NO.: 8177 or a homolog of said nucleic acid molecule or polypeptide, e.g. if the activity of a nucleic acid molecule or a polypeptide comprising the nucleic acid or polypeptide or the consensus sequence or the polypeptide motif, as depicted in Table I, II or IV, column 7 in the respective same line as the nucleic acid molecule SEQ ID NO.: 8177 or polypeptide SEQ ID NO.: 8178, respectively is increased or generated or if the activity "glutathione-dependent oxidoreductase" is increased or generated in an organism, preferably
an increased biomass production compared with the wild type control of 5% to 100% or even more, preferably 10% to 90%, 20% to 80%, more preferably 25% to 65%, 30% to 60%, is conferred in said organism.

In this embodiment, said molecule, which activity is to be increased in the process of the invention and which is the gene product with an activity of described as a "glutathione-dependent oxidoreductase", is expressed non-targeted by using the constituteive promoter.

In one embodiment, said molecule, which activity is to be increased in the process of the invention and which is the gene product with an activity of described as a "glutathione-dependent oxidoreductase", is selected from the group consisting of the polypeptides with the with the SEQ IDs NO: 8180, 8182, 8188, 8190, 8198, 8200, 8212, 8214, 8216, 8218, 8234, 8236, 8238, 8240, 8242, 8244, 8246, 8248, 8250, 8252, 8254, 8256, 8258, 8260, 8262, 8264, 8266, 10083 encoded by the SEQ IDs NO: 8179, 8181, 8187, 8189, 8197, 8199, 8211, 8213, 8215, 8217, 8233, 8235, 8237, 8239, 8241, 8243, 8245, 8247, 8249, 8251, 8253, 8255, 8257, 8259, 8261, 8263, 8265, 10082 respectively.

The term "expression" refers to the transcription and/or translation of a codogenic gene segment or gene. As a rule, the resulting product is an mRNA or a protein. However, expression products can also include functional RNAs such as, for example, antisense, nucleic acids, tRNAs, snRNAs, rRNAs, RNAi, siRNA, ribozymes etc. Expression may be systemic, local or temporal, for example limited to certain cell types, tissues organs or organelles or time periods.

In one embodiment, the process of the present invention comprises one or more of the following steps
a) stabilizing a protein conferring the increased expression of a protein encoded by the nucleic acid molecule of the invention or of the polypeptid of the invention having the herein-mentioned activity selected from the group consisting of 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Yal049c-protein, YCR059C-protein, YEL005C-protein, YER156C-protein, Yfr042w-protein, YGL045W-protein, and YOR024w-protein and confering an increased tolerance and/or resistance to environmental stress and increased biomass production as compared to a corresponding non-transformed wild type plant cell, plant or part thereof;
b) stabilizing a mRNA conferring the increased expression of a protein encoded by the nucleic acid molecule of the invention or its homologs or of a mRNA encoding the polypeptide of the present invention having the herein-mentioned activity selected from the group consisting of 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipo-polysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Yal049c-protein, YCR059C-protein, YEL005C-protein, YER156C-protein, Yfr042w-protein, YGL045W-protein, and YOR024w-protein and confering an increased tolerance and/or resistance to environmental stress and increased biomass production as compared to a corresponding non-transformed wild type plant cell, plant or part thereof;
c) increasing the specific activity of a protein conferring the increased expression of a protein encoded by the nucleic acid molecule of the invention or of the polypeptide of the present invention or decreasing the inhibitory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having the herein-mentioned activity selected from the group consisting of 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Yal049c-protein, YCR059C-protein, YEL005C-protein, YER156C-protein, Yfr042w-protein, YGL045W-protein, and YOR024w-protein and conferring an increased tolerance and/or resistance to environmental stress and increased biomass production as compared to a corresponding non-transformed wild type plant cell, plant or part thereof;
e) stimulating activity of a protein conferring the increased expression of a protein encoded by the nucleic acid molecule of the present invention or a polypeptide of the present invention having the herein-mentioned activity selected from the group consisting of 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Yal049c-protein, YCR059C-protein, YEL005C-protein, YER156C-protein, Yfr042w-protein, YGL045W-protein, and YOR024w-protein and confering an increased tolerance and/or resistance to environmental stress and increased biomass production as compared to a corresponding non-transformed wild type plant cell, plant or part thereof by adding one or more exogenous inducing factors to the organismus or parts thereof;
f) expressing a transgenic gene encoding a protein conferring the increased expression of a polypeptide encoded by the nucleic acid molecule of the present invention or a polypeptide of the present invention, having the herein-mentioned activity selected from the group consisting of 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Yal049c-protein, YCR059C-protein, YEL005C-protein, YER156C-protein, Yfr042w-protein, YGL045W-protein, and YOR024w-protein and confering an increased tolerance and/or resistance to environmental stress and increased biomass production as compared to a corresponding non-transformed wild type plant cell, plant or part thereof; and/or
g) increasing the copy number of a gene conferring the increased expression of a nucleic acid molecule encoding a polypeptide encoded by the nucleic acid molecule of the invention or the polypeptide of the invention having the herein-mentioned activity selected from the group consisting of 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Yal049c-protein, YCR059C-protein, YEL005C-protein, YER156C-protein, Yfr042w-protein, YGL045W-protein, and YOR024w-protein and confering an increased tolerance and/or resistance to environmental stress and increased biomass production as compared to a corresponding non-transformed wild type plant cell, plant or part thereof;
h) increasing the expression of the endogenous gene encoding the polypeptide of the invention or its homologs by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements- positive elements can be randomly introduced in plants by T-DNA or transposon mutagenesis and lines can be identified in which the positive elements have been integrated near to a gene of the invention, the expression of which is thereby enhanced; and/or
i) modulating growth conditions of the plant in such a manner, that the expression or activity of the gene encoding the protein of the invention or the protein itself is enhanced;
j) selecting of organisms with especially high activity of the proteins of the invention from natural or from mutagenized resources and breeding them into the target organisms, e.g. the elite crops.

Preferably, said mRNA is the nucleic acid molecule of the present invention and/or the protein conferring the increased expression of a protein encoded by the nucleic acid molecule of the present invention alone or linked to a transit nucleic acid sequence or transit peptide encoding nucleic acid sequence or the polypeptide having the herein mentioned activity, e.g. conferring an increased tolerance and/or resistance to environmental stress and increased biomass production as compared to a corresponding non-transformed wild type plant cell, plant or part thereof after increasing the expression or activity of the encoded polypeptide or having the activity of a polypeptide having an activity as the protein as shown in table II column 3 or its homologs.

In general, the amount of mRNA or polypeptide in a cell or a compartment of an organism correlates with the amount of encoded protein and thus with the overall activity of the encoded protein in said volume. Said correlation is not always linear, the activity in the volume is dependent on the stability of the molecules or the presence of activating or inhibiting co-factors. Further, product and educt inhibitions of enzymes are well known and described in textbooks, e.g. Stryer, Biochemistry. In general, the amount of mRNA, polynucleotide or nucleic acid molecule in a cell or a compartment of an organism correlates with the amount of encoded protein and thus with the overall activity of the encoded protein in said volume. Said correlation is not always linear, the activity in the volume is dependent on the stability of the molecules, the degradation of the molecules or the presence of activating or inhibiting co-factors. Further, product and educt inhibitions of enzymes are well known, e.g. Zinser et al. "Enzyminhibitoren"/Enzyme inhibitors".

The activity of the abovementioned proteins and/or polypeptides encoded by the nucleic acid molecule of the present invention can be increased in various ways. For example, the activity in an organism or in a part thereof, like a cell, is increased via increasing the gene product number, e.g. by increasing the expression rate, like introducing a stronger promoter, or by increasing the stability of the mRNA expressed, thus increasing the translation rate, and/or increasing the stability of the gene product, thus reducing the proteins decayed. Further, the activity or turnover of enzymes can be influenced in such a way that a reduction or increase of the reaction rate or a modification (reduction or increase) of the affinity to the substrate results, is reached. A mutation in the catalytic center of an polypeptide of the invention, e.g. as enzyme, can modulate the turn over rate of the enzyme, e.g. a knock out of an essential amino acid can lead to a reduced or completely knock out activity of the enzyme, or the deletion or mutation of regulator binding sites can reduce a negative regulation like a feedback inhibition (or a substrate inhibition, if the substrate level is also increased). The specific activity of an enzyme of the present invention can be increased such that the turn over rate is increased or the binding of a co-factor is improved. Improving the stability of the encoding mRNA or the protein can also increase the activity of a gene product. The stimulation of the activity is also under the scope of the term "increased activity".

Moreover, the regulation of the abovementioned nucleic acid sequences may be modified so that gene expression is increased. This can be achieved advantageously by means of heterologous regulatory sequences or by modifying, for example mutating, the natural regulatory sequences which are present. The advantageous methods may also be combined with each other.

In general, an activity of a gene product in an organism or part thereof, in particular in a plant cell or organelle of a plant cell, a plant, or a plant tissue or a part thereof or in a microorganism can be increased by increasing the amount of the specific encoding mRNA or the corresponding protein in said organism or part thereof. "Amount of protein or mRNA" is understood as meaning the molecule number of polypeptides or mRNA molecules in an organism, a tissue, a cell or a cell compartment. "Increase" in the amount of a protein means the quantitative increase of the molecule number of said protein in an organism, a tissue, a cell or a cell compartment such as an organelle like a plastid or mitochondria or part thereof - for example by one of the methods described herein below - in comparison to a wild type, control or reference.

The increase in molecule number amounts preferably to at least 1%, preferably to more than 10%, more preferably to 30% or more, especially preferably to 50%, 70% or more, very especially preferably to 100%, most preferably to 500% or more. However, a de novo expression is also regarded as subject of the present invention.

A modification, i.e. an increase, can be caused by endogenous or exogenous factors. For example, an increase in activity in an organism or a part thereof can be caused by adding a gene product or a precursor or an activator or an agonist to the media or nutrition or can be caused by introducing said subjects into a organism, transient or stable. Furthermore such an increase can be reached by the introduction of the inventive nucleic acid sequence or the encoded protein in the correct cell compartment for example into the , nucleus, or cytoplasm respectively or into plastids either by transformation and/or targeting.

In one embodiment the increase or decrease in tolerance and/or resistance to environmental stress as compared to a corresponding non-transformed wild type plant cell in the plant or a part thereof, e.g. in a cell, a tissue, a organ, an organelle etc., is achieved by increasing the endogenous level of the polypeptide of the invention. Accordingly, in an embodiment of the present invention, the present invention relates to a process wherein the gene copy number of a gene encoding the polynucleotide or nucleic acid molecule of the invention is increased. Further, the endogenous level of the polypeptide of the invention can for example be increased by modifying the transcriptional or translational regulation of the polypeptide.

In one embodiment the increased tolerance and/or resistance to environmental stress in the plant or part thereof can be altered by targeted or random mutagenesis of the endogenous genes of the invention. For example homologous recombination can be used to either introduce positive regulatory elements like for plants the 35S enhancer into the promoter or to remove repressor elements form regulatory regions. In addition gene conversion like methods described by Kochevenko and Willmitzer (Plant Physiol. 2003 May;132(1):174-84) and citations therein can be used to disrupt repressor elements or to enhance to activity of positive regulatory elements. Furthermore positive elements can be randomly introduced in (plant) genomes by T-DNA or transposon mutagenesis and lines can be screened for, in which the positive elements has be integrated near to a gene of the invention, the expression of which is thereby enhanced. The activation of plant genes by random integrations of enhancer elements has been described by Hayashi et al., 1992 (Science 258:1350-1353) or Weigel et al., 2000 (Plant Physiol. 122, 1003-1013) and others citated therein. Reverse genetic strategies to identify insertions (which eventually carrying the activation elements) near in genes of interest have been described for various cases e.g.. Krysan et al., 1999 (Plant Cell 1999, 11, 2283-2290); Sessions et al., 2002 (Plant Cell 2002, 14, 2985-2994); Young et al., 2001, (Plant Physiol. 2001, 125, 513-518); Koprek et al., 2000 (Plant J. 2000, 24, 253-263) ; Jeon et al., 2000 (Plant J. 2000, 22, 561-570) ; Tissier et al., 1999 (Plant Cell 1999, 11, 1841-1852); Speulmann et al., 1999 (Plant Cell 1999 ,11 , 1853-1866). Briefly material from all plants of a large T-DNA or transposon mutagenized plant population is harvested and genomic DNA prepared. Then the genomic DNA is pooled following specific architectures as described for example in Krysan et al., 1999 (Plant Cell 1999, 11, 2283-2290). Pools of genomics DNAs are then screened by specific multiplex PCR reactions detecting the combination of the insertional mutagen (eg T-DNA or Transposon) and the gene of interest. Therefore PCR reactions are run on the DNA pools with specific combinations of T-DNA or transposon border primers and gene specific primers. General rules for primer design can again be taken from Krysan et al., 1999 (Plant Cell 1999, 11, 2283-2290) Re-screening of lower levels DNA pools lead to the identifcation of individual plants in which the gene of interest is activated by the insertional mutagen.

The enhancement of positive regulatory elements or the disruption or weaking of negative regulatory elements can also be achieved through common mutagenesis techniques: The production of chemically or radiation mutated populations is a common technique and known to the skilled worker. Methods for plants are described by Koorneef et al. 1982 and the citations therein and by Lightner and Caspar in "Methods in Molecular Biology" Vol 82. These techniques usually induce pointmutations that can be identified in any known gene using methods such as TILLING (Colbert et al. 2001).

Accordingly, the expression level can be increased if the endogenous genes encoding a polypeptide conferring an increased expression of the polypeptide of the present invention, in particular genes comprising the nucleic acid molecule of the present invention, are modified via homologous recombination, Tilling approaches or gene conversion. It also possible to add as mentioned herein targeting sequences to the inventive nucleic acid sequences.

Regulatory sequences preferably in addition to a target sequence or part thereof can be operatively linked to the coding region of an endogenous protein and control its transcription and translation or the stability or decay of the encoding mRNA or the expressed protein. In order to modify and control the expression, promoter, UTRs, splicing sites, processing signals, polyadenylation sites, terminators, enhancers, repressors, post transcriptional or posttranslational modification sites can be changed, added or amended. For example, the activation of plant genes by random integrations of enhancer elements has been described by Hayashi et al., 1992 (Science 258:1350-1353) or Weigel et al., 2000 (Plant Physiol. 122, 1003-1013) and others citated therein. For example, the expression level of the endogenous protein can be modulated by replacing the endogenous promoter with a stronger transgenic promoter or by replacing the endogenous 3'UTR with a 3'UTR, which provides more stability without amending the coding region. Further, the transcriptional regulation can be modulated by introduction of an artificial transcription factor as described in the examples. Alternative promoters, terminators and UTR are described below.

The activation of an endogenous polypeptide having above-mentioned activity, e.g. having the activity of a protein as shown in table II, column 3 or of the polypeptide of the invention, e.g. conferring the increase of the tolerance and/or resistance to environmental stress and increased biomass production as compared to a corresponding non-transformed wild type plant cell, plant or part thereof after increase of expression or activity in the cytosol and/or in an organelle like a plastid, can also be increased by introducing a synthetic transcription factor, which binds close to the coding region of the gene encoding the protein as shown in table II, column 3 and activates its transcription. A chimeric zinc finger protein can be constructed, which comprises a specific DNA-binding domain and an activation domain as e.g. the VP16 domain of Herpes Simplex virus. The specific binding domain can bind to the regulatory region of the gene encoding the protein as shown in table II, column 3. The expression of the chimeric transcription factor in a organism, in particular in a plant, leads to a specific expression of the protein as shown in table II, column 3, see e.g. in WO01/52620, Oriz, Proc. Natl. Acad. Sci. USA, 2002, Vol. 99, 13290 or Guan, Proc. Natl. Acad. Sci. USA, 2002, Vol. 99, 13296.

In one further embodiment of the process according to the invention, organisms are used in which one of the abovementioned genes, or one of the above-mentioned nucleic acids, is mutated in a way that the activity of the encoded gene products is less influenced by cellular factors, or not at all, in comparison with the un-mutated proteins. For example, well known regulation mechanism of enzymic activity are substrate inhibition or feed back regulation mechanisms. Ways and techniques for the introduction of substitution, deletions and additions of one or more bases, nucleotides or amino acids of a corresponding sequence are described herein below in the corresponding paragraphs and the references listed there, e.g. in Sambrook et al., Molecular Cloning, Cold Spring Habour, NY, 1989. The person skilled in the art will be able to identify regulation domains and binding sites of regulators by comparing the sequence of the nucleic acid molecule of the present invention or the expression product thereof with the state of the art by computer software means which comprise algorithms for the identifying of binding sites and regulation domains or by introducing into a nucleic acid molecule or in a protein systematically mutations and assaying for those mutations which will lead to an increased specific activity or an increased activity per volume, in particular per cell.

It can therefore be advantageous to express in an organism a nucleic acid molecule of the invention or a polypeptide of the invention derived from a evolutionary distantly related organism, as e.g. using a prokaryotic gene in a eukaryotic host, as in these cases the regulation mechanism of the host cell may not weaken the activity (cellular or specific) of the gene or its expression product.

The mutation is introduced in such a way that the increased tolerance and/or resistance to environmental stress and biomass increase are not adversely affected.

Less influence on the regulation of a gene or its gene product is understood as meaning a reduced regulation of the enzymatic activity leading to an increased specific or cellular activity of the gene or its product. An increase of the enzymatic activity is understood as meaning an enzymatic activity, which is increased by at least 10%, advantageously at least 20, 30 or 40%, especially advantageously by at least 50, 60 or 70% in comparison with the starting organism. This leads to an increased tolerance and/or resistance to environmental stress and increased biomass production as compared to a corresponding non-transformed wild type plant cell, plant or part thereof.

The invention provides that the above methods can be performed such that the stress tolerance is increased. It is also possible to obtain a decrease in stress tolerance.

The invention is not limited to specific nucleic acids, specific polypeptides, specific cell types, specific host cells, specific conditions or specific methods etc. as such, but may vary and numerous modifications and variations therein will be apparent to those skilled in the art. It is also to be understood that the terminology used herein is for the purpose of describing specific embodiments only and is not intended to be limiting.

The present invention also relates to isolated nucleic acids comprising a nucleic acid molecule selected from the group consisting of:
a) a nucleic acid molecule encoding the polypeptide shown in column 7 of Table II B;
b) a nucleic acid molecule shown in column 7 of Table I B;
c) a nucleic acid molecule, which, as a result of the degeneracy of the genetic code, can be derived from a polypeptide sequence depicted in column 5 or 7 of Table II and confers an increased tolerance and/or resistance to environmental stress and increased biomass production as compared to a corresponding non-transformed wild type plant cell, a plant or a part thereof;
d) a nucleic acid molecule having at least 30 % identity with the nucleic acid molecule sequence of a polynucleotide comprising the nucleic acid molecule shown in column 5 or 7 of Table I and confers an increased tolerance and/or resistance to environmental stress and increased biomass production as compared to a corresponding non-transformed wild type plant cell, a plant or a part thereof;
e) a nucleic acid molecule encoding a polypeptide having at least 30 % identity with the amino acid sequence of the polypeptide encoded by the nucleic acid molecule of (a) to (c) and having the activity represented by a nucleic acid molecule comprising a polynucleotide as depicted in column 5 of Table I and confers an increased tolerance and/or resistance to environmental stress and increased biomass production as compared to a corresponding non-transformed wild type plant cell, a plant or a part thereof;
f) nucleic acid molecule which hybridizes with a nucleic acid molecule of (a) to (c) under stringent hybridization conditions and confers an increased tolerance and/or resistance to environmental stress and increased biomass production as compared to a corresponding non-transformed wild type plant cell, a plant or a part thereof;
g) a nucleic acid molecule encoding a polypeptide which can be isolated with the aid of monoclonal or polyclonal antibodies made against a polypeptide encoded by one of the nucleic acid molecules of (a) to (e) and having the activity represented by the nucleic acid molecule comprising a polynucleotide as depicted in column 5 of Table I;
h) a nucleic acid molecule encoding a polypeptide comprising the consensus sequence or one or more polypeptide motifs as shown in column 7 of Table IV and preferably having the activity represented by a nucleic acid molecule comprising a polynucleotide as depicted in column 5 of Table II or IV;
h) a nucleic acid molecule encoding a polypeptide having the activity represented by a protein as depicted in column 5 of Table II and confers an increased tolerance and/or resistance to environmental stress and increased biomass production as compared to a corresponding non-transformed wild type plant cell, a plant or a part thereof;
i) nucleic acid molecule which comprises a polynucleotide, which is obtained by amplifying a cDNA library or a genomic library using the primers in column 7 of Table III which do not start at their 5'-end with the nucleotides ATA and preferably having the activity represented by a nucleic acid molecule comprising a polynucleotide as depicted in column 5 of Table II or IV;
   and
j) a nucleic acid molecule which is obtainable by screening a suitable nucleic acid library under stringent hybridization conditions with a probe comprising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence characterized in (a) to (e) and encoding a polypeptide having the activity represented by a protein comprising a polypeptide as depicted in column 5 of Table II;
whereby the nucleic acid molecule according to (a) to (j) is at least in one or more nucleotides different from the sequence depicted in column 5 or 7 of Table I A and preferably which encodes a protein which differs at least in one or more amino acids from the protein sequences depicted in column 5 or 7 of Table II A.

In one embodiment the invention relates to homologs of the aforementioned sequences, which can be isolated advantageously from yeast, fungi, viruses, algae, bacteria, such as Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.; Coxiella burnetii; Deinococcus radiodurans; Dichelobacter nodosus; Edwardsiella ictaluri; Enterobacter sp.; Erysipelothrix rhusiopathiae; Escherichia coli; Flavobacterium sp.; Francisella tularensis; Frankia sp. Cpl1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer; Ruminococcus flavefaciens; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis, preferably Salmonella sp. or Escherichia coli or plants, preferably from yeasts such as from the genera Saccharomyces, Pichia, Candida, Hansenula, Torulopsis or Schizosaccharomyces or plants such as Arabidopsis thaliana, maize, wheat, rye, oat, triticale, rice, barley, soybean, peanut, cotton, borage, sunflower, linseed, primrose, rapeseed, canola and turnip rape, manihot, pepper, sunflower, tagetes, solanaceous plant such as potato, tobacco, eggplant and tomato, Vicia species, pea, alfalfa, bushy plants such as coffee, cacao, tea, Salix species, trees such as oil palm, coconut, perennial grass, such as ryegrass and fescue, and forage crops, such as alfalfa and clover and from spruce, pine or fir for example. More preferably homologs of aforementioned sequences can be isolated from *Saccharomyces cerevisiae, E. coli* or *Synechocystis sp.* or plants, preferably *Brassica napus, Glycine max, Zea mays, cotton,* or *Oryza sativa.*

The (stress related) proteins of the present invention are preferably produced by recombinant DNA techniques. For example, a nucleic acid molecule encoding the protein is cloned into an expression vector, for example in to a binary vector, the expression vector is introduced into a host cell, for example the Arabidopsis thaliana wild type NASC N906 or any other plant cell as described in the examples see below, and the stress related protein is expressed in said host cell. Examples for binary vectors are pBIN19, pBI101, pBinAR, pGPTV, pCAMBIA, pBIB-HYG, pBecks, pGreen or pPZP (Hajukiewicz, P. et al., 1994, Plant Mol. Biol., 25: 989-994 and Hellens et al, Trends in Plant Science (2000) 5, 446-451.).

In one embodiment the (stess related) protein of the present inventnion is preferably produced in an compartment of the cell, more preferably in the plastids. Ways of introducing nucleic acids into plastids and producing proteins in this compartment are know to the person skilled in the art have been also described in this application.

Advantageously, the nucleic acid sequences according to the invention or the gene construct together with at least one reporter gene are cloned into an expression cassette, which is introduced into the organism via a vector or directly into the genome. This reporter gene should allow easy detection via a growth, fluorescence, chemical, bioluminescence or resistance assay or via a photometric measurement. Examples of reporter genes which may be mentioned are antibiotic- or herbicide-resistance genes, hydrolase genes, fluorescence protein genes, bioluminescence genes, sugar or nucleotide metabolic genes or biosynthesis genes such as the Ura3 gene, the IIv2 gene, the luciferase gene, the β-galactosidase gene, the gfp gene, the 2-desoxyglucose-6-phosphate phosphatase gene, the β-glucuronidase gene, β-lactamase gene, the neomycin phosphotransferase gene, the hygromycin phosphotransferase gene, a mutated acetohydroxyacid synthase (AHAS) gene, also known as acetolactate synthase (ALS) gene], a gene for a D-amino acid metabolizing enzmye or the BASTA (= gluphosinate-resistance) gene. These genes permit easy measurement and quantification of the transcription activity and hence of the expression of the genes. In this way genome positions may be identified which exhibit differing productivity.

In a preferred embodiment a nucleic acid construct, for example an expression cassette, comprises upstream, i.e. at the 5' end of the encoding sequence, a promoter and downstream, i.e. at the 3' end, a polyadenylation signal and optionally other regulatory elements which are operably linked to the intervening encoding sequence with one of the nucleic acids of SEQ ID NO as depicted in table I, column 5 and 7. By an operable linkage is meant the sequential arrangement of promoter, encoding sequence, terminator and optionally other regulatory elements in such a way that each of the regulatory elements can fulfill its function in the expression of the encoding sequence in due manner. The sequences preferred for operable linkage are targeting sequences for ensuring subcellular localization in plastids. However, targeting sequences for ensuring subcellular localization in the mitochondrium, in the endoplasmic reticulum (= ER), in the nucleus, in oil corpuscles or other compartments may also be employed as well as translation promoters such as the 5' lead sequence in tobacco mosaic virus (Gallie et al., Nucl. Acids Res. 15 (1987), 8693 -8711).

A nucleic acid construct, for example an expression cassette may, for example, contain a constitutive promoter or a tissue-specific promoter (preferably the USP or napin promoter) the gene to be expressed and the ER retention signal. For the ER retention signal the KDEL amino acid sequence (lysine, aspartic acid, glutamic acid, leucine) or the KKX amino acid sequence (lysine-lysine-X-stop, wherein X means every other known amino acid) is preferably employed.

For expression in a host organism, for example a plant, the expression cassette is advantageously inserted into a vector such as by way of example a plasmid, a phage or other DNA which allows optimal expression of the genes in the host organism. Examples of suitable plasmids are: in E. coli pLG338, pACYC184, pBR series such as e.g. pBR322, pUC series such as pUC18 or pUC19, M113mp series, pKC30, pRep4, pHS1, pHS2, pPLc236, pMBL24, pLG200, pUR290, pIN-III¹¹³-B1, λgt11 or pBdCl; in Streptomyces pIJ101, pIJ364, pIJ702 or pIJ361; in Bacillus pUB110, pC194 or pBD214; in Corynebacterium pSA77 or pAJ667; in fungi pALS1, pIL2 or pBB116; other advantageous fungal vectors are described by Romanos, M.A. et al., [(1992) "Foreign gene expression in yeast: a review", Yeast 8: 423-488] and by van den Hondel, C.A.M.J.J. et al. [(1991) "Heterologous gene expression in filamentous fungi" as well as in More Gene Manipulations in Fungi [J.W. Bennet & L.L. Lasure, eds., pp. 396-428: Academic Press: San Diego] and in "Gene transfer systems and vector development for filamentous fungi" [van den Hondel, C.A.M.J.J. & Punt, P.J. (1991) in: Applied Molecular Genetics of Fungi, Peberdy, J.F. et al., eds., pp. 1-28, Cambridge University Press: Cambridge]. Examples of advantageous yeast promoters are 2µM, pAG-1, YEp6, YEp13 or pEMBLYe23. Examples of algal or plant promoters are pLGV23, pGHlac⁺, pBIN19, pAK2004, pVKH or pDH51 (see Schmidt, R. and Willmitzer, L., 1988). The vectors identified above or derivatives of the vectors identified above are a small selection of the possible plasmids. Further plasmids are well known to those skilled in the art and may be found, for example, in the book Cloning Vectors (Eds. Pouwels P.H. et al. Elsevier, Amsterdam-New York-Oxford, 1985 , ISBN 0 444 904018). Suitable plant vectors are described inter alia in "Methods in Plant Molecular Biology and Biotechnology" (CRC Press), Ch. 6/7, pp. 71-119. Advantageous vectors are known as shuttle vectors or binary vectors which replicate in E. coli and Agrobacterium.

By vectors is meant with the exception of plasmids all other vectors known to those skilled in the art such as by way of example phages, viruses such as SV40, CMV, baculovirus, adenovirus, transposons, IS elements, phasmids, phagemids, cosmids, linear or circular DNA. These vectors can be replicated autonomously in the host organism or be chromosomally replicated, chromosomal replication being preferred.

In a further embodiment of the vector the expression cassette according to the invention may also advantageously be introduced into the organisms in the form of a linear DNA and be integrated into the genome of the host organism by way of heterologous or homologous recombination. This linear DNA may be composed of a linearized plasmid or only of the expression cassette as vector or the nucleic acid sequences according to the invention.

In a further advantageous embodiment the nucleic acid sequence according to the invention can also be introduced into an organism on its own.

If in addition to the nucleic acid sequence according to the invention further genes are to be introduced into the organism, all together with a reporter gene in a single vector or each single gene with a reporter gene in a vector in each case can be introduced into the organism, whereby the different vectors can be introduced simultaneously or successively.

The vector advantageously contains at least one copy of the nucleic acid sequences according to the invention and/or the expression cassette (= gene construct) according to the invention.

The invention further provides an isolated recombinant expression vector comprising a nucleic acid encoding a polypeptide as depicted in table II, column 5 or 7, wherein expression of the vector in a host cell results in increased tolerance to environmental stress as compared to a wild type variety of the host cell. As used herein, the term "vector" refers to a nucleic acid molecule capable of transporting another nucleic acid to which it has been linked. One type of vector is a "plasmid," which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell or a organelle upon introduction into the host cell, and thereby are replicated along with the host or organelle genome. Moreover, certain vectors are capable of directing the expression of genes to which they are operatively linked. Such vectors are referred to herein as "expression vectors." In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses, and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. As used herein with respect to a recombinant expression vector, "operatively linked" is intended to mean that the nucleotide sequence of interest is linked to the regulatory sequence(s) in a manner which allows for expression of the nucleotide sequence (e.g., in an *in vitro* transcription/translation system or in a host cell when the vector is introduced into the host cell). The term "regulatory sequence" is intended to include promoters, enhancers, and other expression control elements (e.g., polyadenylation signals). Such regulatory sequences are described, for example, in Goeddel, Gene Expression Technology: Methods in Enzymology 185, Academic Press, San Diego, CA (1990) and Gruber and Crosby, in: Methods in Plant Molecular Biology and Biotechnology, eds. Glick and Thompson, Chapter 7, 89-108, CRC Press: Boca Raton, Florida, including the references therein. Regulatory sequences include those that direct constitutive expression of a nucleotide sequence in many types of host cells and those that direct expression of the nucleotide sequence only in certain host cells or under certain conditions. It will be appreciated by those skilled in the art that the design of the expression vector can depend on such factors as the choice of the host cell to be transformed, the level of expression of polypeptide desired, etc. The expression vectors of the invention can be introduced into host cells to thereby produce polypeptides or peptides, including fusion polypeptides or peptides, encoded by nucleic acids as described herein (e.g., SRPs, mutant forms of SRPs, fusion polypeptides, etc.).

The recombinant expression vectors of the invention can be designed for expression of the polypeptide of the invention in plant cells. For example, SRP genes can be expressed in plant cells (See Schmidt, R. and Willmitzer, L., 1988, High efficiency Agrobacterium tumefaciens-mediated transformation of Arabidopsis thaliana leaf and cotyledon explants, Plant Cell Rep. 583-586; Plant Molecular Biology and Biotechnology, C Press, Boca Raton, Florida, chapter 6/7, S.71-119 (1993); F.F. White, B. Jenes et al., Techniques for Gene Transfer, in: Transgenic Plants, Vol. 1, Engineering and Utilization, eds. Kung und R. Wu, 128-43, Academic Press: 1993; Potrykus, 1991, Annu. Rev. Plant Physiol. Plant Molec. Biol. 42:205-225 and references cited therein). Suitable host cells are discussed further in Goeddel, Gene Expression Technology: Methods in Enzymology 185, Academic Press: San Diego, CA (1990). Alternatively, the recombinant expression vector can be transcribed and translated *in vitro,* for example using T7 promoter regulatory sequences and T7 polymerase.

Expression of polypeptides in prokaryotes is most often carried out with vectors containing constitutive or inducible promoters directing the expression of either fusion or non-fusion polypeptides. Fusion vectors add a number of amino acids to a polypeptide encoded therein, usually to the amino terminus of the recombinant polypeptide but also to the C-terminus or fused within suitable regions in the polypeptides. Such fusion vectors typically serve three purposes: 1) to increase expression of a recombinant polypeptide; 2) to increase the solubility of a recombinant polypeptide; and 3) to aid in the purification of a recombinant polypeptide by acting as a ligand in affinity purification. Often, in fusion expression vectors, a proteolytic cleavage site is introduced at the junction of the fusion moiety and the recombinant polypeptide to enable separation of the recombinant polypeptide from the fusion moiety subsequent to purification of the fusion polypeptide. Such enzymes, and their cognate recognition sequences, include Factor Xa, thrombin, and enterokinase.

By way of example the plant expression cassette can be installed in the pRT transformation vector ((a) Toepfer et al., 1993, Methods Enzymol., 217: 66-78; (b) Toepfer et al. 1987, Nucl. Acids. Res. 15: 5890 ff.).

Alternatively, a recombinant vector (= expression vector) can also be transcribed and translated in vitro, e.g. by using the T7 promoter and the T7 RNA polymerase.

Expression vectors employed in prokaryotes frequently make use of inducible systems with and without fusion proteins or fusion oligopeptides, wherein these fusions can ensue in both N-terminal and C-terminal manner or in other useful domains of a protein. Such fusion vectors usually have the following purposes: i.) to increase the RNA expression rate; ii.) to increase the achievable protein synthesis rate; iii.) to increase the solubility of the protein; iv.) or to simplify purification by means of a binding sequence usable for affinity chromatography. Proteolytic cleavage points are also frequently introduced via fusion proteins, which allow cleavage of a portion of the fusion protein and purification. Such recognition sequences for proteases are recognized, e.g. factor Xa, thrombin and enterokinase.

Typical advantageous fusion and expression vectors are pGEX [Pharmacia Biotech Inc; Smith, D.B. and Johnson, K.S. (1988) Gene 67: 31-40], pMAL (New England Biolabs, Beverly, MA) and pRIT5 (Pharmacia, Piscataway, NJ) which contains glutathione S-transferase (GST), maltose binding protein or protein A.

In one embodiment, the coding sequence of the polypeptide of the invention is cloned into a pGEX expression vector to create a vector encoding a fusion polypeptide comprising, from the N-terminus to the C-terminus, GST-thrombin cleavage site-X polypeptide. The fusion polypeptide can be purified by affinity chromatography using glutathione-agarose resin. Recombinant PKSRP unfused to GST can be recovered by cleavage of the fusion polypeptide with thrombin.

Other examples of E. coli expression vectors are pTrc [Amann et al., (1988) Gene 69:301-315] and pET vectors [Studier et al., Gene Expression Technology: Methods in Enzymology 185, Academic Press, San Diego, California (1990) 60-89; Stratagene, Amsterdam, The Netherlands].

Target gene expression from the pTrc vector relies on host RNA polymerase transcription from a hybrid trp-lac fusion promoter. Target gene expression from the pET 11 d vector relies on transcription from a T7 gn10-lac fusion promoter mediated by a co-expressed viral RNA polymerase (T7 gn1). This viral polymerase is supplied by host strains BL21 (DE3) or HMS174(DE3) from a resident I prophage harboring a T7 gn1 gene under the transcriptional control of the lacUV 5 promoter.

In a preferred embodiment of the present invention, the SRPs are expressed in plants and plants cells such as unicellular plant cells (e.g. algae) (See Falciatore et al., 1999, Marine Biotechnology 1(3):239-251 and references therein) and plant cells from higher plants (e.g., the spermatophytes, such as crop plants). A nucleic acid molecule coding for SRP as depicted in table II, column 5 or 7 may be "introduced" into a plant cell by any means, including transfection, transformation or transduction, electroporation, particle bombardment, agroinfection, and the like. One transformation method known to those of skill in the art is the dipping of a flowering plant into an *Agro-bacteria* solution, wherein the *Agrobacteria* contains the nucleic acid of the invention, followed by breeding of the transformed gametes.

Other suitable methods for transforming or transfecting host cells including plant cells can be found in Sambrook, et al., Molecular Cloning: A Laboratory Manual. 2nd, ed., Cold Spring Harbor Laboratory, Cold Spring Harbor Laboratory Press, Cold Spring Harbor, NY, 1989, and other laboratory manuals such as Methods in Molecular Biology, 1995, Vol. 44, Agrobacterium protocols, ed: Gartland and Davey, Humana Press, Totowa, New Jersey. As biotic and abiotic stress tolerance is a general trait wished to be inherited into a wide variety of plants like maize, wheat, rye, oat, triticale, rice, barley, soybean, peanut, cotton, rapeseed and canola, manihot, pepper, sunflower and tagetes, solanaceous plants like potato, tobacco, eggplant, and tomato, Vicia species, pea, alfalfa, bushy plants (coffee, cacao, tea), Salix species, trees (oil palm, coconut), perennial grasses, and forage crops, these crop plants are also preferred target plants for a genetic engineering as one further embodiment of the present invention. Forage crops include, but are not limited to, Wheatgrass, Canarygrass, Bromegrass, Wildrye Grass, Bluegrass, Orchardgrass, Alfalfa, Salfoin, Birdsfoot Trefoil, Alsike Clover, Red Clover, and Sweet Clover.

In one embodiment of the present invention, transfection of a nucleic acid molecule coding for SRP as depicted in table II, column 5 or 7 into a plant is achieved by *Agrobacterium* mediated gene transfer. *Agrobacterium* mediated plant transformation can be performed using for example the GV3101(pMP90) (Koncz and Schell, 1986, Mol. Gen. Genet. 204:383-396) or LBA4404 (Clontech) *Agrobacterium tumefaciens* strain. Transformation can be performed by standard transformation and regeneration techniques (Deblaere et al., 1994, Nucl. Acids Res. 13:4777-4788; Gelvin, Stanton B. and Schilperoort, Robert A, Plant Molecular Biology Manual, 2nd Ed. - Dordrecht : Kluwer Academic Publ., 1995. - in Sect., Ringbuc Zentrale Signatur: BT11-P ISBN 0-7923-2731-4; Glick, Bernard R.; Thompson, John E., Methods in Plant Molecular Biology and Biotechnology, Boca Raton : CRC Press, 1993 360 S., ISBN 0-8493-5164-2). For example, rapeseed can be transformed via cotyledon or hypocotyl transformation (Moloney et al., 1989, Plant cell Report 8:238-242; De Block et al., 1989, Plant Physiol. 91:694-701). Use of antibiotics for *Agrobacterium* and plant selection depends on the binary vector and the *Agrobacterium* strain used for transformation. Rapeseed selection is normally performed using kanamycin as selectable plant marker. *,Agrobacterium* mediated gene transfer to flax can be performed using, for example, a technique described by Mlynarova et al., 1994, Plant Cell Report 13:282-285. Additionally, transformation of soybean can be performed using for example a technique described in European Patent No. 0424 047, U.S. Patent No. 5,322,783, European Patent No. 0397 687, U.S. Patent No. 5,376,543, or U.S. Patent No. 5,169,770. Transformation of maize can be achieved by particle bombardment, polyethylene glycol mediated DNA uptake or via the silicon carbide fiber technique. (See, for example, Freeling and Walbot "The maize handbook" Springer Verlag: New York (1993) ISBN 3-540-97826-7). A specific example of maize transformation is found in U.S. Patent No. 5,990,387, and a specific example of wheat transformation can be found in PCT Application No. WO 93/07256.

According to the present invention, the introduced nucleic acid molecule coding for SRP as depicted in table II, column 5 or 7 may be maintained in the plant cell stably if it is incorporated into a non-chromosomal autonomous replicon or integrated into the plant chromosomes or organelle genome. Alternatively, the introduced SRP may be present on an extra-chromosomal non-replicating vector and be transiently expressed or transiently active.

In one embodiment, a homologous recombinant microorganism can be created wherein the SRP is integrated into a chromosome, a vector is prepared which contains at least a portion of a nucleic acid molecule coding for SRP as depicted in table II, column 5 or 7 into which a deletion, addition, or substitution has been introduced to thereby alter, e.g., functionally disrupt, the SRP gene. Preferably, the SRP gene is a yeast, E.coli, *Synechocystis sp.* gene, but it can be a homolog from a related plant or even from a mammalian or insect source. The vector can be designed such that, upon homologous recombination, the endogenous nucleic acid molecule coding for SRP as depicted in table II, column 5 or 7 is mutated or otherwise altered but still encodes a functional polypeptide (e.g., the upstream regulatory region can be altered to thereby alter the expression of the endogenous SRP). In a preferred embodiment the biological activity of the protein of the invention is increased upon homologous recombination. To create a point mutation via homologous recombination, DNA-RNA hybrids can be used in a technique known as chimeraplasty (Cole-Strauss et al., 1999, Nucleic Acids Research 27(5):1323-1330 and Kmiec, 1999 Gene therapy American Scientist. 87(3):240-247). Homologous recombination procedures in *Physcomitrella patens* are also well known in the art and are contemplated for use herein.

Whereas in the homologous recombination vector, the altered portion of the nucleic acid molecule coding for SRP as depicted in table II, column 5 or 7 is flanked at its 5' and 3' ends by an additional nucleic acid molecule of the SRP gene to allow for homologous recombination to occur between the exogenous SRP gene carried by the vector and an endogenous SRP gene, in a microorganism or plant. The additional flanking SRP nucleic acid molecule is of sufficient length for successful homologous recombination with the endogenous gene. Typically, several hundreds of base pairs up to kilobases of flanking DNA (both at the 5' and 3' ends) are included in the vector. See, e.g., Thomas, K.R., and Capecchi, M.R., 1987, Cell 51:503 for a description of homologous recombination vectors or Strepp et al., 1998, PNAS, 95 (8):4368-4373 for cDNA based recombination in *Physcomitrella patens*). The vector is introduced into a microorganism or plant cell (e.g., via polyethylene glycol mediated DNA), and cells in which the introduced SRP gene has homologously recombined with the endogenous SRP gene are selected using art-known techniques.

Whether present in an extra-chromosomal non-replicating vector or a vector that is integrated into a chromosome, the nucleic acid molecule coding for SRP as depicted in table II, column 5 or 7 preferably resides in a plant expression cassette. A plant expression cassette preferably contains regulatory sequences capable of driving gene expression in plant cells that are operatively linked so that each sequence can fulfill its function, for example, termination of transcription by polyadenylation signals. Preferred polyadenylation signals are those originating from *Agrobacterium tumefaciens* t-DNA such as the gene 3 known as octopine synthase of the Ti-plasmid pTiACH5 (Gielen et al., 1984, EMBO J. 3:835) or functional equivalents thereof but also all other terminators functionally active in plants are suitable. As plant gene expression is very often not limited on transcriptional levels, a plant expression cassette preferably contains other operatively linked sequences like translational enhancers such as the overdrive-sequence containing the 5'-untranslated leader sequence from tobacco mosaic virus enhancing the polypeptide per RNA ratio (Gallie et al., 1987, Nucl. Acids Research 15:8693-8711). Examples of plant expression vectors include those detailed in: Becker, D. et al., 1992, New plant binary vectors with selectable markers located proximal to the left border, Plant Mol. Biol. 20: 1195-1197; and Bevan, M.W., 1984, Binary Agrobacterium vectors for plant transformation, Nucl. Acid. Res. 12:8711-8721; and Vectors for Gene Transfer in Higher Plants; in: Transgenic Plants, Vol. 1, Engineering and Utilization, eds.: Kung and R. Wu, Academic Press, 1993, S. 15-38.

"Transformation" is defined herein as a process for introducing heterologous DNA into a plant cell, plant tissue, or plant. It may occur under natural or artificial conditions using various methods well known in the art. Transformation may rely on any known method for the insertion of foreign nucleic acid sequences into aprokaryotic or eukaryotic host cell. The method is selected based on the host cell being transformed and may include, but is not limited to, viral infection, electroporation, lipofection, and particle bombardment. Such "transformed" cells include stably transformed cells in which the inserted DNA is capable of replication either as an autonomously replicating plasmid or as part of the host chromosome. They also include cells which transiently express the inserted DNA or RNA for limited periods of time. Transformed plant cells, plant tissue, or plants are understood to encompass not only the end product of a transformation process, but also transgenic progeny thereof.

The terms "transformed," "transgenic," and "recombinant" refer to a host organism such as a bacterium or a plant into which a heterologous nucleic acid molecule has been introduced. The nucleic acid molecule can be stably integrated into the genome of the host or the nucleic acid molecule can also be present as an extrachromosomal molecule. Such an extrachromosomal molecule can be auto-replicating. Transformed cells, tissues, or plants are understood to encompass not only the end product of a transformation process, but also transgenic progeny thereof. A "non-transformed," "non-transgenic," or "non-recombinant" host refers to a wild-type organism, e.g., a bacterium or plant, which does not contain the heterologous nucleic acid molecule.

A "transgenic plant", as used herein, refers to a plant which contains a foreign nucleotide sequence inserted into either its nuclear genome or organellar genome. It encompasses further the offspring generations i.e. the T1-, T2- and consecutively generations or BC1-, BC2- and consecutively generation as well as crossbreeds thereof with non-transgenic or other transgenic plants.

The host organism (= transgenic organism) advantageously contains at least one copy of the nucleic acid according to the invention and/or of the nucleic acid construct according to the invention.

In principle all plants can be used as host organism. Preferred transgenic plants are, for example, selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants such as plants advantageously selected from the group of the genus peanut, oilseed rape, canola, sunflower, safflower, olive, sesame, hazelnut, almond, avocado, bay, pumpkin/squash, linseed, soya, pistachio, borage, maize, wheat, rye, oats, sorghum and millet, triticale, rice, barley, cassava, potato, sugarbeet, egg plant, alfalfa, and perennial grasses and forage plants, oil palm, vegetables (brassicas, root vegetables, tuber vegetables, pod vegetables, fruiting vegetables, onion vegetables, leafy vegetables and stem vegetables), buckwheat, Jerusalem artichoke, broad bean, vetches, lentil, dwarf bean, lupin, clover and Lucerne for mentioning only some of them.

In one embodiment of the invention transgenic plants are selected from the group comprising corn, soy, oil seed rape (including canola and winter oil seed reap), cotton, wheat and rice.

In one prefered embodiment, the host plant is selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants and in particular plants mentioned herein above as host plants such as the families and genera mentioned above for example preferred the species *Anacardium occidentale, Calendula officinalis, Carthamus tinctorius, Cichorium intybus, Cynara scolymus, Helianthus annus, Tagetes lucida, Tagetes erecta, Tagetes tenuifolia; Daucus carota; Corylus avellana, Corylus colurna, Borago officinalis; Brassica napus, Brassica rapa* ssp., *Sinapis arvensis Brassica juncea, Brassica juncea var. juncea, Brassica juncea var. crispifolia, Brassica juncea var. foliosa, Brassica nigra, Brassica sinapioides, Melanosinapis communis, Brassica oleracea,* Arabidopsis thaliana, *Anana comosus, Ananas ananas, Bromelia comosa, Carica papaya, Cannabis sative, Ipomoea batatus, Ipomoea pandurata, Convolvulus batatas, Convolvulus tiliaceus, Ipomoea fastigiata, Ipomoea tiliacea, Ipomoea triloba, Convolvulus panduratus, Beta vulgaris, Beta vulgaris var. altissima, Beta vulgaris var. vulgaris, Beta maritima, Beta vulgaris var. perennis, Beta vulgaris var. conditiva, Beta vulgaris var. esculenta, Cucurbita maxima, Cucurbita mixta, Cucurbita pepo, Cucurbita moschata, Olea europaea, Manihot utilissima, Janipha manihot" Jatropha manihot., Manihot aipil, Manihot dulcis, Manihot manihot, Manihot melanobasis, Manihot esculenta, Ricinus communis, Pisum sativum, Pisum arvense, Pisum humile, Medicago sativa, Medicago falcata, Medicago varia, Glycine max Dolichos soja, Glycine gracilis, Glycine hispida, Phaseolus max, Soja hispida, Soja max, Cocos nucifera, Pelargonium grossularioides, Oleum cocoas, Laurus nobilis, Persea americana, Arachis hypogaea, Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense, Linum trigynum, Punica granatum, Gossypium hirsutum, Gossypium arboreum, Gossypium barbadense, Gossypium herbaceum, Gossypium thurberi, Musa nana, Musa acuminata, Musa paradisiaca, Musa* spp., *Elaeis guineensis, Papaver orientale, Papaver rhoeas, Papaver dubium, Sesamum indicum, Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata, , Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum, Avena sativa, Avena fatua, Avena byzantina, Avena fatua var. sativa, Avena hybrida, Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum millet, Panicum militaceum, Zea mays, Triticum aestivum, Triticum durum, Triticum turgidum, Triticum hybernum, Triticum macha, Triticum sativum or Triticum vulgare, Cofea spp., Coffea arabica, Coffea canephora, Coffea liberica, Capsicum annuum, Capsicum annuum var. glabriusculum, Capsicum frutescens, Capsicum annuum, Nicotiana tabacum, Solanum tuberosum, Solanum melongena, Lycopersicon esculentum, Lycopersicon lycopersicum., Lycopersicon pyriforme, Solanum integrifolium, Solanum lycopersicum Theobroma cacao* or *Camellia sinensis.*

Anacardiaceae such as the genera Pistacia, Mangifera, Anacardium e.g. the species *Pistacia vera* [pistachios, Pistazie], *Mangifer indica* [Mango] or *Anacardium occidentale* [Cashew]; Asteraceae such as the genera Calendula, Carthamus, Centaurea, Cichorium, Cynara, Helianthus, Lactuca, Locusta, Tagetes, Valeriana e.g. the species *Calendula officinalis* [Marigold], *Carthamus tinctorius* [safflower], *Centaurea cyanus* [cornflower], *Cichorium intybus* [blue daisy], *Cynara scolymus* [Artichoke], *Helianthus annus* [sunflower], Lactuca sativa, *Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta* [lettuce], *Tagetes lucida, Tagetes erecta or Tagetes tenuifolia* [Marigold]; Apiaceae such as the genera Daucus e.g. the species *Daucus carota* [carrot]; Betulaceae such as the genera Corylus e.g. the species *Corylus avellana* or *Corylus colurna* [hazelnut]; Boraginaceae such as the genera Borago e.g. the species *Borago officinalis* [borage]; Brassicaceae such as the genera Brassica, Melanosinapis, Sinapis, Arabadopsis e.g. the species *Brassica napus, Brassica rapa* ssp. [canola, oilseed rape, turnip rape], *Sinapis arvensis Brassica juncea, Brassica juncea var. juncea, Brassica juncea var. crispifolia, Brassica juncea var. foliosa, Brassica nigra, Brassica sinapioides, Melanosinapis communis* [mustard], *Brassica oleracea* [fodder beet] or Arabidopsis thaliana; Bromeliaceae such as the genera Anana, Bromelia e.g. the species *Anana comosus, Ananas ananas* or *Bromelia comosa* [pineapple]; Caricaceae such as the genera Carica e.g. the species *Carica papaya* [papaya]; Cannabaceae such as the genera Cannabis e.g. the species *Cannabis sative* [hemp], Convolvulaceae such as the genera Ipomea, Convolvulus e.g. the species *Ipomoea batatus, Ipomoea pandurata, Convolvulus batatas, Convolvulus tiliaceus, Ipomoea fastigiata, Ipomoea tiliacea, Ipomoea triloba* or *Convolvulus panduratus* [sweet potato, Man of the Earth, wild potato], Chenopodiaceae such as the genera Beta, i.e. the species *Beta vulgaris, Beta vulgaris var. altissima, Beta vulgaris var. Vulgaris, Beta maritima, Beta vulgaris var. perennis, Beta vulgaris var. conditiva* or *Beta vulgaris var. esculenta* [sugar beet]; Cucurbitaceae such as the genera Cucubita e.g. the species *Cucurbita maxima, Cucurbita mixta, Cucurbita pepo* or *Cucurbita moschata* [pumpkin, squash]; Elaeagnaceae such as the genera Elaeagnus e.g. the species *Olea europaea* [olive]; Ericaceae such as the genera Kalmia e.g. the species *Kalmia latifolia, Kalmia angustifolia, Kalmia microphylla, Kalmia polifolia, Kalmia occidentalis, Cistus chamaerhodendros* or *Kalmia lucida* [American laurel, broad-leafed laurel, calico bush, spoon wood, sheep laurel, alpine laurel, bog laurel, western bog-laurel, swamp-laurel]; Euphorbiaceae such as the genera Manihot, Janipha, Jatropha, Ricinus e.g. the species *Manihot utilissima, Janipha manihot" Jatropha manihot., Manihot aipil, Manihot dulcis, Manihot manihot, Manihot melanobasis, Manihot esculenta* [manihot, arrowroot, tapioca, cassava] or *Ricinus communis* [castor bean, Castor Oil Bush, Castor Oil Plant, Palma Christi, Wonder Tree]; Fabaceae such as the genera Pisum, Albizia, Cathormion, Feuillea, Inga, Pithecolobium, Acacia, Mimosa, Medicajo, Glycine, Dolichos, Phaseolus, Soja e.g. the species *Pisum sativum, Pisum arvense, Pisum humile* [pea], *Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa* speciosa [bastard logwood, silk tree, East Indian Walnut], *Medicago sativa, Medicago falcata, Medicago varia* [alfalfa] *Glycine max Dolichos soja, Glycine gracilis, Glycine hispida, Phaseolus max, Soja hispida* or *Soja max* [soybean]; Geraniaceae such as the genera Pelargonium, Cocos, Oleum e.g. the species *Cocos nucifera, Pelargonium grossularioides* or *Oleum cocois* [coconut]; Gramineae such as the genera Saccharum e.g. the species *Saccharum officinarum;* Juglandaceae such as the genera Juglans, Wallia e.g. the species *Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* or *Wallia nigra* [walnut, black walnut, common walnut, persian walnut, white walnut, butternut, black walnut]; Lauraceae such as the genera Persea, Laurus e.g. the species laurel *Laurus nobilis* [bay, laurel, bay laurel, sweet bay], *Persea americana Persea americana, Persea gratissima* or *Persea persea* [avocado]; Leguminosae such as the genera Arachis e.g. the species *Arachis hypogaea* [peanut]; Linaceae such as the genera Linum, Adenolinum e.g. the species *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* or *Linum trigynum* [flax, linseed]; Lythrarieae such as the genera Punica e.g. the species *Punica granatum* [pomegranate]; Malvaceae such as the genera Gossypium e.g. the species *Gossypium hirsutum, Gossypium arboreum, Gossypium barbadense, Gossypium herbaceum* or *Gossypium thurberi* [cotton]; Musaceae such as the genera Musa e.g. the species *Musa nana, Musa acuminata, Musa paradisiaca, Musa* spp. [banana]; Onagraceae such as the genera Camissonia, Oenothera e.g. the species *Oenothera biennis* or *Camissonia brevipes* [primrose, evening primrose]; Palmae such as the genera Elacis e.g. the species *Elaeis guineensis* [oil plam]; Papaveraceae such as the genera Papaver e.g. the species *Papaver orientale, Papaver rhoeas, Papaver dubium* [poppy, oriental poppy, corn poppy, field poppy, shirley poppies, field poppy, long-headed poppy, long-pod poppy]; Pedaliaceae such as the genera Sesamum e.g. the species *Sesamum indicum* [sesame]; Piperaceae such as the genera Piper, Artanthe, Peperomia, Steffensia e.g. the species *Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata.* [Cayenne pepper, wild pepper]; Poaceae such as the genera Hordeum, Secale, Avena, Sorghum, Andropogon, Holcus, Panicum, Oryza, Zea, Triticum e.g. the species *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [barley, pearl barley, foxtail barley, wall barley, meadow barley], *Secale cereale* [rye], Avena sativa, *Avena fatua, Avena byzantina, Avena fatua var. sativa, Avena hybrida* [oat], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum* millet, *Panicum militaceum [Sorghum, millet], Oryza sativa, Oryza latifolia* [rice], *Zea mays* [corn, maize] *Triticum aestivum, Triticum durum, Triticum turgidum, Triticum hybernum, Triticum macha, Triticum sativum* or *Triticum vulgare* [wheat, bread wheat, common wheat], Proteaceae such as the genera Macadamia e.g. the species *Macadamia intergrifolia* [macadamia]; Rubiaceae such as the genera Coffea e.g. the species Cofea spp., *Coffea arabica, Coffea canephora* or *Coffea liberi*ca [coffee]; Scrophulariaceae such as the genera Verbascum e.g. the species *Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* or *Verbascum thapsus* [mullein, white moth mullein, nettle-leaved mullein, dense-flowered mullein, silver mullein, long-leaved mullein, white mullein, dark mullein, greek mullein, orange mullein, purple mullein, hoary mullein, great mullein]; Solanaceae such as the genera Capsicum, Nicotiana, Solanum, Lycopersicon e.g. the species *Capsicum annuum, Capsicum annuum var. glabriusculum, Capsicum frutescens* [pepper], *Capsicum annuum* [paprika], *Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [tobacco], *Solanum tuberosum* [potato], *Solanum melongena* [egg-plant] (*Lycopersicon esculentum, Lycopersicon lycopersicum., Lycopersicon pyriforme, Solanum integrifolium* or *Solanum lycopersicum [tomato*]; Sterculiaceae such as the genera Theobroma e.g. the species *Theobroma cacao* [cacao]; Theaceae such as the genera Camellia e.g. the species *Camellia sinensis*) [tea].

The introduction of the nucleic acids according to the invention, the expression cassette or the vector into organisms, plants for example, can in principle be done by all of the methods known to those skilled in the art. The introduction of the nucleic acid sequences gives rise to recombinant or transgenic organisms.

Unless otherwise specified, the terms "polynucleotides", "nucleic acid" and "nucleic acid molecule" as used herein are interchangeably. Unless otherwise specified, the terms "peptide", "polypeptide" and "protein" are interchangeably in the present context. The term "sequence" may relate to polynucleotides, nucleic acids, nucleic acid molecules, peptides, polypeptides and proteins, depending on the context in which the term "sequence" is used. The terms "gene(s)", "polynucleotide", "nucleic acid sequence", "nucleotide sequence", or "nucleic acid molecule(s)" as used herein refers to a polymeric form of nucleotides of any length, either ribonucleotides or deoxyribonucleotides. The terms refer only to the primary structure of the molecule.

Thus, the terms "gene(s)", "polynucleotide", "nucleic acid sequence", "nucleotide sequence", or "nucleic acid molecule(s)" as used herein include double- and single-stranded DNA and RNA. They also include known types of modifications, for example, methylation, "caps", substitutions of one or more of the naturally occurring nucleotides with an analog. Preferably, the DNA or RNA sequence of the invention comprises a coding sequence encoding the herein defined polypeptide.

The genes of the invention, coding for an activity selected from the group consisting of: 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Yal049c-protein, YCR059C-protein, YEL005C-protein, YER156C-protein, Yfr042w-protein, YGL045W-protein, and YOR024w-protein are also called "SRP gene".

A "coding sequence" is a nucleotide sequence, which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleotide sequences or genomic DNA, while introns may be present as well under certain circumstances.

The transfer of foreign genes into the genome of a plant is called transformation. In doing this the methods described for the transformation and regeneration of plants from plant tissues or plant cells are utilized for transient or stable transformation. Suitable methods are protoplast transformation by poly(ethylene glycol)-induced DNA uptake, the "biolistic" method using the gene cannon - referred to as the particle bombardment method, electroporation, the incubation of dry embryos in DNA solution, microinjection and gene transfer mediated by Agrobacterium. Said methods are described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector which is suitable for transforming Agrobacterium tumefaciens, for example pBin19 (Bevan et al., Nucl. Acids Res. 12 (1984) 8711). Agrobacteria transformed by such a vector can then be used in known manner for the transformation of plants, in particular of crop plants such as by way of example tobacco plants, for example by bathing bruised leaves or chopped leaves in an agrobacterial solution and then culturing them in suitable media. The transformation of plants by means of Agrobacterium tumefaciens is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

Agrobacteria transformed by an expression vector according to the invention may likewise be used in known manner for the transformation of plants such as test plants like Arabidopsis or crop plants such as cereal crops, corn, oats, rye, barley, wheat, soybean, rice, cotton, sugar beet, canola, sunflower, flax, hemp, potatoes, tobacco, tomatoes, carrots, paprika, oilseed rape, tapioca, cassava, arrowroot, tagetes, alfalfa, lettuce and the various tree, nut and vine species, in particular of oil-containing crop plants such as soybean, peanut, castor oil plant, sunflower, corn, cotton, flax, oilseed rape, coconut, oil palm, safflower (Carthamus tinctorius) or cocoa bean, e.g. by bathing bruised leaves or chopped leaves in an agrobacterial solution and then culturing them in suitable media.

The genetically modified plant cells may be regenerated by all of the methods known to those skilled in the art. Appropriate methods can be found in the publications referred to above by S.D. Kung and R. Wu, Potrykus or Höfgen and Willmitzer.

Accordingly, a further aspect of the invention relates to transgenic organisms transformed by at least one nucleic acid sequence, expression cassette or vector according to the invention as well as cells, cell cultures, tissue, parts - such as, for example, leaves, roots, etc. in the case of plant organisms - or reproductive material derived from such organisms. The terms "host organism", "host cell", "recombinant (host) organism" and "transgenic (host) cell" are used here interchangeably. Of course these terms relate not only to the particular host organism or the particular target cell but also to the descendants or potential descendants of these organisms or cells. Since, due to mutation or environmental effects certain modifications may arise in successive generations, these descendants need not necessarily be identical with the parental cell but nevertheless are still encompassed by the term as used here.

For the purposes of the invention "transgenic" or "recombinant" means with regard for example to a nucleic acid sequence, an expression cassette (= gene construct, nucleic acid construct) or a vector containing the nucleic acid sequence according to the invention or an organism transformed by the nucleic acid sequences, expression cassette or vector according to the invention all those constructions produced by genetic engineering methods in which either
a) the nucleic acid sequence depicted in table I, column 5 or 7 or its derivatives or parts thereof or
b) a genetic control sequence functionally linked to the nucleic acid sequence described under (a), for example a 3'- and/or 5'- genetic control sequence such as a promoter or terminator, or
c) (a) and (b)
are not found in their natural, genetic environment or have been modified by genetic engineering methods, wherein the modification may by way of example be a substitution, addition, deletion, inversion or insertion of one or more nucleotide residues. Natural genetic environment means the natural genomic or chromosomal locus in the organism of origin or inside the host organism or presence in a genomic library. In the case of a genomic library the natural genetic environment of the nucleic acid sequence is preferably retained at least in part. The environment borders the nucleic acid sequence at least on one side and has a sequence length of at least 50 bp, preferably at least 500 bp, particularly preferably at least 1,000 bp, most particularly preferably at least 5,000 bp. A naturally occurring expression cassette - for example the naturally occurring combination of the natural promoter of the nucleic acid sequence according to the invention with the corresponding delta-8-desaturase, delta-9-elongase and/or delta-5-desaturase gene - turns into a transgenic expression cassette when the latter is modified by unnatural, synthetic ("artificial") methods such as by way of example a mutagenation. Appropriate methods are described by way of example in US 5,565,350 or WO 00/15815.

Suitable organisms or host organisms for the nucleic acid, expression cassette or vector according to the invention are advantageously in principle all organisms, which are suitable for the expression of recombinant genes as described above. Further examples which may be mentioned are plants such as Arabidopsis, Asteraceae such as Calendula or crop plants such as soybean, peanut, castor oil plant, sunflower, flax, corn, cotton, flax, oilseed rape, coconut, oil palm, safflower (Carthamus tinctorius) or cocoa bean.

In one embodiment of the invention host plants for the nucleic acid, expression cassette or vector according to the invention are selected from the group comprising corn, soy, oil seed rape (including canola and winter oil seed reap), cotton, wheat and rice.

A further object of the invention relates to the use of a nucleic acid construct, e.g. an expression cassette, containing DNA sequences encoding polypeptides shown in table II or DNA sequences hybridizing therewith for the transformation of plant cells, tissues or parts of plants.

In doing so, depending on the choice of promoter, the sequences of shown in table I can be expressed specifically in the leaves, in the seeds, the nodules, in roots, in the stem or other parts of the plant. Those transgenic plants overproducing sequences as depicted in table I , the reproductive material thereof, together with the plant cells, tissues or parts thereof are a further object of the present invention.

The expression cassette or the nucleic acid sequences or construct according to the invention containing sequences according to table I can, moreover, also be employed for the transformation of the organisms identified by way of example above such as bacteria, yeasts, filamentous fungi and plants.

Within the framework of the present invention, increased tolerance and/or resistance to environmental stress means, for example, the artificially acquired trait of increased environmental stress resistancee due to functional over expression of polypeptide sequences of table II encoded by the corresponding nucleic acid molecules as depicted in table I, column 5 or 7 and/or homologs in the organisms according to the invention, advantageously in the transgenic plants according to the invention, by comparison with the nongenetically modified initial plants at least for the duration of at least one plant generation.

A constitutive expression of the polypeptide sequences of the of table II encoded by the corresponding nucleic acid molecule as depicted in table I, column 5 or 7 and/or homologs is, moreover, advantageous. On the other hand, however, an inducible expression may also appear desirable. Expression of the polypeptide sequences of the invention can be either direct to the cytsoplasm or the organelles preferably the plastids of the host cells, preferably the plant cells.

The efficiency of the expression of the sequences of the of table II encoded by the corresponding nucleic acid molecule as depicted in table I, column 5 or 7 and/or homologs can be determined, for example, in vitro by shoot meristem propagation. In addition, an expression of the sequences of of table II encoded by the corresponding nucleic acid molecule as depicted in table I, column 5 or 7 and/or homologs modified in nature and level and its effect on the metabolic pathways performance can be tested on test plants in greenhouse trials.

An additional object of the invention comprises transgenic organisms such as transgenic plants transformed by an expression cassette containing sequences of as depicted in table I, column 5 or 7 according to the invention or DNA sequences hybridizing therewith, as well as transgenic cells, tissue, parts and reproduction material of such plants. Particular preference is given in this case to transgenic crop plants such as by way of example barley, wheat, rye, oats, corn, soybean, rice, cotton, sugar beet, oilseed rape and canola, sunflower, flax, hemp, thistle, potatoes, tobacco, tomatoes, tapioca, cassava, arrowroot, alfalfa, lettuce and the various tree, nut and vine species.

In one embodiment of the invention transgenic plants transformed by an expression cassette containing sequences of as depicted in table I, column 5 or 7 according to the invention or DNA sequences hybridizing therewith are selected from the group comprising corn, soy, oil seed rape (including canola and winter oil seed rape), cotton, wheat and rice.

For the purposes of the invention plants are mono- and dicotyledonous plants, mosses or algae.

A further refinement according to the invention are transgenic plants as described above which contain a nucleic acid sequence or construct according to the invention or a expression cassette according to the invention.

However, transgenic also means that the nucleic acids according to the invention are located at their natural position in the genome of an organism, but that the sequence has been modified in comparison with the natural sequence and/or that the regulatory sequences of the natural sequences have been modified. Preferably, transgenic/recombinant is to be understood as meaning the transcription of the nucleic acids of the invention and shown in table I, occurs at a non-natural position in the genome, that is to say the expression of the nucleic acids is homologous or, preferably, heterologous. This expression can be transiently or of a sequence integrated stably into the genome.

The term "transgenic plants" used in accordance with the invention also refers to the progeny of a transgenic plant, for example the T₁, T₂, T₃ and subsequent plant generations or the BC₁, BC₂, BC₃ and subsequent plant generations. Thus, the transgenic plants according to the invention can be raised and selfed or crossed with other individuals in order to obtain further transgenic plants according to the invention. Transgenic plants may also be obtained by propagating transgenic plant cells vegetatively. The present invention also relates to transgenic plant material, which can be derived from a transgenic plant population according to the invention. Such material includes plant cells and certain tissues, organs and parts of plants in all their manifestations, such as seeds, leaves, anthers, fibers, tubers, roots, root hairs, stems, embryo, calli, cotelydons, petioles, harvested material, plant tissue, reproductive tissue and cell cultures, which are derived from the actual transgenic plant and/or can be used for bringing about the transgenic plant.

Any transformed plant obtained according to the invention can be used in a conventional breeding scheme or in in vitro plant propagation to produce more transformed plants with the same characteristics and/or can be used to introduce the same characteristic in other varieties of the same or related species. Such plants are also part of the invention. Seeds obtained from the transformed plants genetically also contain the same characteristic and are part of the invention. As mentioned before, the present invention is in principle applicable to any plant and crop that can be transformed with any of the transformation method known to those skilled in the art.

Advantageous inducible plant promoters are by way of example the PRP1 promoter [Ward et al., Plant.Mol. Biol.22(1993), 361-366], a promoter inducible by benzenesulfonamide (EP 388 186), a promoter inducible by tetracycline [Gatz et al., (1992) Plant J. 2,397-404], a promoter inducible by salicylic acid (WO 95/19443), a promoter inducible by abscisic acid (EP 335 528) and a promoter inducible by ethanol or cyclohexanone (WO93/21334). Other examples of plant promoters which can advantageously be used are the promoter of cytosolic FBPase from potato, the ST-LSI promoter from potato (Stockhaus et al., EMBO J. 8 (1989) 2445-245), the promoter of phosphoribosyl pyrophosphate amidotransferase from Glycine max (see also gene bank accession number U87999) or a nodiene-specific promoter as described in EP 249 676. Particular advantageous are those promoters which ensure expression expression upon the early onset of environmental stress like for example drought or cold.

In one embodiment seed-specific promoters may be used for monocotylodonous or dicotylodonous plants.

In principle all natural promoters with their regulation sequences can be used like those named above for the expression cassette according to the invention and the method according to the invention. Over and above this, synthetic promoters may also advantageously be used.

In the preparation of an expression cassette various DNA fragments can be manipulated in order to obtain a nucleotide sequence, which usefully reads in the correct direction and is equipped with a correct reading frame. To connect the DNA fragments (= nucleic acids according to the invention) to one another adaptors or linkers may be attached to the fragments.

The promoter and the terminator regions can usefully be provided in the transcription direction with a linker or polylinker containing one or more restriction points for the insertion of this sequence. Generally, the linker has 1 to 10, mostly 1 to 8, preferably 2 to 6, restriction points. In general the size of the linker inside the regulatory region is less than 100 bp, frequently less than 60 bp, but at least 5 bp. The promoter may be both native or homologous as well as foreign or heterologous to the host organism, for example to the host plant. In the 5'-3' transcription direction the expression cassette contains the promoter, a DNA sequence which shown in table I and a region for transcription termination. Different termination regions can be exchanged for one another in any desired fashion.

As also used herein, the terms "nucleic acid" and "nucleic acid molecule" are intended to include DNA molecules (e.g., cDNA or genomic DNA) and RNA molecules (e.g., mRNA) and analogs of the DNA or RNA generated using nucleotide analogs. This term also encompasses untranslated sequence located at both the 3' and 5' ends of the coding region of the gene: at least about 1000 nucleotides of sequence upstream from the 5' end of the coding region and at least about 200 nucleotides of sequence downstream from the 3' end of the coding region of the gene. The nucleic acid molecule can be single-stranded or double-stranded, but preferably is double-stranded DNA.

An "isolated" nucleic acid molecule is one that is substantially separated from other nucleic acid molecules, which are present in the natural source of the nucleic acid. That means other nucleic acid molecules are present in an amount less than 5% based on weight of the amount of the desired nucleic acid, preferably less than 2% by weight, more preferably less than 1% by weight, most preferably less than 0.5% by weight. Preferably, an "isolated" nucleic acid is free of some of the sequences that naturally flank the nucleic acid (i.e., sequences located at the 5' and 3' ends of the nucleic acid) in the genomic DNA of the organism from which the nucleic acid is derived. For example, in various embodiments, the isolated stress related protein encoding nucleic acid molecule can contain less than about 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be free from some of the other cellular material with which it is naturally associated, or culture medium when produced by recombinant techniques, or chemical precursors or other chemicals when chemically synthesized.

A nucleic acid molecule of the present invention, e.g., a nucleic acid molecule encoding an SRP or a portion thereof which confers tolerance and/or resistance to environmental stress and increased biomass production in plants, can be isolated using standard molecular biological techniques and the sequence information provided herein. For example, an Arabidopsis thaliana stress related protein encoding cDNA can be isolated from a A. thaliana c-DNA library or a *Synechocystis sp., Brassica napus, Glycine max*, *Zea mays* or *Oryza sativa* stress related protein encoding cDNA can be isolated from a *Synechocystis sp., Brassica napus, Glycine max, Zea mays* or *Oryza sativa* c-DNA library respectively using all or portion of one of the sequences shown in table I. Moreover, a nucleic acid molecule encompassing all or a portion of one of the sequences of table I can be isolated by the polymerase chain reaction using oligonucleotide primers designed based upon this sequence. For example, mRNA can be isolated from plant cells (e.g., by the guanidinium-thiocyanate extraction procedure of Chirgwin et al., 1979 Biochemistry 18:5294-5299) and cDNA can be prepared using reverse transcriptase (e.g., Moloney MLV reverse transcriptase, available from Gibco/BRL, Bethesda, MD; or AMV reverse transcriptase, available from Seikagaku America, Inc., St. Petersburg, FL). Synthetic oligonucleotide primers for polymerase chain reaction amplification can be designed based upon one of the nucleotide sequences shown in table I. A nucleic acid molecule of the invention can be amplified using cDNA or, alternatively, genomic DNA, as a template and appropriate oligonucleotide primers according to standard PCR amplification techniques. The nucleic acid molecule so amplified can be cloned into an appropriate vector and characterized by DNA sequence analysis. Furthermore, oligonucleotides corresponding to a SRP encoding nucleotide sequence can be prepared by standard synthetic techniques, e.g., using an automated DNA synthesizer.

In a preferred embodiment, an isolated nucleic acid molecule of the invention comprises one of the nucleotide sequences shown in table I encoding the SRP (i.e., the "coding region"), as well as 5' untranslated sequences and 3' untranslated sequences.

Moreover, the nucleic acid molecule of the invention can comprise only a portion of the coding region of one of the sequences of the nucleic acid of table I, for example, a fragment which can be used as a probe or primer or a fragment encoding a biologically active portion of a SRP.

Portions of proteins encoded by the SRP encoding nucleic acid molecules of the invention are preferably biologically active portions described herein. As used herein, the term "biologically active portion of" a SRP is intended to include a portion, e.g., a domain/motif, of stress related protein that participates in a stress tolerance and/or resistance response in a plant. To determine whether a SRP, or a biologically active portion thereof, results in increased stress tolerance in a plant, a stress analysis of a plant comprising the SRP may be performed. Such analysis methods are well known to those skilled in the art, as detailed in the Examples. More specifically, nucleic acid fragments encoding biologically active portions of a SRP can be prepared by isolating a portion of one of the sequences of the nucleic acid of table I expressing the encoded portion of the SRP or peptide (e.g., by recombinant expression in vitro) and assessing the activity of the encoded portion of the SRP or peptide.

Biologically active portions of a SRP are encompassed by the present invention and include peptides comprising amino acid sequences derived from the amino acid sequence of a SRP encoding gene, or the amino acid sequence of a protein homologous to a SRP, which include fewer amino acids than a full length SRP or the full length protein which is homologous to a SRP, and exhibits at least some enzymatic or biological activity of a SRP. Typically, biologically active portions (e.g., peptides which are, for example, 5, 10, 15, 20, 30, 35, 36, 37, 38, 39, 40, 50, 100 or more amino acids in length) comprise a domain or motif with at least one activity of a SRP. Moreover, other biologically active portions in which other regions of the protein are deleted, can be prepared by recombinant techniques and evaluated for one or more of the activities described herein. Preferably, the biologically active portions of a SRP include one or more selected domains/motifs or portions thereof having biological activity.

The term "biological active portion" or "biological activity" means a polypeptide as depicted in table II, column 3 or a portion of said polypeptide which still has at least 10 % or 20 %, preferably 20 %, 30 %, 40 % or 50 %, especially preferably 60 %, 70 % or 80 % of the enzymatic or biological activity of the natural or starting enzyme or protein.

In the process according to the invention nucleic acid sequences can be used, which, if appropriate, contain synthetic, non-natural or modified nucleotide bases, which can be incorporated into DNA or RNA. Said synthetic, non-natural or modified bases can for example increase the stability of the nucleic acid molecule outside or inside a cell. The nucleic acid molecules of the invention can contain the same modifications as aforementioned.

As used in the present context the term "nucleic acid molecule" may also encompass the untranslated sequence located at the 3' and at the 5' end of the coding gene region, for example at least 500, preferably 200, especially preferably 100, nucleotides of the sequence upstream of the 5' end of the coding region and at least 100, preferably 50, especially preferably 20, nucleotides of the sequence downstream of the 3' end of the coding gene region. It is often advantageous only to choose the coding region for cloning and expression purposes.

Preferably, the nucleic acid molecule used in the process according to the invention or the nucleic acid molecule of the invention is an isolated nucleic acid molecule.

An "isolated" polynucleotide or nucleic acid molecule is separated from other polynucleotides or nucleic acid molecules, which are present in the natural source of the nucleic acid molecule. An isolated nucleic acid molecule may be a chromosomal fragment of several kb, or preferably, a molecule only comprising the coding region of the gene. Accordingly, an isolated nucleic acid molecule of the invention may comprise chromosomal regions, which are adjacent 5' and 3' or further adjacent chromosomal regions, but preferably comprises no such sequences which naturally flank the nucleic acid molecule sequence in the genomic or chromosomal context in the organism from which the nucleic acid molecule originates (for example sequences which are adjacent to the regions encoding the 5'- and 3'-UTRs of the nucleic acid molecule). In various embodiments, the isolated nucleic acid molecule used in the process according to the invention may, for example comprise less than approximately 5 kb, 4 kb, 3 kb, 2 kb, 1 kb, 0.5 kb or 0.1 kb nucleotide sequences which naturally flank the nucleic acid molecule in the genomic DNA of the cell from which the nucleic acid molecule originates.

The nucleic acid molecules used in the process, for example the polynucleotide of the invention or of a part thereof can be isolated using molecular-biological standard techniques and the sequence information provided herein. Also, for example a homologous sequence or homologous, conserved sequence regions at the DNA or amino acid level can be identified with the aid of comparison algorithms. The former can be used as hybridization probes under standard hybridization techniques (for example those described in Sambrook et al., Molecular Cloning: A Laboratory Manual. 2nd Ed., Cold Spring Harbor Laboratory, Cold Spring Harbor Laboratory Press, Cold Spring Harbor, NY, 1989) for isolating further nucleic acid sequences useful in this process.

A nucleic acid molecule encompassing a complete sequence of the nucleic acid molecules used in the process, for example the polynucleotide of the invention, or a part thereof may additionally be isolated by polymerase chain reaction, oligonucleotide primers based on this sequence or on parts thereof being used. For example, a nucleic acid molecule comprising the complete sequence or part thereof can be isolated by polymerase chain reaction using oligonucleotide primers which have been generated on the basis of this very sequence. For example, mRNA can be isolated from cells (for example by means of the guanidinium thiocyanate extraction method of Chirgwin et al. (1979) Biochemistry 18:5294-5299) and cDNA can be generated by means of reverse transcriptase (for example Moloney MLV reverse transcriptase, available from Gibco/BRL, Bethesda, MD, or AMV reverse transcriptase, obtainable from Seikagaku America, Inc., St.Petersburg, FL).

Synthetic oligonucleotide primers for the amplification, e.g. as shown in table III, column 7, by means of polymerase chain reaction can be generated on the basis of a sequence shown herein, for example the sequence shown in table I, columns 5 and 7 or the sequences derived from table II, columns 5 and 7.

Moreover, it is possible to identify conserved protein by carrying out protein sequence alignments with the polypeptide encoded by the nucleic acid molecules of the present invention, in particular with the sequences encoded by the nucleic acid molecule shown in, column 5 or 7 of Table I, from which conserved regions, and in turn, degenerate primers can be derived.

Conserved regions are those, which show a very little variation in the amino acid in one particular position of several homologs from different origin. The consenus sequence and polypeptide motifs shown in column 7 of Table IV are derived from said aligments. Moreover, it is possible to identify conserved regions from various organisms by carrying out protein sequence alignments with the polypeptide encoded by the nucleic acid of the present invention, in particular with the sequences encoded by the polypeptide molecule shown in column 5 or 7 of Table II, from which conserved regions, and in turn, degenerate primers can be derived.

In one advantageous embodiment, in the method of the present invention the activity of a polypeptide is increased comprising or consisting of a consensus sequence or a polypeptide motif shown in table IV column 7 and in one another embodiment, the present invention relates to a polypeptide comprising or consisting of a consensus sequence or a polypeptide motif shown in table IV, column 7 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid. In one embodiment not more than 15%, preferably 10%, even more preferred 5%, 4%, 3%, or 2%, most preferred 1% or 0% of the amino acid position indicated by a letter are/is replaced another amino acid. In one embodiment 20 or less, preferably 15 or 10, preferably 9, 8, 7, or 6, more preferred 5 or 4, even more preferred 3, even more preferred 2, even more preferred 1, most preferred 0 amino acids are inserted into a consensus sequence or protein motif.

The consensus sequence was derived from a multiple alignment of the sequences as listed in table II. The letters represent the one letter amino acid code and indicate that the amino acids are conserved in at least 80% of the aligned proteins, whereas the letter X stands for amino acids, which are not conserved in at least 80% of the aligned sequences. The consensus sequence starts with the first conserved amino acid in the alignment, and ends with the last conserved amino acid in the alignment of the investigated sequences. The number of given X indicates the distances between conserved amino acid residues, e.g. Y-x(21,23)-F means that conserved tyrosine and phenylalanine residues in the alignment are separated from each other by minimum 21 and maximum 23 amino acid residues in the alignment of all investigated sequences.

Conserved domains were identified from all sequences and are described using a subset of the standard Prosite notation, e.g the pattern Y-x(21,23)-[FW] means that a conserved tyrosine is separated by minimum 21 and maximum 23 amino acid residues from either a phenylalanine or tryptophane. Patterns had to match at least 80% of the investigated proteins.

Conserved patterns were identified with the software tool MEME version 3.5.1 or manually. MEME was developed by Timothy L. Bailey and Charles Elkan, Dept. of Computer Science and Engeneering, University of California, San Diego, USA and is described by Timothy L. Bailey and Charles Elkan [Fitting a mixture model by expectation maximization to discover motifs in biopolymers, Proceedings of the Second International Conference on Intelligent Systems for Molecular Biology, pp. 28-36, AAAI Press, Menlo Park, California, 1994]. The source code for the stand-alone program is public available from the San Diego Supercomputer center (http://meme.sdsc.edu).

For identifying common motifs in all sequences with the software tool MEME, the following settings were used: -maxsize 500000, -nmotifs 15, -evt 0.001, -maxw 60, - distance 1 e-3, -minsites number of sequences used for the analysis. Input sequences for MEME were non-aligned sequences in Fasta format. Other parameters were used in the default settings in this software version.

Prosite patterns for conserved domains were generated with the software tool Pratt version 2.1 or manually. Pratt was developed by Inge Jonassen, Dept. of Informatics, University of Bergen, Norway and is described by Jonassen et al. [I.Jonassen, J.F.Collins and D.G.Higgins, Finding flexible patterns in unaligned protein sequences, Protein Science 4 (1995), pp. 1587-1595; I.Jonassen, Efficient discovery of conserved patterns using a pattern graph, Submitted to CABIOS Febr. 1997]. The source code (ANSI C) for the stand-alone program is public available, e.g. at establisched Bioinformatic centers like EBI (European Bioinformatics Institute).

For generating patterns with the software tool Pratt, following settings were used: PL (max Pattern Length): 100, PN (max Nr of Pattern Symbols): 100, PX (max Nr of consecutive x's): 30, FN (max Nr of flexible spacers): 5, FL (max Flexibility): 30, FP (max Flex.Product): 10, ON (max number patterns): 50. Input sequences for Pratt were distinct regions of the protein sequences exhibiting high similarity as identified from software tool MEME. The minimum number of sequences, which have to match the generated patterns (CM, min Nr of Seqs to Match) was set to at least 80% of the provided sequences. Parameters not mentioned here were used in their default settings.

The Prosite patterns of the conserved domains can be used to search for protein sequences matching this pattern. Various establisched Bioinformatic centers provide public internet portals for using those patterns in database searches (e.g. PIR [Protein Information Resource, located at Georgetown University Medical Center] or ExPASy [Expert Protein Analysis System]). Alternatively, stand-alone software is available, like the program Fuzzpro, which is part of the EMBOSS software package. For example, the program Fuzzpro not only allows to search for an exact pattern-protein match but also allows to set various ambiguities in the performed search.

The alignment was performed with the software ClustalW (version 1.83) and is described by Thompson et al. [Thompson, J.D., Higgins, D.G. and Gibson, T.J. (1994) CLUSTAL W: improving the sensitivity of progressive multiple sequence alignment through sequence weighting, positions-specific gap penalties and weight matrix choice. Nucleic Acids Research, 22:4673-4680]. The source code for the stand-alone program is public available from the European Molecular Biology Laboratory; Heidelberg, Germany. The analysis was performed using the default parameters of ClustalW v1.83 (gap open penalty: 10.0; gap extension penalty: 0.2; protein matrix: Gonnet; pprotein/DNA endgap: -1; protein/DNA gapdist: 4).

Degenerated primers can then be utilized by PCR for the amplification of fragments of novel proteins having above-mentioned activity, e.g. conferring the increased tolerance and/or resistance to environmental stress and increased biomass production as compared to a corresponding non-transformed wild type plant cell, plant or part thereof after increasing the expression or activity or having the activity of a protein as shown in table II, column 3 or further functional homologs of the polypeptide of the invention from other organisms.

These fragments can then be utilized as hybridization probe for isolating the complete gene sequence. As an alternative, the missing 5' and 3' sequences can be isolated by means of RACE-PCR. A nucleic acid molecule according to the invention can be amplified using cDNA or, as an alternative, genomic DNA as template and suitable oligonucleotide primers, following standard PCR amplification techniques. The nucleic acid molecule amplified thus can be cloned into a suitable vector and characterized by means of DNA sequence analysis. Oligonucleotides, which correspond to one of the nucleic acid molecules used in the process can be generated by standard synthesis methods, for example using an automatic DNA synthesizer.

Nucleic acid molecules which are advantageously for the process according to the invention can be isolated based on their homology to the nucleic acid molecules disclosed herein using the sequences or part thereof as hybridization probe and following standard hybridization techniques under stringent hybridization conditions. In this context, it is possible to use, for example, isolated nucleic acid molecules of at least 15, 20, 25, 30, 35, 40, 50, 60 or more nucleotides, preferably of at least 15, 20 or 25 nucleotides in length which hybridize under stringent conditions with the above-described nucleic acid molecules, in particular with those which encompass a nucleotide sequence ofthe nucleic acid molecule used in the process of the invention or encoding a protein used in the invention or of the nucleic acid molecule of the invention. Nucleic acid molecules with 30, 50, 100, 250 or more nucleotides may also be used.

The term "homology" means that the respective nucleic acid molecules or encoded proteins are functionally and/or structurally equivalent. The nucleic acid molecules that are homologous to the nucleic acid molecules described above and that are derivatives of said nucleic acid molecules are, for example, variations of said nucleic acid molecules which represent modifications having the same biological function, in particular encoding proteins with the same or substantially the same biological function. They may be naturally occurring variations, such as sequences from other plant varieties or species, or mutations. These mutations may occur naturally or may be obtained by mutagenesis techniques. The allelic variations may be naturally occurring allelic variants as well as synthetically produced or genetically engineered variants. Structurally equivalents can, for example, be identified by testing the binding of said polypeptide to antibodies or computer based predictions. Structurally equivalent have the similar immunological characteristic, e.g. comprise similar epitopes.

By "hybridizing" it is meant that such nucleic acid molecules hybridize under conventional hybridization conditions, preferably under stringent conditions such as described by, e.g., Sambrook (Molecular Cloning; A Laboratory Manual, 2nd Edition, Cold Spring Harbor Laboratory Press, Cold Spring Harbor, NY (1989)) or in Current Protocols in Molecular Biology, John Wiley & Sons, N. Y. (1989), 6.3.1-6.3.6.

According to the invention, DNA as well as RNA molecules of the nucleic acid of the invention can be used as probes. Further, as template for the identification of functional homologues Northern blot assays as well as Southern blot assays can be performed. The Northern blot assay advantageously provides further informations about the expressed gene product: e.g. expression pattern, occurance of processing steps, like splicing and capping, etc. The Southern blot assay provides additional information about the chromosomal localization and organization of the gene encoding the nucleic acid molecule of the invention.

A preferred, nonlimiting example of stringent hydridization conditions are hybridizations in 6 x sodium chloride/sodium citrate (= SSC) at approximately 45°C, followed by one or more wash steps in 0.2 x SSC, 0.1 % SDS at 50 to 65°C, for example at 50°C, 55°C or 60°C. The skilled worker knows that these hybridization conditions differ as a function of the type of the nucleic acid and, for example when organic solvents are present, with regard to the temperature and concentration of the buffer. The temperature under "standard hybridization conditions" differs for example as a function of the type of the nucleic acid between 42°C and 58°C, preferably between 45°C and 50°C in an aqueous buffer with a concentration of 0.1 x 0.5 x, 1 x, 2x, 3x, 4x or 5 x SSC (pH 7.2). If organic solvent(s) is/are present in the abovementioned buffer, for example 50% formamide, the temperature under standard conditions is approximately 40°C, 42°C or 45°C. The hybridization conditions for DNA:DNA hybrids are preferably for example 0.1 x SSC and 20°C, 25°C, 30°C, 35°C, 40°C or 45°C, preferably between 30°C and 45°C. The hybridization conditions for DNA:RNA hybrids are preferably for example 0.1 x SSC and 30°C, 35°C, 40°C, 45°C, 50°C or 55°C, preferably between 45°C and 55°C. The abovementioned hybridization temperatures are determined for example for a nucleic acid approximately 100 bp (= base pairs) in length and a G + C content of 50% in the absence of formamide. The skilled worker knows to determine the hybridization conditions required with the aid of textbooks, for example the ones mentioned above, or from the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford.

A further example of one such stringent hybridization condition is hybridization at 4XSSC at 65°C, followed by a washing in 0.1XSSC at 65°C for one hour. Alternatively, an exemplary stringent hybridization condition is in 50 % formamide, 4XSSC at 42°C. Further, the conditions during the wash step can be selected from the range of conditions delimited by low-stringency conditions (approximately 2X SSC at 50°C) and high-stringency conditions (approximately 0.2X SSC at 50°C, preferably at 65°C) (20X SSC: 0.3M sodium citrate, 3M NaCl, pH 7.0). In addition, the temperature during the wash step can be raised from low-stringency conditions at room temperature, approximately 22°C, to higher-stringency conditions at approximately 65°C. Both of the parameters salt concentration and temperature can be varied simultaneously, or else one of the two parameters can be kept constant while only the other is varied. Denaturants, for example formamide or SDS, may also be employed during the hybridization. In the presence of 50% formamide, hybridization is preferably effected at 42°C. Relevant factors like i) length of treatment, ii) salt conditions, iii) detergent conditions, iv) competitor DNAs, v) temperature and vi) probe selection can be combined case by case so that not all possibilities can be mentioned herein.

Thus, in a preferred embodiment, Northern blots are prehybridized with Rothi-Hybri-Quick buffer (Roth, Karlsruhe) at 68°C for 2h. Hybridzation with radioactive labelled probe is done overnight at 68°C. Subsequent washing steps are performed at 68°C with 1xSSC.

For Southern blot assays the membrane is prehybridized with Rothi-Hybri-Quick buffer (Roth, Karlsruhe) at 68°C for 2h. The hybridzation with radioactive labelled probe is conducted over night at 68°C. Subsequently the hybridization buffer is discarded and the filter shortly washed using 2xSSC; 0,1 % SDS. After discarding the washing buffer new 2xSSC; 0,1 % SDS buffer is added and incubated at 68°C for 15 minutes. This washing step is performed twice followed by an additional washing step using 1xSSC; 0,1% SDS at 68°C for 10 min.

Some examples of conditions for DNA hybridization (Southern blot assays) and wash step are shown hereinbelow:
(1) Hybridization conditions can be selected, for example, from the following conditions:
   a) 4X SSC at 65°C,
   b) 6X SSC at 45°C,
   c) 6X SSC, 100 mg/ml denatured fragmented fish sperm DNA at 68°C,
   d) 6X SSC, 0.5% SDS, 100 mg/ml denatured salmon sperm DNA at 68°C,
   e) 6X SSC, 0.5% SDS, 100 mg/ml denatured fragmented salmon sperm DNA, 50% formamide at 42°C,
   f) 50% formamide, 4X SSC at 42°C,
   g) 50% (vol/vol) formamide, 0.1% bovine serum albumin, 0.1% Ficoll, 0.1% polyvinylpyrrolidone, 50 mM sodium phosphate buffer pH 6.5, 750 mM NaCl, 75 mM sodium citrate at 42°C,
   h) 2X or 4X SSC at 50°C (low-stringency condition), or
   i) 30 to 40% formamide, 2X or 4X SSC at 42°C (low-stringency condition).
(2) Wash steps can be selected, for example, from the following conditions:
   a) 0.015 M NaCl/0.0015 M sodium citrate/0.1% SDS at 50°C.
   b) 0.1X SSC at 65°C.
   c) 0.1XSSC, 0.5 % SDS at 68°C.
   d) 0.1X SSC, 0.5% SDS, 50% formamide at 42°C.
   e) 0.2X SSC, 0.1 % SDS at 42°C.
   f) 2X SSC at 65°C (low-stringency condition).

Polypeptides having above-mentioned activity, i.e. conferring the increased tolerance and/or resistance to environmental stress and increased biomass production as compared to a corresponding non-transformed wild type plant cell, plant or part thereof, derived from other organisms, can be encoded by other DNA sequences which hybridize to the sequences shown in table I, columns 5 and 7 under relaxed hybridization conditions and which code on expression for peptides conferring the increased tolerance and/or resistance to environmental stress and increased biomass production as compared to a corresponding non-transformed wild type plant cell, plant or part thereof.

Further, some applications have to be performed at low stringency hybridisation conditions, without any consequences for the specificity of the hybridisation. For example, a Southern blot analysis of total DNA could be probed with a nucleic acid molecule of the present invention and washed at low stringency (55°C in 2xSSPE, 0,1% SDS). The hybridisation analysis could reveal a simple pattern of only genes encoding polypeptides of the present invention or used in the process of the invention, e.g. having herein-mentioned activity of increasing the tolerance and/or resistance to environmental stress and the biomass production as compared to a corresponding non-transformed wild type plant cell, plant or part thereof. A further example of such low-stringent hybridization conditions is 4XSSC at 50°C or hybridization with 30 to 40% formamide at 42°C. Such molecules comprise those which are fragments, analogues or derivatives of the polypeptide of the invention or used in the process of the invention and differ, for example, by way of amino acid and/or nucleotide deletion(s), insertion(s), substitution (s), addition(s) and/or recombination (s) or any other modification(s) known in the art either alone or in combination from the above-described amino acid sequences or their underlying nucleotide sequence(s). However, it is preferred to use high stringency hybridisation conditions.

Hybridization should advantageously be carried out with fragments of at least 5, 10, 15, 20, 25, 30, 35 or 40 bp, advantageously at least 50, 60, 70 or 80 bp, preferably at least 90, 100 or 110 bp. Most preferably are fragments of at least 15, 20, 25 or 30 bp. Preferably are also hybridizations with at least 100 bp or 200, very especially preferably at least 400 bp in length. In an especially preferred embodiment, the hybridization should be carried out with the entire nucleic acid sequence with conditions described above.

The terms "fragment", "fragment of a sequence" or "part of a sequence" mean a truncated sequence of the original sequence referred to. The truncated sequence (nucleic acid or protein sequence) can vary widely in length; the minimum size being a sequence of sufficient size to provide a sequence with at least a comparable function and/or activity of the original sequence referred to or hybidizing with the nucleic acid molecule of the invention or used in the process of the invention under stringend conditions, while the maximum size is not critical. In some applications, the maximum size usually is not substantially greater than that required to provide the desired activity and/or function(s) of the original sequence.

Typically, the truncated amino acid sequence will range from about 5 to about 310 amino acids in length. More typically, however, the sequence will be a maximum of about 250 amino acids in length, preferably a maximum of about 200 or 100 amino acids. It is usually desirable to select sequences of at least about 10, 12 or 15 amino acids, up to a maximum of about 20 or 25 amino acids.

The term "epitope" relates to specific immunoreactive sites within an antigen, also known as antigenic determinates. These epitopes can be a linear array of monomers in a polymeric composition - such as amino acids in a protein - or consist of or comprise a more complex secondary or tertiary structure. Those of skill will recognize that immunogens (i.e., substances capable of eliciting an immune response) are antigens; however, some antigen, such as haptens, are not immunogens but may be made immunogenic by coupling to a carrier molecule. The term "antigen" includes references to a substance to which an antibody can be generated and/or to which the antibody is specifically immunoreactive.

In one embodiment the present invention relates to a epitope of the polypeptide of the present invention or used in the process of the present invention and confers an increased tolerance and/or resistance to environmental stress and increased biomass production as compared to a corresponding non-transformed wild type plant cell, plant or part thereof.

The term "one or several amino acids" relates to at least one amino acid but not more than that number of amino acids, which would result in a homology of below 50% identity. Preferably, the identity is more than 70% or 80%, more preferred are 85%, 90%, 91%, 92%, 93%, 94% or 95%, even more preferred are 96%, 97%, 98%, or 99% identity.

Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences shown in table I, columns 5 and 7 is one which is sufficiently complementary to one of the nucleotide sequences shown in table I, columns 5 and 7 such that it can hybridize to one of the nucleotide sequences shown in table I, columns 5 and 7, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence shown in table I, columns 5 and 7, or a portion thereof and preferably has above mentioned activity, in particular having a tolerance and/or resistance to environmental stress and biomass production increasing activity after increasing the acitivity or an activity of a gene product as shown in table II, column 3 by for example expression either in the cytsol or in an organelle such as a plastid or mitochondria or both, preferably in plastids.

The nucleic acid molecule of the invention comprises a nucleotide sequence which hybridizes, preferably hybridizes under stringent conditions as defined herein, to one of the nucleotide sequences shown in table I, columns 5 and 7, or a portion thereof and encodes a protein having above-mentioned activity, e.g. conferring an increased tolerance and/or resistance to environmental stress and increased biomass production as compared to a corresponding non-transformed wild type plant cell, plant or part thereof by for example expression either in the cytsol or in an organelle such as a plastid or mitochondria or both, preferably in plastids, and optionally, the activity selected from the group consisting of: 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Yal049c-protein, YCR059C-protein, YEL005C-protein, YER156C-protein, Yfr042w-protein, YGL045W-protein, and YOR024w-protein..

Moreover, the nucleic acid molecule of the invention can comprise only a portion of the coding region of one of the sequences shown in table I, columns 5 and 7, for example a fragment which can be used as a probe or primer or a fragment encoding a biologically active portion of the polypeptide of the present invention or of a polypeptide used in the process of the present invention, i.e. having above-mentioned activity, e.g.conferring an increase of the tolerance and/or resistance to environmental stress and biomass production as compared to a corresponding non-transformed wild type plant cell, plant or part thereof if its activity is increased by for example expression either in the cytsol or in an organelle such as a plastid or mitochondria or both, preferably in plastids. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., in table I, columns 5 and 7, an anti-sense sequence of one of the sequences, e.g., set forth in table I, columns 5 and 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primers shown in table III, column 7 will result in a fragment of the gene product as shown in table II, column 3.

Primer sets are interchangable. The person skilled in the art knows to combine said primers to result in the desired product, e.g. in a full length clone or a partial sequence. Probes based on the sequences of the nucleic acid molecule of the invention or used in the process of the present invention can be used to detect transcripts or genomic sequences encoding the same or homologous proteins. The probe can further comprise a label group attached thereto, e.g. the label group can be a radioisotope, a fluorescent compound, an enzyme, or an enzyme co-factor. Such probes can be used as a part of a genomic marker test kit for identifying cells which express an polypepetide of the invention or used in the process of the present invention, such as by measuring a level of an encoding nucleic acid molecule in a sample of cells, e.g., detecting mRNA levels or determining, whether a genomic gene comprising the sequence of the polynucleotide of the invention or used in the processs of the present invention has been mutated or deleted.

The nucleic acid molecule of the invention encodes a polypeptide or portion thereof which includes an amino acid sequence which is sufficiently homologous to the amino acid sequence shown in table II, columns 5 and 7 such that the protein or portion thereof maintains the ability to participate in the increase of tolerance and/or resistance to environmental stress and increase of biomass production as compared to a corresponding non-transformed wild type plant cell, plant or part thereof, in particular increasing the activity as mentioned above or as described in the examples in plants is comprised.

As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence shown in table II, columns 5 and 7 such that the protein or portion thereof is able to participate in the increase of the increase of tolerance and/or resistance to environmental stress and increase of biomass production as compared to a corresponding non-transformed wild type plant cell, plant or part thereof. For examples having the activity of a protein as shown in table II, column 3 and as described herein.

In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence of table II, columns 5 and 7 and having above-mentioned activity, e.g. conferring an increased tolerance and/or resistance to environmental stress and increased biomass production as compared to a corresponding non-transformed wild type plant cell, plant or part thereof by for example expression either in the cytsol or in an organelle such as a plastid or mitochondria or both, preferably in plastids.

Portions of proteins encoded by the nucleic acid molecule of the invention are preferably biologically active, preferably having above-mentioned annotated activity, e.g. conferring an increase in tolerance and/or resistance to environmental stress and increase in biomass production as compared to a corresponding non-transformed wild type plant cell, plant or part thereof after increase of activity.

As mentioned herein, the term "biologically active portion" is intended to include a portion, e.g., a domain/motif, that confers increase in tolerance and/or resistance to environmental stress and increase in biomass production as compared to a corresponding non-transformed wild type plant cell, plant or part thereof or has an immunological activity such that it is binds to an antibody binding specifially to the polypeptide of the present invention or a polypeptide used in the process of the present invention for increased tolerance and/or resistance to environmental stress and increased biomass production as compared to a corresponding non-transformed wild type plant cell, plant or part thereof.

The invention further relates to nucleic acid molecules that differ from one of the nucleotide sequences shown in table I A, columns 5 and 7 (and portions thereof) due to degeneracy of the genetic code and thus encode a polypeptide of the present invention, in particular a polypeptide having above mentioned activity, e.g. as that polypeptides depicted by the sequence shown in table II, columns 5 and 7 or the functional homologues. Advantageously, the nucleic acid molecule of the invention comprises, or in an other embodiment has, a nucleotide sequence encoding a protein comprising, or in an other embodiment having, an amino acid sequence shown in table II, columns 5 and 7 or the functional homologues. In a still further embodiment, the nucleic acid molecule of the invention encodes a full length protein which is substantially homologous to an amino acid sequence shown in table II, columns 5 and 7 or the functional homologues. However, in a preferred embodiment, the nucleic acid molecule of the present invention does not consist of the sequence shown in table I, preferably table IA, columns 5 and 7.

In addition, it will be appreciated by those skilled in the art that DNA sequence polymorphisms that lead to changes in the amino acid sequences may exist within a population. Such genetic polymorphism in the gene encoding the polypeptide of the invention or comprising the nucleic acid molecule of the invention may exist among individuals within a population due to natural variation.

As used herein, the terms "gene" and "recombinant gene" refer to nucleic acid molecules comprising an open reading frame encoding the polypeptide of the invention or comprising the nucleic acid molecule of the invention or encoding the polypeptide used in the process of the present invention, preferably from a crop plant or from a microorgansim useful for the method of the invention. Such natural variations can typically result in 1-5% variance in the nucleotide sequence of the gene. Any and all such nucleotide variations and resulting amino acid polymorphisms in genes encoding a polypeptide of the invention or comprising a the nucleic acid molecule of the invention that are the result of natural variation and that do not alter the functional activity as described are intended to be within the scope of the invention.

Nucleic acid molecules corresponding to natural variants homologues of a nucleic acid molecule of the invention, which can also be a cDNA, can be isolated based on their homology to the nucleic acid molecules disclosed herein using the nucleic acid molecule of the invention, or a portion thereof, as a hybridization probe according to standard hybridization techniques under stringent hybridization conditions.

Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising the sequence shown in table I, columns 5 and 7. The nucleic acid molecule is preferably at least 20, 30, 50, 100, 250 or more nucleotides in length.

The term "hybridizes under stringent conditions" is defined above. In one embodiment, the term "hybridizes under stringent conditions" is intended to describe conditions for hybridization and washing under which nucleotide sequences at least 30 %, 40 %, 50 % or 65% identical to each other typically remain hybridized to each other. Preferably, the conditions are such that sequences at least about 70%, more preferably at least about 75% or 80%, and even more preferably at least about 85%, 90% or 95% or more identical to each other typically remain hybridized to each other.

Preferably, nucleic acid molecule of the invention that hybridizes under stringent conditions to a sequence shown in table I, columns 5 and 7 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the tolerance and/or resistance to environmental stress and biomass production increase after increasing the expression or activity thereof or the activity of a protein of the invention or used in the process of the invention by for example expression the nucleic acid sequence of the gene product in the cytsol and/or in an organelle such as a plastid or mitochondria, preferably in plastids.

In addition to naturally-occurring variants of the sequences of the polypeptide or nucleic acid molecule of the invention as well as of the polypeptide or nucleic acid molecule used in the process of the invention that may exist in the population, the skilled artisan will further appreciate that changes can be introduced by mutation into a nucleotide sequence of the nucleic acid molecule encoding the polypeptide of the invention or used in the process of the present invention, thereby leading to changes in the amino acid sequence of the encoded said polypeptide, without altering the functional ability of the polypeptide, preferably not decreasing said activity.

For example, nucleotide substitutions leading to amino acid substitutions at "non-essential" amino acid residues can be made in a sequence of the nucleic acid molecule of the invention or used in the process of the invention, e.g. shown in table I, columns 5 and 7.

A"non-essential" amino acid residue is a residue that can be altered from the wild-type sequence of one without altering the activity of said polypeptide, whereas an "essential" amino acid residue is required for an activity as mentioned above, e.g. leading to an increase in the tolerance and/or resistance to environmental stress and biomass production as compared to a corresponding non-transformed wild type plant cell, plant or part thereof in an organism after an increase of activity of the polypeptide. Other amino acid residues, however, (e.g., those that are not conserved or only semi-conserved in the domain having said activity) may not be essential for activity and thus are likely to be amenable to alteration without altering said activity.

Further, a person skilled in the art knows that the codon usage between organisms can differ. Therefore, he may adapt the codon usage in the nucleic acid molecule of the present invention to the usage of the organism or the cell compartment for example of the plastid or mitochondria in which the polynuclestide or polypeptide is expressed.

Accordingly, the invention relates to nucleic acid molecules encoding a polypeptide having above-mentioned activity, in an organisms or parts thereof by for example expression either in the cytsol or in an organelle such as a plastid or mitochondria or both, preferably in plastids that contain changes in amino acid residues that are not essential for said activity. Such polypeptides differ in amino acid sequence from a sequence contained in the sequences shown in table II, columns 5 and 7 yet retain said activity described herein. The nucleic acid molecule can comprise a nucleotide sequence encoding a polypeptide, wherein the polypeptide comprises an amino acid sequence at least about 50% identical to an amino acid sequence shown in table II, columns 5 and 7 and is capable of participation in the increase of tolerance and/or resistance to environmental stress and increased biomass production as compared to a corresponding non-transformed wild type plant cell, plant or part thereof after increasing its activity, e.g. its expression by for example expression either in the cytsol or in an organelle such as a plastid or mitochondria or both, preferably in plastids. Preferably, the protein encoded by the nucleic acid molecule is at least about 60% identical to the sequence shown in table II, columns 5 and 7, more preferably at least about 70% identical to one of the sequences shown in table II, columns 5 and 7, even more preferably at least about 80%, 90%, 95% homologous to the sequence shown in table II, columns 5 and 7, and most preferably at least about 96%, 97%, 98%, or 99% identical to the sequence shown in table II, columns 5 and 7.

To determine the percentage homology (= identity, herein used interchangeably) of two amino acid sequences or of two nucleic acid molecules, the sequences are written one underneath the other for an optimal comparison (for example gaps may be inserted into the sequence of a protein or of a nucleic acid in order to generate an optimal alignment with the other protein or the other nucleic acid).

The amino acid residues or nucleic acid molecules at the corresponding amino acid positions or nucleotide positions are then compared. If a position in one sequence is occupied by the same amino acid residue or the same nucleic acid molecule as the corresponding position in the other sequence, the molecules are homologous at this position (i.e. amino acid or nucleic acid "homology" as used in the present context corresponds to amino acid or nucleic acid "identity". The percentage homology between the two sequences is a function of the number of identical positions shared by the sequences (i.e. % homology = number of identical positions/total number of positions x 100). The terms "homology" and "identity" are thus to be considered as synonyms.

For the determination of the percentage homology (=identity) of two or more amino acids or of two or more nucleotide sequences several computer software programs have been developed. The homology of two or more sequences can be calculated with for example the software fasta, which presently has been used in the version fasta 3 (W. R. Pearson and D. J. Lipman (1988), Improved Tools for Biological Sequence Comparison.PNAS 85:2444- 2448; W. R. Pearson (1990) Rapid and Sensitive Sequence Comparison with FASTP and FASTA, Methods in Enzymology 183:63 - 98; W. R. Pearson and D. J. Lipman (1988) Improved Tools for Biological Sequence Comparison.PNAS 85:2444- 2448; W. R. Pearson (1990); Rapid and Sensitive Sequence Comparison with FASTP and FASTAMethods in Enzymology 183:63 - 98). Another useful program for the calculation of homologies of different sequences is the standard blast program, which is included in the Biomax pedant software (Biomax, Munich, Federal Republic of Germany). This leads unfortunately sometimes to suboptimal results since blast does not always include complete sequences of the subject and the querry. Nevertheless as this program is very efficient it can be used for the comparison of a huge number of sequences. The following settings are typically used for such a comparisons of sequences:
-p Program Name [String]; -d Database [String]; default = nr; -i Query File [File In]; default = stdin; -e Expectation value (E) [Real]; default = 10.0; -m alignment view options: 0 = pairwise; 1 = query-anchored showing identities; 2 = query-anchored no identities; 3 = flat query-anchored, show identities; 4 = flat query-anchored, no identities; 5 = query-anchored no identities and blunt ends; 6 = flat query-anchored, no identities and blunt ends; 7 = XML Blast output; 8 = tabular; 9 tabular with comment lines [Integer]; default = 0; -o BLAST report Output File [File Out] Optional; default = stdout; -F Filter query sequence (DUST with blastn, SEG with others) [String]; default = T; -G Cost to open a gap (zero invokes default behavior) [Integer]; default = 0; -E Cost to extend a gap (zero invokes default behavior) [Integer]; default = 0; -X X dropoff value for gapped alignment (in bits) (zero invokes default behavior); blastn 30, megablast 20, tblastx 0, all others 15 [Integer]; default = 0; -I Show Gl's in deflines [T/F]; default = F; - q Penalty for a nucleotide mismatch (blastn only) [Integer]; default = -3; -r Reward for a nucleotide match (blastn only) [Integer]; default = 1; -v Number of database sequences to show one-line descriptions for (V) [Integer]; default = 500; -b Number of database sequence to show alignments for (B) [Integer]; default = 250; -f Threshold for extending hits, default if zero; blastp 11, blastn 0, blastx 12, tblastn 13; tblastx 13, megablast 0 [Integer]; default = 0; -g Perfom gapped alignment (not available with tblastx) [T/F]; default = T; -Q Query Genetic code to use [Integer]; default = 1; -D DB Genetic code (for tblast[nx] only) [Integer]; default = 1; -a Number of processors to use [Integer]; default = 1; -O SeqAlign file [File Out] Optional; -J Believe the query defline [T/F]; default = F; -M Matrix [String]; default = BLOSUM62; -W Word size, default if zero (blastn 11, megablast 28, all others 3) [Integer]; default = 0; -z Effective length of the database (use zero for the real size) [Real]; default = 0; -K Number of best hits from a region to keep (off by default, if used a value of 100 is recommended) [Integer]; default = 0; -P 0 for multiple hit, 1 for single hit [Integer]; default = 0; -Y Effective length of the search space (use zero for the real size) [Real]; default = 0; -S Query strands to search against database (for blast[nx], and tblastx); 3 is both, 1 is top, 2 is bottom [Integer]; default = 3; -T Produce HTML output [T/F]; default = F; -I Restrict search of database to list of Gl's [String] Optional; -U Use lower case filtering of FASTA sequence [T/F] Optional; default = F; -y X dropoff value for ungapped extensions in bits (0.0 invokes default behavior); blastn 20, megablast 10, all others 7 [Real]; default = 0.0; -Z X dropoff value for final gapped alignment in bits (0.0 invokes default behavior); blastn/megablast 50, tblastx 0, all others 25 [Integer]; default = 0; -R PSI-TBLASTN checkpoint file [File In] Optional; -n MegaBlast search [T/F]; default = F; -L Location on query sequence [String] Optional; -A Multiple Hits window size, default if zero (blastn/megablast 0, all others 40 [Integer]; default = 0; -w Frame shift penalty (OOF algorithm for blastx) [Integer]; default = 0; -t Length of the largest intron allowed in tblastn for linking HSPs (0 disables linking) [Integer]; default = 0.

Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351 (1987), Higgins et al., CABIOS 5, 151 (1989)) or preferably with the programs "Gap" and "Needle", which are both based on the algorithms of Needleman and Wunsch (J. Mol. Biol. 48; 443 (1970)), and "BestFit", which is based on the algorithm of Smith and Waterman (Adv. Appl. Math. 2; 482 (1981)). "Gap" and "BestFit" are part of the GCG software-package (Genetics Computer Group, 575 Science Drive, Madison, Wisconsin, USA 53711 (1991); Altschul et al., (Nucleic Acids Res. 25, 3389 (1997)), "Needle" is part of the The European Molecular Biology Open Software Suite (EMBOSS) (Trends in Genetics 16 (6), 276 (2000)). Therefore preferably the calculations to determine the percentages of sequence homology are done with the programs "Gap" or "Needle" over the whole range of the sequences. The following standard adjustments for the comparison of nucleic acid sequences were used for "Needle": matrix: EDNAFULL, Gap_penalty: 10.0, Extend_penalty: 0.5. The following standard adjustments for the comparison of nucleic acid sequences were used for "Gap": gap weight: 50, length weight: 3, average match: 10.000, average mismatch: 0.000.

For example a sequence, which has 80% homology with sequence SEQ ID NO: 38 at the nucleic acid level is understood as meaning a sequence which, upon comparison with the sequence SEQ ID NO: 38 by the above program "Needle" with the above parameter set, has a 80% identity.

Homology between two polypeptides is understood as meaning the identity of the amino acid sequence over in each case the entire sequence length which is calculated by comparison with the aid of the above program "Needle" using Matrix: EBLOSUM62, Gap_penalty: 8.0, Extend_penalty: 2.0.

For example a sequence which has a 80% homology with sequence SEQ ID NO: 39 at the protein level is understood as meaning a sequence which, upon comparison with the sequence SEQ ID NO: 39 by the above program "Needle" with the above parameter set, has a 80% identity.

Functional equivalents derived from one of the polypeptides as shown in table II, columns 5 and 7 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in table II, columns 5 and 7 according to the invention and are distinguished by essentially the same properties as the polypeptide as shown in table II, columns 5 and 7.

Functional equivalents derived from the nucleic acid sequence as shown in table I, columns 5 and 7 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in table II, columns 5 and 7 according to the invention and encode polypeptides having essentially the same properties as the polypeptide as shown in table II, columns 5 and 7.

"Essentially the same properties" of a functional equivalent is above all understood as meaning that the functional equivalen has above mentioned acitivty, by for example expression either in the cytsol or in an organelle such as a plastid or mitochondria or both, preferably in plastids while increasing the amount of protein, activity or function of said functional equivalent in an organism, e.g. a microorgansim, a plant or plant or animal tissue, plant or animal cells or a part of the same.

A nucleic acid molecule encoding an homologous to a protein sequence of table II, columns 5 and 7 can be created by introducing one or more nucleotide substitutions, additions or deletions into a nucleotide sequence of the nucleic acid molecule of the present invention, in particular of table I, columns 5 and 7 such that one or more amino acid substitutions, additions or deletions are introduced into the encoded protein. Mutations can be introduced into the encoding sequences of table I, columns 5 and 7 by standard techniques, such as site-directed mutagenesis and PCR-mediated mutagenesis.

Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophane), beta-branched side chains (e.g., threonine, valine, isoleucine) and aromatic side chains (e.g., tyrosine, phenylalanine, tryptophane, histidine).

Thus, a predicted nonessential amino acid residue in a polypeptide of the invention or a polypeptide used in the process of the invention is preferably replaced with another amino acid residue from the same family. Alternatively, in another embodiment, mutations can be introduced randomly along all or part of a coding sequence of a nucleic acid molecule of the invention or used in the process of the invention, such as by saturation mutagenesis, and the resultant mutants can be screened for activity described herein to identify mutants that retain or even have increased above mentioned activity, e.g. conferring an increased tolerance and/or resistance to environmental stress and increased biomass production as compared to a corresponding non-transformed wild type plant cell, plant or part thereof.

Following mutagenesis of one of the sequences of shown herein, the encoded protein can be expressed recombinantly and the activity of the protein can be determined using, for example, assays described herein (see Examples).

The highest homology of the nucleic acid molecule used in the process according to the invention was found for the following database entries by Gap search.

Homologues of the nucleic acid sequences used, with the sequence shown in table I, columns 5 and 7, comprise also allelic variants with at least approximately 30%, 35%, 40% or 45% homology, by preference at least approximately 50%, 60% or 70%, more preferably at least approximately 90%, 91 %, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from table I, columns 5 and 7, or from the derived nucleic acid sequences, the intention being, however, that the enzyme activity or the biological activity of the resulting proteins synthesized is advantageously retained or increased.

In one embodiment of the present invention, the nucleic acid molecule of the invention or used in the process of the invention comprises the sequences shown in any of the table I, columns 5 and 7. It is preferred that the nucleic acid molecule comprises as little as possible other nucleotides not shown in any one of table I, columns 5 and 7. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, the nucleic acid molecule use in the process of the invention is identical to the sequences shown in table I, columns 5 and 7.

Also preferred is that the nucleic acid molecule used in the process of the invention encodes a polypeptide comprising the sequence shown in table II, columns 5 and 7. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment used in the inventive process, the encoded polypeptide is identical to the sequences shown in table II, columns 5 and 7.

In one embodiment, the nucleic acid molecule of the invention or used in the process encodes a polypeptide comprising the sequence shown in table II, columns 5 and 7 comprises less than 100 further nucleotides. In a further embodiment, said nucleic acid molecule comprises less than 30 further nucleotides. In one embodiment, the nucleic acid molecule used in the process is identical to a coding sequence of the sequences shown in table I, columns 5 and 7.

Polypeptides (= proteins), which still have the essential biological or enzymatic activity of the polypeptide of the present invention conferring an increase tolerance and/or resistance to environmental stress and increased biomass production as compared to a corresponding non-transformed wild type plant cell, plant or part thereof i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide shown in table II, columns 5 and 7 expressed under identical conditions.

Homologues of table I, columns 5 and 7 or of the derived sequences of table II, columns 5 and 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

In addition to the nucleic acid molecules encoding the SRPs described above, another aspect of the invention pertains to negative regulators of the activity of a nucleic acid molecules selected from the group according to table I, column 5 and/or 7, preferably column 7. Antisense polynucleotides thereto are thought to inhibit the downregulating activity of those negative regulatorsby specifically binding the target polynucleotide and interfering with transcription, splicing, transport, translation, and/or stability of the target polynucleotide. Methods are described in the prior art for targeting the antisense polynucleotide to the chromosomal DNA, to a primary RNA transcript, or to a processed mRNA. Preferably, the target regions include splice sites, translation initiation codons, translation termination codons, and other sequences within the open reading frame.

The term "antisense," for the purposes of the invention, refers to a nucleic acid comprising a polynucleotide that is sufficiently complementary to all or a portion of a gene, primary transcript, or processed mRNA, so as to interfere with expression of the endogenous gene. "Complementary" polynucleotides are those that are capable of base pairing according to the standard Watson-Crick complementarity rules. bpecifically, purines will base pair with pyrimidines to form a combination of guanine paired with cytosine (G:C) and adenine paired with either thymine (A:T) in the case of DNA, or adenine paired with uracil (A:U) in the case of RNA. It is understood that two polynucleotides may hybridize to each other even if they are not completely complementary to each other, provided that each has at least one region that is substantially complementary to the other. The term "antisense nucleic acid" includes single stranded RNA as well as double-stranded DNA expression cassettes that can be transcribed to produce an antisense RNA. "Active" antisense nucleic acids are antisense RNA molecules that are capable of selectively hybridizing with a negative regulator of the activity of a nucleic acid molecules encoding a polypeptide having at least 80% sequence identity with the polypeptide selected from the group according to table II, column 5 and/or 7, preferably column 7..

The antisense nucleic acid can be complementary to an entire negative regulator strand, or to only a portion thereof. In an embodiment, the antisense nucleic acid molecule is antisense to a "noncoding region" of the coding strand of a nucleotide sequence encoding a SRP. The term "noncoding region" refers to 5' and 3' sequences that flank the coding region that are not translated into amino acids (i.e., also referred to as 5' and 3' untranslated regions). The antisense nucleic acid molecule can be complementary to only a portion of the noncoding region of SRP mRNA. For example, the antisense oligonucleotide can be complementary to the region surrounding the translation start site of SRP mRNA. An antisense oligonucleotide can be, for example, about 5, 10, 15, 20, 25, 30, 35, 40, 45 or 50 nucleotides in length. Typically, the antisense molecules of the present invention comprise an RNA having 60-100% sequence identity with at least 14 consecutive nucleotides of a noncoding region of one of the nucleic acid of table I. Preferably, the sequence identity will be at least 70%, more preferably at least 75%, 80%, 85%, 90%, 95%, 98% and most preferably 99%.

An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

In yet another embodiment, the antisense nucleic acid molecule of the invention is an alpha-anomeric nucleic acid molecule. An alpha-anomeric nucleic acid molecule forms specific double-stranded hybrids with complementary RNA in which, contrary to the usual b-units, the strands run parallel to each other (Gaultier et al., 1987, Nucleic Acids. Res. 15:6625-6641). The antisense nucleic acid molecule can also comprise a 2'-o-methylribonucleotide (Inoue et al., 1987, Nucleic Acids Res. 15:6131-6148) or a chimeric RNA-DNA analogue (Inoue et al., 1987, FEBS Lett. 215:327-330).

The antisense nucleic acid molecules of the invention are typically administered to a cell or generated *in situ* such that they hybridize with or bind to cellular mRNA and/or genomic DNA. The hybridization can be by conventional nucleotide complementarity to form a stable duplex, or, for example, in the case of an antisense nucleic acid molecule which binds to DNA duplexes, through specific interactions in the major groove of the double helix. The antisense molecule can be modified such that it specifically binds to a receptor or an antigen expressed on a selected cell surface, e.g., by linking the antisense nucleic acid molecule to a peptide or an antibody which binds to a cell surface receptor or antigen. The antisense nucleic acid molecule can also be delivered to cells using the vectors described herein. To achieve sufficient intracellular concentrations of the antisense molecules, vector constructs in which the antisense nucleic acid molecule is placed under the control of a strong prokaryotic, viral, or eukaryotic (including plant) promoter are preferred.

As an alternative to antisense polynucleotides, ribozymes, sense polynucleotides, or double stranded RNA (dsRNA) can be used to reduce expression of a SRP polypeptide. By "ribozyme" is meant a catalytic RNA-based enzyme with ribonuclease activity which is capable of cleaving a single-stranded nucleic acid, such as an mRNA, to which it has a complementary region. Ribozymes (e.g., hammerhead ribozymes described in Haselhoff and Gerlach, 1988, Nature 334:585-591) can be used to catalytically cleave SRP mRNA transcripts to thereby inhibit translation of SRP mRNA. A ribozyme having specificity for a SRP-encoding nucleic acid can be designed based upon the nucleotide sequence of a SRP cDNA, as disclosed herein or on the basis of a heterologous sequence to be isolated according to methods taught in this invention. For example, a derivative of a Tetrahymena L-19 IVS RNA can be constructed in which the nucleotide sequence of the active site is complementary to the nucleotide sequence to be cleaved in a SRP-encoding mRNA. See, e.g., U.S. Patent Nos. 4,987,071 and 5,116,742 to Cech et al. Alternatively, SRP mRNA can be used to select a catalytic RNA having a specific ribonuclease activity from a pool of RNA molecules. See, e.g., Bartel, D. and Szostak, J.W., 1993, Science 261:1411-1418. In preferred embodiments, the ribozyme will contain a portion having at least 7, 8, 9, 10, 12, 14, 16, 18 or 20 nucleotides, and more preferably 7 or 8 nucleotides, that have 100% complementarity to a portion of the target RNA. Methods for making ribozymes are known to those skilled in the art. See, e.g., U.S. Patent Nos. 6,025,167; 5,773,260; and 5,496,698.

The term "dsRNA," as used herein, refers to RNA hybrids comprising two strands of RNA. The dsRNAs can be linear or circular in structure. In a preferred embodiment, dsRNA is specific for a polynucleotide encoding either the polypeptide according to table II or a polypeptide having at least 70% sequence identity with a polypeptide according to table II. The hybridizing RNAs may be substantially or completely complementary. By "substantially complementary," is meant that when the two hybridizing RNAs are optimally aligned using the BLAST program as described above, the hybridizing portions are at least 95% complementary. Preferably, the dsRNA will be at least 100 base pairs in length. Typically, the hybridizing RNAs will be of identical length with no over hanging 5' or 3' ends and no gaps. However, dsRNAs having 5' or 3' overhangs of up to 100 nucleotides may be used in the methods of the invention.

The dsRNA may comprise ribonucleotides or ribonucleotide analogs, such as 2'-O-methyl ribosyl residues, or combinations thereof. See, e.g., U.S. Patent Nos. 4,130,641 and 4,024,222. A dsRNA polyriboinosinic acid:polyribocytidylic acid is described in U.S. patent 4,283,393. Methods for making and using dsRNA are known in the art. One method comprises the simultaneous transcription of two complementary DNA strands, either *in vivo,* or in a single *in vitro* reaction mixture. See, e.g., U.S. Patent No. 5,795,715. In one embodiment, dsRNA can be introduced into a plant or plant cell directly by standard transformation procedures. Alternatively, dsRNA can be expressed in a plant cell by transcribing two complementary RNAs.

Other methods for the inhibition of endogenous gene expression, such as triple helix formation (Moser et al., 1987, Science 238:645-650 and Cooney et al., 1988, Science 241:456-459) and cosuppression (Napoli et al., 1990, The Plant Cell 2:279-289) are known in the art. Partial and full-length cDNAs have been used for the cosuppression of endogenous plant genes. See, e.g., U.S. Patent Nos. 4,801,340, 5,034,323, 5,231,020, and 5,283,184; Van der Kroll et al., 1990, The Plant Cell 2:291-299; Smith et al., 1990, Mol. Gen. Genetics 224:477-481 and Napoli et al., 1990, The Plant Cell 2:279-289.

For sense suppression, it is believed that introduction of a sense polynucleotide blocks transcription of the corresponding target gene. The sense polynucleotide will have at least 65% sequence identity with the target plant gene or RNA. Preferably, the percent identity is at least 80%, 90%, 95% or more. The introduced sense polynucleotide need not be full length relative to the target gene or transcript. Preferably, the sense polynucleotide will have at least 65% sequence identity with at least 100 consecutive nucleotides of one of the nucleic acids as depicted in Table I. The regions of identity can comprise introns and and/or exons and untranslated regions. The introduced sense polynucleotide may be present in the plant cell transiently, or may be stably integrated into a plant chromosome or extrachromosomal replicon.

Further, object of the invention is an expression vector comprising a nucleic acid molecule comprising a nucleic acid molecule selected from the group consisting of:
a) a nucleic acid molecule encoding the polypeptide shown in column 5 or 7 of Table II;
b) a nucleic acid molecule shown in column 5 or 7 of Table I;
c) a nucleic acid molecule, which, as a result of the degeneracy of the genetic code, can be derived from a polypeptide sequence depicted in column 5 or 7 of Table II and confers an increased tolerance and/or resistance to environmental stress and increased biomass production as compared to a corresponding non-transformed wild type plant cell, a plant or a part thereof;
d) a nucleic acid molecule having at least 30 % identity with the nucleic acid molecule sequence of a polynucleotide comprising the nucleic acid molecule shown in column 5 or 7 of Table I and confers an increased tolerance and/or resistance to environmental stress and increased biomass production as compared to a corresponding non-transformed wild type plant cell, a plant or a part thereof;
e) a nucleic acid molecule encoding a polypeptide having at least 30 % identity with the amino acid sequence of the polypeptide encoded by the nucleic acid molecule of (a) to (c) and having the activity represented by a nucleic acid molecule comprising a polynucleotide as depicted in column 5 of Table I and confers an increased tolerance and/or resistance to environmental stress and increased biomass production as compared to a corresponding non-transformed wild type plant cell, a plant or a part thereof;
f) nucleic acid molecule which hybridizes with a nucleic acid molecule of (a) to (c) under stringent hybridization conditions and confers an increased tolerance and/or resistance to environmental stress and increased biomass production as compared to a corresponding non-transformed wild type plant cell, a plant or a part thereof;
g) a nucleic acid molecule encoding a polypeptide which can be isolated with the aid of monoclonal or polyclonal antibodies made against a polypeptide encoded by one of the nucleic acid molecules of (a) to (e) and having the activity represented by the nucleic acid molecule comprising a polynucleotide as depicted in column 5 of Table I;
h) a nucleic acid molecule encoding a polypeptide comprising the consensus sequence or one or more polypeptide motifs as shown in column 7 of Table IV and preferably having the activity represented by a nucleic acid molecule comprising a polynucleotide as depicted in column 5 of Table II or IV;
h) a nucleic acid molecule encoding a polypeptide having the activity represented by a protein as depicted in column 5 of Table II and confers an increased tolerance and/or resistance to environmental stress and increased biomass production as compared to a corresponding non-transformed wild type plant cell, a plant or a part thereof;
i) nucleic acid molecule which comprises a polynucleotide, which is obtained by amplifying a cDNA library or a genomic library using the primers in column 7 of Table III which do not start at their 5'-end with the nucleotides ATA and preferably having the activity represented by a nucleic acid molecule comprising a polynucleotide as depicted in column 5 of Table II or IV;
   and
j) a nucleic acid molecule which is obtainable by screening a suitable nucleic acid library under stringent hybridization conditions with a probe comprising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence characterized in (a) to (e) and encoding a polypeptide having the activity represented by a protein comprising a polypeptide as depicted in column 5 of Table II;

The invention further provides an isolated recombinant expression vector comprising a stress related protein encoding nucleic acid as described above, wherein expression of the vector or stress related protein encoding nucleic acid, respectively in a host cell results in increased tolerance and/or resistance to environmental stress as compared to the corresponding non-transformed wild type of the host cell. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Further types of vectors can be linearized nucleic acid sequences, such as transposons, which are pieces of DNA which can copy and insert themselves. There have been 2 types of transposons found: simple transposons, known as Insertion Sequences and composite transposons, which can have several genes as well as the genes that are required for transposition.

Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

A plant expression cassette preferably contains regulatory sequences capable of driving gene expression in plant cells and operably linked so that each sequence can fulfill its function, for example, termination of transcription by polyadenylation signals. Preferred polyadenylation signals are those originating from Agrobacterium tumefaciens T-DNA such as the gene 3 known as octopine synthase of the Tiplasmid pTiACH5 (Gielen et al., 1984 EMBO J. 3:835) or functional equivalents thereof but also all other terminators functionally active in plants are suitable.

As plant gene expression is very often not limited on transcriptional levels, a plant expression cassette preferably contains other operably linked sequences like translational enhancers such as the overdrive-sequence containing the 5'-untranslated leader sequence from tobacco mosaic virus enhancing the protein per RNA ratio (Gallie et al., 1987 Nucl. Acids Research 15:8693-8711).

Plant gene expression has to be operably linked to an appropriate promoter conferring gene expression in a timely, cell or tissue specific manner. Preferred are promoters driving constitutive expression (Benfey et al., 1989 EMBO J. 8:2195-2202) like those derived from plant viruses like the 35S CaMV (Franck et al., 1980 Cell 21:285-294), the 19S CaMV (see also U.S. Patent No. 5352605 and PCT Application No. WO 8402913) or plant promoters like those from Rubisco small subunit described in U.S. Patent No. 4,962,028.

Additional advantageous regulatory sequences are, for example, included in the plant promoters such as CaMV/35S [Franck et al., Cell 21 (1980) 285 - 294], PRP1 [Ward et al., Plant. Mol. Biol. 22 (1993)], SSU, OCS, lib4, usp, STLS1, B33, LEB4, nos or in the ubiquitin, napin or phaseolin promoter. Also advantageous in this connection are inducible promoters such as the promoters described in EP-A-0 388 186 (benzyl sulfonamide inducible), Plant J. 2, 1992: 397 - 404 (Gatz et al., Tetracyclin inducible), EP-A-0 335 528 (abscisic acid inducible) or WO 93/21334 (ethanol or cyclohexenol inducible). Additional useful plant promoters are the cytosolic FBPase promotor or ST-LSI promoter of the potato (Stockhaus et al., EMBO J. 8, 1989, 2445), the phosphorybosyl phyrophoshate amido transferase promoter of Glycine max (gene bank accession No. U87999) or the noden specific promoter described in EP-A-0 249 676. Additional particularly advantageous promoters are seed specific promoters which can be used for monokotyledones or dikotyledones and are described in US 5,608,152 (napin promoter from rapeseed), WO 98/45461 (phaseolin promoter from Arobidopsis), US 5,504,200 (phaseolin promoter from Phaseolus vulgaris), WO 91/13980 (Bce4 promoter from Brassica) and Baeumlein et al., Plant J., 2, 2, 1992: 233-239 (LEB4 promoter from leguminosa). Said promoters are useful in dikotyledones. The following promoters are useful for example in monokotyledones Ipt-2- or Ipt-1- promoter from barley (WO 95/15389 and WO 95/23230) or hordein promoter from barley. Other useful promoters are described in WO 99/16890.

It is possible in principle to use all natural promoters with their regulatory sequences like those mentioned above for the novel process. It is also possible and advantageous in addition to use synthetic promoters.

The gene construct may also comprise further genes which are to be inserted into the organisms and which are for example involved in stress resistance and biomass production increase. It is possible and advantageous to insert and express in host organisms regulatory genes such as genes for inducers, repressors or enzymes which intervene by their enzymatic activity in the regulation, or one or more or all genes of a biosynthetic pathway. These genes can be heterologous or homologous in origin. The inserted genes may have their own promoter or else be under the control of same promoter as the sequences of the nucleic acid of table I or their homologs.

The gene construct advantageously comprises, for expression of the other genes present, additionally 3' and/or 5' terminal regulatory sequences to enhance expression, which are selected for optimal expression depending on the selected host organism and gene or genes.

These regulatory sequences are intended to make specific expression of the genes and protein expression possible as mentioned above. This may mean, depending on the host organism, for example that the gene is expressed or overexpressed only after induction, or that it is immediately expressed and/or overexpressed.

The regulatory sequences or factors may moreover preferably have a beneficial effect on expression of the introduced genes, and thus increase it. It is possible in this way for the regulatory elements to be enhanced advantageously at the transcription level by using strong transcription signals such as promoters and/or enhancers. However, in addition, it is also possible to enhance translation by, for example, improving the stability of the mRNA.

Other preferred sequences for use in plant gene expression cassettes are targeting-sequences necessary to direct the gene product in its appropriate cell compartment (for review see Kermode, 1996 Crit. Rev. Plant Sci. 15(4):285-423 and references cited therein) such as the vacuole, the nucleus, all types of plastids like amyloplasts, chloroplasts, chromoplasts, the extracellular space, mitochondria, the endoplasmic reticulum, oil bodies, peroxisomes and other compartments of plant cells.

Plant gene expression can also be facilitated via an inducible promoter (for review see Gatz, 1997 Annu. Rev. Plant Physiol. Plant Mol. Biol. 48:89-108). Chemically inducible promoters are especially suitable if gene expression is wanted to occur in a time specific manner.

Table VI lists several examples of promoters that may be used to regulate transcription of the stress related protein nucleic acid coding sequences.

**Tab. VI: Examples of tissue-specific and stress-inducible promoters in plants**

| Expression | Reference |
|---|---|
| Cor78- Cold, drought, salt, ABA, wounding-inducible | Ishitani, et al., Plant Cell 9:1935-1949 (1997). |
| | Yamaguchi-Shinozaki and Shinozaki, Plant Cell 6:251-264 (1994). |
| Rci2A - Cold, dehydration-inducible | Capel et al., Plant Physiol 115:569-576 (1997) |
| Rd22 - Drought, salt | Yamaguchi-Shinozaki and Shinozaki, Mol Gen Genet 238:17-25 (1993). |
| Cor15A - Cold, dehydration, ABA | Baker et al., Plant Mol. Biol. 24:701-713 (1994). |
| GH3- Auxin inducible | Liu et al., Plant Cell 6:645-657 (1994) |
| ARSK1-Root, salt inducible | Hwang and Goodman, Plant J 8:37-43 (1995). |
| PtxA - Root, salt inducible | GenBank accession X67427 |
| SbHRGP3 - Root specific | Ahn et al., Plant Cell 8:1477-1490 (1998). |
| KST1 - Guard cell specific | Plesch et al., Plant Journal. 28(4):455-64, (2001) |
| KAT1 - Guard cell specific | Plesch et al., Gene 249:83-89 (2000) |
| | Nakamura et al., Plant Physiol. 109:371-374 (1995) |
| salicylic acid inducible | PCT Application No. WO 95/19443 |
| tetracycline inducible | Gatz et al. Plant J. 2:397-404 (1992) |
| Ethanol inducible | PCT Application No. WO 93/21334 |
| pathogen inducible PRP1 | Ward et al., 1993 Plant. Mol. Biol. 22:361-366 |
| heat inducible hsp80 | U.S. Patent No. 5187267 |
| cold inducible alpha-amylase | PCT Application No. WO 96/12814 |
| Wound-inducible pinll | European Patent No. 375091 |
| RD29A - salt-inducible | Yamaguchi-Shinozalei et al. (1993) Mol. Gen. Genet. 236:331-340 |
| plastid-specific viral RNA-polymerase | PCT Application No. WO 95/16783 and. WO 97/06250 |

Other promotors, e.g. superpromotor (Ni et al,., Plant Journal 7, 1995: 661-676), Ubiquitin promotor (Callis et al., J. Biol. Chem., 1990, 265: 12486-12493; US 5,510,474; US 6,020,190; Kawalleck et al., Plant. Molecular Biology, 1993, 21: 673-684) or 34S promotor (GenBank Accession numbers M59930 and X16673) were similar useful for the present invention and are known to a person skilled in the art.

Developmental stage-preferred promoters are preferentially expressed at certain stages of development. Tissue and organ preferred promoters include those that are preferentially expressed in certain tissues or organs, such as leaves, roots, seeds, or xylem. Examples of tissue preferred and organ preferred promoters include, but are not limited to fruit-preferred, ovule-preferred, male tissue-preferred, seed-preferred, integument-preferred, tuber-preferred, stalk-preferred, pericarp-preferred, and leaf-preferred, stigma-preferred, pollen-preferred, anther-preferred, a petal-preferred, sepal-preferred, pedicel-preferred, silique-preferred, stem-preferred, root-preferred promoters, and the like. Seed preferred promoters are preferentially expressed during seed development and/or germination. For example, seed preferred promoters can be embryo-preferred, endosperm preferred, and seed coat-preferred. See Thompson et al., 1989, BioEssays 10:108. Examples of seed preferred promoters include, but are not limited to, cellulose synthase (celA), Cim1, gamma-zein, globulin-1, maize 19 kD zein (cZ19B1), and the like.

Other promoters useful in the expression cassettes of the invention include, but are not limited to, the major chlorophyll a/b binding protein promoter, histone promoters, the Ap3 promoter, the β-conglycin promoter, the napin promoter, the soybean lectin promoter, the maize 15kD zein promoter, the 22kD zein promoter, the 27kD zein promoter, the g-zein promoter, the waxy, shrunken 1, shrunken 2 and bronze promoters, the Zm13 promoter (U.S. Patent No. 5,086,169), the maize polygalacturonase promoters (PG) (U.S. Patent Nos. 5,412,085 and 5,545,546), and the SGB6 promoter (U.S. Patent No. 5,470,359), as well as synthetic or other natural promoters.

Additional flexibility in controlling heterologous gene expression in plants may be obtained by using DNA binding domains and response elements from heterologous sources (*i.e*., DNA binding domains from non-plant sources). An example of such a heterologous DNA binding domain is the LexA DNA binding domain (Brent and Ptashne, 1985, Cell 43:729-736).

The invention further provides a recombinant expression vector comprising a SRP DNA molecule of the invention cloned into the expression vector in an antisense orientation. That is, the DNA molecule is operatively linked to a regulatory sequence in a manner that allows for expression (by transcription of the DNA molecule) of an RNA molecule that is antisense to a SRP mRNA. Regulatory sequences operatively linked to a nucleic acid molecule cloned in the antisense orientation can be chosen which direct the continuous expression of the antisense RNA molecule in a variety of cell types. For instance, viral promoters and/or enhancers, or regulatory sequences can be chosen which direct constitutive, tissue specific, or cell type specific expression of antisense RNA. The antisense expression vector can be in the form of a recombinant plasmid, phagemid, or attenuated virus wherein antisense nucleic acids are produced under the control of a high efficiency regulatory region. The activity of the regulatory region can be determined by the cell type into which the vector is introduced. For a discussion of the regulation of gene expression using antisense genes, see Weintraub, H. et al., 1986, Antisense RNA as a molecular tool for genetic analysis, Reviews - Trends in Genetics, Vol. 1(1), and Mol et al., 1990, FEBS Letters 268:427-430.

Another aspect of the invention pertains to isolated SRPs, and biologically active portions thereof. An "isolated" or "purified" polypeptide or biologically active portion thereof is free of some of the cellular material when produced by recombinant DNA techniques, or chemical precursors or other chemicals when chemically synthesized. The language "substantially free of cellular material" includes preparations of SRP in which the polypeptide is separated from some of the cellular components of the cells in which it is naturally or recombinantly produced. In one embodiment, the language "substantially free of cellular material" includes preparations of a SRP having less than about 30% (by dry weight) of non-SRP material (also referred to herein as a "contaminating polypeptide"), more preferably less than about 20% of non-SRP material, still more preferably less than about 10% of non-SRP material, and most preferably less than about 5% non-SRP material.

When the SRP or biologically active portion thereof is recombinantly produced, it is also preferably substantially free of culture medium, i.e., culture medium represents less than about 20%, more preferably less than about 10%, and most preferably less than about 5% of the volume of the polypeptide preparation. The language "substantially free of chemical precursors or other chemicals" includes preparations of SRP in which the polypeptide is separated from chemical precursors or other chemicals that are involved in the synthesis of the polypeptide. In one embodiment, the language "substantially free of chemical precursors or other chemicals" includes preparations of a SRP having less than about 30% (by dry weight) of chemical precursors or non-SRP chemicals, more preferably less than about 20% chemical precursors or non-SRP chemicals, still more preferably less than about 10% chemical precursors or non-SRP chemicals, and most preferably less than about 5% chemical precursors or non-SRP chemicals. In preferred embodiments, isolated polypeptides, or biologically active portions thereof, lack contaminating polypeptides from the same organism from which the SRP is derived. Typically, such polypeptides are produced by recombinant expression of, for example, a *Saccharomyces cerevisiae,* E.coli or *Brassica napus, Glycine max, Zea mays* or *Oryza sativa* SRP in plants other than *Saccharomyces cerevisiae,* E.coli, or microorganisms such as *C. glutamicum,* ciliates, algae or fungi.

The nucleic acid molecules, polypeptides, polypeptide homologs, fusion polypeptides, primers, vectors, and host cells described herein can be used in one or more of the following methods: identification of *Saccharomyces cerevisiae, E.coli or Brassica napus, Glycine max, Zea mays* or *Oryza sativa* and related organisms; mapping of genomes of organisms related to *Saccharomyces cerevisiae, E.coli*; identification and localization of *Saccharomyces cerevisiae, E.coli* or *Brassica napus, Glycine max, Zea mays* or *Oryza sativa* sequences of interest; evolutionary studies; determination of SRP regions required for function; modulation of a SRP activity; modulation of the metabolism of one or more cell functions; modulation of the transmembrane transport of one or more compounds; modulation of stress resistance; and modulation of expression of SRP nucleic acids.

The SRP nucleic acid molecules of the invention are also useful for evolutionary and polypeptide structural studies. The metabolic and transport processes in which the molecules of the invention participate are utilized by a wide variety of prokaryotic and eukaryotic cells; by comparing the sequences of the nucleic acid molecules of the present invention to those encoding similar enzymes from other organisms, the evolutionary relatedness of the organisms can be assessed. Similarly, such a comparison permits an assessment of which regions of the sequence are conserved and which are not, which may aid in determining those regions of the polypeptide that are essential for the functioning of the enzyme. This type of determination is of value for polypeptide engineering studies and may give an indication of what the polypeptide can tolerate in terms of mutagenesis without losing function.

Manipulation of the SRP nucleic acid molecules of the invention may result in the production of SRPs having functional differences from the wild-type SRPs. These polypeptides may be improved in efficiency or activity, may be present in greater numbers in the cell than is usual, or may be decreased in efficiency or activity.

There are a number of mechanisms by which the alteration of a SRP of the invention may directly affect stress response and/or stress tolerance. In the case of plants expressing SRPs, increased transport can lead to improved salt and/or solute partitioning within the plant tissue and organs. By either increasing the number or the activity of transporter molecules which export ionic molecules from the cell, it may be possible to affect the salt and cold tolerance of the cell.

The effect of the genetic modification in plants, on stress tolerance can be assessed by growing the modified plant under less than suitable conditions and then analyzing the growth characteristics and/or metabolism of the plant. Such analysis techniques are well known to one skilled in the art, and include dry weight, wet weight, polypeptide synthesis, carbohydrate synthesis, lipid synthesis, evapotranspiration rates, general plant and/or crop yield, flowering, reproduction, seed setting, root growth, respiration rates, photosynthesis rates, etc. (Applications of HPLC in Biochemistry in: Laboratory Techniques in Biochemistry and Molecular Biology, vol. 17; Rehm et al., 1993 Biotechnology, vol. 3, Chapter III: Product recovery and purification, page 469-714, VCH: Weinheim; Belter, P.A. et al., 1988, Bioseparations: downstream processing for biotechnology, John Wiley and Sons; Kennedy, J.F. and Cabral, J.M.S., 1992, Recovery processes for biological materials, John Wiley and Sons; Shaeiwitz, J.A. and Henry, J.D., 1988, Biochemical separations, in: Ulmann's Encyclopedia of Industrial Chemistry, vol. B3, Chapter 11, page 1-27, VCH: Weinheim; and Dechow, F.J., 1989, Separation and purification techniques in biotechnology, Noyes Publications).

For example, yeast expression vectors comprising the nucleic acids disclosed herein, or fragments thereof, can be constructed and transformed into *Saccharomyces cerevisiae* using standard protocols. The resulting transgenic cells can then be assayed for fail or alteration of their tolerance to drought, salt, and cold stress. Similarly, plant expression vectors comprising the nucleic acids disclosed herein, or fragments thereof, can be constructed and transformed into an appropriate plant cell such as *Arabidopsis,* soy, rape, maize, cotton, rice, wheat, *Medicago truncatula,* etc., using standard protocols. The resulting transgenic cells and/or plants derived therefrom can then be assayed for fail or alteration of their tolerance to drought, salt, cold stress.

The engineering of one or more genes according to table I and coding for the SRP of table II of the invention may also result in SRPs having altered activities which indirectly impact the stress response and/or stress tolerance of algae, plants, ciliates, or fungi, or other microorganisms like *C*. *glutamicum.*

Additionally, the sequences disclosed herein, or fragments thereof, can be used to generate knockout mutations in the genomes of various organisms, such as bacteria, mammalian cells, yeast cells, and plant cells (Girke, T., 1998, The Plant Journal 15:39-48). The resultant knockout cells can then be evaluated for their ability or capacity to tolerate various stress conditions, their response to various stress conditions, and the effect on the phenotype and/or genotype of the mutation. For other methods of gene inactivation, see U.S. Patent No. 6,004,804 "Non-Chimeric Mutational Vectors" and Puttaraju et al., 1999, Spliceosome-mediated RNA trans-splicing as a tool for gene therapy, Nature Biotechnology 17:246-252.

The aforementioned mutagenesis strategies for SRPs resulting in increased stress resistance are not meant to be limiting; variations on these strategies will be readily apparent to one skilled in the art. Using such strategies, and incorporating the mechanisms disclosed herein, the nucleic acid and polypeptide molecules of the invention may be utilized to generate algae, ciliates, plants, fungi, or other microorganisms like *C*. *glutamicum* expressing mutated SRP nucleic acid and polypeptide molecules such that the stress tolerance is improved.

The present invention also provides antibodies that specifically bind to a SRP, or a portion thereof, as encoded by a nucleic acid described herein. Antibodies can be made by many well-known methods (See, e.g. Harlow and Lane, "Antibodies; A Laboratory Manual," Cold Spring Harbor Laboratory, Cold Spring Harbor, New York, (1988)). Briefly, purified antigen can be injected into an animal in an amount and in intervals sufficient to elicit an immune response. Antibodies can either be purified directly, or spleen cells can be obtained from the animal. The cells can then fused with an immortal cell line and screened for antibody secretion. The antibodies can be used to screen nucleic acid clone libraries for cells secreting the antigen. Those positive clones can then be sequenced. See, for example, Kelly et al., 1992, Bio/Technology 10:163-167; Bebbington et al., 1992, Bio/Technology 10:169-175.

The phrases "selectively binds" and "specifically binds" with the polypeptide refer to a binding reaction that is determinative of the presence of the polypeptide in a heterogeneous population of polypeptides and other biologics. Thus, under designated immunoassay conditions, the specified antibodies bound to a particular polypeptide do not bind in a significant amount to other polypeptides present in the sample. Selective binding of an antibody under such conditions may require an antibody that is selected for its specificity for a particular polypeptide. A variety of immunoassay formats may be used to select antibodies that selectively bind with a particular polypeptide. For example, solid-phase ELISA immunoassays are routinely used to select antibodies selectively immunoreactive with a polypeptide. See Harlow and Lane, "Antibodies, A Laboratory Manual," Cold Spring Harbor Publications, New York, (1988), for a description of immunoassay formats and conditions that could be used to determine selective binding.

In some instances, it is desirable to prepare monoclonal antibodies from various hosts. A description of techniques for preparing such monoclonal antibodies may be found in Stites et al., eds., "Basic and Clinical Immunology," (Lange Medical Publications, Los Altos, Calif., Fourth Edition) and references cited therein, and in Harlow and Lane, "Antibodies, A Laboratory Manual," Cold Spring Harbor Publications, New York, (1988).

Gene expression in plants is regulated by the interaction of protein transcription factors with specific nucleotide sequences within the regulatory region of a gene. One example of transcription factors are polypeptides that contain zinc finger (ZF) motifs. Each ZF module is approximately 30 amino acids long folded around a zinc ion. The DNA recognition domain of a ZF protein is a α-helical structure that inserts into the major grove of the DNA double helix. The module contains three amino acids that bind to the DNA with each amino acid contacting a single base pair in the target DNA sequence. ZF motifs are arranged in a modular repeating fashion to form a set of fingers that recognize a contiguous DNA sequence. For example, a three-fingered ZF motif will recognize 9 bp of DNA. Hundreds of proteins have been shown to contain ZF motifs with between 2 and 37 ZF modules in each protein (Isalan M, et al., 1998 Biochemistry 37(35):12026-33; Moore M, et al., 2001 Proc. Natl. Acad. Sci. USA 98(4):1432-1436 and 1437-1441; US patents US 6007988 and US 6013453).

The regulatory region of a plant gene contains many short DNA sequences (cis-acting elements) that serve as recognition domains for transcription factors, including ZF proteins. Similar recognition domains in different genes allow the coordinate expression of several genes encoding enzymes in a metabolic pathway by common transcription factors. Variation in the recognition domains among members of a gene family facilitates differences in gene expression within the same gene family, for example, among tissues and stages of development and in response to environmental conditions.

Typical ZF proteins contain not only a DNA recognition domain but also a functional domain that enables the ZF protein to activate or repress transcription of a specific gene. Experimentally, an activation domain has been used to activate transcription of the target gene (US patent 5789538 and patent application WO9519431), but it is also possible to link a transcription repressor domain to the ZF and thereby inhibit transcription (patent applications WO00/47754 and WO2001002019). It has been reported that an enzymatic function such as nucleic acid cleavage can be linked to the ZF (patent application WO00/20622)

The invention provides a method that allows one skilled in the art to isolate the regulatory region of one or more stress related protein encoding genes from the genome of a plant cell and to design zinc finger transcription factors linked to a functional domain that will interact with the regulatory region of the gene. The interaction of the zinc finger protein with the plant gene can be designed in such a manner as to alter expression of the gene and preferably thereby to confer increased tolerance of abiotic stress and increased biomass production.

In particular, the invention provides a method of producing a transgenic plant with a stress related protein coding nucleic acid, wherein expression of the nucleic acid(s) in the plant results in increased tolerance to environmental stress as compared to a wild type plant comprising: (a) transforming a plant cell with an expression vector comprising a stress related protein encoding nucleic acid, and (b) generating from the plant cell a transgenic plant with an increased tolerance to environmental stress as compared to a wild type plant. For such plant transformation, binary vectors such as pBinAR can be used (Höfgen and Willmitzer, 1990 Plant Science 66:221-230). Moreover suitable binary vectors are for example pBIN19, pBI101, pGPTV or pPZP (Hajukiewicz, P. et al., 1994, Plant Mol. Biol., 25: 989-994).

Construction of the binary vectors can be performed by ligation of the cDNA into the T-DNA. 5' to the cDNA a plant promoter activates transcription of the cDNA. A polyadenylation sequence is located 3' to the cDNA. Tissue-specific expression can be achieved by using a tissue specific promoter as listed above. Also, any other promoter element can be used. For constitutive expression within the whole plant, the CaMV 35S promoter can be used. The expressed protein can be targeted to a cellular compartment using a signal peptide, for example for plastids, mitochondria or endoplasmic reticulum (Kermode, 1996 Crit. Rev. Plant Sci. 4(15):285-423). The signal peptide is cloned 5' in frame to the cDNA to archive subcellular localization of the fusion protein. Additionally, promoters that are responsive to abiotic stresses can be used with, such as the Arabidopsis promoter RD29A. One skilled in the art will recognize that the promoter used should be operatively linked to the nucleic acid such that the promoter causes transcription of the nucleic acid which results in the synthesis of a mRNA which encodes a polypeptide.

Alternate methods of transfection include the direct transfer of DNA into developing flowers via electroporation or Agrobacterium mediated gene transfer. Agrobacterium mediated plant transformation can be performed using for example the GV3101(pMP90) (Koncz and Schell, 1986 Mol. Gen. Genet. 204:383-396) or LBA4404 (Ooms et al., Plasmid, 1982, 7: 15-29; Hoekema et al., Nature, 1983, 303: 179-180) Agrobacterium tumefaciens strain. Transformation can be performed by standard transformation and regeneration techniques (Deblaere et al., 1994 Nucl. Acids. Res. 13:4777-4788; Gelvin and Schilperoort, Plant Molecular Biology Manual, 2nd Ed. - Dordrecht: Kluwer Academic Publ., 1995. - in Sect., Ringbuc Zentrale Signatur: BT11-P ISBN 0-7923-2731-4; Glick, B R and Thompson, J E, Methods in Plant Molecular Biology and Biotechnology, Boca Raton : CRC Press, 1993. - 360 S., ISBN 0-8493-5164-2). For example, rapeseed can be transformed via cotyledon or hypocotyl transformation (Moloney et al., 1989 Plant Cell Reports 8:238-242; De Block et al., 1989 Plant Physiol. 91:694-701). Use of antibiotics for Agrobacterium and plant selection depends on the binary vector and the Agrobacterium strain used for transformation. Rapeseed selection is normally performed using kanamycin as selectable plant marker. Agrobacterium mediated gene transfer to flax can be performed using, for example, a technique described by Mlynarova et al., 1994 Plant Cell Report 13:282-285. Additionally, transformation of soybean can be performed using for example a technique described in European Patent No. 0424 047, U.S. Patent No. 5,322,783, European Patent No. 0397 687, U.S. Patent No. 5,376,543 or U.S. Patent No. 5,169,770. Transformation of maize can be achieved by particle bombardment, polyethylene glycol mediated DNA uptake or via the silicon carbide fiber technique (see, for example, Freeling and Walbot "The maize handbook" Springer Verlag: New York (1993) ISBN 3-540-97826-7). A specific example of maize transformation is found in U.S. Patent No. 5,990,387 and a specific example of wheat transformation can be found in PCT Application No. WO 93/07256.

Growing the modified plants under stress conditions and then screening and analyzing the growth characteristics and/or metabolic activity assess the effect of the genetic modification in plants on stress tolerance and/or resistance and/or increased biomass production. Such analysis techniques are well known to one skilled in the art. They include next to screening (Römpp Lexikon Biotechnologie, Stuttgart/New York: Georg Thieme Verlag 1992, "screening" p. 701) dry weight, wet weight, protein synthesis, carbohydrate synthesis, lipid synthesis, evapotranspiration rates, general plant and/or crop yield, flowering, reproduction, seed setting, root growth, respiration rates, photosynthesis rates, etc. (Applications of HPLC in Biochemistry in: Laboratory Techniques in Biochemistry and Molecular Biology, vol. 17; Rehm et al., 1993 Biotechnology, vol. 3, Chapter III: Product recovery and purification, page 469-714, VCH: Weinheim; Belter, P.A. et al., 1988 Bioseparations: downstream processing for biotechnology, John Wiley and Sons; Kennedy, J.F. and Cabral, J.M.S., 1992 Recovery processes for biological materials, John Wiley and Sons; Shaeiwitz, J.A. and Henry, J.D., 1988 Biochemical separations, in: Ulmann's Encyclopedia of Industrial Chemistry, vol. B3, Chapter 11, page 1-27, VCH: Weinheim; and Dechow, F.J. (1989) Separation and purification techniques in biotechnology, Noyes Publications).

In one embodiment, the present invention relates to a method for the identification of a gene product conferring increased tolerance and/or resistance to environmental stress and increased biomass production as compared to a corresponding non-transformed wild type cell in a cell of an organism for example plant, comprising the following steps:
a) contacting, e.g. hybridising, some or all nucleic acid molecules of a sample, e.g. cells, tissues, plants or microorganisms or a nucleic acid library, which can contain a candidate gene encoding a gene product conferring increased tolerance and/or resistance to environmental stress and increased biomass, with a nucleic acid molecule as shown in column 5 or 7 of Table I A or B or a functional homologue thereof;
b) identifying the nucleic acid molecules, which hybridize under relaxed stringent conditions with said nucleic acid molecule, in particular to the nucleic acid molecule sequence shown in column 5 or 7 of Table I and, optionally, isolating the full length cDNA clone or complete genomic clone;
c) identifying the candidate nucleic acid molecules or a fragment thereof in host cells, preferably in a plant cell
d) increasing the expressing of the identified nucleic acid molecules in the host cells for which increased tolerance and/or resistance to environmental stress and increased biomass production as desired
e) assaying the level of tolerance and/or resistance to environmental stress and increased biomass production of the host cells; and
f) identifying the nucleic acid molecule and its gene product which increased expression confers increased tolerance and/or resistance to environmental stress and increased biomass production in the host cell compared to the wild type.

Relaxed hybridisation conditions are: After standard hybridisation procedures washing steps can be performed at low to medium stringency conditions usually with washing conditions of 40°-55°C and salt conditions between 2xSSC and 0,2x SSC with 0,1% SDS in comparison to stringent washing conditions as e.g. 60°to 68°C with 0,1% SDS. Further examples can be found in the references listed above for the stringend hybridization conditions. Usually washing steps are repeated with increasing stringency and length until a useful signal to noise ratio is detected and depend on many factors as the target, e.g. its purity, GC-content, size etc, the probe, e.g.its length, is it a RNA or a DNA probe, salt conditions, washing or hybridisation temperature, washing or hybridisation time etc.

In another embodiment, the present invention relates to a method for the identification of a gene product the expression of which confers an increased tolerance and/or resistance to environmental stress and increased biomass production in a cell, comprising the following steps:
a) identifiying a nucleic acid molecule in an organism, which is at least 20%, preferably 25%, more preferably 30%, even more preferred are 35%. 40% or 50%, even more preferred are 60%, 70% or 80%, most preferred are 90% or 95% or more homolog to the nucleic acid molecule encoding a protein comprising the polypeptide molecule as shown in column 5 or 7 of Table II or comprising a consensus sequence or a polypeptide motif as shown in column 7 of Table IV or being encoded by a nucleic acid molecule comprising a polynucleotide as shown in column 5 or 7 of Table I or a homologue thereof as described herein , for example via homology search in a data bank;
b) enhancing the expression of the identified nucleic acid molecules in the host cells;
c) assaying the level of tolerance and/or resistance to environmental stress and increased biomass production in the host cells; and
d) identifying the host cell, in which the enhanced expression confers increased tolerance and/or resistance to environmental stress and increased biomass production in the host cell compared to a wild type.

Further, the nucleic acid molecule disclosed herein, in particular the nucleic acid molecule shown column 5 or 7 of Table I A or B, may be sufficiently homologous to the sequences of related species such that these nucleic acid molecules may serve as markers for the construction of a genomic map in related organism or for association mapping. Furthermore natural variation in the genomic regions corresponding to nucleic acids disclosed herein, in particular the nucleic acid molecule shown column 5 or 7 of Table I A or B, or homologous thereof may lead to variation in the activity of the proteins disclosed herein, in particular the proteins comprising polypeptides as shown in column 5 or 7 of Table II A or B or comprising the consensus sequence or the polypeptide motif as shown in column 7 of Table IV, and their homolgous and in consequence in natural variation in tolerance and/or resistance to environmental stess and biomass production.

In consequnce natural variation eventually also exists in form of more active allelic variants leading already to a relative increase in the tolerance and/or resistance to environmental stress and biomass production. Different variants of the nucleic acids molecule disclosed herein, in particular the nucleic acid comprising the nucleic acid molecule as shown column 5 or 7 of Table I A or B, which corresponds to different tolerance and/or environmental stress resistance and biomass production levels can be indentified and used for marker assisted breeding for increase toerance and/or resistance to environmental stress and increased biomass production.

Accordingly, the present invention relates to a method for breeding plants for increased tolerance and/or resistance to environmental stress and increased biomass production, comprising
a) selecting a first plant variety with increased tolerance and/or resistance to environmental stress and increased biomass production based on increased expression of a nucleic acid of the invention as disclosed herein, in particular of a nucleic acid molecule comprising a nucleic acid molecule as shown in column 5 or 7 of Table I A or B or a polypeptide comprising a polypeptide as shown in column 5 or 7 of Table II A or B or comprising a consensus sequence or a polypeptide motif as shown in column 7 of Table IV, or a homologue thereof as described herein;
b) associating the level of tolerance and/or resistance to environmental stress and biomass production with the expression level or the genomic structure of a gene encoding said polypeptide or said nucleic acid molecule;
c) crossing the first plant variety with a second plant variety, which significantly differs in its level of tolerance and/or resistance to environmental stress and biomass production and
e) identifying, which of the offspring varieties has got increased levels level of tolerance and/or resistance to environmental stress and biomass production by the expression level of said polypeptide or nucleic acid molecule or the genomic structure of the genes encoding said polypeptide or nucleic acid molecule of the invention.

In one embodiment, the expression level of the gene according to step (b) is increased.

Yet another embodiment of the invention relates to a process for the identification of a compound conferring increased tolerance and/or resistance to environmental stress and increased biomass production as compared to a corresponding non-transformed wild type plant cell, a plant or a part thereof in a plant cell, a plant or a part thereof, a plant or a part thereof, comprising the steps:
a) culturing a plant cell; a plant or a part thereof maintaining a plant expressing the polypeptide as shown in column 5 or 7 of Table II or being encoded by a nucleic acid molecule comprising a polynucleotide as shown in column 5 or 7 of Table I or a homologue thereof as described herein or a polynucleotide encoding said polypeptide and conferring an increased tolerance and/or resistance to environmental stress and increased biomass production as compared to a corresponding non-transformed wild type plant cell, a plant or a part thereof; a non-transformed wild type plant or a part thereof and providing a readout system capable of interacting with the polypeptide under suitable conditions which permit the interaction of the polypeptide with this readout system in the presence of a chemical compound or a sample comprising a plurality of chemical compounds and capable of providing a detectable signal in response to the binding of a chemical compound to said polypeptide under conditions which permit the expression of said readout system and of the protein as shown in column 5 or 7 of Table II or being encoded by a nucleic acid molecule comprising a polynucleotide as shown in column 5 or 7 of Table I or a homologue thereof as described herein; and
b) identifying if the chemical compound is an effective agonist by detecting the presence or absence or decrease or increase of a signal produced by said readout system.

Said compound may be chemically synthesized or microbiologically produced and/or comprised in, for example, samples, e.g., cell extracts from, e.g., plants, animals or microorganisms, e.g. pathogens. Furthermore, said compound(s) may be known in the art but hitherto not known to be capable of suppressing the polypeptide of the present invention. The reaction mixture may be a cell free extract or may comprise a cell or tissue culture. Suitable set ups for the process for identification of a compound of the invention are known to the person skilled in the art and are, for example, generally described in Alberts et al., Molecular Biology of the Cell, third edition (1994), in particular Chapter 17. The compounds may be, e.g., added to the reaction mixture, culture medium, injected into the cell or sprayed onto the plant.

If a sample containing a compound is identified in the process, then it is either possible to isolate the compound from the original sample identified as containing the compound capable of activating or increasing tolerance and/or resistance to environmental stress and increased biomass production as compared to a corresponding non-transformed wild type, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the said process only comprises a limited number of or only one substance(s). Preferably said sample comprises substances of similar chemical and/or physical properties, and most preferably said substances are identical. Preferably, the compound identified according to the described method above or its derivative is further formulated in a form suitable for the application in plant breeding or plant cell and tissue culture.

The compounds which can be tested and identified according to said process may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Said compounds can also be functional derivatives or analogues of known inhibitors or activators. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art. Furthermore, peptidomimetics and/or computer aided design of appropriate derivatives and analogues can be used, for example, according to the methods described above. The cell or tissue that may be employed in the process preferably is a host cell, plant cell or plant tissue of the invention described in the embodiments hereinbefore.

Thus, in a further embodiment the invention relates to a compound obtained or identified according to the method for identifying an agonist of the invention said compound being an antagonist of the polypeptide of the present invention.

Accordingly, in one embodiment, the present invention further relates to a compound identified by the method for identifying a compound of the present invention.

In one embodiment, the invention relates to an antibody specifically recognizing the compound or agonist of the present invention.

The invention also relates to a diagnostic composition comprising at least one of the aforementioned nucleic acid molecules, antisense nucleic acid molecule, RNAi, snRNA, dsRNA, siRNA, miRNA, ta-siRNA, cosuppression molecule, ribozyme, vectors, proteins, antibodies or compounds of the invention and optionally suitable means for detection.

The diagnostic composition of the present invention is suitable for the isolation of mRNA from a cell and contacting the mRNA so obtained with a probe comprising a nucleic acid probe as described above under hybridizing conditions, detecting the presence of mRNA hybridized to the probe, and thereby detecting the expression of the protein in the cell. Further methods of detecting the presence of a protein according to the present invention comprise immunotechniques well known in the art, for example enzyme linked immunoadsorbent assay. Furthermore, it is possible to use the nucleic acid molecules according to the invention as molecular markers or primers in plant breeding. Suitable means for detection are well known to a person skilled in the art, e.g. buffers and solutions for hydridization assays, e.g. the afore-mentioned solutions and buffers, further and means for Southern-, Western-, Northern- etc. -blots, as e.g. described in Sambrook et al. are known. In one embodiment diagnostic composition contain PCR primers designed to specifically detect the presense or the expression level of the nucleic acid molecule to be reduced in the process of the invention, e.g. of the nucleic acid molecule of the invention, or to descriminate between different variants or alleles of the nucleic acid molecule of the invention or which activity is to be reduced in the process of the invention.

In another embodiment, the present invention relates to a kit comprising the nucleic acid molecule, the vector, the host cell, the polypeptide, or the antisense, RNAi, snRNA, dsRNA, siRNA, miRNA, ta-siRNA, cosuppression molecule, or ribozyme molecule, or the viral nucleic acid molecule, the antibody, plant cell, the plant or plant tissue, the harvestable part, the propagation material and/or the compound and/or agonist identified according to the method of the invention.

The compounds of the kit of the present invention may be packaged in containers such as vials, optionally with/in buffers and/or solution. If appropriate, one or more of said components might be packaged in one and the same container. Additionally or alternatively, one or more of said components might be adsorbed to a solid support as, e.g. a nitrocellulose filter, a glas plate, a chip, or a nylon membrane or to the well of a micro titerplate. The kit can be used for any of the herein described methods and embodiments, e.g. for the production of the host cells, transgenic plants, pharmaceutical compositions, detection of homologous sequences, identification of antagonists or agonists, as food or feed or as a supplement thereof or as supplement for the treating of plants, etc.

Further, the kit can comprise instructions for the use of the kit for any of said embodiments.

In one embodiment said kit comprises further a nucleic acid molecule encoding one or more of the aforementioned protein, and/or an antibody, a vector, a host cell, an antisense nucleic acid, a plant cell or plant tissue or a plant. In another embodiment said kit comprises PCR primers to detect and discrimante the nucleic acid molecule to be reduced in the process of the invention, e.g. of the nucleic acid molecule of the invention.

In a further embodiment, the present invention relates to a method for the production of an agricultural composition providing the nucleic acid molecule for the use according to the process of the invention, the nucleic acid molecule of the invention, the vector of the invention, the antisense, RNAi, snRNA, dsRNA, siRNA, miRNA, ta-siRNA, cosuppression molecule, ribozyme, or antibody of the invention, the viral nucleic acid molecule of the invention, or the polypeptide of the invention or comprising the steps of the method according to the invention for the identification of said compound or agonist; and formulating the nucleic acid molecule, the vector or the polypeptide of the invention or the agonist, or compound identified according to the methods or processes of the present invention or with use of the subject matters of the present invention in a form applicable as plant agricultural composition.

In another embodiment, the present invention relates to a method for the production of the plant culture composition comprising the steps of the method of the present invention; and formulating the compound identified in a form acceptable as agri-cultural composition.

Under "acceptable as agricultural composition" is understood, that such a composition is in agreement with the laws regulating the content of fungicides, plant nutrients, herbizides, etc. Preferably such a composition is without any harm for the protected plants and the animals (humans included) fed therewith.

Throughout this application, various publications are referenced. The disclosures of all of these publications and those references cited within those publications in their entireties are hereby incorporated by reference into this application in order to more fully describe the state of the art to which this invention pertains.

It should also be understood that the foregoing relates to preferred embodiments of the present invention and that numerous changes and variations may be made therein without departing from the scope of the invention. The invention is further illustrated by the following examples, which are not to be construed in any way as limiting. On the contrary, it is to be clearly understood that various other embodiments, modifications and equivalents thereof, which, after reading the description herein, may suggest themselves to those skilled in the art without departing from the spirit of the present invention and/or the scope of the claims.

In one embodiment the invention relates to subject matter summarized in the following Items:
Item 1. A method for producing a transgenic plant cell, a plant or a part thereof with increased tolerance and/or resistance to environmental stress and increased biomass production as compared to a corresponding non-transformed wild type plant cell, a plant or a part thereof by increasing or generating one or more activities selected from the group consisting of: 2,3-dihydroxy-2,3-dihydrophenylpropionatedehydrogenase, 3-deoxy-D-arabino-heptulosonate-7-phosphate (DAHP) synthase, 3-deoxy-D-arabinoheptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Yal049c-protein, YCR059C-protein, YEL005C-protein, YER156C-protein, Yfr042w-protein, YGL045W-protein, and YOR024w-protein.
Item 2. A method according to item 1 wherein the activity of at least one polypeptide comprising a polypeptide selected from the group consisting of:
   (i) a polypeptide comprising a polypeptide, a consensus sequence or at least one polypeptide motif as depicted in column 5 or 7 of Table II or of Table IV, respectively; or
   (ii) an expression product of a nucleic acid molecule comprising a polynucleotide as depicted in column 5 or 7 of Table I,
   (iii) or a functional equivalent of (i) or (ii);
   is increased or generated.
Item 3. A method of any one of the items 1 or 2 wherein the expression of at least one nucleic acid molecule comprising a nucleic acid molecule selected from the group consisting of:
   a) a nucleic acid molecule encoding the polypeptide shown in column 5 or 7 of Table II;
   b) a nucleic acid molecule shown in column 5 or 7 of Table I;
   c) a nucleic acid molecule, which, as a result of the degeneracy of the genetic code, can be derived from a polypeptide sequence depicted in column 5 or 7 of Table II and confers an increased tolerance and/or resistance to environmental stress and increased biomass production as compared to a corresponding non-transformed wild type plant cell, a plant or a part thereof;
   d) a nucleic acid molecule having at least 30 % identity with the nucleic acid molecule sequence of a polynucleotide comprising the nucleic acid molecule shown in column 5 or 7 of Table I and confers an increased tolerance and/or resistance to environmental stress and increased biomass production as compared to a corresponding non-transformed wild type plant cell, a plant or a part thereof;
   e) a nucleic acid molecule encoding a polypeptide having at least 30 % identity with the amino acid sequence of the polypeptide encoded by the nucleic acid molecule of (a) to (c) and having the activity represented by a nucleic acid molecule comprising a polynucleotide as depicted in column 5 of Table I and confers an increased tolerance and/or resistance to environmental stress and increased biomass production as compared to a corresponding non-transformed wild type plant cell, a plant or a part thereof;
   f) nucleic acid molecule which hybridizes with a nucleic acid molecule of (a) to (c) under stringent hybridization conditions and confers an increased tolerance and/or resistance to environmental stress and increased biomass production as compared to a corresponding non-transformed wild type plant cell, a plant or a part thereof;
   g) a nucleic acid molecule encoding a polypeptide which can be isolated with the aid of monoclonal or polyclonal antibodies made against a polypeptide encoded by one of the nucleic acid molecules of (a) to (e) and having the activity represented by the nucleic acid molecule comprising a polynucleotide as depicted in column 5 of Table I;
   h) a nucleic acid molecule encoding a polypeptide comprising the consensus sequence or one or more polypeptide motifs as shown in column 7 of Table IV and preferably having the activity represented by a nucleic acid molecule comprising a polynucleotide as depicted in column 5 of Table II or IV;
   h) a nucleic acid molecule encoding a polypeptide having the activity represented by a protein as depicted in column 5 of Table II and confers an increased tolerance and/or resistance to environmental stress and increased biomass production as compared to a corresponding non-transformed wild type plant cell, a plant or a part thereof;
   i) nucleic acid molecule which comprises a polynucleotide, which is obtained by amplifying a cDNA library or a genomic library using the primers in column 7 of Table III which do not start at their 5'-end with the nucleotides ATA and preferably having the activity represented by a nucleic acid molecule comprising a polynucleotide as depicted in column 5 of Table II or IV;
      and
   j) a nucleic acid molecule which is obtainable by screening a suitable nucleic acid library under stringent hybridization conditions with a probe com-prising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence characterized in (a) to (e) and encoding a polypeptide having the activity represented by a protein comprising a polypeptide as depicted in column 5 of Table II;
   is increased or generated.
Item 4. A trangenic plant cell, a plant or a part thereof with increased tolerance and/or resistance to environmental stress and increased biomass production as compared to a corresponding non-transformed wild type plant cell, a plant or a part thereof produced by a method according to item 1.
Item 5. The transgenic plant cell, a plant or a part thereof of item 4 derived from a monocotyledonous plant.
Item 6. The transgenic plant cell, a plant or a part thereof of item 4 derived from a dicotyledonous plant.
Item 7. The transgenic plant cell, a plant or a part thereof of item 4 , wherein the plant is selected from the group consisting of maize, wheat, rye, oat, triticale, rice, barley, soybean, peanut, cotton, oil seed rape, including canola and winter oil seed rape, corn, manihot, pepper, sunflower, flax, borage, safflower, linseed, primrose, rapeseed, turnip rape, tagetes, solanaceous plants, potato, tobacco, eggplant, tomato, Vicia species, pea, alfalfa, coffee, cacao, tea, Salix species, oil palm, coconut, perennial grass, forage crops and Arabidopsis thaliana.
Item 8. The transgenic plant cell, a plant or a part thereof of item 4 , derived from a gymnosperm plant, preferably spruce, pine and fir.
Item 9. A seed produced by a transgenic plant of any of items 5 to 8, wherein the seed is genetically homozygous for a transgene conferring increased tolerance and/or resistance to environmental stress and increased biomass production as compared to a corresponding non-transformed wild type plant cell, a plant or a part thereof resulting in an increased tolerance to environmental stress and increased biomass production as compared to a corresponding non-transformed wild type plant.
Item 10. An isolated nucleic acid molecule comprising a nucleic acid molecule selected from the group consisting of:
   a) a nucleic acid molecule encoding the polypeptide shown in column 5 or 7 of Table II B;
   b) a nucleic acid molecule shown in column 5 or 7 of Table I B;
   c) a nucleic acid molecule, which, as a result of the degeneracy of the genetic code, can be derived from a polypeptide sequence depicted in column 5 or 7 of Table II and confers an increased tolerance and/or resistance to environmental stress and increased biomass production as compared to a corresponding non-transformed wild type plant cell, a plant or a part thereof;
   d) a nucleic acid molecule having at least 30 % identity with the nucleic acid molecule sequence of a polynucleotide comprising the nucleic acid molecule shown in column 5 or 7 of Table I and confers an increased tolerance and/or resistance to environmental stress and increased biomass production as compared to a corresponding non-transformed wild type plant cell, a plant or a part thereof;
   e) a nucleic acid molecule encoding a polypeptide having at least 30 % identity with the amino acid sequence of the polypeptide encoded by the nucleic acid molecule of (a) to (c) and having the activity represented by a nucleic acid molecule comprising a polynucleotide as depicted in column 5 of Table I and confers an increased tolerance and/or resistance to environmental stress and increased biomass production as compared to a corresponding non-transformed wild type plant cell, a plant or a part thereof;
   f) nucleic acid molecule which hybridizes with a nucleic acid molecule of (a) to (c) under stringent hybridization conditions and confers an increased tolerance and/or resistance to environmental stress and increased biomass production as compared to a corresponding non-transformed wild type plant cell, a plant or a part thereof;
   g) a nucleic acid molecule encoding a polypeptide which can be isolated with the aid of monoclonal or polyclonal antibodies made against a polypeptide encoded by one of the nucleic acid molecules of (a) to (e) and having the activity represented by the nucleic acid molecule comprising a polynucleotide as depicted in column 5 of Table I;
   h) a nucleic acid molecule encoding a polypeptide comprising the consensus sequence or one or more polypeptide motifs as shown in column 7 of Table IV and preferably having the activity represented by a nucleic acid molecule comprising a polynucleotide as depicted in column 5 of Table II or IV;
   h) a nucleic acid molecule encoding a polypeptide having the activity represented by a protein as depicted in column 5 of Table II and confers an increased tolerance and/or resistance to environmental stress and increased biomass production as compared to a corresponding non-transformed wild type plant cell, a plant or a part thereof;
   i) nucleic acid molecule which comprises a polynucleotide, which is obtained by amplifying a cDNA library or a genomic library using the primers in column 7 of Table III which do not start at their 5'-end with the nucleotides ATA and preferably having the activity represented by a nucleic acid molecule comprising a polynucleotide as depicted in column 5 of Table II or IV;
      and
   j) a nucleic acid molecule which is obtainable by screening a suitable nucleic acid library under stringent hybridization conditions with a probe com-prising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence characterized in (a) to (e) and encoding a polypeptide having the activity represented by a protein comprising a polypeptide as depicted in column 5 of Table II;
   whereby the nucleic acid molecule according to (a) to (j) is at least in one or more nucleotides different from the sequence depicted in column 5 or 7 of Table I A and preferably which encodes a protein which differs at least in one or more amino acids from the protein sequences depicted in column 5 or 7 of Table II A.
Item 11. A nucleic acid construct which confers the expression of said nucleic acid molecule of item 10, comprising one or more regulatory elements, whereby expression of the nucleic acid in a host cell results in increased tolerance and/or resistance to environmental stress and increased biomass production as compared to a corresponding non-transformed wild type plant cell, a plant or a part thereof.
Item 12. A vector comprising the nucleic acid molecule as shown in item 10 or the nucleic acid construct of item 11, whereby expression of said coding nucleic acid in a host cell results in increased tolerance and/or resistance to environmental stress and increased biomass production as compared to a corresponding non-transformed wild type plant cell, a plant or a part thereof.
Item 13. A host cell, which has been transformed stably or transiently with the vector as shown in item 12 or the nucleic acid molecule as shown in item 10 or the nucleic acid construct of item 11 and which shows due to the transformation an increased tolerance and/or resistance to environmental stress and increased biomass production as compared to a corresponding non-transformed wild type plant cell, a plant or a part thereof.
Item 14. A process for producing a polypeptide, wherein the polypeptide is expressed in a host cell as shown in item 13.
Item 15. A polypeptide produced by the process as shown in item 14 or encoded by the nucleic acid molecule as shown in item 10 whereby the polypeptide distinguishes over the sequence as shown in table II by one or more amino acids
Item 16. An antibody, which binds specifically to the polypeptide as shown in item 15.
Item 17. A plant tissue, propagation material, harvested material or a plant comprising the host cell as shown in item 13.
Item 18. A process for the identification of a compound conferring an increased tolerance and/or resistance to environmental stress and increased biomass production as compared to a corresponding non-transformed wild type plant cell, a plant or a part thereof in a plant cell, a plant or a part thereof, a plant or a part thereof, comprising the steps:
   a) culturing a plant cell; a plant or a part thereof maintaining a plant expressing the polypeptide encoded by the nucleic acid molecule of item 10 conferring an increased tolerance and/or resistance to environmental stress and increased biomass production as compared to a corresponding non-transformed wild type plant cell, a plant or a part thereof; a non-transformed wild type plant or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of item 10 conferring an increased tolerance and/or resistance to environmental stress and increased biomass production as compared to a corresponding non-transformed wild type plant cell, a plant or a part thereof; a non-transformed wild type plant or a part thereof;
   b) identifying if the compound is an effective agonist by detecting the presence or absence or increase of a signal produced by said readout system.
Item 19. A method for the production of an agricultural composition comprising the steps of the method of item 18 and formulating the compound identified in item 18 in a form acceptable for an application in agriculture.
Item 20. A composition comprising the nucleic acid molecule of any of the items 10, the polypeptide of item 15, the nucleic acid construct of item 11, the vector of item 12, the compound of item 18, the antibody of item 16, and optionally an agricultural acceptable carrier.
Item 21. An isolated polypeptide as depicted in table II, preferably table II B which is selected from yeast, preferably Saccharomyces cerevisiae, or Escherichia coli, preferably Escherichia coli K12, and/or Synechocystis sp. PCC 6803 .
Item 22. A method of producing a transgenic plant cell, a plant or a part thereof with increased tolerance and/or resistance to environmental stress and increased biomass production compared to a corresponding non transformed wild type plant cell, a plant or a part thereof, wherein the tolerance and/or resistance to environmental stress and increased biomass production is increased by expression of a polypeptide encoded by a nucleic acid according to item 10 and results in increased tolerance and/or resistance to an environmental stress and increased biomass production as compared to a corresponding non-transformed wild type plant cell, a plant or a part thereof, comprising
   a) transforming a plant cell, or a part of a plant with an expression vector according to item 12 and
   b) generating from the plant cell or the part of a plant a transgenic plant with an increased tolerance to environmental stress and increased biomass production as compared to a corresponding non-transformed wild type plant.
Item 23. A method of producing a transgenic plant with increased biomass production compared to a corresponding non transformed wild type plant under conditions of environmental stress by increasing or generating one or more activities selected from the group of Stress-Related Proteins (SRP) consisting of: 2,3-dihydroxy-2,3-dihydrophenylpropionatedehydrogenase, 3-deoxy-D-arabino-heptulosonate-7-phosphate (DAHP) synthase, 3-deoxy-D-arabino-heptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Yal049c-protein, YCR059C-protein, YEL005C-protein, YER156C-protein, Yfr042w-protein, YGL045W-protein, and YOR024w-protein.
Item 24. A method according to item 22 comprising
   a) transforming a plant cell or a part of a plant with an expression vector according to item 12 and
   b) generating from the plant cell or the part of a plant a transgenic plant with an increased tolerance to environmental stress and increased biomass production as compared to a corresponding non-transformed wild type plant.
Item 25. Use of a SRP encoding nucleic acid molecule selected from the group comprising the nucleic acid of item 10 for preparing a plant cell with increased tolerance and/or resistance to environmental stress and increased biomass production as compared to a corresponding non-transformed wild type plant cell, a plant or part of a plant.
Item 26. Use of a SRP encoding nucleic acid molecule selected from the group comprising the nucleic acid according to item 10 or parts thereof as markers for selection of plants or plant cells with an increased tolerance and/or resistance to environmental stress and increased biomass production as compared to a corresponding non-transformed wild type plant cell; a non-transformed wild type plant or a part thereof.
Item 27. Use of a SRP encoding nucleic acid molecule selected from the group comprising the nucleic acid according to item 10 or parts thereof as markers for detection of stress in plants or plant cells.
Item 28. The transformed plant cell of item 1, wherein the environmental stress is selected from the group comprised of salinity, drought, temperature, metal, chemical, pathogenic and oxidative stresses, or combinations thereof.
Item 29. The transformed plant cell of item 1, wherein the environmental stress is drought and/or desiccation.
Item 30. A transgenic plant cell comprising a nucleic acid molecule encoding a polypeptide having a activity selected from the group of Stress-Related Proteins (SRP) consisting of: 2,3-dihydroxy-2,3-dihydrophenylpropionatedehydrogenase, 3-deoxy-D-arabino-heptulosonate-7-phosphate (DAHP) synthase, 3-deoxy-D-arabinoheptulosonate-7-phosphatesynthase, 3-oxoacyl-(acyl carrier protein) synthase, acid shock protein precursor, aspartate ammonia-lyase, b0081-protein, b0482-protein, b0631-protein, b0753-protein, b0866-protein, b1052-protein, b1161-protein, b1423-protein, b1878-protein, b2226-protein, b2475-protein, cellobiose/arbutin/salicin-specific PTS enzyme (IIB component/IC component), Checkpoint protein, CP4-57 prophage/ RNase LS, Dihydrouridine synthase, DNA-binding transcriptional dual regulator protein, D-xylose transporter subunit, gamma-Glu-putrescine synthase, gluconate transporter, glucose-1-phosphate thymidylyltransferase, Glutamine tRNA synthetase, glutathione-dependent oxidoreductase, glycine betaine transporter subunit protein, glycogen synthase, GTP cyclohydrolase I, heat shock protein, heat shock protein HtpX, heme lyase (CcmH subunit), hexuronate transporter, histidine/lysine/arginine/ornithine transporter subunit protein, HyaA/HyaB-processing protein, inner membrane protein, L-arabinose transporter subunit, Lsm (Like Sm) protein, L-threonine 3-dehydrogenase, methylglyoxal synthase, multidrug efflux system (subunit B), N,N'-diacetylchitobiose-specific enzyme IIA component of PTS, NADH dehydrogenase (subunit N), neutral amino-acid efflux system, nicotinamide-nucleotide adenylyltransferase, ornithine decarboxylase, pantothenate kinase, peptidyl-prolyl cis-trans isomerase A (rotamase A), phosphate transporter, phosphatidylglycerophosphate synthetase, polyphosphate kinase, potassium-transporting ATPase (subunit B), predicted antimicrobial peptide transporter subunit, predicted arginine/ornithine transporter, predicted hydrolase, predicted kinase, predicted ligase, predicted outer membrane lipoprotein, predicted oxidoreductase (flavin:NADH component), predicted porin, predicted PTS enzymes (IIB component/IIC component), predicted serine transporter protein, predicted transporter protein, Protein component of the small (40S) ribosomal subunit, regulator of length of O-antigen component of lipopolysaccharide chains , ribonuclease activity regulator protein RraA, sensory histidine kinase in two-component regulatory system with NarP (NarL), sodium/proton antiporter, Splicing factor, threonine and homoserine efflux system, transcriptional regulator protein, transcriptional repressor protein MetJ, transporter subunit / periplasmic-binding component of ABC superfamily, tRNA pseudouridine synthase, tRNA-specific adenosine deaminase, universal stress protein UP12, Yal049c-protein, YCR059C-protein, YEL005C-protein, YER156C-protein, Yfr042w-protein, YGL045W-protein, and YOR024w-protein. , wherein said polypeptide confers increased tolerance and/or resistance to environmental stress and increased biomass production as compared to a corresponding non-transformed wild type plant cell, a plant or part thereof, preferably when said polypeptide is overexpressed.
Item 31. A plant of item 1 or 29 that has
   i) a increased biomass production under conditions where water would be limiting for growth for a non-transformed wild type plant cell, a plant or part thereof
   ii) a increased biomass production under conditions of drought and/or desiccation where said conditions would be limiting for growth for a non-transformed wild type plant cell, a plant or part thereof
      and/or
   iii) a increased biomass production under conditions of low humidity where said conditions would be limiting for growth for a non-transformed wild type plant cell, a plant or part thereof.

### Example 1

### Engineering stress-tolerant Arabidopsis plants by over-expressing stress related protein genes.

### Cloning of the inventive sequences as shown in table I, column 5 and 7 for the expression in plants

* Sequences as shown in table I, column 5, marked with asterisk * reflect the respective sequence derived from public data bases information.

Unless otherwise specified, standard methods as described in Sambrook et al., Molecular Cloning: A laboratory manual, Cold Spring Harbor 1989, Cold Spring Harbor Laboratory Press are used.

The inventive sequences as shown in table I, column 5 and 7, were amplified by PCR as described in the protocol of the *Pfu* Ultra, *Pfu* Turbo or Herculase DNA polymerase (Stratagene).

The composition for the protocol of the *Pfu* Ultra, *Pfu* Turbo or Herculase DNA polymerase was as follows: 1x PCR buffer (Stratagene), 0.2 mM of each dNTP, 100 ng genomic DNA of *Saccharomyces cerevisiae* (strain S288C; Research Genetics, Inc., now Invitrogen), *Escherichia coli* (strain MG1655; *E.coli* Genetic Stock Center), or *Synechocystis sp.,* 50 pmol forward primer, 50 pmol reverse primer, 2.5 u *Pfu* Ultra, *Pfu* Turbo or Herculase DNA polymerase.

The amplification cycles were as follows:
1 cycle of 2-3 minutes at 94-95°C, followed by 25-36 cycles of in each case 30-60 seconds at 94-95°C, 30-45 seconds at 50-60°C and 210-480 seconds at 72°C, followed by 1 cycle of 5-10 minutes at 72°C, then 4°C.

The following adapter sequences were added to *Saccharomyces cerevisiae* ORF specific primers (see table III) for cloning purposes:
i) foward primer: 5'-GGAATTCCAGCTGACCACC-3'
   SEQ ID NO: **7**
ii) reverse primer: 5'-GATCCCCGGGAATTGCCATG-3'
   SEQ ID NO: **8**
   These adaptor sequences allow cloning of the ORF into the various vectors containing the *Resgen* adaptors, see table VII...

The following adapter sequences were added to *Escherichia coli* or *Synechocystis sp.* ORF specific primers for cloning purposes:
iii) forward primer: 5'-TTGCTCTTCC- 3'
   SEQ ID NO: **9**
iiii) reverse primer: 5'-TTGCTCTTCG-3'
   SEQ ID NO: **10**
   The adaptor sequences allow cloning of the ORF into the various vectors containing the *Colic* adaptors, see table VII...

Therefore for amplification and cloning of *Saccharomyces cerevisiae* SEQ ID NO: 6823, a primer consisting of the adaptor sequence i) and the ORF specific sequence SEQ ID NO: 6863 and a second primer consisting of the adaptor sequence ii) and the ORF specific sequence SEQ ID NO: 6864 were used.

For amplification and cloning of *Echerischia coli* SEQ ID NO: 38, a primer consisting of the adaptor sequence iii) and the ORF specific sequence SEQ ID NO: **48** and a second primer consisting of the adaptor sequence iiii) and the ORF specific sequence SEQ ID NO: **49** were used.

For amplification and cloning of *Synechocystis sp.* SEQ ID NO: 6542, a primer consisting of the adaptor sequence iii) and the ORF specific sequence SEQ ID NO: 6812 and a second primer consisting of the adaptor sequence iiii) and the ORF specific sequence SEQ ID NO: 6813 were used.

Following these examples every sequence disclosed in table I, preferably column 5, can be cloned by fusing the adaptor sequences to the respective specific primers sequences as disclosed in table III, column 7.

**Table VII. Overview of the different vectors used for cloning the ORFs and shows their SEQIDs (column A), their vector names (column B), the promotors they contain for expression of the ORFs (column C), the additional artificial targeting sequence (column D), the adapter sequence (column E), the expression type conferred by the promoter mentioned in column B (column F) and the figure number (column G).**

| **A** | **B** | **C** | **D** | **E** | **F** | **G** |
|---|---|---|---|---|---|---|
| **SeqID** | **Vector Name** | **Promoter Name** | **Target Sequence** | **Adapter Sequence** | **Expression Type** | **Figure** |
| **1** | VC-MME220-1 | Super | | Colic | non targeted constitutive expression in preferentially green tissues | **1a** |
| **2** | VC-MME221-1 | PcUbi | | Colic | non targeted constitutive expression in preferentially green tissues | **2a** |
| **3** | VC-MME354-1 | Super | FNR | Resgen | plastidic targeted constitutive expression in preferentially green tissues | **3a** |
| **5** | VC-MME432-1 | Super | FNR | Colic | plastidic targeted constitutive expression in preferentially green tissues | **4a** |
| **15** | VC-MME489-1p | Super | | Resgen | non targeted constitutive expression in preferentially green tissues | **5a** |
| **16** | pMTX0270p | Super | | Colic | non targeted constitutive expression in preferentially green tissues | **6** |
| **8431** | VC-MME354-1QCZ | Super | FNR | Resgen | plastidic targeted constitutive expression in preferentially green tissues | **3b** |
| **8433** | VC-MME220-1qcz | Super | | Colic | non targeted constitutive expression in preferentially green tissues | **1b** |
| **8434** | VC-MME432-1qcz | Super | FNR | Colic | plastidic targeted constitutive expression in preferentially green tissues | **4b** |
| **8436** | VC-MME221-1qcz | PcUbi | | Colic | non targeted constitutive expression in preferentially green tissues | **2b** |
| **8437** | VC-MME489-1 QCZ | Super | | Resgen | non targeted constitutive expression in preferentially green tissues | **5b** |

### Construction of binary vectors for non-targeted expression of proteins.

"Non-targeted" expression in this context means, that no additional targeting sequence were added to the ORF to be expressed.

For non-targeted expression in preferentially green tissues the following binary vectors used for cloning were VC-MME220-1 SEQ ID NO: **1 (****figure 1 a)** or VC-MME220-1 qcz SEQ ID NO: 8433 (figure 1b)" VC-MME221-1 SEQ ID NO: **2 (****figure 2a****)** or VC-MME221-1qcz SEQ ID NO: 8436 (figure 2b), VC-MME489-1p SEQ ID NO: **15 (****figure 5a****)** or VC-MME489-1QCZ SEQ ID NO: 8437 (figure 5b). In case of VC-MME489-1p and VC-MME489-1QCZ the super promoter (Ni et al,.Plant Journal 7, 661 (1995) sequence can be replaced by the sequence of the enhanced 35S promotor (Comai et al., Plant Mol Biol 15, 373-383 (1990) leading to similar results as shown in the table 1 below.

### Amplification of the targeting sequence of the gene FNR from Spinacia oleracea and construction of vector for plastid-targeted expression in preferential green tissues.

In order to amplify the targeting sequence of the FNR gene from *S*. *oleracea,* genomic DNA was extracted from leaves of 4 weeks old *S*. *oleracea* plants (DNeasy Plant Mini Kit, Qiagen, Hilden). The gDNA was used as the template for a PCR.

To enable cloning of the transit sequence into the vector VC-MME0489-1p and VC-MME489-1QCZ an EcoRI restriction enzyme recognition sequence was added to both the forward and reverse primers, whereas for cloning in the vectors pMTX0270p, VC-MME220-1, VC-MME220-1qcz, VC-MME221-1 and VC-MME221-1qcz a Pmel restriction enzyme recognition sequence was added to the forward primer and a Ncol site was added to the reverse primer.

| | |
|---|---|
| FNR5EcoResgen | ATA GAA TTC GCA TAA ACT TAT CTT CAT AGT TGC C SEQ ID NO: **11** |
| FNR3EcoResgen | ATA GAA TTC AGA GGC GAT CTG GGC CCT SEQ ID NO: **12** |
| FNR5PmeColic | ATA GTT TAA ACG CAT AAA CTT ATC TTC ATA GTT GCC SEQ ID NO: **13** |
| FNR3NcoColic | ATA CCA TGG AAG AGC AAG AGG CGA TCT GGG CCC T SEQ ID NO: **14** |

The resulting sequence SEQ ID NO: **36 ,** amplified from genomic spinach DNA, comprised a 5'UTR (**bp 1-165**), and the coding region (**bp 166-273** and **351-419**). The coding sequence is interrupted by an intronic sequence from bp **274** to bp **350.**

gcataaacttatcttcatagttgccactccaatttgctcctt-
gaatctcctccacccaatacataatccactcctccatcacccacttcactacta-
aatcaaacttaactctgtttttctctctcctcctttcatttcttattcttccaatcatcg-
tactccgccatgaccaccgctgtcaccgccgctgtttctttcccctctaccaaaccac-
ctctctctccgcccgaagctcctccgtcatttcccctgacaaaatcagc-
tacaaaaaggtgattcccaatttcactgtgtttttattaataatttgttatttt-

gatgatgagatgattaatttgggtgctgcaggttcctttgtactacaggaatgtatctg-
caactgggaaaatgggacccatcagggcccagatcgcctct
SEQ ID NO: 36

The PCR fragment derived with the primers FNRSEcoResgen and FNR3EcoResgen was digested with EcoRI and ligated in the vector VC-MME0489-1p or VC-MME489-1QCZ that had also been digested with EcoRI. The correct orientation of the FNR targeting sequence was tested by sequencing. The vector generated in this ligation step was VC-MME354-1 SEQ ID NO: 3 or VC-MME354-1QCZ SEQ ID NO: **8431.**

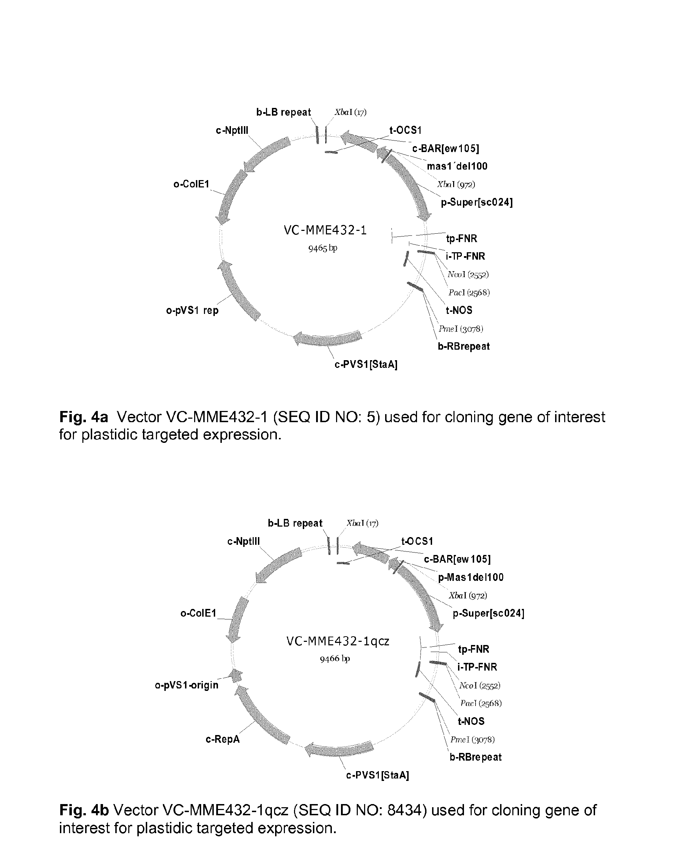

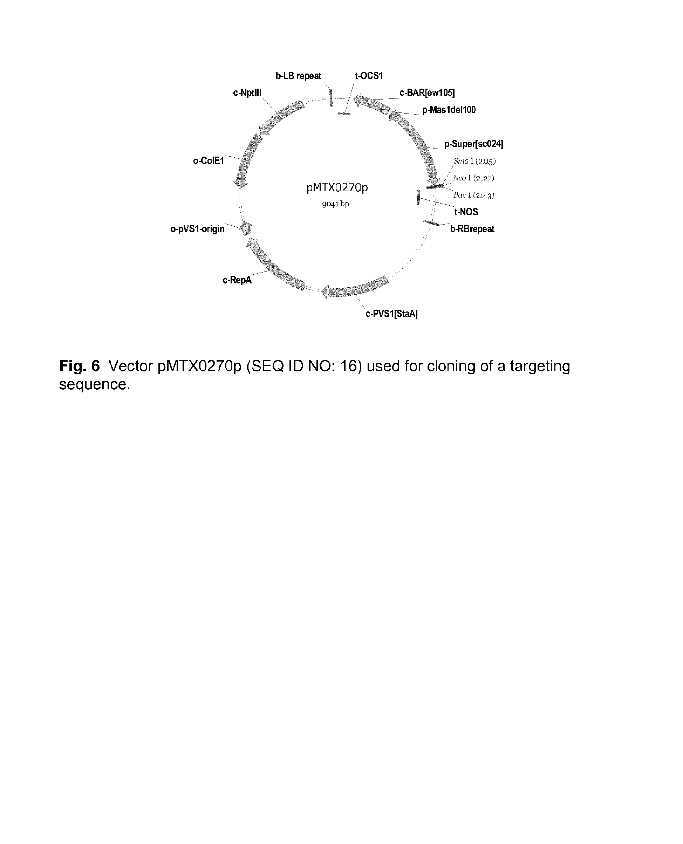

The PCR fragment derived with the primers FNRSPmeColic and FNR3NcoColic was digested with Pmel and Ncol and ligated in the vector pMTX0270p **(****figure 6****)** SEQ ID NO: **16,** VC-MME220-1 or VC-MME220-1qcz that had been digested with Smal and Ncol. The vector generated in this ligation step was VC-MME432-1 SEQ ID NO: **5 (figure 4a)** or VC-MME432-1 qcz SEQ ID NO: 8434 (figure 4b).

For plastidic-targeted constitutive expression in preferentially green tissues an artifical promoter A(ocs)3AmasPmas promoter (Super promoter)) (Ni et al,. Plant Journal 7, 661 (1995), WO 95/14098) was used in context of the vector VC-MME354-1 orVC-MME354-1QCZ for ORFs from *Saccharomyces cerevisiae* and in context of the vector VC-MME432-1 or VC-MME432-1qcz for ORFs from *Escherichia coli,* resulting in each case in an "in-frame" fusion of the FNR targeting sequence with the ORFs.

Other useful binary vectors are known to the skilled worker; an overview of binary vectors and their use can be found in Hellens R., Mullineaux P. and Klee H., (Trends in Plant Science, 5 (10), 446 (2000)). Such vectors have to be equally equipped with appropriate promoters and targeting sequences.

### Cloning of inventive sequences as shown in table I, column 5 and 7 in the different expression vectors.

* Sequences as shown in table I, column 5, marked with asterisk * reflect the respective sequence derived from public data bases information.

For cloning the ORF of SEQ ID NO: 6823, from *S*. *cerevisiae* into vectors containing the *Resgen* adaptor sequence the respective vector DNA was treated with the restriction enzyme Ncol. For cloning of ORFs from *E. coli* or *Synechocystis sp.* the vector DNA was treated with the restriction enzymes Pacl and Ncol following the standard protocol (MBI Fermentas). In all cases the reaction was stopped by inactivation at 70°C for 20 minutes and purified over QIAquick or NucleoSpin Extract II columns following the standard protocol (Qiagen or Macherey-Nagel).

Then the PCR-product representing the amplified ORF with the respective adapter sequences and the vector DNA were treated with T4 DNA polymerase according to the standard protocol (MBI Fermentas) to produce single stranded overhangs with the parameters 1 unit T4 DNA polymerase at 37°C for 2-10 minutes for the vector and 1-2 u T4 DNA polymerase at 15-17°C for 10-60 minutes for the PCR product representing SEQ ID NO: 6823.

The reaction was stopped by addition of high-salt buffer and purified over QIAquick or NucleoSpin Extract II columns following the standard protocol (Qiagen or Macherey-Nagel).

According to this example the skilled person is able to clone all sequences disclosed in table I, preferably column 5.

Approximately 30-60 ng of prepared vector and a defined amount of prepared amplificate were mixed and hybridized at 65°C for 15 minutes followed by 37°C 0.1 °C / 1 seconds, followed by 37°C 10 minutes, followed by 0.1 °C/1 seconds, then 4-10 °C.

The ligated constructs were transformed in the same reaction vessel by addition of competent *E. coli cells* (strain DH5alpha) and incubation for 20 minutes at 1°C followed by a heat shock for 90 seconds at 42°C and cooling to 1-4°C. Then, complete medium (SOC) was added and the mixture was incubated for 45 minutes at 37°C. The entire mixture was subsequently plated onto an agar plate with 0.05 mg/ml kanamycine and incubated overnight at 37°C.

The outcome of the cloning step was verified by amplification with the aid of primers which bind upstream and downstream of the integration site, thus allowing the amplification of the insertion. The amplifications were carried as described in the protocol of *Taq* DNA polymerase (Gibco-BRL).

The amplification cycles were as follows: 1 cycle of 1-5 minutes at 94°C, followed by 35 cycles of in each case 15-60 seconds at 94°C, 15-60 seconds at 50-66°C and 5-15 minutes at 72°C, followed by 1 cycle of 10 minutes at 72°C, then 4-16°C.

Several colonies were checked, but only one colony for which a PCR product of the expected size was detected was used in the following steps.

A portion of this positive colony was transferred into a reaction vessel filled with complete medium (LB) supplemented with kanamycin and incubated overnight at 37°C.

The plasmid preparation was carried out as specified in the Qiaprep or NucleoSpin Multi-96 Plus standard protocol (Qiagen or Macherey-Nagel).

### Generation of transgenic plants which express SEQ ID NO: 6823 or any other sequence disclosed in table I, preferably column 5

* Sequences as shown in table I, column 5, marked with asterisk * reflect the respective sequence derived from public data bases information.

1-5 ng of the plasmid DNA isolated was transformed by electroporation or transformation into competent cells of *Agrobacterium tumefaciens, of* strain GV 3101 pMP90 (Koncz and Schell, Mol. Gen. Gent. 204, 383-396, 1986). Thereafter, complete medium (YEP) was added and the mixture was transferred into a fresh reaction vessel for 3 hours at 28°C. Thereafter, all of the reaction mixture was plated onto YEP agar plates supplemented with the respective antibiotics, e.g. rifampicine (0.1 mg/ml), gentamycine (0.025 mg/ml and kanamycine (0.05 mg/ml) and incubated for 48 hours at 28°C.

The agrobacteria that contains the plasmid construct were then used for the transformation of plants.

A colony was picked from the agar plate with the aid of a pipette tip and taken up in 3 ml of liquid TB medium, which also contained suitable antibiotics as described above. The preculture was grown for 48 hours at 28°C and 120 rpm.

400 ml of LB medium containing the same antibiotics as above were used for the main culture. The preculture was transferred into the main culture. It was grown for 18 hours at 28°C and 120 rpm. After centrifugation at 4 000 rpm, the pellet was resuspended in infiltration medium (MS medium, 10% sucrose).

In order to grow the plants for the transformation, dishes (Piki Saat 80, green, provided with a screen bottom, 30 x 20 x 4.5 cm, from Wiesauplast, Kunststofftechnik, Germany) were half-filled with a GS 90 substrate (standard soil, Werkverband E.V., Germany). The dishes were watered overnight with 0.05% Proplant solution (Chimac-Apriphar, Belgium). *Arabidopsis thaliana* C24 seeds (Nottingham Arabidopsis Stock Centre, UK ; NASC Stock N906) were scattered over the dish, approximately 1 000 seeds per dish. The dishes were covered with a hood and placed in the stratification facility (8 h, 110 µmol/m²/s⁻¹, 22°C; 16 h, dark, 6°C). After 5 days, the dishes were placed into the short-day controlled environment chamber (8 h 130 µmol/m²/s⁻¹, 22°C; 16 h, dark 20°C), where they remained for approximately 10 days until the first true leaves had formed.

The seedlings were transferred into pots containing the same substrate (Teku pots, 7 cm, LC series, manufactured by Pöppelmann GmbH & Co, Germany). Five plants were pricked out into each pot. The pots were then returned into the short-day controlled environment chamber for the plant to continue growing.

After 10 days, the plants were transferred into the greenhouse cabinet (supplementary illumination, 16 h, 340 µE, 22°C; 8 h, dark, 20°C), where they were allowed to grow for further 17 days.

For the transformation, 6-week-old Arabidopsis plants, which had just started flowering were immersed for 10 seconds into the above-described agrobacterial suspension which had previously been treated with 10 µl Silwett L77 (Crompton S.A., Osi Specialties, Switzerland). The method in question is described in Clough and Bent, 1998 (Clough, JC and Bent, AF. 1998 Floral dip: a simplified method for Agrobacterium-mediated transformation of Arabidopsis thaliana, Plant J. 16:735-743.

The plants were subsequently placed for 18 hours into a humid chamber. Thereafter, the pots were returned to the greenhouse for the plants to continue growing. The plants remained in the greenhouse for another 10 weeks until the seeds were ready for harvesting.

Depending on the resistance marker used for the selection of the transformed plants the harvested seeds were planted in the greenhouse and subjected to a spray selection or else first sterilized and then grown on agar plates supplemented with the respective selection agent. Since the vector contained the bar gene as the resistance marker" plantlets were sprayed four times at an interval of 2 to 3 days with 0.02 % BASTA® and transformed plants were allowed to set seeds.

The seeds of the transgenic *A. thaliana* plants were stored in the freezer (at -20°C).

Transgenic A. thaliana plants were grown individually in pots containing a 4:1 (v/v) mixture of soil and quartz sand in a York growth chamber. Standard growth conditions were: photoperiod of 16 h light and 8 h dark, 20 °C, 60% relative humidity, and a photon flux density of 150 µE. To induce germination, sown seeds were kept at 4°C, in the dark, for 3 days. Subsequently conditions were changed for 3 d to 20°C/6°C day/night temperature with a 16/8h day-night cycle at 150 µE/m²s. Standard growth conditions were: photoperiod of 16 h light and 8 h dark, 20 °C, 60% relative humidity, and a photon flux density of 200 µE. Plants were watered daily until they were approximately 3 weeks old at which time drought was imposed by withholding water. After approximately 12 days of withholding water, most plants showed visual symptoms of injury, such as wilting and leaf browning, whereas tolerant or resistant plants were identified as being visually turgid and healthy green in color. Plants were scored for symptoms of drought symptoms and biomass production comparison to wild type and neighboring plants for 5 - 6 days in succession.

Three successive experiments were conducted. In the first experiment, one individual of each transformed line was tested.

In the second experiment, the lines that had been scored as drought tolerant or resistant in the first experiment, i.e. survived longer than the wild type control and showed increaed biomass production in comparison to wild type and neighbouring plants, were put through a confirmation screen according to the same experimental procedures. In this experiment, max. 10 plants of each tolerant or resistant line were grown, treated and scored as before.

In the first two experiments, drought resistance or tolerance and biomass production was measured compared to neighboring and wild type plants.

In the third experiment (table 1), 15 replicates of each confirmed tolerant line, i.e. those that had been scored as tolerant or resistant in the second experiment, were grown, treated and scored as before.

In the third experiment, after approximately 10 days of drought, the control (non-transformed Arabidopsis thaliana) in the test showed extreme visual symptoms of stress including necrosis and cell death. Several transformed plants retained viability as shown by their turgid appearance and maintenance of green color.

Table 1: Duration of survival and biomass production of transformed Arabidopsis thaliana after imposition of drought stress on 3-week-old plants. Drought tolerance and biomass production was measured visually at daily intervals. Average performance is the average of transgenic plants that survived longer than the wild type control. Maximum performance is the longest period that any single transformed plant survived longer than the wild type control. Average biomass is the average of days of transgenic plants plants increase in biomass in comparison to the wild type control and neighbouring plants. Maximum biomass is the longest period that any single transformed plant showed increase in biomass in comparison to the wild type control and neighbouring plants.

**Table 1.**

| SeqID | Target | Locus | Average Performance | Maximum Performance | Average Biomass | Maximum Biomass |
|---|---|---|---|---|---|---|
| 38 | non-targeted | B0081 | 3.4 | 6 | 1.1 | 3 |
| 54 | non-targeted | B0445 | 3 | 5 | 0.9 | 4 |
| 70 | non-targeted | B0482 | 2.8 | 5 | 0.6 | 3 |
| 89 | non-targeted | B0607 | 2.9 | 5 | 0.9 | 4 |
| 143 * | non-targeted | B0629 | 2.8 | 5 | 0.8 | 4 |
| 162 | non-targeted | B0631 | 3.4 | 6 | 1.2 | 5 |
| 213 | non-targeted | B0697 | 2.1 | 3 | 1.8 | 3 |
| 358 | non-targeted | B0753 | 3.3 | 5 | 1.6 | 4 |
| 367 | non-targeted | B0813 | 3 | 5 | 1.5 | 3 |
| 420 * | non-targeted | B0845 | 2.2 | 5 | 1 | 4 |
| 455 | non-targeted | B0866 | 3 | 5 | 0.8 | 5 |
| 535 * | non-targeted | B0963 | 4.4 | 5 | 0.5 | 2 |
| 618 | non-targeted | B0975 | 2.6 | 4 | 1.2 | 4 |
| 671 * | non-targeted | B1007 | 3.3 | 6 | 0.6 | 2 |
| 764 | non-targeted | B1052 | 3 | 5 | 0.4 | 2 |
| 768 | plastidic | B1091 | 3.4 | 5 | 0.3 | 3 |
| 907 | non-targeted | B1161 | 2.9 | 4 | 2 | 4 |
| 927 | non-targeted | B1186 | 4 | 6 | 0.8 | 4 |
| 1009 | plastidic | B1291 | 3.4 | 5 | 1.2 | 3 |
| 1154 | plastidic | B1294 | 2.3 | 5 | 1 | 4 |
| 1308 | non-targeted | B1423 | 3.1 | 6 | 0.1 | 1 |
| 1368 * | non-targeted | B1597 | 3.7 | 5 | 0.1 | 1 |
| 1374 | non-targeted | B1605 | 2.8 | 5 | 0.9 | 3 |
| 1507 | non-targeted | B1704 | 4.2 | 5 | 0.6 | 2 |
| 1953 | plastidic | B1736 | 4.1 | 5 | 0.8 | 2 |
| 2156 * | non-targeted | B1798 | 3.4 | 5 | 1 | 4 |
| 2195 | non-targeted | B1878 | 3.4 | 5 | 1.3 | 3 |
| 2219 * | plastidic | B1901 | 2.8 | 4 | 1.8 | 3 |
| 2277 | plastidic | B1912 | 2.5 | 4 | 1.6 | 3 |
| 2470 * | non-targeted | B2027 | 2.6 | 4 | 0.3 | 2 |
| 2493 | non-targeted | B2039 | 2.1 | 4 | 0.1 | 1 |
| 2627 | non-targeted | B2075 | 2.5 | 4 | 0.5 | 3 |
| 2858 | plastidic | B2153 | 2.5 | 4 | 1.5 | 4 |
| 2942 | non-targeted | B2194 | 3.4 | 6 | 0.7 | 3 |
| 2965 * | non-targeted | B2226 | 2.9 | 4 | 1.6 | 4 |
| 2981 | plastidic | B2309 | 1.9 | 3 | 0.9 | 3 |
| 3130 * | non-targeted | B2469 | 2.2 | 5 | 0.9 | 3 |
| 3216 | non-targeted | B2475 | 2.9 | 5 | 0.3 | 2 |
| 3335 | non-targeted | B2482 | 1.9 | 3 | 0.6 | 2 |
| 3401 | non-targeted | B2541 | 2.9 | 5 | 0.6 | 2 |
| 3590 | plastidic | B2559 | 1.6 | 3 | 1.4 | 3 |
| 3831 | non-targeted | B2605 | 1.6 | 4 | 0.7 | 2 |
| 3857 | non-targeted | B2630 | 1.8 | 3 | 0.6 | 2 |
| 3861 | plastidic | B2678 | 4.3 | 5 | 0.1 | 1 |
| 4022 * | plastidic | B2715 | 4 | 5 | 0.4 | 2 |
| 4059 | non-targeted | B2776 | 2.9 | 5 | 0.4 | 2 |
| 4076 | non-targeted | B2791 | 3.2 | 6 | 0.7 | 3 |
| 4157 | non-targeted | B2912 | 3 | 5 | 1.7 | 3 |
| 4260 * | plastidic | B2965 | 3.8 | 5 | 0.7 | 3 |
| 4350 | plastidic | B2987 | 2.9 | 4 | 1.3 | 4 |
| 4350 | non-targeted | B2987 | 2.7 | 4 | 0.1 | 0.1 |
| 4459 | plastidic | B3093 | 2.8 | 5 | 1 | 3 |
| 4505 | plastidic | B3363 | 3.7 | 4 | 0.1 | 0.1 |
| 4640 | plastidic | B3429 | 2.8 | 4 | 0.6 | 2 |
| 4806 | plastidic | B3568 | 2.7 | 4 | 0.8 | 3 |
| 5124 | plastidic | B3616 | 2.5 | 5 | 1.1 | 5 |
| 5124 | non-targeted | B3616 | 2.8 | 5 | 1.3 | 3 |
| 5417 | non-targeted | B3812 | 3 | 5 | 0.9 | 3 |
| 5495 * | non-targeted | B3899 | 2.5 | 4 | 0.8 | 3 |
| 5585 | plastidic | B3929 | 2.3 | 4 | 0.7 | 3 |
| 5800 | non-targeted | B3938 | 2.3 | 3 | 1.1 | 3 |
| 5850 | non-targeted | B3974 | 2.9 | 5 | 1.4 | 2 |
| 5992 | non-targeted | B3989 | 2.8 | 5 | 0.7 | 2 |
| 5999 | non-targeted | B4029 | 3.2 | 5 | 0.5 | 3 |
| 6056 * | plastidic | B4139 | 3.1 | 5 | 1.6 | 4 |
| 6500 * | non-targeted | B4390 | 3.5 | 6 | 0.8 | 3 |
| 6542 | non-targeted | SII0290 | 2.5 | 4 | 0.9 | 3 |
| 6823 | non-targeted | YAL049C | 3.5 | 5 | 0.7 | 2 |
| 6870 | non-targeted | YCR059C | 3.5 | 5 | 1 | 3 |
| 6910 | plastidic | YDR035W | 2.7 | 4 | 1.3 | 3 |
| 7261 | non-targeted | YEL005C | 3.6 | 6 | 1.1 | 4 |
| 7265 * | non-targeted | YER112W | 3.1 | 4 | 0.8 | 2 |
| 7301 | non-targeted | YER156C | 2.2 | 4 | 0.1 | 0.1 |
| 7384 | non-targeted | YER173W | 4.3 | 5 | 0.2 | 1 |
| 7407 * | non-targeted | YGL045W | 3.3 | 4 | 1.2 | 4 |
| 7429 | non-targeted | YGL189C | 2.3 | 4 | 0.1 | 0.1 |
| 7558 | non-targeted | YNR015W | 4.5 | 7 | 0.1 | 0.1 |
| 7606 | non-targeted | YOR024W | 4.8 | 5 | 0.6 | 3 |
| 7610 * | non-targeted | YOR168W | 3.1 | 5 | 0.6 | 2 |
| 7685 | non-targeted | YPL151C | 3.9 | 5 | 0.1 | 0.1 |
| 1201 * | plastidic | B1297 | 2.1 | 3 | 1.1 | 2 |
| 7741 | non-targeted | B0970 | 3.2 | 5 | 0.2 | 2 |
| 7850 | non-targeted | B1829 | 2.4 | 6 | 1 | 3 |
| 7971 * | non-targeted | B2664 | 3.9 | 5 | 0.4 | 2 |
| 8021 | non-targeted | B2796 | 2.9 | 5 | 0.7 | 4 |
| 8177 | non-targeted | YER174C | 4.5 | 5 | 0.1 | 0.1 |
| 8272 | non-targeted | YFR042W | 3.7 | 5 | 1 | 4 |
| 8288 | non-targeted | YKR057W | 3.3 | 5 | 1.9 | 4 |
| 8438 | non-targeted | B0629_2 | 2.8 | 5 | 0.8 | 4 |
| 8630 | non-targeted | B1007_2 | 3.3 | 6 | 0.6 | 2 |
| 9268 | plastidic | B2715_2 | 4 | 5 | 0.4 | 2 |
| 9444 | non-targeted | B3899_2 | 2.5 | 4 | 0.8 | 3 |
| 9824 | non-targeted | B4390_2 | 3.5 | 6 | 0.8 | 3 |
| 9905 | non-targeted | YGL045W_2 | 3.3 | 4 | 1.2 | 4 |
| 9193 | non-targeted | B2664_2 | 3.9 | 5 | 0.4 | 2 |
| 8497 | non-targeted | B0963_2 | 4.4 | 5 | 0.5 | 2 |
| 8742 | plastidic | B1297_2 | 2.1 | 3 | 1.1 | 2 |
| 8891 | non-targeted | B1597_2 | 3.7 | 5 | 0.1 | 1 |
| 9031 | non-targeted | B2027_2 | 2.6 | 4 | 0.3 | 2 |
| 9315 | plastidic | B2965_2 | 3.8 | 5 | 0.7 | 3 |
| 9529 | plastidic | B4139_2 | 3.1 | 5 | 1.6 | 4 |
| 8462 | non-targeted | B0845_2 | 2.2 | 5 | 1 | 4 |
| 8973 | plastidic | B1901_2 | 2.8 | 4 | 1.8 | 3 |
| 9883 | non-targeted | YER112W_2 | 3.1 | 4 | 0.8 | 2 |
| 8934 | non-targeted | B1798_2 | 3.4 | 5 | 1 | 4 |
| 9093 | non-targeted | B2226_2 | 2.9 | 4 | 1.6 | 4 |
| 9109 | non-targeted | B2469_2 | 2.2 | 5 | 0.9 | 3 |
| 9931 | non-targeted | YOR168W_2 | 3.1 | 5 | 0.6 | 2 |
| 10096 | non-targeted | B4321 | 2.5 | 4 | 0.8 | 3 |

Sequences as shown in table 1, column 1, marked with asterisk * reflect the respective sequence derived from public data bases information.

### Example 2

### Engineering stress-tolerant Arabidopsis plants by over-expressing stress related protein encoding genes from Saccharomyces cereviesae or E. coli or Synechocystis sp. using stress-inducible and tissue-specific promoters.

Transgenic Arabidopsis plants are created as in example 1 to express the stress related protein encoding transgenes under the control of either a tissue-specific or stress-inducible promoter.

T2 generation plants are produced and treated with drought stress in two experiments. The plants are deprived of water until the plant and soil were desiccated. Biomass production is determinedat an equivalent degree of drought stress, tolerant plants produced more biomass than non-transgenic control plants.

### Example 3

### Over-expression of stress related genes from Saccharomyces cerevisiae or E. coli or Synechocystis sp. provides tolerance of multiple abiotic stresses.

Plants that exhibit tolerance of one abiotic stress often exhibit tolerance of another environmental stress. This phenomenon of cross-tolerance is not understood at a mechanistic level (McKersie and Leshem, 1994). Nonetheless, it is reasonable to expect that plants exhibiting enhanced drought tolerance due to the expression of a transgene might also exhibit tolerance of cold or salt and other abiotic stresses. In support of this hypothesis, the expression of several genes are up or down-regulated by multiple abiotic stress factors including cold, salt, osmoticum, ABA, etc (e.g. Hong et al. (1992) Developmental and organ-specific expression of an ABA- and stress-induced protein in barley. Plant Mol Biol 18: 663-674; Jagendorf and Takabe (2001) Inducers of glycinebetaine synthesis in barley. Plant Physiol 127: 1827-1835); Mizoguchi et al. (1996) A gene encoding a mitogen-activated protein kinase is induced simultaneously with genes for a mitogen-activated protein kinase and an S6 ribosomal protein kinase by touch, cold, and water stress in Arabidopsis thaliana. Proc Natl Acad Sci U S A 93: 765-769; Zhu (2001) Cell signaling under salt, water and cold stresses. Curr Opin Plant Biol 4: 401-406).

To determine salt tolerance, seeds of Arabidopsis thaliana are sterilized (100% bleach, 0.1% TritonX for five minutes two times and rinsed five times with ddH2O). Seeds were plated on non-selection media (1/2 MS, 0.6% phytagar, 0.5g/L MES, 1% sucrose, 2 µg/ml benamyl). Seeds are allowed to germinate for approximately ten days. At the 4-5 leaf stage, transgenic plants were potted into 5.5 cm diameter pots and allowed to grow (22 °C, continuous light) for approximately seven days, watering as needed. To begin the assay, two liters of 100 mM NaCl and 1/8 MS are added to the tray under the pots. To the tray containing the control plants, three liters of 1/8 MS are added. The concentrations of NaCl supplementation are increased stepwise by 50 mM every 4 days up to 200 mM. After the salt treatment with 200 mM, fresh and survival and biomass production of the plants is determined.

To determine cold tolerance, seeds of the transgenic and cold lines are germinated and grown for approximately 10 days to the 4-5 leaf stage as above. The plants are then transferred to cold temperatures (5 °C) and can be grown through the flowering and seed set stages of development. Photosynthesis can be measured using chlorophyll fluorescence as an indicator of photosynthetic fitness and integrity of the photosystems. Survival and plant biomass production as an indicator for seed yield is determined.

Plants that have tolerance to salinity or cold have higher survival rates and biomass production including seed yieldand dry matter production than susceptible plants.

### Example 4

### Engineering stress-tolerant alfalfa plants by over-expressing stress related genes from Saccharomyces cerevisiae or E. coli or Synechocystis sp.

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) is selected for use in tissue culture (Walker et al., 1978 Am J Bot 65:654-659).

Petiole explants are cocultivated with an overnight culture of Agrobacterium tumefaciens C58C1 pMP90 (McKersie et al., 1999 Plant Physiol 119: 839-847) or LBA4404 containing a binary vector. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene that provides constitutive, developmental, tissue or environmental regulation of gene transcription. In this example, the 34S promoter (GenBank Accession numbers M59930 and X16673) is used to provide constitutive expression of the trait gene.

The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings are transplanted into pots and grown in a greenhouse.

The T0 transgenic plants are propagated by node cuttings and rooted in Turface growth medium. The plants are defoliated and grown to a height of about 10 cm (approximately 2 weeks after defoliation). The plants are then subjected to drought stress in two experiments.

For the drought experiment, the seedlings receive no water for a period up to 3 weeks at which time the plant and soil are desiccated and survival and biomass production of the shoots is determined. At an equivalent degree of drought stress, tolerant plants are able to resume normal growth whereas susceptible plants die or suffer significant injury resulting in loss of biomass production.

Tolerance of salinity and cold are measured using methods as described in example 3. Plants that have tolerance to salinity or cold have higher survival rates and biomass production including seed yield, photosynthesis and dry matter production than susceptible plants.

### Example 5

### Engineering stress-tolerant ryegrass plants by over-expressing stress related genes from Saccharomyces cerevisiae or E. coli or Synechocystis sp.

Seeds of several different ryegrass varieties may be used as explant sources for transformation, including the commercial variety Gunne available from Svalof Weibull seed company or the variety Affinity. Seeds are surface-sterilized sequentially with 1% Tween-20 for 1 minute, 100 % bleach for 60 minutes, 3 rinses with 5 minutes each with de-ionized and distilled H2O, and then germinated for 3-4 days on moist, sterile filter paper in the dark. Seedlings are further sterilized for 1 minute with 1% Tween-20, 5 minutes with 75% bleach, and rinsed 3 times with ddH2O, 5 min each.

Surface-sterilized seeds are placed on the callus induction medium containing Murashige and Skoog basal salts and vitamins, 20 g/l sucrose, 150 mg/l asparagine, 500 mg/l casein hydrolysate, 3 g/l Phytagel, 10 mg/l BAP, and 5 mg/l dicamba. Plates are incubated in the dark at 25C for 4 weeks for seed germination and embryogenic callus induction.

After 4 weeks on the callus induction medium, the shoots and roots of the seedlings are trimmed away, the callus is transferred to fresh media, maintained in culture for another 4 weeks, and then transferred to MSO medium in light for 2 weeks. Several pieces of callus (11-17 weeks old) are either strained through a 10 mesh sieve and put onto callus induction medium, or cultured in 100 ml of liquid ryegrass callus induction media (same medium as for callus induction with agar) in a 250 ml flask. The flask is wrapped in foil and shaken at 175 rpm in the dark at 23 C for 1 week. Sieving the liquid culture with a 40-mesh sieve collected the cells. The fraction collected on the sieve is plated and cultured on solid ryegrass callus induction medium for 1 week in the dark at 25°C. The callus is then transferred to and cultured on MS medium containing 1% sucrose for 2 weeks.

Transformation can be accomplished with either Agrobacterium of with particle bombardment methods. An expression vector is created containing a constitutive plant promoter and the cDNA of the gene in a pUC vector. The plasmid DNA is prepared from E. coli cells using with Qiagen kit according to manufacturer's instruction. Approximately 2 g of embryogenic callus is spread in the center of a sterile filter paper in a Petri dish. An aliquot of liquid MSO with 10 g/l sucrose is added to the filter paper. Gold particles (1.0 µm in size) are coated with plasmid DNA according to method of Sanford et al., 1993 and delivered to the embryogenic callus with the following parameters: 500 µg particles and 2 µg DNA per shot, 1300 psi and a target distance of 8.5 cm from stopping plate to plate of callus and 1 shot per plate of callus.

After the bombardment, calli are transferred back to the fresh callus development medium and maintained in the dark at room temperature for a 1-week period. The callus is then transferred to growth conditions in the light at 25 °C to initiate embryo differentiation with the appropriate selection agent, e.g. 250 nM Arsenal, 5 mg/l PPT or 50 mg/L kanamycin. Shoots resistant to the selection agent appeare and once rotted are transferred to soil.

Samples of the primary transgenic plants (TO) are analyzed by PCR to confirm the presence of T-DNA. These results are confirmed by Southern hybridization in which DNA is electrophoresed on a 1% agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

Transgenic T0 ryegrass plants are propagated vegetatively by excising tillers. The transplanted tillers are maintained in the greenhouse for 2 months until well established. The shoots are defoliated and allowed to grow for 2 weeks.

For the drought experiment, the seedlings receive no water for a period up to 3 weeks at which time the plant and soil are desiccated and survival and biomass production of the shoots is determined. At an equivalent degree of drought stress, tolerant plants are able to resume normal growth whereas susceptible plants die or suffer significant injury resulting in loss of biomass production.

### Example 6

### Engineering stress-tolerant soybean plants by over-expressing stress related genes from Saccharomyces cerevisiae or E. coli or Synechocystis sp.

Soybean is transformed according to the following modification of the method described in the Texas A&M patent US 5,164,310. Several commercial soybean varieties are amenable to transformation by this method. The cultivar Jack (available from the Illinois Seed Foundation) is a commonly used for transformation. Seeds are sterilized by immersion in 70% (v/v) ethanol for 6 min and in 25 % commercial bleach (NaOCl) supplemented with 0.1 % (v/v) Tween for 20 min, followed by rinsing 4 times with sterile double distilled water. Seven-day seedlings are propagated by removing the radicle, hypocotyl and one cotyledon from each seedling. Then, the epicotyl with one cotyledon is transferred to fresh germination media in petri dishes and incubated at 25 °C under a 16-hr photoperiod (approx. 100 µE-m-2s-1) for three weeks. Axillary nodes (approx. 4 mm in length) were cut from 3 - 4 week-old plants. Axillary nodes are excised and incubated in Agrobacterium LBA4404 culture.

Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription. In this example, the 34S promoter (GenBank Accession numbers M59930 and X16673) can be used to provide constitutive expression of the trait gene.

After the co-cultivation treatment, the explants are washed and transferred to selection media supplemented with 500 mg/L timentin. Shoots are excised and placed on a shoot elongation medium. Shoots longer than 1 cm are placed on rooting medium for two to four weeks prior to transplanting to soil.

The primary transgenic plants (TO) are analyzed by PCR to confirm the presence of T-DNA. These results are confirmed by Southern hybridization in which DNA is electrophoresed on a 1 % agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

Tolerant plants have higher seed yields.

Tolerance of drought, salinity and cold are measured using methods as described in example3. Tolerant plants have higher survival rates and biomass production including seed yield, photosynthesis and dry matter production than susceptible plants.

### Example 7

### Engineering stress-tolerant Rapeseed/Canola plants by over-expressing stress related genes from Saccharomyces cerevisiae or E. coli or Synechocystis sp.

Cotyledonary petioles and hypocotyls of 5-6 day-old young seedlings are used as explants for tissue culture and transformed according to Babic et al.(1998, Plant Cell Rep 17: 183-188). The commercial cultivar Westar (Agriculture Canada) is the standard variety used for transformation, but other varieties can be used.

Agrobacterium tumefaciens LBA4404 containing a binary vector can be used for canola transformation. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription. In this example, the 34S promoter (GenBank Accession numbers M59930 and X16673) can be used to provide constitutive expression of the trait gene.

Canola seeds are surface-sterilized in 70% ethanol for 2 min., and then in 30% Clorox with a drop of Tween-20 for 10 min, followed by three rinses with sterilized distilled water. Seeds are then germinated in vitro 5 days on half strength MS medium without hormones, 1% sucrose, 0.7% Phytagar at 23oC, 16 hr. light. The cotyledon petiole explants with the cotyledon attached are excised from the in vitro seedlings, and inoculated with Agrobacterium by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots were 5 - 10 mm in length, they are cut and transferred to shoot elongation medium (MSBAP-0.5, containing 0.5 mg/l BAP). Shoots of about 2 cm in length are transferred to the rooting medium (MS0) for root induction.

Samples of the primary transgenic plants (TO) are analyzed by PCR to confirm the presence of T-DNA. These results are confirmed by Southern hybridization in which DNA is electrophoresed on a 1 % agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

The transgenic plants are then evaluated for their improved stress tolerance according to the method described in Example 3. Plants that have higher survival rates and biomass production including seed yield, photosynthesis and dry matter production than susceptible plants

Tolerance of drought, salinity and cold are measured using methods as described in the previous example 3. Tolerant plants have higher survival rates and biomass production including seed yield, photosynthesis and dry matter production than susceptible plants.

### Example 8

### Engineering stress-tolerant corn plants by over-expressing stress related genes from Saccharomyces cerevisiae or E. coli or Synechocystis sp.

Transformation of maize (Zea Mays L.) is performed with a modification of the method described by Ishida et al. (1996. Nature Biotech 14745-50). Transformation is genotype-dependent in corn and only specific genotypes are amenable to transformation and regeneration. The inbred line A188 (University of Minnesota) or hybrids with A188 as a parent are good sources of donor material for transformation (Fromm et al. 1990 Biotech 8:833-839), but other genotypes can be used successfully as well. Ears are harvested from corn plants at approximately 11 days after pollination (DAP) when the length of immature embryos is about 1 to 1.2 mm. Immature embryos are co-cultivated with Agrobacterium tumefaciens that carry "super binary" vectors and transgenic plants are recovered through organogenesis. The super binary vector system of Japan Tobacco is described in WO patents WO94/00977 and WO95/06722. Vectors were constructed as described. Various selection marker genes can be used including the maize gene encoding a mutated acetohydroxy acid synthase (AHAS) enzyme (US patent 6025541). Similarly, various promoters can be used to regulate the trait gene to provide constitutive, developmental, tissue or environmental regulation of gene transcription. In this example, the 34S promoter (GenBank Accession numbers M59930 and X16673) was used to provide constitutive expression of the trait gene.

Excised embryos are grown on callus induction medium, then maize regeneration medium, containing imidazolinone as a selection agent. The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the imidazolinone herbicides and which are PCR positive for the transgenes.

The T1 transgenic plants are then evaluated for their improved stress tolerance according to the method described in Example 3. The T1 generation of single locus insertions of the T-DNA will segregate for the transgene in a 3:1 ratio. Those progeny containing one or two copies of the transgene are tolerant of the imidazolinone herbicide, and exhibit greater tolerance of drought stress than those progeny lacking the transgenes. Tolerant plants have higher survival rates and biomass production including seed yield, photosynthesis and dry matter production than susceptible plants.Homozygous T2 plants exhibited similar phenotypes. Hybrid plants (F1 progeny) of homozygous transgenic plants and non-transgenic plants also exhibited increased environmental stress tolerance.

Tolerance of salinity and cold are measured using methods as described in the previous example 3. Tolerant plants have higher survival rates and biomass production including seed yield, photosynthesis and dry matter production than susceptible plants.

### Example 9

### Engineering stress-tolerant wheat plants by over-expressing stress related genes from Saccharomyces cerevisiae or E. colior Synechocystis sp.

Transformation of wheat is performed with the method described by Ishida et al. (1996 Nature Biotech. 14745-50). The cultivar Bobwhite (available from CYMMIT, Mexico) is commonly used in transformation. Immature embryos are co-cultivated with Agrobacterium tumefaciens that carry "super binary" vectors, and transgenic plants are recovered through organogenesis. The super binary vector system of Japan Tobacco is described in WO patents WO94/00977 and WO95/06722. Vectors were constructed as described. Various selection marker genes can be used including the maize gene encoding a mutated acetohydroxy acid synthase (AHAS) enzyme (US patent 6025541). Similarly, various promoters can be used to regulate the trait gene to provide constitutive, developmental, tissue or environmental regulation of gene transcription. In this example, the 34S promoter (GenBank Accession numbers M59930 and X16673) was used to provide constitutive expression of the trait gene.

After incubation with Agrobacterium, the embryos are grown on callus induction medium, then regeneration medium, containing imidazolinone as a selection agent. The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the imidazolinone herbicides and which are PCR positive for the transgenes.

The T1 transgenic plants are then evaluated for their improved stress tolerance according to the method described in the previous example 3. The T1 generation of single locus insertions of the T-DNA will segregate for the transgene in a 3:1 ratio. Those progeny containing one or two copies of the transgene are tolerant of the imidazolinone herbicide, and exhibit greater tolerance of drought stress than those progeny lacking the transgenes. Tolerant plants have higher survival rates and biomass production including seed yield, photosynthesis and dry matter production than susceptible plants. Homozygous T2 plants exhibited similar phenotypes. Tolerance of salinity and cold are measured using methods as described in the previous examples Tolerant plants have higher survival rates and biomass production including seed yield, photosynthesis and dry matter production than susceptible plants.

### Example 10

### Identification of Identical and Heterologous Genes

Gene sequences can be used to identify identical or heterologous genes from cDNA or genomic libraries. Identical genes (e. g. full-length cDNA clones) can be isolated via nucleic acid hybridization using for example cDNA libraries. Depending on the abundance of the gene of interest, 100,000 up to 1,000,000 recombinant bacteriophages are plated and transferred to nylon membranes. After denaturation with alkali, DNA is immobilized on the membrane by e. g. UV cross linking. Hybridization is carried out at high stringency conditions. In aqueous solution, hybridization and washing is performed at an ionic strength of 1 M NaCl and a temperature of 68°C. Hybridization probes are generated by e.g. radioactive (³²P) nick transcription labeling (High Prime, Roche, Mannheim, Germany). Signals are detected by autoradiography.

Partially identical or heterologous genes that are related but not identical can be identified in a manner analogous to the above-described procedure using low stringency hybridization and washing conditions. For aqueous hybridization, the ionic strength is normally kept at 1 M NaCl while the temperature is progressively lowered from 68 to 42°C.

Isolation of gene sequences with homology (or sequence identity/similarity) only in a distinct domain of (for example 10-20 amino acids) can be carried out by using synthetic radio labeled oligonucleotide probes. Radiolabeled oligonucleotides are prepared by phosphorylation of the 5-prime end of two complementary oligonucleotides with T4 polynucleotide kinase. The complementary oligonucleotides are annealed and ligated to form concatemers. The double stranded concatemers are than radiolabeled by, for example, nick transcription. Hybridization is normally performed at low stringency conditions using high oligonucleotide concentrations.

Oligonucleotide hybridization solution:
6 x SSC
0.01 M sodium phosphate
1 mM EDTA (pH 8)
0.5 % SDS
100 µg/ml denatured salmon sperm DNA
0.1 % nonfat dried milk

During hybridization, temperature is lowered stepwise to 5-10°C below the estimated oligonucleotide Tₘ or down to room temperature followed by washing steps and autoradiography. Washing is performed with low stringency such as 3 washing steps using 4 x SSC. Further details are described by Sambrook, J. et al., 1989, "Molecular Cloning: A Laboratory Manual," Cold Spring Harbor Laboratory Press or Ausubel, F.M. et al., 1994, "Current Protocols in Molecular Biology," John Wiley & Sons.

### Example 11

### Identification of Identical Genes by Screening Expression Libraries with Antibodies

c-DNA clones can be used to produce recombinant polypeptide for example in *E. coli* (e.g. Qiagen QIAexpress pQE system). Recombinant polypeptides are then normally affinity purified via Ni-NTA affinity chromatography (Qiagen). Recombinant polypeptides are then used to produce specific antibodies for example by using standard techniques for rabbit immunization. Antibodies are affinity purified using a Ni-NTA column saturated with the recombinant antigen as described by Gu et al., 1994, BioTechniques 17:257-262. The antibody can than be used to screen expression cDNA libraries to identify identical or heterologous genes via an immunological screening (Sambrook, J. et al., 1989, "Molecular Cloning: A Laboratory Manual," Cold Spring Harbor Laboratory Press or Ausubel, F.M. et al., 1994, "Current Protocols in Molecular Biology", John Wiley & Sons).

### Example 12

### In vivo Mutagenesis

*In vivo* mutagenesis of microorganisms can be performed by passage of plasmid (or other vector) DNA through *E. coli* or other microorganisms (e.g. *Bacillus* spp. or yeasts such as *Saccharomyces cerevisiae)* which are impaired in their capabilities to maintain the integrity of their genetic information. Typical mutator strains have mutations in the genes for the DNA repair system (e.g., mutHLS, mutD, mutT, etc.; for reference, see Rupp, W.D., 1996, DNA repair mechanisms, in: Escherichia coli and Salmonella, p. 2277-2294, ASM: Washington.) Such strains are well known to those skilled in the art. The use of such strains is illustrated, for example, in Greener, A. and Callahan, M., 1994, Strategies 7: 32-34. Transfer of mutated DNA molecules into plants is preferably done after selection and testing in microorganisms. Transgenic plants are generated according to various examples within the exemplification of this document.

### Example 13

### Engineering stress-tolerant Arabidopsis plants by over-expressing stress related protein encoding genes for example from Brassica napus, Glycine max, Zea mays or Oryza sativa using stress-inducible and tissue-specific promoters.

Transgenic Arabidopsis plants over-expressing stress related protein encoding genes from *Brassica napus, Glycine max, Zea mays* and *Oryza sativa* for example are created as described in example 1 to express the stress related protein encoding transgenes under the control of either a tissue-specific or stress-inducible promoter. Stress inducible expression is achieved using promoters selected from those listed above in Table VI.

T2 generation plants are produced and treated with drought stress. For the drought experiment, the plants are deprived of water until the plant and soil are desiccatedAt an equivalent degree of drought stress, tolerant plants are able to resume normal growth and produced more biomass than non-transgenic control plants.

Tolerant plants have higher survival rates and biomass production including seed yield, photosynthesis and dry matter production than susceptible plants.

### Example 14

### Over-expression of stress related genes for example from Brassica napus, Glycine max, Zea mays or Oryza sativa for example provides tolerance of multiple abiotic stresses.

Plants that exhibit tolerance of one abiotic stress often exhibit tolerance of another environmental stress. This phenomenon of cross-tolerance is not understood at a mechanistic level (McKersie and Leshem, 1994). Nonetheless, it is reasonable to expect that plants exhibiting enhanced drought tolerance due to the expression of a transgene might also exhibit tolerance of cold, salt, and other abiotic stresses. In support of this hypothesis, the expression of several genes are up or down-regulated by multiple abiotic stress factors including cold, salt, osmoticum, ABA, etc (e.g. Hong et al. (1992) Developmental and organ-specific expression of an ABA- and stress-induced protein in barley. Plant Mol Biol 18: 663-674; Jagendorf and Takabe (2001) Inducers of glycinebetaine synthesis in barley. Plant Physiol 127: 1827-1835); Mizoguchi et al. (1996) A gene encoding a mitogen-activated protein kinase is induced simultaneously with genes for a mitogen-activated protein kinase and an S6 ribosomal protein kinase by touch, cold, and water stress in Arabidopsis thaliana. Proc Natl Acad Sci U S A 93: 765-769; Zhu (2001) Cell signaling under salt, water and cold stresses. Curr Opin Plant Biol 4: 401-406).

Transgenic Arabidopsis plants over-expressing stress related protein encoding genes from *Brassica napus, Glycine max, Zea mays* and *Oryza sativa* for example are created as described in example 1 and tested for tolerance to salt and cold stress.

To determine salt tolerance, seeds of Arabidopsis thaliana are sterilized (incubated in 100% bleach, 0.1 % TritonX100 for five minutes (twice) and rinsed five times with ddH2O). Seeds are plated on non-selective medium (1/2 MS, 0.6% phytagar, 0.5g/L MES, 1% sucrose, 2 µg/ml benamyl). Seeds are allowed to germinate for approximately ten days. At the 4-5 leaf stage, transgenic plants are potted into 5.5cm diameter pots and allowed to grow (22 °C, continuous light) for approximately seven days, watering as needed. To begin the assay, two liters of 100 mM NaCl and 1/8 MS are added to the tray under the pots. To the tray containing the control plants, three liters of 1/8 MS is added. The concentrations of NaCl supplementation are increased stepwise by 50 mM every 4 days up to 200 mM. After the salt treatment with 200 mM, fresh and dry weights of the plants as well as seed yields are determined. Transgenic plants over-epxression stress related protein encoding genes from *Brassica napus, Glycine max,* Zea *mays* and *Oryza sativa for example* show higher fresh and dry weights and more seed yield in comparison to wildtype or mock transformed plants.

To determine cold tolerance, seeds of the transgenic and cold lines are germinated and grown for approximately 10 days to the 4-5 leaf stage as above. The plants are then transferred to cold temperatures (5 °C). Photosynthesis can be measured using chlorophyll fluorescence as an indicator of photosynthetic fitness and integrity of the photosystems. Seed yield and plant dry weight are measured as an indictor of plant biomass production.

It is found that the over-expression of stress related genes from *Brassica napus, Glycine max, Zea mays* or *Oryza sativa* for example provided tolerance to salt and cold as well as drought. Tolerant plants have higher survival rates and biomass production including seed yield, photosynthesis and dry matter production than susceptible plants.

### Example 15

### Engineering stress-tolerant alfalfa plants by over-expressing stress related genes for example from Brassica napus, Glycine max, Zea mays or Oryza sativa for example

A regenerating clone of alfalfa (Medicago sativa) is transformed using the method of McKersie et al., 1999 (Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown and Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659).

Petiole explants are cocultivated with an overnight culture of Agrobacterium tumefaciens C58C1 pMP90 (McKersie et al., 1999 Plant Physiol 119: 839-847) or LBA4404 containing a binary vector. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene that provides constitutive, developmental, tissue or environmental regulation of gene transcription. In this example, the 34S promoter (GenBank Accession numbers M59930 and X16673) was used to provide constitutive expression of the trait gene.

The explants are cocultivated for 3 d in the dark on SH induction medium containing 288 mg/ L Pro, 53 mg/ L thioproline, 4.35 g/ L K2SO4, and 100 µm acetosyringinone. The explants were washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings are transplanted into pots and grown in a greenhouse.

The T0 transgenic plants are propagated by node cuttings and rooted in Turface growth medium. The plants are defoliated and grown to a height of about 10 cm (approximately 2 weeks after defoliation). The plants are then subjected to drought stress in two experiments.

For the drought experiment, the seedlings receive no water for a period up to 3 weeks at which time the plant and soil are desiccated and the suvival and biomass production is determined. At an equivalent degree of drought stress, the tolerant transgenic plants are able to grow normally whereas susceptible wild type plants have died or suffer significant injury resulting in less dry matter.

Tolerance of salinity and cold is measured using methods as described in example 3. It is found that alfalfa plants over-expressing stress related genes from *Brassica napus, Glycine max, Zea mays* or *Oryza sativa* for example are more resistant to salinity and cold stress than non-transgenic control plants. Tolerant plants have higher survival rates and biomass production including seed yield, photosynthesis and dry matter production than susceptible plants.

### Example 16

### Engineering stress-tolerant ryegrass plants by over-expressing stress related genes for example from Brassica napus, Glycine max, Zea mays or Oryza sativa for example

Seeds of several different ryegrass varieties may be used as explant sources for transformation, including the commercial variety Gunne available from Svalof Weibull seed company or the variety Affinity. Seeds are surface-sterilized sequentially with 1% Tween-20 for 1 minute, 100 % bleach for 60 minutes, 3 rinses with 5 minutes each with de-ionized and distilled H2O, and then germinated for 3-4 days on moist, sterile filter paper in the dark. Seedlings are further sterilized for 1 minute with 1% Tween-20, 5 minutes with 75% bleach, and rinsed 3 times with ddH2O, 5 min each.

Surface-sterilized seeds are placed on the callus induction medium containing Murashige and Skoog basal salts and vitamins, 20 g/l sucrose, 150 mg/l asparagine, 500 mg/l casein hydrolysate, 3 g/l Phytagel, 10 mg/l BAP, and 5 mg/l dicamba. Plates are incubated in the dark at 25C for 4 weeks for seed germination and embryogenic callus induction.

After 4 weeks on the callus induction medium, the shoots and roots of the seedlings are trimmed away, the callus is transferred to fresh media, maintained in culture for another 4 weeks, and then transferred to MSO medium in light for 2 weeks. Several pieces of callus (11-17 weeks old) are either strained through a 10 mesh sieve and put onto callus induction medium, or cultured in 100 ml of liquid ryegrass callus induction media (same medium as for callus induction with agar) in a 250 ml flask. The flask is wrapped in foil and shaken at 175 rpm in the dark at 23°C for 1 week. Sieving the liquid culture with a 40-mesh sieve collect the cells. The fraction collected on the sieve is plated and cultured on solid ryegrass callus induction medium for 1 week in the dark at 25C. The callus is then transferred to and cultured on MS medium containing 1% sucrose for 2 weeks.

Transformation can be accomplished with either Agrobacterium of with particle bombardment methods. An expression vector is created containing a constitutive plant promoter and the cDNA of the gene in a pUC vector. The plasmid DNA is prepared from E. coli cells using with Qiagen kit according to manufacturer's instruction. Approximately 2 g of embryogenic callus is spread in the center of a sterile filter paper in a Petri dish. An aliquot of liquid MSO with 10 g/l sucrose is added to the filter paper. Gold particles (1.0 µm in size) are coated with plasmid DNA according to method of Sanford et al., 1993 and delivered to the embryogenic callus with the following parameters: 500 µg particles and 2 µg DNA per shot, 1300 psi and a target distance of 8.5 cm from stopping plate to plate of callus and 1 shot per plate of callus.

After the bombardment, calli are transferred back to the fresh callus development medium and maintained in the dark at room temperature for a 1-week period. The callus is then transferred to growth conditions in the light at 25 °C to initiate embryo differentiation with the appropriate selection agent, e.g. 250 nM Arsenal, 5 mg/l PPT or 50 mg/L kanamycin. Shoots resistant to the selection agent appeared and once rooted are transferred to soil.

Samples of the primary transgenic plants (TO) are analyzed by PCR to confirm the presence of T-DNA. These results are confirmed by Southern hybridization in which DNA is electrophoresed on a 1% agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

Transgenic T0 ryegrass plants are propagated vegetatively by excising tillers. The transplanted tillers are maintained in the greenhouse for 2 months until well established. The shoots are defoliated and allowed to grow for 2 weeks.

The drought experiment is conducted in a manner similar to that described in example 3. The seedlings receive no water for a period up to 3 weeks at which time the plant and soil are desiccated. and survival and biomass production is determined. At an equivalent degree of drought stress, tolerant plants are able to resume normal growth whereas susceptible plants have died or suffer significant injury resulting in shorter leaves and less dry matter.

A second experiment imposing drought stress on the transgenic plants was by treatment with a solution of PEG as described in the previous examples. Tolerance of salinity and cold were measured using methods as described in example 3. It is found that ryegrass over-expressing stress related genes from *Brassica napus, Glycine max, Zea mays* or *Oryza sativa* for example are more resistant to salinity and cold stress that non-transgenic control plants. Tolerant plants have higher survival rates and biomass production including seed yield, photosynthesis and dry matter production than susceptible plants.

### Example 17

### Engineering stress-tolerant soybean plants by over-expressing stress related genes for example from Brassica napus, Glycine max, Zea mays or Oryza sativa for example

Soybean is transformed according to the following modification of the method described in the Texas A&M patent US 5,164,310. Several commercial soybean varieties are amenable to transformation by this method. The cultivar Jack (available from the Illinois Seed Foundation) is a commonly used for transformation. Seeds are sterilized by immersion in 70% (v/v) ethanol for 6 min and in 25 % commercial bleach (NaOCl) supplemented with 0.1 % (v/v) Tween for 20 min, followed by rinsing 4 times with sterile double distilled water. Seven-day old seedlings are propagated by removing the radicle, hypocotyl and one cotyledon from each seedling. Then, the epicotyl with one cotyledon is transferred to fresh germination media in petri dishes and incubated at 25 °C under a 16-hr photoperiod (approx. 100 µE/(m-2s-1) for three weeks. Axillary nodes (approx. 4 mm in length) are cut from 3 - 4 week-old plants. Axillary nodes are excised and incubated in Agrobacterium LBA4404 culture.

Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription. In this example, the 34S promoter (GenBank Accession numbers M59930 and X16673) is used to provide constitutive expression of the trait gene.

After the co-cultivation treatment, the explants are washed and transferred to selection media supplemented with 500 mg/L timentin. Shoots are excised and placed on a shoot elongation medium. Shoots longer than 1 cm are placed on rooting medium for two to four weeks prior to transplanting to soil.

The primary transgenic plants (TO) are analyzed by PCR to confirm the presence of T-DNA. These results are confirmed by Southern hybridization in which DNA is electrophoresed on a 1 % agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

Stress-tolerant soybean plants over-expressing stress related genes from *Brassica napus, Glycine max, Zea mays* or *Oryza sativa for example* have higher seed yields

Tolerance of drought, salinity and cold are measured using methods as described in example 3. Tolerant plants have higher survival rates and biomass production including seed yield, photosynthesis and dry matter production than susceptible plants.

### Example 18

### Engineering stress-tolerant Rapeseed/Canola plants by over-expressing stress related genes for example from Brassica napus, Glycine max, Zea mays or Oryza sativa for example

Cotyledonary petioles and hypocotyls of 5-6 day-old young seedlings are used as explants for tissue culture and transformed according to Babic et al. (1998, Plant Cell Rep 17: 183-188). The commercial cultivar Westar (Agriculture Canada) is the standard variety used for transformation, but other varieties can be used.

Agrobacterium tumefaciens LBA4404 containing a binary vector is used for canola transformation. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription. In this example, the 34S promoter (GenBank Accession numbers M59930 and X16673) is used to provide constitutive expression of the trait gene.

Canola seeds are surface-sterilized in 70% ethanol for 2 min., and then in 30% Clorox with a drop of Tween-20 for 10 min, followed by three rinses with sterilized distilled water. Seeds are then germinated in vitro 5 days on half strength MS medium without hormones, 1% sucrose, 0.7% Phytagar at 23oC, 16 hr. light. The cotyledon petiole explants with the cotyledon attached are excised from the in vitro seedlings, and inoculated with Agrobacterium by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction.

Samples of the primary transgenic plants (TO) are analyzed by PCR to confirm the presence of T-DNA. These results are confirmed by Southern hybridization in which DNA is electrophoresed on a 1 % agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

The transgenic plants are then evaluated for their improved stress tolerance according to the method described in Example 3. It is found that transgenic Rapeseed/Canola over-expressing stress related genes from *Brassica napus, Glycine max, Zea mays* or *Oryza sativa* for example are more tolerant to environmental stress than non-transgenic control plants. Tolerant plants have higher survival rates and biomass production including seed yield, photosynthesis and dry matter production than susceptible plants.

### Example 19

### Engineering stress-tolerant corn plants by over-expressing stress related genes for example from Brassica napus, Glycine max, Zea mays or Oryza sativa for example

Transformation of corn (Zea mays L.) is performed with a modification of the method described by Ishida et al. (1996. Nature Biotech 14745-50). Transformation is genotype-dependent in corn and only specific genotypes are amenable to transformation and regeneration. The inbred line A188 (University of Minnesota) or hybrids with A188 as a parent are good sources of donor material for transformation (Fromm et al. 1990 Biotech 8:833-839), but other genotypes can be used successfully as well. Ears are harvested from corn plants at approximately 11 days after pollination (DAP) when the length of immature embryos is about 1 to 1.2 mm. Immature embryos are co-cultivated with Agrobacterium tumefaciens that carry "super binary" vectors and transgenic plants are recovered through organogenesis. The super binary vector system of Japan Tobacco is described in WO patents WO94/00977 and WO95/06722. Vectors are constructed as described. Various selection marker genes can be used including the corn gene encoding a mutated acetohydroxy acid synthase (AHAS) enzyme (US patent 6025541). Similarly, various promoters can be used to regulate the trait gene to provide constitutive, developmental, tissue or environmental regulation of gene transcription. In this example, the 34S promoter (GenBank Accession numbers M59930 and X16673) is used to provide constitutive expression of the trait gene.

Excised embryos are grown on callus induction medium, then corn regeneration medium, containing imidazolinone as a selection agent. The Petri plates were incubated in the light at 25 °C for 2-3 weeks, or until shoots develop. The green shoots from each embryo are transferred to corn rooting medium and incubated at 25 °C for 2-3 weeks, until roots develop. The rooted shoots are transplanted to soil in the greenhouse. T1 seeds are produced from plants that exhibit tolerance to the imidazolinone herbicides and are PCR positive for the transgenes.

The T1 transgenic plants are then evaluated for their improved stress tolerance according to the methods described in Example 3. The T1 generation of single locus insertions of the T-DNA will segregate for the transgene in a 1:2:1 ratio. Those progeny containing one or two copies of the transgene (3/4 of the progeny) are tolerant of the imidazolinone herbicide, and exhibit greater tolerance of drought stress than those progeny lacking the transgenes. Tolerant plants have higher seed yields. Homozygous T2 plants exhibited similar phenotypes. Hybrid plants (F1 progeny) of homozygous transgenic plants and non-transgenic plants also exhibited increased environmental stress tolerance.

Tolerance to salinity and cold are measured using methods as described in the previous example 3. Again, transgenic corn plants over-expressing stress related genes from *Brassica napus, Glycine max, Zea mays* or *Oryza* sativa for example are found to be tolerant to environmental stresses. Tolerant plants have higher survival rates and biomass production including seed yield, photosynthesis and dry matter production than susceptible plants.

### Example 20

### Engineering stress-tolerant wheat plants by over-expressing stress related genes for example from Brassica napus, Glycine max, Zea mays or Oryza sativa for example

Transformation of wheat is performed with the method described by Ishida et al. (1996 Nature Biotech. 14745-50). The cultivar Bobwhite (available from CYMMIT, Mexico) is commonly used in transformation. Immature embryos are co-cultivated with Agrobacterium tumefaciens that carry "super binary" vectors, and transgenic plants are recovered through organogenesis. The super binary vector system of Japan Tobacco is described in WO patents WO94/00977 and WO95/06722. Vectors are constructed as described. Various selection marker genes can be used including the maize gene encoding a mutated acetohydroxy acid synthase (AHAS) enzyme (US patent 6025541). Similarly, various promoters can be used to regulate the trait gene to provide constitutive, developmental, tissue or environmental regulation of gene transcription. In this example, the 34S promoter (GenBank Accession numbers M59930 and X16673) is used to provide constitutive expression of the trait gene.

After incubation with Agrobacterium, the embryos are grown on callus induction medium, then regeneration medium, containing imidazolinone as a selection agent. The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the imidazolinone herbicides and which are PCR positive for the transgenes.

The T1 transgenic plants are then evaluated for their improved stress tolerance according to the method described in the previous example 3. The T1 generation of single locus insertions of the T-DNA will segregate for the transgene in a 1:2:1 ratio. Those progeny containing one or two copies of the transgene (3/4 of the progeny) are tolerant of the imidazolinone herbicide, and exhibit greater tolerance of drought stress than those progeny lacking the transgenes. Tolerant plants have higher survival rates and biomass production including seed yield, photosynthesis and dry matter production than susceptible plants.Homozygous T2 plants exhibited similar phenotypes. Tolerance of salinity and cold are measured using methods as described in the previous examples. Plants that overexpressed *stress related genes from Brassica napus, Glycine max, Zea mays or Oryza sativa for example have* tolerance to drought, salinity or cold and displayed higher survival rates and biomass production including seed yield, photosynthesis and dry matter production than susceptible plants.

### Example 21

### Identification of Identical and Heterologous Genes

Gene sequences can be used to identify identical or heterologous genes from cDNA or genomic libraries. Identical genes (e. g. full-length cDNA clones) can be isolated via nucleic acid hybridization using for example cDNA libraries. Depending on the abundance of the gene of interest, 100,000 up to 1,000,000 recombinant bacteriophages are plated and transferred to nylon membranes. After denaturation with alkali, DNA is immobilized on the membrane by e. g. UV cross linking. Hybridization is carried out at high stringency conditions. In aqueous solution, hybridization and washing is performed at an ionic strength of 1 M NaCl and a temperature of 68°C. Hybridization probes are generated by e.g. radioactive (³²P) nick transcription labeling (High Prime, Roche, Mannheim, Germany). Signals are detected by autoradiography.

Partially identical or heterologous genes that are similar but not identical can be identified in a manner analogous to the above-described procedure using low stringency hybridization and washing conditions. For aqueous hybridization, the ionic strength is normally kept at 1 M NaCl while the temperature is progressively loared from 68 to 42°C.

Isolation of gene sequences with homology (or sequence identity/similarity) in only a distinct domain of for example 10-20 amino acids can be carried out using synthetic radio labeled oligonucleotide probes. Radiolabeled oligonucleotides are prepared by phosphorylation of the 5-prime end of two complementary oligonucleotides with T4 polynucleotide kinase. The complementary oligonucleotides are annealed and ligated to form concatemers. The double stranded concatemers are then radiolabeled by, for example, nick transcription. Hybridization is normally performed at low stringency conditions using high oligonucleotide concentrations.

Oligonucleotide hybridization solution:
6 x SSC
0.01 M sodium phosphate
1 mM EDTA (pH 8)
0.5 % SDS
100 µg/ml denatured salmon sperm DNA
0.1 % nonfat dried milk

During hybridization, temperature is lowered stepwise to 5-10°C below the estimated oligonucleotide Tₘ or down to room temperature followed by washing steps and autoradiography. Washing is performed with low stringency such as 3 washing steps using 4 x SSC. Further details are described by Sambrook, J. et al., 1989, "Molecular Cloning: A Laboratory Manual," Cold Spring Harbor Laboratory Press or Ausubel, F.M. et al., 1994, "Current Protocols in Molecular Biology," John Wiley & Sons.

### Example 22

### Identification of Identical or homologous Genes by Screening Expression Libraries with Antibodies

c-DNA clones can be used to produce recombinant polypeptide for example in *E. coli* (e.g. Qiagen QIAexpress pQE system). Recombinant polypeptides are then normally affinity purified via Ni-NTA affinity chromatography (Qiagen). Recombinant polypeptides are then used to produce specific antibodies for example by using standard techniques for rabbit immunization. Antibodies are affinity purified using a Ni-NTA column saturated with the recombinant antigen as described by Gu et al., 1994, BioTechniques 17:257-262. The antibody can then be used to screen expression cDNA libraries to identify identical or heterologous genes via an immunological screening (Sambrook, J. et al., 1989, "Molecular Cloning: A Laboratory Manual," Cold Spring Harbor Laboratory Press or Ausubel, F.M. et al., 1994, "Current Protocols in Molecular Biology", John Wiley & Sons).

### Example 23

### In vivo Mutagenesis

*In vivo* mutagenesis of microorganisms can be performed by passage of plasmid (or other vector) DNA through *E. coli* or other microorganisms (e.g. *Bacillus* spp. or yeasts such as *Saccharomyces cerevisiae*), which are impaired in their capabilities to maintain the integrity of their genetic information. Typical mutator strains have mutations in the genes for the DNA repair system (e.g., mutHLS, mutD, mutT, etc.; for reference, see Rupp, W.D., 1996, DNA repair mechanisms, in: Escherichia coli and Salmonella, p. 2277-2294, ASM: Washington.) Such strains are well known to those skilled in the art. The use of such strains is illustrated, for example, in Greener, A. and Callahan, M., 1994, Strategies 7: 32-34. Transfer of mutated DNA molecules into plants is preferably done after selection and testing in microorganisms. Transgenic plants are generated according to various examples within the exemplification of this document.

### Plant Screening for growth under low temperature conditions

In a standard experiment soil was prepared as 3.5:1 (v/v) mixture of nutrient rich soil (GS90, Tantau, Wansdorf, Germany) and sand. Pots were filled with soil mixture and placed into trays. Water was added to the trays to let the soil mixture take up appropriate amount of water for the sowing procedure. The seeds for transgenic A. thaliana plants were sown in pots (6cm diameter). Pots were collected until they filled a tray for the growth chamber. Then the filled tray was covered with a transparent lid and transferred into the shelf system of the precooled (4°C-5°C) growth chamber. Stratification was established for a period of 2-3 days in the dark at 4°C-5°C. Germination of seeds and growth was initiated at a growth condition of 20°C, 60% relative humidity, 16h photoperiod and illumination with fluorescent light at 200 µmol/m2s. Covers were removed 7 days after sowing. BASTA selection was done at day 9 after sowing by spraying pots with plantlets from the top. Therefore, a 0.07% (v/v) solution of BASTA concentrate (183 g/l glufosinate-ammonium) in tap water was sprayed. Transgenic events and wildtype control plants were distributed randomly over the chamber. The location of the trays inside the chambers was changed on working days from day 7 after sowing. Watering was carried out every two days after covers were removed from the trays. Plants were individualized 12-13 days after sowing by removing the surplus of seedlings leaving one seedling in a pot. Cold (chilling to 11°C-12°C) was applied 14 days after sowing until the end of the experiment. For measuring biomass performance, plant fresh weight was determined at harvest time (34-36 days after sowing) by cutting shoots and weighing them. Beside weighing, phenotypic information was added in case of plants that differ from the wild type control. Plants were in the stage prior to flowering and prior to growth of inflorescence when harvested. Significance values for the statistical significance of the biomass changes were calculated by applying the `student's' t test (parameters: two-sided, unequal variance).

Three successive experiments were conducted. In the first experiment, one individual of each transformed line was tested.

In the second experiment, the event that had been determined as chilling tolerant or resistant in the first experiment, i.e. showed increased yield, in this case increased biomass production, in comparison to wild type, were put through a confirmation screen according to the same experimental procedures. In this experiment, max. 10 plants of each tolerant or resistant event were grown, treated and measured as before.

In the first two experiments, chilling tolerance or tolerance and biomass production was compared to wild type plants.

In the third experiment up to 20 replicates of each confirmed tolerant event, i.e. those that had been scored as tolerant or resistant in the second experiment, were grown, treated and scored as before. The results thereof are summarized in table 2.

Table 2: Biomass production of transgenic A. thaliana after imposition of chilling stress.

Biomass production was measured by weighing plant rosettes. Biomass increase was calculated as ratio of average weight for trangenic plants compared to average weight of wild type control plants from the same experiment. The minimum and maximum biomass increase seen within the group of transgenic events is given for a locus with all those events showing a significance value < 0.1 and a biomass increase > 10% (ratio > 1.1).

**Table 2:**

| SeqID | Target | Locus | Biomass Increase min | Biomass Increase max |
|---|---|---|---|---|
| 8178 | Non-targeted | YER174C | 1.380 | 1.459 |

### Plant screening for growth under cycling drought conditions

In a standard experiment soil is prepared as 1:1 (v/v) mixture of nutrient rich soil (GS90, Tantau, Wansdorf, Germany) and quarz sand. Pots (6cm diameter) were filled with this mixture and placed into trays. Water was added to the trays to let the soil mixture take up appropriate amount of water for the sowing procedure (day 1) and subsequently seeds for transgenic A. thaliana plants and their non-trangenic controls were sown in pots. Then the filled tray was covered with a transparent lid and transferred into a precooled (4°C-5°C) and darkened growth chamber. Stratification was established for a period of 3 days in the dark at 4°C-5°C or, alternatively, for 4 days in the dark at 4°C. Germination of seeds and growth was initiated at a growth condition of 20°C, 60% relative humidity, 16h photoperiod and illumination with fluorescent light at 200µmol/m2s or, alternatively at 220µmol/m2s. Covers were removed 7-8 days after sowing. BASTA selection was done at day 10 or day 11 (9 or 10 days after sowing) by spraying pots with plantlets from the top. In the standard experiment, a 0.07% (v/v) solution of BASTA concentrate (183 g/l glufosinate-ammonium) in tap water was sprayed once or, alternatively, a 0.02% (v/v) solution of BASTA was sprayed three times. The non-transgenic control plants were sprayed with tap water only (instead of spraying with BASTA dissolved in tap water) but were otherwise treated identically. Plants were individualized 13-14 days after sowing by removing the surplus of seedlings and leaving one seedling in soil. Transgenic events and wildtype control plants were evenly distributed over the chamber.

The water supply throughout the experiment was limited and plants were subjected to cycles of drought and re-watering. Watering was carried out at day 1 (before sowing), day 14 or day 15, day 21 or day 22, and, finally, day 27 or day 28. For measuring biomass production, plant fresh weight was determined one day after the final watering (day 28 or day 29) by cutting shoots and weighing them. Besides weighing, phenotypic information was added in case of plants that differ from the wild type control. Plants were in the stage prior to flowering and prior to growth of inflorescence when harvested. Significance values for the statistical significance of the biomass changes were calculated by applying the `student's' t test (parameters: two-sided, unequal variance).

In a standard procedure three successive experiments were conducted. In the first experiment, one individual of each transformed line/event was tested. In the second experiment, the events that had been determined as cycling drought tolerant or resistant in the first experiment, i.e. showed increased yield, in this case increased biomass production, in comparison to wild type, were put through a confirmation screen according to the same experimental procedures. In this experiment, max. 10 plants of each tolerant or resistant event were grown, treated and measured as before.

In the first two experiments, cycling drought resistance or tolerance and biomass production was compared to wild type plants.

In the third experiment up to 20 replicates of each confirmed tolerant event, i.e. those that had been scored as tolerant or resistant in the second experiment, were grown, treated and scored as before. The results thereof are summarized in table 3.

Table 3: Biomass production of transgenic A. thaliana developed under cycling drought growth conditions.

Biomass production was measured by weighing plant rosettes. Biomass increase was calculated as ratio of average weight for trangenic plants compared to average weight of wild type control plants from the same experiment. The minimum and maximum biomass increase seen within the group of transgenic events is given for a locus with all those events showing a significance value < 0.1 and a biomass increase > 10% (ratio > 1.1).

**Table 3:**

| SeqID | Target | Locus | Biomass Increase min | Biomass Increase max |
|---|---|---|---|---|
| 8178 | Plastidic | YER174C | 1.211 | 1.275 |

### Plant screening for biomass increase under standardised growth conditions

In this experiment, a plant screening for biomass under standardised growth conditions in the absence of substantial abiotic stress has been performed. In a standard experiment soil is prepared as 3.5:1 (v/v) mixture of nutrient rich soil (GS90, Tantau, Wansdorf, Germany) and quarz sand. Alternatively, plants were sown on nutrient rich soil (GS90, Tantau, Germany). Pots were filled with soil mixture and placed into trays. Water was added to the trays to let the soil mixture take up appropriate amount of water for the sowing procedure. The seeds for transgenic A. thaliana plants and their non-trangenic controls were sown in pots (6cm diameter). Then the filled tray was covered with a transparent lid and transferred into a precooled (4°C-5°C) and darkened growth chamber. Stratification was established for a period of 3 days in the dark at 4°C-5°C or, alternatively, for 4 days in the dark at 4°C. Germination of seeds and growth was initiated at a growth condition of 20°C, 60% relative humidity, 16h photoperiod and illumination with fluorescent light at 200µmol/m2s or, alternatively at 220µmol/m2s. Covers were removed 7-8 days after sowing. BASTA selection was done at day 9 after sowing by spraying pots with plantlets from the top. In the standard experiment, a 0.07% (v/v) solution of BASTA concentrate (183 g/l glufosinate-ammonium) in tap water was sprayed once or, alternatively, a 0.02% (v/v) solution of BASTA was sprayed three times. Plants were individualized 13-14 days after sowing by removing the surplus of seedlings and leaving one seedling in soil. Transgenic events and wildtype control plants were evenly distributed over the chamber. Watering was carried out every two days after removing the covers in a standard experiment or, alternatively, every day.

For measuring biomass performance, plant fresh weight was determined at harvest time (26-27 days after sowing) by cutting shoots and weighing them. Alternatively, the harvest time was 24-25 days after sowing. Beside weighing, phenotypic information was added in case of plants that differ from the wild type control. Plants were in the stage prior to flowering and prior to growth of inflorescence when harvested. Significance values for the statistical significance of the biomass changes were calculated by applying the `student's' t test (parameters: two-sided, unequal variance).

In a standard procedure three successive experiments were conducted. In the first experiment, one individual of each transformed line/event was tested. In the second experiment, the events that had been determined as high biomass yielding from the first experiment in comparison to wild type, were put through a confirmation screen according to the same experimental procedures. A maximum of 10 plants of each high biomass yielding event were grown, treated and measured as before.

In the first two experiments biomass production was compared to wild type plants.

In the third experiment up to 20 replicates of each confirmed tolerant event, i.e. those that had been scored as high yielding in the second experiment, were grown, treated and scored as before. The results thereof are summarized in table 4.

Table 4: Biomass production of transgenic A. thaliana developed under ambient growth conditions.

Biomass production was measured by weighing plant rosettes. Biomass increase was calculated as ratio of average weight for trangenic plants compared to average weight of wild type control plants from the same experiment. The minimum and maximum biomass increase seen within the group of transgenic events is given for a locus with all those events showing a significance value < 0.1 and a biomass increase > 10% (ratio > 1.1).

**Table 4:**

| SeqID | Target | Locus | Biomass Increase min | Biomass Increase max |
|---|---|---|---|---|
| 8178 | Non-targeted | YER174C | 1.416 | 1.453 |

### Plant Screening (Arabidopsis) for growth under limited nitrogen supply

For screening of transgenic plants a specific culture facility was used. For high-throughput purposes plants were screened for biomass production on agar plates with limited supply of nitrogen (adapted from Estelle and Somerville, 1987). This screening pipeline consists of two level. Transgenic lines are subjected to subsequent level if biomass production was significantly improved in comparison to wild type plants. With each level number of replicates and statistical stringency was increased.

For the sowing, the seeds, which had been stored in the refrigerator (at -20°C), were removed from the Eppendorf tubes with the aid of a toothpick and transferred onto the above-mentioned agar plates, with limited supply of nitrogen (0.05 mM KNO₃). In total, approximately 15-30 seeds were distributed horizontally on each plate (12 x 12 cm).

After the seeds had been sown, plates are subjected to stratification for 2-4 days in the dark at 4°C. After the stratification, the test plants were grown for 22 to 25 days at a 16-h-light, 8-h-dark rhythm at 20°C, an atmospheric humidity of 60% and a CO₂ concentration of approximately 400 ppm. The light sources used generate a light resembling the solar color spectrum with a light intensity of approximately 100 µE/m²s. After 10 to 11 days the plants are individualized. Improved growth under nitrogen limited conditions was assessed by biomass production of shoots and roots of transgenic plants in comparison to wild type control plants after 20-25 days growth.

Transgenic lines showing a significant improved biomass production in comparison to wild type plants are subjected to following experiment of the subsequent level:

Arabidopsis thaliana seeds are sown in pots containing a 1:1 (v:v) mixture of nutrient depleted soil ("Einheitserde Typ 0", 30% clay, Tantau, Wansdorf Germany) and sand. Germination is induced by a four day period at 4°C, in the dark. Subsequently the plants are grown under standard growth conditions (photoperiod of 16 h light and 8 h dark, 20 °C, 60% relative humidity, and a photon flux density of 200 µE). The plants are grown and cultured, inter alia they are watered every second day with a N-depleted nutrient solution. The N-depleted nutrient solution e.g. contains beneath water the components of table 5.

**Table 5**

| mineral nutrient | final concentration |
|---|---|
| KCI | 3.00 mM |
| MgSO₄ x 7H₂O | 0.5 mM |
| CaCl₂ x 6 H₂O | 1.5 mM |
| K₂SO₄ | 1.5 mM |
| NaH₂PO₄ | 1.5 mM |
| Fe-EDTA | 40 µM |
| H₃BO₃ | 25 µM |
| MnSO₄ x H₂O | 1 µM |
| ZnSO₄ x 7 H₂O | 0.5 µM |
| Cu₂SO₄ x 5 H₂O | 0.3 µM |
| Na₂MoO₄ x 2 H₂O | 0.05 µM |

After 9 to 10 days the plants are individualized. After a total time of 29 to 31 days the plants are harvested and rated by the fresh weight of the arial parts of the plants. The results thereof are summarized in table 6. The biomass increase has been measured as ratio of the fresh weight of the aerial parts of the respective transgene plant and the non-transgenic wild type plant.

Table 6: Biomass production of transgenic A. thaliana grown under limited nitrogen supply

Biomass production was measured by weighing plant rosettes. Biomass increase was calculated as ratio of average weight for trangenic plants compared to average weight of wild type control plants from the same experiment. The minimum and maximum biomass increase seen within a group of transgenic events is given for a locus with all those events that show a significance value < 0.1 and a biomass increase ≥ 10% (ratio ≥ 1.1).

**Table 6**

| SeqID | Target | Locus | Biomass Increase min | Biomass Increase max |
|---|---|---|---|---|
| 8178 | Non-targeted | YER174C | 1.306 | 1.604 |

### Figures:

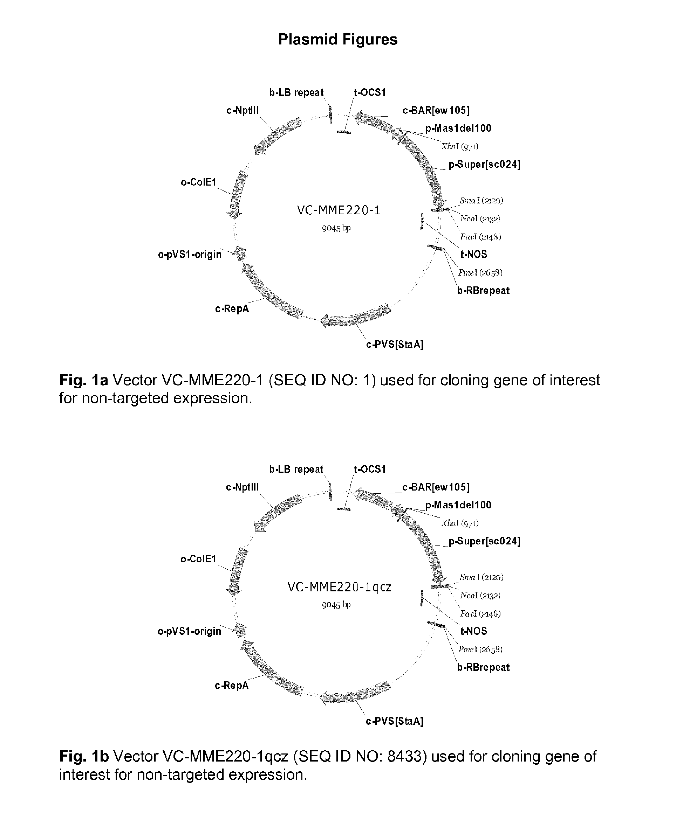

**Fig. 1a** Vector VC-MME220-1 SEQ ID NO: 1 used for cloning gene of interest for non-targeted expression.
**Fig. 1 b** Vector VC-MME220-1qcz SEQ ID NO: 8433 used for cloning gene of interest for non-targeted expression.

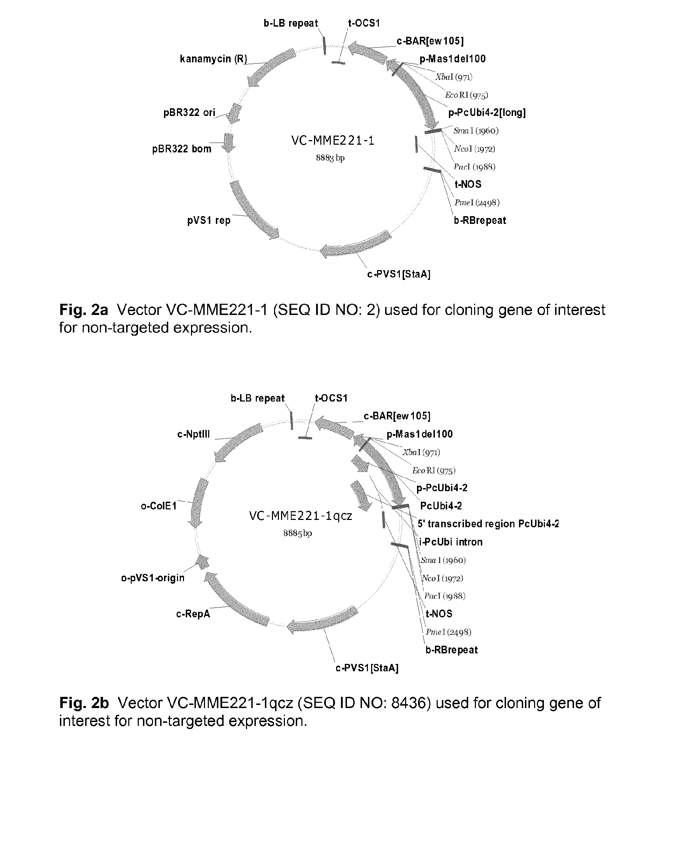

**Fig. 2a** Vector VC-MME221-1 SEQ ID NO: 2 used for cloning gene of interest for non-targeted expression.
**Fig. 2b** Vector VC-MME221-1qcz SEQ ID NO: 8436 used for cloning gene of interest for non-targeted expression.

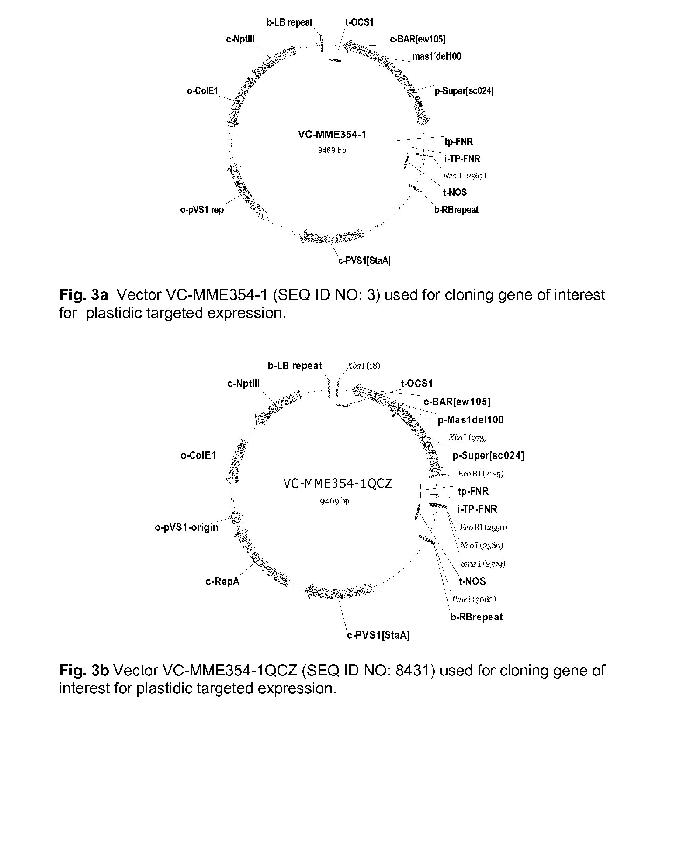

**Fig. 3a** Vector VC-MME354-1 SEQ ID NO: 3 used for cloning gene of interest for targeted expression.
**Fig. 3b** Vector VC-MME354-1QCZ SEQ ID NO: 8431 used for cloning gene of interest for targeted expression.
**Fig. 4a** Vector VC-MME432-1 SEQ ID NO: 5 used for cloning gene of interest for targeted expression.
**Fig. 4b** Vector VC-MME432-1qcz SEQ ID NO: 8434 used for cloning gene of interest for targeted expression.

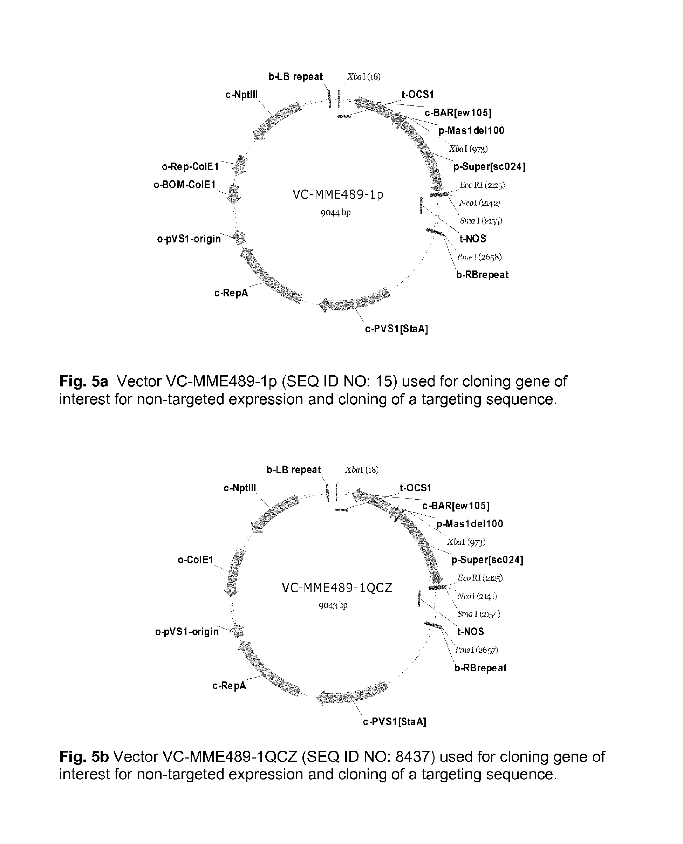

**Fig. 5a** Vector VC-MME489-1p SEQ ID NO: 15 used for cloning gene of interest for non-targeted expression and cloning of a targeting sequence.
**Fig. 5b** Vector VC-MME489-1QCZ SEQ ID NO: 8437 used for cloning gene of interest for non-targeted expression and cloning of a targeting sequence.
**Fig. 6a** Vector pMTX02270p SEQ ID NO: 16 used for cloning of a targeting sequence.

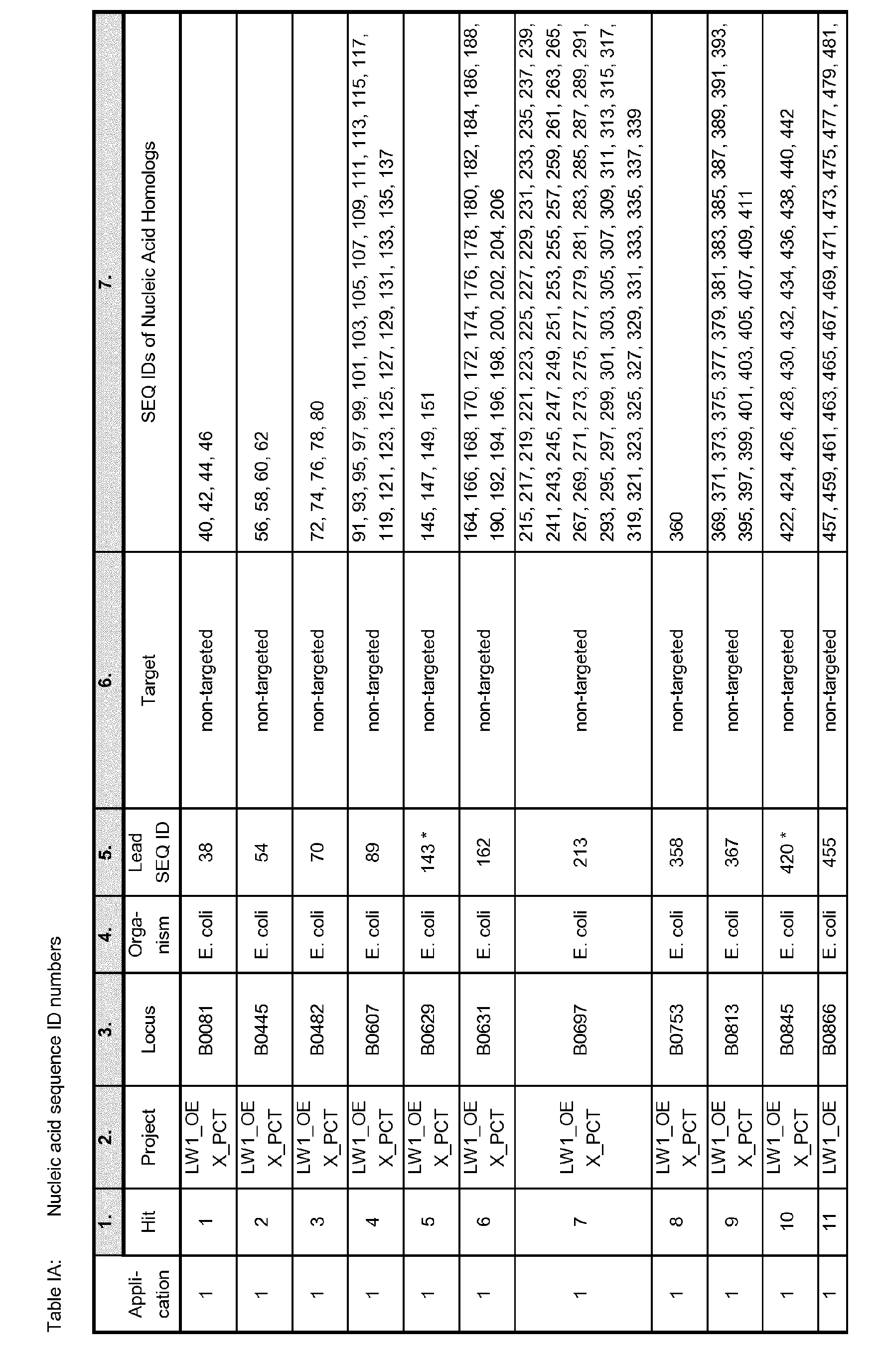

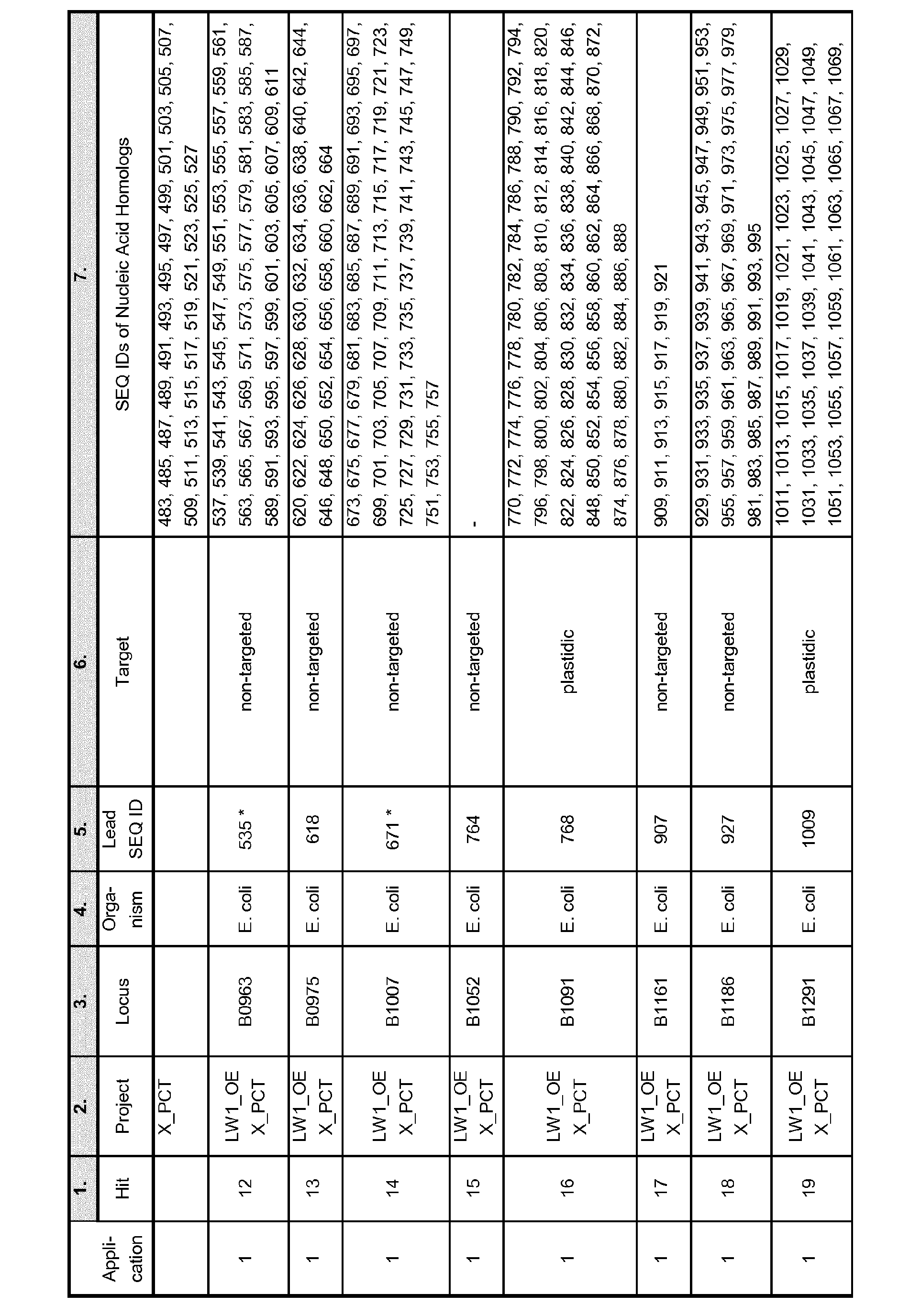

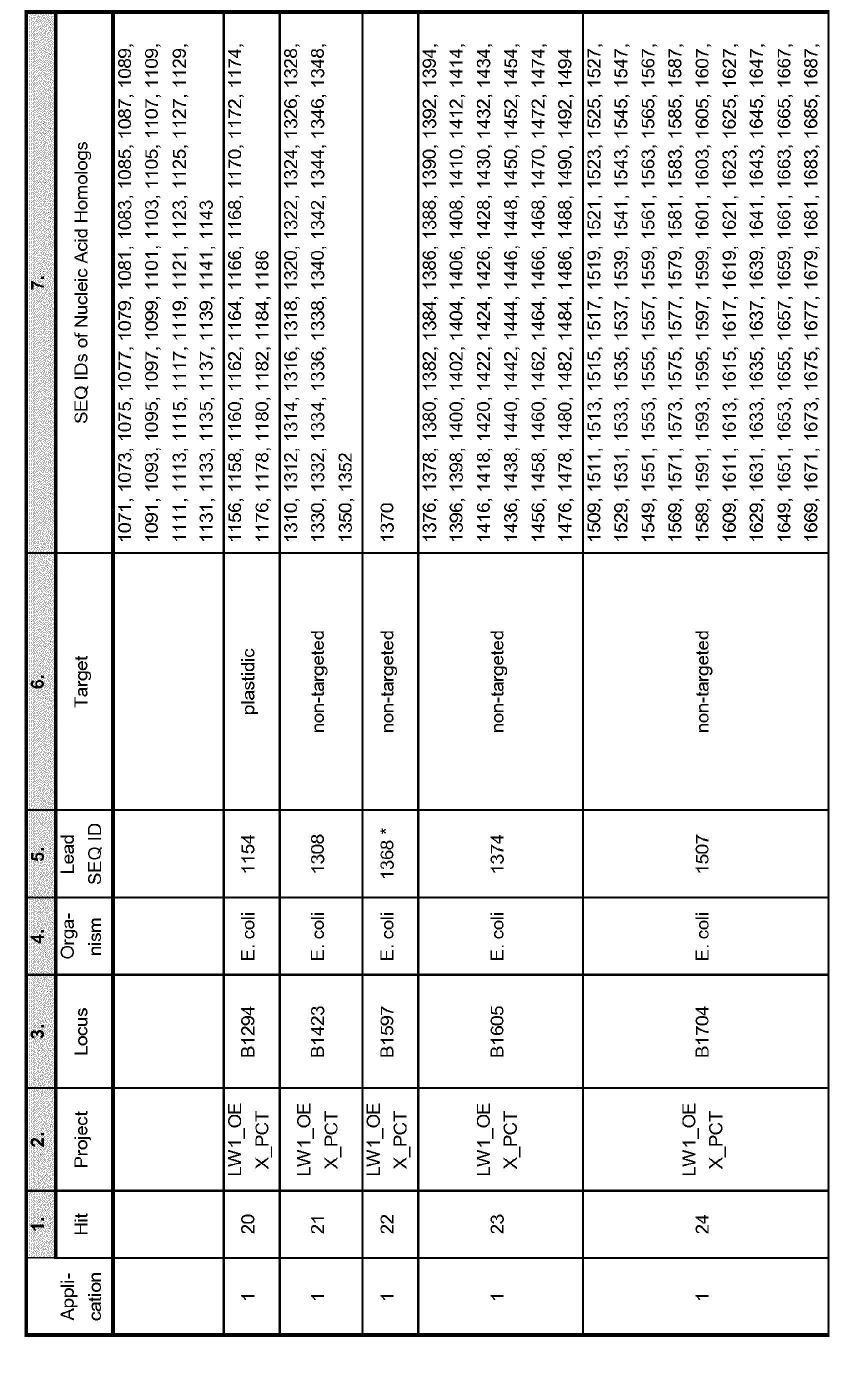

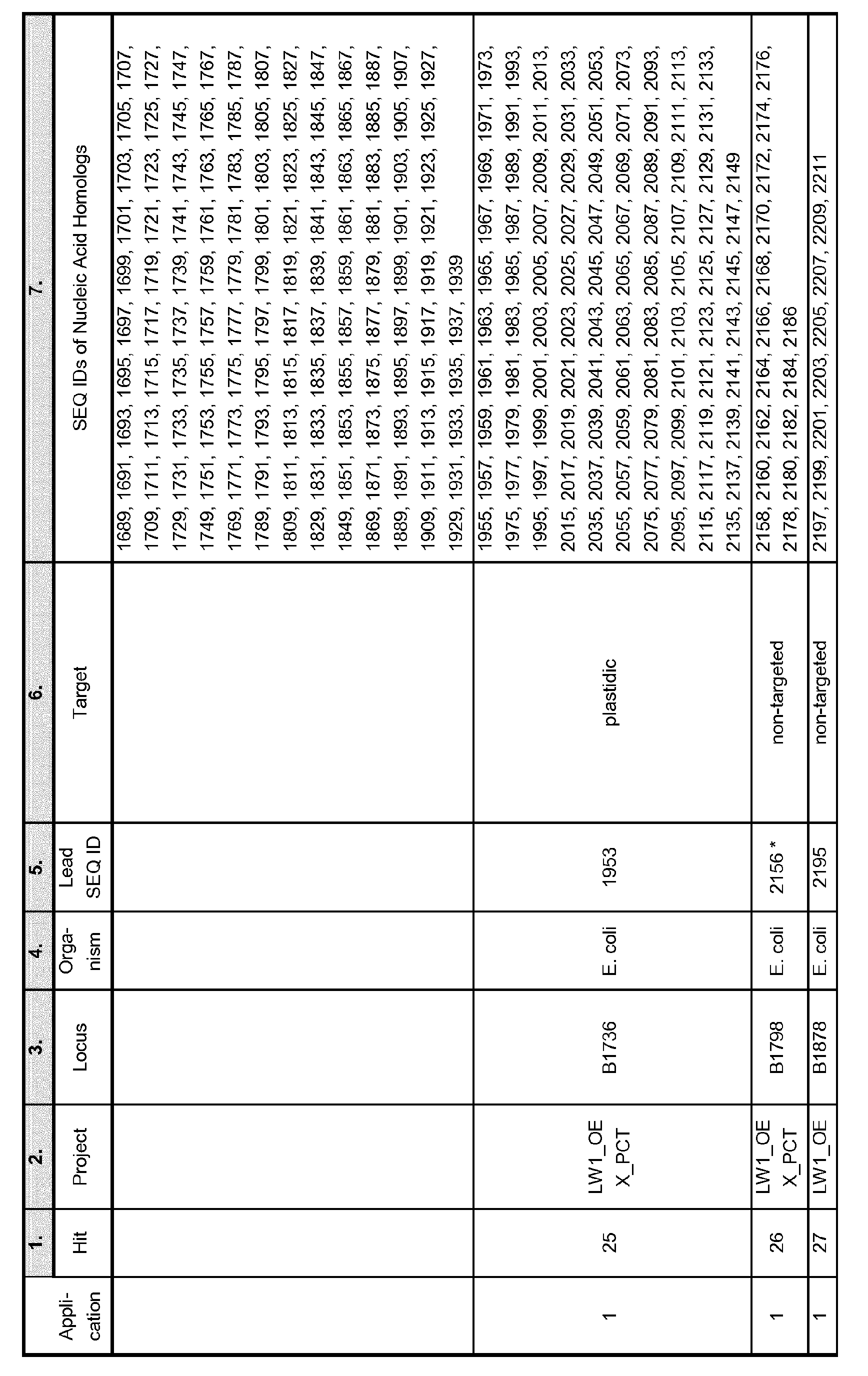

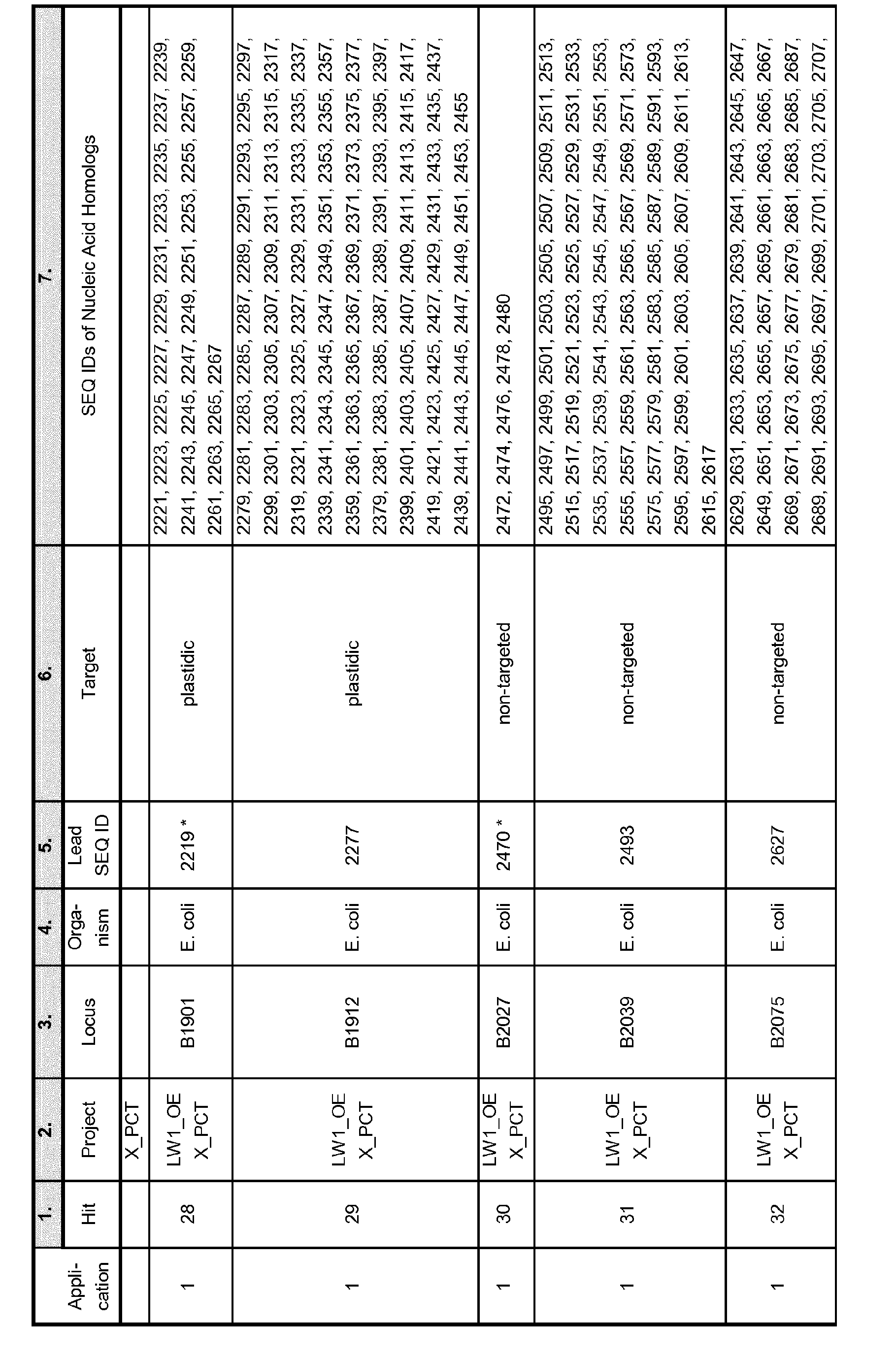

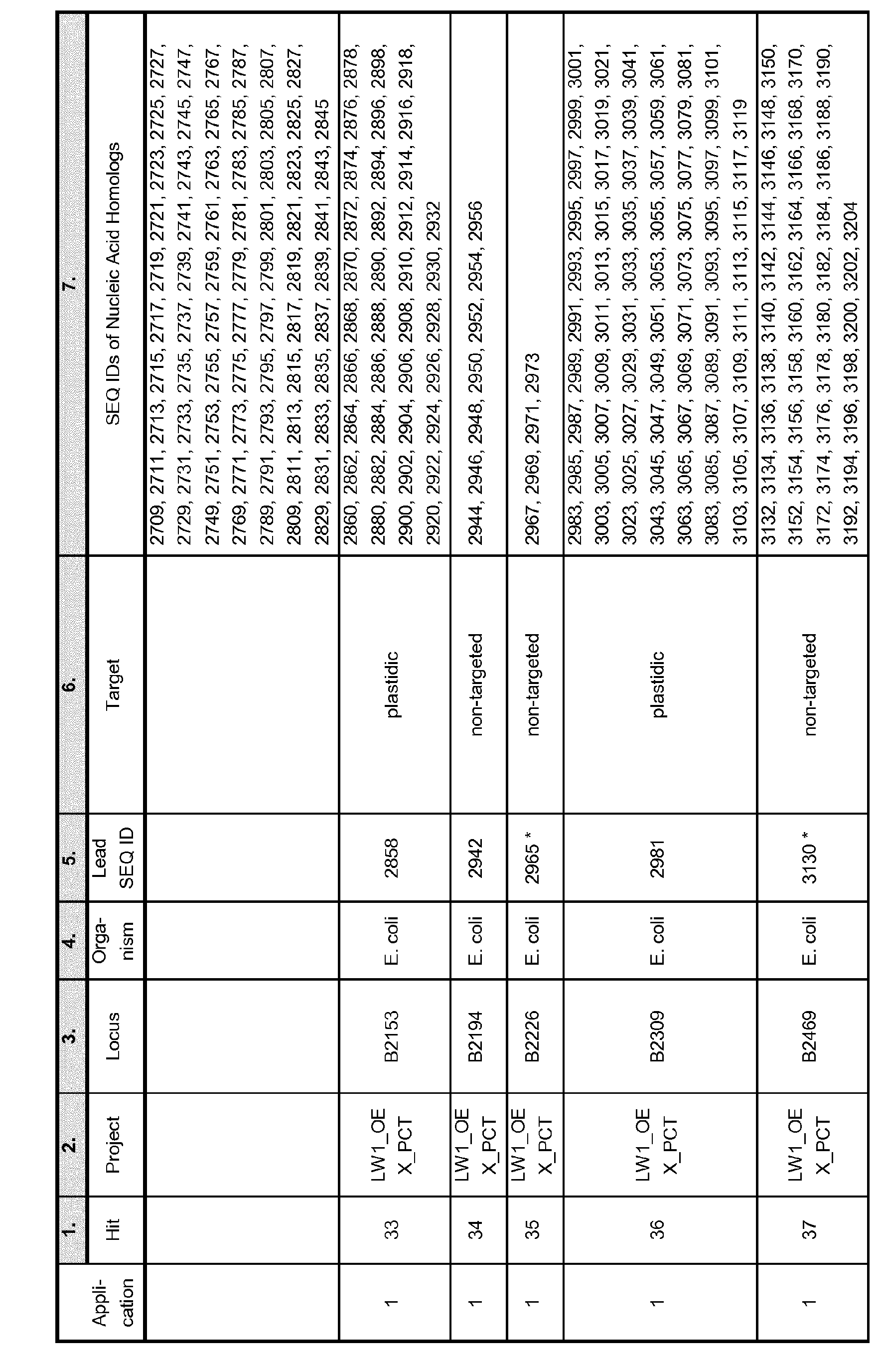

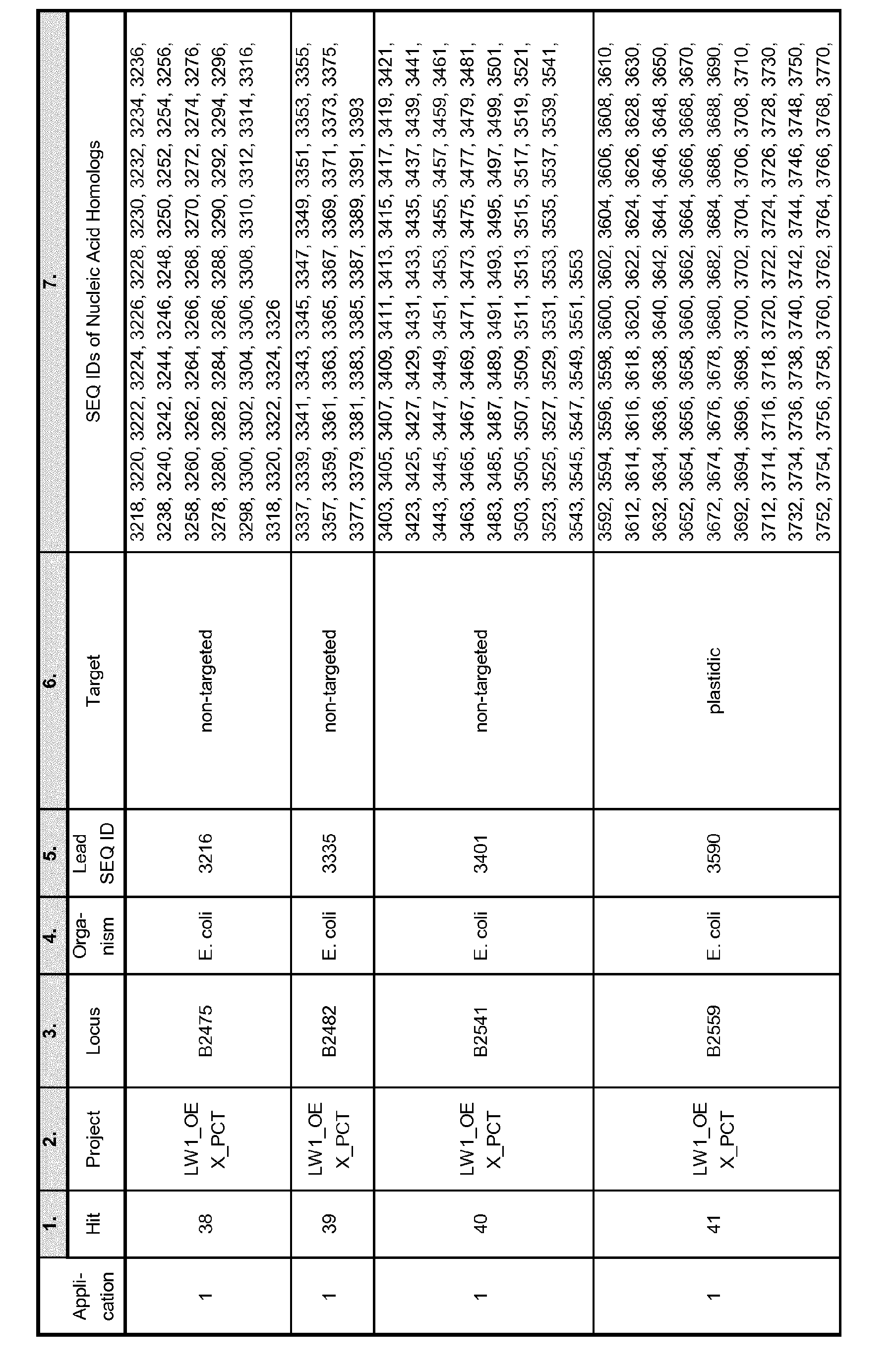

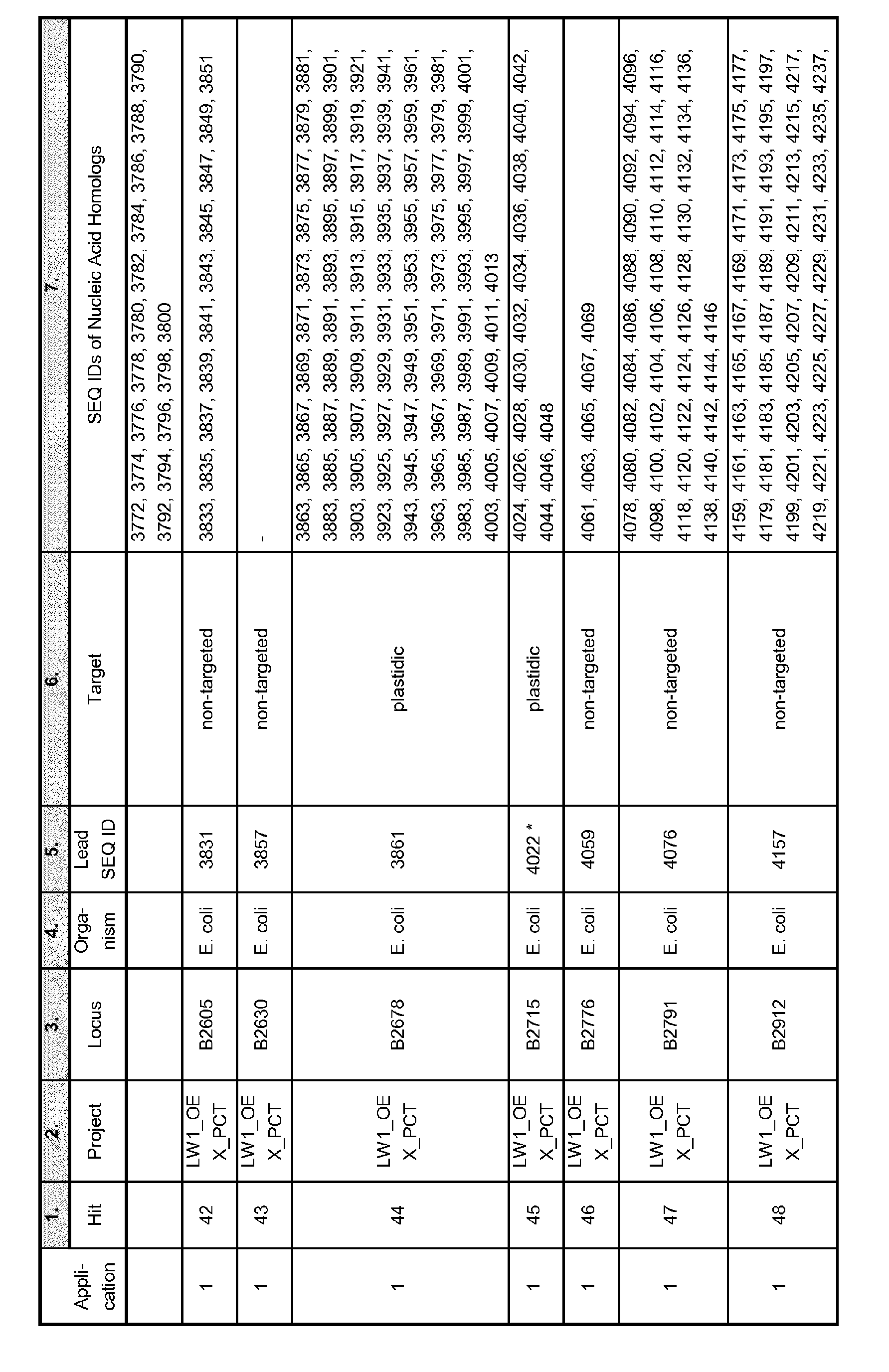

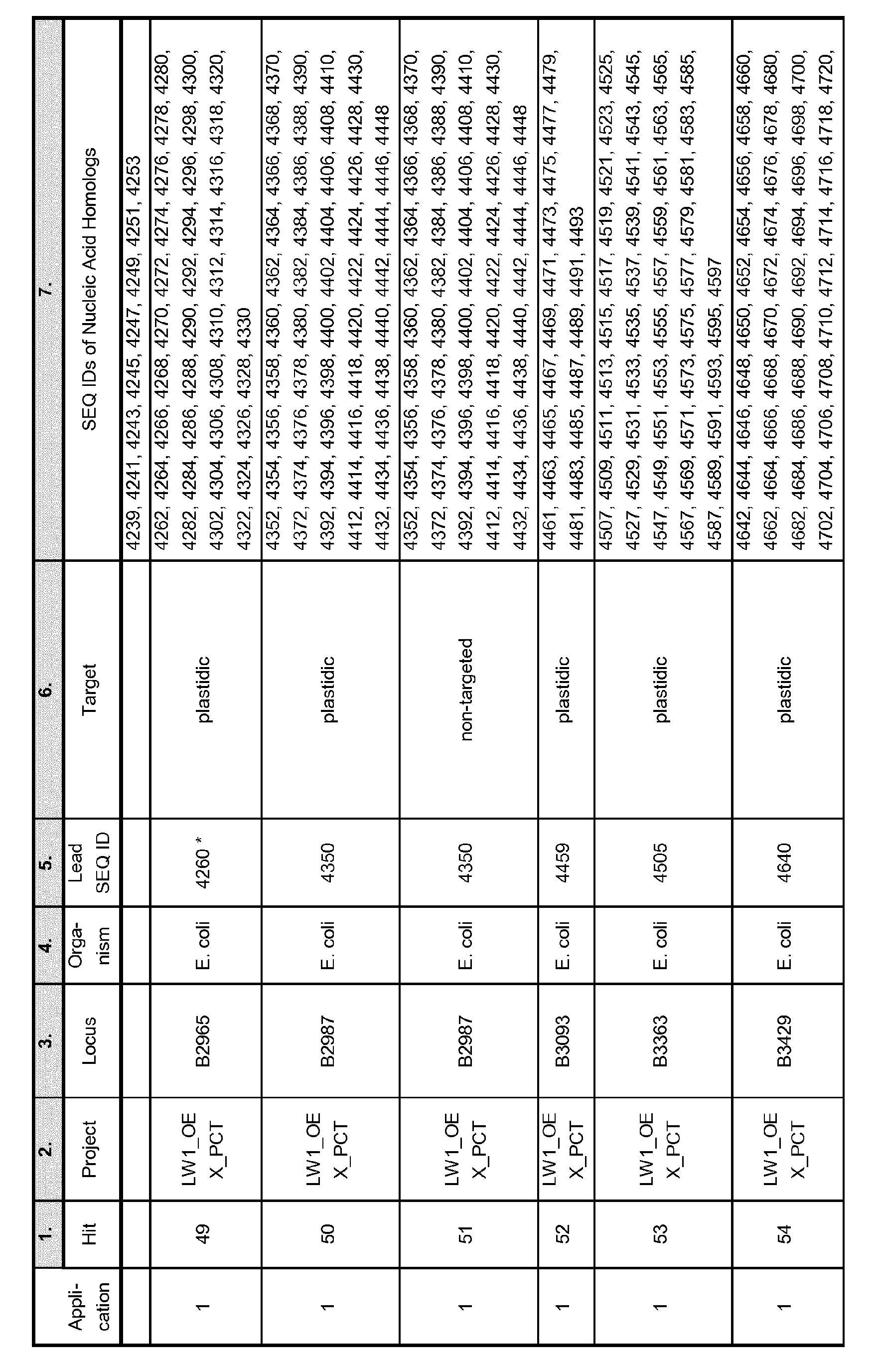

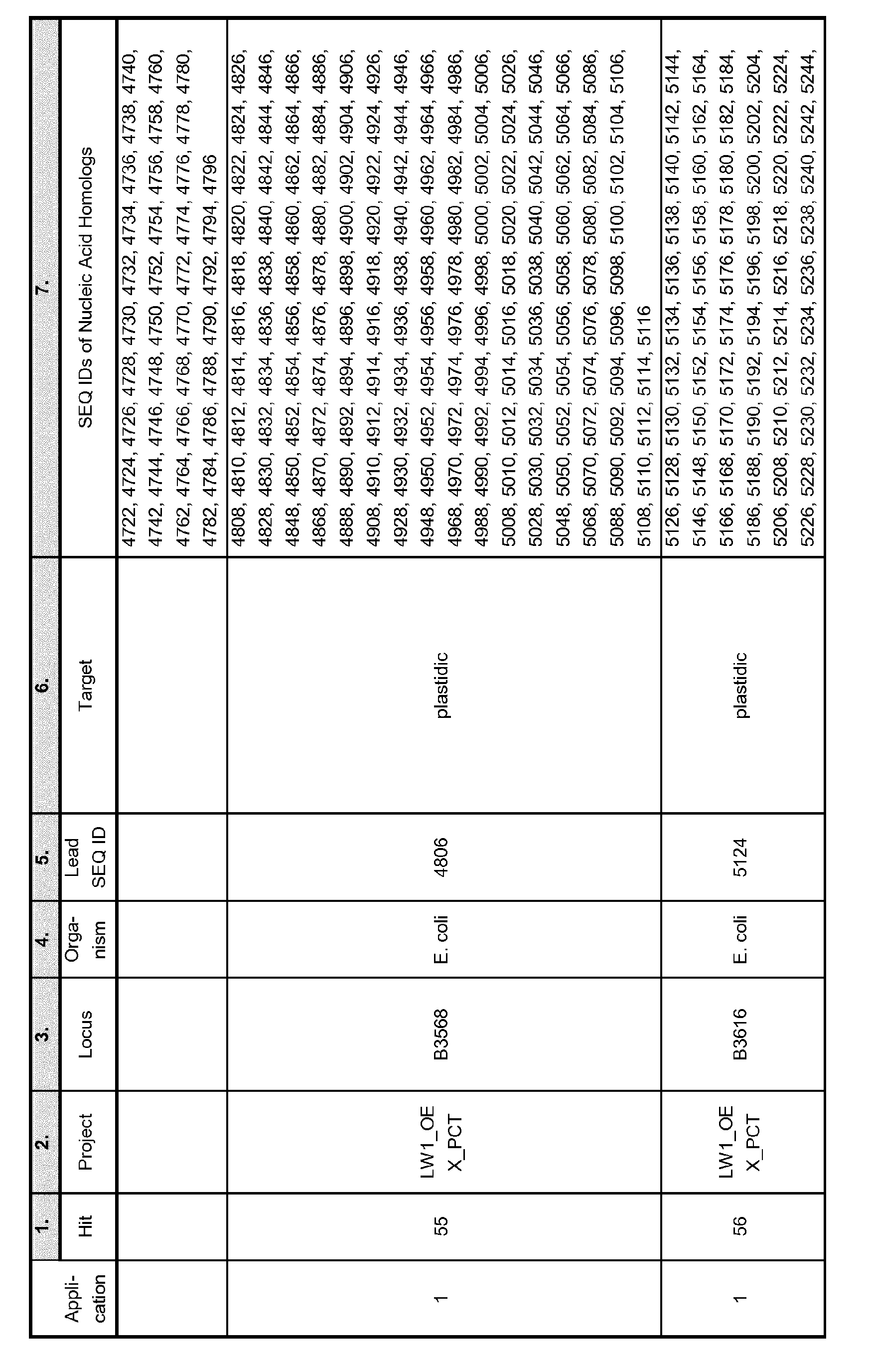

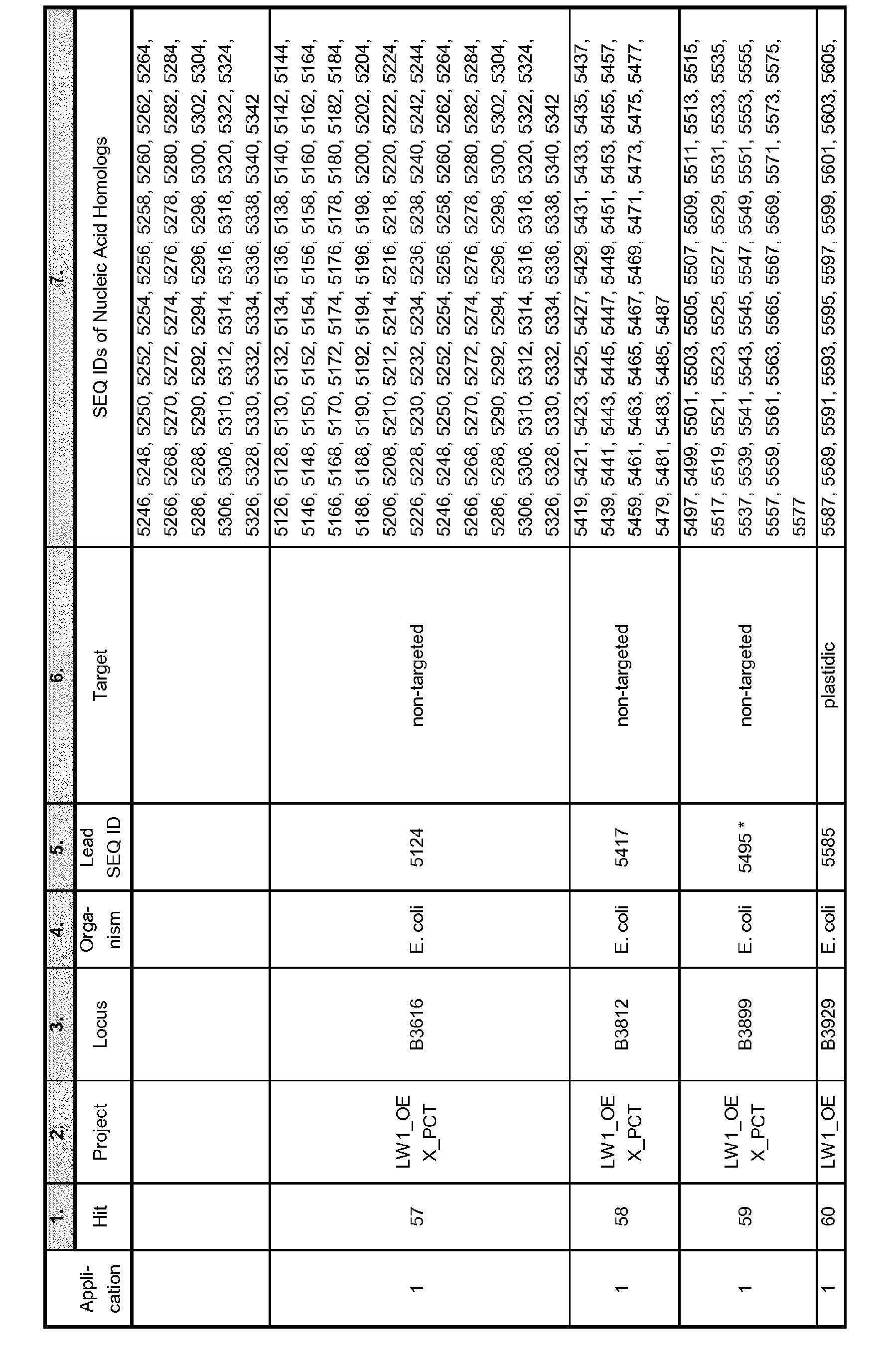

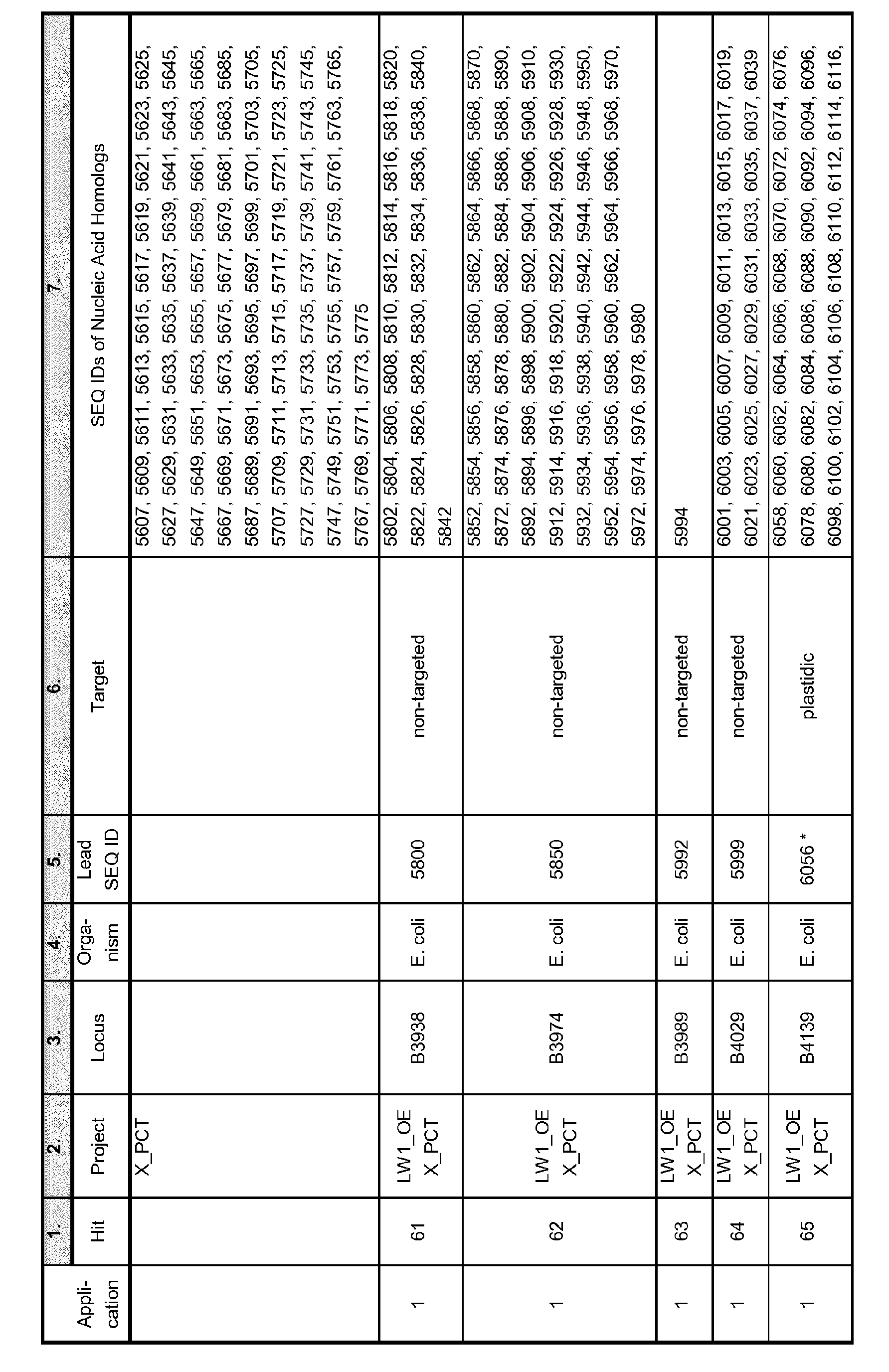

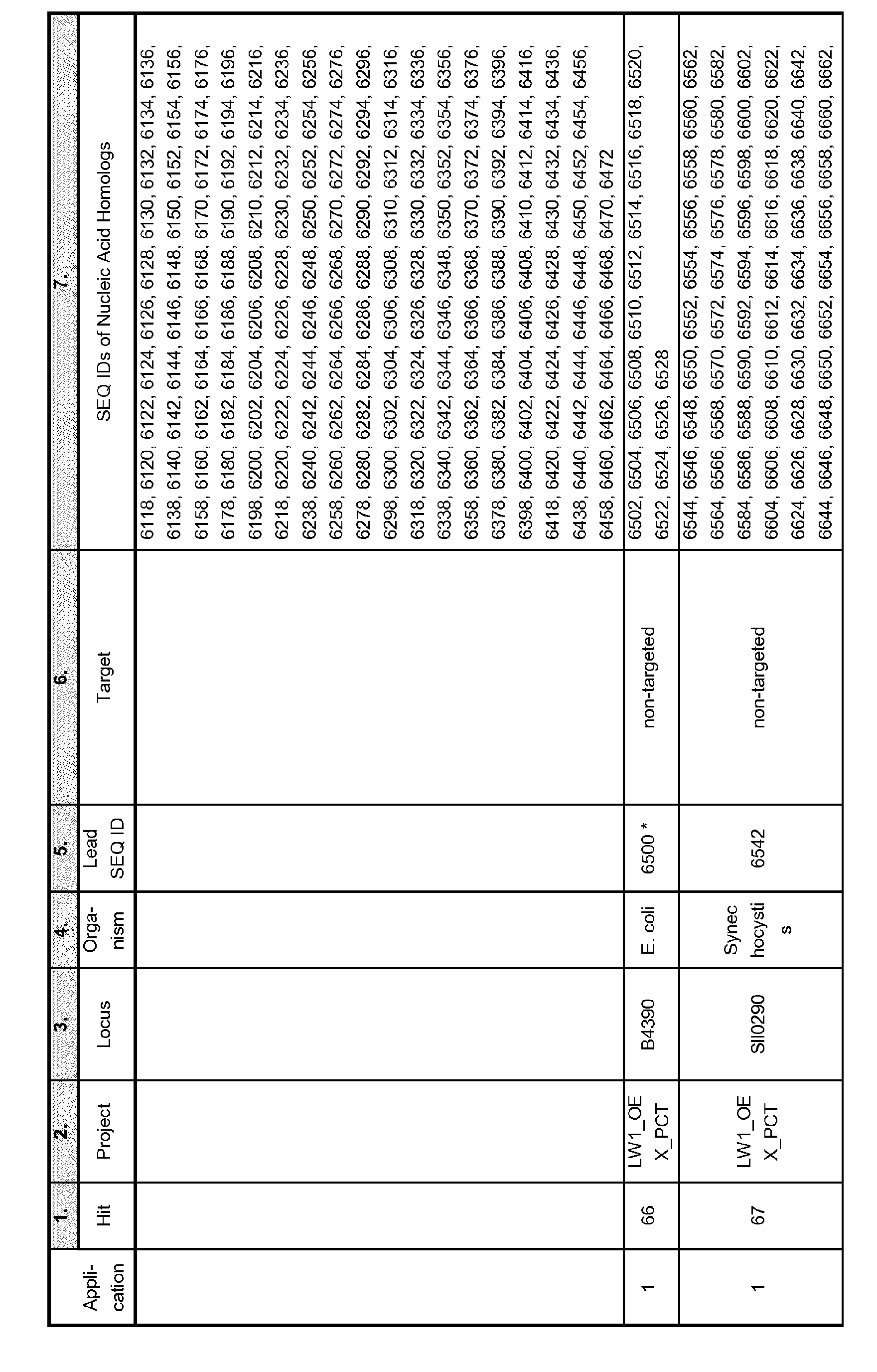

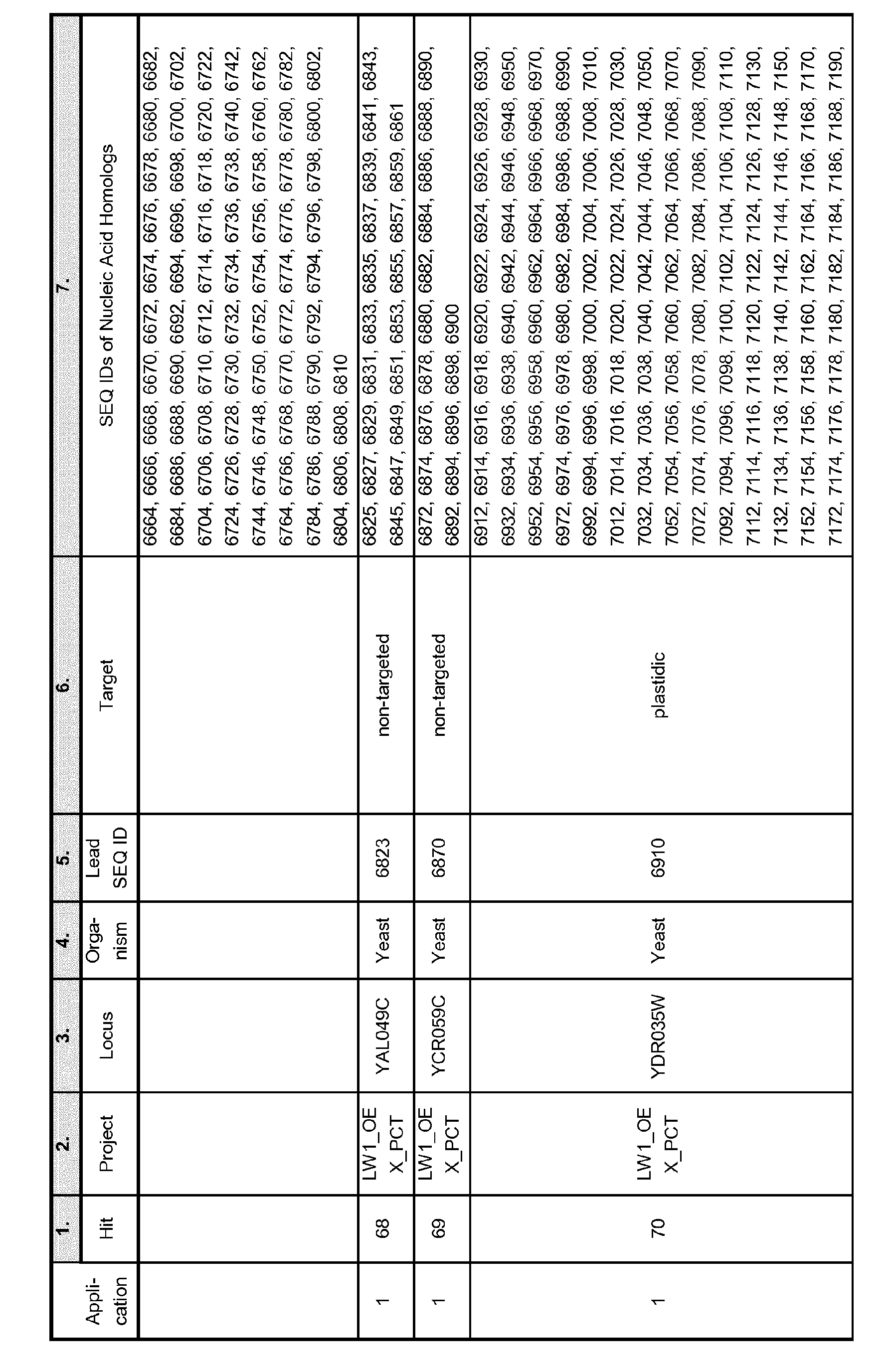

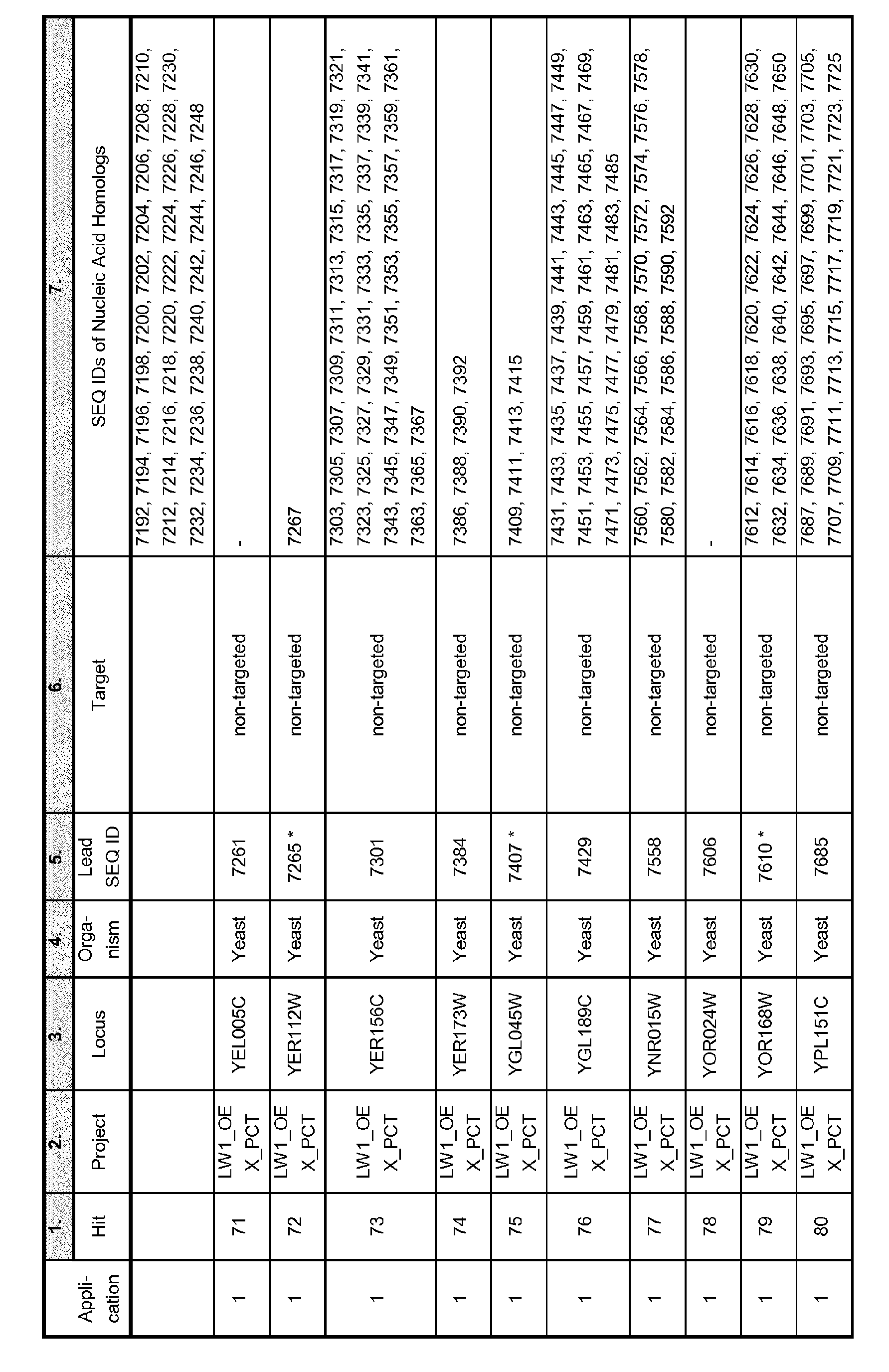

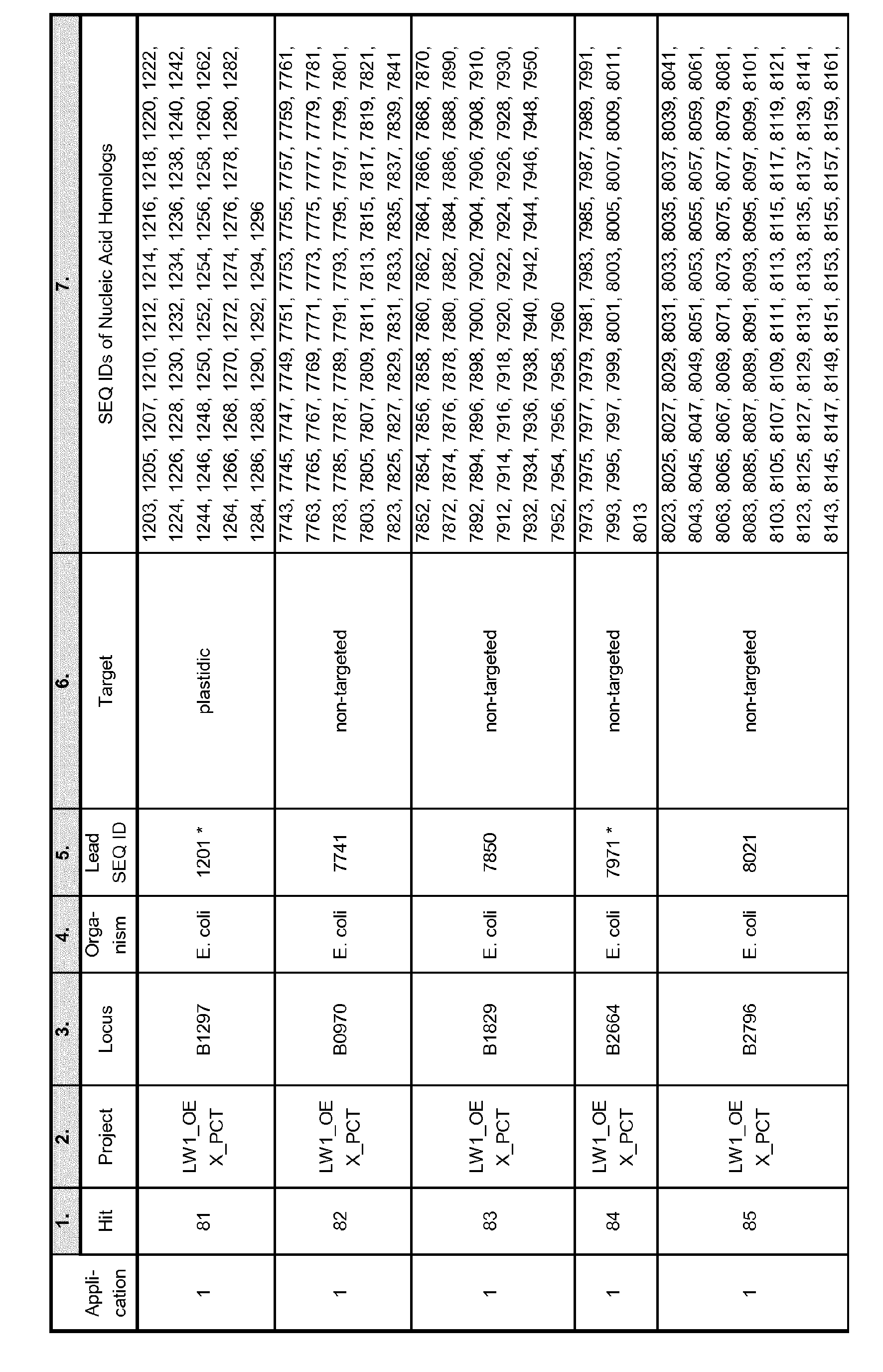

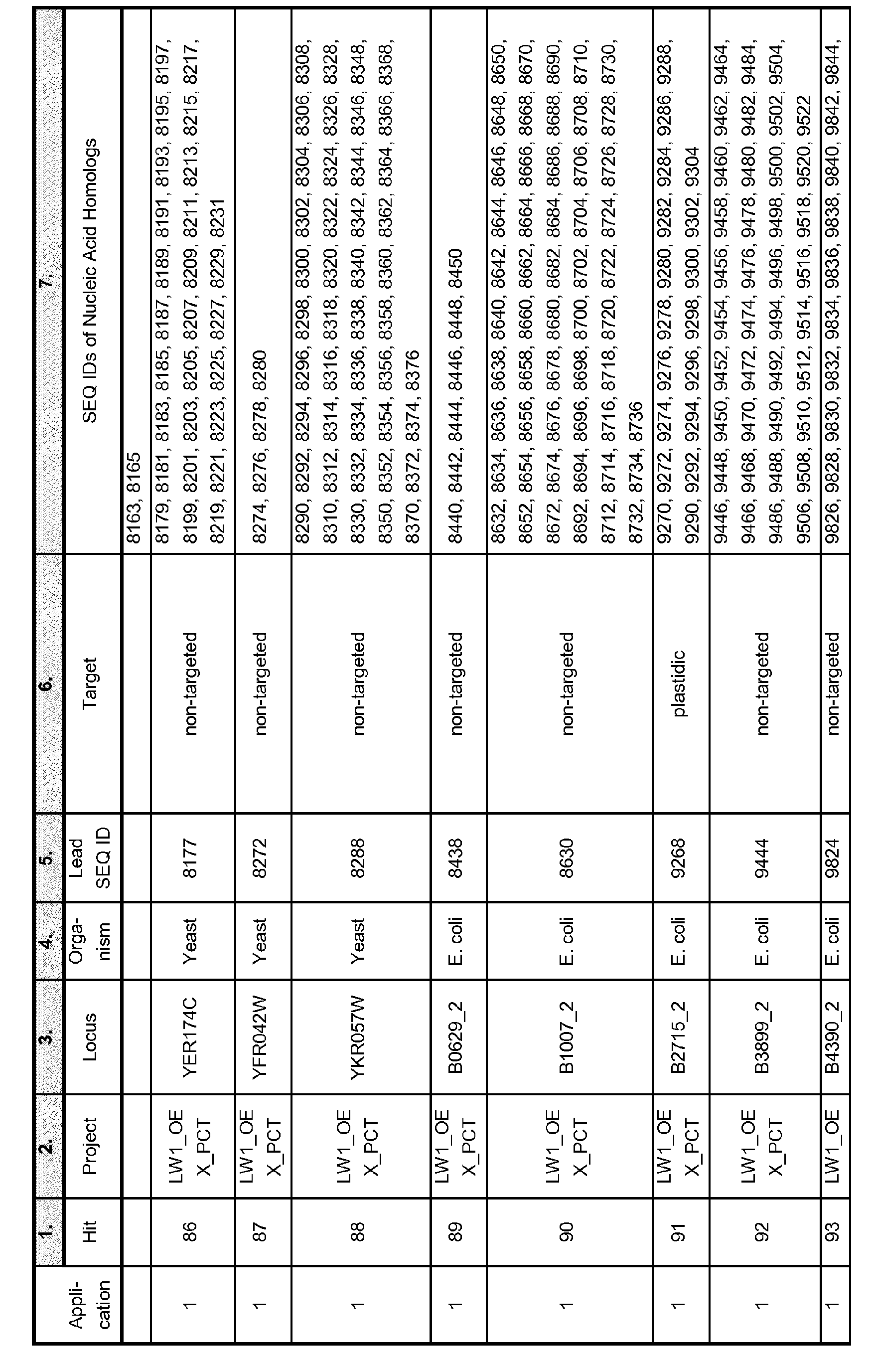

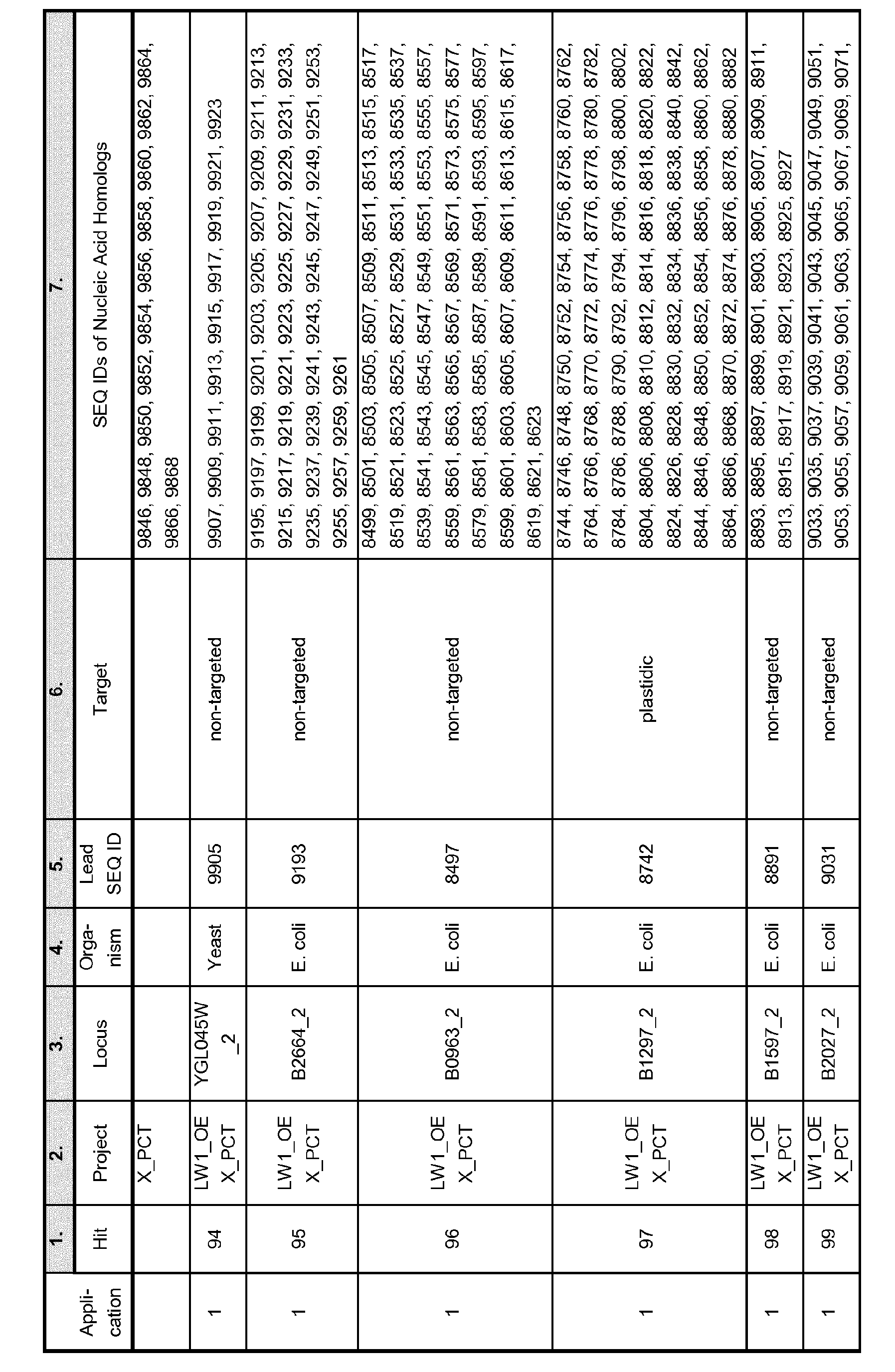

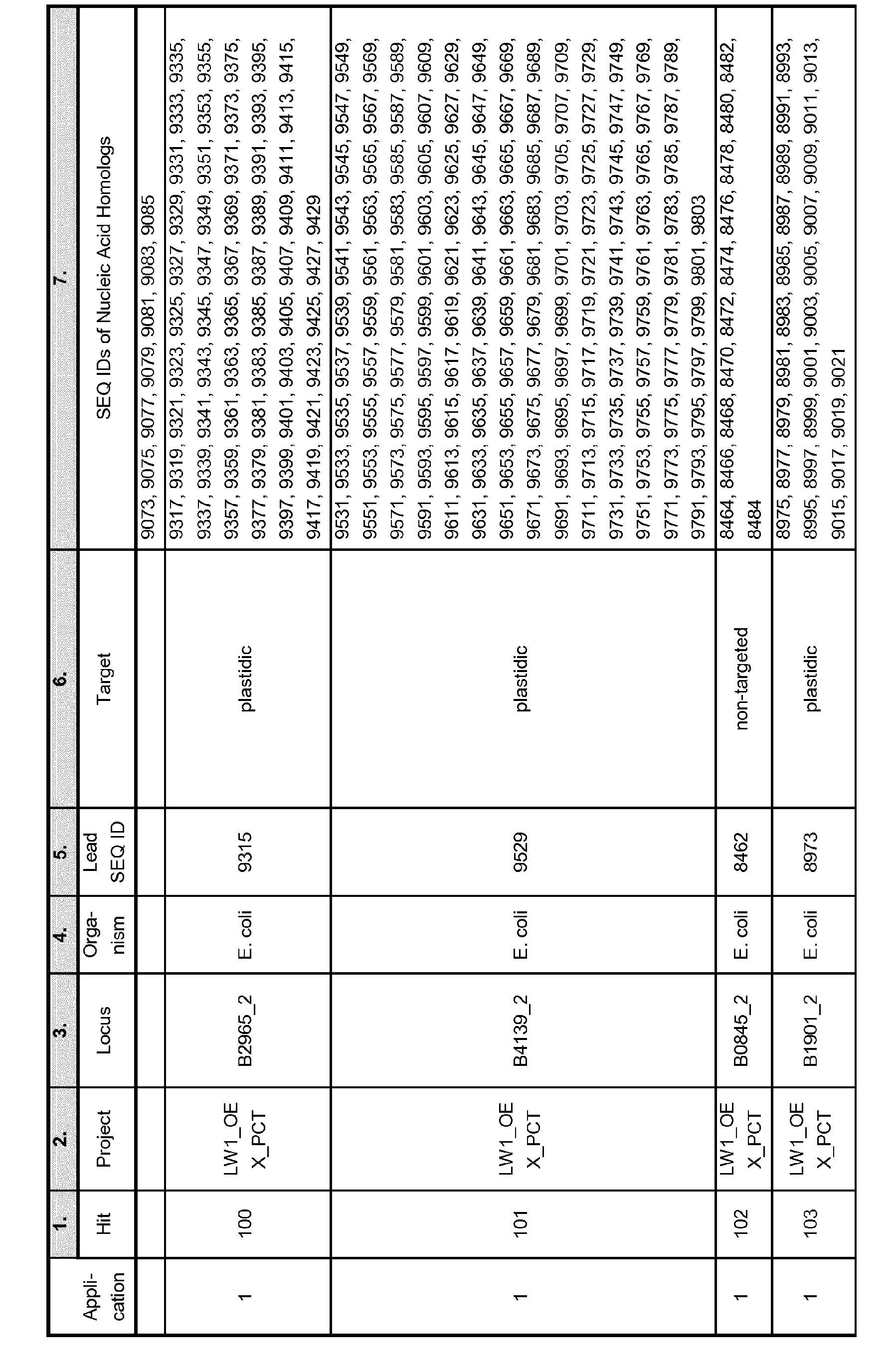

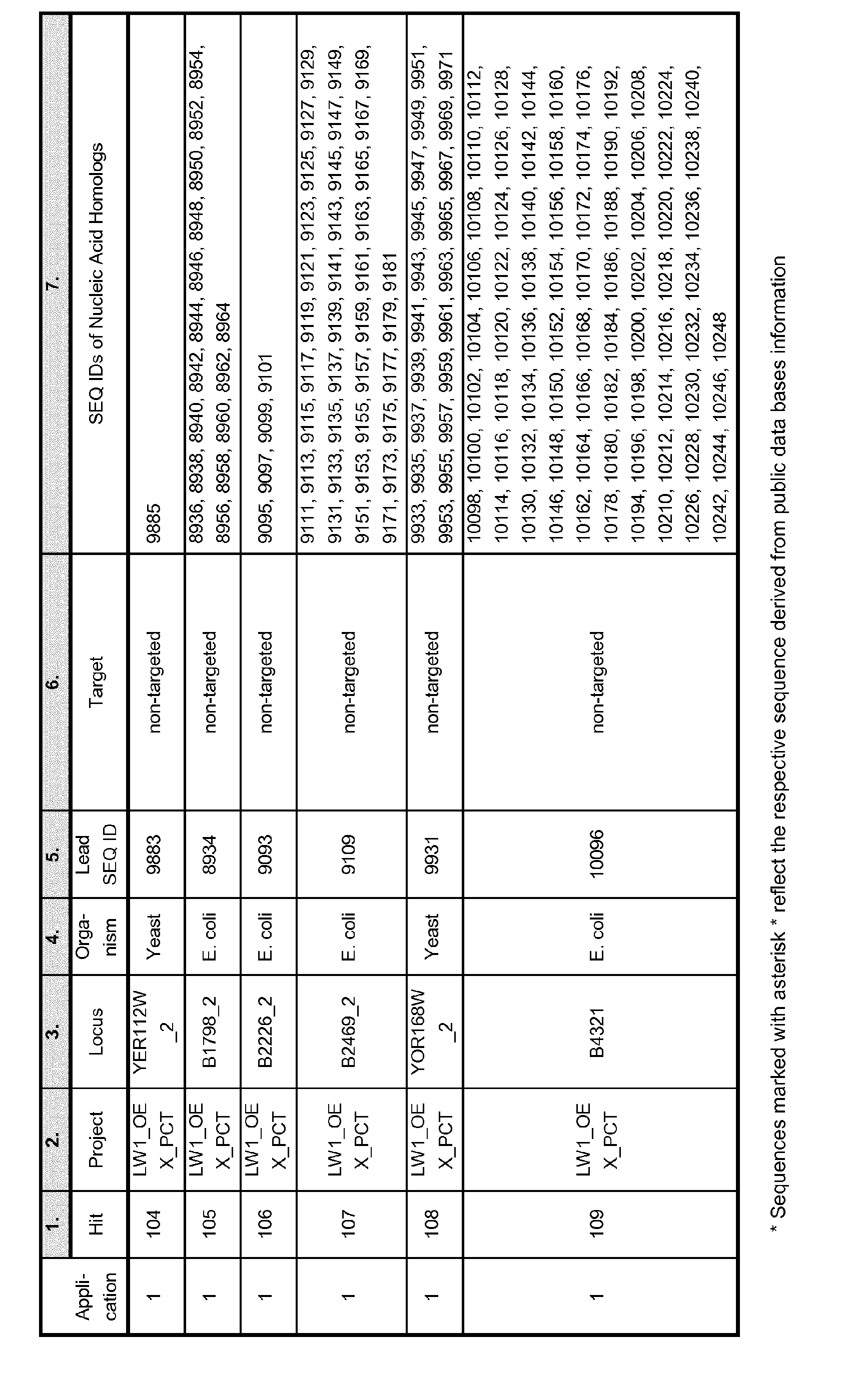

**Table IB: Nucleic acid sequence ID numbers**

| | **1.** | **2.** | **3.** | **4.** | **5.** | **6.** | **7.** |
|---|---|---|---|---|---|---|---|
| Application | Hit | Project | Locus | Organism | Lead SEQ ID | Target | SEQ IDs of Nucleic Acid Homologs |
| 1 | 1 | LW1_OE X_PCT | B0081 | E. coli | 38 | non-targeted | - |
| 1 | 2 | LW1_OE X_PCT | B0445 | E. coli | 54 | non-targeted | - |
| 1 | 3 | LW1_OE X_PCT | B0482 | E. coli | 70 | non-targeted | - |
| 1 | 4 | LW1_OE X_PCT | B0607 | E. coli | 89 | non-targeted | - |
| 1 | 5 | LW1_OE X_PCT | B0629 | E. coli | 143 * | non-targeted | - |
| 1 | 6 | LW1_OE X_PCT | B0631 | E. coli | 162 | non-targeted | - |
| 1 | 7 | LW1_OE X_PCT | B0697 | E. coli | 213 | non-targeted | - |
| 1 | 8 | LW1_OE X_PCT | B0753 | E. coli | 358 | non-targeted | - |
| 1 | 9 | LW1_OE X_PCT | B0813 | E. coli | 367 | non-targeted | - |
| 1 | 10 | LW1_OE X_PCT | B0845 | E. coli | 420 * | non-targeted | - |
| 1 | 11 | LW1_OE X_PCT | B0866 | E. coli | 455 | non-targeted | - |
| 1 | 12 | LW1_OE X_PCT | B0963 | E. coli | 535 * | non-targeted | - |
| 1 | 13 | LW1_OE X_PCT | B0975 | E. coli | 618 | non-targeted | - |
| 1 | 14 | LW1_OE X_PCT | B1007 | E. coli | 671 * | non-targeted | - |
| 1 | 15 | LW1_OE X_PCT | B1052 | E. coli | 764 | non-targeted | - |
| 1 | 16 | LW1_OE X_PCT | B1091 | E. coli | 768 | plastidic | 890, 892, 894 |
| 1 | 17 | LW1_OE X_PCT | B1161 | E. coli | 907 | non-targeted | - |
| 1 | 18 | LW1_OE X_PCT | B1186 | E. coli | 927 | non-targeted | - |
| 1 | 19 | LW1_OE X_PCT | B1291 | E. coli | 1009 | plastidic | - |
| 1 | 20 | LW1_OE X_PCT | B1294 | E. coli | 1154 | plastidic | - |
| 1 | 21 | LW1_OE X_PCT | B1423 | E. coli | 1308 | non-targeted | - |
| 1 | 22 | LW1_OE X_PCT | B1597 | E. coli | 1368 * | non-targeted | - |
| 1 | 23 | LW1_OE X_PCT | B1605 | E. coli | 1374 | non-targeted | - |
| 1 | 24 | LW1_OE X_PCT | B1704 | E. coli | 1507 | non-targeted | - |
| 1 | 25 | LW1_OE X_PCT | B1736 | E. coli | 1953 | plastidic | - |
| 1 | 26 | LW1_OE X_PCT | B1798 | E. coli | 2156 * | non-targeted | - |
| 1 | 27 | LW1_OE X_PCT | B1878 | E. coli | 2195 | non-targeted | - |
| 1 | 28 | LW1_OE X_PCT | B1901 | E. coli | 2219 * | plastidic | - |
| 1 | 29 | LW1_OE X_PCT | B1912 | E. coli | 2277 | plastidic | 2457, 2459, 2461, 10012 |
| 1 | 30 | LW1_OE X_PCT | B2027 | E. coli | 2470 * | non-targeted | - |
| 1 | 31 | LW1_OE X_PCT | B2039 | E. coli | 2493 | non-targeted | - |
| 1 | 32 | LW1_OE X_PCT | B2075 | E. coli | 2627 | non-targeted | - |
| 1 | 33 | LW1_OE X_PCT | B2153 | E. coli | 2858 | plastidic | - |
| 1 | 34 | LW1_OE X_PCT | B2194 | E. coli | 2942 | non-targeted | - |
| 1 | 35 | LW1_OE X_PCT | B2226 | E. coli | 2965 * | non-targeted | - |
| 1 | 36 | LW1_OE X_PCT | B2309 | E. coli | 2981 | plastidic | - |
| 1 | 37 | LW1_OE X_PCT | B2469 | E. coli | 3130 * | non-targeted | - |
| 1 | 38 | LW1_OE X_PCT | B2475 | E. coli | 3216 | non-targeted | - |
| 1 | 39 | LW1_OE X_PCT | B2482 | E. coli | 3335 | non-targeted | - |
| 1 | 40 | LW1_OE X_PCT | B2541 | E. coli | 3401 | non-targeted | 3555, 3557, 3559, 3561, 3563, 3565, 3567, 3569, 3571, 3573, 3575, 3577, 3579, 3581, 3583, 10016, 10018 |
| 1 | 41 | LW1_OE X_PCT | B2559 | E. coli | 3590 | plastidic | 3802, 3804, 3806, 3808, 3810, 3812, 3814, 3816, 3818, 3820, 3822, 3824 |
| 1 | 42 | LW1_OE X_PCT | B2605 | E. coli | 3831 | non-targeted | - |
| 1 | 43 | LW1_OE X_PCT | B2630 | E. coli | 3857 | non-targeted | - |
| 1 | 44 | LW1_OE X_PCT | B2678 | E. coli | 3861 | plastidic | - |
| 1 | 45 | LW1_OE X_PCT | B2715 | E. coli | 4022 * | plastidic | - |
| 1 | 46 | LW1_OE X_PCT | B2776 | E. coli | 4059 | non-targeted | - |
| 1 | 47 | LW1_OE X_PCT | B2791 | E. coli | 4076 | non-targeted | - |
| 1 | 48 | LW1_OE X_PCT | B2912 | E. coli | 4157 | non-targeted | - |
| 1 | 49 | LW1_OE X_PCT | B2965 | E. coli | 4260 * | plastidic | - |
| 1 | 50 | LW1_OE X_PCT | B2987 | E. coli | 4350 | plastidic | - |
| 1 | 51 | LW1_OE X_PCT | B2987 | E. coli | 4350 | non-targeted | - |
| 1 | 52 | LW1_OE X_PCT | B3093 | E. coli | 4459 | plastidic | - |
| 1 | 53 | LW1_OE X_PCT | B3363 | E. coli | 4505 | plastidic | 4599, 4601, 4603, 4605, 4607, 4609, 4611, 4613, 4615, 4617, 4619, 4621, 4623, 4625, 4627, 4629, 4631, 4633, 10022, 10024, 10026, 10028, 10030, 10032, 10034, 10036 |
| 1 | 54 | LW1_OE X_PCT | B3429 | E. coli | 4640 | plastidic | - |
| 1 | 55 | LW1_OE X_PCT | B3568 | E. coli | 4806 | plastidic | - |
| 1 | 56 | LW1_OE X_PCT | B3616 | E. coli | 5124 | plastidic | 5344, 5346, 5348, 5350, 5352, 5354, 5356, 5358, 5360, 5362, 5364, 5366, 5368, 5370, 5372, 5374, 5376, 5378, 5380, 5382, 5384, 5386, 5388, 5390, 5392, 5394, 5396, 5398, 5400, 5402, 5404, 5406, 5408, 5410, 10040, 10042, 10044, 10046, 10048, 10050, 10052, 10054, 10056 |
| 1 | 57 | LW1_OE X_PCT | B3616 | E. coli | 5124 | non-targeted | 5344, 5346, 5348, 5350, 5352, 5354, 5356, 5358, 5360, 5362, 5364, 5366, 5368, 5370, 5372, 5374, 5376, 5378, 5380, 5382, 5384, 5386, 5388, 5390, 5392, 5394, 5396, 5398, 5400, 5402, 5404, 5406, 5408, 5410, 10040, 10042, 10044, 10046, 10048, 10050, 10052, 10054, 10056 |
| 1 | 58 | LW1_OE X_PCT | B3812 | E. coli | 5417 | non-targeted | - |
| 1 | 59 | LW1_OE X_PCT | B3899 | E. coli | 5495 * | non-targeted | - |
| 1 | 60 | LW1_OE X_PCT | B3929 | E. coli | 5585 | plastidic | 5777, 5779, 5781, 5783, 5785, 5787, 5789, 5791, 5793, 10060 |
| 1 | 61 | LW1_OE X_PCT | B3938 | E. coli | 5800 | non-targeted | - |
| 1 | 62 | LW1_OE X_PCT | B3974 | E. coli | 5850 | non-targeted | - |
| 1 | 63 | LW1_OE X_PCT | B3989 | E. coli | 5992 | non-targeted | - |
| 1 | 64 | LW1_OE X_PCT | B4029 | E. coli | 5999 | non-targeted | - |
| 1 | 65 | LW1_OE X_PCT | B4139 | E. coli | 6056 * | plastidic | 6474, 6476, 6478, 6480, 6482, 6484, 6486, 10068 |
| 1 | 66 | LW1_OE X_PCT | B4390 | E. coli | 6500 * | non-targeted | - |
| 1 | 67 | LW1_OE X_PCT | SII0290 | Synec hocysti s | 6542 | non-targeted | - |
| 1 | 68 | LW1_OE X_PCT | YAL049C | Yeast | 6823 | non-targeted | 10072, 10074 |
| 1 | 69 | LW1_OE X_PCT | YCR059C | Yeast | 6870 | non-targeted | - |
| 1 | 70 | LW1_OE X_PCT | YDR035W | Yeast | 6910 | plastidic | - |
| 1 | 71 | LW1_OE X_PCT | YEL005C | Yeast | 7261 | non-targeted | - |
| 1 | 72 | LW1_OE X_PCT | YER112W | Yeast | 7265 * | non-targeted | 7269, 7271, 7273, 7275, 7277, 7279, 7281, 7283, 7285, 7287, 7289, 7291, 7293, 7295 |
| 1 | 73 | LW1_OE X_PCT | YER156C | Yeast | 7301 | non-targeted | 7369, 7371, 7373, 10078 |
| 1 | 74 | LW1_OE X_PCT | YER173W | Yeast | 7384 | non-targeted | - |
| 1 | 75 | LW1_OE X_PCT | YGL045W | Yeast | 7407 * | non-targeted | - |
| 1 | 76 | LW1_OE X_PCT | YGL189C | Yeast | 7429 | non-targeted | 7487, 7489, 7491, 7493, 7495, 7497, 7499, 7501, 7503, 7505, 7507, 7509, 7511, 7513, 7515, 7517, 7519, 7521, 7523, 7525, 7527, 7529, 7531, 7533, 7535, 7537, 7539, 7541, 7543, 7545, 7547, 7549, 7551, 10086, 10088, 10090 |
| 1 | 77 | LW1_OE X_PCT | YNR015W | Yeast | 7558 | non-targeted | 7594, 7596, 10094 |
| 1 | 78 | LW1_OE X_PCT | YOR024W | Yeast | 7606 | non-targeted | - |
| 1 | 79 | LW1_OE X_PCT | YOR168W | Yeast | 7610 * | non-targeted | 7652, 7654, 7656, 7658, 7660, 7662, 7664, 7666, 7668, 7670, 7672 |
| 1 | 80 | LW1_OE X_PCT | YPL151C | Yeast | 7685 | non-targeted | 7727, 7729, 7731 |
| 1 | 81 | LW1_OE X_PCT | B1297 | E. coli | 1201 * | plastidic | - |
| 1 | 82 | LW1_OE X_PCT | B0970 | E. coli | 7741 | non-targeted | 10008 |
| 1 | 83 | LW1_OE X_PCT | B1829 | E. coli | 7850 | non-targeted | - |
| 1 | 84 | LW1_OE X_PCT | B2664 | E. coli | 7971 * | non-targeted | - |
| 1 | 85 | LW1_OE X_PCT | B2796 | E. coli | 8021 | non-targeted | - |
| 1 | 86 | LW1_OE X_PCT | YER174C | Yeast | 8177 | non-targeted | 8233, 8235, 8237, 8239, 8241, 8243, 8245, 8247, 8249, 8251, 8253, 8255, 8257, 8259, 8261, 8263, 8265, 10082 |
| 1 | 87 | LW1_OE X_PCT | YFR042W | Yeast | 8272 | non-targeted | - |
| 1 | 88 | LW1_OE X_PCT | YKR057W | Yeast | 8288 | non-targeted | 8378, 8380, 8382, 8384, 8386, 8388, 8390, 8392, 8394, 8396, 8398, 8400, 8402, 8404, 8406, 8408, 8410, 8412, 8414, 8416, 8418, 8420, 8422, 8424 |
| 1 | 89 | LW1_OE X_PCT | B0629_2 | E. coli | 8438 | non-targeted | - |
| 1 | 90 | LW1_OE X_PCT | B1007_2 | E. coli | 8630 | non-targeted | - |
| 1 | 91 | LW1_OE X_PCT | B2715_2 | E. coli | 9268 | plastidic | - |
| 1 | 92 | LW1_OE X_PCT | B3899_2 | E. coli | 9444 | non-targeted | - |
| 1 | 93 | LW1_OE X_PCT | B4390_2 | E. coli | 9824 | non-targeted | - |
| 1 | 94 | LW1_OE X_PCT | YGL045W_2 | Yeast | 9905 | non-targeted | - |
| 1 | 95 | LW1_OE X_PCT | B2664_2 | E. coli | 9193 | non-targeted | - |
| 1 | 96 | LW1_OE X_PCT | B0963_2 | E. coli | 8497 | non-targeted | - |
| 1 | 97 | LW1_OE X_PCT | B1297_2 | E. coli | 8742 | plastidic | - |
| 1 | 98 | LW1_OE X_PCT | B1597_2 | E. coli | 8891 | non-targeted | - |
| 1 | 99 | LW1_OE X_PCT | B2027_2 | E. coli | 9031 | non-targeted | - |
| 1 | 100 | LW1_OE X_PCT | B2965_ 2 | E. coli | 9315 | plastidic | - |
| 1 | 101 | LW1_OE X_PCT | B4139_2 | E. coli | 9529 | plastidic | 9805, 9807, 9809, 9811, 10064 |
| 1 | 102 | LW1_OE X_PCT | B0845_2 | E. coli | 8462 | non-targeted | - |
| 1 | 103 | LW1_OE X_PCT | B1901_2 | E. coli | 8973 | plastidic | - |
| 1 | 104 | LW1_OE X_PCT | YER112W_2 | Yeast | 9883 | non-targeted | 9887, 9889, 9891, 9893, 9895, 9897, 9899 |
| 1 | 105 | LW1_OE X_PCT | B1798_2 | E. coli | 8934 | non-targeted | - |
| 1 | 106 | LW1_OE X_PCT | B2226_2 | E. coli | 9093 | non-targeted | - |
| 1 | 107 | LW1_OE X_PCT | B2469_2 | E. coli | 9109 | non-targeted | - |
| 1 | 108 | LW1_OE X_PCT | YOR168W_2 | Yeast | 9931 | non-targeted | 9973, 9975, 9977, 9979, 9981, 9983, 9985, 9987, 9989, 9991, 9993 |
| 1 | 109 | LW1_OE X_PCT | B4321 | E. coli | 10096 | non-targeted | - |

| | | | | | | | |
|---|---|---|---|---|---|---|---|
| * Sequences marked with asterisk * reflect the respective sequence derived from public data bases information | | | | | | | |

**Table IIA: Amino acid sequence ID numbers**

| | **1.** | **2.** | **3.** | **4.** | **5.** | **6.** | **7.** |
|---|---|---|---|---|---|---|---|
| Application | Hit | Project | Locus | Organism | Lead SEQ ID | Target | SEQ IDs of Polypeptide Homologs |
| 1 | 1 | LW1_OE X_PCT | B0081 | E. coli | 39 | non-targeted | 41, 43, 45, 47 |
| 1 | 2 | LW1_OE X_PCT | B0445 | E. coli | 55 | non-targeted | 57, 59, 61, 63 |
| 1 | 3 | LW1_OE X_PCT | B0482 | E. coli | 71 | non-targeted | 73, 75, 77, 79, 81 |
| 1 | 4 | LW1_OE X_PCT | B0607 | E. coli | 90 | non-targeted | 92, 94, 96, 98, 100, 102, 104, 106, 108, 110, 112, 114, 116, 118, 120, 122, 124, 126, 128, 130, 132, 134, 136, 138 |
| 1 | 5 | LW1_OE X_PCT | B0629 | E. coli | 144 * | non-targeted | 146, 148, 150, 152 |
| 1 | 6 | LW1_OE X_PCT | B0631 | E. coli | 163 | non-targeted | 165, 167, 169, 171, 173, 175, 177, 179, 181, 183, 185, 187, 189, 191, 193, 195, 197, 199, 201, 203, 205, 207 |
| 1 | 7 | LW1_OE X_PCT | B0697 | E. coli | 214 | non-targeted | 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340 |
| 1 | 8 | LW1_OE X_PCT | B0753 | E. coli | 359 | non-targeted | 361 |
| 1 | 9 | LW1_OE X_PCT | B0813 | E. coli | 368 | non-targeted | 370, 372, 374, 376, 378, 380, 382, 384, 386, 388, 390, 392, 394, 396, 398, 400, 402, 404, 406, 408, 410, 412 |
| 1 | 10 | LW1_OE X_PCT | B0845 | E. coli | 421 * | non-targeted | 423, 425, 427, 429, 431, 433, 435, 437, 439, 441, 443 |
| 1 | 11 | LW1_OE X_PCT | B0866 | E. coli | 456 | non-targeted | 458, 460, 462, 464, 466, 468, 470, 472, 474, 476, 478, 480, 482, 484, 486, 488, 490, 492, 494, 496, 498, 500, 502, 504, 506, 508, 510, 512, 514, 516, 518, 520, 522, 524, 526, 528 |
| 1 | 12 | LW1_OE X_PCT | B0963 | E. coli | 536 * | non-targeted | 538, 540, 542, 544, 546, 548, 550, 552, 554, 556, 558_{,} 560_{,} 562, 564, 566, 568, 570, 572, 574, 576, 578, 580, 582, 584, 586, 588, 590, 592, 594, 596, 598, 600, 602, 604, 606, 608, 610, 612 |
| 1 | 13 | LW1_OE X_PCT | B0975 | E. coli | 619 | non-targeted | 621, 623, 625, 627, 629, 631, 633, 635, 637, 639, 641, 643, 645, 647, 649, 651, 653, 655, 657, 659, 661, 663, 665 |
| 1 | 14 | LW1_OE X_PCT | B1007 | E. coli | 672 * | non-targeted | 674, 676, 678, 680, 682, 684, 686, 688, 690, 692, 694, 696, 698, 700, 702, 704, 706, 708, 710, 712, 714, 716, 718, 720, 722, 724, 726, 728, 730, 732, 734, 736, 738, 740, 742, 744, 746, 748, 750, 752, 754, 756, 758 |
| 1 | 15 | LW1_OE X_PCT | B1052 | E. coli | 765 | non-targeted | - |
| 1 | 16 | LW1_OE X_PCT | B1091 | E. coli | 769 | plastidic | 771, 773, 775, 777, 779, 781, 783, 785, 787, 789, 791, 793, 795, 797, 799, 801, 803, 805, 807, 809, 811, 813, 815, 817, 819, 821, 823, 825, 827, 829, 831, 833, 835, 837, 839, 841, 843, 845, 847, 849, 851, 853, 855, 857, 859, 861, 863, 865, 867, 869, 871, 873, 875, 877, 879, 881, 883, 885, 887, 889 |
| 1 | 17 | LW1_OE X_PCT | B1161 | E. coli | 908 | non-targeted | 910, 912, 914, 916, 918, 920, 922 |
| 1 | 18 | LW1_OE X_PCT | B1186 | E. coli | 928 | non-targeted | 930, 932, 934, 936, 938, 940, 942, 944, 946, 948, 950, 952, 954, 956, 958, 960, 962, 964, 966, 968, 970, 972, 974, 976, 978, 980, 982, 984, 986, 988, 990, 992, 994, 996 |
| 1 | 19 | LW1_OE X_PCT | B1291 | E. coli | 1010 | plastidic | 1012, 1014, 1016, 1018, 1020, 1022, 1024, 1026, 1028, 1030, 1032, 1034, 1036, 1038, 1040, 1042, 1044, 1046, 1048, 1050, 1052, 1054, 1056, 1058, 1060, 1062, 1064, 1066, 1068, 1070, 1072, 1074, 1076, 1078, 1080, 1082, 1084, 1086, 1088, 1090, 1092, 1094, 1096, 1098, 1100, 1102, 1104, 1106, 1108, 1110, 1112, 1114, 1116, 1118, 1120, 1122, 1124, 1126, 1128, 1130, 1132, 1134, 1136, 1138, 1140, 1142, 1144 |
| 1 | 20 | LW1_OE X_PCT | B1294 | E. coli | 1155 | plastidic | 1157, 1159, 1161, 1163, 1165, 1167, 1169, 1171, 1173, 1175, 1177, 1179, 1181, 1183, 1185, 1187 |
| 1 | 21 | LW1_OE X_PCT | B1423 | E. coli | 1309 | non-targeted | 1311, 1313, 1315, 1317, 1319, 1321, 1323, 1325, 1327, 1329, 1331, 1333, 1335, 1337, 1339, 1341, 1343, 1345, 1347, 1349, 1351, 1353 |
| 1 | 22 | LW1_OE X_PCT | B1597 | E. coli | 1369 * | non-targeted | 1371 |
| 1 | 23 | LW1_OE X_PCT | B1605 | E. coli | 1375 | non-targeted | 1377, 1379, 1381, 1383, 1385, 1387, 1389, 1391, 1393, 1395, 1397, 1399, 1401, 1403, 1405, 1407, 1409, 1411, 1413, 1415, 1417, 1419, 1421, 1423, 1425, 1427, 1429, 1431, 1433, 1435, 1437, 1439, 1441, 1443, 1445, 1447, 1449, 1451, 1453, 1455, 1457, 1459, 1461, 1463, 1465, 1467, 1469, 1471, 1473, 1475, 1477, 1479, 1481, 1483, 1485, 1487, 1489, 1491, 1493, 1495 |
| 1 | 24 | LW1_OE X_PCT | B1704 | E. coli | 1508 | non-targeted | 1510, 1512, 1514, 1516, 1518, 1520, 1522, 1524, 1526, 1528, 1530, 1532, 1534, 1536, 1538, 1540, 1542, 1544, 1546, 1548, 1550, 1552, 1554, 1556, 1558, 1560, 1562, 1564, 1566, 1568, 1570, 1572, 1574, 1576, 1578, 1580, 1582, 1584, 1586, 1588, 1590, 1592, 1594, 1596, 1598, 1600, 1602, 1604, 1606, 1608, 1610, 1612, 1614, 1616, 1618, 1620, 1622, 1624, 1626, 1628, 1630, 1632, 1634, 1636, 1638, 1640, 1642, 1644, 1646, 1648, 1650, 1652, 1654, 1656, 1658, 1660, 1662, 1664, 1666, 1668, 1670, 1672, 1674, 1676, 1678, 1680, 1682, 1684, 1686, 1688, 1690, 1692, 1694, 1696, 1698, 1700, 1702, 1704, 1706, 1708, 1710, 1712, 1714, 1716, 1718, 1720, 1722, 1724, 1726, 1728, 1730, 1732, 1734, 1736, 1738, 1740, 1742, 1744, 1746, 1748, 1750, 1752, 1754, 1756, 1758, 1760, 1762, 1764, 1766, 1768, 1770, 1772, 1774, 1776, 1778, 1780, 1782, 1784, 1786, 1788, 1790, 1792, 1794, 1796, 1798, 1800, 1802, 1804, 1806, 1808, 1810, 1812, 1814, 1816, 1818, 1820, 1822, 1824, 1826, 1828, 1830, 1832, 1834, 1836, 1838, 1840, 1842, 1844, 1846, 1848, 1850, 1852, 1854, 1856, 1858, 1860, 1862, 1864, 1866, 1868, 1870, 1872, 1874, 1876, 1878, 1880, 1882, 1884, 1886, 1888, 1890, 1892, 1894, 1896, 1898, 1900, 1902, 1904, 1906, 1908, 1910, 1912, 1914, 1916, 1918, 1920, 1922, 1924, 1926, 1928, 1930, 1932, 1934, 1936, 1938, 1940 |
| 1 | 25 | LW1_OE X_PCT | B1736 | E. coli | 1954 | plastidic | 1956, 1958, 1960, 1962, 1964, 1966, 1968, 1970, 1972, 1974, 1976, 1978, 1980, 1982, 1984, 1986, 1988, 1990, 1992, 1994, 1996, 1998, 2000, 2002, 2004, 2006, 2008, 2010, 2012, 2014, 2016, 2018, 2020, 2022, 2024, 2026, 2028, 2030, 2032, 2034, 2036, 2038, 2040, 2042, 2044, 2046, 2048, 2050, 2052, 2054, 2056, 2058, 2060, 2062, 2064, 2066, 2068, 2070, 2072, 2074, 2076, 2078, 2080, 2082, 2084, 2086, 2088, 2090, 2092, 2094, 2096, 2098, 2100, 2102, 2104, 2106, 2108, 2110, 2112, 2114, 2116, 2118, 2120, 2122, 2124, 2126, 2128, 2130, 2132, 2134, 2136, 2138, 2140, 2142, 2144, 2146, 2148, 2150 |
| 1 | 26 | LW1_OE X_PCT | B1798 | E. coli | 2157 * | non-targeted | 2159, 2161, 2163, 2165, 2167, 2169, 2171, 2173, 2175, 2177, 2179, 2181, 2183, 2185, 2187 |
| 1 | 27 | LW1_OE X_PCT | B1878 | E. coli | 2196 | non-targeted | 2198, 2200, 2202, 2204, 2206, 2208, 2210, 2212 |
| 1 | 28 | LW1_OE X_PCT | B1901 | E. coli | 2220 * | plastidic | 2222, 2224, 2226, 2228, 2230, 2232, 2234, 2236, 2238, 2240, 2242, 2244, 2246, 2248, 2250, 2252, 2254, 2256, 2258, 2260, 2262, 2264, 2266, 2268 |
| 1 | 29 | LW1_OE X_PCT | B1912 | E. coli | 2278 | plastidic | 2280, 2282, 2284, 2286, 2288, 2290, 2292, 2294, 2296, 2298, 2300, 2302, 2304, 2306, 2308, 2310, 2312, 2314, 2316, 2318, 2320, 2322, 2324, 2326, 2328, 2330, 2332, 2334, 2336, 2338, 2340, 2342, 2344, 2346, 2348, 2350, 2352, 2354, 2356, 2358, 2360, 2362, 2364, 2366, 2368, 2370, 2372, 2374, 2376, 2378, 2380, 2382, 2384, 2386, 2388, 2390, 2392, 2394, 2396, 2398, 2400, 2402, 2404, 2406, 2408, 2410, 2412, 2414, 2416, 2418, 2420, 2422, 2424, 2426, 2428, 2430, 2432, 2434, 2436, 2438, 2440, 2442, 2444, 2446, 2448, 2450, 2452, 2454, 2456 |
| 1 | 30 | LW1_OE X_PCT | B2027 | E. coli | 2471 * | non-targeted | 2473, 2475, 2477, 2479, 2481 |
| 1 | 31 | LW1_OE X_PCT | B2039 | E. coli | 2494 | non-targeted | 2496, 2498, 2500, 2502, 2504, 2506, 2508, 2510, 2512, 2514, 2516, 2518, 2520, 2522, 2524, 2526, 2528, 2530, 2532, 2534, 2536, 2538, 2540, 2542, 2544, 2546, 2548, 2550, 2552, 2554, 2556, 2558, 2560, 2562, 2564, 2566, 2568, 2570, 2572, 2574, 2576, 2578, 2580, 2582, 2584, 2586, 2588, 2590, 2592, 2594, 2596, 2598, 2600, 2602, 2604, 2606, 2608, 2610, 2612, 2614, 2616, 2618 |
| 1 | 32 | LW1_OE X_PCT | B2075 | E. coli | 2628 | non-targeted | 2630, 2632, 2634, 2636, 2638, 2640, 2642, 2644, 2646, 2648, 2650, 2652, 2654, 2656, 2658, 2660, 2662, 2664, 2666, 2668, 2670, 2672, 2674, 2676, 2678, 2680, 2682, 2684, 2686, 2688, 2690, 2692, 2694, 2696, 2698, 2700, 2702, 2704, 2706, 2708, 2710, 2712, 2714, 2716, 2718, 2720, 2722, 2724, 2726, 2728, 2730, 2732, 2734, 2736, 2738, 2740, 2742, 2744, 2746, 2748, 2750, 2752, 2754, 2756, 2758, 2760, 2762, 2764, 2766, 2768, 2770, 2772, 2774, 2776, 2778, 2780, 2782, 2784, 2786, 2788, 2790, 2792, 2794, 2796, 2798, 2800, 2802, 2804, 2806, 2808, 2810, 2812, 2814, 2816, 2818, 2820, 2822, 2824, 2826, 2828, 2830, 2832, 2834, 2836, 2838, 2840, 2842, 2844, 2846 |
| 1 | 33 | LW1_OE X_PCT | B2153 | E. coli | 2859 | plastidic | 2861, 2863, 2865, 2867, 2869, 2871, 2873, 2875, 2877, 2879, 2881, 2883, 2885, 2887, 2889, 2891, 2893, 2895, 2897, 2899, 2901, 2903, 2905, 2907, 2909, 2911, 2913, 2915, 2917, 2919, 2921, 2923, 2925, 2927, 2929, 2931, 2933 |
| 1 | 34 | LW1_OE X_PCT | B2194 | E. coli | 2943 | non-targeted | 2945, 2947, 2949, 2951, 2953, 2955, 2957 |
| 1 | 35 | LW1_OE X_PCT | B2226 | E. coli | 2966 * | non-targeted | 2968, 2970, 2972, 2974 |
| 1 | 36 | LW1_OE X_PCT | B2309 | E. coli | 2982 | plastidic | 2984, 2986, 2988, 2990, 2992, 2994, 2996, 2998, 3000, 3002, 3004, 3006, 3008, 3010, 3012, 3014, 3016, 3018, 3020, 3022, 3024, 3026, 3028, 3030, 3032, 3034, 3036, 3038, 3040, 3042, 3044, 3046, 3048, 3050, 3052, 3054, 3056, 3058, 3060, 3062, 3064, 3066, 3068, 3070, 3072, 3074, 3076, 3078, 3080, 3082, 3084, 3086, 3088, 3090, 3092, 3094, 3096, 3098, 3100, 3102, 3104, 3106, 3108, 3110, 3112, 3114, 3116, 3118, 3120 |
| 1 | 37 | LW1_OE X_PCT | B2469 | E. coli | 3131 * | non-targeted | 3133, 3135, 3137, 3139, 3141, 3143, 3145, 3147, 3149, 3151, 3153, 3155, 3157, 3159, 3161, 3163, 3165, 3167, 3169, 3171, 3173, 3175, 3177, 3179, 3181, 3183, 3185, 3187, 3189, 3191, 3193, 3195, 3197, 3199, 3201, 3203, 3205 |
| 1 | 38 | LW1_OE X_PCT | B2475 | E. coli | 3217 | non-targeted | 3219, 3221, 3223, 3225, 3227, 3229, 3231, 3233, 3235, 3237, 3239, 3241, 3243, 3245, 3247, 3249, 3251, 3253, 3255, 3257, 3259, 3261, 3263, 3265, 3267, 3269, 3271, 3273, 3275, 3277, 3279, 3281, 3283, 3285, 3287, 3289, 3291, 3293, 3295, 3297, 3299, 3301, 3303, 3305, 3307, 3309, 3311, 3313, 3315, 3317, 3319, 3321, 3323, 3325, 3327 |
| 1 | 39 | LW1_OE X_PCT | B2482 | E. coli | 3336 | non-targeted | 3338, 3340, 3342, 3344, 3346, 3348, 3350, 3352, 3354, 3356, 3358, 3360, 3362, 3364, 3366, 3368, 3370, 3372, 3374, 3376, 3378, 3380, 3382, 3384, 3386, 3388, 3390, 3392, 3394 |
| 1 | 40 | LW1_OE X_PCT | B2541 | E. coli | 3402 | non-targeted | 3404, 3406, 3408, 3410, 3412, 3414, 3416, 3418, 3420, 3422, 3424, 3426, 3428, 3430, 3432, 3434, 3436, 3438, 3440, 3442, 3444, 3446, 3448, 3450, 3452, 3454, 3456, 3458, 3460, 3462, 3464, 3466, 3468, 3470, 3472, 3474, 3476, 3478, 3480, 3482, 3484, 3486, 3488, 3490, 3492, 3494, 3496, 3498, 3500, 3502, 3504, 3506, 3508, 3510, 3512, 3514, 3516, 3518, 3520, 3522, 3524, 3526, 3528, 3530, 3532, 3534, 3536, 3538, 3540, 3542, 3544, 3546, 3548, 3550, 3552, 3554 |
| 1 | 41 | LW1_OE X_PCT | B2559 | E. coli | 3591 | plastidic | 3593, 3595, 3597, 3599, 3601, 3603, 3605, 3607, 3609, 3611, 3613, 3615, 3617, 3619, 3621, 3623, 3625, 3627, 3629, 3631, 3633, 3635, 3637, 3639, 3641, 3643, 3645, 3647, 3649, 3651, 3653, 3655, 3657, 3659, 3661, 3663, 3665, 3667, 3669, 3671, 3673, 3675, 3677, 3679, 3681, 3683, 3685, 3687, 3689, 3691, 3693, 3695, 3697, 3699, 3701, 3703, 3705, 3707, 3709, 3711, 3713, 3715, 3717, 3719, 3721, 3723, 3725, 3727, 3729, 3731, 3733, 3735, 3737, 3739, 3741, 3743, 3745, 3747, 3749, 3751, 3753, 3755, 3757, 3759, 3761, 3763, 3765, 3767, 3769, 3771, 3773, 3775, 3777, 3779, 3781, 3783, 3785, 3787, 3789, 3791, 3793, 3795, 3797, 3799, 3801 |
| 1 | 42 | LW1_OE X_PCT | B2605 | E. coli | 3832 | non-targeted | 3834, 3836, 3838, 3840, 3842, 3844, 3846, 3848, 3850, 3852 |
| 1 | 43 | LW1_OE X_PCT | B2630 | E. coli | 3858 | non-targeted | - |
| 1 | 44 | LW1_OE X_PCT | B2678 | E. coli | 3862 | plastidic | 3864, 3866, 3868, 3870, 3872, 3874, 3876, 3878, 3880, 3882, 3884, 3886, 3888, 3890, 3892, 3894, 3896, 3898, 3900, 3902, 3904, 3906, 3908, 3910, 3912, 3914, 3916, 3918, 3920, 3922, 3924, 3926, 3928, 3930, 3932, 3934, 3936, 3938, 3940, 3942, 3944, 3946, 3948, 3950, 3952, 3954, 3956, 3958, 3960, 3962, 3964, 3966, 3968, 3970, 3972, 3974, 3976, 3978, 3980, 3982, 3984, 3986, 3988, 3990, 3992, 3994, 3996, 3998, 4000, 4002, 4004, 4006, 4008, 4010, 4012, 4014 |
| 1 | 45 | LW1_OE X_PCT | B2715 | E. coli | 4023 * | plastidic | 4025, 4027, 4029, 4031, 4033, 4035, 4037, 4039, 4041, 4043, 4045, 4047, 4049 |
| 1 | 46 | LW1_OE X_PCT | B2776 | E. coli | 4060 | non-targeted | 4062, 4064, 4066, 4068, 4070 |
| 1 | 47 | LW1_OE X_PCT | B2791 | E. coli | 4077 | non-targeted | 4079, 4081, 4083, 4085, 4087, 4089, 4091, 4093, 4095, 4097, 4099, 4101, 4103, 4105, 4107, 4109, 4111, 4113, 4115, 4117, 4119, 4121, 4123, 4125, 4127, 4129, 4131, 4133, 4135, 4137, 4139, 4141, 4143, 4145, 4147 |
| 1 | 48 | LW1_OE X_PCT | B2912 | E. coli | 4158 | non-targeted | 4160, 4162, 4164, 4166, 4168, 4170, 4172, 4174, 4176, 4178, 4180, 4182, 4184, 4186, 4188, 4190, 4192, 4194, 4196, 4198, 4200, 4202, 4204, 4206, 4208, 4210, 4212, 4214, 4216, 4218, 4220, 4222, 4224, 4226, 4228, 4230, 4232, 4234, 4236, 4238, 4240, 4242, 4244, 4246, 4248, 4250, 4252, 4254 |
| 1 | 49 | LW1_OE X_PCT | B2965 | E. coli | 4261 * | plastidic | 4263, 4265, 4267, 4269, 4271, 4273, 4275, 4277, 4279, 4281, 4283, 4285, 4287, 4289, 4291, 4293, 4295, 4297, 4299, 4301, 4303, 4305, 4307, 4309, 4311, 4313, 4315, 4317, 4319, 4321, 4323, 4325, 4327, 4329, 4331 |
| 1 | 50 | LW1_OE X_PCT | B2987 | E. coli | 4351 | plastidic | 4353, 4355, 4357, 4359, 4361, 4363, 4365, 4367, 4369, 4371, 4373, 4375, 4377, 4379, 4381, 4383, 4385, 4387, 4389, 4391, 4393, 4395, 4397, 4399, 4401, 4403, 4405, 4407, 4409, 4411, 4413, 4415, 4417, 4419, 4421, 4423, 4425, 4427, 4429, 4431, 4433, 4435, 4437, 4439, 4441, 4443, 4445, 4447, 4449 |
| 1 | 51 | LW1_OE X_PCT | B2987 | E. coli | 4351 | non-targeted | 4353, 4355, 4357, 4359, 4361, 4363, 4365, 4367, 4369, 4371, 4373, 4375, 4377, 4379, 4381, 4383, 4385, 4387, 4389, 4391, 4393, 4395, 4397, 4399, 4401, 4403, 4405, 4407, 4409, 4411, 4413, 4415, 4417, 4419, 4421, 4423, 4425, 4427, 4429, 4431, 4433, 4435, 4437, 4439, 4441, 4443, 4445, 4447, 4449 |
| 1 | 52 | LW1_OE X_PCT | B3093 | E. coli | 4460 | plastidic | 4462, 4464, 4466, 4468, 4470, 4472, 4474, 4476, 4478, 4480, 4482, 4484, 4486, 4488, 4490, 4492, 4494 |
| 1 | 53 | LW1_OE X_PCT | B3363 | E. coli | 4506 | plastidic | 4508, 4510, 4512, 4514, 4516, 4518, 4520, 4522, 4524, 4526, 4528, 4530, 4532, 4534, 4536, 4538, 4540, 4542, 4544, 4546, 4548, 4550, 4552, 4554, 4556, 4558, 4560, 4562, 4564, 4566, 4568, 4570, 4572, 4574, 4576, 4578, 4580, 4582, 4584, 4586, 4588, 4590, 4592, 4594, 4596, 4598 |
| 1 | 54 | LW1_OE X_PCT | B3429 | E. coli | 4641 | plastidic | 4643, 4645, 4647, 4649, 4651, 4653, 4655, 4657, 4659, 4661, 4663, 4665, 4667, 4669, 4671, 4673, 4675, 4677, 4679, 4681, 4683, 4685, 4687, 4689, 4691, 4693, 4695, 4697, 4699, 4701, 4703, 4705, 4707, 4709, 4711, 4713, 4715, 4717, 4719, 4721, 4723, 4725, 4727, 4729, 4731, 4733, 4735, 4737, 4739, 4741, 4743, 4745, 4747, 4749, 4751, 4753, 4755, 4757, 4759, 4761, 4763, 4765, 4767, 4769, 4771, 4773, 4775, 4777, 4779, 4781, 4783, 4785, 4787, 4789, 4791, 4793, 4795, 4797 |
| 1 | 55 | LW1_OE X_PCT | B3568 | E. coli | 4807 | plastidic | 4809, 4811, 4813, 4815, 4817, 4819, 4821, 4823, 4825, 4827, 4829, 4831, 4833, 4835, 4837, 4839, 4841, 4843, 4845, 4847, 4849, 4851, 4853, 4855, 4857, 4859, 4861, 4863, 4865, 4867, 4869, 4871, 4873, 4875, 4877, 4879, 4881, 4883, 4885, 4887, 4889, 4891, 4893, 4895, 4897, 4899, 4901, 4903, 4905, 4907, 4909, 4911, 4913, 4915, 4917, 4919, 4921, 4923, 4925, 4927, 4929, 4931, 4933, 4935, 4937, 4939, 4941, 4943, 4945, 4947, 4949, 4951, 4953, 4955, 4957, 4959, 4961, 4963, 4965, 4967, 4969, 4971, 4973, 4975, 4977, 4979, 4981, 4983, 4985, 4987, 4989, 4991, 4993, 4995, 4997, 4999, 5001, 5003, 5005, 5007, 5009, 5011, 5013, 5015, 5017, 5019, 5021, 5023, 5025, 5027, 5029, 5031, 5033, 5035, 5037, 5039, 5041, 5043, 5045, 5047, 5049, 5051, 5053, 5055, 5057, 5059, 5061, 5063, 5065, 5067, 5069, 5071, 5073, 5075, 5077, 5079, 5081, 5083, 5085, 5087, 5089, 5091, 5093, 5095, 5097, 5099, 5101, 5103, 5105, 5107, 5109, 5111, 5113, 5115, 5117 |
| 1 | 56 | LW1_OE X_PCT | B3616 | E. coli | 5125 | plastidic | 5127, 5129, 5131, 5133, 5135, 5137, 5139, 5141, 5143, 5145, 5147, 5149, 5151, 5153, 5155, 5157, 5159, 5161, 5163, 5165, 5167, 5169, 5171, 5173, 5175, 5177, 5179, 5181, 5183, 5185, 5187, 5189, 5191, 5193, 5195, 5197, 5199, 5201, 5203, 5205, 5207, 5209, 5211, 5213, 5215, 5217, 5219, 5221, 5223, 5225, 5227, 5229, 5231, 5233, 5235, 5237, 5239, 5241, 5243, 5245, 5247, 5249, 5251, 5253, 5255, 5257, 5259, 5261, 5263, 5265, 5267, 5269, 5271, 5273, 5275, 5277, 5279, 5281, 5283, 5285, 5287, 5289, 5291, 5293, 5295, 5297, 5299, 5301, 5303, 5305, 5307, 5309, 5311, 5313, 5315, 5317, 5319, 5321, 5323, 5325, 5327, 5329, 5331, 5333, 5335, 5337, 5339, 5341, 5343 |
| 1 | 57 | LW1_OE X_PCT | B3616 | E. coli | 5125 | non-targeted | 5127, 5129, 5131, 5133, 5135, 5137, 5139, 5141, 5143, 5145, 5147, 5149, 5151, 5153, 5155, 5157, 5159, 5161, 5163, 5165, 5167, 5169, 5171, 5173, 5175, 5177, 5179, 5181, 5183, 5185, 5187, 5189, 5191, 5193, 5195, 5197, 5199, 5201, 5203, 5205, 5207, 5209, 5211, 5213, 5215, 5217, 5219, 5221, 5223, 5225, 5227, 5229, 5231, 5233, 5235, 5237, 5239, 5241, 5243, 5245, 5247, 5249, 5251, 5253, 5255, 5257, 5259, 5261, 5263, 5265, 5267, 5269, 5271, 5273, 5275, 5277, 5279, 5281, 5283, 5285, 5287, 5289, 5291, 5293, 5295, 5297, 5299, 5301, 5303, 5305, 5307, 5309, 5311, 5313, 5315, 5317, 5319, 5321, 5323, 5325, 5327, 5329, 5331, 5333, 5335, 5337, 5339, 5341, 5343 |
| 1 | 58 | LW1_OE X_PCT | B3812 | E. coli | 5418 | non-targeted | 5420, 5422, 5424, 5426, 5428, 5430, 5432, 5434, 5436, 5438, 5440, 5442, 5444, 5446, 5448, 5450, 5452, 5454, 5456, 5458, 5460, 5462, 5464, 5466, 5468, 5470, 5472, 5474, 5476, 5478, 5480, 5482, 5484, 5486, 5488 |
| 1 | 59 | LW1_OE X_PCT | B3899 | E. coli | 5496 * | non-targeted | 5498, 5500, 5502, 5504, 5506, 5508, 5510, 5512, 5514, 5516, 5518, 5520, 5522, 5524, 5526, 5528, 5530, 5532, 5534, 5536, 5538, 5540, 5542, 5544, 5546, 5548, 5550, 5552, 5554, 5556, 5558, 5560, 5562, 5564, 5566, 5568, 5570, 5572, 5574, 5576, 5578 |
| 1 | 60 | LW1_OE X_PCT | B3929 | E. coli | 5586 | plastidic | 5588, 5590, 5592, 5594, 5596, 5598, 5600, 5602, 5604, 5606, 5608, 5610, 5612, 5614, 5616, 5618, 5620, 5622, 5624, 5626, 5628, 5630, 5632, 5634, 5636, 5638, 5640, 5642, 5644, 5646, 5648, 5650, 5652, 5654, 5656, 5658, 5660, 5662, 5664, 5666, 5668, 5670, 5672, 5674, 5676, 5678, 5680, 5682, 5684, 5686, 5688, 5690, 5692, 5694, 5696, 5698, 5700, 5702, 5704, 5706, 5708, 5710, 5712, 5714, 5716, 5718, 5720, 5722, 5724, 5726, 5728, 5730, 5732, 5734, 5736, 5738, 5740, 5742, 5744, 5746, 5748, 5750, 5752, 5754, 5756, 5758, 5760, 5762, 5764, 5766, 5768, 5770, 5772, 5774, 5776 |
| 1 | 61 | LW1_OE X_PCT | B3938 | E. coli | 5801 | non-targeted | 5803, 5805, 5807, 5809, 5811, 5813, 5815, 5817, 5819, 5821, 5823, 5825, 5827, 5829, 5831, 5833, 5835, 5837, 5839, 5841, 5843 |
| 1 | 62 | LW1_OE X_PCT | B3974 | E. coli | 5851 | non-targeted | 5853, 5855, 5857, 5859, 5861, 5863, 5865, 5867, 5869, 5871, 5873, 5875, 5877, 5879, 5881, 5883, 5885, 5887, 5889, 5891, 5893, 5895, 5897, 5899, 5901, 5903, 5905, 5907, 5909, 5911, 5913, 5915, 5917, 5919, 5921, 5923, 5925, 5927, 5929, 5931, 5933, 5935, 5937, 5939, 5941, 5943, 5945, 5947, 5949, 5951, 5953, 5955, 5957, 5959, 5961, 5963, 5965, 5967, 5969, 5971, 5973, 5975, 5977, 5979, 5981 |
| 1 | 63 | LW1_OE X_PCT | B3989 | E. coli | 5993 | non-targeted | 5995 |
| 1 | 64 | LW1_OE X_PCT | B4029 | E. coli | 6000 | non-targeted | 6002, 6004, 6006, 6008, 6010, 6012, 6014, 6016, 6018, 6020, 6022, 6024, 6026, 6028, 6030, 6032, 6034, 6036, 6038, 6040 |
| 1 | 65 | LW1_OE X_PCT | B4139 | E. coli | 6057 * | plastidic | 6059, 6061, 6063, 6065, 6067, 6069, 6071, 6073, 6075, 6077, 6079, 6081, 6083, 6085, 6087, 6089, 6091, 6093, 6095, 6097, 6099, 6101, 6103, 6105, 6107, 6109, 6111, 6113, 6115, 6117, 6119, 6121, 6123, 6125, 6127, 6129, 6131, 6133, 6135, 6137, 6139, 6141, 6143, 6145, 6147, 6149, 6151, 6153, 6155, 6157, 6159, 6161, 6163, 6165, 6167, 6169, 6171, 6173, 6175, 6177, 6179, 6181, 6183, 6185, 6187, 6189, 6191, 6193, 6195, 6197, 6199, 6201, 6203, 6205, 6207, 6209, 6211, 6213, 6215, 6217, 6219, 6221, 6223, 6225, 6227, 6229, 6231, 6233, 6235, 6237, 6239, 6241, 6243, 6245, 6247, 6249, 6251, 6253, 6255, 6257, 6259, 6261, 6263, 6265, 6267, 6269, 6271, 6273, 6275, 6277, 6279, 6281, 6283, 6285, 6287, 6289, 6291, 6293, 6295, 6297, 6299, 6301, 6303, 6305, 6307, 6309, 6311, 6313, 6315, 6317, 6319, 6321, 6323, 6325, 6327, 6329, 6331, 6333, 6335, 6337, 6339, 6341, 6343, 6345, 6347, 6349, 6351, 6353, 6355, 6357, 6359, 6361, 6363, 6365, 6367, 6369, 6371, 6373, 6375, 6377, 6379, 6381, 6383, 6385, 6387, 6389, 6391, 6393, 6395, 6397, 6399, 6401, 6403, 6405, 6407, 6409, 6411, 6413, 6415, 6417, 6419, 6421, 6423, 6425, 6427, 6429, 6431, 6433, 6435, 6437, 6439, 6441, 6443, 6445, 6447, 6449, 6451, 6453, 6455, 6457, 6459, 6461, 6463, 6465, 6467, 6469, 6471, 6473 |
| 1 | 66 | LW1_OE X_PCT | B4390 | E. coli | 6501 * | non-targeted | 6503, 6505, 6507, 6509, 6511, 6513, 6515, 6517, 6519, 6521, 6523, 6525, 6527, 6529 |
| 1 | 67 | LW1_OE X_PCT | SII0290 | Synec hocysti s | 6543 | non-targeted | 6545, 6547, 6549, 6551, 6553, 6555, 6557, 6559, 6561, 6563, 6565, 6567, 6569, 6571, 6573, 6575, 6577, 6579, 6581, 6583, 6585, 6587, 6589, 6591, 6593, 6595, 6597, 6599, 6601, 6603, 6605, 6607, 6609, 6611, 6613, 6615, 6617, 6619, 6621, 6623, 6625, 6627, 6629, 6631, 6633, 6635, 6637, 6639, 6641, 6643, 6645, 6647, 6649, 6651, 6653, 6655, 6657, 6659, 6661, 6663, 6665, 6667, 6669, 6671, 6673, 6675, 6677, 6679, 6681, 6683, 6685, 6687, 6689, 6691, 6693, 6695, 6697, 6699, 6701, 6703, 6705, 6707, 6709, 6711, 6713, 6715, 6717, 6719, 6721, 6723, 6725, 6727, 6729, 6731, 6733, 6735, 6737, 6739, 6741, 6743, 6745, 6747, 6749, 6751, 6753, 6755, 6757, 6759, 6761, 6763, 6765, 6767, 6769, 6771, 6773, 6775, 6777, 6779, 6781, 6783, 6785, 6787, 6789, 6791, 6793, 6795, 6797, 6799, 6801, 6803, 6805, 6807, 6809, 6811 |
| 1 | 68 | LW1_OE X_PCT | YAL049C | Yeast | 6824 | non-targeted | 6826, 6828, 6830, 6832, 6834, 6836, 6838, 6840, 6842, 6844, 6846, 6848, 6850, 6852, 6854, 6856, 6858, 6860, 6862 |
| 1 | 69 | LW1_OE X_PCT | YCR059C | Yeast | 6871 | non-targeted | 6873, 6875, 6877, 6879, 6881, 6883, 6885, 6887, 6889, 6891, 6893, 6895, 6897, 6899, 6901 |
| 1 | 70 | LW1_OE X_PCT | YDR035W | Yeast | 6911 | plastidic | 6913, 6915, 6917, 6919, 6921, 6923, 6925, 6927, 6929, 6931, 6933, 6935, 6937, 6939, 6941, 6943, 6945, 6947, 6949, 6951, 6953, 6955, 6957, 6959, 6961, 6963, 6965, 6967, 6969, 6971, 6973, 6975, 6977, 6979, 6981, 6983, 6985, 6987, 6989, 6991, 6993, 6995, 6997, 6999, 7001, 7003, 7005, 7007, 7009, 7011, 7013, 7015, 7017, 7019, 7021, 7023, 7025, 7027, 7029, 7031, 7033, 7035, 7037, 7039, 7041, 7043, 7045, 7047, 7049, 7051, 7053, 7055, 7057, 7059, 7061, 7063, 7065, 7067, 7069, 7071, 7073, 7075, 7077, 7079, 7081, 7083, 7085, 7087, 7089, 7091, 7093, 7095, 7097, 7099, 7101, 7103, 7105, 7107, 7109, 7111, 7113, 7115, 7117, 7119, 7121, 7123, 7125, 7127, 7129, 7131, 7133, 7135, 7137, 7139, 7141, 7143, 7145, 7147, 7149, 7151, 7153, 7155, 7157, 7159, 7161, 7163, 7165, 7167, 7169, 7171, 7173, 7175, 7177, 7179, 7181, 7183, 7185, 7187, 7189, 7191, 7193, 7195, 7197, 7199, 7201, 7203, 7205, 7207, 7209, 7211, 7213, 7215, 7217, 7219, 7221, 7223, 7225, 7227, 7229, 7231, 7233, 7235, 7237, 7239, 7241, 7243, 7245, 7247, 7249 |
| 1 | 71 | LW1_OE X_PCT | YEL005C | Yeast | 7262 | non-targeted | - |
| 1 | 72 | LW1_OE X_PCT | YER112W | Yeast | 7266 * | non-targeted | 7268 |
| 1 | 73 | LW1_OE X_PCT | YER156C | Yeast | 7302 | non-targeted | 7304, 7306, 7308, 7310, 7312, 7314, 7316, 7318, 7320, 7322, 7324, 7326, 7328, 7330, 7332, 7334, 7336, 7338, 7340, 7342, 7344, 7346, 7348, 7350, 7352, 7354, 7356, 7358, 7360, 7362, 7364, 7366, 7368 |
| 1 | 74 | LW1_OE X_PCT | YER173W | Yeast | 7385 | non-targeted | 7387, 7389, 7391, 7393 |
| 1 | 75 | LW1_OE X_PCT | YGL045W | Yeast | 7408 * | non-targeted | 7410, 7412, 7414, 7416 |
| 1 | 76 | LW1_OE X_PCT | YGL189C | Yeast | 7430 | non-targeted | 7432, 7434, 7436, 7438, 7440, 7442, 7444, 7446, 7448, 7450, 7452, 7454, 7456, 7458, 7460, 7462, 7464, 7466, 7468, 7470, 7472, 7474, 7476, 7478, 7480, 7482, 7484, 7486 |
| 1 | 77 | LW1_OE X_PCT | YNR015W | Yeast | 7559 | non-targeted | 7561, 7563, 7565, 7567, 7569, 7571, 7573, 7575, 7577, 7579, 7581, 7583, 7585, 7587, 7589, 7591, 7593 |
| 1 | 78 | LW1_OE X_PCT | YOR024W | Yeast | 7607 | non-targeted | - |
| 1 | 79 | LW1_OE X_PCT | YOR168W | Yeast | 7611 * | non-targeted | 7613, 7615, 7617, 7619, 7621, 7623, 7625, 7627, 7629, 7631, 7633, 7635, 7637, 7639, 7641, 7643, 7645, 7647, 7649, 7651 |
| 1 | 80 | LW1_OE X_PCT | YPL151C | Yeast | 7686 | non-targeted | 7688, 7690, 7692, 7694, 7696, 7698, 7700, 7702, 7704, 7706, 7708, 7710, 7712, 7714, 7716, 7718, 7720, 7722, 7724, 7726 |
| 1 | 81 | LW1_OE X_PCT | B1297 | E. coli | 1202 * | plastidic | 1204, 1206, 1208, 1209, 1211, 1213, 1215, 1217, 1219, 1221, 1223, 1225, 1227, 1229, 1231, 1233, 1235, 1237, 1239, 1241, 1243, 1245, 1247, 1249, 1251, 1253, 1255, 1257, 1259, 1261, 1263, 1265, 1267, 1269, 1271, 1273, 1275, 1277, 1279, 1281, 1283, 1285, 1287, 1289, 1291, 1293, 1295, 1297 |
| 1 | 82 | LW1_OE X_PCT | B0970 | E. coli | 7742 | non-targeted | 7744, 7746, 7748, 7750, 7752, 7754, 7756, 7758, 7760, 7762, 7764, 7766, 7768, 7770, 7772, 7774, 7776, 7778, 7780, 7782, 7784, 7786, 7788, 7790, 7792, 7794, 7796, 7798, 7800, 7802, 7804, 7806, 7808, 7810, 7812, 7814, 7816, 7818, 7820, 7822, 7824, 7826, 7828, 7830, 7832, 7834, 7836, 7838, 7840, 7842 |
| 1 | 83 | LW1_OE X_PCT | B1829 | E. coli | 7851 | non-targeted | 7853, 7855, 7857, 7859, 7861, 7863, 7865, 7867, 7869, 7871, 7873, 7875, 7877, 7879, 7881, 7883, 7885, 7887, 7889, 7891, 7893, 7895, 7897, 7899, 7901, 7903, 7905, 7907, 7909, 7911, 7913, 7915, 7917, 7919, 7921, 7923, 7925, 7927, 7929, 7931, 7933, 7935, 7937, 7939, 7941, 7943, 7945, 7947, 7949, 7951, 7953, 7955, 7957, 7959, 7961 |
| 1 | 84 | LW1_OE X_PCT | B2664 | E. coli | 7972 * | non-targeted | 7974, 7976, 7978, 7980, 7982, 7984, 7986, 7988, 7990, 7992, 7994, 7996, 7998, 8000, 8002, 8004, 8006, 8008, 8010, 8012, 8014 |
| 1 | 85 | LW1_OE X_PCT | B2796 | E. coli | 8022 | non-targeted | 8024, 8026, 8028, 8030, 8032, 8034, 8036, 8038, 8040, 8042, 8044, 8046, 8048, 8050, 8052, 8054, 8056, 8058, 8060, 8062, 8064, 8066, 8068, 8070, 8072, 8074, 8076, 8078, 8080, 8082, 8084, 8086, 8088, 8090, 8092, 8094, 8096, 8098, 8100, 8102, 8104, 8106, 8108, 8110, 8112, 8114, 8116, 8118, 8120, 8122, 8124, 8126, 8128, 8130, 8132, 8134, 8136, 8138, 8140, 8142, 8144, 8146, 8148, 8150, 8152, 8154, 8156, 8158, 8160, 8162, 8164, 8166 |
| 1 | 86 | LW1_OE X_PCT | YER174C | Yeast | 8178 | non-targeted | 8180, 8182, 8184, 8186, 8188, 8190, 8192, 8194, 8196, 8198, 8200, 8202, 8204, 8206, 8208, 8210, 8212, 8214, 8216, 8218, 8220, 8222, 8224, 8226, 8228, 8230, 8232 |
| 1 | 87 | LW1_OE X_PCT | YFR042W | Yeast | 8273 | non-targeted | 8275, 8277, 8279, 8281 |
| 1 | 88 | LW1_OE X_PCT | YKR057W | Yeast | 8289 | non-targeted | 8291, 8293, 8295, 8297, 8299, 8301, 8303, 8305, 8307, 8309, 8311, 8313, 8315, 8317, 8319, 8321, 8323, 8325, 8327, 8329, 8331, 8333, 8335, 8337, 8339, 8341, 8343, 8345, 8347, 8349, 8351, 8353, 8355, 8357, 8359, 8361, 8363, 8365, 8367, 8369, 8371, 8373, 8375, 8377 |
| 1 | 89 | LW1_OE X_PCT | B0629_2 | E. coli | 8439 | non-targeted | 8441, 8443, 8445, 8447, 8449, 8451 |
| 1 | 90 | LW1_OE X_PCT | B1007_2 | E. coli | 8631 | non-targeted | 8633, 8635, 8637, 8639, 8641, 8643, 8645, 8647, 8649, 8651, 8653, 8655, 8657, 8659, 8661, 8663, 8665, 8667, 8669, 8671, 8673, 8675, 8677, 8679, 8681, 8683, 8685, 8687, 8689, 8691, 8693, 8695, 8697, 8699, 8701, 8703, 8705, 8707, 8709, 8711, 8713, 8715, 8717, 8719, 8721, 8723, 8725, 8727, 8729, 8731, 8733, 8735, 8737 |
| 1 | 91 | LW1_OE X_PCT | B2715_2 | E. coli | 9269 | plastidic | 9271, 9273, 9275, 9277, 9279, 9281, 9283, 9285, 9287, 9289, 9291, 9293, 9295, 9297, 9299, 9301, 9303, 9305 |
| 1 | 92 | LW1_OE X_PCT | B3899_2 | E. coli | 9445 | non-targeted | 9447, 9449, 9451, 9453, 9455, 9457, 9459, 9461, 9463, 9465, 9467, 9469, 9471, 9473, 9475, 9477, 9479, 9481, 9483, 9485, 9487, 9489, 9491, 9493, 9495, 9497, 9499, 9501, 9503, 9505, 9507, 9509, 9511, 9513, 9515, 9517, 9519, 9521, 9523 |
| 1 | 93 | LW1_OE X_PCT | B4390_2 | E. coli | 9825 | non-targeted | 9827, 9829, 9831, 9833, 9835, 9837, 9839, 9841, 9843, 9845, 9847, 9849, 9851, 9853, 9855, 9857, 9859, 9861, 9863, 9865, 9867, 9869 |
| 1 | 94 | LW1_OE X_PCT | YGL045W_2 | Yeast | 9906 | non-targeted | 9908, 9910, 9912, 9914, 9916, 9918, 9920, 9922, 9924 |
| 1 | 95 | LW1_OE X_PCT | B2664_2 | E. coli | 9194 | non-targeted | 9196, 9198, 9200, 9202, 9204, 9206, 9208, 9210, 9212, 9214, 9216, 9218, 9220, 9222, 9224, 9226, 9228, 9230, 9232, 9234, 9236, 9238, 9240, 9242, 9244, 9246, 9248, 9250, 9252, 9254, 9256, 9258, 9260, 9262 |
| 1 | 96 | LW1_OE X_PCT | B0963_2 | E. coli | 8498 | non-targeted | 8500, 8502, 8504, 8506, 8508, 8510, 8512, 8514, 8516, 8518, 8520, 8522, 8524, 8526, 8528, 8530, 8532, 8534, 8536, 8538, 8540, 8542, 8544, 8546, 8548, 8550, 8552, 8554, 8556, 8558, 8560, 8562, 8564, 8566, 8568, 8570, 8572, 8574, 8576, 8578, 8580, 8582, 8584, 8586, 8588, 8590, 8592, 8594, 8596, 8598, 8600, 8602, 8604, 8606, 8608, 8610, 8612, 8614, 8616, 8618, 8620, 8622, 8624 |
| 1 | 97 | LW1_OE X_PCT | B1297_2 | E. coli | 8743 | plastidic | 8745, 8747, 8749, 8751, 8753, 8755, 8757, 8759, 8761, 8763, 8765, 8767, 8769, 8771, 8773, 8775, 8777, 8779, 8781, 8783, 8785, 8787, 8789, 8791, 8793, 8795, 8797, 8799, 8801, 8803, 8805, 8807, 8809, 8811, 8813, 8815, 8817, 8819, 8821, 8823, 8825, 8827, 8829, 8831, 8833, 8835, 8837, 8839, 8841, 8843, 8845, 8847, 8849, 8851, 8853, 8855, 8857, 8859, 8861, 8863, 8865, 8867, 8869, 8871, 8873, 8875, 8877, 8879, 8881, 8883 |
| 1 | 98 | LW1_OE X_PCT | B1597_2 | E. coli | 8892 | non-targeted | 8894, 8896, 8898, 8900, 8902, 8904, 8906, 8908, 8910, 8912, 8914, 8916, 8918, 8920, 8922, 8924, 8926, 8928 |
| 1 | 99 | LW1_OE X_PCT | B2027_2 | E. coli | 9032 | non-targeted | 9034, 9036, 9038, 9040, 9042, 9044, 9046, 9048, 9050, 9052, 9054, 9056, 9058, 9060, 9062, 9064, 9066, 9068, 9070, 9072, 9074, 9076, 9078, 9080, 9082, 9084, 9086 |
| 1 | 100 | LW1_OE X_PCT | B2965_2 | E. coli | 9316 | plastidic | 9318, 9320, 9322, 9324, 9326, 9328, 9330, 9332, 9334, 9336, 9338, 9340, 9342, 9344, 9346, 9348, 9350, 9352, 9354, 9356, 9358, 9360, 9362, 9364, 9366, 9368, 9370, 9372, 9374, 9376, 9378, 9380, 9382, 9384, 9386, 9388, 9390, 9392, 9394, 9396, 9398, 9400, 9402, 9404, 9406, 9408, 9410, 9412, 9414, 9416, 9418, 9420, 9422, 9424, 9426, 9428, 9430 |
| 1 | 101 | LW1_OE X_PCT | B4139_2 | E. coli | 9530 | plastidic | 9532, 9534, 9536, 9538, 9540, 9542, 9544, 9546, 9548, 9550, 9552, 9554, 9556, 9558, 9560, 9562, 9564, 9566, 9568, 9570, 9572, 9574, 9576, 9578, 9580, 9582, 9584, 9586, 9588, 9590, 9592, 9594, 9596, 9598, 9600, 9602, 9604, 9606, 9608, 9610, 9612, 9614, 9616, 9618, 9620, 9622, 9624, 9626, 9628, 9630, 9632, 9634, 9636, 9638, 9640, 9642, 9644, 9646, 9648, 9650, 9652, 9654, 9656, 9658, 9660, 9662, 9664, 9666, 9668, 9670, 9672, 9674, 9676, 9678, 9680, 9682, 9684, 9686, 9688, 9690, 9692, 9694, 9696, 9698, 9700, 9702, 9704, 9706, 9708, 9710, 9712, 9714, 9716, 9718, 9720, 9722, 9724, 9726, 9728, 9730, 9732, 9734, 9736, 9738, 9740, 9742, 9744, 9746, 9748, 9750, 9752, 9754, 9756, 9758, 9760, 9762, 9764, 9766, 9768, 9770, 9772, 9774, 9776, 9778, 9780, 9782, 9784, 9786, 9788, 9790, 9792, 9794, 9796, 9798, 9800, 9802, 9804 |
| 1 | 102 | LW1_OE X_PCT | B0845_2 | E. coli | 8463 | non-targeted | 8465, 8467, 8469, 8471, 8473, 8475, 8477, 8479, 8481, 8483, 8485 |
| 1 | 103 | LW1_OE X_PCT | B1901_2 | E. coli | 8974 | plastidic | 8976, 8978, 8980, 8982, 8984, 8986, 8988, 8990, 8992, 8994, 8996, 8998, 9000, 9002, 9004, 9006, 9008, 9010, 9012, 9014, 9016, 9018, 9020, 9022 |
| 1 | 104 | LW1_OE X_PCT | YER112W_2 | Yeast | 9884 | non-targeted | 9886 |
| 1 | 105 | LW1_OE X_PCT | B1798_2 | E. coli | 8935 | non-targeted | 8937, 8939, 8941, 8943, 8945, 8947, 8949, 8951, 8953, 8955, 8957,8959,8961,8963,8965 |
| 1 | 106 | LW1_OE X_PCT | B2226_2 | E. coli | 9094 | non-targeted | 9096, 9098, 9100, 9102 |
| 1 | 107 | LW1_OE X_PCT | B2469_2 | E. coli | 9110 | non-targeted | 9112, 9114, 9116, 9118, 9120, 9122, 9124, 9126, 9128, 9130, 9132, 9134, 9136, 9138, 9140, 9142, 9144, 9146, 9148, 9150, 9152, 9154, 9156, 9158, 9160, 9162, 9164, 9166, 9168, 9170, 9172, 9174, 9176, 9178, 9180, 9182 |
| 1 | 108 | LW1_OE X_PCT | YOR168W_2 | Yeast | 9932 | non-targeted | 9934, 9936, 9938, 9940, 9942, 9944, 9946, 9948, 9950, 9952, 9954, 9956, 9958, 9960, 9962, 9964, 9966, 9968, 9970, 9972 |
| 1 | 109 | LW1_OE X_PCT | B4321 | E. coli | 10097 | non-targeted | 10099, 10101, 10103, 10105, 10107, 10109, 10111, 10113, 10115, 10117, 10119, 10121, 10123, 10125, 10127, 10129, 10131, 10133, 10135, 10137, 10139, 10141, 10143, 10145, 10147, 10149, 10151, 10153, 10155, 10157, 10159, 10161, 10163, 10165, 10167, 10169, 10171, 10173, 10175, 10177, 10179, 10181, 10183, 10185, 10187, 10189, 10191, 10193, 10195, 10197, 10199, 10201, 10203, 10205, 10207, 10209, 10211, 10213, 10215, 10217, 10219, 10221, 10223, 10225, 10227, 10229, 10231, 10233, 10235, 10237, 10239, 10241, 10243, 10245, 10247, 10249 |

| | | | | | | | |
|---|---|---|---|---|---|---|---|
| * Sequences marked with asterisk * reflect the respective sequence derived from public data bases information | | | | | | | |

**Table IIB: Amino acid sequence ID numbers**

| | **1.** | **2.** | **3.** | **4.** | **5.** | **6.** | **7**. |
|---|---|---|---|---|---|---|---|
| Application | Hit | Project | Locus | Organism | Lead SEQ ID | Target | SEQ IDs of Polypeptide Homologs |
| 1 | 1 | LW1_OE X_PCT | B0081 | E. coli | 39 | non-targeted | - |
| 1 | 2 | LW1_OE X_PCT | B0445 | E. coli | 55 | non-targeted | - |
| 1 | 3 | LW1_OE X_PCT | B0482 | E. coli | 71 | non-targeted | - |
| 1 | 4 | LW1_OE X_PCT | B0607 | E. coli | 90 | non-targeted | - |
| 1 | 5 | LW1_OE X_PCT | B0629 | E. coli | 144 * | non-targeted | - |
| 1 | 6 | LW1_OE X_PCT | B0631 | E. coli | 163 | non-targeted | - |
| 1 | 7 | LW1_OE X_PCT | B0697 | E. coli | 214 | non-targeted | - |
| 1 | 8 | LW1_OE X_PCT | B0753 | E. coli | 359 | non-targeted | - |
| 1 | 9 | LW1_OE X_PCT | B0813 | E. coli | 368 | non-targeted | - |
| 1 | 10 | LW1_OE X_PCT | B0845 | E. coli | 421 * | non-targeted | - |
| 1 | 11 | LW1_OE X_PCT | B0866 | E. coli | 456 | non-targeted | - |
| 1 | 12 | LW1_OE X_PCT | B0963 | E. coli | 536 * | non-targeted | - |
| 1 | 13 | LW1_OE X_PCT | B0975 | E. coli | 619 | non-targeted | - |
| 1 | 14 | LW1_OE X_PCT | B1007 | E. coli | 672 * | non-targeted | - |
| 1 | 15 | LW1_OE X_PCT | B1052 | E. coli | 765 | non-targeted | - |
| 1 | 16 | LW1_OE X_PCT | B1091 | E. coli | 769 | plastidic | 891, 893, 895 |
| 1 | 17 | LW1_OE X_PCT | B1161 | E. coli | 908 | non-targeted | - |
| 1 | 18 | LW1_OE X_PCT | B1186 | E. coli | 928 | non-targeted | - |
| 1 | 19 | LW1_OE X_PCT | B1291 | E. coli | 1010 | plastidic | - |
| 1 | 20 | LW1_OE X_PCT | B1294 | E. coli | 1155 | plastidic | - |
| 1 | 21 | LW1_OE X_PCT | B1423 | E. coli | 1309 | non-targeted | - |
| 1 | 22 | LW1_OE X_PCT | B1597 | E. coli | 1369 * | non-targeted | - |
| 1 | 23 | LW1_OE X_PCT | B1605 | E. coli | 1375 | non-targeted | - |
| 1 | 24 | LW1_OE X_PCT | B1704 | E. coli | 1508 | non-targeted | - |
| 1 | 25 | LW1_OE X_PCT | B1736 | E. coli | 1954 | plastidic | - |
| 1 | 26 | LW1_OE X_PCT | B1798 | E. coli | 2157 * | non-targeted | - |
| 1 | 27 | LW1_OE X_PCT | B1878 | E. coli | 2196 | non-targeted | - |
| 1 | 28 | LW1_OE X_PCT | B1901 | E. coli | 2220 * | plastidic | - |
| 1 | 29 | LW1_OE X_PCT | B1912 | E. coli | 2278 | plastidic | 2458,2460,2462,10013 |
| 1 | 30 | LW1_OE X_PCT | B2027 | E. coli | 2471 * | non-targeted | - |
| 1 | 31 | LW1_OE X_PCT | B2039 | E. coli | 2494 | non-targeted | - |
| 1 | 32 | LW1_OE X_PCT | B2075 | E. coli | 2628 | non-targeted | - |
| 1 | 33 | LW1_OE X_PCT | B2153 | E. coli | 2859 | plastidic | - |
| 1 | 34 | LW1_OE X_PCT | B2194 | E. coli | 2943 | non-targeted | - |
| 1 | 35 | LW1_OE X_PCT | B2226 | E. coli | 2966 * | non-targeted | - |
| 1 | 36 | LW1_OE X_PCT | B2309 | E. coli | 2982 | plastidic | - |
| 1 | 37 | LW1_OE X_PCT | B2469 | E. coli | 3131 * | non-targeted | - |
| 1 | 38 | LW1_OE X_PCT | B2475 | E. coli | 3217 | non-targeted | - |
| 1 | 39 | LW1_OE X_PCT | B2482 | E. coli | 3336 | non-targeted | - |
| 1 | 40 | LW1_OE X_PCT | B2541 | E. coli | 3402 | non-targeted | 3556, 3558, 3560, 3562, 3564, 3566, 3568, 3570, 3572, 3574, 3576, 3578, 3580, 3582, 3584, 10017, 10019 |
| 1 | 41 | LW1_OE X_PCT | B2559 | E. coli | 3591 | plastidic | 3803, 3805, 3807, 3809, 3811, 3813, 3815, 3817, 3819, 3821, 3823, 3825 |
| 1 | 42 | LW1_OE X_PCT | B2605 | E. coli | 3832 | non-targeted | - |
| 1 | 43 | LW1_OE X_PCT | B2630 | E. coli | 3858 | non-targeted | - |
| 1 | 44 | LW1_OE X_PCT | B2678 | E. coli | 3862 | plastidic | - |
| 1 | 45 | LW1_OE X_PCT | B2715 | E. coli | 4023 * | plastidic | - |
| 1 | 46 | LW1_OE X_PCT | B2776 | E. coli | 4060 | non-targeted | - |
| 1 | 47 | LW1_OE X_PCT | B2791 | E. coli | 4077 | non-targeted | - |
| 1 | 48 | LW1_OE X_PCT | B2912 | E. coli | 4158 | non-targeted | - |
| 1 | 49 | LW1_OE X_PCT | B2965 | E. coli | 4261 * | plastidic | - |
| 1 | 50 | LW1_OE X_PCT | B2987 | E. coli | 4351 | plastidic | - |
| 1 | 51 | LW1_OE X_PCT | B2987 | E. coli | 4351 | non-targeted | - |
| 1 | 52 | LW1_OE X_PCT | B3093 | E. coli | 4460 | plastidic | - |
| 1 | 53 | LW1_OE X_PCT | B3363 | E. coli | 4506 | plastidic | 4600, 4602, 4604, 4606, 4608, 4610, 4612, 4614, 4616, 4618, 4620, 4622, 4624, 4626, 4628, 4630, 4632, 4634, 10023, 10025, 10027, 10029, 10031, 10033, 10035, 10037 |
| 1 | 54 | LW1_OE X_PCT | B3429 | E. coli | 4641 | plastidic | - |
| 1 | 55 | LW1_OE X_PCT | B3568 | E. coli | 4807 | plastidic | - |
| 1 | 56 | LW1_OE X_PCT | B3616 | E. coli | 5125 | plastidic | 5345, 5347, 5349, 5351, 5353, 5355, 5357, 5359, 5361, 5363, 5365, 5367, 5369, 5371, 5373, 5375, 5377, 5379, 5381, 5383, 5385, 5387, 5389, 5391, 5393, 5395, 5397, 5399, 5401, 5403, 5405, 5407, 5409, 5411, 10041, 10043, 10045, 10047, 10049, 10051, 10053, 10055, 10057 |
| 1 | 57 | LW1_OE X_PCT | B3616 | E. coli | 5125 | non-targeted | 5345, 5347, 5349, 5351, 5353, 5355, 5357, 5359, 5361, 5363, 5365, 5367, 5369, 5371, 5373, 5375, 5377, 5379, 5381, 5383, 5385, 5387, 5389, 5391, 5393, 5395, 5397, 5399, 5401, 5403, 5405, 5407, 5409, 5411, 10041, 10043, 10045, 10047, 10049, 10051, 10053, 10055, 10057 |
| 1 | 58 | LW1_OE X_PCT | B3812 | E. coli | 5418 | non-targeted | - |
| 1 | 59 | LW1_OE X_PCT | B3899 | E. coli | 5496 * | non-targeted | - |
| 1 | 60 | LW1_OE X_PCT | B3929 | E. coli | 5586 | plastidic | 5778, 5780, 5782, 5784, 5786, 5788, 5790, 5792, 5794, 10061 |
| 1 | 61 | LW1_OE X_PCT | B3938 | E. coli | 5801 | non-targeted | - |
| 1 | 62 | LW1_OE X_PCT | B3974 | E. coli | 5851 | non-targeted | - |
| 1 | 63 | LW1_OE X_PCT | B3989 | E. coli | 5993 | non-targeted | - |
| 1 | 64 | LW1_OE X_PCT | B4029 | E. coli | 6000 | non-targeted | - |
| 1 | 65 | LW1_OE X_PCT | B4139 | E. coli | 6057 * | plastidic | 6475, 6477, 6479, 6481, 6483, 6485, 6487, 10069 |
| 1 | 66 | LW1_OE X_PCT | B4390 | E. coli | 6501 * | non-targeted | - |
| 1 | 67 | LW1_OE X_PCT | SII0290 | Synec hocysti s | 6543 | non-targeted | - |
| 1 | 68 | LW1_OE X_PCT | YAL049C | Yeast | 6824 | non-targeted | 10073, 10075 |
| 1 | 69 | LW1_OE X_PCT | YCR059C | Yeast | 6871 | non-targeted | - |
| 1 | 70 | LW1_OE X_PCT | YDR035W | Yeast | 6911 | plastidic | - |
| 1 | 71 | LW1_OE X_PCT | YEL005C | Yeast | 7262 | non-targeted | - |
| 1 | 72 | LW1_OE X_PCT | YER112W | Yeast | 7266 * | non-targeted | 7270, 7272, 7274, 7276, 7278, 7280, 7282, 7284, 7286, 7288, 7290, 7292, 7294, 7296 |
| 1 | 73 | LW1_OE X_PCT | YER156C | Yeast | 7302 | non-targeted | 7370, 7372, 7374, 10079 |
| 1 | 74 | LW1_OE X_PCT | YER173W | Yeast | 7385 | non-targeted | - |
| 1 | 75 | LW1_OE X_PCT | YGL045W | Yeast | 7408 * | non-targeted | - |
| 1 | 76 | LW1_OE X_PCT | YGL189C | Yeast | 7430 | non-targeted | 7488, 7490, 7492, 7494, 7496, 7498, 7500, 7502, 7504, 7506, 7508, 7510, 7512, 7514, 7516, 7518, 7520, 7522, 7524, 7526, 7528, 7530, 7532, 7534, 7536, 7538, 7540, 7542, 7544, 7546, 7548, 7550, 7552, 10087, 10089, 10091 |
| 1 | 77 | LW1_OE X_PCT | YNR015W | Yeast | 7559 | non-targeted | 7595, 7597, 10095 |
| 1 | 78 | LW1_OE X_PCT | YOR024W | Yeast | 7607 | non-targeted | - |
| 1 | 79 | LW1_OE X_PCT | YOR168W | Yeast | 7611 * | non-targeted | 7653, 7655, 7657, 7659, 7661, 7663, 7665, 7667, 7669, 7671, 7673 |
| 1 | 80 | LW1_OE X_PCT | YPL151C | Yeast | 7686 | non-targeted | 7728, 7730, 7732 |
| 1 | 81 | LW1_OE X_PCT | B1297 | E. coli | 1202 * | plastidic | - |
| 1 | 82 | LW1_OE X_PCT | B0970 | E. coli | 7742 | non-targeted | 10009 |
| 1 | 83 | LW1_OE X_PCT | B1829 | E. coli | 7851 | non-targeted | - |
| 1 | 84 | LW1_OE X_PCT | B2664 | E. coli | 7972 * | non-targeted | - |
| 1 | 85 | LW1_OE X_PCT | B2796 | E. coli | 8022 | non-targeted | - |
| 1 | 86 | LW1_OE X_PCT | YER174C | Yeast | 8178 | non-targeted | 8234, 8236, 8238, 8240, 8242, 8244, 8246, 8248, 8250, 8252, 8254, 8256, 8258, 8260, 8262, 8264, 8266, 10083 |
| 1 | 87 | LW1_OE X_PCT | YFR042W | Yeast | 8273 | non-targeted | - |
| 1 | 88 | LW1_OE X_PCT | YKR057W | Yeast | 8289 | non-targeted | 8379, 8381, 8383, 8385, 8387, 8389, 8391, 8393, 8395, 8397, 8399, 8401, 8403, 8405, 8407, 8409, 8411, 8413, 8415, 8417, 8419, 8421, 8423, 8425 |
| 1 | 89 | LW1_OE X_PCT | B0629_2 | E. coli | 8439 | non-targeted | - |
| 1 | 90 | LW1_OE X_PCT | B1007_2 | E. coli | 8631 | non-targeted | - |
| 1 | 91 | LW1_OE X_PCT | B2715_2 | E. coli | 9269 | plastidic | - |
| 1 | 92 | LW1_OE X_PCT | B3899 2 | E. coli | 9445 | non-targeted | - |
| 1 | 93 | LW1_OE X_PCT | B4390_2 | E. coli | 9825 | non-targeted | - |
| 1 | 94 | LW1_OE X_PCT | YGL045W_2 | Yeast | 9906 | non-targeted | - |
| 1 | 95 | LW1_OE X_PCT | B2664_2 | E. coli | 9194 | non-targeted | - |
| 1 | 96 | LW1_OE X_PCT | B0963_2 | E. coli | 8498 | non-targeted | - |
| 1 | 97 | LW1_OE X_PCT | B1297_2 | E. coli | 8743 | plastidic | - |
| 1 | 98 | LW1_OE X_PCT | B1597_2 | E. coli | 8892 | non-targeted | - |
| 1 | 99 | LW1_OE X_PCT | B2027_2 | E. coli | 9032 | non-targeted | - |
| 1 | 100 | LW1_OE X_PCT | B2965_2 | E. coli | 9316 | plastidic | - |
| 1 | 101 | LW1_OE X_PCT | B4139_2 | E. coli | 9530 | plastidic | 9806, 9808, 9810, 9812, 10065 |
| 1 | 102 | LW1_OE X_PCT | B0845_2 | E. coli | 8463 | non-targeted | - |
| 1 | 103 | LW1_OE X_PCT | B1901_2 | E. coli | 8974 | plastidic | - |
| 1 | 104 | LW1_OE X_PCT | YER112W_2 | Yeast | 9884 | non-targeted | 9888, 9890, 9892, 9894, 9896, 9898, 9900 |
| 1 | 105 | LW1_OE X_PCT | B1798_2 | E. coli | 8935 | non-targeted | - |
| 1 | 106 | LW1_OE X_PCT | B2226_2 | E. coli | 9094 | non-targeted | - |
| 1 | 107 | LW1_OE X_PCT | B2469_2 | E. coli | 9110 | non-targeted | - |
| 1 | 108 | LW1_OE X_PCT | YOR168W_2 | Yeast | 9932 | non-targeted | 9974, 9976, 9978, 9980, 9982, 9984, 9986, 9988, 9990, 9992, 9994 |
| 1 | 109 | LW1_OE X_PCT | B4321 | E. coli | 10097 | non-targeted | - |

| | | | | | | | |
|---|---|---|---|---|---|---|---|
| * Sequences marked with asterisk * reflect the respective sequence derived from public data bases information | | | | | | | |

**Table III: Primer nucleic acid sequence ID numbers**

| | **1.** | **2.** | **3.** | **4.** | **5.** | **6.** | **7.** |
|---|---|---|---|---|---|---|---|
| Application | Hit | Project | Locus | Organism | Lead SEQ ID | Target | SEQ IDs of Primers |
| 1 | 1 | LW1_OE X_PCT | B0081 | E. coli | 38 | non-targeted | 48, 49 |
| 1 | 2 | LW1_OE X_PCT | B0445 | E. coli | 54 | non-targeted | 64, 65 |
| 1 | 3 | LW1_OE X_PCT | B0482 | E. coli | 70 | non-targeted | 82, 83 |
| 1 | 4 | LW1_OE X_PCT | B0607 | E. coli | 89 | non-targeted | 139, 140 |
| 1 | 5 | LW1_OE X_PCT | B0629 | E. coli | 143 | non-targeted | 153, 154 |
| 1 | 6 | LW1_OE X_PCT | B0631 | E. coli | 162 | non-targeted | 208, 209 |
| 1 | 7 | LW1_OE X_PCT | B0697 | E. coli | 213 | non-targeted | 341, 342 |
| 1 | 8 | LW1_OE X_PCT | B0753 | E. coli | 358 | non-targeted | 362, 363 |
| 1 | 9 | LW1_OE X_PCT | B0813 | E. coli | 367 | non-targeted | 413, 414 |
| 1 | 10 | LW1_OE X_PCT | B0845 | E. coli | 420 | non-targeted | 444, 445 |
| 1 | 11 | LW1_OE X_PCT | B0866 | E. coli | 455 | non-targeted | 529, 530 |
| 1 | 12 | LW1_OE X_PCT | B0963 | E. coli | 535 | non-targeted | 613, 614 |
| 1 | 13 | LW1_OE X_PCT | B0975 | E. coli | 618 | non-targeted | 666, 667 |
| 1 | 14 | LW1_OE X_PCT | B1007 | E. coli | 671 | non-targeted | 759, 760 |
| 1 | 15 | LW1_OE X_PCT | B1052 | E. coli | 764 | non-targeted | 766, 767 |
| 1 | 16 | LW1_OE X_PCT | B1091 | E. coli | 768 | plastidic | 896, 897 |
| 1 | 17 | LW1_OE X_PCT | B1161 | E. coli | 907 | non-targeted | 923, 924 |
| 1 | 18 | LW1_OE X_PCT | B1186 | E. coli | 927 | non-targeted | 997, 998 |
| 1 | 19 | LW1_OE X_PCT | B1291 | E. coli | 1009 | plastidic | 1145, 1146 |
| 1 | 20 | LW1_OE X_PCT | B1294 | E. coli | 1154 | plastidic | 1188, 1189 |
| 1 | 21 | LW1_OE X_PCT | B1423 | E. coli | 1308 | non-targeted | 1354, 1355 |
| 1 | 22 | LW1_OE X_PCT | B1597 | E. coli | 1368 | non-targeted | 1372, 1373 |
| 1 | 23 | LW1_OE X_PCT | B1605 | E. coli | 1374 | non-targeted | 1496, 1497 |
| 1 | 24 | LW1_OE X_PCT | B1704 | E. coli | 1507 | non-targeted | 1941, 1942 |
| 1 | 25 | LW1_OE X_PCT | B1736 | E. coli | 1953 | plastidic | 2151, 2152 |
| 1 | 26 | LW1_OE X_PCT | B1798 | E. coli | 2156 | non-targeted | 2188, 2189 |
| 1 | 27 | LW1_OE X_PCT | B1878 | E. coli | 2195 | non-targeted | 2213, 2214 |
| 1 | 28 | LW1_OE X_PCT | B1901 | E. coli | 2219 | plastidic | 2269, 2270 |
| 1 | 29 | LW1_OE X_PCT | B1912 | E. coli | 2277 | plastidic | 2463, 2464 |
| 1 | 30 | LW1_OE X_PCT | B2027 | E. coli | 2470 | non-targeted | 2482, 2483 |
| 1 | 31 | LW1_OE X_PCT | B2039 | E. coli | 2493 | non-targeted | 2619, 2620 |
| 1 | 32 | LW1_OE X_PCT | B2075 | E. coli | 2627 | non-targeted | 2847, 2848 |
| 1 | 33 | LW1_OE X_PCT | B2153 | E. coli | 2858 | plastidic | 2934, 2935 |
| 1 | 34 | LW1_OE X_PCT | B2194 | E. coli | 2942 | non-targeted | 2958, 2959 |
| 1 | 35 | LW1_OE X_PCT | B2226 | E. coli | 2965 | non-targeted | 2975, 2976 |
| 1 | 36 | LW1_OE X_PCT | B2309 | E. coli | 2981 | plastidic | 3121, 3122 |
| 1 | 37 | LW1_OE X_PCT | B2469 | E. coli | 3130 | non-targeted | 3206, 3207 |
| 1 | 38 | LW1_OE X_PCT | B2475 | E. coli | 3216 | non-targeted | 3328, 3329 |
| 1 | 39 | LW1_OE X_PCT | B2482 | E. coli | 3335 | non-targeted | 3395, 3396 |
| 1 | 40 | LW1_OE X_PCT | B2541 | E. coli | 3401 | non-targeted | 3585, 3586 |
| 1 | 41 | LW1_OE X_PCT | B2559 | E. coli | 3590 | plastidic | 3826, 3827 |
| 1 | 42 | LW1_OE X_PCT | B2605 | E. coli | 3831 | non-targeted | 3853, 3854 |
| 1 | 43 | LW1_OE X_PCT | B2630 | E. coli | 3857 | non-targeted | 3859, 3860 |
| 1 | 44 | LW1_OE X_PCT | B2678 | E. coli | 3861 | plastidic | 4015, 4016 |
| 1 | 45 | LW1_OE X_PCT | B2715 | E. coli | 4022 | plastidic | 4050, 4051 |
| 1 | 46 | LW1_OE X_PCT | B2776 | E. coli | 4059 | non-targeted | 4071, 4072 |
| 1 | 47 | LW1_OE X_PCT | B2791 | E. coli | 4076 | non-targeted | 4148, 4149 |
| 1 | 48 | LW1_OE X_PCT | B2912 | E. coli | 4157 | non-targeted | 4255, 4256 |
| 1 | 49 | LW1_OE X_PCT | B2965 | E. coli | 4260 | plastidic | 4332, 4333 |
| 1 | 50 | LW1_OE X_PCT | B2987 | E. coli | 4350 | plastidic | 4450, 4451 |
| 1 | 51 | LW1_OE X_PCT | B2987 | E. coli | 4350 | non-targeted | 4450, 4451 |
| 1 | 52 | LW1_OE X_PCT | B3093 | E. coli | 4459 | plastidic | 4495, 4496 |
| 1 | 53 | LW1_OE X_PCT | B3363 | E. coli | 4505 | plastidic | 4635, 4636 |
| 1 | 54 | LW1_OE X_PCT | B3429 | E. coli | 4640 | plastidic | 4798, 4799 |
| 1 | 55 | LW1_OE X_PCT | B3568 | E. coli | 4806 | plastidic | 5118, 5119 |
| 1 | 56 | LW1_OE X_PCT | B3616 | E. coli | 5124 | plastidic | 5412, 5413 |
| 1 | 57 | LW1_OE X_PCT | B3616 | E. coli | 5124 | non-targeted | 5412, 5413 |
| 1 | 58 | LW1_OE X_PCT | B3812 | E. coli | 5417 | non-targeted | 5489, 5490 |
| 1 | 59 | LW1_OE X_PCT | B3899 | E. coli | 5495 | non-targeted | 5579, 5580 |
| 1 | 60 | LW1_OE X_PCT | B3929 | E. coli | 5585 | plastidic | 5795, 5796 |
| 1 | 61 | LW1_OE X_PCT | B3938 | E. coli | 5800 | non-targeted | 5844, 5845 |
| 1 | 62 | LW1_OE X_PCT | B3974 | E. coli | 5850 | non-targeted | 5982, 5983 |
| 1 | 63 | LW1_OE X_PCT | B3989 | E. coli | 5992 | non-targeted | 5996, 5997 |
| 1 | 64 | LW1_OE X_PCT | B4029 | E. coli | 5999 | non-targeted | 6041, 6042 |
| 1 | 65 | LW1_OE X_PCT | B4139 | E. coli | 6056 | plastidic | 6488, 6489 |
| 1 | 66 | LW1_OE X_PCT | B4390 | E. coli | 6500 | non-targeted | 6530, 6531 |
| 1 | 67 | LW1_OE X_PCT | SII0290 | Synec hocysti s | 6542 | non-targeted | 6812, 6813 |
| 1 | 68 | LW1_OE X_PCT | YAL049C | Yeast | 6823 | non-targeted | 6863, 6864 |
| 1 | 69 | LW1_OE X_PCT | YCR059C | Yeast | 6870 | non-targeted | 6902, 6903 |
| 1 | 70 | LW1_OE X_PCT | YDR035W | Yeast | 6910 | plastidic | 7250, 7251 |
| 1 | 71 | LW1_OE X_PCT | YEL005C | Yeast | 7261 | non-targeted | 7263, 7264 |
| 1 | 72 | LW1_OE X_PCT | YER112W | Yeast | 7265 | non-targeted | 7297, 7298 |
| 1 | 73 | LW1_OE X_PCT | YER156C | Yeast | 7301 | non-targeted | 7375, 7376 |
| 1 | 74 | LW1_OE X_PCT | YER173W | Yeast | 7384 | non-targeted | 7394, 7395 |
| 1 | 75 | LW1_OE X_PCT | YGL045W | Yeast | 7407 | non-targeted | 7417, 7418 |
| 1 | 76 | LW1_OE X_PCT | YGL189C | Yeast | 7429 | non-targeted | 7553, 7554 |
| 1 | 77 | LW1_OE X_PCT | YNR015W | Yeast | 7558 | non-targeted | 7598, 7599 |
| 1 | 78 | LW1_OE X_PCT | YOR024W | Yeast | 7606 | non-targeted | 7608, 7609 |
| 1 | 79 | LW1_OE X_PCT | YOR168W | Yeast | 7610 | non-targeted | 7674, 7675 |
| 1 | 80 | LW1_OE X_PCT | YPL151C | Yeast | 7685 | non-targeted | 7733, 7734 |
| 1 | 81 | LW1_OE X_PCT | B1297 | E. coli | 1201 | plastidic | 1298, 1299 |
| 1 | 82 | LW1_OE X_PCT | B0970 | E. coli | 7741 | non-targeted | 7843, 7844 |
| 1 | 83 | LW1_OE X_PCT | B1829 | E. coli | 7850 | non-targeted | 7962, 7963 |
| 1 | 84 | LW1_OE X_PCT | B2664 | E. coli | 7971 | non-targeted | 8015, 8016 |
| 1 | 85 | LW1_OE X_PCT | B2796 | E. coli | 8021 | non-targeted | 8167, 8168 |
| 1 | 86 | LW1_OE X_PCT | YER174C | Yeast | 8177 | non-targeted | 8267, 8268 |
| 1 | 87 | LW1_OE X_PCT | YFR042W | Yeast | 8272 | non-targeted | 8282, 8283 |
| 1 | 88 | LW1_OE X_PCT | YKR057W | Yeast | 8288 | non-targeted | 8426, 8427 |
| 1 | 89 | LW1_OE X_PCT | B0629_2 | E. coli | 8438 | non-targeted | 8452, 8453 |
| 1 | 90 | LW1_OE X_PCT | B1007_2 | E. coli | 8630 | non-targeted | 8738, 8739 |
| 1 | 91 | LW1_OE X_PCT | B2715_2 | E. coli | 9268 | plastidic | 9306, 9307 |
| 1 | 92 | LW1_OE X_PCT | B3899_2 | E. coli | 9444 | non-targeted | 9524, 9525 |
| 1 | 93 | LW1_OE X_PCT | B4390_2 | E. coli | 9824 | non-targeted | 9870, 9871 |
| 1 | 94 | LW1_OE X_PCT | YGL045W_2 | Yeast | 9905 | non-targeted | 9925, 9926 |
| 1 | 95 | LW1_OE X_PCT | B2664 2 | E. coli | 9193 | non-targeted | 9263, 9264 |
| 1 | 96 | LW1_OE X_PCT | B0963_2 | E. coli | 8497 | non-targeted | 8625, 8626 |
| 1 | 97 | LW1_OE X_PCT | B1297_2 | E. coli | 8742 | plastidic | 8884, 8885 |
| 1 | 98 | LW1_OE X_PCT | B1597_2 | E. coli | 8891 | non-targeted | 8929, 8930 |
| 1 | 99 | LW1_OE X_PCT | B2027_2 | E. coli | 9031 | non-targeted | 9087, 9088 |
| 1 | 100 | LW1_OE X_PCT | B2965_2 | E. coli | 9315 | plastidic | 9431, 9432 |
| 1 | 101 | LW1_OE X_PCT | B4139_2 | E. coli | 9529 | plastidic | 9813, 9814 |
| 1 | 102 | LW1_OE X_PCT | B0845 2 | E. coli | 8462 | non-targeted | 8486, 8487 |
| 1 | 103 | LW1_OE X_PCT | B1901_2 | E. coli | 8973 | plastidic | 9023, 9024 |
| 1 | 104 | LW1_OE X_PCT | YER112W_2 | Yeast | 9883 | non-targeted | 9901, 9902 |
| 1 | 105 | LW1_OE X_PCT | B1798_2 | E. coli | 8934 | non-targeted | 8966, 8967 |
| 1 | 106 | LW1_OE X_PCT | B2226_2 | E. coli | 9093 | non-targeted | 9103, 9104 |
| 1 | 107 | LW1_OE X_PCT | B2469_2 | E. coli | 9109 | non-targeted | 9183, 9184 |
| 1 | 108 | LW1_OE X_PCT | YOR168W_2 | Yeast | 9931 | non-targeted | 9995, 9996 |
| 1 | 109 | LW1_OE X_PCT | B4321 | E. coli | 10096 | non-targeted | 10250, 10251 |

**Table IV: Consensus amino acid sequence ID numbers**

| | **1.** | **2.** | **3.** | **4.** | **5.** | **6.** | **7.** |
|---|---|---|---|---|---|---|---|
| Application | Hit | Project | Locus | Organism | Lead SEQ ID | Target | SEQ IDs of Consensus / Pattern Sequences |
| 1 | 1 | LW1_OE X_PCT | B0081 | E. coli | 39 | non-targeted | 50, 51, 52, 53 |
| 1 | 2 | LW1_OE X_PCT | B0445 | E. coli | 55 | non-targeted | 66, 67, 68, 69 |
| 1 | 3 | LW1_OE X_PCT | B0482 | E. coli | 71 | non-targeted | 84, 85, 86, 87, 88 |
| 1 | 4 | LW1_OE X_PCT | B0607 | E. coli | 90 | non-targeted | 141, 142 |
| 1 | 5 | LW1_OE X_PCT | B0629 | E. coli | 144 | non-targeted | 155, 156, 157, 158, 159, 160, 161 |
| 1 | 6 | LW1_OE X_PCT | B0631 | E. coli | 163 | non-targeted | 210,211,212 |
| 1 | 7 | LW1_OE X_PCT | B0697 | E. coli | 214 | non-targeted | 343, 344, 345, 346, 347, 348, 349, 350, 351, 352, 353, 354, 355, 356, 357 |
| 1 | 8 | LW1_OE X_PCT | B0753 | E. coli | 359 | non-targeted | 364, 365, 366 |
| 1 | 9 | LW1_OE X_PCT | B0813 | E. coli | 368 | non-targeted | 415, 416, 417, 418, 419 |
| 1 | 10 | LW1_OE X_PCT | B0845 | E. coli | 421 | non-targeted | 446, 447, 448, 449, 450, 451, 452, 453, 454 |
| 1 | 11 | LW1_OE X_PCT | B0866 | E. coli | 456 | non-targeted | 531, 532, 533, 534 |
| 1 | 12 | LW1_OE X_PCT | B0963 | E. coli | 536 | non-targeted | 615, 616, 617 |
| 1 | 13 | LW1_OE X_PCT | B0975 | E. coli | 619 | non-targeted | 668, 669, 670 |
| 1 | 14 | LW1_OE X_PCT | B1007 | E. coli | 672 | non-targeted | 761, 762, 763 |
| 1 | 15 | LW1_OE X_PCT | B1052 | E. coli | 765 | non-targeted | - |
| 1 | 16 | LW1_OE X_PCT | B1091 | E. coli | 769 | plastidic | 898, 899, 900, 901, 902, 903, 904, 905, 906 |
| 1 | 17 | LW1_OE X_PCT | B1161 | E. coli | 908 | non-targeted | 925, 926 |
| 1 | 18 | LW1_OE X_PCT | B1186 | E. coli | 928 | non-targeted | 999, 1000, 1001, 1002, 1003, 1004, 1005, 1006, 1007, 1008 |
| 1 | 19 | LW1_OE X_PCT | B1291 | E. coli | 1010 | plastidic | 1147, 1148, 1149, 1150, 1151, 1152, 1153 |
| 1 | 20 | LW1_OE X_PCT | B1294 | E. coli | 1155 | plastidic | 1190, 1191, 1192, 1193, 1194, 1195, 1196, 1197, 1198, 1199, 1200 |
| 1 | 21 | LW1_OE X_PCT | B1423 | E. coli | 1309 | non-targeted | 1356, 1357, 1358, 1359, 1360, 1361, 1362, 1363, 1364, 1365, 1366, 1367 |
| 1 | 22 | LW1_OE X_PCT | B1597 | E. coli | 1369 | non-targeted | - |
| 1 | 23 | LW1_OE X_PCT | B1605 | E. coli | 1375 | non-targeted | 1498, 1499, 1500, 1501, 1502, 1503, 1504, 1505, 1506 |
| 1 | 24 | LW1_OE X_PCT | B1704 | E. coli | 1508 | non-targeted | 1943, 1944, 1945, 1946, 1947, 1948, 1949, 1950, 1951, 1952 |
| 1 | 25 | LW1_OE X_PCT | B1736 | E. coli | 1954 | plastidic | 2153, 2154, 2155 |
| 1 | 26 | LW1_OE X_PCT | B1798 | E. coli | 2157 | non-targeted | 2190, 2191, 2192, 2193, 2194 |
| 1 | 27 | LW1_OE X_PCT | B1878 | E. coli | 2196 | non-targeted | 2215, 2216, 2217, 2218 |
| 1 | 28 | LW1_OE X_PCT | B1901 | E. coli | 2220 | plastidic | 2271, 2272, 2273, 2274, 2275, 2276 |
| 1 | 29 | LW1_OE X_PCT | B1912 | E. coli | 2278 | plastidic | 2465, 2466, 2467, 2468, 2469 |
| 1 | 30 | LW1_OE X_PCT | B2027 | E. coli | 2471 | non-targeted | 2484, 2485, 2486, 2487, 2488, 2489, 2490, 2491, 2492 |
| 1 | 31 | LW1_OE X_PCT | B2039 | E. coli | 2494 | non-targeted | 2621, 2622, 2623, 2624, 2625, 2626 |
| 1 | 32 | LW1_OE X_PCT | B2075 | E. coli | 2628 | non-targeted | 2849, 2850, 2851, 2852, 2853, 2854, 2855, 2856, 2857 |
| 1 | 33 | LW1_OE X_PCT | B2153 | E. coli | 2859 | plastidic | 2936, 2937, 2938, 2939, 2940, 2941 |
| 1 | 34 | LW1_OE X_PCT | B2194 | E. coli | 2943 | non-targeted | 2960, 2961, 2962, 2963, 2964 |
| 1 | 35 | LW1_OE X_PCT | B2226 | E. coli | 2966 | non-targeted | 2977, 2978, 2979, 2980 |
| 1 | 36 | LW1_OE X_PCT | B2309 | E. coli | 2982 | plastidic | 3123, 3124, 3125, 3126, 3127, 3128, 3129 |
| 1 | 37 | LW1_OE X_PCT | B2469 | E. coli | 3131 | non-targeted | 3208, 3209, 3210, 3211, 3212, 3213, 3214, 3215 |
| 1 | 38 | LW1_OE X_PCT | B2475 | E. coli | 3217 | non-targeted | 3330,3331,3332,3333,3334 |
| 1 | 39 | LW1_OE X_PCT | B2482 | E. coli | 3336 | non-targeted | 3397, 3398, 3399, 3400 |
| 1 | 40 | LW1_OE X_PCT | B2541 | E. coli | 3402 | non-targeted | 3587, 3588, 3589 |
| 1 | 41 | LW1_OE X_PCT | B2559 | E. coli | 3591 | plastidic | 3828, 3829, 3830 |
| 1 | 42 | LW1_OE X_PCT | B2605 | E. coli | 3832 | non-targeted | 3855, 3856 |
| 1 | 43 | LW1_OE X_PCT | B2630 | E. coli | 3858 | non-targeted | - |
| 1 | 44 | LW1_OE X_PCT | B2678 | E. coli | 3862 | plastidic | 4017, 4018, 4019, 4020, 4021 |
| 1 | 45 | LW1_OE X_PCT | B2715 | E. coli | 4023 | plastidic | 4052, 4053, 4054, 4055, 4056, 4057, 4058 |
| 1 | 46 | LW1_OE X_PCT | B2776 | E. coli | 4060 | non-targeted | 4073, 4074, 4075 |
| 1 | 47 | LW1_OE X_PCT | B2791 | E. coli | 4077 | non-targeted | 4150, 4151, 4152, 4153, 4154, 4155, 4156 |
| 1 | 48 | LW1_OE X_PCT | B2912 | E. coli | 4158 | non-targeted | 4257, 4258, 4259 |
| 1 | 49 | LW1_OE X_PCT | B2965 | E. coli | 4261 | plastidic | 4334, 4335, 4336, 4337, 4338, 4339, 4340, 4341, 4342, 4343, 4344, 4345, 4346, 4347, 4348, 4349 |
| 1 | 50 | LW1_OE X_PCT | B2987 | E. coli | 4351 | plastidic | 4452, 4453, 4454, 4455, 4456, 4457, 4458 |
| 1 | 51 | LW1_OE X_PCT | B2987 | E. coli | 4351 | non-targeted | 4452, 4453, 4454, 4455, 4456, 4457, 4458 |
| 1 | 52 | LW1_OE X_PCT | B3093 | E. coli | 4460 | plastidic | 4497, 4498, 4499, 4500, 4501, 4502, 4503, 4504 |
| 1 | 53 | LW1_OE X_PCT | B3363 | E. coli | 4506 | plastidic | 4637, 4638, 4639 |
| 1 | 54 | LW1_OE X_PCT | B3429 | E. coli | 4641 | plastidic | 4800, 4801, 4802, 4803, 4804, 4805 |
| 1 | 55 | LW1_OE X_PCT | B3568 | E. coli | 4807 | plastidic | 5120, 5121, 5122, 5123 |
| 1 | 56 | LW1_OE X_PCT | B3616 | E. coli | 5125 | plastidic | 5414, 5415, 5416 |
| 1 | 57 | LW1_OE X_PCT | B3616 | E. coli | 5125 | non-targeted | 5414, 5415, 5416 |
| 1 | 58 | LW1_OE X_PCT | B3812 | E. coli | 5418 | non-targeted | 5491, 5492, 5493, 5494 |
| 1 | 59 | LW1_OE X_PCT | B3899 | E. coli | 5496 | non-targeted | 5581, 5582, 5583, 5584 |
| 1 | 60 | LW1_OE X_PCT | B3929 | E. coli | 5586 | plastidic | 5797, 5798, 5799 |
| 1 | 61 | LW1_OE X_PCT | B3938 | E. coli | 5801 | non-targeted | 5846, 5847, 5848, 5849 |
| 1 | 62 | LW1_OE X_PCT | B3974 | E. coli | 5851 | non-targeted | 5984, 5985, 5986, 5987, 5988, 5989, 5990, 5991 |
| 1 | 63 | LW1_OE X_PCT | B3989 | E. coli | 5993 | non-targeted | 5998 |
| 1 | 64 | LW1_OE X_PCT | B4029 | E. coli | 6000 | non-targeted | 6043, 6044, 6045, 6046, 6047, 6048, 6049, 6050, 6051, 6052, 6053, 6054, 6055 |
| 1 | 65 | LW1_OE X_PCT | B4139 | E. coli | 6057 | plastidic | 6490, 6491, 6492, 6493, 6494, 6495, 6496, 6497, 6498, 6499 |
| 1 | 66 | LW1_OE X_PCT | B4390 | E. coli | 6501 | non-targeted | 6532, 6533, 6534, 6535, 6536, 6537, 6538, 6539, 6540, 6541 |
| 1 | 67 | LW1_OE X_PCT | SII0290 | Synec hocysti s | 6543 | non-targeted | 6814, 6815, 6816, 6817, 6818, 6819, 6820, 6821, 6822 |
| 1 | 68 | LW1_OE X_PCT | YAL049C | Yeast | 6824 | non-targeted | 6865, 6866, 6867, 6868, 6869 |
| 1 | 69 | LW1_OE X_PCT | YCR059C | Yeast | 6871 | non-targeted | 6904, 6905, 6906, 6907, 6908, 6909 |
| 1 | 70 | LW1_OE X_PCT | YDR035W | Yeast | 6911 | plastidic | 7252, 7253, 7254, 7255, 7256, 7257, 7258, 7259, 7260 |
| 1 | 71 | LW1_OE X_PCT | YEL005C | Yeast | 7262 | non-targeted | - |
| 1 | 72 | LW1_OE X_PCT | YER112W | Yeast | 7266 | non-targeted | 7299, 7300 |
| 1 | 73 | LW1_OE X_PCT | YER156C | Yeast | 7302 | non-targeted | 7377, 7378, 7379, 7380, 7381, 7382, 7383 |
| 1 | 74 | LW1_OE X_PCT | YER173W | Yeast | 7385 | non-targeted | 7396, 7397, 7398, 7399, 7400, 7401, 7402, 7403, 7404, 7405, 7406 |
| 1 | 75 | LW1_OE X_PCT | YGL045W | Yeast | 7408 | non-targeted | 7419, 7420, 7421, 7422, 7423, 7424, 7425, 7426, 7427, 7428 |
| 1 | 76 | LW1_OE X_PCT | YGL189C | Yeast | 7430 | non-targeted | 7555, 7556, 7557 |
| 1 | 77 | LW1_OE X_PCT | YNR015W | Yeast | 7559 | non-targeted | 7600, 7601, 7602, 7603, 7604, 7605 |
| 1 | 78 | LW1_OE X_PCT | YOR024W | Yeast | 7607 | non-targeted | - |
| 1 | 79 | LW1_OE X_PCT | YOR168W | Yeast | 7611 | non-targeted | 7676, 7677, 7678, 7679, 7680, 7681, 7682, 7683, 7684 |
| 1 | 80 | LW1_OE X_PCT | YPL151C | Yeast | 7686 | non-targeted | 7735, 7736, 7737, 7738, 7739, 7740 |
| 1 | 81 | LW1_OE X_PCT | B1297 | E. coli | 1202 | plastidic | 1300, 1301, 1302, 1303, 1304, 1305, 1306, 1307 |
| 1 | 82 | LW1_OE X_PCT | B0970 | E. coli | 7742 | non-targeted | 7845, 7846, 7847, 7848, 7849 |
| 1 | 83 | LW1_OE X_PCT | B1829 | E. coli | 7851 | non-targeted | 7964, 7965, 7966, 7967, 7968, 7969, 7970 |
| 1 | 84 | LW1_OE X_PCT | B2664 | E. coli | 7972 | non-targeted | 8017, 8018, 8019, 8020 |
| 1 | 85 | LW1_OE X_PCT | B2796 | E. coli | 8022 | non-targeted | 8169, 8170, 8171, 8172, 8173, 8174, 8175, 8176 |
| 1 | 86 | LW1_OE X_PCT | YER174C | Yeast | 8178 | non-targeted | 8269, 8270, 8271 |
| 1 | 87 | LW1_OE X_PCT | YFR042W | Yeast | 8273 | non-targeted | 8284, 8285, 8286, 8287 |
| 1 | 88 | LW1_OE X_PCT | YKR057W | Yeast | 8289 | non-targeted | 8428, 8429, 8430 |
| 1 | 89 | LW1_OE X_PCT | B0629_2 | E. coli | 8439 | non-targeted | 8454, 8455, 8456, 8457, 8458, 8459, 8460, 8461 |
| 1 | 90 | LW1_OE X_PCT | B1007_2 | E. coli | 8631 | non-targeted | 8740,8741 |
| 1 | 91 | LW1_OE X_PCT | B2715_2 | E. coli | 9269 | plastidic | 9308, 9309, 9310, 9311, 9312, 9313, 9314 |
| 1 | 92 | LW1_OE X_PCT | B3899_2 | E. coli | 9445 | non-targeted | 9526, 9527, 9528 |
| 1 | 93 | LW1_OE X_PCT | B4390_2 | E. coli | 9825 | non-targeted | 9872, 9873, 9874, 9875, 9876, 9877, 9878, 9879, 9880, 9881, 9882 |
| 1 | 94 | LW1_OE X_PCT | YGL045W_2 | Yeast | 9906 | non-targeted | 9927, 9928, 9929, 9930 |
| 1 | 95 | LW1_OE X_PCT | B2664 2 | E. coli | 9194 | non-targeted | 9265, 9266, 9267 |
| 1 | 96 | LW1_OE X_PCT | B0963_2 | E. coli | 8498 | non-targeted | 8627, 8628, 8629 |
| 1 | 97 | LW1_OE X_PCT | B1297_2 | E. coli | 8743 | plastidic | 8886, 8887, 8888, 8889, 8890 |
| 1 | 98 | LW1_OE X_PCT | B1597_2 | E. coli | 8892 | non-targeted | 8931,8932,8933 |
| 1 | 99 | LW1_OE X_PCT | B2027_2 | E. coli | 9032 | non-targeted | 9089, 9090, 9091, 9092 |
| 1 | 100 | LW1_OE X_PCT | B2965_2 | E. coli | 9316 | plastidic | 9433, 9434, 9435, 9436, 9437, 9438, 9439, 9440, 9441, 9442, 9443 |
| 1 | 101 | LW1_OE X_PCT | B4139_2 | E. coli | 9530 | plastidic | 9815, 9816, 9817, 9818, 9819, 9820, 9821, 9822, 9823 |
| 1 | 102 | LW1_OE X_PCT | B0845_2 | E. coli | 8463 | non-targeted | 8488, 8489, 8490, 8491, 8492, 8493, 8494, 8495, 8496 |
| 1 | 103 | LW1_OE X_PCT | B1901_2 | E. coli | 8974 | plastidic | 9025, 9026, 9027, 9028, 9029, 9030 |
| 1 | 104 | LW1_OE X_PCT | YER112W_2 | Yeast | 9884 | non-targeted | 9903, 9904 |
| 1 | 105 | LW1_OE X_PCT | B1798_2 | E. coli | 8935 | non-targeted | 8968, 8969, 8970, 8971, 8972 |
| 1 | 106 | LW1_OE X_PCT | B2226_2 | E. coli | 9094 | non-targeted | 9105, 9106, 9107, 9108 |
| 1 | 107 | LW1_OE X_PCT | B2469_2 | E. coli | 9110 | non-targeted | 9185, 9186, 9187, 9188, 9189, 9190, 9191, 9192 |
| 1 | 108 | LW1_OE X_PCT | YOR168W_2 | Yeast | 9932 | non-targeted | 9997, 9998, 9999, 10000, 10001, 10002, 10003, 10004, 10005 |
| 1 | 109 | LW1_OE X_PCT | B4321 | E. coli | 10097 | non-targeted | 10252, 10253, 10254, 10255, 10256, 10257 |

## Claims

1. A method for producing a transgenic plant cell, a plant or a part thereof with increased tolerance and/or resistance to environmental stress
and increased biomass production as compared to a corresponding non-transformed wild type plant cell, a plant or a part thereof by increasing or generating D-xylose transporter subunit activity

2. A method according to claim 1 wherein the activity of at least one polypeptide comprising a polypeptide selected from the group consisting of:
(i) a polypeptide comprising a polypeptide, a consensus sequence or at least one polypeptide motif as depicted in column 5 or 7 of Table II, Application No. 1, Hit 55 or of Table IV, Application No. 1, Hit 55, respectively; or
(ii) an expression product of a nucleic acid molecule comprising a polynucleotide as depicted in column 5 or 7 of Table I, Application No. 1, Hit 55,
(iii) or a functional equivalent of (i) or (ii);
is increased or generated
preferably wherein the expression of at least one nucleic acid molecule comprising a nucleic acid molecule selected from the group consisting of:
a) a nucleic acid molecule encoding the polypeptide shown in column 5 or 7 of Table II, Application No. 1, Hit 55;
b) a nucleic acid molecule shown in column 5 or 7 of Table I, Application No. 1, Hit 55;
c) a nucleic acid molecule, which, as a result of the degeneracy of the genetic code, can be derived from a polypeptide sequence depicted in column 5 or 7 of Table II, Application No. 1, Hit 55 and confers an increased tolerance and/or resistance to environmental stress
and increased biomass production as compared to a corresponding non-transformed wild type plant cell, a plant or a part thereof;
d) a nucleic acid molecule having at least 30 % identity with the nucleic acid molecule sequence of a polynucleotide comprising the nucleic acid molecule shown in column 5 or 7 of Table I, Application No. 1, Hit 55 and confers an increased tolerance and/or resistance to environmental stress
and increased biomass production as compared to a corresponding non-transformed wild type plant cell, a plant or a part thereof;
e) a nucleic acid molecule encoding a polypeptide having at least 30 % identity with the amino acid sequence of the polypeptide encoded by the nucleic acid molecule of (a) to (c) and having the activity represented by a nucleic acid molecule comprising a polynucleotide as depicted in column 5 of Table I, Application No. 1, Hit 55 and confers an increased tolerance and/or resistance to environmental stress
and increased biomass production as compared to a corresponding non-transformed wild type plant cell, a plant or a part thereof;
f) nucleic acid molecule which hybridizes with a nucleic acid molecule of (a) to (c) under stringent hybridization conditions and confers an increased tolerance and/or resistance to environmental stress
and increased biomass production as compared to a corresponding non-transformed wild type plant cell, a plant or a part thereof;
g) a nucleic acid molecule encoding a polypeptide which can be isolated with the aid of monoclonal or polyclonal antibodies made against a polypeptide encoded by one of the nucleic acid molecules of (a) to (e) and having the activity represented by the nucleic acid molecule comprising a polynucleotide as depicted in column 5 of Table I, Application No. 1, Hit 55;
h) a nucleic acid molecule encoding a polypeptide comprising the consensus sequence or one or more polypeptide motifs as shown in column 7 of Table IV, Application No. 1, Hit 55 and preferably having the activity represented by a nucleic acid molecule comprising a polynucleotide as depicted in columns 5 of Table II, Application No. 1, Hit 55 or IV, Application No. 1, Hit 55;
h) a nucleic acid molecule encoding a polypeptide having the activity represented by a protein as depicted in column 5 of Table II, Application No. 1, Hit 55 and confers an increased tolerance and/or resistance to environmental stress
and increased biomass production as compared to a corresponding non-transformed wild type plant cell, a plant or a part thereof;
i) nucleic acid molecule which comprises a polynucleotide, which is obtained by amplifying a cDNA library or a genomic library using the primers in column 7 of Table III, Application No. 1, Hit 55 which do not start at their 5'-end with the nucleotides ATA and preferably having the activity represented by a nucleic acid molecule comprising a polynucleotide as depicted in column 5 of Table II, Application No. 1, Hit 55 or IV, Application No. 1, Hit 55;
and
j) a nucleic acid molecule which is obtainable by screening a suitable nucleic acid library under stringent hybridization conditions with a probe comprising a complementary sequence of a nucleic acid molecule of (a) or (b) or with a fragment thereof, having at least 15 nt, preferably 2^0 nt, 30 nt, 50 nt, 100 nt, 200 nt or 500 nt of a nucleic acid molecule complementary to a nucleic acid molecule sequence **characterized in** (a) to (e) and encoding a polypeptide having the activity represented by a protein comprising a polypeptide as depicted in column 5 of Table II, Application No. 1, Hit 55;
is increased or generated.

3. A transgenic plant cell, a plant or a part thereof with increased tolerance and/or resistance to environmental stress
and increased biomass production as compared to a corresponding non-transformed wild type plant cell, a plant or a part thereof produced by a method according to claim 1.

4. The transgenic plant cell, a plant or a part thereof of claim 3 derived from a monocotyledonous or a dicotyledonous plant.

5. A seed produced by a transgenic plant of claim 4, wherein the seed is genetically homozygous for a transgene conferring increased tolerance and/or resistance to environmental stress
and increased biomass production as compared to a corresponding non-transformed wild type plant cell, a plant or a part thereof resulting in an increased tolerance to environmental stress
and increased biomass production as compared to a corresponding non-transformed wild type plant.

6. An isolated nucleic acid molecule comprising a nucleic acid molecule selected from the group consisting of:
a) a nucleic acid molecule encoding the polypeptide shown in column 5 or 7 of Table II B, Application No. 1, Hit 55;
b) a nucleic acid molecule shown in column 5 or 7 of Table I B, Application No. 1, Hit 55;
c) a nucleic acid molecule, which, as a result of the degeneracy of the genetic code, can be derived from a polypeptide sequence depicted in column 5 or 7 of Table II, Application No. 1, Hit 55 and confers an increased tolerance and/or resistance to environmental stress
and increased biomass production as compared to a corresponding non-transformed wild type plant cell, a plant or a part thereof.

7. A nucleic acid construct which confers the expression of said nucleic acid molecule of claim 6, comprising one or more regulatory elements, whereby expression of the nucleic acid in a host cell results in increased tolerance and/or resistance to environmental stress and increased biomass production as compared to a corresponding non-transformed wild type plant cell, a plant or a part thereof.

8. A vector comprising the nucleic acid molecule as claimed in claim 6 or the nucleic acid construct of claim 7, whereby expression of said coding nucleic acid in a host cell results in increased tolerance and/or resistance to environmental stress
and increased biomass production as compared to a corresponding non-transformed wild type plant cell, a plant or a part thereof.

9. A host cell, which has been transformed stably or transiently with the vector as claimed in claim 8 or the nucleic acid molecule as claimed in claim 6 or the nucleic acid construct of claim 7 and which shows due to the transformation an increased tolerance and/or resistance to environmental stress
and increased biomass production as compared to a corresponding non-transformed wild type plant cell, a plant or a part thereof.

10. A plant tissue, propagation material, harvested material or a plant comprising the host cell as claimed in claim 9.

11. A composition comprising the nucleic acid molecule of any of the claims 6, the nucleic acid construct of claim 7, the vector of claim 8, and optionally an agricultural acceptable carrier.

12. A method of producing a transgenic plant cell, a plant or a part thereof with increased tolerance and/or resistance to environmental stress
and increased biomass production compared to a corresponding non transformed wild type plant cell, a plant or a part thereof, wherein the tolerance and/or resistance to environmental stress
and increased biomass production is increased by expression of a polypeptide encoded by a nucleic acid according to claim 6 and results in increased tolerance and/or resistance to an environmental stress
and increased biomass production as compared to a corresponding non-transformed wild type plant cell, a plant or a part thereof, comprising
a) transforming a plant cell, or a part of a plant with an expression vector according to claim 8 and
b) generating from the plant cell or the part of a plant a transgenic plant with an increased tolerance to environmental stress
and increased biomass production as compared to a corresponding non-transformed wild type plant.

13. Use of a SRP encoding nucleic acid molecule selected from the group comprising the nucleic acid of claim 6 for preparing a plant cell with increased tolerance and/or resistance to environmental stress
and increased biomass production as compared to a corresponding non-transformed wild type plant cell, a plant or part of a plant.

14. A transgenic plant cell comprising a nucleic acid molecule encoding a polypeptide havingD-xylose transporter subunit activity, wherein said polypeptide confers increased tolerance and/or resistance to environmental stress
and increased biomass production as compared to a corresponding non-transformed wild type plant cell, a plant or part thereof, preferably when said polypeptide is overexpressed.

15. A plant of claim 1 that has
i) an increased biomass production under conditions where water would be limiting for growth for a non-transformed wild type plant cell, a plant or part thereof
ii) an increased biomass production under conditions of drought and/or desiccation where said conditions would be limiting for growth for a non-transformed wild type plant cell, a plant or part thereof
and/or
iii) an increased biomass production under conditions of low humidity where said conditions would be limiting for growth for a non-transformed wild type plant cell, a plant or part thereof.
